# EUROPEAN PATENT APPLICATION

(11) **EP 1 626 086 A2**
(43) Date of publication of application: **15.02.2006**
(21) Application number: 05017879.7
(22) Date of filing: 19.04.1999
(51) Int. Cl.: C12N 15/11, C12N 9/00, A61K 31/713, C07H 21/04

(54) **Double-stranded nucleic acid molecules with novel chemical compositions capable of modulating gene expression**

(30) Priority: 20.04.1998 US 82404 P; 23.06.1998 US 103636
(62) Divisional of application: 99919896.3
(71) Applicant: RIBOZYME PHARMACEUTICALS, INC., Boulder, CO 80301-5411 (US)
(72) Inventor: Thompson, James D., Lafayette CO 80026 (US); Beigelman, Leonid, Longmont CO 80503 (US); McSwiggen, James A., Boulder CO 80301 (US); Karpeisky, Alexander, Lafayette CO 80026 (US); Bellon, Laurent, Frederick MD 21704 (US); Reynolds, Mark, Pleasanton CA 94588 (US); Zwick, Michael, Madison WI 53711 (US); Jarvis, Thale, Boulder CO 80303 (US); Woolf, Tod, Natick MA 01760 (US); Haeberli, Peter, Berthoud CO 80513 (US); Matulic-Adamic, Jasenka, Boulder CO 80303 (US)
(74) Representative: Viering, Jentschura & Partner

(57) **Abstract**

The present invention refers to a chemically synthesized double stranded nucleic acid molecule comprising:
(a) nucleotide sequence having between 14 and 24 nucleotides complementary to nucleotide sequence of a target RNA or a portion thereof;
(b) a 5'-cap, a 3'-cap, or both a 5' and 3'-cap on one of the strands of the double stranded nucleic acid molecule; and
(c) a mixture of modified nucleotides and unmodified nucleotides, wherein the modified nucleotides are 2'-flouro, 2'-O-methyl, 2'H or a combination thereof and the unmodified nucleotides are ribonucleotides,
and pharmaceutical compositions containing the same.

## Description

This patent application relates to the patent application entitled, "NUCLEIC ACID MOLECULES WITH NOVEL CHEMICAL COMPOSITIONS CAPABLE OF MODULATING GENE EXPRESSION", U.S.S.N. 60/082,404, which was filed with the U.S. patent and trademark office on April 20, 1998. The earlier patent application listed Thompson *et al*. as inventors.

### Background Of The Invention

This invention relates to novel chemically modified nucleic acid molecules that are capable of modulating gene expression through a variety of mechanisms. Specifically, the invention concerns novel combinations of chemical modifications in an oligonucleotide which enhance nuclease resistance, binding affinity, and/ or potency.

The following is a discussion of relevant art, none of which is admitted to be prior art to the present invention.

Since the discovery of the mechanisms underlying gene expression, specifically nucleic acid based transcription and translation, a great deal of effort has been placed on blocking or altering these processes for a variety of purposes, such as understanding biology, gene function, disease processes, and identifying novel therapeutic targets. Approaches involving nucleic acid molecules for modulating gene expression have gained popularity in recent years. For example, nucleic acid molecules have been designed which are capable of binding to specific mRNA sequences by Watson-Crick base pairing interaction and blocking translation (Crooke, 1996, *Medicinal Res. Rev.* 16, 319-344). Another approach involves complexation of DNA with triplex forming oligonucleotides to prevent transcription of bound DNA sequences thereby inhibiting gene expression (Kim *et al*., 1998, *Biochemistry.* 37, 2299-2304). The interaction of antisense oligonucleotides, 2-5A antisense chimera, or ribozymes with target RNA have been used to prevent gene expression. All of these nucleic acid molecules are highly specific to their matching target sequences and therefore may offer lower toxicity compared to traditional approaches such as chemotherapy.

The use of oligonucleotides for modulation of gene expression generally requires stabilization of oligonucleotides from degradation by nucleases that are present in biological systems. Cellular efficacy may be effected if the nucleic acid molecule is degraded before it reaches its desired target. Chemical modifications of nucleic acid molecules have been found to be advantageous in making them inaccessible to degradation by cellular nucleases. Uhlmann and Peyman, 1990, *Chem. Reviews* 90, 543, review the use of nucleoside modifications to stabilize antisense oligonucleotides. Besides improved stability, chemical modifications have also been shown to increase binding affinity, improve cellular penetration, and enhanced target specificity (Monia *et al*., 1993, *J. Biol. Chem.* 268,14514-14522; Wu-Pong, 1994, BioPharm, 22-33).

One of the most studied and utilized chemical alteration in oligonucleotides has been backbone modifications such as phosphorothioates. Phosphorothioate oligonucleotides are nucleic acid molecules whose phosphodiester linkage has been modified by substituting a sulfur atom in place of an oxygen atom. In addition to increased nuclease resistance, phosphorothioate oligonucleotides are substrates for ribonuclease H (RNase H) (Monia, *supra*; Crooke *et al.,* 1995*, Biochem. J.* 3112, 599-608). RNase H is an endonuclease which catalyzes the degradation of RNA in an RNA-DNA heteroduplex (Hostomsky *et al.,* 1993 in *Nucleases,* Linn *et al.,* eds., Cold Spring Harbor Laboratory Press, NY, 341-376). RNA/DNA heteroduplexes, called Okazaki fragments, are formed naturally during DNA replication. Therefore, the normal function of RNase H is to degrade the RNA portion of the heteroduplex to complete DNA replication. In experiments with *E. coli* RNase H, the phosphorothioate oligonucleotide activated the enzyme more efficiently (2-5 fold) compared to a standard phosphodiester containing oligonucleotide (Crooke, 1995, *supra).*

Binding of DNA to RNA is not as thermodynamically favorable as an RNA to RNA interaction (Altmann *et al.,* 1996, *Chimia* 50, 168-176). Inoe & Ohtsuka, 1987, *Nucleic Acids Research* 115, 6131, first proposed an oligonucleotide with a central region consisting of oligodeoxynucleotides flanked by 2'-*O*-methyl modified nucleotide regions. The region of oligodeoxynucleotides in such a chimeric molecule is recognized by RNase H when bound to target RNA; and facilitates cleavage of target RNA by RNase H. (Inoe & Ohtsuka, 1987, *FEBS* Lett. 215, 327; Shibahara & Morisava, 1987, *Nucleic Acids Res.* 15*,* 4403). Such chimeric oligonucleotides were proposed to interact with target RNA more stably than an all DNA oligonucleotide.

Subsequent developments included the introduction of nuclease resistant modifications of the chimeric oligonucleotides, such as methylphosphonates (Tidd & Gibson, 1988, *Anticancer Drug Design* 3, 117), phosphorothioates (Agrawal & Pederson, 1990, *Proc Nat. Acad. Sci. USA* 87, 1407), and phosphoramidates (Potts & Runyun, 1991, *Proc Nat. Acad Sci. USA* 88, 1516). Additionally, the flanking sequences have been modified with 2'-*O*-methyl and 2'-*F*-modifications (Cook, 1993, *Antisense Research and Applications,* CRC Press, 150-181).

Agrawal et al., US Patent No. 5,652,355, describe a phosphorothioate-containing nucleic acid molecule with at least two 2'-*O*-methyl modifications on the 5' and 3' ends.

Agrawal, US Patent No. 5,652,356, describes an oligonucleotide which consists of a region of 2'-*O*-substituted oligonucleotide located between two oligodeoxyribonucleotide regions. The DNA regions of this nucleic acid molecule consists of phosphorothioate modifications at every position.

Cook *et al.,* US Patent No. 5,623,065, describe the use of a nucleic acid molecule which contains an RNase H cleavable region flanked by certain specifically modified nucleotides, for inhibition of gene expression of a ras gene.

Cook *et al.,* US Patent No. 5,587,362, describe a nucleic acid molecule having "substantially chirally pure inter-sugar linkages", for modulation of gene expression.

Ohtsuka *et al*., US Patent No. 5,013,830, describe mixed oligomers having a DNA region and a 2'-*O*-methyl modified region, useful for modulation of gene expression.

Walder *et al*., US Patent No. 5,491,133, describe a method for modulating gene expression using chimeric oligonucleotides with 3'-phosphodiester linkage modifications.

Cohen *et al*., US Patent No. 5,276,019, and Cohen *et al*., US Patent No. 5,264,423 describe the use of oligodeoxynucleotides of no more than 32 nucleotides in length, containing at least one phosphorothioate internucleoside linkage which are capable of preventing foreign nucleic acid replication.

Cohen *et al.,* US Patent No. 5,286,717, describe an oligodeoxyribonucleotide with at least one phosphorothioate modification capable of inhibiting oncogenes.

Sproat *et al*., US Patent No. 5,334,711, describe 2'-O-R modified hammerhead and hairpin ribozymes, where R IS ALKYL, ALKYNYL OR ALKENYL.

Crooke *et al*., 1996, *Exp. Opin. Ther. Patents* 6, 855, list and discuss various patents and PCT publications in the field of antisense technology.

Sproat *et al*., US Patent No. 5,678,731, describe 2'-*O*-R modified oligonucleotides where R IS ALKYL, ALKYNYL OR ALKENYL.

Usman *et al*., US Patent No. 5,652,094, describe enzymatic nucleic acid molecules which include nucleic acid analogues or deoxyribonucleotides.

Joyce, International Publication No. WO 96/17086, describes a DNA enzyme capable of cleaving RNA.

Rossi *et al*., US Patent No. 5,144,019, describe chimeric hammerhead ribozymes with the binding arms and stem II region modified with deoxyribonucleotides.

Molecules have also been devised which include non-nucleotides capable of binding to nucleic acid. These peptide nucleic acid (PNA) molecules bind by Watson-Crick base-pairing and may also function through an antisense mechanism. These molecules have been used to augment hammerhead ribozyme activity by altering the structure of target RNAs and increasing accessibility of cleavage sites (Jankowsky *et al.,* 1997, *Nucleic Acids Research* 25, 2690-2693).

### Summary of the Invention

This invention relates to novel nucleic acid molecules which are useful for modulation of gene expression. The nucleic acid molecule of the instant invention are distinct from other nucleic acid molecules known in the art. Specifically, the nucleic acid molecules of the present invention have novel combinations of chemical modifications and are capable of binding to RNA or DNA to facilitate modulation of gene expression. These novel combinations of chemical modifications may be used to form antisense oligonucleotides, triplex forming oligonucleotides, 2-5A antisense chimera, and enzymatic nucleic acid molecules.

In a preferred embodiment, the invention features a nucleic acid molecule having the following formulae:

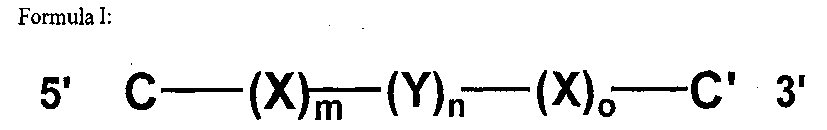

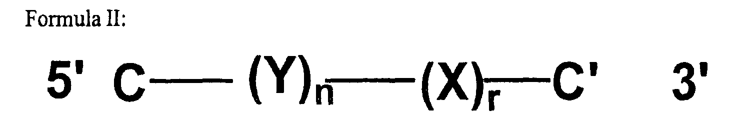

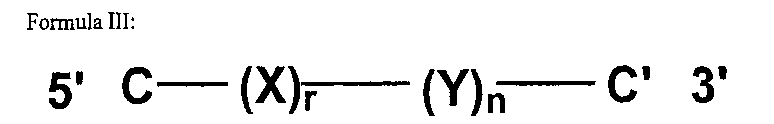

In a preferred embodiment, the invention features an enzymatic nucleic acid molecule having the formula:

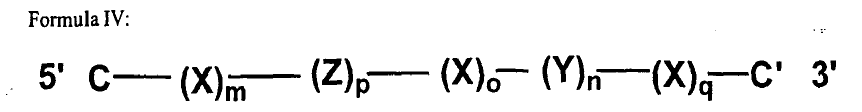

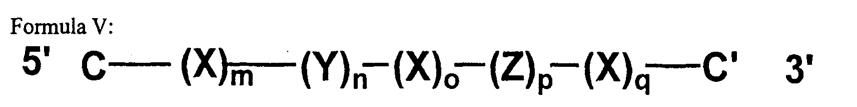

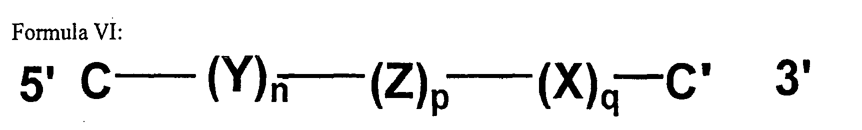

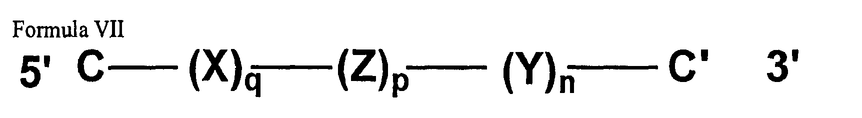

In each of the above formula (I-VII), X represents independently a nucleotide which may be same or different; where m and o are integers independently greater than or equal to 4 and preferably less than about 100, more specifically 5, 6, 7, 8, 9, 10, 11, 12, 15, or 20; r is an integer greater than or equal to four, more specifically 5, 6, 7, 10, 15, or 20; the nucleic acid molecule may be symmetric (m = O) or asymmetric (m O); (X)ₘ, (X)ₒ, and (X)_{q} are oligonucleotides which are of sufficient length to stably interact independently with a target nucleic acid molecule (the target can be an RNA, DNA or RNA/DNA mixed polymers); Y represents independently a deoxyribonucleotide which may be same or different; n is an integer greater than or equal to 4, specifically 5, 6 7, 8, 9, 10, 11, or 12; Z represents an oligonucleotide including nucleotides capable of facilitating the cleavage of a target sequence; p is of length greater than or equal to 4 but less than 100, preferably 5, between 10-20, specifically 25-55, specifically between 30-45, more specifically 35-50; q is an integer greater than or equal to 0, preferably 1, 2, 3, 4, 5, 6, 7, 8, 10, 15, 20; _ represents a chemical linkage (*e*.*g*. a phosphate ester linkage, amide linkage or others known in the art); and each (X)ₘ, (X)ₒ, (X)ᵣ, (X)_{q}, and/or (Y)ₙ independently comprise phosphorothioate linkages, more specifically each (X)ₘ, (X)ₒ, (X)ᵣ, (X)_{q}, and/or (Y)ₙ independently comprise at least one phosphodiester linkage and one phosphorothioate linkage; each C and C' independently represents a cap structure which may independently be present or absent; and (Z)ₚ may optionally include a phosphorothioate linkage. The nucleotides in the each of the formula I-VII are unmodified or modified at the sugar, base, and/or phosphate as known in the art.

Preferably, each of X represents independently a nucleotide which may be same or different; where m and o are integers independently greater than or equal to 5; (X)ₘ and (X)ₒ are oligonucleotides which are of sufficient length to stably interact independently with a target nucleic acid molecule; each (X)ᵣ comprises independently at least one phosphodiester linkage and one phosphorothioate linkage; Y represents independently a deoxyribonucleotide which may be same or different; (Y)ₙ is an oligonucleotide which is of sufficient length to stably interact independently with a target nucleic acid molecule; n is an integer greater than or equal to 4; each (X)ₘ, and (X)ₒ comprise independently at least one phosphodiester linkage and one phosphorothioate linkage; (Y)ₙ comprises a phosphorothioate linkage or a phosphorodithioate linkage or a 5'-S-phosphorothioate, or 5'-S-phosphorodithioate, or a 3'-S-phosphorothioate or a 3'-S-phosphorodithioate linkage or a mixture thereof; and each C and C' independently represents a cap structure which may independently be present or absent.

By "nucleotide" as used herein is as recognized in the art to include natural bases (standard), and modified bases well known in the art. Such bases are generally located at the 1' position of a sugar moiety. Nucleotide generally comprise a base, sugar and a phosphate group. The nucleotides can be unmodified or modified at the sugar, phosphate and/or base moiety, (also referred to interchangeably as nucleotide analogs, modified nucleotides, non-natural nucleotides, non-standard nucleotides and other; see for example, Usman and McSwiggen, *supra;* Eckstein *et al.,* International PCT Publication No. WO 92/07065; Usman *et al*., International PCT Publication No. WO 93/15187; Uhlman & Peyman, *supra*) all are hereby incorporated by reference herein). There are several examples of modified nucleic acid bases known in the art and has recently been summarized by Limbach *et al.,* 1994, *Nucleic Acids Res.* 22, 2183. Some of the non-limiting examples of base modifications that can be introduced into nucleic acids include, inosine, purine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidines (*e*.*g*., 5-methylcytidine), 5-alkyluridines (*e.g.,* ribothymidine), 5-halouridine (*e.g.,* 5-bromouridine) or 6-azapyrimidines or 6-alkylpyrimidines (*e*.*g*. 6-methyluridine), propyne, and others (Burgin *et al.,* 1996, *Biochemistry*, 35, 14090; Uhlman & Peyman, *supra*)*.* By "modified bases" in this aspect is meant nucleotide bases other than adenine, guanine, cytosine and uracil at 1' position or their equivalents; such bases may be used at any position, for example, within the catalytic core of an enzymatic nucleic acid molecule and/or in the substrate-binding regions of the nucleic acid molecule.

By "unmodified nucleotide" is meant a nucleotide with one of the bases adenine, cytosine, guanine, thymine, uracil joined to the 1' carbon of β-D-ribo-furanose.

By "modified nucleotide" is meant a nucleotide which contains a modification in the chemical structure of an unmodified nucleotide base, sugar and/or phosphate.

By "sufficient length" is meant an oligonucleotide of greater than or equal to 4 nucleotides.

By "stably interact" is meant, interaction of the oligonucleotides with target nucleic acid (*e*.*g*., by forming hydrogen bonds with complementary nucleotides in the target under physiological conditions). The interaction is stable either alone or in conjunction with (Y)ₙ and (Z)ₚ where applicable.

By "chimeric nucleic acid molecule" or "chimeric oligonucleotide" is meant that, the molecule may be comprised of both modified or unmodified DNA or RNA.

By "cap structure" is meant chemical modifications which have been incorporated at the terminus of the oligonucleotide. These terminal modifications protect the nucleic acid molecule from exonuclease degradation, and may help in delivery and/or localization within a cell.

In another preferred embodiment (X)ₘ, (X)ₒ, (X)_{q}, (Y)ₙ and/or (Z)ₚ independently include modifications selected from a group comprising 2'-*O*-alkyl (*e*.*g*. 2'-*O*-allyl; Sproat *et al., supra*)*;* 2'-O-alkylthioalkyl (*e.g.* 2'-*O*-methylthiomethyl; Karpeisky *et al.,* 1998, *Nucleosides & Nucleotides* 16, 955-958); L-nucleotides (Tazawa *et al.,* 1970, *Biochemistry* 3499; Ashley, 1992, *J. Am. Chem. Soc.* 114, 9731; Klubmann *et al.,* 1996, Nature Biotech 14, 1112); 2'-*C*-alkyl (Beigelman *et al.,* 1995,*J*. *Biol. Chem.* 270, 25702); 1-5-Anhydrohexitol; 2,6-diaminopurine (Strobel *et al.,* 1994, *Biochem.* 33, 13824-13835); 2'-(*N-*alanyl) amino-2'-deoxynucleotide; 2'-(*N-*beta-alanyl) amino; 2'-deoxy-2'-(lysyl) amino; 2'-*O*-amino (Karpeisky *et al*., 1995, *Tetrahedron Lett.* 39, 1131); 2'-deoxy-2'-(*N-*histidyl) amino; 5-methyl (Strobel, *supra*)*;* 2'-(*N*-b-carboxamidine-beta-alanyl) amino; 2'-deoxy-2'-(N-beta-alanyl) (Matulic-Adamic *et al*., 1995, *Bioorg. & Med. Chem. Lett.* 5,2721-2724); xylofuranosyl (Rosemeyer *et al*., 1991, *Helvetica Chem. Acta,* 74, 748; Seela *et al*., 1994, *Helvetica Chem. Acta,* 77, 883; Seela *et al*., 1996, *Helvetica Chem. Acta,* 79,1451).

In a preferred embodiment, the invention features a nucleic acid molecule of any of formula I-VII, where each X and/or Z, independently include a nucleotide modification having formula IX:

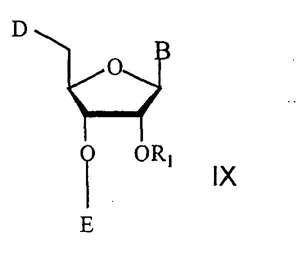

Where, each B is independently a modified or an unmodified nucleic acid base; R1 is independently a fluoroalkyl or an alkylthiofluoroalkyl; E is independently a phosphorus-containing group; and D is independently an O, blocking group or a phosphorus-containing group.

In another preferred embodiment, the invention features a nucleic acid molecule of any of formula I-VII, where each X and/or Z, independently include a nucleotide modification having formula X:

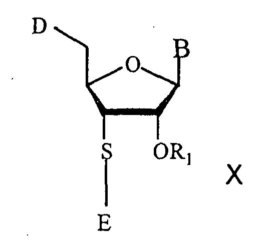

Wherein, each B is independently a modified or an unmodified nucleic acid base; R1 is independently an aklyl, an alkylthioalkyl, a fluoroalkyl or an alkylthiofluoroalkyl; E is independently a phosphorus-containing group; and D is independently an O, blocking group or a phosphorus-containing group.

An "alkyl" group refers to a saturated aliphatic hydrocarbon, including straight-chain, branched-chain, and cyclic alkyl groups. Preferably, the alkyl group has 1 to 12 carbons. More preferably it is a lower alkyl of from 1 to 7 carbons, more preferably 1 to 4 carbons. The alkyl group may be substituted or unsubstituted. When substituted the substituted group(s) is preferably, hydroxyl, cyano, alkoxy, =O, =S, NO₂ or N(CH₃)₂, amino, or SH. The term also includes alkenyl groups which are unsaturated hydrocarbon groups containing at least one carbon-carbon double bond, including straight-chain, branched-chain, and cyclic groups. Preferably, the alkenyl group has 1 to 12 carbons. More preferably it is a lower alkenyl of from 1 to 7 carbons, more preferably 1 to 4 carbons. The alkenyl group may be substituted or unsubstituted. When substituted the substituted group(s) is preferably, hydroxyl, cyano, alkoxy, =O, =S, NO₂, halogen, N(CH₃)₂, amino, or SH. The term "alkyl" also includes alkynyl groups which have an unsaturated hydrocarbon group containing at least one carbon-carbon triple bond, including straight-chain, branched-chain, and cyclic groups. Preferably, the alkynyl group has 1 to 12 carbons. More preferably it is a lower alkynyl of from 1 to 7 carbons, more preferably 1 to 4 carbons. The alkynyl group may be substituted or unsubstituted. When substituted the substituted group(s) is preferably, hydroxyl, cyano, alkoxy, =O, =S, NO₂ or N(CH₃)₂, amino or SH.

Such alkyl groups may also include aryl, alkylaryl, carbocyclic aryl, heterocyclic aryl, amide and ester groups. An "aryl" group refers to an aromatic group which has at least one ring having a conjugated p electron system and includes carbocyclic aryl, heterocyclic aryl and biaryl groups, all of which may be optionally substituted. The preferred substituent(s) of aryl groups are halogen, trihalomethyl, hydroxyl, SH, OH, cyano, alkoxy, alkyl, alkenyl, alkynyl, and amino groups. An "alkylaryl" group refers to an alkyl group (as described above) covalently joined to an aryl group (as described above. Carbocyclic aryl groups are groups wherein the ring atoms on the aromatic ring are all carbon atoms. The carbon atoms are optionally substituted. Heterocyclic aryl groups are groups having from 1 to 3 heteroatoms as ring atoms in the aromatic ring and the remainder of the ring atoms are carbon atoms. Suitable heteroatoms include oxygen, sulfur, and nitrogen, and include furanyl, thienyl, pyridyl, pyrrolyl, N-lower alkyl pyrrolo, pyrimidyl, pyrazinyl, imidazolyl and the like, all optionally substituted. An "amide" refers to an -C(O)-NH-R, where R is either alkyl, aryl, alkylaryl or hydrogen. An "ester" refers to an -C(O)-OR', where R is either alkyl, aryl, alkylaryl or hydrogen.

A "blocking group" is a group which is able to be removed after polynucleotide synthesis and/or which is compatible with solid phase polynucleotide synthesis.

A "phosphorus containing group" can include phosphorus in forms such as dithioates, phosphoramidites and/or as part of an oligonucleotide.

In yet another preferred embodiment C' is selected from a group comprising inverted abasic residue, 4',5'-methylene nucleotide; 1-(beta-D-erythrofuranosyl) nucleotide, 4'-thio nucleotide, carbocyclic nucleotide; 1,5-anhydrohexitol nucleotide; L-nucleotides; alpha-nucleotides; modified base nucleotide; phosphorodithioate linkage; *threo*-pentofuranosyl nucleotide; acyclic 3',4'-seco nucleotide; acyclic 3,4-dihydroxybutyl nucleotide; acyclic 3,5-dihydroxypentyl nucleotide, 3'-3'-inverted nucleotide moiety; 3'-3'-inverted abasic moiety; 3'-2'-inverted nucleotide moiety; 3'-2'-inverted abasic moiety; 1,4-butanediol phosphate; 3'-phosphoramidate; hexylphosphate; aminohexyl phosphate; 3'-phosphate; 3'-phosphorothioate; phosphorodithioate; or bridging or non-bridging methylphosphonate moiety (for more details see Beigelman *et al*., International PCT publication No. WO 97/26270, incorporated by reference herein).

In yet another preferred embodiment C is selected from a group comprising, 4',5'-methylene nucleotide; 1-(beta-D-erythrofuranosyl) nucleotide; 4'-thio nucleotide, carbocyclic nucleotide; 5'-amino-alkyl phosphate; 1,3-diamino-2-propyl phosphate, 3-aminopropyl phosphate; 6-aminohexyl phosphate; 1,2-aminododecyl phosphate; hydroxypropyl phosphate; 1,5-anhydrohexitol nucleotide; L-nucleotide; alpha-nucleotide; modified base nucleotide; phosphorodithioate; *threo*-pentofuranosyl nucleotide; acyclic 3',4'-seco nucleotide; 3,4-dihydroxybutyl nucleotide; 3,5-dihydroxypentyl nucleotide, 5'-5'-inverted nucleotide moeity; 5'-5'-inverted abasic moeity; 5'-phosphoramidate; 5'-phosphorothioate; 1,4-butanediol phosphate; 5'-amino; bridging and/or non-bridging 5'-phosphoramidate, phosphorothioate and/or phosphorodithioate, bridging or non bridging methylphosphonate and 5'-mercapto moeities (for more details see Beaucage and lyer, 1993, *Tetrahedron* 49, 1925; incorporated by reference herein).

In another preferred embodiment (Z)ₚ includes a non-nucleotide linker. Thus, in a preferred embodiment, the invention features an enzymatic nucleic acid molecule having one or more non-nucleotide moieties, and having enzymatic activity to cleave an RNA or DNA molecule. By the term "non-nucleotide" is meant any group or compound which can be incorporated into a nucleic acid chain in the place of one or more nucleotide units, including either sugar and/or phosphate substitutions, and allows the remaining bases to exhibit their enzymatic activity. The group or compound is abasic in that it does not contain a commonly recognized nucleotide base, such as adenosine, guanine, cytosine, uracil or thymine. The terms "abasic" or "abasic nucleotide" as used herein encompass sugar moieties lacking a base or having other chemical groups in place of base at the 1' position.

By the phrase "enzymatic nucleic acid" is meant a nucleic acid molecule capable of catalyzing (altering the velocity and/or rate of) a variety of reactions including the ability to repeatedly cleave other separate nucleic acid molecules (endonuclease activity) in a nucleotide base sequence-specific manner. Such a molecule with endonuclease activity may have complementarity in a substrate binding region (*e*.*g*. (X)ₘ, (X)ₒ, (X)_{q} and (Y)ₙ in formulae IV-VII) to a specified gene target, and also has an enzymatic activity that specifically cleaves RNA or DNA in that target. That is, the nucleic acid molecule with endonuclease activity is able to intramolecularly or intermolecularly cleave RNA or DNA and thereby inactivate a target RNA or DNA molecule. This complementarity functions to allow sufficient hybridization of the enzymatic RNA molecule to the target RNA or DNA to allow the cleavage to occur. 100% complementarity is preferred, but complementarity as low as 50-75% may also be useful in this invention. The nucleic acids may be modified at the base, sugar, and/or phosphate groups. The term enzymatic nucleic acid is used interchangeably with phrases such as ribozymes, catalytic RNA, enzymatic RNA, catalytic DNA, nucleozyme, DNAzyme, RNA enzyme, endoribonuclease, endonuclease, minizyme, leadzyme, oligozyme, chimeric ribozyme, chimeric enzymatic nucleic acid, or DNA enzyme. All of these terminologies describe nucleic acid molecules with enzymatic activity.

By "complementarity" is meant a nucleic acid that can form hydrogen bond(s) with other RNA sequence by either traditional Watson-Crick or other non-traditional types (for example, Hoogsteen type) of base-paired interactions.

By "oligonucleotide" as used herein, is meant a molecule comprising two or more nucleotides.

By "enzymatic portion" is meant that part of the enzymatic nucleic acid molecule essential for cleavage of a nucleic acid substrate

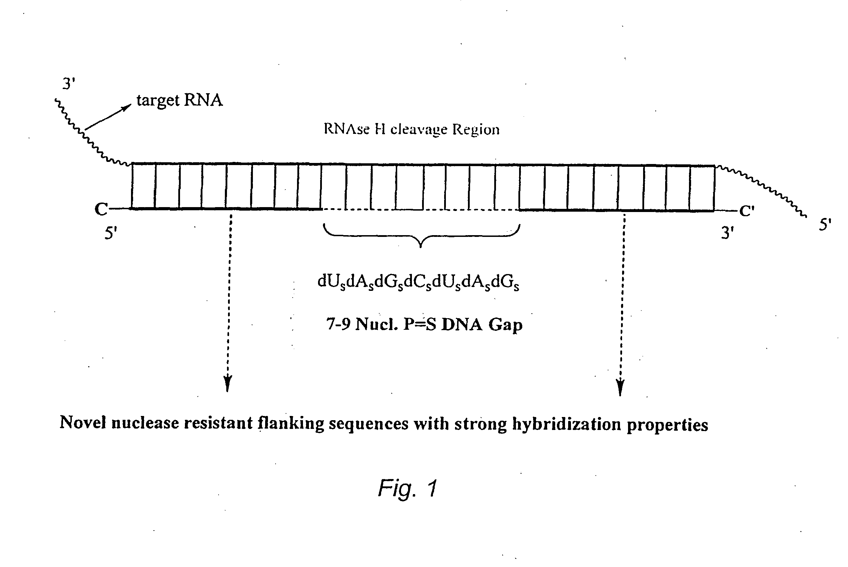

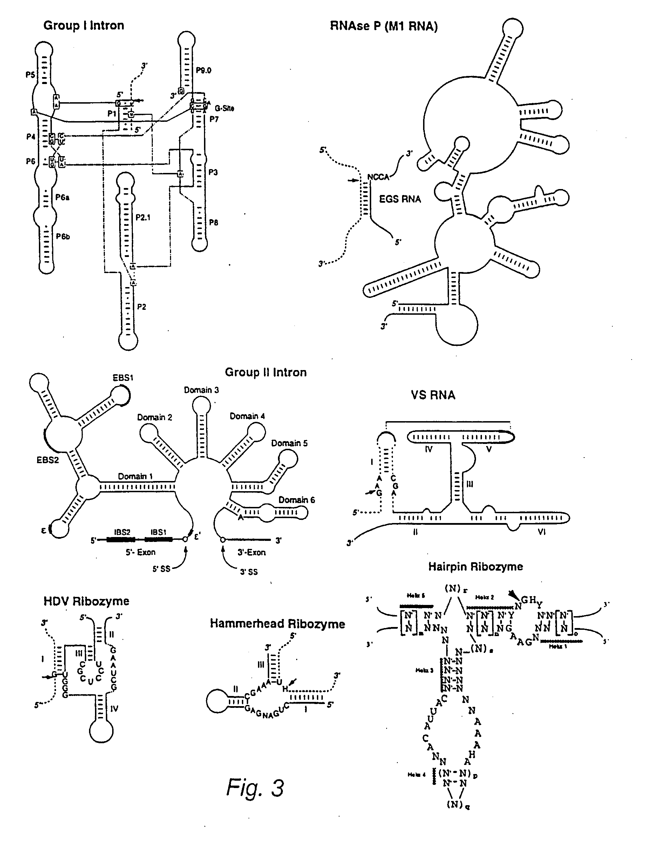

By "substrate binding region" or "substrate binding domain" is meant that portion/region of a nucleic acid molecule (e.g. ribozyme) which is complementary to (*i.e.,* able to base-pair with) a portion of its substrate. Generally, such complementarity is 100%, but can be less if desired. For example, as few as 10 bases out of 14 may be base-paired. Such arms are shown generally in Figures 1 and 3. That is, in a ribozyme example, these arms contain sequences within a ribozyme which are intended to bring ribozyme and target RNA together through complementary base-pairing interactions. The ribozyme of the invention may have binding arms that are contiguous or non-contiguous and may be of varying lengths. The length of the binding arm(s) are preferably greater than or equal to four nucleotides; specifically 12-100 nucleotides; more specifically 14-24 nucleotides long. If two binding arms are chosen, the design is such that the length of the binding arms are symmetrical (*i*.*e*., each of the binding arms is of the same length; *e.g.,* five and five nucleotides, six and six nucleotides or seven and seven nucleotides long) or asymmetrical (*i*.*e*., the binding arms are of different length; *e*.*g*., six and three nucleotides; three and six nucleotides long; four and five nucleotides long; four and six nucleotides long; four and seven nucleotides long; and the like).

By "DNAzyme" or "catalytic DNA" or "DNA enzyme" is meant, an enzymatic nucleic acid molecule lacking a 2'-OH group.

By "nucleic acid molecule" as used herein is meant a molecule comprising nucleotides. The nucleic acid can be composed of modified or unmodified nucleotides or non-nucleotides or various mixtures and combinations thereof.

In another preferred embodiment, the nucleic acid molecule of the present invention is conjugated with another moiety including but not limited to abasic nucleotides, polyether, polyamine, polyamides, peptides, carbohydrates, lipid, or polyhydrocarbon compounds. Those skilled in the art will recognize that these molecules may be linked to one or more of any nucleotides comprising the nucleic acid molecule at several positions on the sugar, base or phosphate group.

In yet another preferred embodiment, the nucleic acid molecule of the present invention can form structures including but not limited to antisense, triplexes, 2-5A chimera antisense, or enzymatic nucleic acid (ribozymes).

By "antisense" is meant a non-enzymatic nucleic acid molecule that binds to target RNA, for example, by means of RNA-RNA or RNA-DNA or RNA-PNA (protein nucleic acid; Egholm *et al.,* 1993 *Nature* 365, 566) interactions and alters the activity of the target RNA (for a review see Stein and Cheng, 1993 *Science* 261,1004).

By "2-5A antisense chimera" it is meant, an antisense oligonucleotide containing a 5' phosphorylated 2'-5'-linked adenylate residues. These chimeras bind to target RNA in a sequence-specific manner and activate a cellular 2-5A-dependent ribonuclease which in turn cleaves the target RNA (Torrence *et al.,* 1993 *Proc. Natl. Acad. Sci. USA* 90, 1300).

By "triplex DNA" it is meant an oligonucleotide that can bind to a double-stranded DNA in a sequence-specific manner to form a triple-strand helix. Triple-helix formation has been shown to inhibit transcription of the targeted gene (Duval-Valentin *et al.,* 1992 *Proc. Natl. Acad. Sci. USA* 89, 504).

In another preferred embodiment, the invention features an antisense oligonucleotide which is capable of interacting with the target RNA and sterically blocking translation, where the oligonucleotide has a 5' and a 3' Cap structure and the oligonucleotide may include modifications at the base, sugar or the phosphate groups.

Other features and advantages of the invention will be apparent from the following description of the preferred embodiments thereof, and from the claims.

### Description of the Preferred Embodiments

The drawings will first briefly be described.

### Drawings:

Figure 1 is a diagrammatic representation of a nucleic acid molecule with 7-9 phosphorothioate oligodeoxyribonucleotide sequence flanked by 9 non-deoxyribonucleotide containing oligonucleotides, binding to a target molecule.

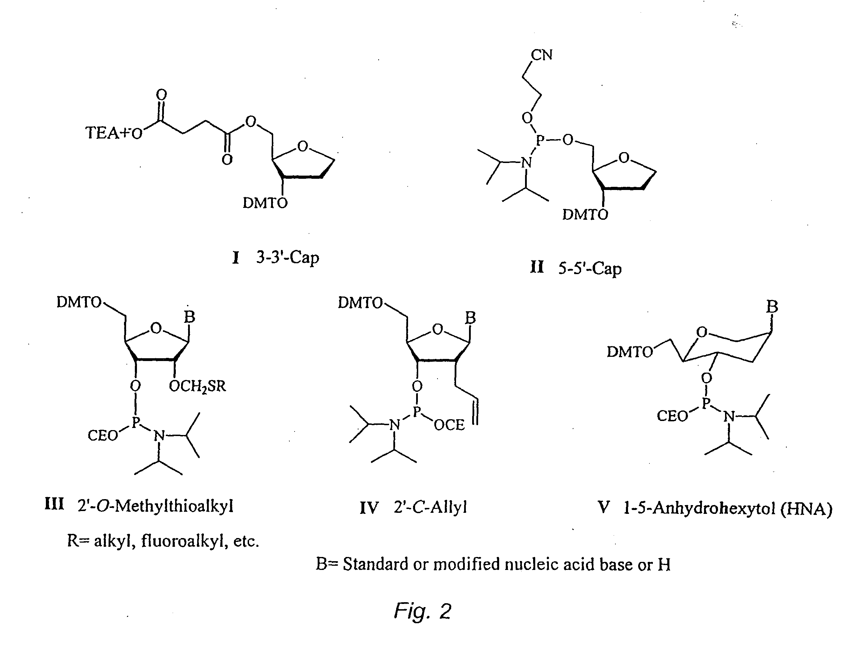

Figure 2 displays schematic representations of certain chemical structures which may be incorporated into the nucleic acid molecule of the invention.
Figure 3 shows the secondary structure model for seven different classes of enzymatic nucleic acid molecules. Arrow indicates the site of cleavage. --------- indicate the target sequence. Lines interspersed with dots are meant to indicate tertiary interactions. - is meant to indicate base-paired interaction. **Group I Intron:** P1-P9.0 represent various stem-loop structures (Cech *et al*., 1994, *Nature Struc. Bio.,* 1, 273). **RNase P (M1RNA):** EGS represents external guide sequence (Forster *et al*., 1990, *Science,* 249, 783; Pace *et al*., 1990, *J. Biol. Chem.,* 265, 3587). **Group II Intron:** 5'SS means 5' splice site; 3' SS means 3'-splice site; IBS means intron binding site; EBS means exon binding site (Pyle *et al*., 1994, *Biochemistry,* 33, 2716). **VS RNA:** I-VI are meant to indicate six stem-loop structures; shaded regions are meant to indicate tertiary interaction (Collins, International PCT Publication No. WO 96/19577). **HDV Ribozyme: :** I-IV are meant to indicate four stem-loop structures (Been *et al.,* US Patent No. 5,625,047). **Hammerhead Ribozyme: :** I-III are meant to indicate three stem-loop structures; stems I-III can be of any length and may be symmetrical or asymmetrical (Usman *et al.,* 1996, *Curr. Op. Struct. Bio.,* 1, 527). **Hairpin Ribozyme:** Helix 1, 4 and 5 can be of any length; Helix 2 is between 3 and 8 base-pairs long; Y is a pyrimidine; Helix 2 (H2) is provided with a least 4 base pairs (*i*.*e*., n is 1, 2, 3 or 4) and helix 5 can be optionally provided of length 2 or more bases (preferably 3 - 20 bases, *i*.*e*., m is from 1 - 20 or more). Helix 2 and helix 5 may be covalently linked by one or more bases (*i.e.,* r is 1 base). Helix 1, 4 or 5 may also be extended by 2 or more base pairs (*e.g.,* 4 - 20 base pairs) to stabilize the ribozyme structure, and preferably is a protein binding site. In each instance, each N and N' independently is any normal or modified base and each dash represents a potential base-pairing interaction. These nucleotides may be modified at the sugar, base or phosphate. Complete base-pairing is not required in the helices, but is preferred. Helix 1 and 4 can be of any size (*i.e.,* o and p is each independently from 0 to any number, *e.g.,* 20) as long as some base-pairing is maintained. Essential bases are shown as specific bases in the structure, but those in the art will recognize that one or more may be modified chemically (abasic, base, sugar and/or phosphate modifications) or replaced with another base without significant effect. Helix 4 can be formed from two separate molecules, i. e., without a connecting loop. The connecting loop when present may be a ribonucleotide with or without modifications to its base, sugar or phosphate. "q" is 2 bases. The connecting loop can also be replaced with a non-nucleotide linker molecule. H refers to bases A, U, or C. Y refers to pyrimidine bases. "_" refers to a covalent bond. (Burke *et al*., 1996, *Nucleic Acids & Mol. Biol*., 10, 129; Chowrira *et al.,* US Patent No. 5,631,359).
Figure 4 is a graph comparing cell proliferation rates of MCF-7 cells treatment with active and inactive ribozyme with mismatch arms targeted to estrogen receptor delivered with GSV transfection reagent. The sequence for the active ribozyme is given as Seq. ID. No.2725 and the inactive is 2726.
Figure 5 is a graph comparing RNA levels of c-raf RNA in PC-3 cells following treatment with antisense (Seq. ID. No 2717) and scrambled antisense (mismatch) controls.

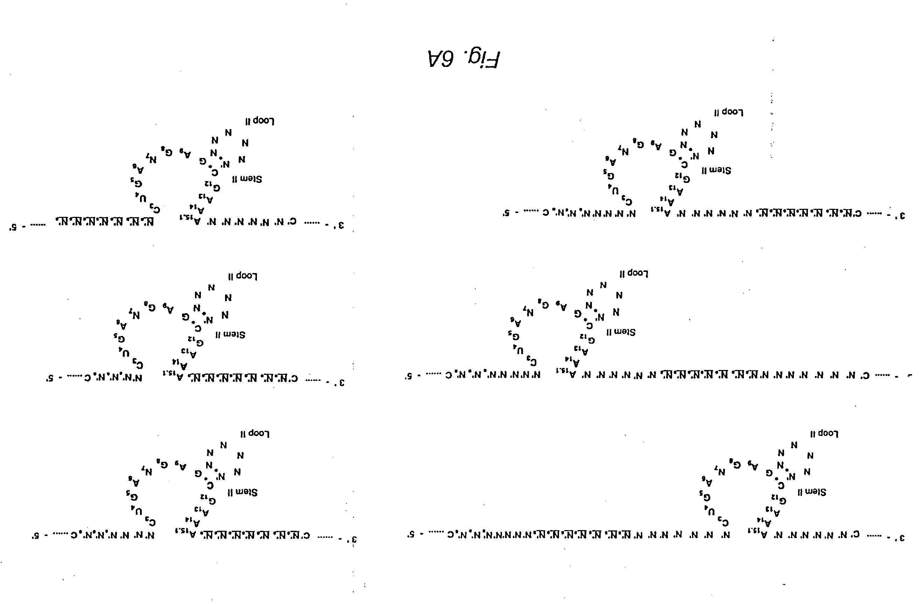

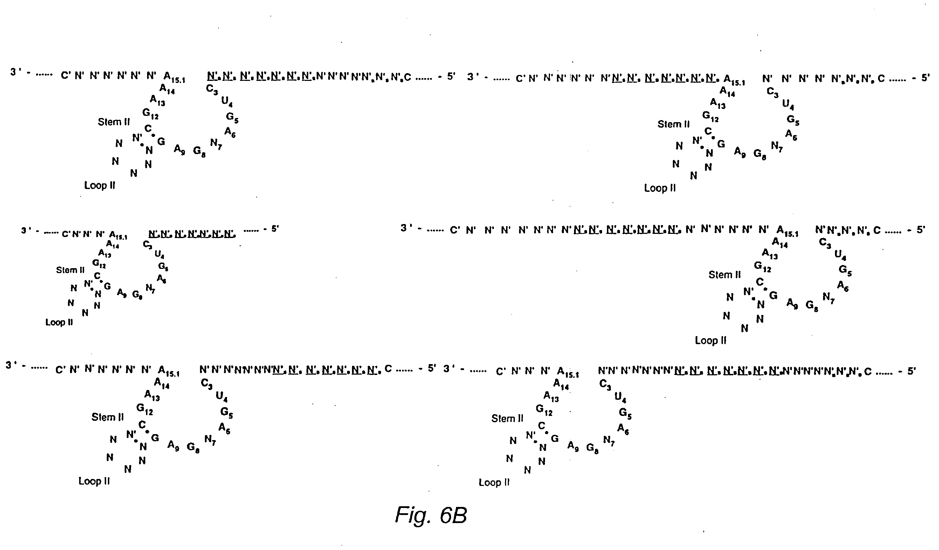

Figure 6a and 6b displays several possible ribozymes comprising oligodeoxyribonucleotides. The symbols used in the diagram include: N' represents a nucleotide complementary to a nucleotide on the target molecule; N7 represents position 7 in the ribozyme molecule (Hertel, K. J., *et al.,* 1992, *Nucleic Acids Res.,* 20, 3252); N' represents a deoxyribonucleotide complementary to a nucleotide on the target molecule; s represents a phosphorothioate modification; C represents a chemical modification at the 5' end of the ribozyme; and C' represents a chemical modification at the 3' end.
Figure 7 shows examples of nucleotide modifications for incorporation into oligonucleotides.
Figure 8 is a graph demonstrating the level of c-raf mRNA in PC-3 cells following treatment with c-raf sequence targeting antisense nucleic acid molecules. The antisense molecules target regions within the intron/exon junction (Seq. I.D. Nos.2731-2735), intron (Seq. I.D. 2736) and exon (Seq. I.D. 2737). The results demonstrate the molecules' ability to inhibit c-raf mRNA compared to the untreated or mismatch control at two concentrations.
Figure 9 is a graph which demonstrates the ability of an antisense molecule represented by Seq. I.D. No. 2738 (phosphorothioate modifications at every deoxynucleotide position) to inhibit c-raf message in PC-3 cells over a period of five days compared to the untreated and mismatch controls at three different concentrations.
Figure 10 is a graph which demonstrates the ability of an antisense molecule represented by Seq. I.D. No. 2737 (three phosphorothoate modified nucleotides at both the 5' and 3' ends of the oligonucleotide) to inhibit c-raf message in PC-3 cells over a period of five days compared to the untreated control at three different concentrations.

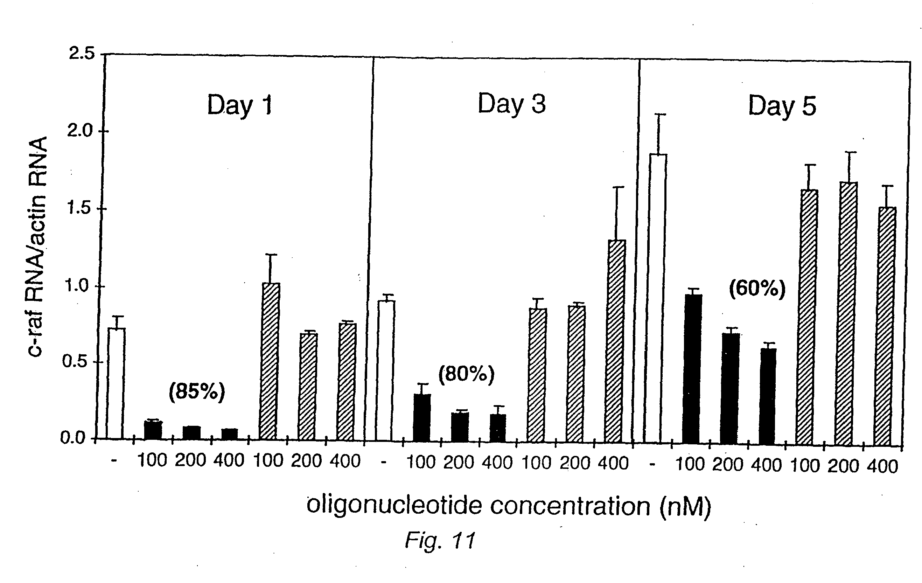

Figure 11 is a graph which demonstrates the ability of an antisense molecule represented by Seq. I.D. No. 2744 (9 phosphorothioate modified DNA flanked by 7 2'-O-methylthiomethyl RNA nucleotides at the 5' and 3' end) to inhibit c-raf message in PC-3 cells over a period of five days compared to the untreated and mismatch control at three different concentrations.

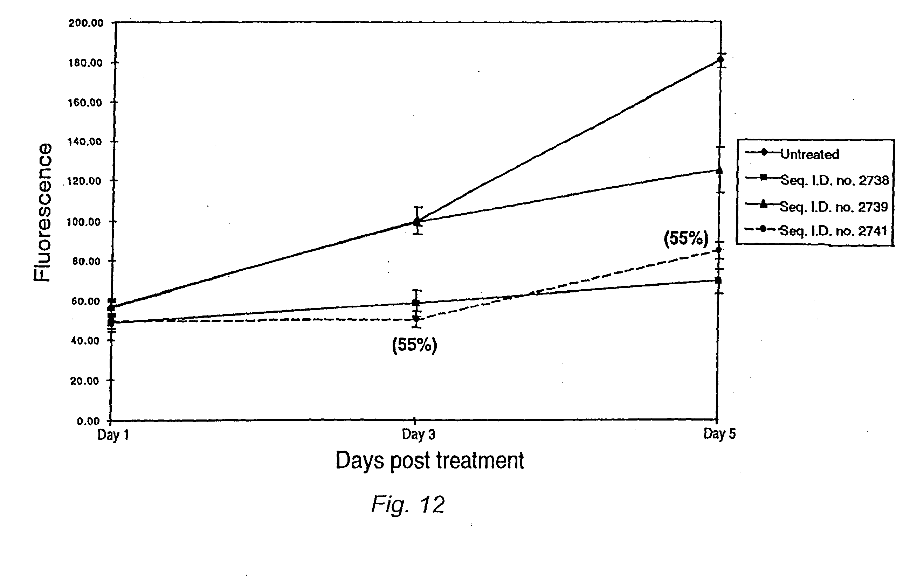

Figure 12 is a graph showing the level of cellular proliferation inhibition exhibited by antisense oligonucleotides represented by Seq. I.D. Nos. 2738 and 2741. Also shown within the graph is the cellular proliferation after treatment of cells with a mismatch control (Seq. I.D. 2739).
Figure 13 is a graph showing the ability of oligonucleotides with different chemical modifications to inhibit c-raf mRNA. Each antisense molecule is compared to an untreated and mismatch control.
Figure 14 is a graph demonstrating a dose dependent inhibition of C-raf in PC-3 cells following treatment with antisense oligonucleotides (Seq. I.D. Nos. 2741 and 2738).
Figure 15 is a graph showing inhibition of bcl-2 mRNA by antisense oligonucleotides compared to untreated and mismatch controls.
Figure 16 is a graph showing the ability of several k-ras targeting antisense molecules to inhibit k-ras message
Figure 17 is a graph showing the ability of oligonucleotides with different chemical modifications to inhibit estrogen receptor mRNA. Each antisense molecule is compared to an untreated and mismatch control.
Figure 18 shows a scheme for the synthesis of 3'-deoxy-3'-thio guanosine nucleoside (scheme 1).
Figure 19 shows a scheme for the synthesis of S-(pyridyl-2-disulfanyl) derivative (scheme 2).
Figure 20 shows a scheme for the synthesis of 3'-deoxy-3'-thio guanosine phosphoramidite.
Figure 21 shows a scheme for the preparation of 5'-thio-nucleoside phosphoramidite and succinates.
Figure 22 shows a scheme for the synthesis of 3'-thio-2'-*O*-methyl uridine.

### Synthesis of Nucleic acid Molecules

Synthesis of nucleic acids greater than 100 nucleotides in length is difficult using automated methods, and the therapeutic cost of such molecules is prohibitive. In this invention, small nucleic acid motifs (*e*.*g*., antisense oligonucleotides, hammerhead or the hairpin ribozymes) are used for exogenous delivery. The simple structure of these molecules increases the ability of the nucleic acid to invade targeted regions of RNA structure. The molecules of the instant invention were chemically synthesized. Oligodeoxyribonucleotides were synthesized using standard protocols as described in Caruthers *et al.,* 1992, *Methods in Enzymology* 211,3-19, and is incorporated by reference.

The method of synthesis used for normal RNA including certain enzymatic nucleic acid molecules follows the procedure as described in Usman *et al.,* 1987 *J*. *Am. Chem. Soc.,* 109, 7845; Scaringe *et al*., 1990 *Nucleic Acids Res.,* 18, 5433; and Wincott *et al*., 1995 *Nucleic Acids Res.* 23, 2677-2684 and makes use of common nucleic acid protecting and coupling groups, such as dimethoxytrityl at the 5'-end, and phosphoramidites at the 3'-end. In a non-limiting example, small scale synthesis were conducted on a 394 Applied Biosystems, Inc. synthesizer using a modified 2.5 µmol scale protocol with a 5 min coupling step for alkylsilyl protected nucleotides and 2.5 min coupling step for 2'-*O-*methylated nucleotides. Table II outlines the amounts, and the contact times, of the reagents used in the synthesis cycle. A 6.5-fold excess (163 µL of 0.1 M = 16.3 µmol) of phosphoramidite and a 24-fold excess of S-ethyl tetrazole (238 µL of 0.25 M = 59.5 µmol) relative to polymer-bound 5'-hydroxyl was used in each coupling cycle. Average coupling yields on the 394 Applied Biosystems, Inc. synthesizer, determined by colorimetric quantitation of the trityl fractions, were 97.5-99%. Other oligonucleotide synthesis reagents for the 394 Applied Biosystems, Inc. synthesizer; detritylation solution was 2% TCA in methylene chloride (ABI); capping was performed with 16% *N*-methyl imidazole in THF (ABI) and 10% acetic anhydride/10% 2,6-lutidine in THF (ABI); oxidation solution was 16.9 mM I₂, 49 mM pyridine, 9% water in THF (Millipore). B & J Synthesis Grade acetonitrile was used directly from the reagent bottle. *S*-Ethyl tetrazole solution (0.25 M in acetonitrile) was made up from the solid obtained from American International Chemical, Inc.

Deprotection of the RNA was performed as follows. The polymer-bound oligoribonucleotide, trityl-off, was transferred from the synthesis column to a 4mL glass screw top vial and suspended in a solution of methylamine (MA) at 65 °C for 10 min. After cooling to -20 °C, the supernatant was removed from the polymer support. The support was washed three times with 1.0 mL of EtOH:MeCN:H₂O/3:1:1, vortexed and the supernatant was then added to the first supernatant. The combined supernatants, containing the oligoribonucleotide, were dried to a white powder.

The base-deprotected oligoribonucleotide was resuspended in anhydrous TEA·HF/NMP solution (250 µL of a solution of 1.5mL *N*-methylpyrrolidinone, 750 µL TEA and 1.0 mL TEA•3HF to provide a 1.4M HF concentration) and heated to 65°C for 1.5 h. The resulting, fully deprotected, oligomer was quenched with 50 mM TEAB (9 mL) prior to anion exchange desalting.

For anion exchange desalting of the deprotected oligomer, the TEAB solution was loaded onto a Qiagen 500® anion exchange cartridge (Qiagen Inc.) that was prewashed with 50 mM TEAB (10 mL). After washing the loaded cartridge with 50 mM TEAB (10 mL), the RNA was eluted with 2 M TEAB (10 mL) and dried down to a white powder.

Inactive hammerhead ribozymes were synthesized by substituting a U for G₅ and a U for A₁₄ (numbering from Hertel, K. J., *et al*., 1992, *Nucleic Acids Res.,* 20, 3252).

The average stepwise coupling yields were >98% (Wincott *et al.,* 1995 *Nucleic Acids Res.* 23,2677-2684).

Alternatively, the nucleic acid molecules of the present invention can be synthesized separately and joined together by ligation (Moore *et al*., 1992, *Science* 256, 9923; Draper *et al.,* International PCT publication No. WO 93/23569; Shabarova *et al*., 1991, *Nucleic Acids Research* 19, 4247)

### Administration of Nucleic Acid Molecules

Methods for the delivery of nucleic acid molecules is described in Akhtar *et al., 1992, Trends Cell Bio.,* 2, 139; and *Delivery Strategies for Antisense Oligonucleotide Therapeutics,* ed. Akhtar, 1995 which are both incorporated herein by reference. Sullivan *et al*., PCT WO 94/02595, further describes the general methods for delivery of enzymatic RNA molecules. These protocols may be utilized for the delivery of virtually any nucleic acid molecule. Nucleic acid molecules may be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. For some indications, nucleic acid molecules may be directly delivered *ex vivo* to cells or tissues with or without the aforementioned vehicles. Alternatively, the nucleic acid/vehicle combination is locally delivered by direct injection or by use of a catheter, infusion pump or stent. Other routes of delivery include, but are not limited to, intravascular, intramuscular, subcutaneous or joint injection, aerosol inhalation, oral (tablet or pill form), topical, systemic, ocular, intraperitoneal and/or intrathecal delivery. More detailed descriptions of nucleic acid delivery and administration are provided in Sullivan *et al*., supra and Draper *et al*., PCT WO93/23569 which have been incorporated by reference herein.

The molecules of the instant invention can be used as pharmaceutical agents. Pharmaceutical agents prevent, inhibit the occurrence, or treat (alleviate a symptom to some extent, preferably all of the symptoms) of a disease state in a patient.

The negatively charged polynucleotides of the invention can be administered (*e*.*g*., RNA, DNA or protein) and introduced into a patient by any standard means, with or without stabilizers, buffers, and the like, to form a pharmaceutical composition. When it is desired to use a liposome delivery mechanism, standard protocols for formation of liposomes can be followed. The compositions of the present invention may also be formulated and used as tablets, capsules or elixirs for oral administration; suppositories for rectal administration; sterile solutions; suspensions for injectable administration; and the like.

The present invention also includes pharmaceutically acceptable formulations of the compounds described. These formulations include salts of the above compounds, *e*.*g*., acid addition salts, for example, salts of hydrochloric, hydrobromic, acetic acid, and benzene sulfonic acid.

A pharmacological composition or formulation refers to a composition or formulation in a form suitable for administration, *e*.*g*., systemic administration, into a cell or patient, preferably a human. Suitable forms, in part, depend upon the use or the route of entry, for example oral, transdermal, or by injection. Such forms should not prevent the composition or formulation to reach a target cell (*i.e.,* a cell to which the negatively charged polymer is desired to be delivered to). For example, pharmacological compositions injected into the blood stream should be soluble. Other factors are known in the art, and include considerations such as toxicity and forms which prevent the composition or formulation from exerting its effect.

By "systemic administration" is meant *in* vivo systemic absorption or accumulation of drugs in the blood stream followed by distribution throughout the entire body. Administration routes which lead to systemic absorption include, without limitations: intravenous, subcutaneous, intraperitoneal, inhalation, oral, intrapulmonary and intramuscular. Each of these administration routes expose the desired negatively charged polymers, *e*.*g*., nucleic acids, to an accessible diseased tissue. The rate of entry of a drug into the circulation has been shown to be a function of molecular weight or size. The use of a liposome or other drug carrier comprising the compounds of the instant invention can potentially localize the drug, for example, in certain tissue types, such as the tissues of the reticular endothelial system (RES). A liposome formulation which can facilitate the association of drug with the surface of cells, such as, lymphocytes and macrophages is also useful. This approach may provide enhanced delivery of the drug to target cells by taking advantage of the specificity of macrophage and lymphocyte immune recognition of abnormal cells, such as the cancer cells.

The invention also features the use of the a composition comprising surface-modified liposomes containing poly (ethylene glycol) lipids (PEG-modified, or long-circulating liposomes or stealth liposomes). These formulations offer an method for increasing the accumulation of drugs in target tissues. This class of drug carriers resists opsonization and elimination by the mononuclear phagocytic system (MPS or RES), thereby enabling longer blood circulation times and enhanced tissue exposure for the encapsulated drug (Lasic *et al*. *Chem. Rev.* 1995, **95,** 2601-2627; Ishiwata *et al*., *Chem. Pharm. Bull.* 1995, **43,** 1005-1011). Such liposomes have been shown to accumulate selectively in tumors, presumably by extravasation and capture in the neovascularized target tissues (Lasic *et al., Science* 1995, **267**, 1275-1276; Oku *et al.,* 1995, *Biochim. Biophys. Acta,* **1238**, 86-90). The long-circulating liposomes enhance the pharmacokinetics and pharmacodynamics of DNA and RNA, particularly compared to conventional cationic liposomes which are known to accumulate in tissues of the MPS (Liu *et al*., *J*. *Biol. Chem*. 1995, **42**, 24864-24870; Choi *et al*., International PCT Publication No. WO 96/10391; Ansell *et al*., International PCT Publication No. WO 96/10390; Holland *et al*., International PCT Publication No. WO 96/10392; all of these are incorporated by reference herein). Long-circulating liposomes are also likely to protect drugs from nuclease degradation to a greater extent compared to cationic liposomes, based on their ability to avoid accumulation in metabolically aggressive MPS tissues such as the liver and spleen. All of these references are incorporated by reference herein.

The present invention also includes compositions prepared for storage or administration which include a pharmaceutically effective amount of the desired compounds in a pharmaceutically acceptable carrier or diluent. Acceptable carriers or diluents for therapeutic use are well known in the pharmaceutical art, and are described, for example, in *Remington's Pharmaceutical Sciences,* Mack Publishing Co. (A.R. Gennaro edit. 1985) hereby incorporated by reference herein. For example, preservatives, stabilizers, dyes and flavoring agents may be provided. *Id*. at 1449. These include sodium benzoate, sorbic acid and esters of *p*-hydroxybenzoic acid. In addition, antioxidants and suspending agents may be used. *Id.*

A pharmaceutically effective dose is that dose required to prevent, inhibit the occurrence, or treat (alleviate a symptom to some extent, preferably all of the symptoms) of a disease state. The pharmaceutically effective dose depends on the type of disease, the composition used, the route of administration, the type of mammal being treated, the physical characteristics of the specific mammal under consideration, concurrent medication, and other factors which those skilled in the medical arts will recognize. Generally, an amount between 0.1 mg/kg and 100 mg/kg body weight/day of active ingredients is administered dependent upon potency of the negatively charged polymer.

### Mechanism of action of Nucleic Acid Molecules of the Invention

Antisense: Antisense molecules may be RNA or DNA oligonucleotides and primarily function by specifically binding to matching sequences resulting in inhibition of peptide synthesis (Wu-Pong, Nov 1994, *BioPharm,* 20-33). The oligonucleotide binds to target RNA by Watson Crick base-pairing and blocks gene expression by preventing ribosomal translation of the bound sequences. Antisense molecules may also alter protein synthesis by interfering with RNA processing or transport from the nucleus into the cytoplasm (Mukhopadhyay & Roth, 1996, *Crit. Rev. in Oncogenesis* 7, 151-190).

In addition, binding of single stranded DNA to RNA may result in nuclease degradation of the heteroduplex (Wu-Pong, *supra;* Crooke, *supra*)*.* To date, the only backbone modified DNA chemistry which will act as substrates for RNase H are phosphorothioates and phosphorodithioates. In experiments with *E. coli,* the oligodeoxyribonucleotides phosphorothioate modification activated RNase H more efficiently (2-5 fold) compared to the natural phosphodiester containing oligodeoxynucleotide (Crooke, 1995, *supra*). Applicant describes here for the first time that oligonucleotides with 5'-thiophosphate modification can activate RNase H cleavage of RNA.

Triplex Forming Oligonucleotides (TFO): Single stranded DNA may be designed to bind to genomic DNA in a sequence specific manner. TFOs are comprised of pyrimidine-rich oligonucleotides which bind DNA helices through Hoogsteen Base-pairing (Wu-Pong, *supra*)The resulting triple helix composed of the DNA sense, DNA antisense, and TFO disrupts RNA synthesis by RNA polymerase. The TFO mechanism may result in gene expression or cell death since binding may be irreversible (Mukhopadhyay & Roth, *supra).*

2-5A Antisense Chimera: The 2-5A system is an interferon mediated mechanism for RNA degradation found in higher vertebrates (Mitra *et al.,* 1996, *Proc Nat Acad Sci USA* 93, 6780-6785). Two types of enzymes, 2-5A synthetase and RNase L, are required for RNA cleavage. The 2-5A synthetases require double stranded RNA to form 2'-5' oligoadenylates (2-5A). 2-5A then acts as an allosteric effector for utilizing RNase L which has the ability to cleave single stranded RNA. The ability to form 2-5A structures with double stranded RNA makes this system particularly useful for inhibition of viral replication.

(2'-5')oligoadenylate structures may be covalently linked to antisense molecules to form chimeric oligonucleotides capable of RNA cleavage (Torrence, *supra*)*.* These molecules putatively bind and active a 2-5A dependent RNase, the oligonucleotide/enzyme complex then binds to a target RNA molecule which can then be cleaved by the RNase enzyme.

Enzymatic Nucleic acid: Seven basic varieties of naturally-occurring enzymatic RNAs are known presently. In addition, several *in vitro* selection (evolution) strategies (Orgel, 1979, *Proc. R. Soc. London,* B 205, 435) have been used to evolve new nucleic acid catalysts capable of catalyzing cleavage and ligation of phosphodiester linkages (Joyce, 1989, *Gene,* 82, 83-87; Beaudry *et al.,* 1992, *Science* 257, 635-641; Joyce, 1992, *Scientific American* 267, 90-97; Breaker *et al*., 1994, *TIBTECH* 12, 268; Bartel *et al.,*1993*, Science* 261:1411-1418; Szostak, 1993, *TIBS* 17, 89-93; Kumar *et al.,* 1995, *FASEB J*., 9, 1183; Breaker, 1996, *Curr. Op. Biotech.,* 7, 442; Santoro *et al.,* 1997, *Proc. Natl. Acad. Sci.,* 94, 4262; Tang *et al.,* 1997, *RNA* 3, 914; Nakamaye & Eckstein, 1994, *supra;* Long & Uhlenbeck, 1994, supra; Ishizaka et al., 1995, *supra;* Vaish *et al.,* 1997, *Biochemistry* 36, 6495; all of these are incorporated by reference herein). Each can catalyze a series of reactions including the hydrolysis of phosphodiester bonds in *trans* (and thus can cleave other RNA molecules) under physiological conditions. Table I summarizes some of the characteristics of some of these ribozymes. In general, enzymatic nucleic acids act by first binding to a target RNA. Such binding occurs through the target binding portion of an enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nature of a ribozyme is advantageous over other technologies, since the concentration of ribozyme necessary to affect a therapeutic treatment is lower. This advantage reflects the ability of the ribozyme to act enzymatically. Thus, a single ribozyme molecule is able to cleave many molecules of target RNA. In addition, the ribozyme is a highly specific inhibitor, with the specificity of inhibition depending not only on the base-pairing mechanism of binding to the target RNA, but also on the mechanism of target RNA cleavage. Single mismatches, or base-substitutions, near the site of cleavage can be chosen to completely eliminate catalytic activity of a ribozyme.

Nucleic acid molecules having an endonuclease enzymatic activity are able to repeatedly cleave other separate RNA molecules in a nucleotide base sequence-specific manner. Such enzymatic nucleic acid molecules can be targeted to virtually any RNA transcript, and efficient cleavage achieved *in vitro* (Zaug *et al*., 324, *Nature* 429 1986 ; Uhlenbeck, 1987 *Nature* 328, 596; Kim et al., 84 *Proc. Natl. Acad. Sci. USA* 8788, 1987; Dreyfus, 1988, *Einstein Quart. J*. *Bio. Med.,* 6, 92; Haseloff and Gerlach, 334 *Nature* 585, 1988; Cech, 260 *JAMA* 3030, 1988; and Jefferies et al., 17 *Nucleic Acids Research* 1371, 1989; Santoro *et al*., 1997 *supra*)*.*

Because of their sequence-specificity, *trans*-cleaving ribozymes show promise as therapeutic agents for human disease (Usman & McSwiggen, 1995 *Ann. Rep. Med. Chem*. **30**, 285-294; Christoffersen and Marr, 1995 *J. Med. Chem.* **38**, 2023-2037). Ribozymes can be designed to cleave specific RNA targets within the background of cellular RNA. Such a cleavage event renders the RNA non-functional and abrogates protein expression from that RNA. In this manner, synthesis of a protein associated with a disease state can be selectively inhibited.

### Optimizing Ribozyme Activity

Catalytic activity of the ribozymes described in the instant invention can be optimized as described by Draper et al., *supra*. The details will not be repeated here, but include altering the length of the ribozyme binding arms, or chemically synthesizing ribozymes with modifications (base, sugar and/or phosphate) that prevent their degradation by serum ribonucleases and/or enhance their enzymatic activity (see *e*.*g*., Eckstein *et al*., International Publication No. WO 92/07065; Perrault *et al.,* 1990 *Nature* 344, 565; Pieken et al., 1991 *Science* 253, 314; Usman and Cedergren, 1992 *Trends in Biochem. Sci.* 17, 334; Usman *et al*., International Publication No. WO 93/15187; and Rossi *et al.,* International Publication No. WO 91/03162; Sproat, US Patent No. 5,334,711; and Burgin *et al., supra;* all of these describe various chemical modifications that can be made to the base, phosphate and/or sugar moieties of enzymatic RNA molecules). Modifications which enhance their efficacy in cells, and removal of bases from stem loop structures to shorten RNA synthesis times and reduce chemical requirements are desired. (All these publications are hereby incorporated by reference herein).

There are several examples in the art describing sugar, base and phosphate modifications that can be introduced into enzymatic nucleic acid molecules without significantly effecting catalysis and with significant enhancement in their nuclease stability and efficacy. Ribozymes are modified to enhance stability and/or enhance catalytic activity by modification with nuclease resistant groups, for example, 2'-amino, 2'-C-allyl, 2'-flouro, 2'-*O*-methyl, 2'-H, nucleotide base modifications (for a review see Usman and Cedergren, 1992 *TIBS* 17, 34; Usman *et al*., 1994 *Nucleic Acids Symp. Ser*. 31, 163; Burgin *et al*., 1996 *Biochemistry* 35, 14090). Sugar modification of enzymatic nucleic acid molecules have been extensively described in the art (see Eckstein *et al*., *International Publication* PCT No. WO 92/07065; Perrault *et al*. *Nature* 1990*, 344,* 565-568; Pieken *et al*. *Science* 1991*, 253,* 314-317; Usman and Cedergren, *Trends in Biochem. Sci.* 1992, *17*, 334-339; Usman *et al*. *International Publication* PCT No. WO 93/15187; Sproat, *US Patent* No. 5,334,711 and Beigelman *et al*., 1995 *J. Biol*. *Chem.* 270, 25702; all of the references are hereby incorporated in their totality by reference herein). Such publications describe general methods and strategies to determine the location of incorporation of sugar, base and/or phosphate modifications and the like into ribozymes without inhibiting catalysis, and are incorporated by reference herein. In view of such teachings, similar modifications can be used as described herein to modify the nucleic acid catalysts of the instant invention.

Nucleic acid catalysts having chemical modifications which maintain or enhance enzymatic activity are provided. Such nucleic acid is also generally more resistant to nucleases than unmodified nucleic acid. Thus, in a cell and/or *in vivo* the activity may not be significantly lowered. As exemplified herein such ribozymes are useful in a cell and/or *in vivo* even if activity over all is reduced 10 fold (Burgin *et al*., 1996, *Biochemistry,* 35*,* 14090). Such ribozymes herein are said to "maintain" the enzymatic activity on all RNA ribozyme.

Therapeutic ribozymes delivered exogenously must optimally be stable within cells until translation of the target RNA has been inhibited long enough to reduce the levels of the undesirable protein. This period of time varies between hours to days depending upon the disease state. Clearly, ribozymes must be resistant to nucleases in order to function as effective intracellular therapeutic agents. Improvements in the chemical synthesis of RNA (Wincott *et al*., 1995 *Nucleic Acids Res.* 23, 2677; incorporated by reference herein) have expanded the ability to modify ribozymes by introducing nucleotide modifications to enhance their nuclease stability as described above.

By "enhanced enzymatic activity" is meant to include activity measured in cells and/or *in vivo* where the activity is a reflection of both catalytic activity and ribozyme stability. In this invention, the product of these properties is increased or not significantly (less that 10 fold) decreased *in vivo* compared to an all RNA ribozyme.

In yet another preferred embodiment, nucleic acid catalysts having chemical modifications which maintain or enhance enzymatic activity is provided. Such nucleic acid is also generally more resistant to nucleases than unmodified nucleic acid. Thus, in a cell and/or *in vivo* the activity may not be significantly lowered. As exemplified herein such ribozymes are useful in a cell and/or *in vivo* even if activity over all is reduced 10 fold (Burgin *et al*., 1996, *Biochemistry,* 35, 14090). Such ribozymes herein are said to "maintain" the enzymatic activity on all RNA ribozyme.

### Inhibition Of Estrogen Receptor Gene Expression

Breast Cancer is one of the leading causes of death in women (Jiang and Jordan, 1992, *J. Natl. Cancer Inst.* 84, 580-591). There has been an intense effort to understand the molecular mechanisms for hormonal regulation of cell proliferation in breast cancer over the last several decades. It has been shown that many breast and endometrial cancers are dependent on estrogen for their growth and progression (Borras *et al,* 1994, *J. Steroid Biochem. Molec. Biol.* 48, 325-336). Estrogen receptor plays a pivotal role in these cancers and thus controlling the expression of this gene is of paramount interest to researchers and clinicians. The estrogen receptor is a member of the steroid hormone receptor gene family that displays it's biological function as a ligand binding-dependent transcription factor. Tamoxifen is a nonsteroidal antiestrogen which treats all stages of breast cancer and may be used as a preventative compound in those predisposed to breast cancer (Jordan and Murphy, 1990, *Endocr. Rev.* 11; 578-610).

Most breast tumors are initially dependent upon estrogen for growth, and the estrogen receptor has been a key indicator for endocrine response, prognosis and survival from breast cancer. The MCF-7 human breast cancer cell line expresses high levels of estrogen receptor and is responsive to the effects of added estrogen (Borras *et al*., 1996, *J. Steroid Biochem. Molec. Biol.* 57, 203-213; Pink and Jordan, 1996, *Cancer Res.* 56, 2321-2330). They are an excellent model system to study the effects of regulation of estrogen receptor in breast cancer. Ribozymes and antisense oligonucleotides represent a direct means of affecting the levels of estrogen receptor message. In estrogen dependent cell lines, decreased amounts of estrogen receptor transcript should lower overall amounts of estrogen receptor protein and prevent proliferation of those cells. The effects of estrogen receptor on sexual differentiation of brain has been examined using antisense oligonucleotides (McCarthy *et al.,* 1993 *Endocrinology* 133, 433-439). This application documents the effects of ribozymes and antisense oligonucleotides to estrogen receptor RNA levels and proliferation in MCF-7 cells.

Estrogen receptor may be inhibited using nucleic acid molecules, including the nucleic acid molecules of the present invention. Other references describe the use of antisense molecules to down regulate estrogen receptor RNA (Defazio *et al.,* 1997, *Cell Growth Differ.* 8, 903-911; Santagati *et al.,* 1997, *Mol. Endocrinol.* 11, 938-949; Williard *et al.,* 1994, *Gene* 149, 21-24; Jiang & Jordan, *supra).*

The nucleic acid molecules may be chemically synthesized and delivered using methods described above, or may be expressed within cells from eukaryotic promoters (*e*.*g*., Izant and Weintraub, 1985 *Science* 229, 345; McGarry and Lindquist, 1986 *Proc. Natl. Acad. Sci.* USA 83, 399; Scanlon *et al*., 1991, *Proc. Natl. Acad*. *Sci. USA,* 88, 10591-5; Kashani-Sabet *et al*., 1992 *Antisense Res. Dev.,* 2, 3-15; Dropulic *et al*., 1992 *J. Virol,* 66, 1432-41; Weerasinghe *et al.,* 1991 *J. Virol,* **65**, 5531-4; Ojwang *et al*., 1992 *Proc. Natl. Acad. Sci. USA* 89, 10802-6; Chen *et al*., 1992 *Nucleic Acids Res.,* 20, 4581-9; Sarver *et al*., 1990 *Science* 247, 1222-1225; Thompson *et al*., 1995 *Nucleic Acids Res.* 23*,* 2259; Good *et al.,* 1997, *Gene* Therapy, 4, 45; all of the references are hereby incorporated in their totality by reference herein). Those skilled in the art realize that any nucleic acid can be expressed in eukaryotic cells from the appropriate DNA/RNA vector. The activity of such nucleic acids can be augmented by their release from the primary transcript by a ribozyme (Draper *et al*., PCT WO 93/23569, and Sullivan *et al.,* PCT WO 94/02595; Ohkawa *et al.,* 1992 *Nucleic Acids Symp. Ser.,* 27, 15-6; Taira *et al.,* 1991, *Nucleic Acids Res.,* 19, 5125-30; Ventura *et al.,* 1993 *Nucleic Acids Res.,* 21, 3249-55; Chowrira *et al.,* 1994 *J. Biol. Chem.* 269, 25856; all of the references are hereby incorporated in their totality by reference herein).

One type of nucleic acid molecules known as ribozymes, which can cleave target molecules, are expressed from transcription units (see for example Couture *et al.,* 1996, *TIG.,* 12, 510) inserted into DNA or RNA vectors. The recombinant vectors are preferably DNA plasmids or viral vectors. Ribozyme expressing viral vectors could be constructed based on, but not limited to, adeno-associated virus, retrovirus, adenovirus, or alphavirus. Preferably, the recombinant vectors capable of expressing the ribozymes are delivered as described above, and persist in target cells. Alternatively, viral vectors may be used that provide for transient expression of ribozymes. Such vectors might be repeatedly administered as necessary. Once expressed, the ribozymes cleave the target mRNA. The active ribozyme contains an enzymatic center or core equivalent to those in the examples, and binding arms able to bind target nucleic acid molecules such that cleavage at the target site occurs. Other sequences may be present which do not interfere with such cleavage. Delivery of ribozyme expressing vectors could be systemic, such as by intravenous or intramuscular administration, by administration to target cells ex-planted from the patient followed by reintroduction into the patient, or by any other means that would allow for introduction into the desired target cell (for a review see Couture *et al*., 1996, *TIG.,* 12, 510).

In one aspect the invention features, an expression vector comprising nucleic acid sequence encoding at least one of the nucleic acid molecules of the instant invention is disclosed. The nucleic acid sequence encoding the nucleic acid catalyst of the instant invention is operable linked in a manner which allows expression of that nucleic acid molecule.

In another aspect the invention features, the expression vector comprises: a transcription initiation region (*e.g*., eukaryotic pol I, II or III initiation region); b) a transcription termination region (*e.g*., eukaryotic pol I, II or III termination region); c) a gene encoding at least one of the nucleic acid catalyst of the instant invention; and wherein said gene is operably linked to said initiation region and said termination region, in a manner which allows expression and/or delivery of said nucleic acid molecule. The vector may optionally include an open reading frame (ORF) for a protein operably linked on the 5' side or the 3'-side of the gene encoding the nucleic acid catalyst of the invention; and/or an intron (intervening sequences).

Transcription of the ribozyme sequences are driven from a promoter for eukaryotic RNA polymerase I (pol I), RNA polymerase II (pol II), or RNA polymerase III (pol III). Transcripts from pol II or pol III promoters will be expressed at high levels in all cells; the levels of a given pol II promoter in a given cell type will depend on the nature of the gene regulatory sequences (enhancers, silencers, etc.) present nearby. Prokaryotic RNA polymerase promoters are also used, providing that the prokaryotic RNA polymerase enzyme is expressed in the appropriate cells (Elroy-Stein and Moss, 1990 *Proc. Natl. Acad Sci. U S A,* 87, 6743-7; Gao and Huang 1993 *Nucleic Acids Res.,* 21, 2867-72; Lieber et al., 1993 *Methods Enzymol.,* 217, 47-66; Zhou et al., 1990 *Mol. Cell. Biol.,* 10*,* 4529-37). Several investigators have demonstrated that ribozymes expressed from such promoters can function in mammalian cells (e.g. Kashani-Sabet et al., 1992 *Antisense Res. Dev.,* 2, 3-15; Ojwang et al., 1992 *Proc. Natl. Acad. Sci. U S A,* 89, 10802-6; Chen *et al.,* 1992 *Nucleic Acids Res.,* 20, 4581-9; Yu et al., 1993 *Proc. Natl. Acad. Sci. U S A,* 90, 6340-4; L'Huillier *et al.,* 1992 *EMBO J.* 11, 4411-8; Lisziewicz *et al*., 1993 *Proc. Natl. Acad Sci. U. S*. *A.,* 90, 8000-4; Thompson *et al.,* 1995 *Nucleic Acids Res.* 23, 2259; Sullenger & Cech, 1993, *Science,* 262, 1566). More specifically, transcription units such as the ones derived from genes encoding U6 small nuclear (snRNA), transfer RNA (tRNA) and adenovirus VA RNA are useful in generating high concentrations of desired RNA molecules such as ribozymes in cells (Thompson *et al., supra;* Couture and Stinchcomb, *1996, supra*; Noonberg *et al*., 1994, *Nucleic Acid Res.,* 22, 2830; Noonberg *et al*., US Patent No. 5,624,803; Good *et al.,* 1997, *Gene Ther.* 4, 45; Beigelman *et al.,* International PCT Publication No. *WO 96*/*18736;* all of these publications are incorporated by reference herein. The above ribozyme transcription units can be incorporated into a variety of vectors for introduction into mammalian cells, including but not restricted to, plasmid DNA vectors, viral DNA vectors (such as adenovirus or adeno-associated virus vectors), or viral RNA vectors (such as retroviral or alphavirus vectors) (for a review see Couture and Stinchcomb, *1996, supra).*

In yet another aspect the invention features an expression vector comprising nucleic acid sequence encoding at least one of the catalytic nucleic acid molecule of the invention, in a manner which allows expression of that nucleic acid molecule. The expression vector comprises in one embodiment; a) a transcription initiation region; b) a transcription termination region; c) a gene encoding at least one said nucleic acid molecule; and wherein said gene is operably linked to said initiation region and said termination region, in a manner which allows expression and/or delivery of said nucleic acid molecule. In another preferred embodiment the expression vector comprises: a) a transcription initiation region; b) a transcription termination region; c) an open reading frame; d) a gene encoding at least one said nucleic acid molecule, wherein said gene is operably linked to the 3'-end of said open reading frame; and wherein said gene is operably linked to said initiation region, said open reading frame and said termination region, in a manner which allows expression and/or delivery of said nucleic acid molecule. In yet another embodiment the expression vector comprises: a) a transcription initiation region; b) a transcription termination region; c) an intron; d) a gene encoding at least one said nucleic acid molecule; and wherein said gene is operably linked to said initiation region, said intron and said termination region, in a manner which allows expression and/or delivery of said nucleic acid molecule. In another embodiment, the expression vector comprises: a) a transcription initiation region; b) a transcription termination region; c) an intron; d) an open reading frame; e) a gene encoding at least one said nucleic acid molecule, wherein said gene is operably linked to the 3'-end of said open reading frame; and wherein said gene is operably linked to said initiation region, said intron, said open reading frame and said termination region, in a manner which allows expression and/or delivery of said nucleic acid molecule.

### Target Validation

One of the most challenging tasks in drug discovery is the choice of a therapeutic target. Historically, traditional biochemical and other studies have offered limited information in this regard. However, recent advances in genomics offer the potential to revolutionize both the speed and certainty of therapeutic target identification. Progress in characterizing the genes in the human genome has been very rapid, and it is now estimated that the entire complement of genes in the human genome may be sequenced before the end of this century. However, this mass of information is coming to the scientific world without a road map. Converting pure gene sequence information into a functional understanding of their role in human disease is proving to be a much more difficult problem. Even after a group of genes is associated with a particular disease, the process of validating which genes are appropriate for use as therapeutic targets is often slow and costly. Most companies with genomics activities now have access to myriad partial or full sequences, but do not possess adequate technologies to determine which of those sequences is an appropriate therapeutic target. As a result, only a few genes have been unequivocally identified as the causative agent for a specific disease.

The nucleic acid molecules of the present invention can inhibit gene expression in a highly specific manner by binding to and causing the cleavage of the mRNA corresponding to the gene of interest, and thereby prevent production of the gene product (Christoffersen, *Nature Biotech,* **1997,** 2, 483-484). Appropriate delivery vehicles can be combined with these nucleic acid molecules (including polymers, cationic lipids, liposomes and the like) and delivered to appropriate cell culture or *in vivo* animal disease models as described above. By monitoring inhibition of gene expression and correlation with phenotypic results, the relative importance of the particular gene sequence to disease pathology can be established. The process may be both fast and highly selective, and allow for the process to be used at any point in the development of the organism. The novel chemical composition of these nucleic acid molecules may allowed for added stability and therefore increased efficacy.

### Examples

The following are non-limiting examples demonstrating the utility of the nucleic acid molecules of the instant invention. Those in the art will recognize that certain experimental conditions such as temperatures, reaction times, media conditions, transfection reagents and RNA assays are not meant to be limiting and can be readily modified without significantly altering the protocols.

### Example 1: Identification of Potential Nucleic Acid Molecule Binding Sites

The sequences of target RNAs were screened for accessible sites using a computer folding algorithm. Regions of the mRNA that did not form secondary folding structures were identified. For ribozyme sites, regions of mRNA that did not form secondary structure and contained potential hammerhead and/or haripin cleavage sites were identified.

### Example 2: Selection of Ribozyme Cleavage Sites in Estrogen Receptor RNA

To test whether the sites predicted by the computer-based RNA folding algorithm corresponded to accessible sites in estrogen receptor. Ribozyme target sites were chosen by analyzing genomic sequences of Genbank Sequence HSERRI (Green *et al*., 1986, *Nature* 320, 134-139) and prioritizing the sites on the basis of folding. Ribozymes were designed that could bind each target (see Figure 3) and were individually analyzed by computer folding (Christoffersen *et al*., 1994 *J. Mol. Struc. Theochem,* 311, 273; Jaeger *et al*., 1989, *Proc. Natl. Acad. Sci. USA*, **86,** 7706) to assess whether the ribozyme sequences fold into the appropriate secondary structure. Those ribozymes with unfavorable intramolecular interactions between the binding arms and the catalytic core were eliminated from consideration. As noted below, varying binding arm lengths can be chosen to optimize activity. Generally, at least 5 bases on each arm are able to bind to, or otherwise interact with, the target RNA. Hammerhead (Seq. ID. Nos.1-1245) and hairpin ribozymes (2491-2603) are listed in tables IV and V respectively.

### Example 3: Inhibition of c-raf RNA Targets using Nucleic Acid Molecules

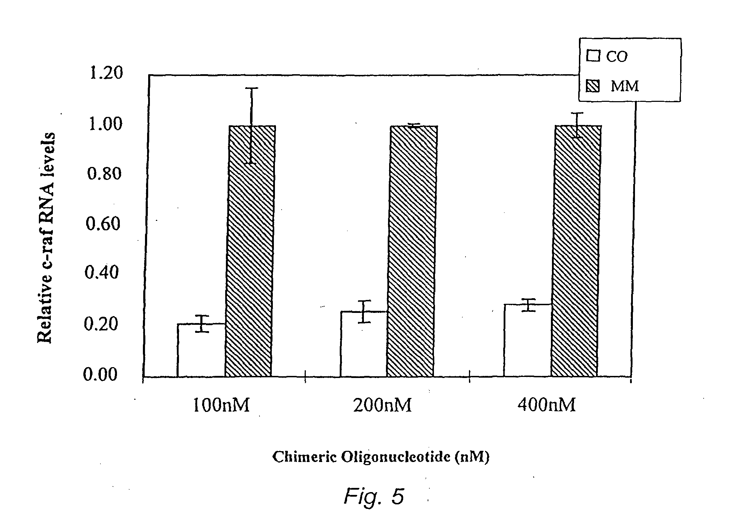

Prostate cancer cells (PC-3) were grown in a growth media consisting of Kaighn's F-12K media, 10% FBS, 1% glutamine, 20 mM HEPES, and 1% pen/strep to sub-confluent densities. A 4X concentration (10 µg/mL) of GSV (Glen Research) was prepared from a 2 mg/mL stock solution as well as a 10µM solution of antisense and its scrambled (mismatch) control. Complexes of antisense and GSV were formed in a 96 well plate by channel pipetting in antisense and GSV to form complex solutions which are twice the final concentrations. 50 µL of the complex solution and 50 µL of growth medium (without antibiotics) were added to PC-3 cells and incubated for 24 hours. The final concentrations of antisense used were 400, 200, and 100 nM, while the GSV concentration was held constant at 2.5 µg/mL. PC-3 cells were then harvested with 150 µL of RLT lysis buffer (Qiagen). RNA was purified using Qiagen's instructions and RNA was quantified using Taqman reagents and the 7700 Prism (Perkin Elmer) using the manufacturer's protocol. The c-raf RNA concentration was normalized to the c-raf RNA concentrations of the scrambled controls. The antisense sequence (Seq. I.D. No. 2717) and the data is shown in table IIIA and figure 5. The antisense molecules were capable of reducing c-raf RNA levels up to 80% compared to the mismatch control in PC-3 cells at several concentrations of antisense molecules.

### Example 4: Ribozyme in vitro Cleavage Assay

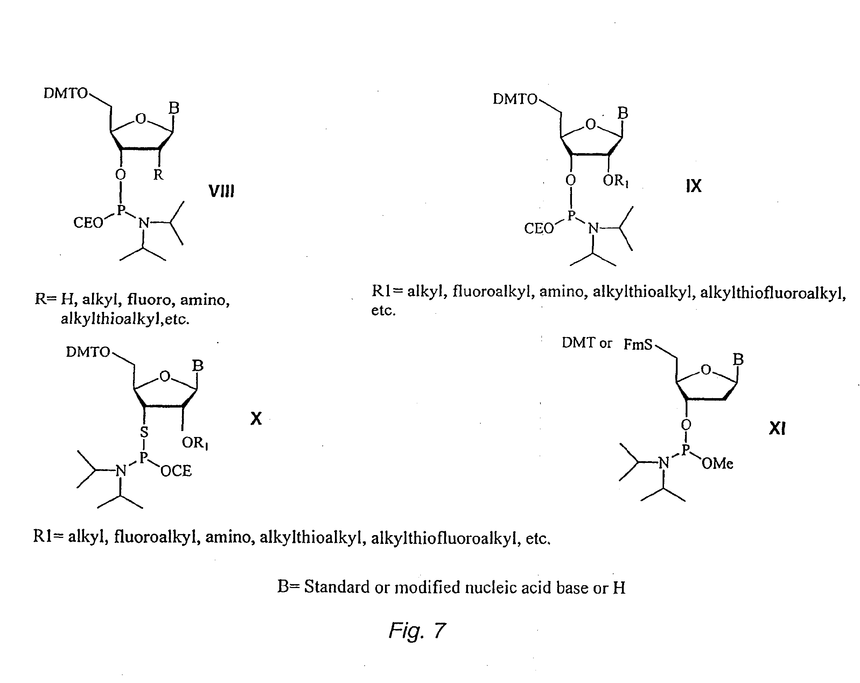

Ribozymes and complementary substrates were synthesized as described above. These ribozymes can be tested for cleavage activity *in vitro,* for example using the following procedure. The ribozyme sequences are shown in figure 7.

*Cleavage Reactions:* Full-length or partially full-length, internally-labeled target RNA for ribozyme cleavage assay was prepared by *in vitro* transcription in the presence of [a-³²P] CTP, passed over a G 50 Sephadex column by spin chromatography and used as substrate RNA without further purification. Alternately, substrates may by 5'-³²P-end labeled using T4 polynucleotide kinase enzyme. Assays were performed by pre-warming a 2X concentration of purified ribozyme in ribozyme cleavage buffer (50 mM Tris-HCl, pH 7.5 at 37°C, 10 mM MgCl₂) and the cleavage reaction was initiated by adding the 2X ribozyme mix to an equal volume of substrate RNA (maximum of 1-5 nM) that was also pre-warmed in cleavage buffer. The assays were carried out for 1 hour at 37°C using a final concentration of either 1 µM ribozyme, *i.e*., ribozyme excess. The reaction was quenched by the addition of an equal volume of 95% formamide, 20 mM EDTA, 0.05% bromophenol blue and 0.05% xylene cyanol after which the sample was heated to 95°C for 2 minutes, quick chilled and loaded onto a denaturing polyacrylamide gel. Substrate RNA and the specific RNA cleavage products generated by ribozyme cleavage were visualized on an autoradiograph of the gel. The percentage of cleavage was determined by Phosphor Imager® quantitation of bands representing the intact substrate and the cleavage products. The ribozymes were able to cleave between 1-80.8% of their complementary substrates after 2 hours (table VI).

### Example 5: Inhibition of Cell Proliferation Using Estrogen Receptor Targeted Ribozyme

MCF-7 cells were were grown in a T-75 flask to 80% confluency in growth media, which was prepared by mixing 500 mL Alpha-MEM, 10 mL 1M Hepes, 5mL sodium Pyruvate, 5 mL NEAA, 5 mL L-glutamine, 250 µL 2.0 mg/ml insulin, 500 µl Gentamycin, and 10% FBS following by sterile filtration.

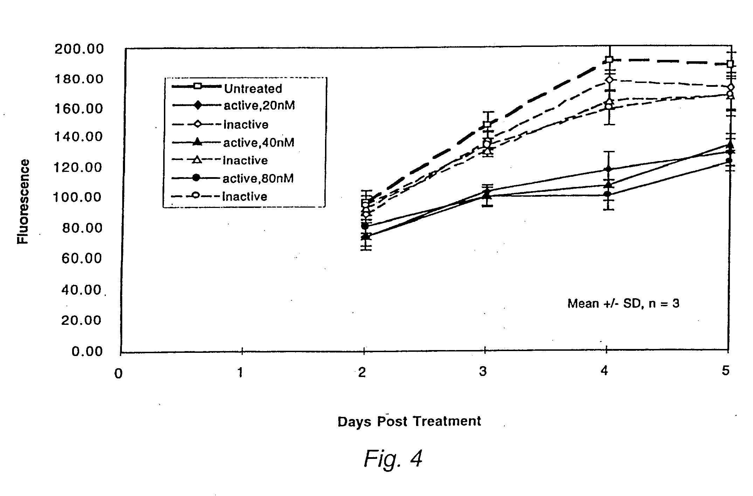

Ribozyme and cationic lipid was mixed as described in example 4 with final concentrations of 20, 40, and 80 nM ribozyme and 1 µg/mL GSV. The MCF-7 cells were treated with serum free alpha-MEM 24 hours prior to exposure to ribozyme/transfection reagent complexes. The complexes were added to the cells and allowed to continously transfect for 1,2 , 3, 4, and 5 days. At the end of each time point, the media was removed off the cells and proliferation was measured using a CyQuant kit (Molecular Probes). Fluorescence was measured at after 10 minutes of incubation at 485 nm (excitation) and 538 (emission). Inhibition of cellular proliferation by active ribozyme was compared to inactive scrambled ribozyme controls. The sequence for the active ribozyme is given as sequence ID. No. 2725 and the inactive scrambled control is given as Seq. ID. No. 2726. The chimeric enzymatic nucleic acid was able to inhibit MCF-7 cellular proliferation at all of the tested concentrations (figure 4).

### Example 6: Inhibition of Estrogen Receptor RNA Targets using antisense nucleic acid molecules

A 5X concentrated solution of oligonucleotide (1µM) and a 10X solution of GSV (25 µg/mL) (Glen Research) was made prior to complexing. The 5X oligonucleotide solution (8 µL), 10X GSV solution (40 µL), and Optimem media (32 µL) were mixed together and incubated at 37°C for 15 minutes. Media was aspirated off the cells followed by the addition of 80 µL of fresh growth media (Optimem media with 10% FBS, 1% non-essential amino acids, 1% sodium pyruvate, 20mM HEPES, 1 µg/mL insulin) and 20 µL of 5X complex solution. The complex was left on the cells for 20 hours and then harvested with 150 µL of RLT lysis buffer (Qiagen). RNA levels were then quantified using Taqman reagents and the 7700 Prism (Perkin Elmer) using the manufacturer's protocol. This cellular delivery assay was used for several RPI targets in varying cell lines and the data is shown in table IIIA. The levels of target RNA were either normalized to mismatch control RNA or to an internal housekeeping gene such as actin. Antisense nucleic acid molecules, which are given as Seq. ID. Nos. 2718-2723, were able to knock down estrogen receptor RNA by varying degrees. The levels of RNA inhibition ranged from 48-64% depending on the antisense sequence.

### Example 7: Inhibition of Estrogen Receptor RNA using Ribozymes

Ribozymes and GSV transfection reagents were mixed together using a protocol similar to the one found in example 6. Target RNA was purified using a Qiagen kit. RNA levels were then quantified using Taqman reagents and the 7700 Prism (Perkin Elmer) using the manufacturer's protocol. The ribozyme specific to estrogen receptor is given as sequence ID. No. 2724 and was able to inhibit the gene by 50%.

### Example 8: Inhibition of c-Raf mRNA and Cellular Proliferation Using Antisense Nucleic Acid Molecules

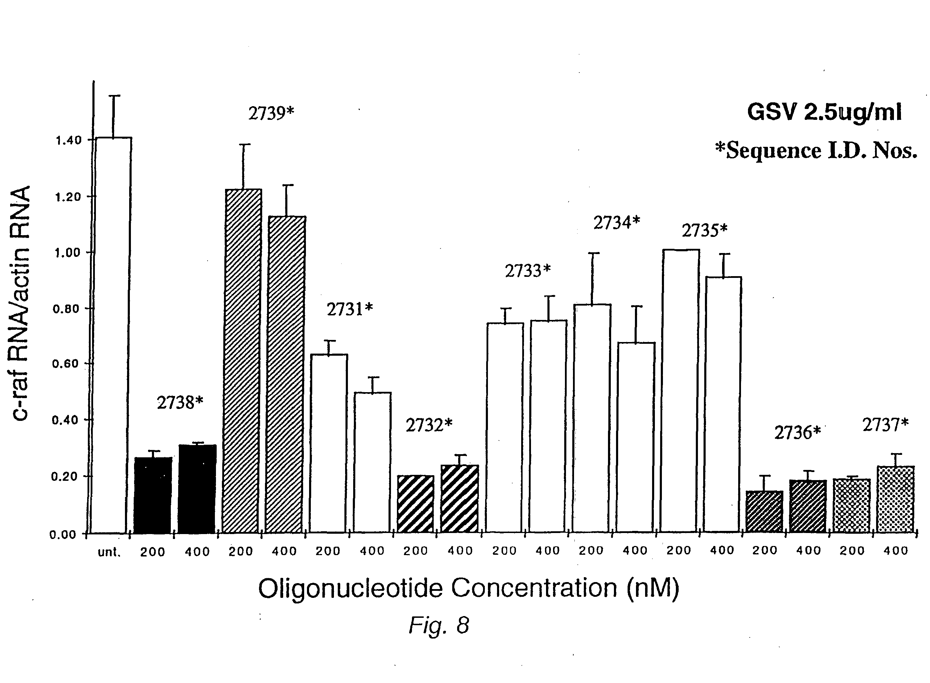

*24 Hour RNA endpoint assay:* The effect of targeting intron, exon, and intron-exon junction sites with antisense molecules was tested on c-raf RNA using *in vitro* cell culture assays. Seven chimeric antisense oligonucleotides with various chemical modifications were tested and the results were compared to an untreated control and an oligonucleotide modified at every nucleotide position with a phosphorothioate modification. Prostate cancer cells (PC-3) were grown in growth media consisting of Kaighn's F-12K media, 10% FBS, 1% glutamine, 20 mM HEPES, and 1% pen/strep in 96 well plates (15,000 cells per well). For the 24 hour delivery experiments, 2.5 mg/mL of GSV transfection reagent (Glen Research) was complexed to either 200 or 400 nM concentration of antisense molecules. The complex was left on the cells for 24 hours and the cells were then harvested with 150 µL of RLT lysis buffer (Qiagen). RNA levels were quantified using Taqman reagents and the 7700 Prism (Perkin Elmer) using the manufacturer's protocol. C-raf RNA levels were normalized to actin controls and the data is shown in Figure 8. All compounds demonstrated an ability to inhibit c-raf message. Of the compounds tested, sequence I.D. 2732, 2736 and 2737 seemed particularly effective.

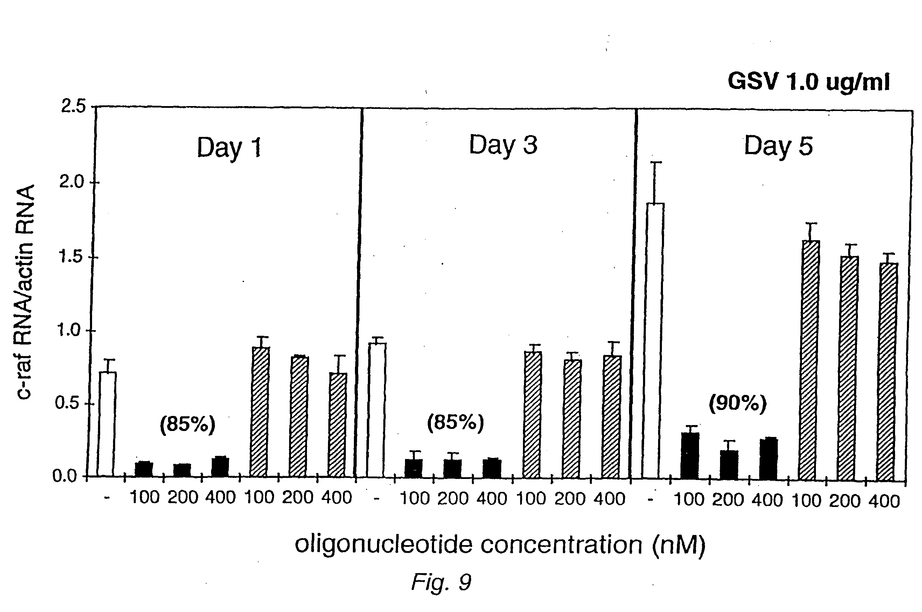

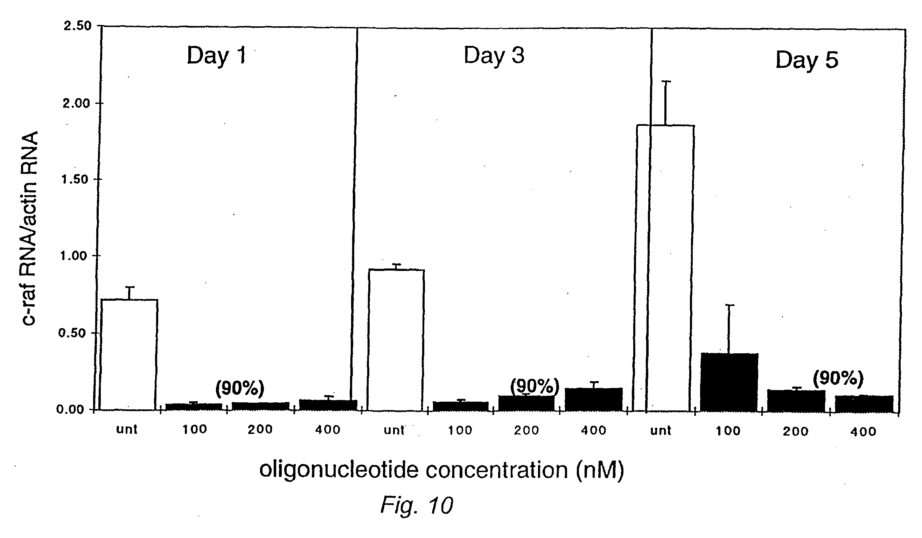

*Sustained delivery RNA endpoint assay:* Prostate cancer cells (PC-3) were grown in growth media consisting of Kaighn's F-12K media, 10% FBS, 1% glutamine, 20 mM HEPES, and 1% pen/strep in 96 well plates (15,000 cells per well). The cells were plated to 2500 cells per well in a 96 well plate and incubated at 37°C. Antisense nucleic acid molecules (Seq. I.D. Nos. 2738, 2737, 2744; Table IV) and GSV transfection reagent were complexed as in described under example 4 to a final concentration of antisense molecules of 100, 200 or 400 nM with 1.0 µg/mL of GSV transfection reagent. For seq. I.D. No. 2744, a mismatch control with similar base composition was also tested as a control. The complex was left on the cells and allowed to continuously transfect for 1, 3, or 5 days. At the end of each time period, the cells were harvested with 150 µL of RLT lysis buffer (Qiagen); and RNA levels quantified using Taqman reagents and the 7700 Prism (Perkin Elmer) using the manufacturer's protocol. The inhibition of c-raf expression by molecules represented by Seq. I.D. Nos. 2738, 2737, and 2744 (table IV) are shown in Figures 9, 10, and I 1 respectively. All three of these molecules demonstrated the ability to reduce c-raf RNA between 60-90% compared to the untreated control.

*Proliferation Assay:* Chimeric oligonucleotides and delivery reagents were mixed as described in example 6 where the final concentration of antisense oligonucleotide is 200 nM (Seq. I.D. No.2738 and 2741) and GSV is 1 µg/mL. A mismatch antisense control was also tested (Seq. I.D. No. 2739) in this experiment. The MCF-7 cells were treated with serum free alpha-MEM 24 hours prior to exposure to antisense/transfection reagent complexes. The complexes were added to the cells and allowed to continuously transfect for 1, 3, or 5 days. At the end of each time point, the media was removed off the cells and proliferation was measured using a CyQuant kit (Molecular Probes). Fluorescence was measured after 10 minutes of incubation at 485 nm (excitation) and 538 (emission). Inhibition of cellular proliferation by active oligonucleotide was compared to scrambled mismatch controls. The antisense molecules targeting c-raf mRNA were able to inhibit cellular proliferation by up to 55%.

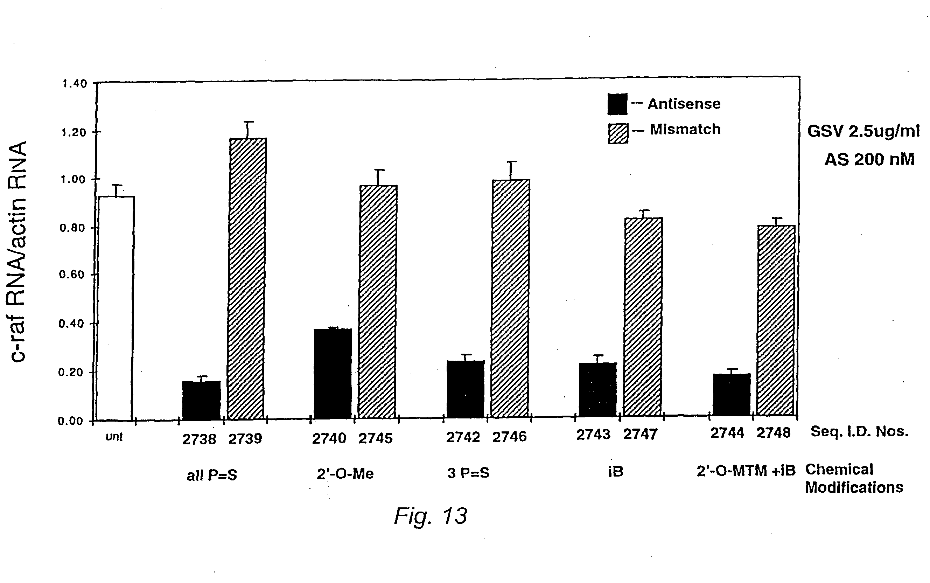

*Varying Chemical Modifications*: Referring to Figure 13 and Table IV, alterations of the chemical composition of antisense molecules were made while keeping the oligonucleotide sequence constant. All of the sequences were the same except for the mismatch controls and Seq. I.D. Nos. 2738 and 2739 (table IV) which were two nucleotides shorter than the others. Antisense molecules and GSV transfection reagent were mixed using the protocol described under example 6. The complexes were added to the cells and allowed to continuously transfect for 24 hours. The cells were then harvested with 150 µL of RLT lysis buffer (Qiagen). RNA levels were then quantified using Taqman reagents and the 7700 Prism (Perkin Elmer) using the manufacturer's protocol. All c-raf levels were normalized using actin RNA as a control. Referring to Figure 13, antisense molecules which utilized phosphorothioate modifications, inverted abasic caps, 2'-*O*-methyl or 2'-*O*-methylthiomethyl modifications inhibit c-raf mRNA.

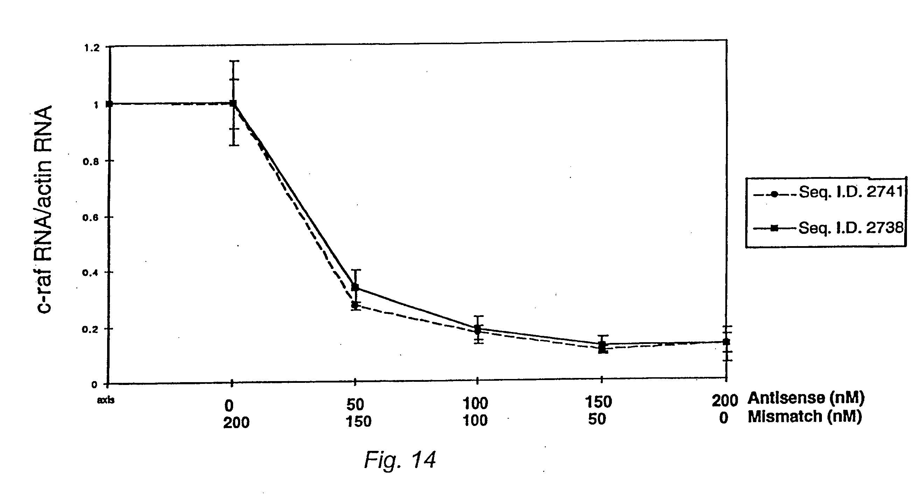

*Dose dependent Inhibition:* 0, 50, 100 150, and 200 nM of antisense molecules (seq. I.D. No. 2738 or 2737) were mixed with mismatch control antisense molecules to give a final antisense/mismatch antisense concentration of 200nM for each sample. The antisense nucleic acid and GSV were complexed as in example 6 with a final GSV transfection reagent concentration at 2.5 µg/mL. The complexes were added to the cells and allowed to continuously transfect for 24 hours. At the end of each time period, the cells were harvested with 150 µL of RLT lysis buffer (Qiagen). RNA levels were then quantified using Taqman reagents and the 7700 Prism (Perkin Elmer) using the manufacturer's protocol. All c-raf levels were normalized using actin RNA as a control. The results (Figure 14) show that c-raf is inhibited in a dose dependent manner and that the IC50 is approximately 35 nM for each of the antisense molecules.

### Example 9: Ribonuclease Protection Assay of MCF-7 Cells treated with Antisense Nucleic Acid Molecules Targeting BCL-2

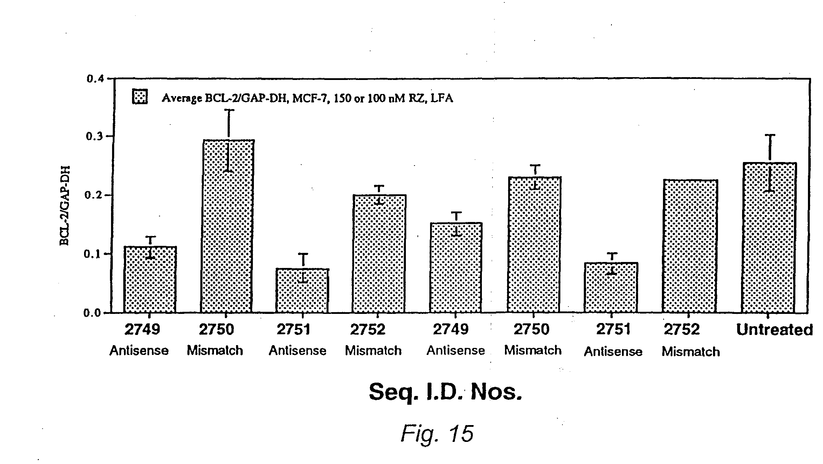

MCF-7 cells were plated in RPMI 1640 (10% FBS, 1% 1-glutamine, 20 mM HEPES) at 100,000 cells per well for 24 hours. On the following day, the cells were treated with 150 nM of antisense nucleic acid molecules (Seq. I.D. Nos. 2749-2753; Table VIII) complexed with 5.4 µM of LipofectAMINE (LFA) for 4 hours. The antisense/LFA complex was then aspirated off and fresh RPMI 1640 media was added to the cells. 24 hours later the cells were harvested using RLT lysis buffer (Qiagen). A ribonuclease protection assay (Ambion) was then performed using the manufacturers protocol to quantitate RNA levels and then harvested with 150 µL of RLT lysis buffer (Qiagen). RNA levels were then quantified using Taqman reagents and the 7700 Prism (Perkin Elmer) using the manufacturer's protocol. Bcl-2 RNA levels were normalized to GAPDH controls and is shown in Figure 15. The antisense oligonucleotides specifically inhibited Bcl-2 expression in MCF-7 cells.

### Example 10: Inhibition of k-ras in DLD-1 Cells

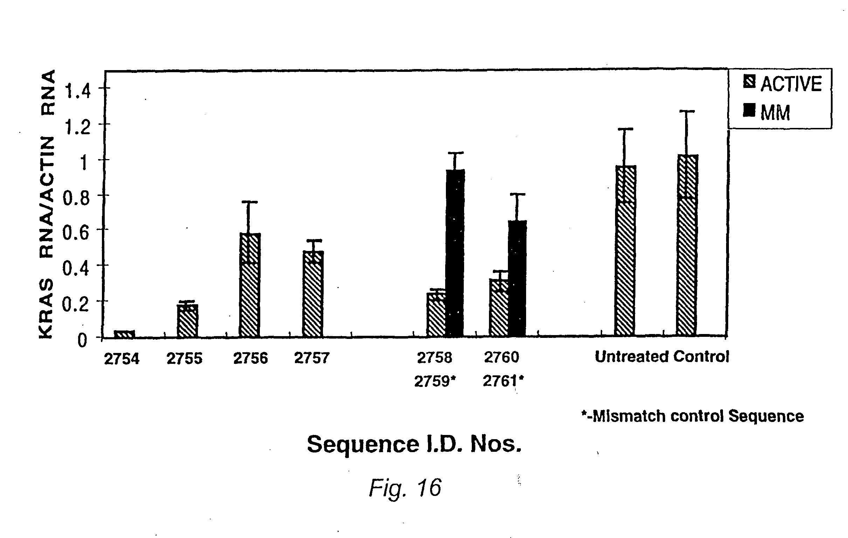

96-well plates with DLD-1 cells at 10,000 cells per well were plated in complete RPMI 1640 media ((10% FBS, 1% 1-glutamine, 20 mM HEPES, 1% pen/strep). Antisense molecules (Seq. I.D. Nos. 2754-2757; Table VIII) were complexed with GSV transfection reagent (Glen Research) using the method described in example 6. The final concentrations delivered to the cells were 200 nM antisense oligonucleotide and 1.25 µg/mL of GSV. The complex was added to the cells for 26 hours and at the end of the time period, the cells were harvested with 150 µL of RLT lysis buffer (Qiagen). RNA levels were then quantified using Taqman reagents and the 7700 Prism (Perkin Elmer) using the manufacturer's protocol. All k-ras levels were normalized using actin RNA as a control. The data (Figure 16) demonstrates that the antisense molecules can inhibit approximately 50-90% k-ras expression compared to the untreated or mismatch controls.

### Example 11: Inhibition of Estrogen Receptor mRNA Using Antisense Molecules of varying Chemical Composition

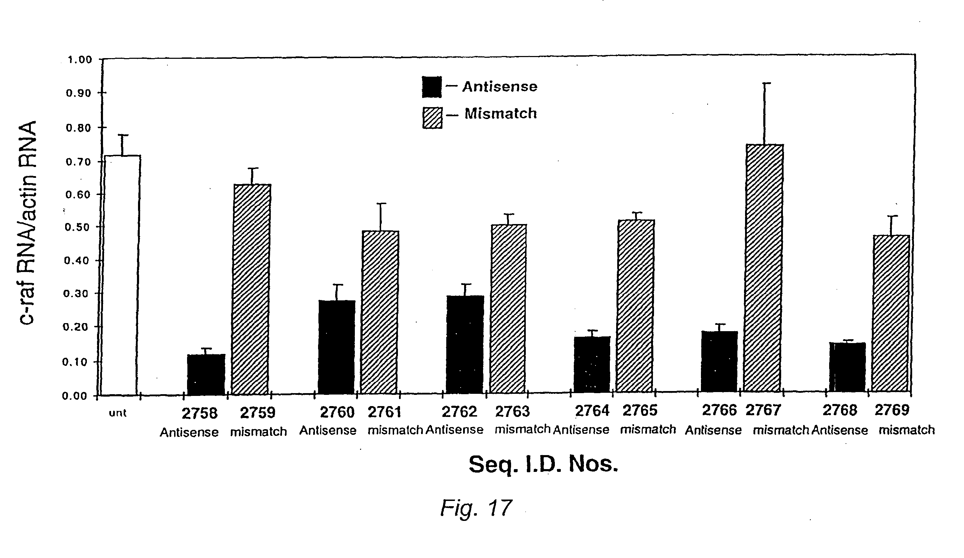

Alteration of the chemical composition of antisense molecules were made while keeping the oligonucleotide sequence constant. All of the sequences (Table VII: Seq. I.D. Nos. 2758, 2760, 2762, 2764, 2766, 2768) were the same except for the mismatch controls (Table VII: Seq. I.D. Nos. 2759, 2761, 2763, 2765, 2767, 2769). Antisense molecules and GSV transfection reagent was mixed using the protocol described in example 6. The complexes were added to MCF-7 cells and allowed to continuously transfect for 24 hours. The cells were harvested with 150 µL of RLT lysis buffer (Qiagen). RNA levels were then quantified using Taqman reagents and the 7700 Prism (Perkin Elmer) using the manufacturer's protocol. All estrogen receptor mRNA levels were normalized using actin RNA as a control. The antisense molecules which included phosphorothioate modifications, inverted abasic caps, 2'-*O*-methyl or 2'-*O*-methylthiomethyl modifications appeared to decrease estrogen receptor RNA (Figure 17).

### Example 12: Synthesis of 3'-deoxy-3'-thio Guanosine and it's 3'-Thiophosphoramidite

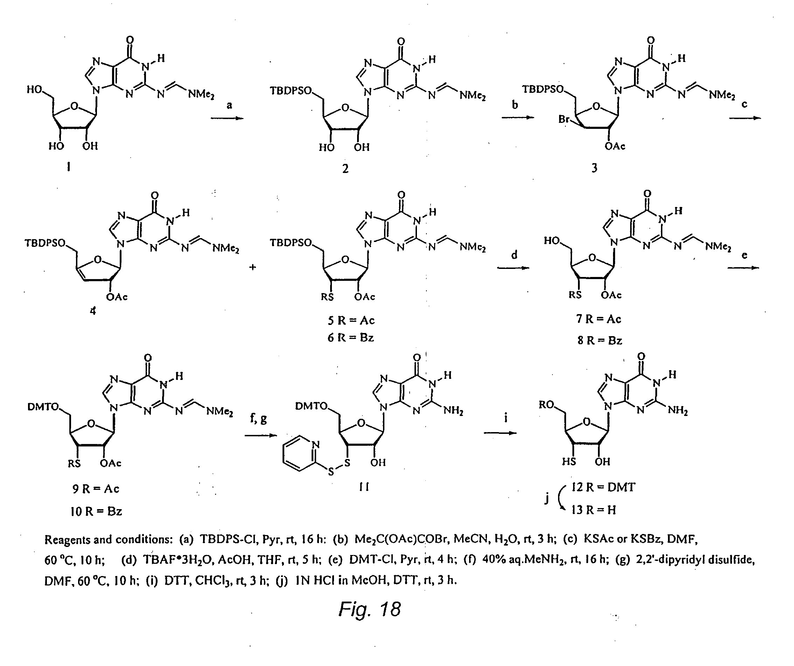

Referring to **Figures 18**, Applicant has developed an efficient method for the synthesis of 3'-deoxy-3'-thio guanosine (13) and its 3'-thiophosphoramidite **23** from guanosine. Reaction of suitably protected guanosine with a-acetoxyisobutyryl bromide (a-AIBBr) afforded stereoselectively 3'-deoxy-3'-bromo-2'-*O*-acetyl-b-D-xylofuranosyl derivative 3 which was converted into the 7:3 mixture of *S*-acyl ribofuranosyl derivatives **5** (or **6**) and 3',4'-unsaturated derivative **4**. *S*-acylated derivatives **5** and **6** were then converted in three steps into 3'-deoxy-3'-*S*-pyridylsulfanyl-5'-*O*-(4,4'-dimethoxytrityl)guanosine (**11**) which served as a common intermediate for the preparation of free nucleoside **13** and 3'-thiophosphoramidite **23**.

Oligonucleotides containing 3'-*S*-phosphorothiolate linkage have attracted increasing interest as probes for studying the interaction of nucleic acids and their processing enzymes. In particular these analogs have been used in revealing the involvement of metal ions in phosphoester transfer reactions catalyzed by RNA (Piccirilli *et al., J. Am. Chem. Soc.* **1996**, *118*, 10341) and ribonucleoprotein enzymes (Sontheimer *et al*., *Nature* **1997,** *308,* 801). The synthesis of 3'-S-phosphorothiolate linked deoxyribodinucleotides have been reported using solution chemistry and solid phase chemistry (Cosstick *et al*., *Nucleic Acids. Res.* **1990,** *18,* 829; Li *et al*., *Tetrahedron* **1992,** 48, 2729; Li *et al*., *J. Chem. Soc. Perkin Trans. 1* **1994,** 2123; Vyle *et al*., *Biochemistry* **1992, 31,** 3012).

The synthesis of ribonucleotide 3'-*S*-phosporothiolate analogs has been limited to the preparation of UspU (Liu *et al*., *Tetrahedron Lett.* **1996,** *37,* 925) and IspU (Weinstein *et al*., *J. Am. Chem*. *Soc.* **1996,** *118*, 10341) dimers using solution chemistry. Recently, Sun *et al*. *(RNA* **1997,** *3,* 1352) described direct incorporation of 3'-*S-*phosphorothioamidites into RNA using standard phosphoramidite solid phase synthesis.

One general approach to the synthesis of 3'-thio ribonucleosides involves preparation of 3-thio ribose derivative, followed by the attachment of the desired nucleoside base (Ryan *et al*., *J Org. Chem.* **1968,** *33*, 1783; Cao*, et al., Bioorg. Med. Chem. Lett.* **1994,** *4*, 807). While glycosylation reactions using pyrimidines proceed in high yields, purine bases generally give more complex mixtures because both N-7 and N-9 of the purine base are reactive towards glycosylation. Sun *et al., supra,* reported the first synthesis of 3'-thio guanosine derivatives using the above described approach. Coupling of per-acylated 3'-thioribose with persilylated *N*²-acetylguanine proceeded in ca 40% yield and subsequent synthetic steps proceeded in a low overall yield.

Synthesis of 3'-thio adenosine (Mengel*, et al., Tetrahedron Lett.* **1977,**1177), 3'-thio uridine (Liu *et al., 1996 supra)* and 3'-thio inosine (Higson *et al., Tetrahedron* **1996,** *52*, 1027) all starting from preformed nucleosides are also reported.

Applicant describes a novel and improved process for the synthesis of 3'-deoxy-3'-thio guanosine (**13**) and its phosphoramidite **23** from guanosine as a starting material. It was recently reported (He *et al., Tetrahedron Lett.* **1995,** *39,* 6991) that the reaction of *N*²-(dimethylaminomethylene)-guanosine **1** with a-AIBBr (the Mattocks-Moffatt reagent; *Russell et al., J. Am. Chem. Soc.* **1973**,*95*, 4025) proceeded stereoselectively yielding exclusively 3'-bromo-3'-deoxy-b-D-xylofuranosyl derivative. In general, reactions of base-unprotected purine nucleosides with this reagent result in the mixtures of trans bromo acetates of xylo- and arabino- configuration. Applicant used this reaction on the suitably 5'-protected *N*²-(dimethylaminomethylene)guanosine derivative 2 (Scheme 1; **Figure 18**). 5'- protection in **2** helps to reduce the complexity of reaction products by eliminating possible formation of the mixture of 5'-OH, 5'-(2,5,5-trimethyl-1,3-dioxolan-4-on-yl) and/or 5'-*O*-acylated derivatives in reaction with a-AIBBr. This way, identification of the reaction products becomes straightforward. Applicant has chosen the *t*-butyldiphenylsilyl (TBDPS) protection because of its relatively high stability towards acidic conditions required during the reaction with a-AIBBr in moist acetonitrile. This group is also expected to undergo selective cleavage in the presence of *S*-acyl groups. Reaction of **1** with TBDPS-Cl proceeded quantitatively to afford the 5'-*O*-silyl derivative **2** which reacted smoothly with a-AIBBr yielding the desired 3'-bromo-3'-deoxy-b-D-xylofuranosyl derivative **3** in a high yield. Reaction of 3 with potassium thioacetate or potassium thiobenzoate yielded the 3'-*S*-Ac or 3'-*S*-Bz derivatives **5** and **6**, respectively, along with 3',4'-unsaturated derivative **4**. The latter is formed by competing elimination reaction. The ratio was 7:3 in favor of the substitution products **5** and **6** which could not be separated from the elimination product **4** at this stage. The mixture of **4** and **5** (or **4** and **6**) was treated with tetrabutylammonium fluoride (TBAF) buffered with an excess of acetic acid; following chromatographic separation the desired 5' de-protected derivative **7** was obtained in a good yield. The unsaturated derivative **4** was unstable under the acidic reaction conditions and could not be isolated in a pure state by silica gel column chromatography. When triethylamine trihydrofluoride (TEA•3HF) reagent was used for deprotection, desilylation did not proceed to completion.

It is desirable to keep guanosine derivatives protected with lipophylic groups during synthetic transformations because of the solubility problems encountered with unprotected derivatives. For that reason **7** and **8** were re-protected in high yields with 4,4'-dimethoxytrityl (DMT) group to give the fully protected derivatives **9** and **10**, respectively. DMT group provided a hydrophobic tag which simplified work-up and purification of subsequent synthetic intermediates. Next, **9** and **10** were converted into *S-*(pyridyl-2-disulfanyl) derivative **11** using aqueous methylamine followed by the disulfide exchange reaction with 2,2'-dipyridyl disulfide. It is reported that the removal of 2'-*O-*acyl protection in ribofuranosyl derivatives similar to **9** and **10** proceeds with difficulty. Applicant found that 40% aqueous methylamine easily removed all acyl protecting groups from **9** and **10** and was the base of choice because, contrary to aqueous ammonia, it completely solubilised the fully protected substrates. *In situ* protection of SH as *S*-pyridyl-2-disulfanyl derivative was achieved using 2,2'-dipyridyl disulfide in DMF to afford **11** in 85% yield for these two steps.

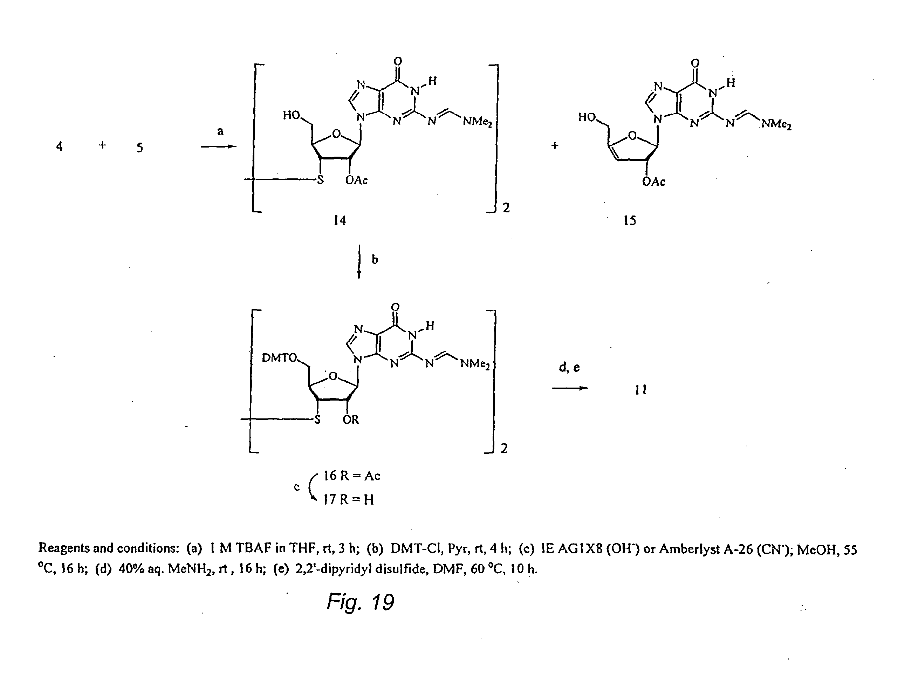

In order to synthesize the free nucleoside, **13**, **11** was treated with dithiothreitol (DTT) in chloroform. When triethylamine was added to the reaction mixture the reaction was faster than in it's absence but at the same time **12** was converted into its *S*-TEA salt. Final deprotection of the DMT group of **12** was achieved with 1N HCl in methanol in the presence of DTT which quenched the released DMT-cation. In the absence of DTT, quantitative *S*-alkylation took place. Applicant is the first to report on the synthesis of the guanosine 3'-thio analog **13.** When unbuffered TBAF in THF was used to desilylate the mixture of **4** and **5**, *S*-Ac protecting group was also removed leading to formation of the disulfide **14** (Scheme 2; **Figure 19**). Under these conditions the 3',4'-unsaturated derivative **15** remained intact and was separated from **14** by silica gel column chromatography. Products were invariably contaminated with TBAF. Attempted rechromatography of **15** led to its decomposition, though. Disulfide **14** was 5'-DMT protected to afford **16**.

The selective removal of the 3'-acetyl group of **16** (Scheme 2; **Figure 19**), followed by the introduction of 2'-*O*-*t*-butyldimethylsilyl (TBDMS) protection, then reduction of 3'-disulfide and 3'-phosphitylation would be the shortest way to prepare the desired 3'-thiophosphoramidite building block. Reactions of **16** with mild deacylating agents like basic ion exchangers in OH- or CN- form selectively removed 2'-*O*-acetyl protection, but at the same time nucleoside was strongly absorbed on the resin, resulting in low recoveries. Applicant used basic treatment followed by *S*-protection with *S*-pyridyl group, as used for the preparation of the *S*-pyridyl-2-disulfanyl derivative **11** from *S-*acylated **9** and **10**. In this manner **11** was obtained from the disulfide **16** in 67% yield.

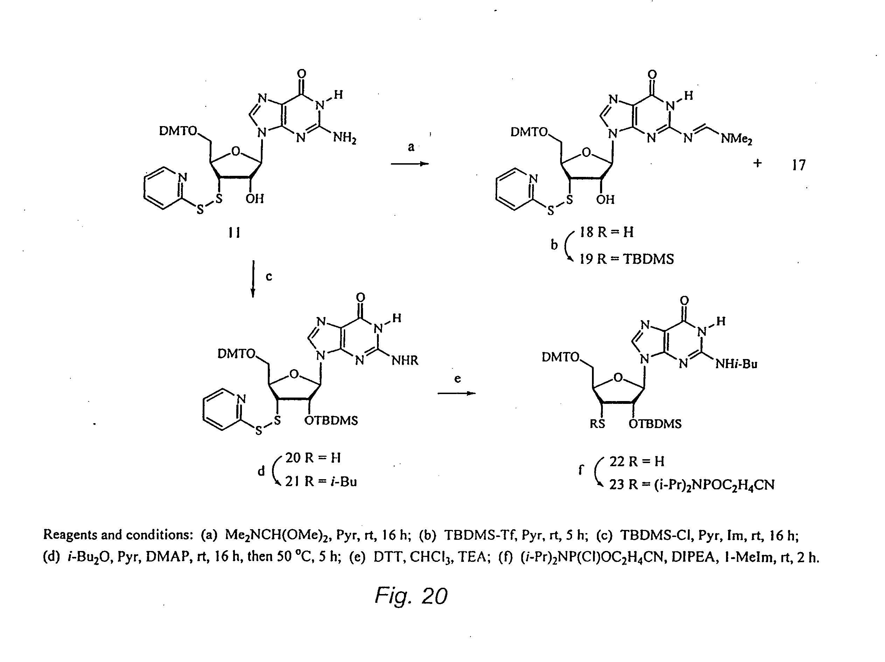

The phosphoramidite synthesis is shown in Scheme 3 (**Figure 20**). Reaction of **11** with *N,N*-dimethylformamide dimethyl acetal yielded the desired *N*-protected derivative **18** in 23% yield. Unfortunately, this reagent also effected the cleavage of the *S*-pyridyl protection leading to formation of disulfide **17** in 33% yield. **18** was smoothly 2' protected with TBDMS group using *t*-butyldimethylsilyl trifluoromethanesulfonate (TBDMS-Tf). Alternatively, **11** was silylated with TBDMS-Cl to afford **20** and then *N-*protected using isobutyric anhydride (*i*-Bu₂O) in the presence of 4-dimethylaminopyridine (DMAP) yielding the fully protected **21**. In the absence of DMAP only starting material was recovered. On the other hand, reaction of **20** with isobutyryl chloride led to *N*-bis-acylation. Reduction of **21** with DTT afforded 3'-SH derivative **22**, which appeared as a mixture of two rotamers in ¹H NMR. Resonances of the major rotamer were in accordance with the ones reported by Sun *et al.* Phosphitylation of **22** under standard conditions afforded 3'-thiophosphoramidite **23**. Applicant has described an efficient synthesis of 3'-deoxy-3'-thio guanosine. Keeping all synthetic intermediates protected with lipophylic groups enabled their chromatographic purification and, consequently, a good recovery of the products.

### Experimental Section

**General**. All reactions were carried out under a positive pressure of argon in anhydrous solvents. Commercially available reagents and anhydrous solvents were used without further purification. ¹H (400.075 MHz) and ³¹P (161.947 MHz) NMR spectra were recorded in CDCl₃, unless stated otherwise, and chemical shifts in ppm refer to TMS and H₃PO₄, respectively. Analytical thin-layer chromatography (TLC) was performed with Merck Art.5554 Kieselgel 60 F₂₅₄ plates and flash column chromatography using Merck 0.040-0.063 mm silica gel 60. Mass spectra were obtained by fast atom bombardment method.

**5'-*O*-*t*-Butyldiphenylsilyl-*N***^{**2**}**-(dimethylaminomethylene)guanosine (2).** To a stirred solution of *N*²-(dimethylaminomethylene)guanosine (**1**) (5.5 g, 16.3 mmol) in pyridine (100 mL) *t*-butyldiphenylsilyl chloride (6.2 ml, 23.8 mmol) was added under argon. The reaction mixture was stirred at rt for 16 h, then quenched with methanol ( 20 ml) and evaporated to a syrup *in vacuo.* The residue was crystallized from ethanol-ether ( 9 g, 96%), mp, ¹H NMR (DMSO-d₆ + D₂O) d 8.46 (s, 1H, CH=N), 7.89 (s, 1H, H-8), 7.58-7.31 (m, 10H, Ph), 5.81 (d, J_{1',2'}=4.8, 1H, H-1'), 4.46 (app t, J_{2',1'}=4.8, 1H, H-2'), 4.23 (app t, J_{3',2'}=5.0, 1H, H-3'), 3.97 (m, 1H, H-4'), 3.84 (dd, J_{5'},₄,=2.8, J_{5',5"}=12.0, 1H, H-5'), 3.74 (dd, J_{5",4'}=4.4, J_{5",5'}=12.0, 1H, H-5"), 3.05 (s, 3H, Me), 2.97 (s, 3H, Me), 0.94 (s, 9H, *t*-Bu), HRMS (FAB⁺) calcd for C₂₉H₃₆N₆O₅Si (MH⁺): calc 577.2595, found 577.26095.

**1-(2-*O*-Acetyl-5-*O*-*t*-Butyldiphenylsilyl-3-deoxy-3-bromo-b-D-xylofuranosyl)-*N***^{**2**}**-(dimethylaminomethylene)guanosine (3)**. To a cooled (0 °C) solution of **2** (5.8 g, 10 mmol) and water (0.12 ml) in acetonitrile (130 ml) a-acetoxyisobutyryl bromide (5.56 ml, 38 mmol) was added and the mixture was stirred at rt for 3 h. The solution was poured into saturated aq. NaHCO₃ (100 mL) and extracted with CH₂Cl₂ (3 x 200 mL). The combined organic layers were dried (Na₂SO₄) and concentrated to give chromatographically pure white foam (6 g, 87%), ¹H NMR d 8.97 (br s, 1H, NH), 8.62 (s, 1H, CH=N), 7.82 (s, 1H, H-8), 7.73-7.31 (m, 10H, Ph), 6.09 (s, 1H, H-2'), 5.92 (d, J_{1',2'}=1.6, 1H, H-1'), 4.42 (m, 1H, H-4'), 4.36 (m, 1H, H-3'), 4.06 (dd, J_{5',4'}=5.6, J_{5',5"}=10.4, 1H, H-5'), 3.97 (dd, J_{5",4},=6.4, J_{5",5},=10.4, 1H, H-5"), 3.17 (s, 3H, *N*- Me), 3.07 (s, 3H, *N*-Me), 2.19 (s, 3H, *O*-Ac), 1.07 (s, 9H, *t*-Bu), HRMS (FAB⁺) calcd for C₃₁H₃₇BrN₆O₅Si (MH⁺): calc 681.1856, found 681.1850.

**1-(2-*O*-Acetyl-5-*O*-*t*-butyldiphenylsilyl-3-deoxy- b -D-*glycero*-pent-3-enofuranosyl-*N***^{**2**}**-(dimethylaminomethylene)guanosine (4) and 2'-*O*-acetyl-5'-*O-t-*butyldiphenyl-silyl-3'-deoxy-3'-*S*-thioacetyl-*N***^{**2**}**(dimethylaminomethylene)- guanosine (5)**. **3** (5.4 g, 7.9 mmol) was dissolved in dry DMF (50 ml) and potassium thioacetate (2.7 g, 23.6 mmol) was added to the solution. The reaction mixture was stirred at 60 °C for 16 h and then evaporated to a syrup under reduced pressure. The residue was partitioned between aq. NaHCO₃-brine 1:1 solution and dichloromethane, the organic layer was dried (Na₂SO₄), evaporated to dryness and chromatographed on the column of silicagel using 2-10% gradient of methanol in dichloromethane **4** and **5** co-eluted yielding, after evaporation, a yellowish foam (4.8 g). ¹H NMR indicated a 3:7 ratio of **4** to **5**.

When potassium thiobenzoate was used instead of potassium thioacetate an inseparable mixture of **4** and **2'-*O*-acetyl-5'-*O*-*t*-butyldiphenylsilyl-3'-deoxy-3'-*S-*thiobenzoyl-*N***^{**2**}**(dimethylaminomethylene)guanosine (6)** was obtained in a similar yield and ratio to the unsaturated derivative 4 as above.

**2'-*O*-Acetyl-3'-deoxy-3'-*S*-thioacetyl-*N***^{**2**}**(dimethylaminomethylene) guanosine** (**7**). The above mixture of **4** an **5** (0.9 g) was dissolved in THF (15 ml) and acetic acid (0.37 ml, 6.5 mmol) was added followed by TBAF•3H₂O (0.82 g, 2.6 mmol). The reaction mixture was stirred at rt for 5 h, then diluted with dichloromethane, washed with water and 10% aq. NaHCO₃. Aqueous layers were back-washed with dichloromethane, organic layers were combined, dried (Na₂SO₄) and evaporated to dryness. Silica gel column chromatography using 2-10% gradient of methanol in dichloromethane afforded **7** as a yellowish foam (300 mg, ca 74%), ¹H NMR d 8.87 (br s, 1H, NH), 8:78 (s, 1H, CH=N), 7.73 (s, 1H, H-8), 5.80 (d, J_{1',2'}=2.0, 1H, H-1'), 5.76 (dd, J_{2',1'}=2.0, J_{2',3'}=6.4, 1H, H-2'), 4.94 (dd, J_{3',2'}=6.4, J_{3'4'}=9.6, 1H, H-3'), 4.22 (d, J_{4',3'}=9.6, 1H, H-4'), 4.04 (br s, 1H, 5'-OH), 3.99 (d, J_{5',5"}=12.0, 1H, H-5'), 3.71 (d, J_{5",5'}=12.0, 1H, H-5"), 3.19 (s, 3H, *N*-Me), 3.04 (s, 3H, *N*-Me), 2.34 (s, 3H, S-Ac), 2.13 (s, 3H, *O*-Ac), HRMS (FAB⁺) calcd for C₁₇H₂₂N₆O₆S (MH⁺): 439.1355, found 439.1405.

**2'-*O*-Acetyl-3'-deoxy-3'-*S*-thiobenzoyl-*N***^{**2**}**(dimethylaminomethylene) guanosine (8)**. Using the same procedure as above, **8** was synthesized from the mixture of 4 and 6 in ca 70% yield, ¹H NMR d 8.90 (br s, 1H, NH), 8.57 (brs, 1H, NH), 7.69 (s, 1H, H-8), 5.88 (m, 2H, H-1',H-2'), 5.30 (m, 1H, H-3'), 4.34 (d, J_{4',3'}=9.2, 1H, H-4'), 4.04 (d, J_{5',5"}=12.8, 1H, H5'), 3.80 (dd, J_{5",OH}=9.6, J_{5",5"}=12.8, 1H, H-5"), 3.69 (br s, 1H, 5'-OH), 3.25 (s, 3H, *N*-Me), 3.10 (s, 3H, *N*-Me), 2.16 (s, 3H, *O*-Ac), HRMS (FAB⁺) calcd for C₁₇H₂₂N₆O₆S (MH⁺): 501.1556, found 501.1561.

**2'-*O*-Acetyl-3'-deoxy-3'-*S*-thioacetyl-5'-*O*-*(4,4'-dimethoxytrityl)-N***^{**2**}**(dimethylamino-methylene)guanosine (9)**. **7** (720 mg, 1.64 mmol) was dissolved in dry pyridine (15 ml) and DMT-Cl (1.1 g, 3.3 mmol) was added. The reaction mixture was stirred at rt for 4 h, qenched with methanol and evaporated to a syrup which was partitioned between 5% aq. NaHCO₃ and CH₂Cl₂. Organic layer was washed with brine, dried (Na₂SO₄) and evaporated to dryness *in vacuo.* The residue was purified by silica gel column chromatography using 1-5% gradient of methanol in dichloromethane to yield the product as a colorless foam (0.85 g, 70%), ¹H NMR d 8.69 (s, 1H, CH=N), 8.58 (br s, 1H, NH), 7.69 (s, 1H, H-8), 7.38-6.74 (m, 13H, H-8, aromatic), 6.06 (dd, J_{2',3},=6.4, J_{2',1'}=1.2, 1H, H-2'), 5.82 (d, J_{1',2'}=1.2, 1H, H-1'), 4.73 (dd, J_{3',4'}=10.6, J_{3',2'}=6.4, 1H, H-3'), 4.21 (dq, J_{4',3'}=10.6, J_{4',5'}=3.0, J_{4',5"}=4.4, 1H, H-4'), 3.78 (s, 6H, 2xOMe), 3.36 (m, 2H, H-5', H-5"), 3.07 (s, 3H, *N*-Me), 3.05 (s, 3H, *N*-Me), 2.26 (s, 3H, *S*-Ac), 2.15 (s, 3H, O-Ac), HRMS (FAB⁺) calcd for C₃₈H₄₀N₆O₈S (MH⁺): 741.2707, found 741.2692.

**2'-*O*-Acetyl-3'-deoxy-3'-*S*-thiobenzoyl-5'-*O*-(4,4'-dimethoxytrityl)-*N***^{**2**}**(dimethyl-aminomethylene)guanosine (10).** Using similar procedure as described above, **8** was converted into 10 in 69% yield, ¹H NMR d 8.80 (s, 1H, CH=N), 8.65 (br s, 1H, NH), 7.70 (s, 1H, H-8), 7.88-6.66 (m, 19H, aromatic), 6.17 (d, J_{2',3'}=5.8, 1H, H-2'), 5.86 (d, J_{1',2'}=1.2, 1H, H-1'), 5.08 (dd, J_{3',,4'}=10.4, J_{3',2'}=5.8, 1H, H-3'), 4.31 (m, 1H, H-4'), 3.67 (s, 6H, 2xOMe), 3.45 (m, 2H, H-5', H-5"), 3.06 (s, 6H, 2X*N*-Me) 2.15 (s, 3H, *O-*Ac), HRMS (FAB⁺) calcd for C₄₃H₄₂N₆O₈S (MH⁺): 803.2863, found 803.2855.

**3'-Deoxy-3'-*S*-pyridyisulfanyl-5'-*O*-(4,4'-dimethoxytrityl)-guanosine (11). A. 9** (530 mg, 0.38 mmol) was dissolved in 40% aqueous methylamine (50 ml) and the mixture is kept at rt for 16 h. The solvent is removed in vacuo and the residual syrup dissolved in argon purged DMF (30 ml) containing 2,2'-dipyridyl disulfide (340 mg, 1.54 mmol). The reaction mixture is heated at 60°C for 10 h and then evaporated to a syrup *in vacuo.* Column chromatography on silica gel using 1-12% gradient of methanol in dichloromethane afforded **11** as a colorless solid (460 mg, 85%), ¹H NMR d 10.64 (br s, 1H, NH), 8.39 (m, 1H, Pyr), 7.83 (s, 1H, H-8), 7.73-6.72 (m, 16H, aromatic), 6.50 (d, J_{OH,2'}=4.80, 1H, OH-2'), 6.45 (br s, 2H, NH₂), 5.81 (d, J_{1',2'}=2.4, 1H, H-1'), 4.83 (m, 1H, H-2'), 4.34 (m, 1H, H-4'), 4.09 (dd, J_{3'2},=6.00, J_{3',4'}=7.8, 1H, H-3'), 3.70 (s, 6H, 2XOMe), 3.11 (dd, J_{5',5"}=11.2, J_{5",4'}=4.8, 1H, H-5"), HRMS (FAB⁺) calcd for C₃₆H₃₄N₆O₆S₂ (MH⁺): 711.2060, found 711.2076.
**B**. Using the same procedure as above, but starting from *S*-benzoyl derivative **10**, **11** was prepared in 80% yield.
**C**. Starting from **16** (830 mg, 1.12 mmol) and using the above conditions
   **11** (570 mg, 67%) was obtained .

**3'-Deoxy-3'-thio-5'-*O*-(4,4'-dimetboxytrityl)-guanosine (12).** To the solution of **11** (240 mg, 0.34 mmol) in chloroform (14 ml) dithiothreitol (DTT) (125 mg, 0.81 mmol) was added and the reaction mixture was stirred at rt for 3 h. It was then evaporated to a syrup *in vacuo,* and the product was precipitated by addition of peroxide-free ether, precipitate was filtered off, washed with ether and dried (230 mg of the crude material), ¹H NMR (DMSO-d₆) d 10.63 (br s, 1H, NH), 7.86 (s, 1H, H-8), 7.32-6.80 (m, 13H, aromatic), 6.49 (br s, 2H, NH₂), 5.81 (s, 1H, H-1'), 4.43 (d, J_{2',3'}=4.8, 1H, H-2'), 3.93 (m, 1H, H-4'), 3.79 (dd, J_{3'2'}=4.8, J_{3',4'}=9.6, 1H, H-3'), 3.71 (s, 6H, 2XOMe), 3.16 (dd, J_{5",5'}=10.4, J_{5",4'}=4.8, 1H, H-5").

**3'-Deoxy-3'-thio-guanosine (13)**. The mixture of the crude **12** (230 mg, 0.33 mmol) and DTT (150 mg) was dissolved in 1 N methanolic HCl (12 ml) and the reaction mixture was kept at rt for 3 h. It was then concentrated *in vacuo* and the residue coevaporated with toluene two times. Addition of ethyl acetate afforded precipitate which was filtered off, washed well with ethyl acetate and dried to afford **13** (90 mg, 79%). The product was reprecipitated from water, ¹H NMR (CD₃OD) d 8.10 (s, 1H, H-8), 5.91 (s, 1H, H-1'), 4.37 (d, _{J2',3'}=5.2, 1H, H-2'), 3.97 (m, 2H, H-4', H-5'), 3.82 (dd, J_{5",5'}=13.0, J_{5",4'}=3.4, 1H, H-5"), 3.64 (dd, J_{3',2'}=5.2, J_{3',4'}=9.6, 1H, H-3'), HRMS (FAB⁺) calcd for C₁₀H₁₃N₅O₄S (MH⁺): 300.0767, found 300.0767.

**Bis (2-*O*-acetyl-*N***^{**2**}**-(dimethylaminomethylene)guanosin-3-yl)disulfide (14 ) and 1-(2-*O*-acetyl-3-deoxy- b -D-*glycero*-pent-3-enofuranosyl)-*N***^{**2**}**-(dimethyl-aminomethylene) guanosine (15 )**. The mixture of **4** and **5** (4.8 g) was dissolved in THF (100 mL) and 1M TBAF in THF (10 mL) was added. The reaction mixture was stirred for 3 h at rt and then evaporated to a syrup *in vacuo.* Silica gel column chromatography using 2-10% gradient of methanol in dichloromethane yielded the faster eluting **15** (1 g, 35% for two steps from **3**, colorless foam), ¹H NMR d 8.96 (br s, 1H, NH), 8.57 (s, 1H, CH=N), 7.65 (s, 1H, H-8), 6.41 (s, 1H, H-2'), 6.04 (s, 1H, H-1'), 5.42 (m, 1H, H-3'), 4.32 (m, 2H, H-5',H-5"), 3.19 (s, 3H, *N*-Me), 3.06 (s, 3H, *N*-Me), 2.11 (s, 3H, Ac). The slower eluting **14** was obtained as a yellowish solid (0.9 g, 29% for two steps), ¹H NMR (DMSO-d₆) d 11.34 (br s, 1H, NH), 8.53 (s, 1H, CH=N), 8.00 (s, 1H, H-8), 5.97 (d, J_{1',2'}=2.4, 1H, H-1'), 5.89 (dd, J_{2',1'}=2.4, J_{2',3'}=6.0, 1H, H-2'), 5.23 (t, J_{OH,5},=5.6, 1H, 5'-OH), 4.11 (m, 1H, H-4'), 4.02 (dd, J_{3',2'}=6.0, J_{3'4'}=8.4, 1H, H-3'), 3.78 (dm, J_{5',5"}=12.0, 1H, H-5'), 3.60 (dm, J_{5",5'}=12.0, 1H, H-5"), 3.10 (s, 3H, *N*-Me), 3.00 (s, 3H, *N*-Me), 2.06 (s, 3H, *O*-Ac), HRMS (FAB⁺) calcd for C₃₀H₃₈N₁₂O₁₀S₂ (MH⁺): 791.2354, found 791.2355.

**Bis (2-*O*-acetyl-5-*O*-(4,4'dimethoxytrityl)-*N***^{**2**}**-(dimethylaminomethylene) gua-nosin-3-yl) disulfide (16)**. To the solution of **14** (400 mg, 0.5 mmol) in dry pyridine (10 ml) DMT-Cl (508 mg, 1.5 mmol) was added and the mixture was stirred 4 h at rt. Methanol (10 ml) was added and the solution evaporated to dryness. The residue is partitioned between saturated NaHCO₃ and dichloromethane, organic layer washed with brine, dried (Na₂SO₄) and evaporated to a syrup. Silica gel column chromatography using 2-10% gradient of methanol in dichloromethane yielded product as a yellowish foam (620 mg, 71% yield), ¹H NMR d 8.72 (br s, 1H, NH), 8.01 (s, 1H, CH=N), 7.48-7.21 (m, 14H, H-8, aromatic), 6.25 (d, J_{2',3},=4.8, 1H, H-2'), 5.78 (s, 1H, H-1'), 4.00 (m, 2H, H-3', H-4'), 3.78 (s, 6H, 2xOMe), 3.50 (br s, 2H, H-5',H-5"), 3.14 (s, 3H, *N*-Me), 3.13 (s, 3H, *N*-Me), 1.82 (s, 3H, *O*-Ac), HRMS (FAB⁺) calcd for C72H74N12014S2 (MH+): 1395.4967, found 1395.4943.

**Bis (5-*O*-(4,4'dimethoxytrityl)-*N***^{**2**}**-(dimethylaminomethylene) guanosin-3-yl)-disulfide (17). A. 16** (60 mg, 0.04 mmol) is dissolved in dry methanol and ion exchange resin AG 1X8 (OH-) (1 g) is added. The mixture was stirred at 55 °C for 16 h, the resin was filtered off and washed well with hot methanol. The filtrate was evaporated to dryness *in vacuo* yielding pure **17** as a colorless solid (16 mg, 28%), ¹H NMR (DMSO-d₆) d 11.34 (br s, 1H, NH), 8.48 (s, 1H, CH=N), 7.92 (s, 1H, H-8), 7.31-6.73 (m, 13H, aromatic), 6.27 (d, J_{OH,2},=5.2, 1H, 2'-OH), 5.88 (d, J_{1',2'}=1.2, 1H, H-1'), 4.60 (m,1H, H-2'), 4.16 (m, 1H, H-4'), 4.08 (m, 1H, H-3'), 3.65 (s, 6H, 2XOMe), 3.65 (m, 2H, H-5', H-5"), 3.02 (s, 3H, *N*-Me), 2.97 (s, 3H, *N*-Me), HRMS (FAB⁺) calcd for C₆₈H₇₀N₁₂O₁₂S₂ (MH⁺): 1311.4756, found 1311.4746.

**B**. Using Amberlyst A-26 (CN⁻) under the the above reaction conditions, **17** was obtained from 16 in 21% yield.

**3'-Deoxy-3'-*S*-pyridylsulfanyl-5'-*O*-(4,4'-dimethoxytrityl)-*N***^{**2**}**-(dimethylamino-methylene)guanosine (18) and 17.** To the solution of **11** (400 mg, 0.56 mmol) in dry pyridine (5 ml), *N,N*-dimethylformamide dimethyl acetal (1.2 ml, 9 mmol) was added and the reaction mixture was stirred at rt for 16 h. Solvents were removed in vacuo and the residue chromatographed on the column of silica gel using 1-50% gradient of methanol in dichloromethane. Fractions containing the faster running material were combined and concentrated *in vacuo* to give **18** (110 mg, 23%), ¹H NMR d 8.73 (br s, 1H, NH), 8.53 (s, 1H, CH=N), 8.49 (m, 1H, pyridine), 7.71 (s, 1H, H-8), 7.62 (m, 1H, pyridine), 7.44-6.79 (m, 15H, aromatic), 6.09 (s, 1H, H-1'), 4.55 (d, J_{2',3'}=4.8, 1H, H-2'), 4.23 (dq, J_{4',3'}=10.5, J_{4',5'}=2.8, J_{4',5"}=3.4, 1H, H-4'), 4.14 (dd, J_{3'2'}=4.8, J_{3',4'}=10.5, 1H, H-3'), 3.78 (s, 6H, 2XOMe), 3.57 (dd, J_{5',5"}=10.6. J_{5',4'}=2.8. 1H, H-5'), 3.41 (dd, J_{5',5"}=10.6, J_{5",4'}=3.4, 1H, H-5"), 3.08 (s, 3H, *N*-CH₃), 3.05 (s, 3H, *N*-CH₃). Fractions containing the slower running compound were collected and evaporated to dryness *in vacuo* to give **17** as a colorless foam (120 mg, 33%), ¹H NMR identical to that of **17** obtained obtained using the above procedures.

**2'-*O*-*t*-Butyldimethylsilyl-3'-deoxy-3'-*S*-pyridylsulfanyl-5'-*O*-(4,4'-dimethoxytrityl)--*N***^{**2**}**-(dimethylamino-methylene) guanosine (19). 18** (110 mg, 0.14 mmol) was dissolved in dry pyridine (1 ml) and TBDMS-Tf (0.103 ml, 0.45 mmol) was added to the solution. The reaction mixture was stirred at rt for 5 h, then qunched with methanol and evaporated to a syrup *in vacuo.* The residue was dissoved in dichloromethane, washed with 5% aqueous NaHCO₃, then brine and the organic layer was dried (Na₂SO₄) and concentrated to the syrup. Column chromatography on silica gel using 1-10% gradient of methanol in ethyl acetate afforded **19** as a colorless solid (90 mg, 71%), ¹H NMR d 8.69 (br s, 1H, NH), 8.50 (s, 1H, CH=N), 8.37 (m, 1H, pyridine), 7.81 (s, 1H, H-8), 7.50-6.74 (m, 16H, aromatic), 5.98 (d, J_{1',2'}=2.4, 1H, H-1'), 4.75 (dd, J_{2',3'}=5.0, J_{2',1'}=2.4, 1H, H-2'), 4.50 (m,1H, H-4'), 3.99 (dd, J_{3'2'}=5.0, J_{3',4},=8.4, 1H, H-3'), 3.76 (s, 6H, 2XOCH₃), 3.62 (dd, J_{5',5"}=10.9. J_{5',4'}=2.2. 1H, H-5'), 3.38 (dd, J_{5',5"}=10.9, J_{5",4'}=4.4, 1H, H-5"), 3.06 (s, 3H, *N*CH₃), 3.04 (s, 3H, *N*CH₃), 0.93 (s, 9H, *t*-Bu), 0.17 (s, 3H, Me), 0.10 (s, 3H, Me), HRMS (FAB⁺) calcd for C₄₅H₅₃N₇O₆S₂Si (MH⁺) 880.3346, found 880.3357.

**2'-*O*-*t*-Butyldimethylsilyl-3'-deoxy-3'-*S*-pyridylsulfanyl-5'-*O*-(4,4'-dimethoxytrityl) guanosine (20).** 3'-Deoxy-3'-*S*-pyridylsulfanyl-5'-*O*-(4,4'-dimethoxytrityl)guanosine **11** (410 mg, 0.58 mmol) was dissolved in dry pyridine (36 ml) and imidazole (2.36 g, 35 mmol) and TBDMS-Cl (4.29 g, 28 mmol) were added. The reaction was stirred at rt 16 h, then evaporated to a syrup in vacuo. The residue was partitioned between dichloromethane and saturated aq. NaHCO₃, organic layer washed with water, dried (Na₂SO₄) and concentrated to a syrup. Column cgromatography on silica gel using 1-10% gradient of methanol in dichloromethane afforded the product as a white foam (430 mg, 85%), ¹H NMR (DMSO-d₆) d 11.99 (br s, 1H, NH), 8.39 (m, 1H, Pyr), 7.84 (s, 1H, H-8), 7.71 (m, 1H, Pyr), 7.62-6.72 (m, 15H, aromatic) 6.41 (br s, 2H, NH₂), 5.80 (d, J_{1',2'}=4.4, 1H, H-1'), 5.04 (app t, J_{2',3'}=4.4, 1H, H-2'), 4.39 (m, 1H, H-4'), 4.01 (app t, J_{3',4'}=6.4, 1H, H-3'), 3.69 (s, 6H, 2XOMe), 3.13 (dd, J_{5",5'}=11.0, J_{5",4'}=4.6, 1H, H-5"), 0.82 (s, 9H, *t*-Bu), 0.08 (s, 3H, Me), 0.06 (s, 3H, Me), HRMS (FAB⁺) calcd for C₄₂H₄₈N₆O₆S₂Si (MH⁺) 825.2924, found 825.2977.

**2'-*O*-*t*-Butyldimethylsilyl-3'-deoxy-3'-*S*-pyridylsulfanyl-5'-*O*-(4,4'-dimethoxytrityl)-*N***^{**2**}**-isobutyrylguanosine (21).** To the solution of **20** (310 mg, 0.38 mmol) in dry pyridine (5 ml) isobutyric anhydride (0.19 ml, 1.14 mmol) and DMAP (46 mg, 0.38 mm) were added and the mixture was stirred at rt 16 h. It was then stirred at 50 °C for 5 h, quenched with methanol (2 ml) and evaporated to a syrup *in vacuo*. The residue was partitioned between dichloromethane and 5% aqueous NaHCO₃, the organic layer was washed with brine, dried (Na₂SO₄) and evaporated to a syrup. Column chromatography on silica gel using 1-5% gradient of methanol in CH₂Cl₂ afforded product as a colorless foam (320 mg, 95%), ¹H NMR d 11.94 (br s, 1H, NH), 8.36 (m, 1H, Pyr), 7.85 (m, 1H, Pyr), 7.80 (s, 1H, H-8), 7.58-6.71 (m, 16H, NH, aromatic), 5.83 (d, J_{1',2'}=5.2, 1H, H-1'), 5.22 (app t, J_{1',2'}=5.2, 1H, H-2'), 4.50 (m, 1H, H-4'), 4.27 (app t, J_{3',4'}=6.4, 1H, H-3'), 3.76 (s, 3H, OMe), 3.75 (s, 3H, OMe), 3.56 (dd, J_{5',5"}=11.0, J_{5',4'}=1.8, 1H, H5'), 2.98 (dd, J_{5",5'}=11.0, J_{5",4'}=3.0, 1H, H-5"), 1.69 (m, 1H, *CH*Me₂), 0.94 (d, J=7.2, 3H, CH₃), 0.76 (d, J=7.2, 3H, CH₃), 0.88 (s, 9H, *t*-Bu), 0.11 (s, 3H, Me), 0.06 (s, 3H, Me), HRMS (FAB⁺) calcd for C₄₆H₅₄N₆O₇S₂Si (MH⁺) 895.3343, found 895.3380.

**2'-*O*-*t*-Butyldimethylsilyl-3'-deoxy-3'-thio-5'-*O*-(4,4'-dimethoxytrityl)-*N***^{**2-**}**isobutyrylguanosine (22)**. To the solution of **21** (340 mg, 0.38 mmol) in chloroform (20 ml) TEA (0.4 ml) and dithiotreitol DTT (140 mg, 0.91 mmol) were added and the reaction mixture was stirred for 1 h at rt. The reaction miture was washed with saturated aqueous NaHCO₃, water, dried (Na₂SO₄) and concentrated to a syrup. Silica gel column chromatography using 0.5-2% gradient of methanol in CH₂Cl₂ afforded **22** (270 mg, 90%), ¹H NMR for the major rotamer: d 11.92 (br s, 1H, NH), 7.93 (s, 1H, H-8), 7.63-6.80 (m, 16H, NH, aromatic), 5.83 (d, J_{1,2'}=2.8, 1H, H-1'), 4.74 (dd, J_{2,1},=2.8, J_{2',3'}=5.4, 1H, H-2'), 4.13 (dd, J_{4',3'}=7.6, J_{4',5'}=1.2, 1H, H-4'), 3.78 (s, 3H, OMe), 3.73 (s, 3H, OMe), 3.63 (dd, J_{5',5'}=11.0, J_{5',4'}=1.2, 1H, H5'), 3.27 (dd, J_{5',5'}=11.0, J_{5",4},=3.0, 1H, H-5"), 1.63 (d, J_{SH,3},=8.4, 1H, SH), 2.08 (m, 1H, *CH*Me₂), 1.09 (d, J=6.8, 3H, CH₃), 0.98 (d, J=6.8, 3H, CH₃), 0.91 (s, 9H, t-Bu), 0.14 (s, 3H, Me), 0.08 (s, 3H, Me), HRMS (FAB⁺) calcd for C₄₁H₅₁N₅O₇SSi (MH⁺) 786.3357, found 786.3354.

**2'-*O*-*t*-Butyldimethylsilyl-3'-deoxy-3'-thio-5'-*O*-(4,4'-dimethoxytrityl)-*N***^{**2-**}**isobutyrylguanosine 3'-*S*-(2-cyanoethyl *N,N*-diisopropylphoramidite (23).** Phosphitylation of **22** as described by Sun *et al*. afforded product which was purified by flash chromatography using 0.5% ethanol in CH₂Cl₂ containing 1% TEA. Final product was obtained as a white powder by precipitation from toluene-pentane at 0 °C (76% yield), ³¹P NMR d 163.5 (s), 159.6 (s), HRMS (FAB⁺) calcd for C₅₀H₆₈N₇O₈PSSi (MH⁺) 986.4435, found 986.4406.

### Example 13: Synthesis of 5'- thiophosphate nucleoside phosphoramidite and preparation of solid support

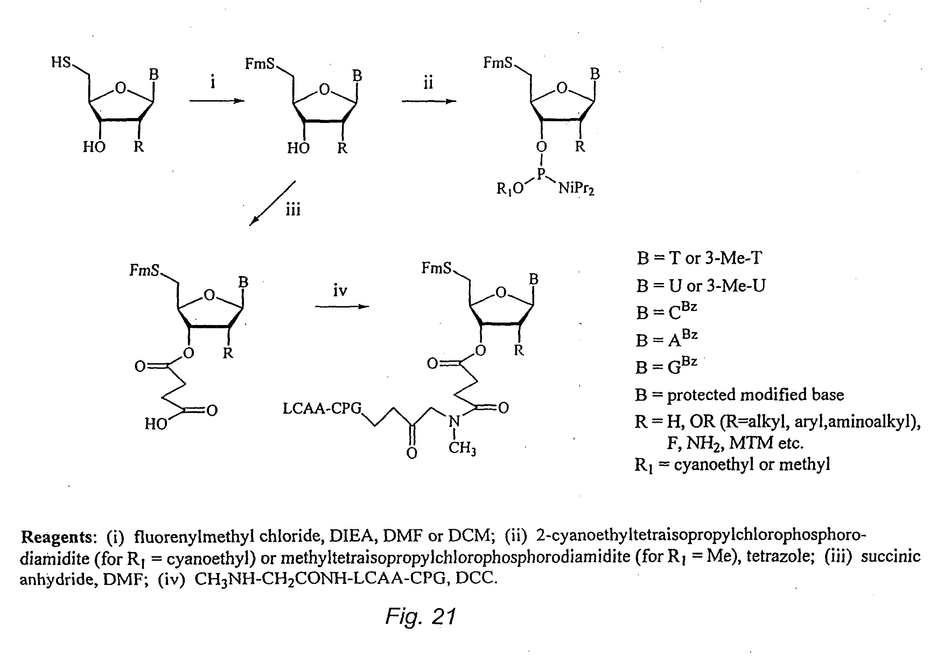

Referring to **Figure 21,** Applicant shows a scheme for synthesis of 5'-deoxy-5'-thionucleoside phosphoramidites and succinates. For example Lehmann *et al*., (*NAR* 1989, 17, 2379; incorporated by reference herein) describes the preparation of the more base-stable sarcosyl modified solid support.

Matulic-Adamic. *et al*. in *Nucleosides & Nucleosides* 1997, 16, 1933, describes the incorporation of 5'-thio modification into oligonucleotides. Applicant additionally describes the a method where, the cleavage of the 5'-protecting group on the solid support in order to elongate the chain is accomplished by using, instead of AgNO3 in CH3CN, 0.1M 1,8-diazabicyclo[5.4.0]undec-7-ene (DBU) in CH3CN or 20% piperidine in CH3CN.

Note that 5'S-DMT protection is cleaved with iodine (Henningfeld *et al. JACS* 1996, 118, 11701) thus complicating the oligo synthesis, while S-Fm is reported to be resistant to 0.1 M iodine in DMF.

Oligonucleotides were synthesized with 5'-thiophosphate linkage and tested *in vitro* in a standard RNase H cleavage assay described in McSwiggen, US Patent No. 5,525,468; incorporated by reference herein.

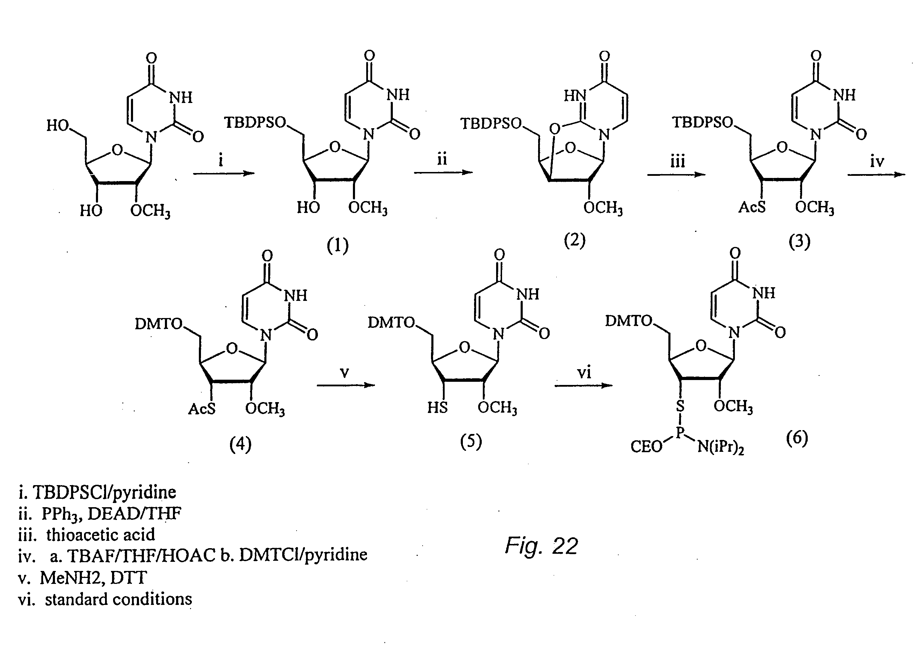

### Example 14: Synthesis of 5'-O-dimethoxytrityl-3'-deoxy-3'-thio-3'-S-(2-Cyanoethyl-N,N-Diisopropylphosphoramidite-2'-O-methyl uridine (6) (Figure 22)

*5'-O-tert-butyldiphenylsilyl-2'-O-methyl uridine* (**1**)*:* To a solution of 2'-*O-*methyl uridine stirring at 0 C under positive pressure argon in anhydrous pyridine was added *tert*-butyldiphenylsilyl chloride (1.2 eq.) The reaction mixture was allowed to warm to rt and was maintained at rt for 18 hours at which time ethanol was added, pyridine removed in vacuo, and the reaction residue partitioned between dichloromethane and sat. aq. sodium bicarbonate. The organic layer was then dried over sodium sulfate. Flash chromatography using an ethyl acetate/hexanes gradient gave (1) as a white foam.

*5'-O-tert-butyldiphenylsilyl-2'-O-methyl-2,3'-anhydro uridine* (**2**): To a solution of (**1**) and DEAD (3.5 eq.) stirring at 0 C under argon in anhydrous THF was added triphenylphosphine (3.5 eq.). The reaction mixture was warmed to rt and stirred at rt under argon for 18 hours, after which THF was removed *in vacuo*. The crude reaction residue was partitioned between dichloromethane and sat. aq. sodium bicarbonate, the organics dried over sodium sulfate preceding flash chromatography. An ethyl acetate/hexanes gradient afforded (2) as an off white foam.

*5'-O-tert-butyldiphenylsilyl-3'-S-acetyl-2'-O-methyl uridine* (**3**): Compound (**2**) was treated with thiolacetic acid in dioxane at 100 C for 18 hours while stirring in a stainless steel bomb. The reaction mixture was evaporated *in vacuo* then purified by flash silica gel chromatography to give (**3**) as a light yellow foam.

*5'-O-dimethoxytrityl-3'-S-acetyl-2'-O-methyl uridine* (**4**): To a solution of (3) stirring at rt under positive pressure argon was added 1M TBAF in THF buffered with acetic acid. The resulting clear, light yellow solution was stirred at rt for one hour, then THF removed *in vacuo.* Crude (4) was flash silica purified using an ethanol/dichloromethane gradient. The purified product was then co-evaporated with anhydrous pyridine, then dissolved in anhydrous pyridine. Dimethoxytrityl chloride was added to the reaction at rt and the resulting clear, reddish solution stirred at rt for 18 hours. Pyridine was removed in vacuo after quenching with ethanol, and the resulting crude foam partitioned between dichloromethane and sat. aq. sodium bicarbonate and the organics dried over sodium sulfate. Flash chromatography using an ethyl acetate/hexanes gradient furnished pure (4).

*5'-O-dimethoxytrityl-3'-deoxy-3'-thio-2'-O-methyl uridine* (**5**)*:* Compound (4) was dissolved in 40% aq. methylamine in the presence of DTT. The reaction mixture was stirred at rt for one hour then evaporated *in vacuo.* Flash chromatography using an ethyl acetate/hexanes gradient gave (5) as an off white foam.

*5'-O-dimethoxytrityl-3'-deoxy-3'-thio-3'-S-(2-Cyanoethyl-N,N-Diisopropylphosphoramidite-2'-O-methyl uridine* (**6**): To a cooled (0 °C) solution of (5) and *N,N*-diisopropylethylamine in dry CH₂Cl₂ was added 2-cyanoethyl *N,N*-diisopropylchlorophosphoramidite dropwise via syringe. The mixture was stirred at room temperature until all starting material was consumed (5 hr.) The reaction mixture was quenched with anhydrous ethanol and diluted with hexanes. Flash chromatography using an ethyl acetate/hexanes gradient provided pure (6).

### Diagnostic uses

Nucleic acid molecules of this invention may be used as diagnostic tools to examine genetic drift and mutations within diseased cells or to detect the presence of specific RNAs in a cell. The close relationship between for example ribozyme activity and the structure of the target RNA allows the detection of mutations in any region of the molecule which alters the base-pairing and three-dimensional structure of the target RNA. By using multiple ribozymes described in this invention, one may map nucleotide changes which are important to RNA structure and function *in vitro,* as well as in cells and tissues. Cleavage of target RNAs with ribozymes may be used to inhibit gene expression and define the role (essentially) of specified gene products in the progression of disease. In this manner, other genetic targets may be defined as important mediators of the disease. These experiments will lead to better treatment of the disease progression by affording the possibility of combinational therapies (e.g., multiple nucleic acid molecules targeted to different genes, nucleic acid molecules coupled with known small molecule inhibitors, or intermittent treatment with combinations of nucleic acid molecules and/or other chemical or biological molecules). Other *in vitro* uses of nucleic acid molecules of this invention are well known in the art, and include detection of the presence of RNAs related to various conditions. Such RNA is detected by determining the presence of a cleavage product after treatment with for example, an enzymatic nucleic acid molecule using standard methodology.

In a specific example, ribozymes which can cleave only wild-type or mutant forms of the target RNA are used for the assay. The first ribozyme is used to identify wild-type RNA present in the sample and the second ribozyme will be used to identify mutant RNA in the sample. As reaction controls, synthetic substrates of both wild-type and mutant RNA will be cleaved by both ribozymes to demonstrate the relative ribozyme efficiencies in the reactions and the absence of cleavage of the "non-targeted" RNA species. The cleavage products from the synthetic substrates will also serve to generate size markers for the analysis of wild-type and mutant RNAs in the sample population. Thus each analysis will require two ribozymes, two substrates and one unknown sample which will be combined into six reactions. The presence of cleavage products will be determined using an RNAse protection assay so that full-length and cleavage fragments of each RNA can be analyzed in one lane of a polyacrylamide gel. It is not absolutely required to quantify the results to gain insight into the expression of mutant RNAs and putative risk of the desired phenotypic changes in target cells. The expression of mRNA whose protein product is implicated in the development of a phenotype is adequate to establish risk. If probes of comparable specific activity are used for both transcripts, then a qualitative comparison of RNA levels will be adequate and will decrease the cost of the initial diagnosis. Higher mutant form to wild-type ratios will be correlated with higher risk whether RNA levels are compared qualitatively or quantitatively.

### Additional Uses

Potential usefulness of sequence-specific enzymatic nucleic acid molecules of the instant invention might have many of the same applications for the study of RNA that DNA restriction endonucleases have for the study of DNA (Nathans *et al*., 1975 *Ann. Rev. Biochem*. 44:273). For example, the pattern of restriction fragments could be used to establish sequence relationships between two related RNAs, and large RNAs could be specifically cleaved to fragments of a size more useful for study. The ability to engineer sequence specificity of the ribozyme is ideal for cleavage of RNAs of unknown sequence.

Other embodiments are within the following claims.

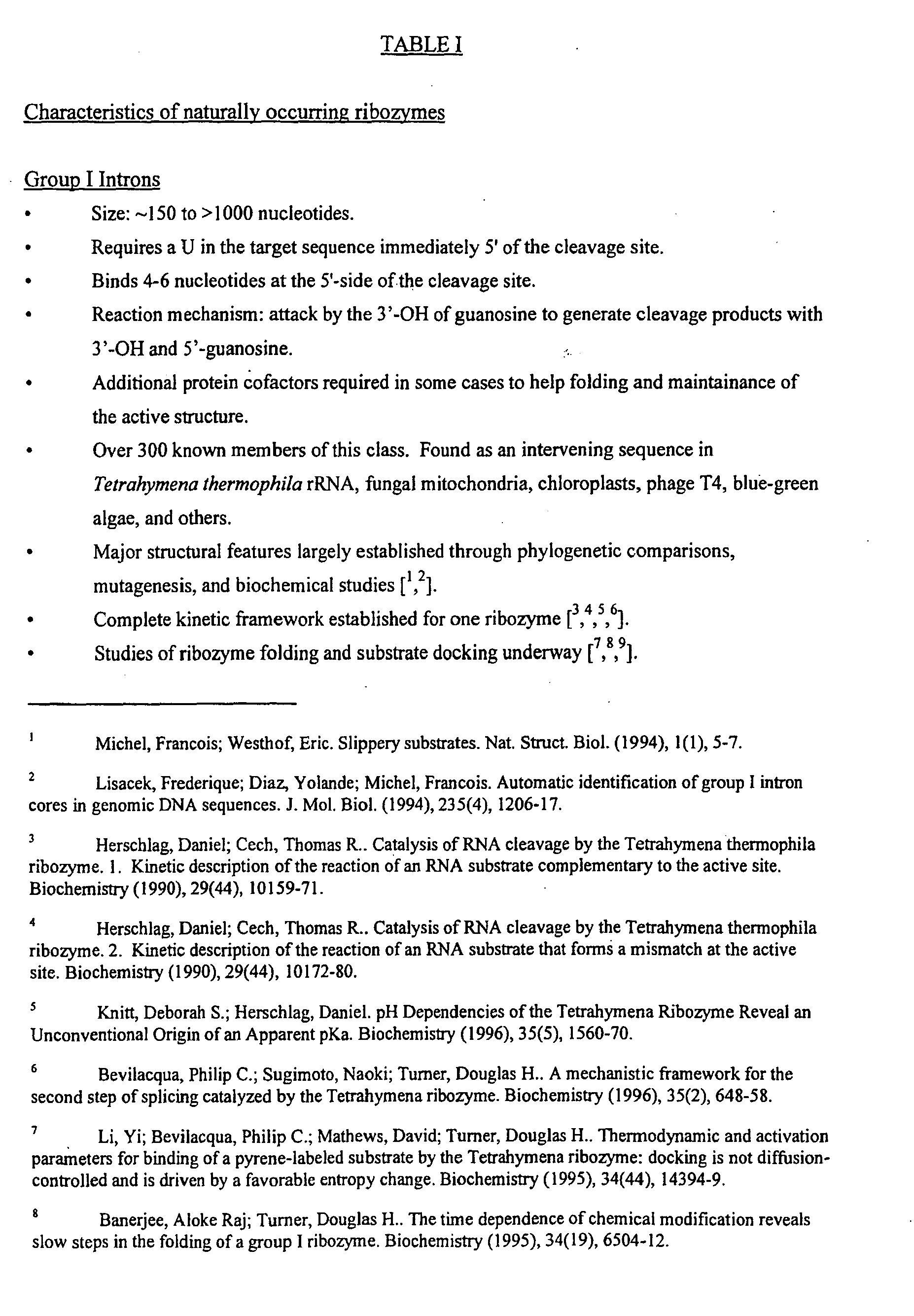

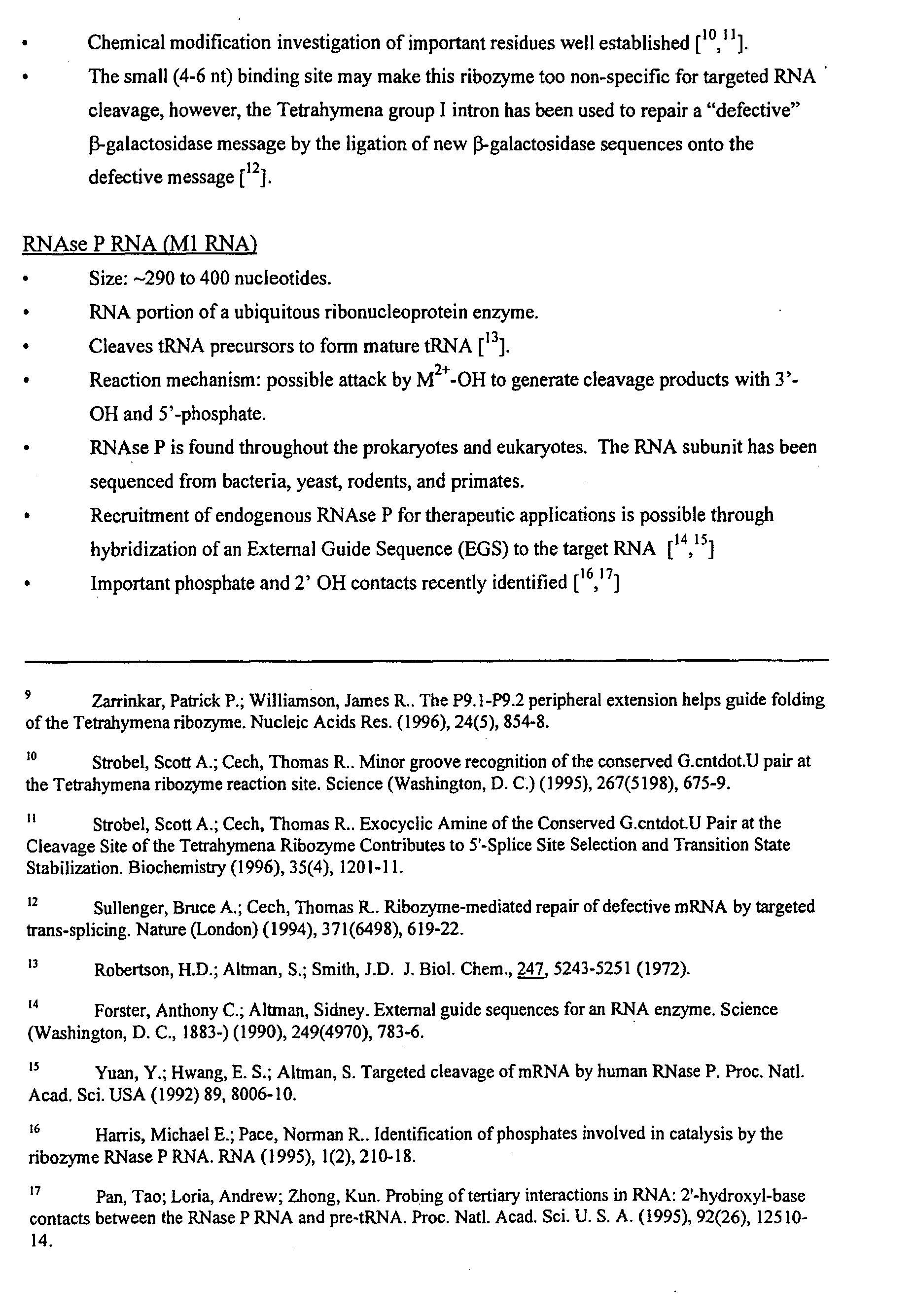

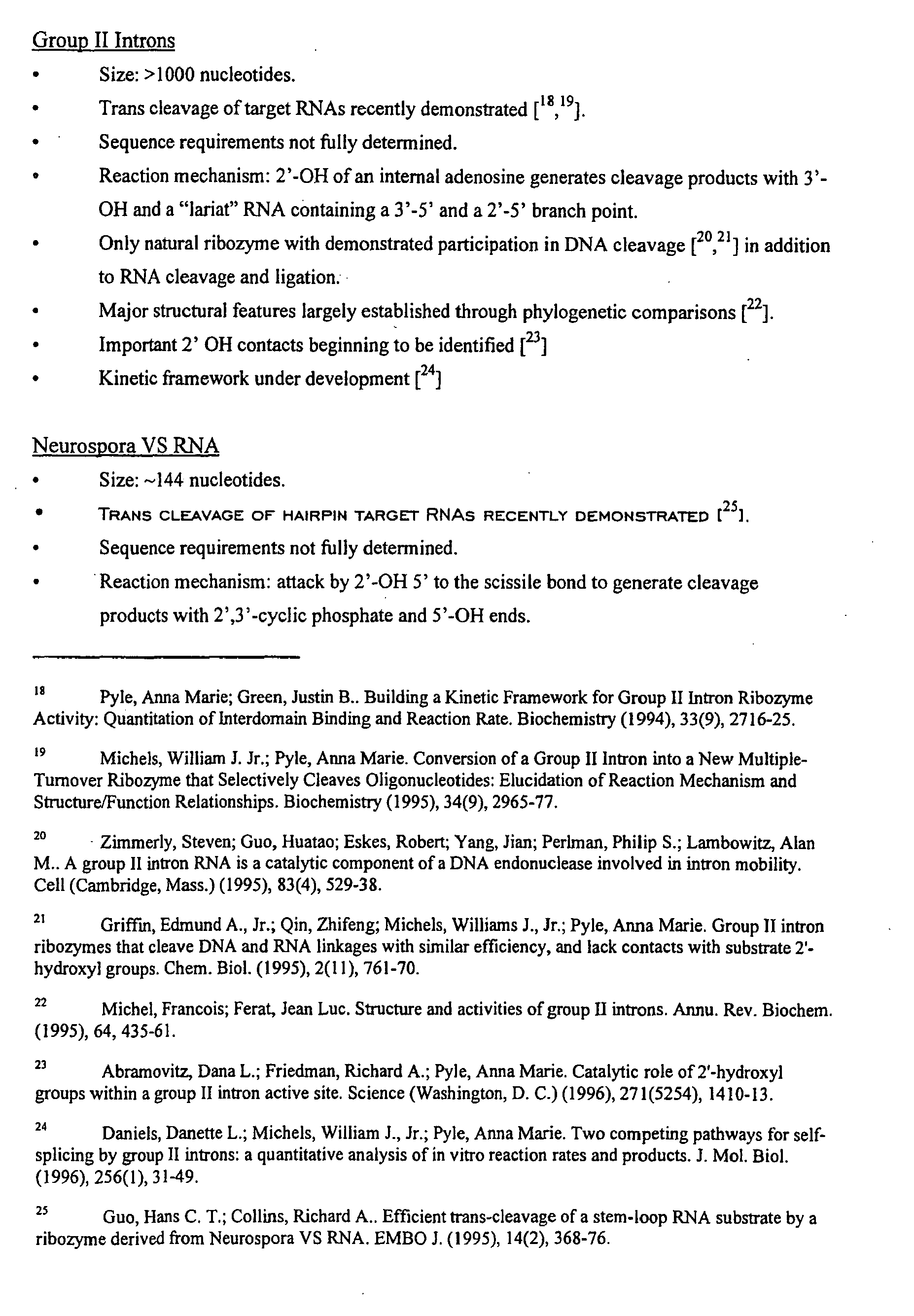

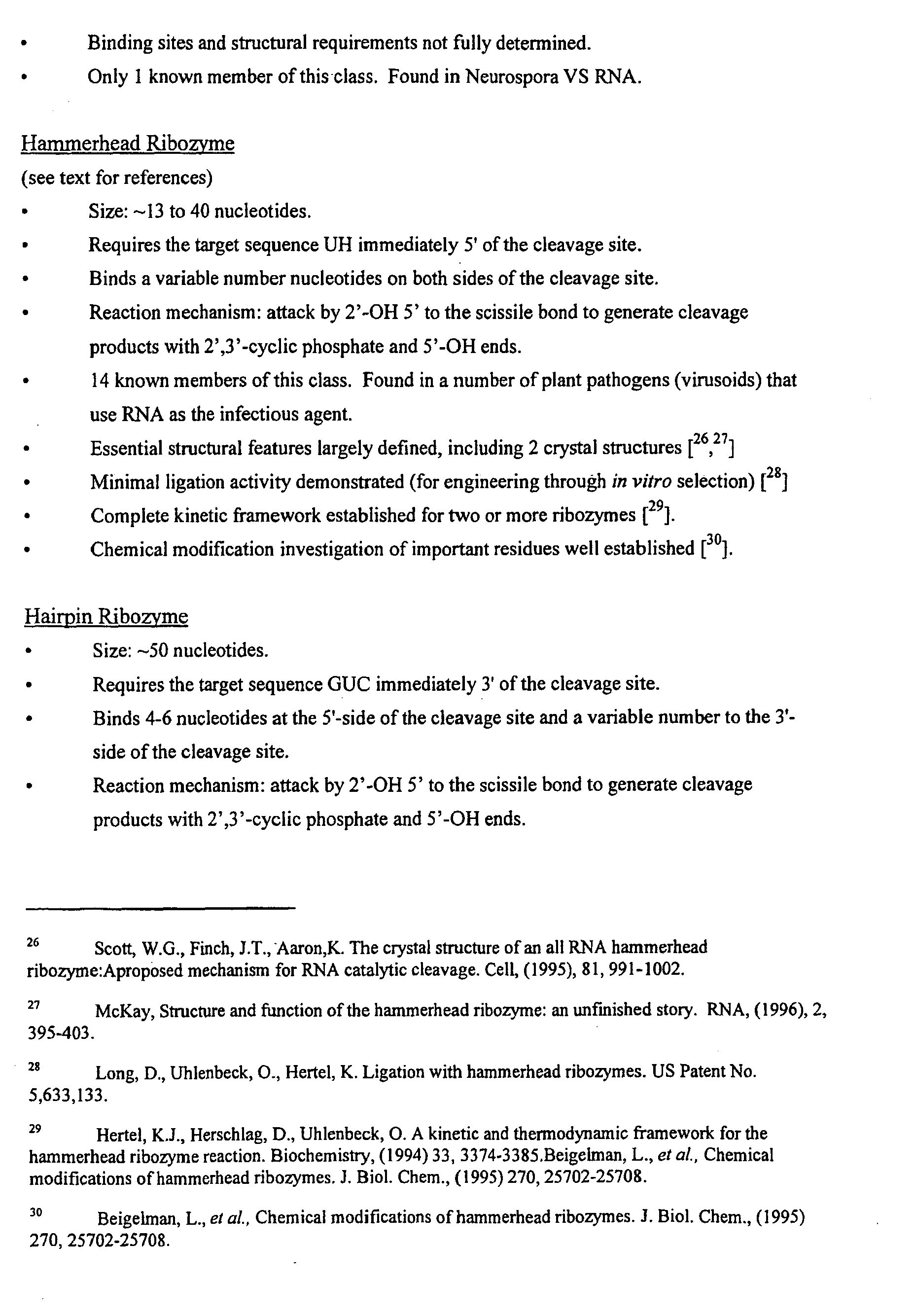

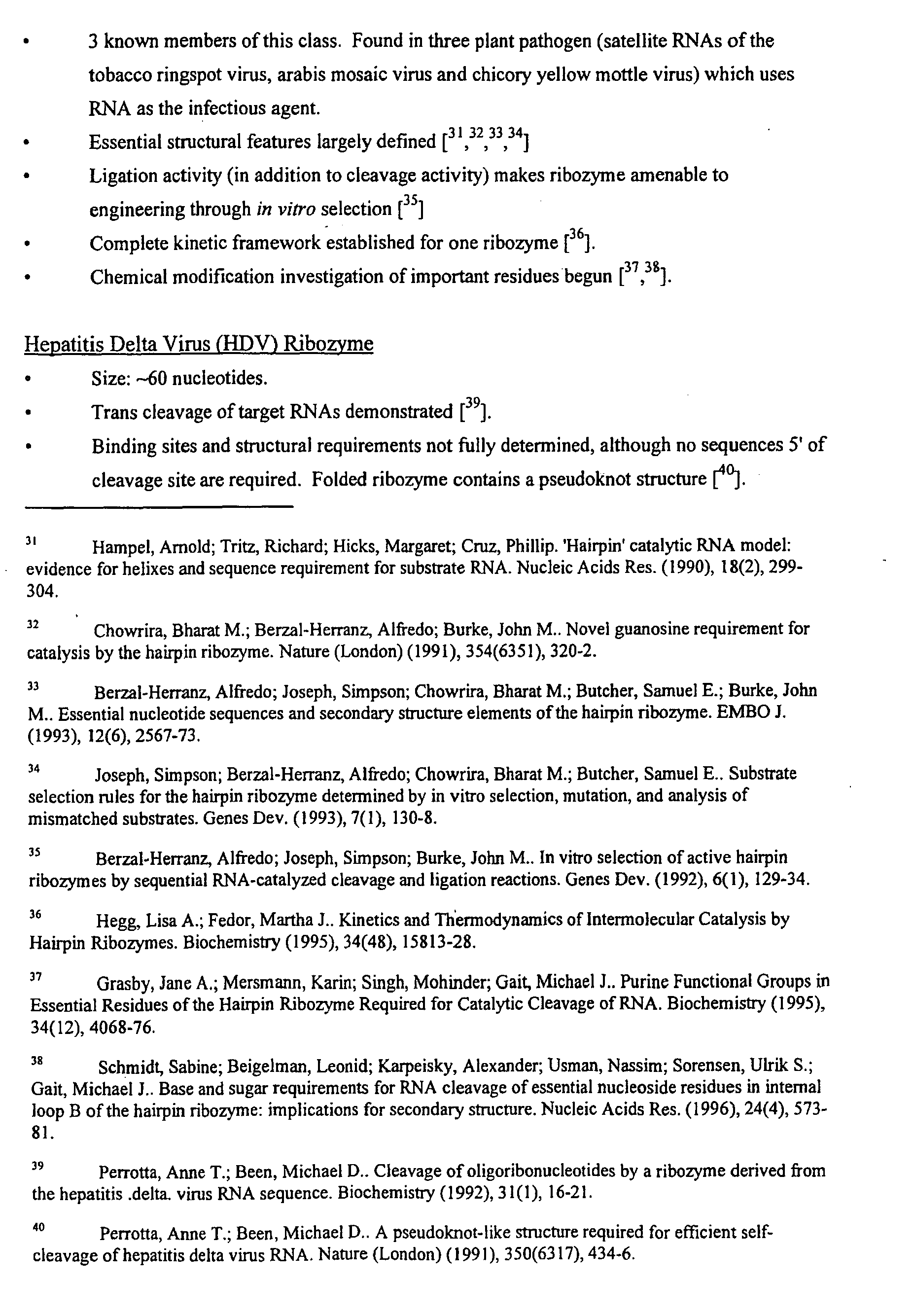

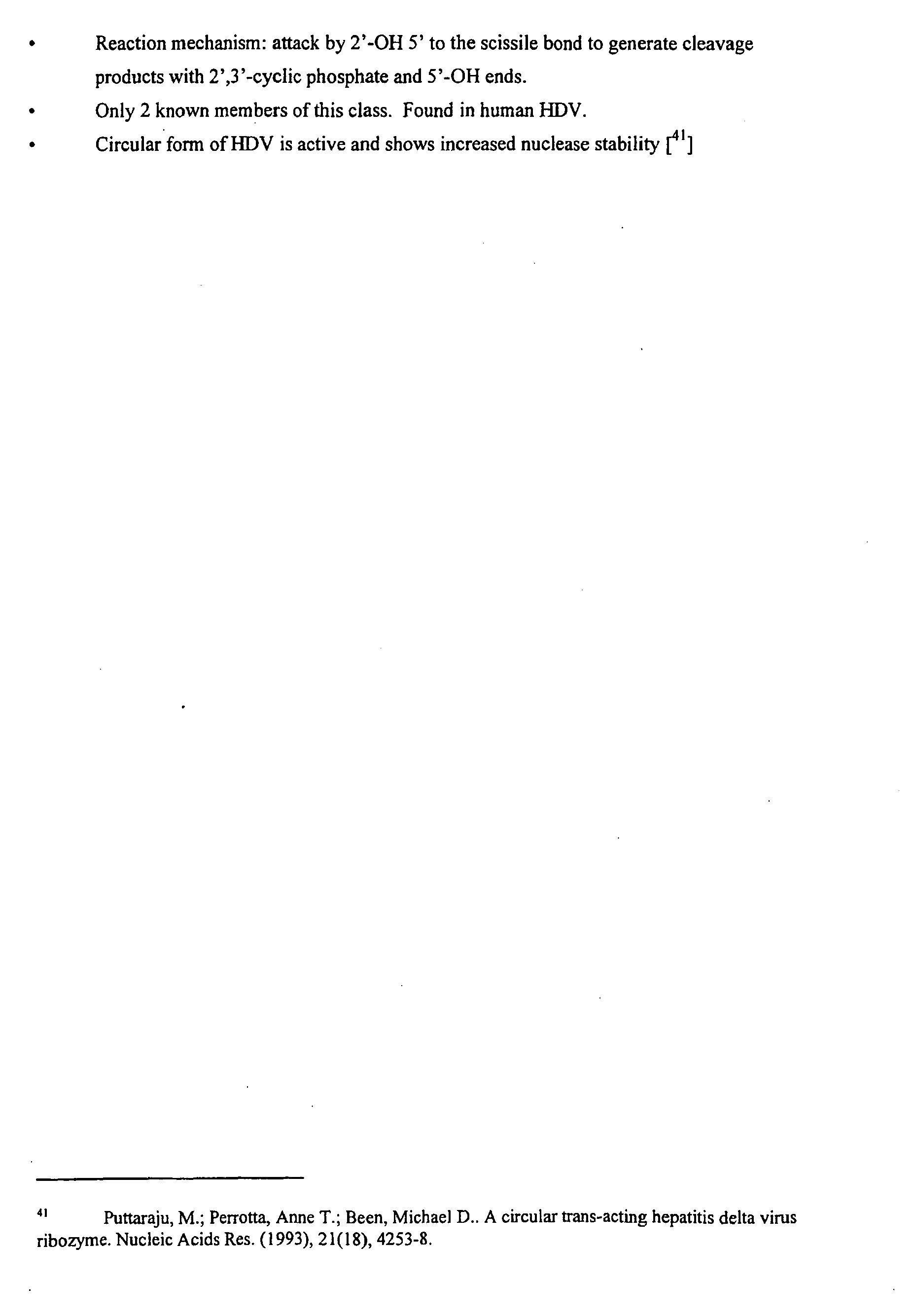

### Table II: 2.5 µmol RNA Synthesis Cycle

| **Reagent** | **Equivalents** | **Amount** | **Wait Time*** |
|---|---|---|---|
| Phosphoramidites | 6.5 | 163 µL | 2.5 |
| *S*-Ethyl Tetrazole | 23.8 | 238 µL | 2.5 |
| Acetic Anhydride | 100 | 233 µL | 5 sec |
| *N*-Methyl Imidazole | 186 | 233 µL | 5 sec |
| TCA | 83.2 | 1.73 mL | 21 sec |
| Iodine | 8.0 | 1.18 mL | 45 sec |
| Acetonitrile | NA | 6.67 mL | NA |

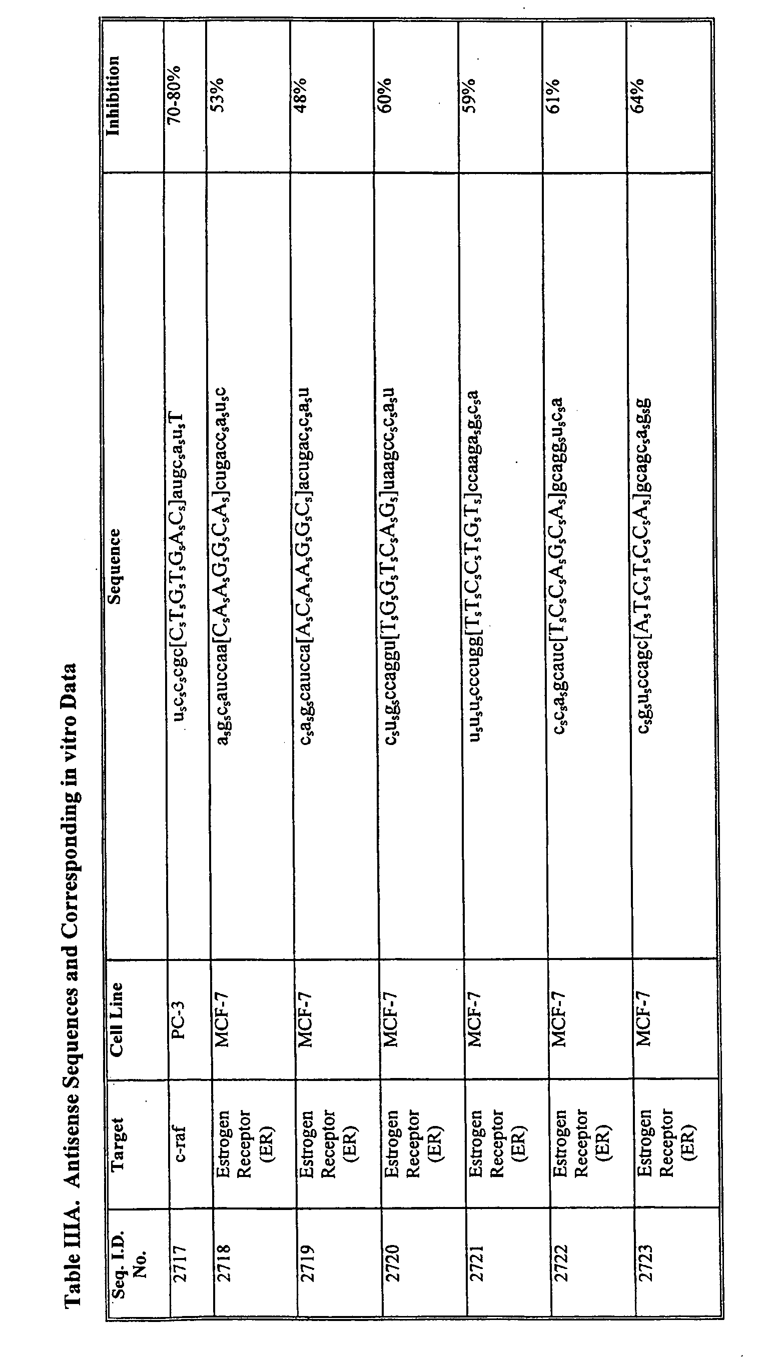

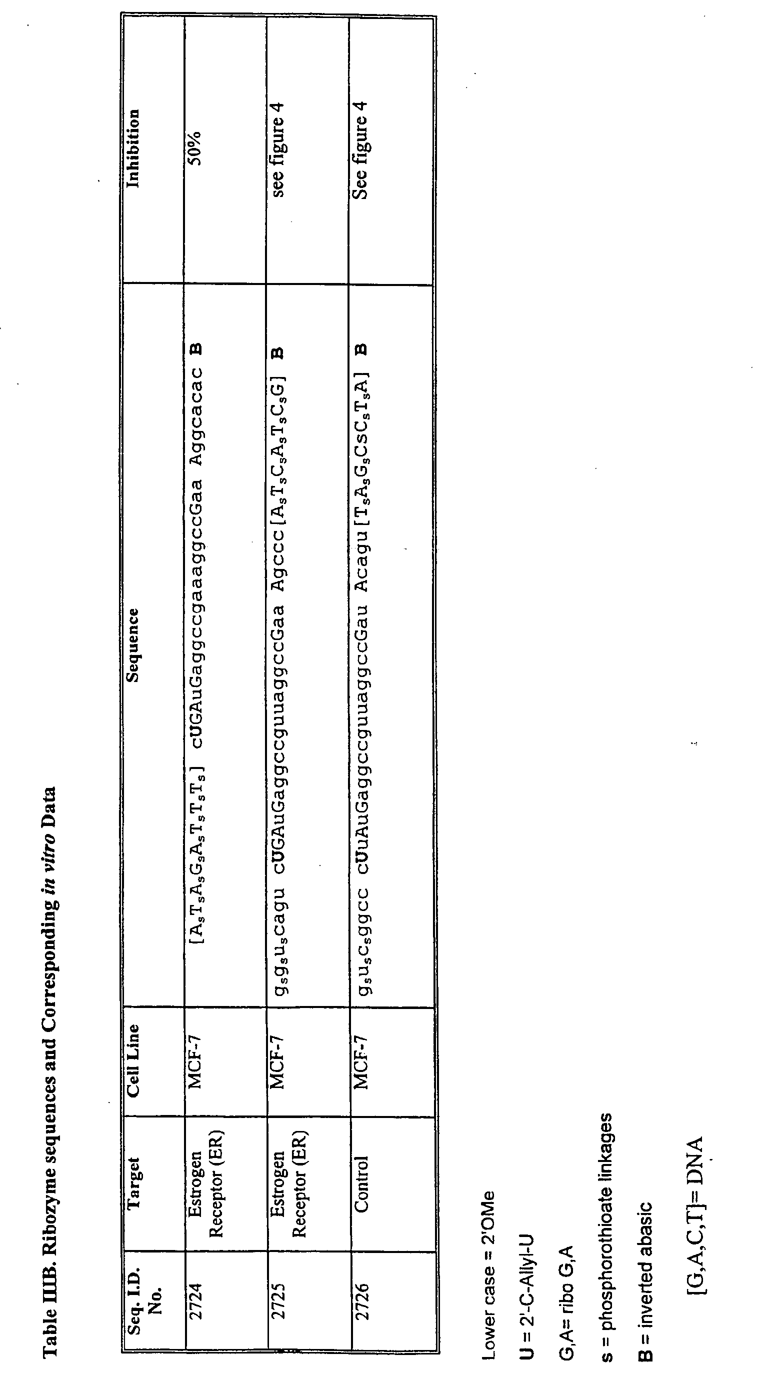

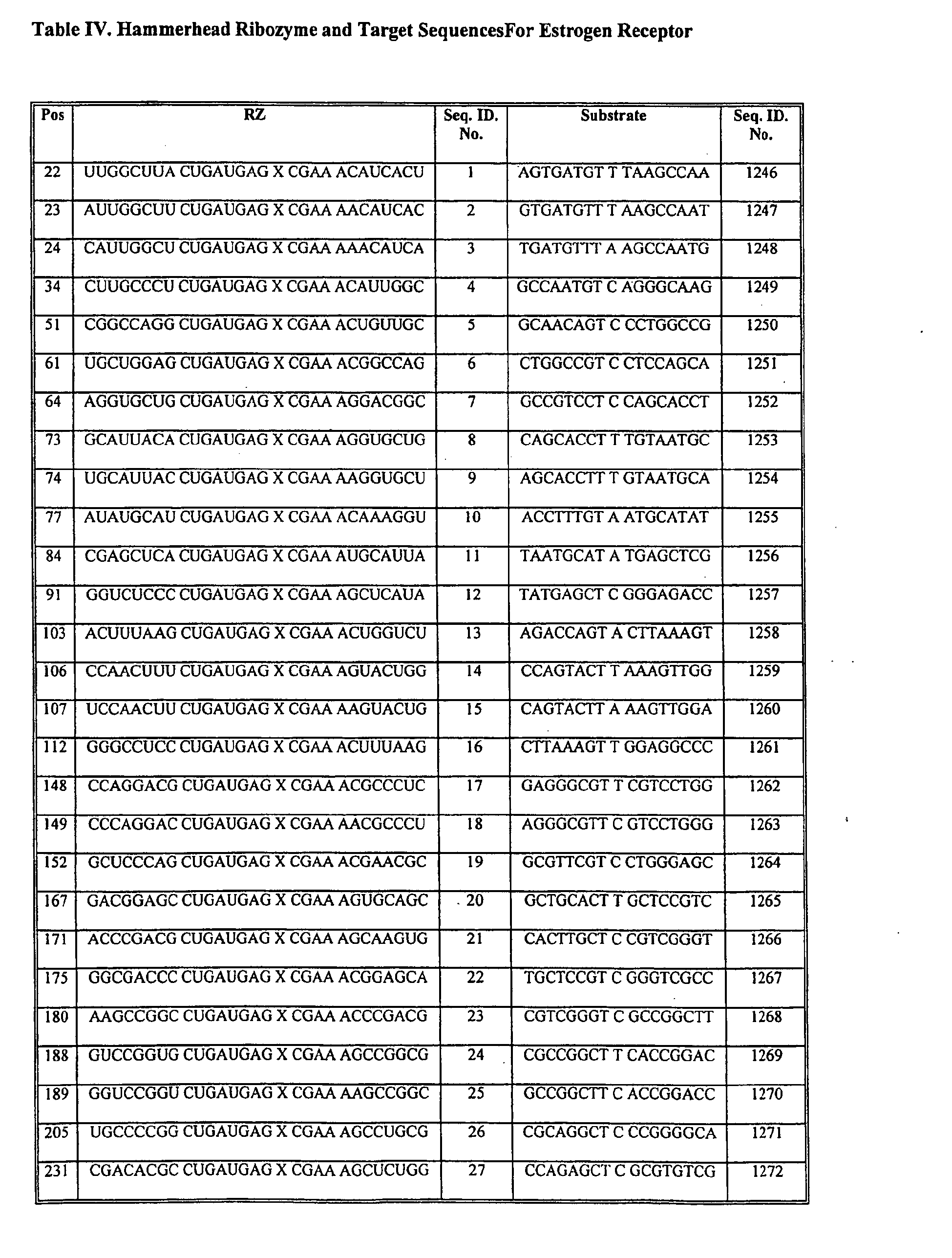

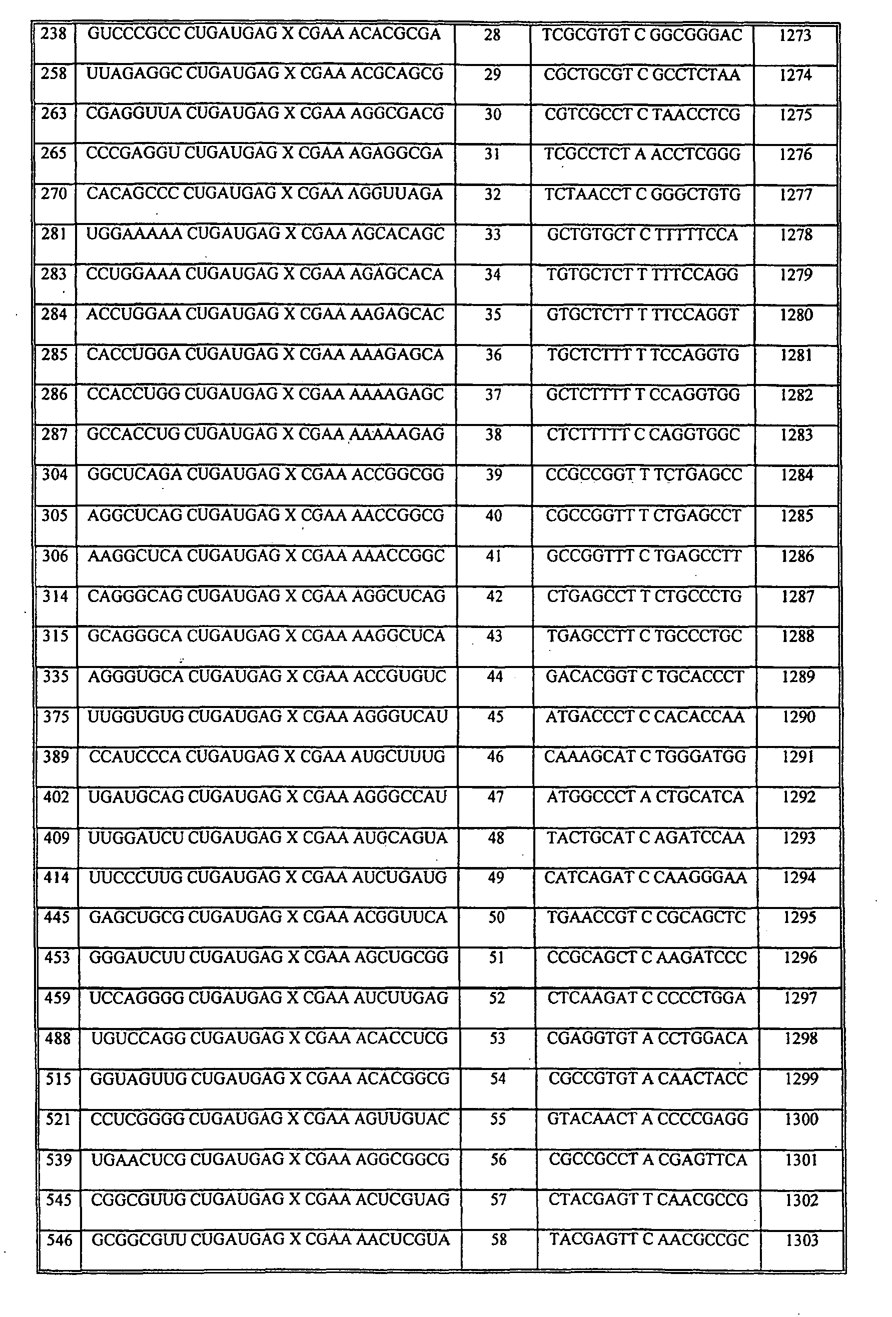

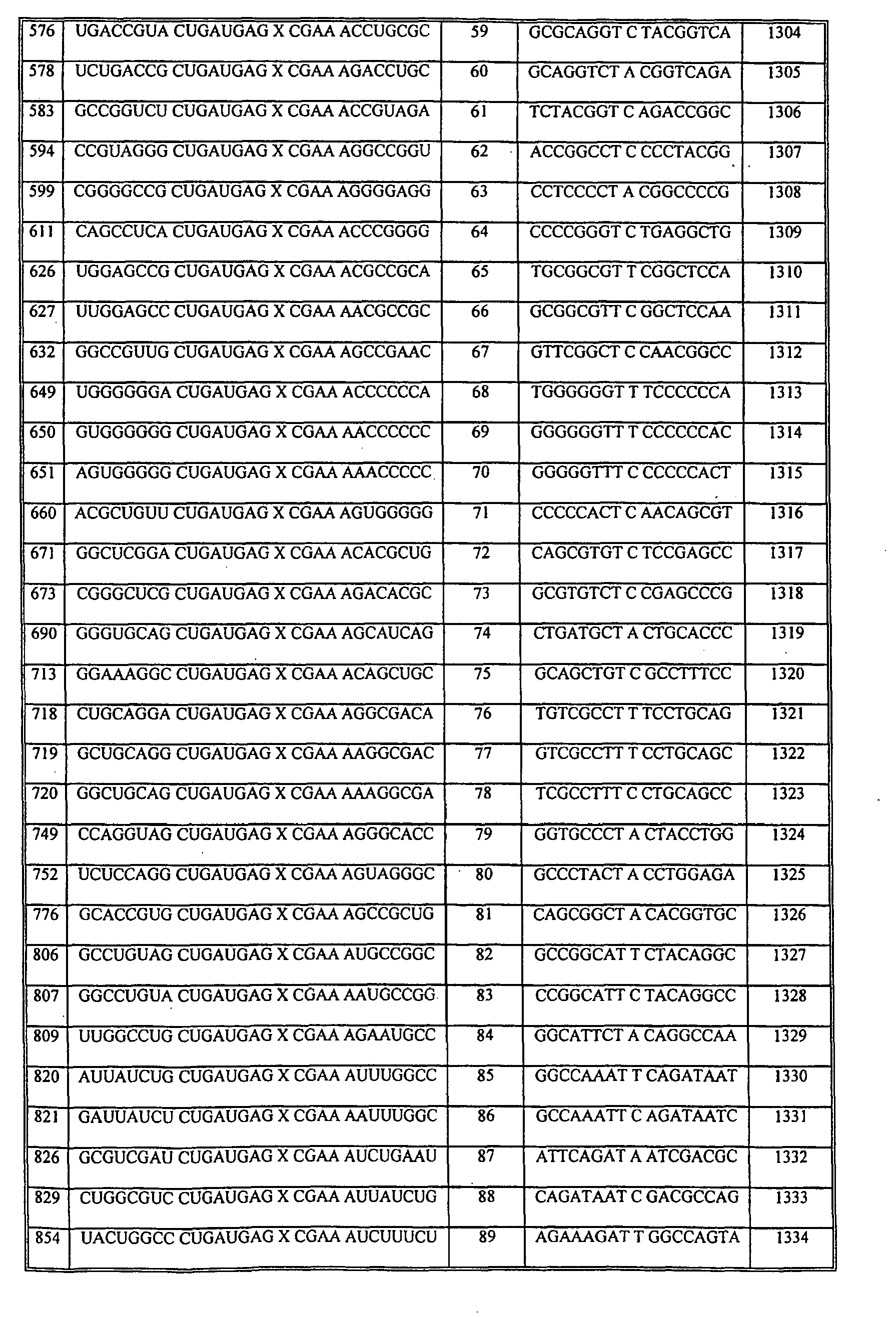

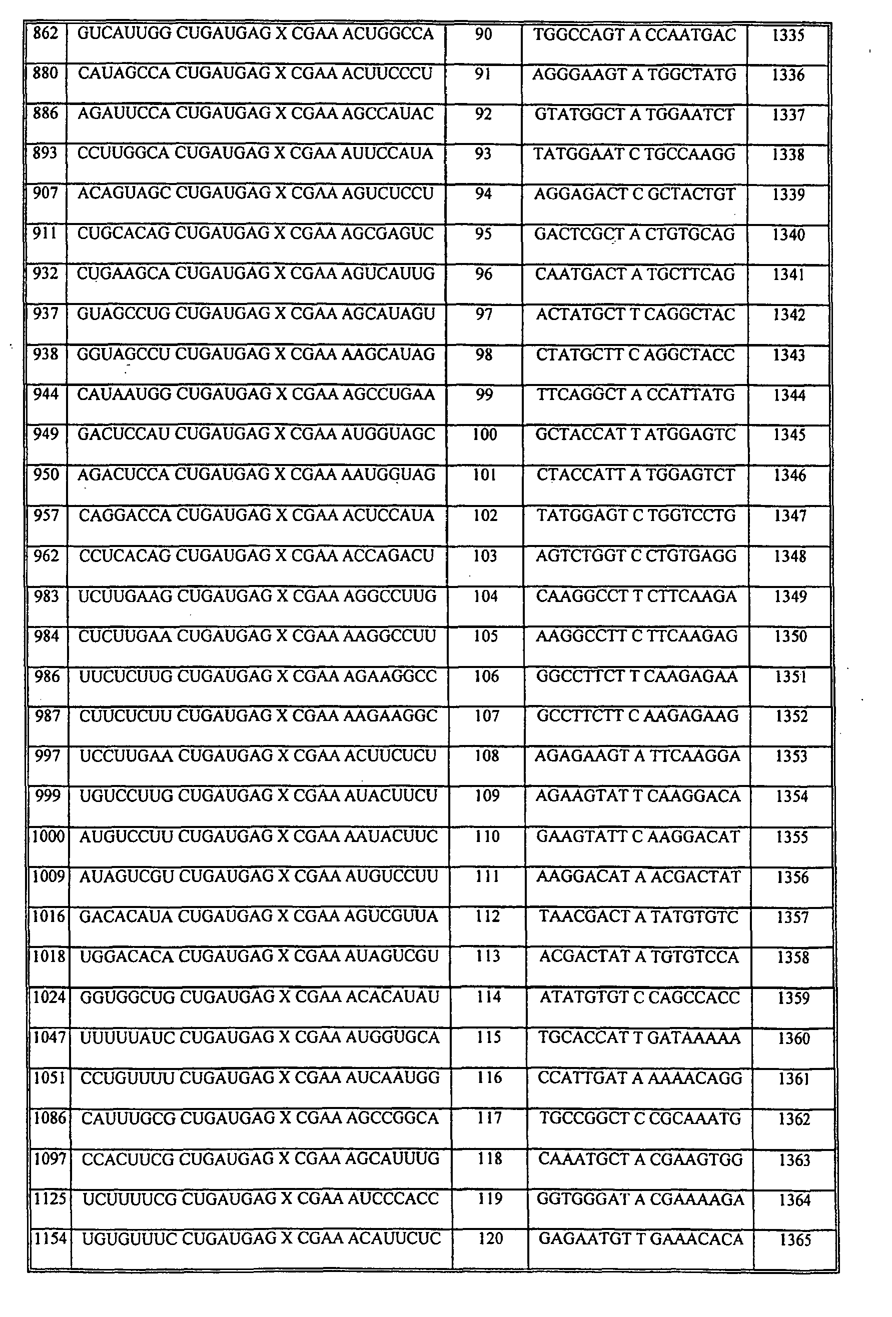

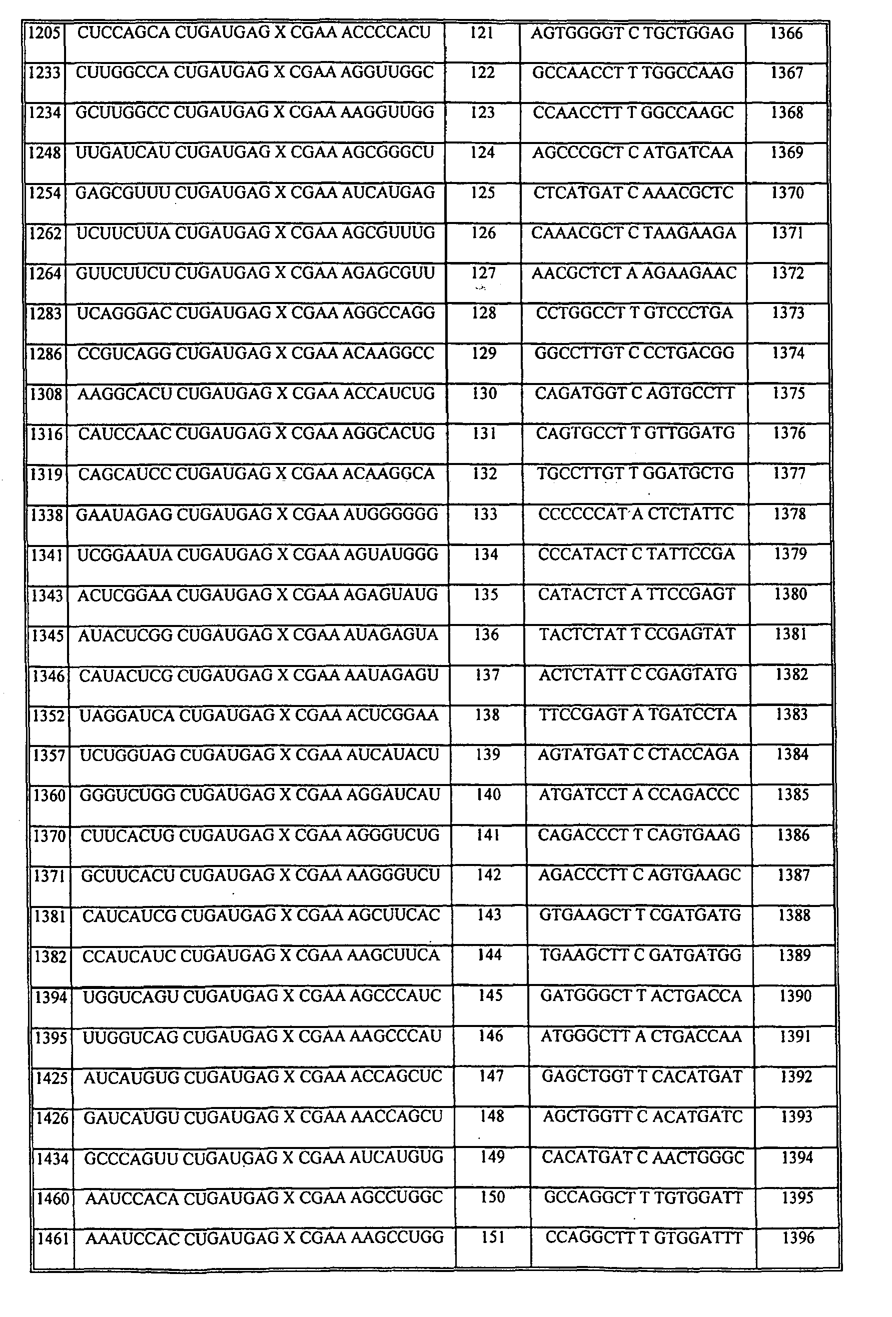

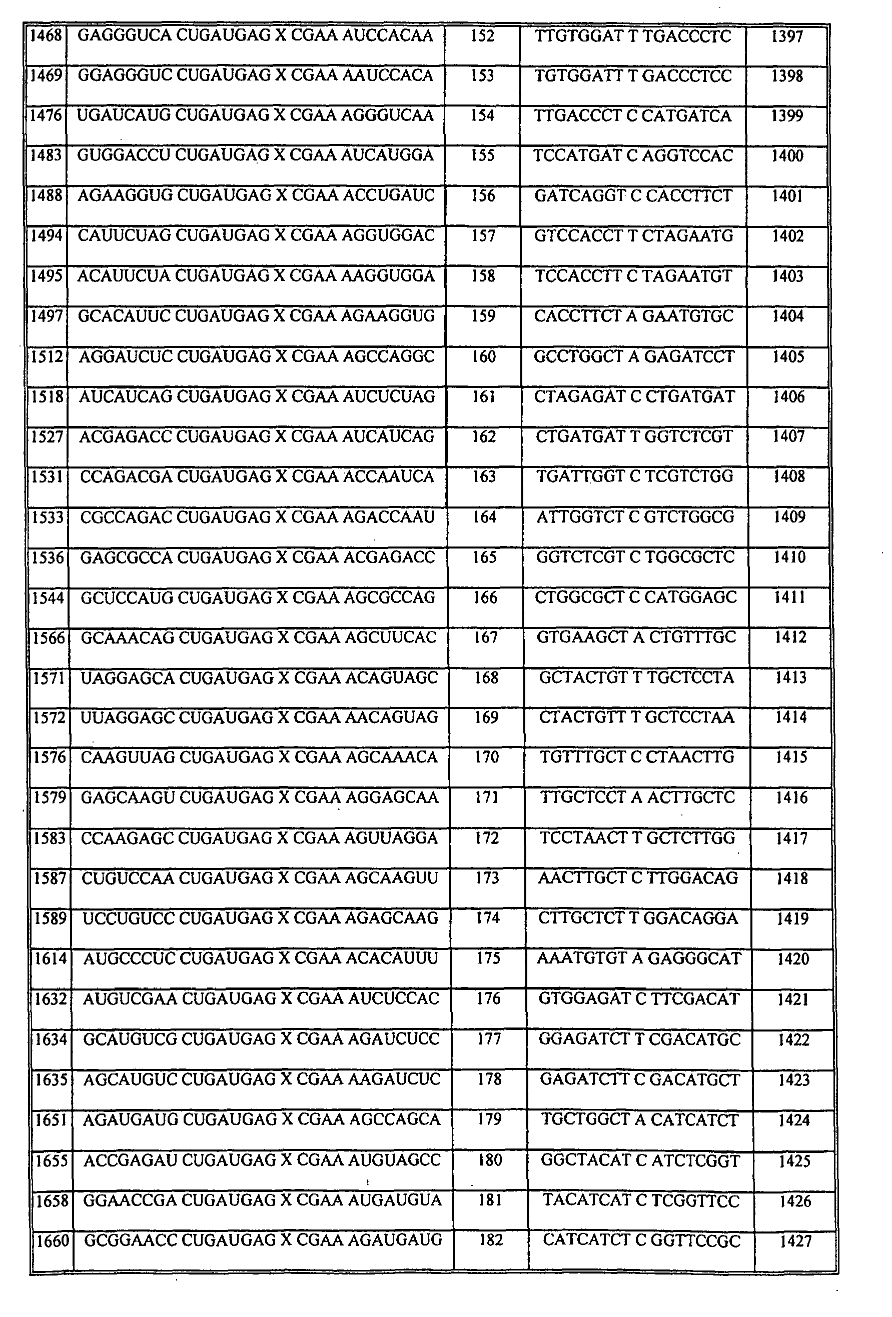

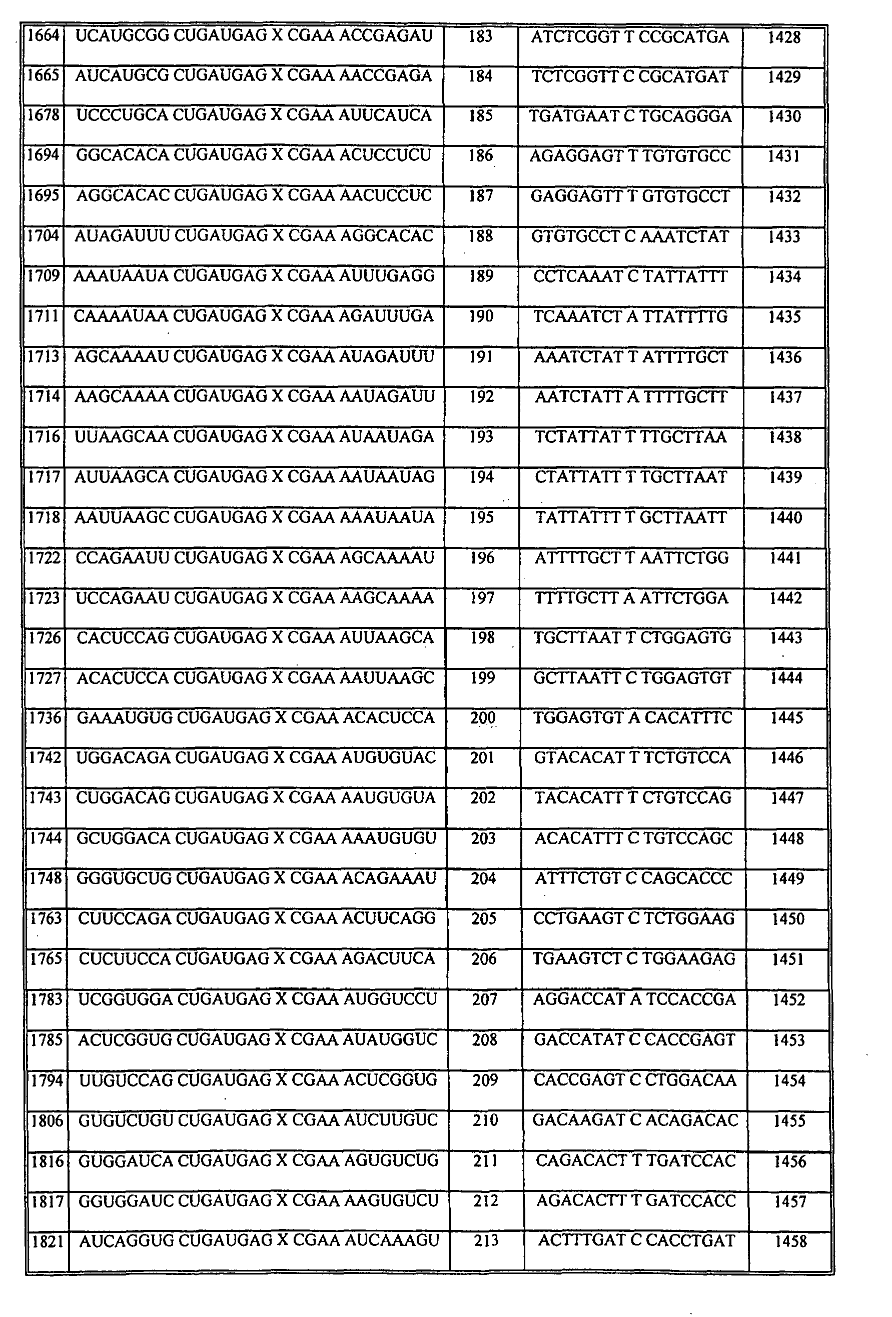

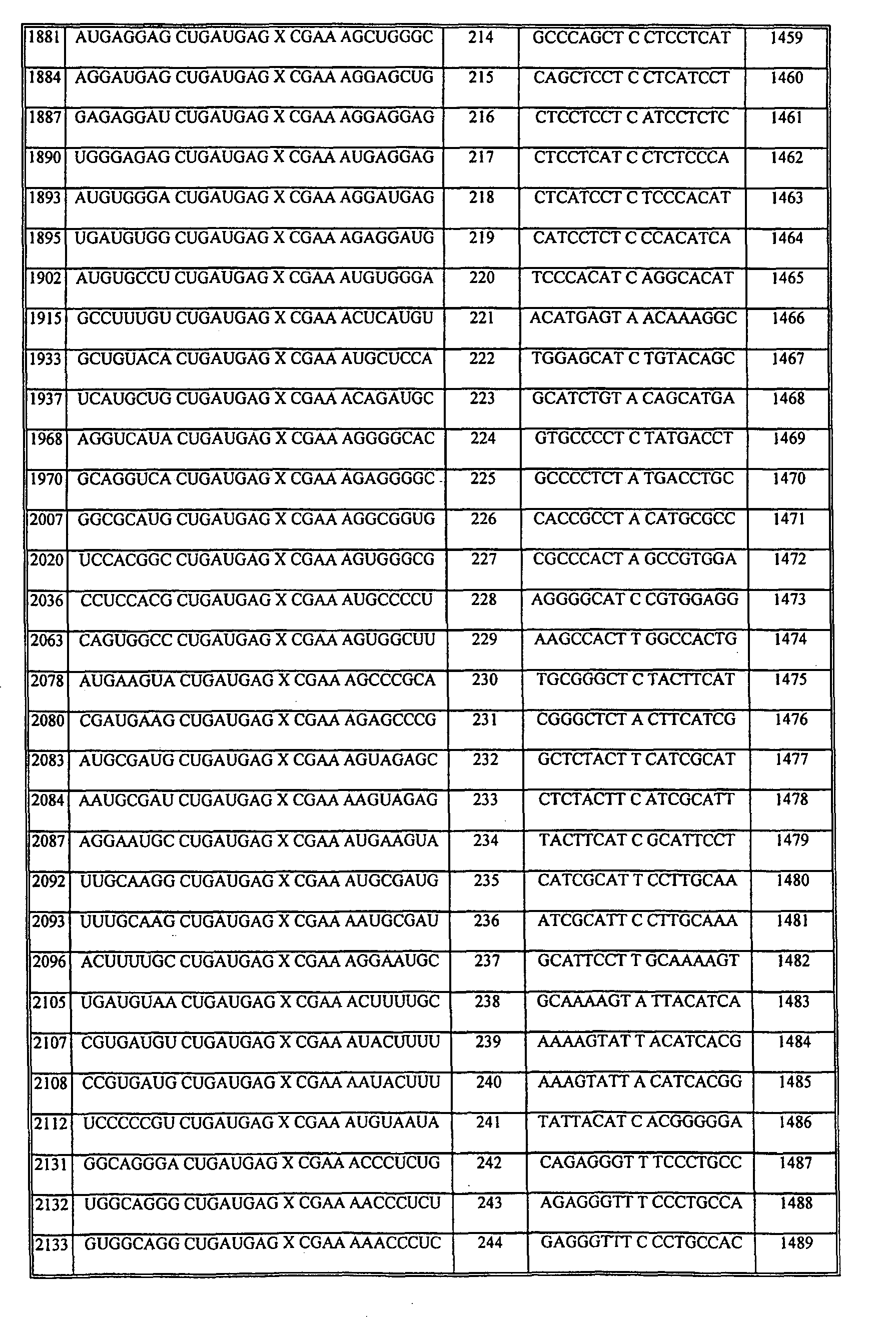

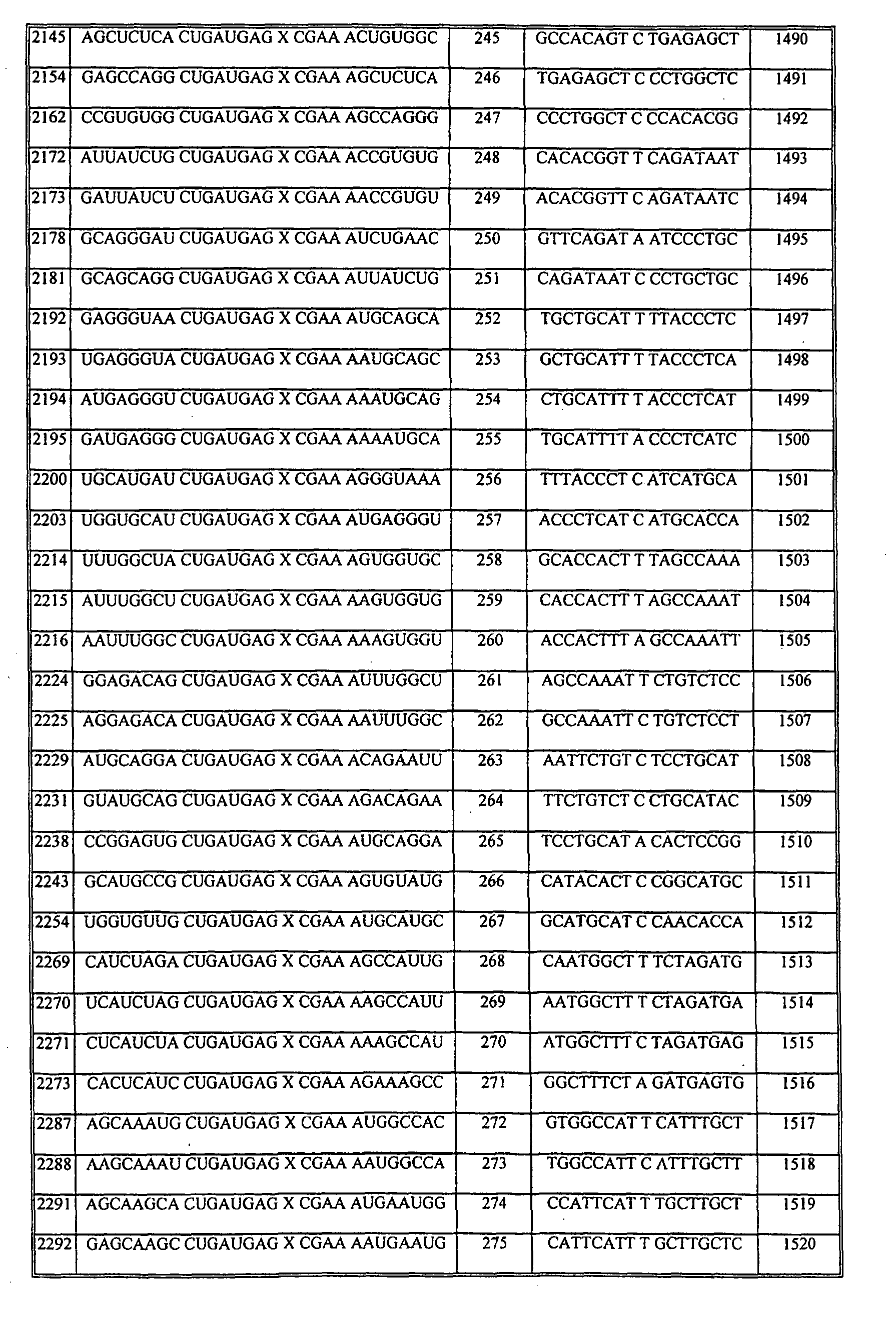

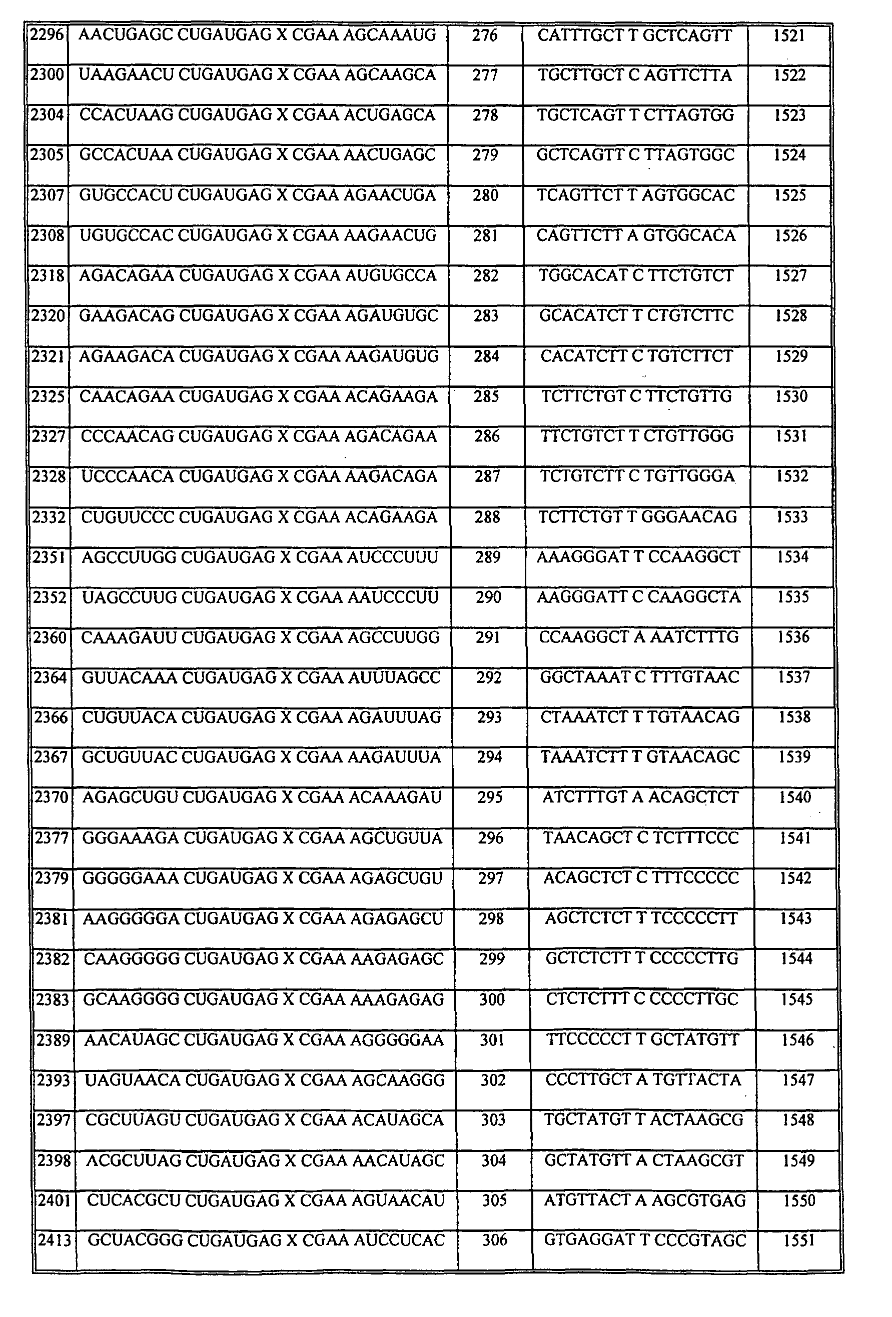

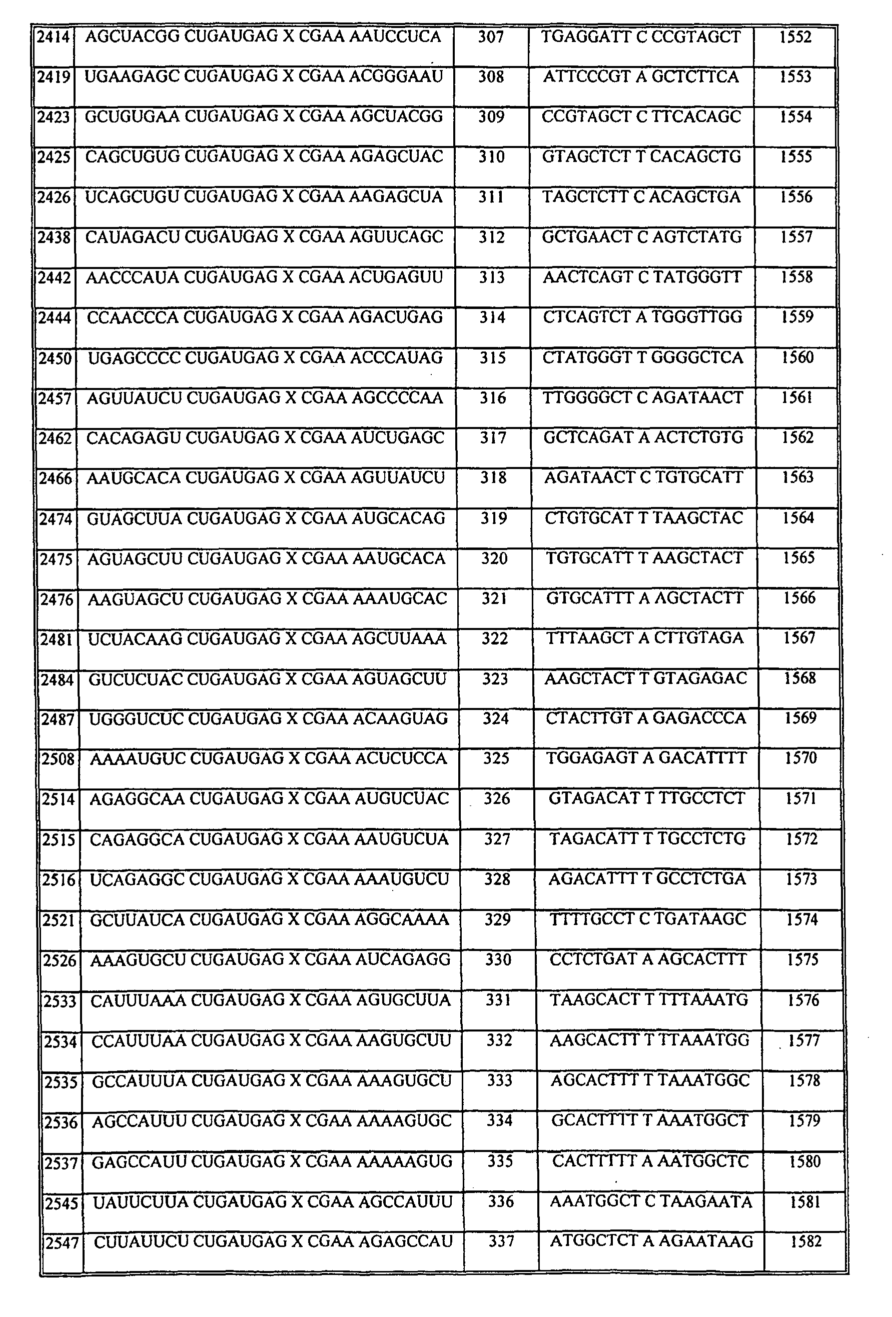

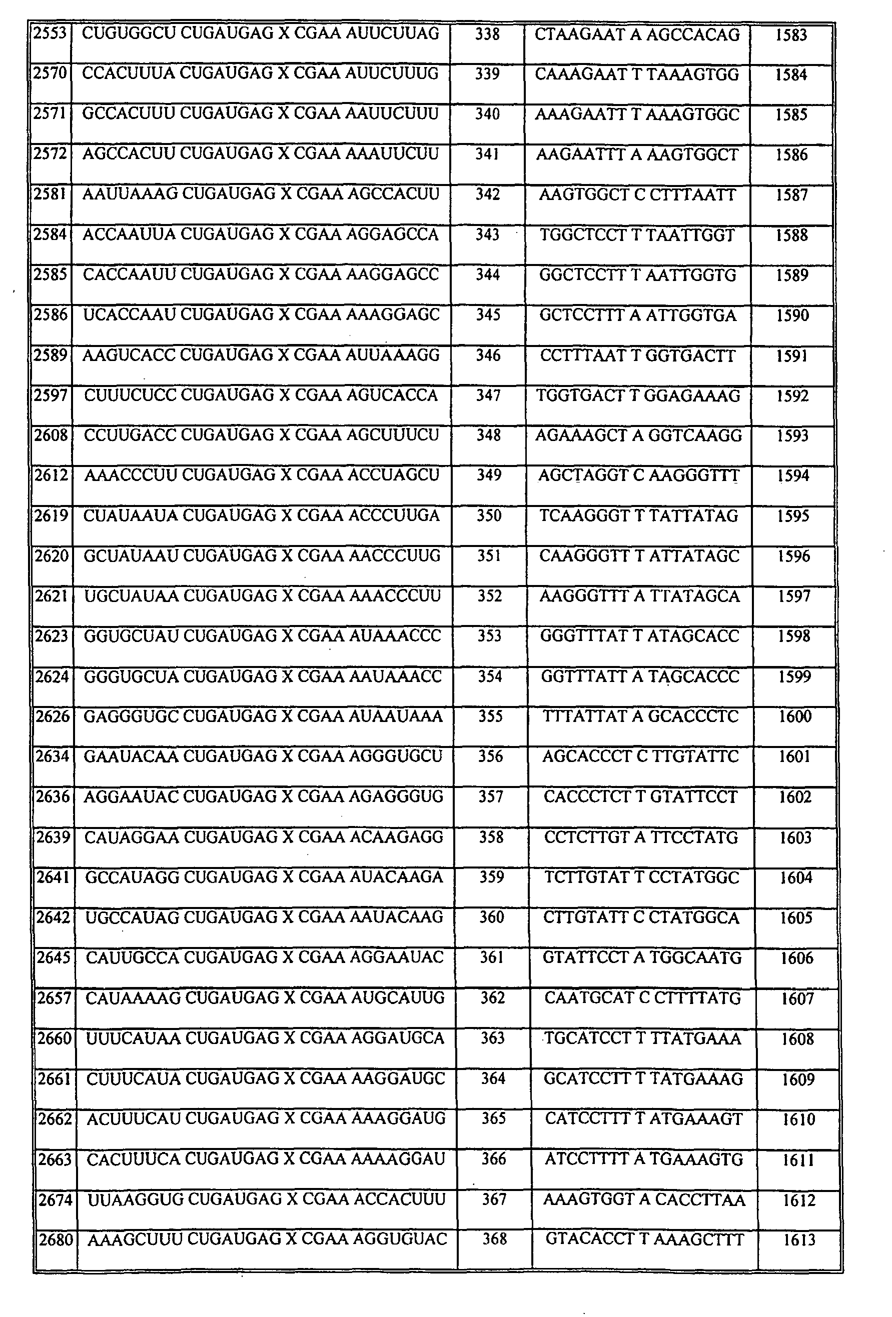

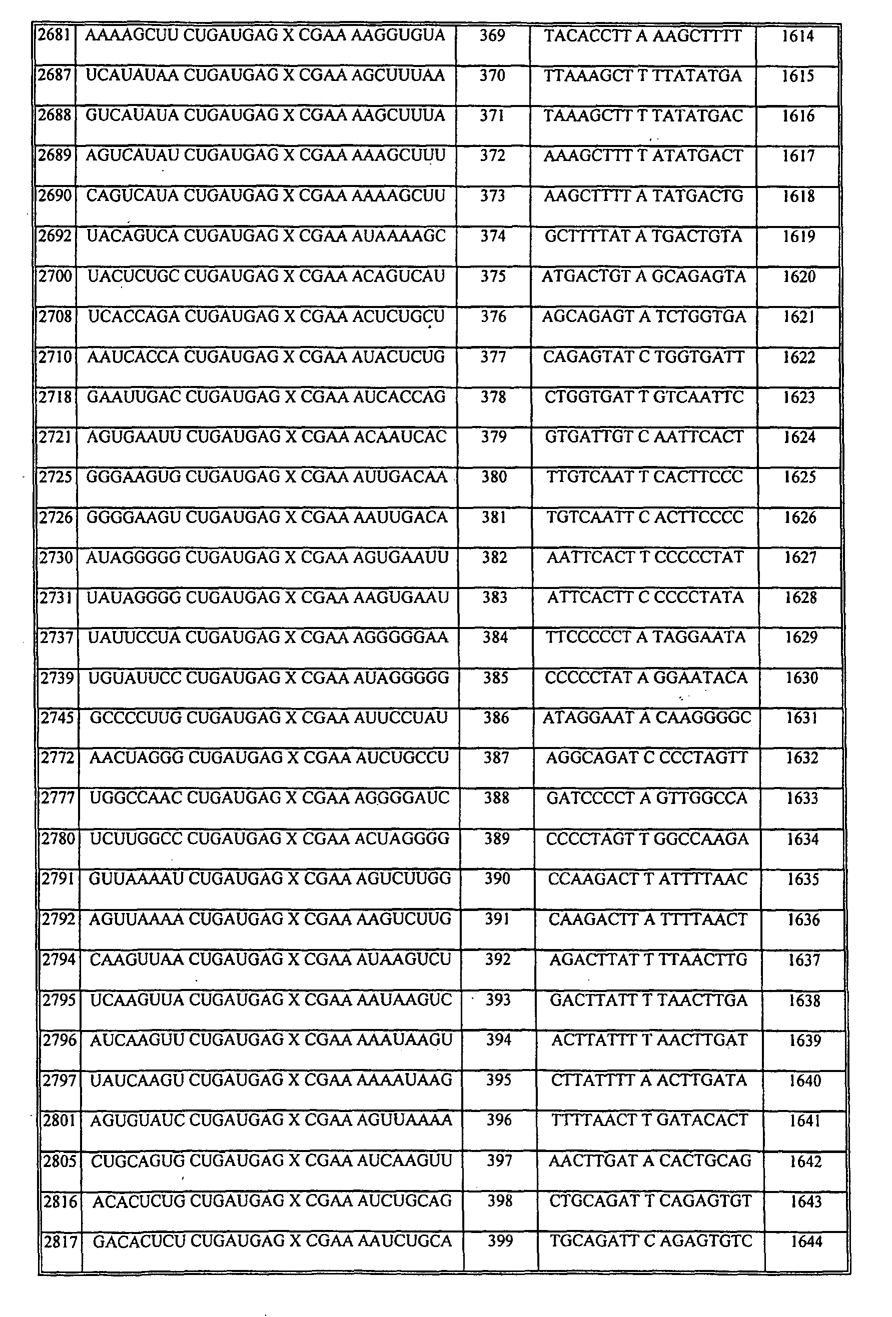

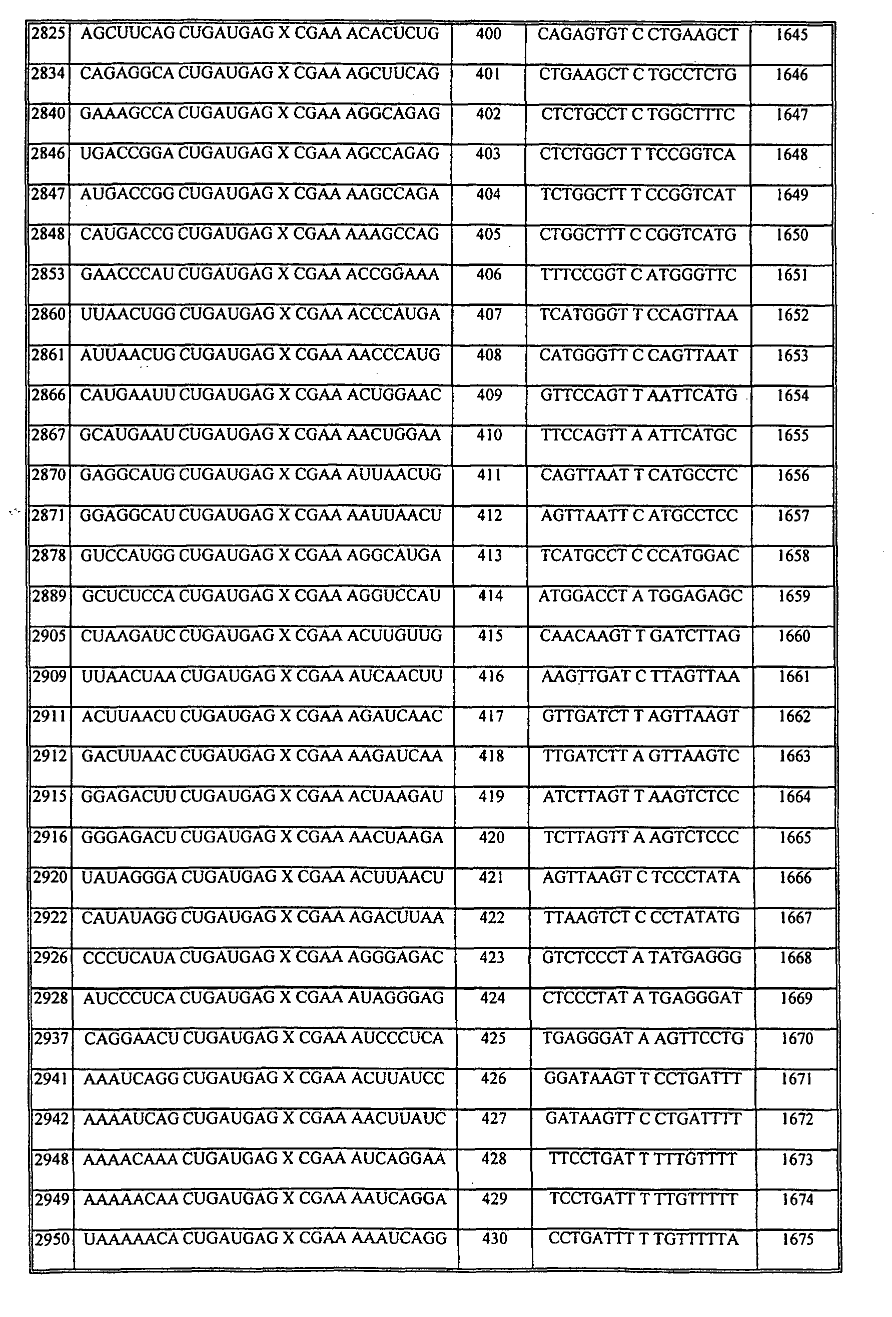

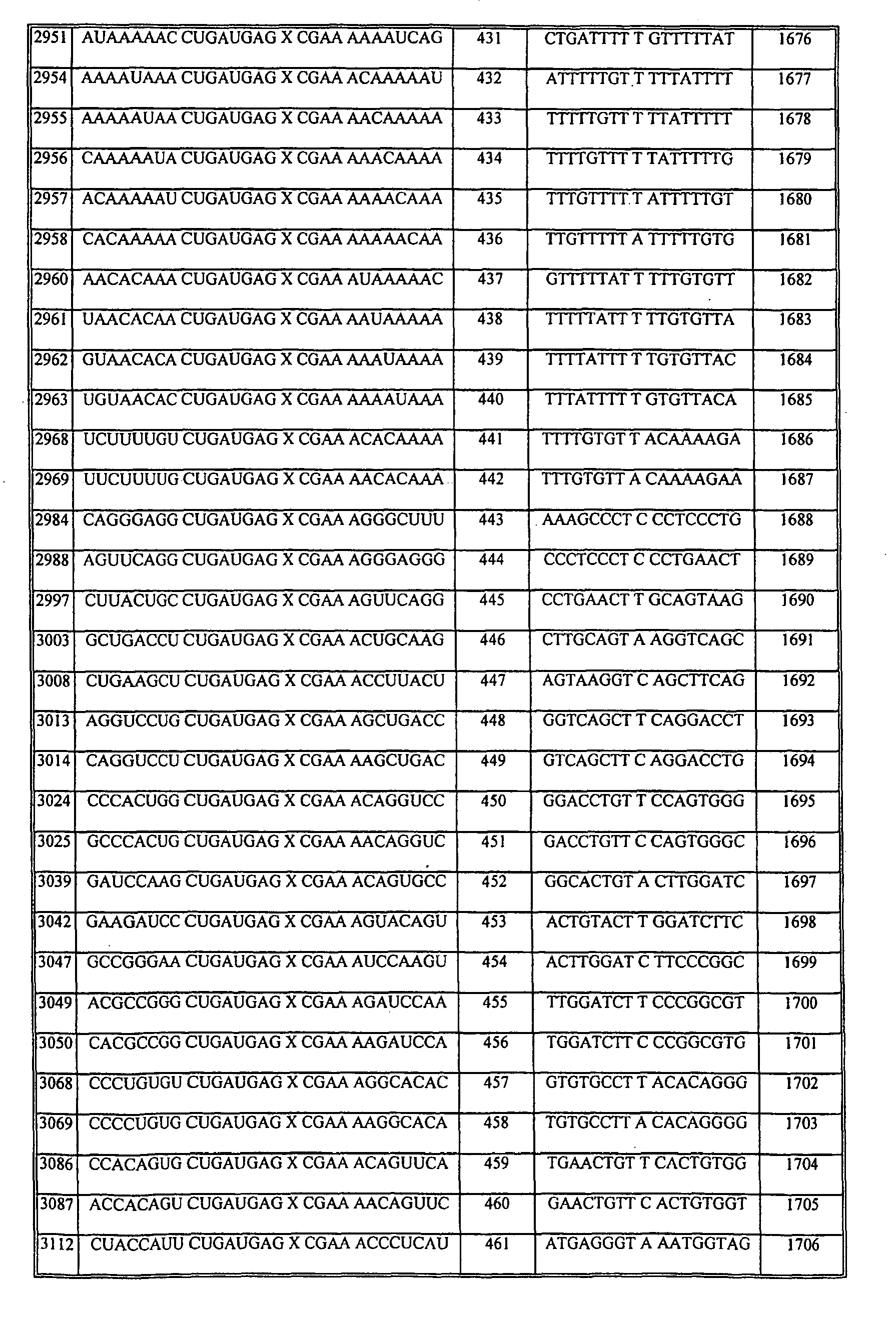

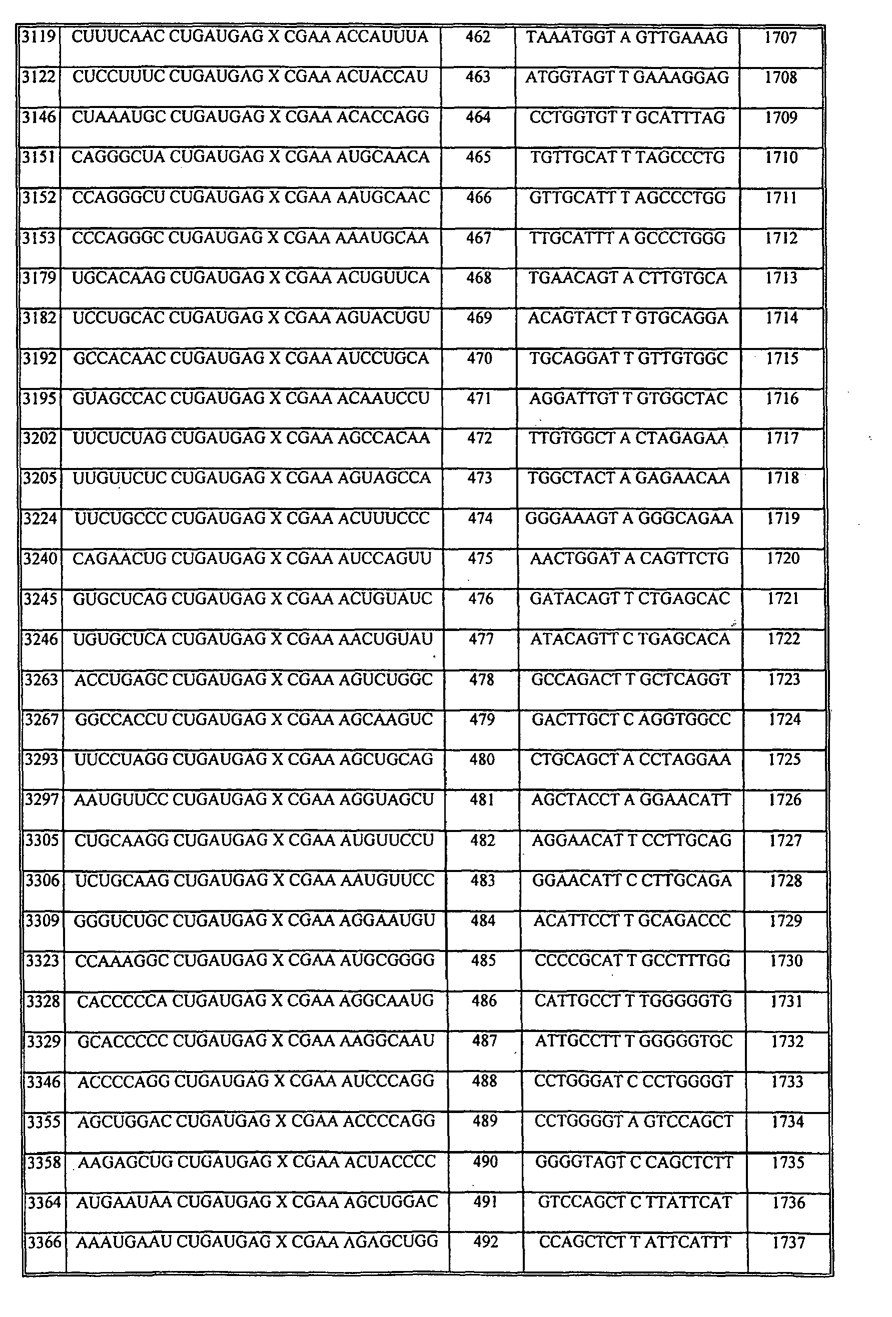

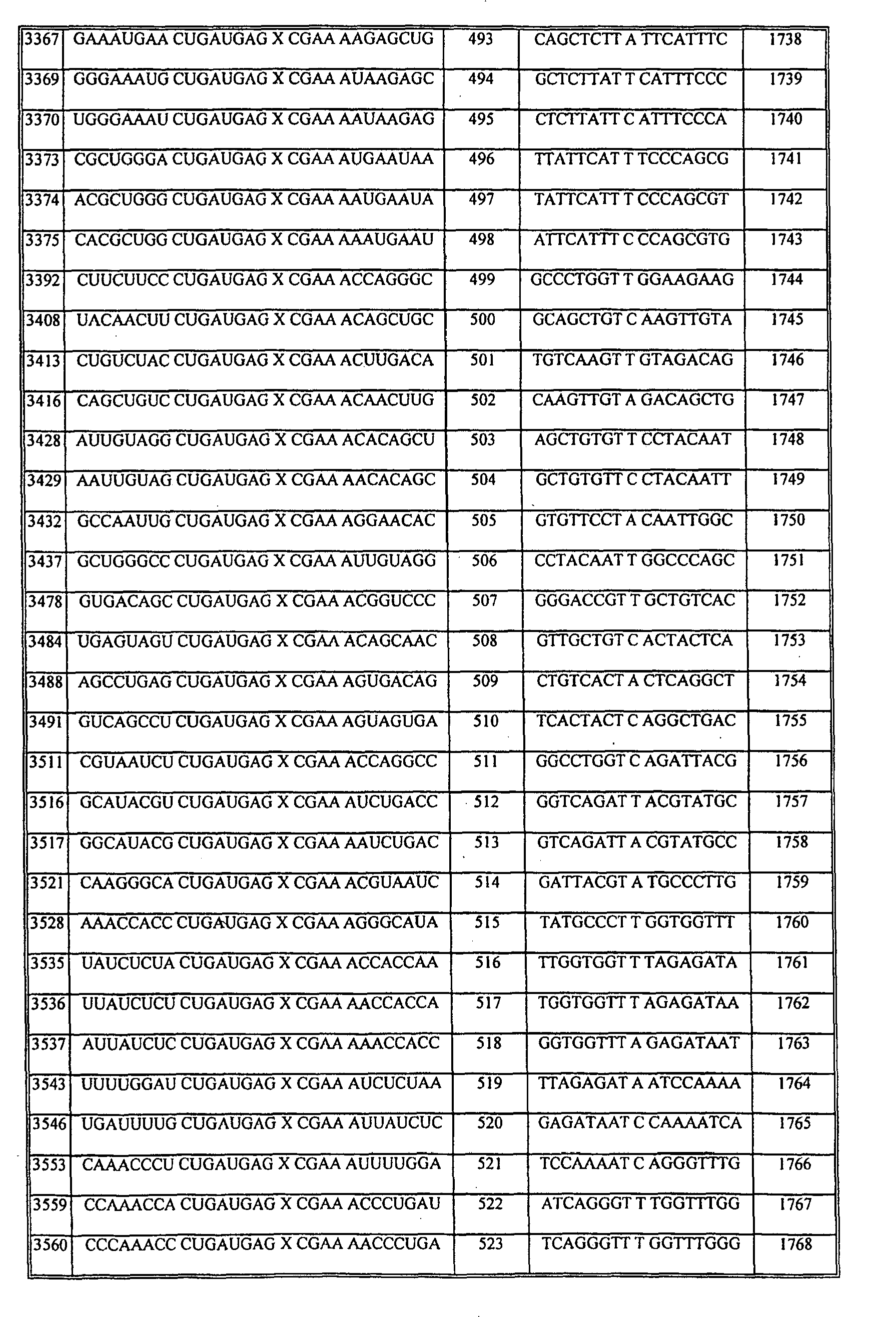

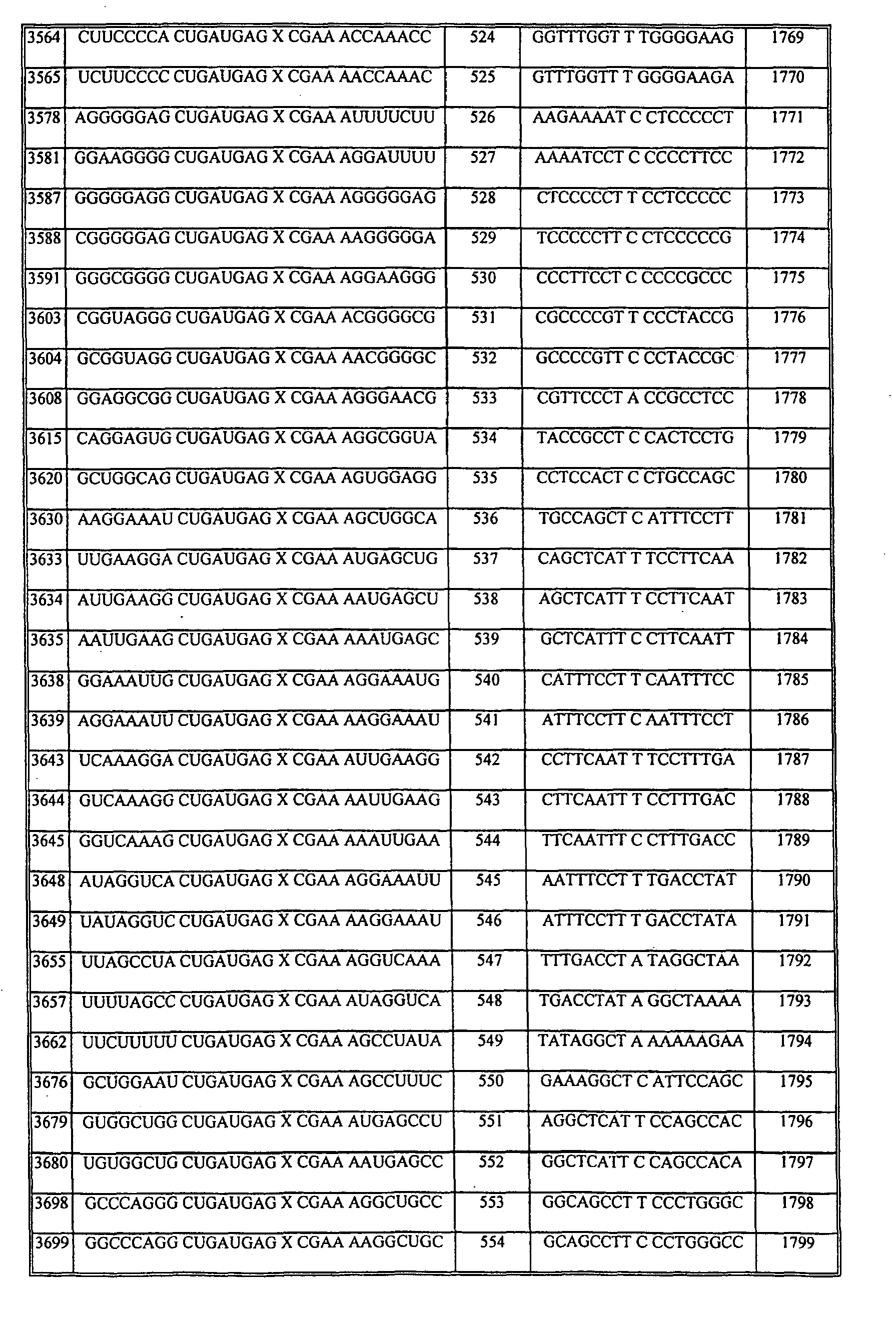

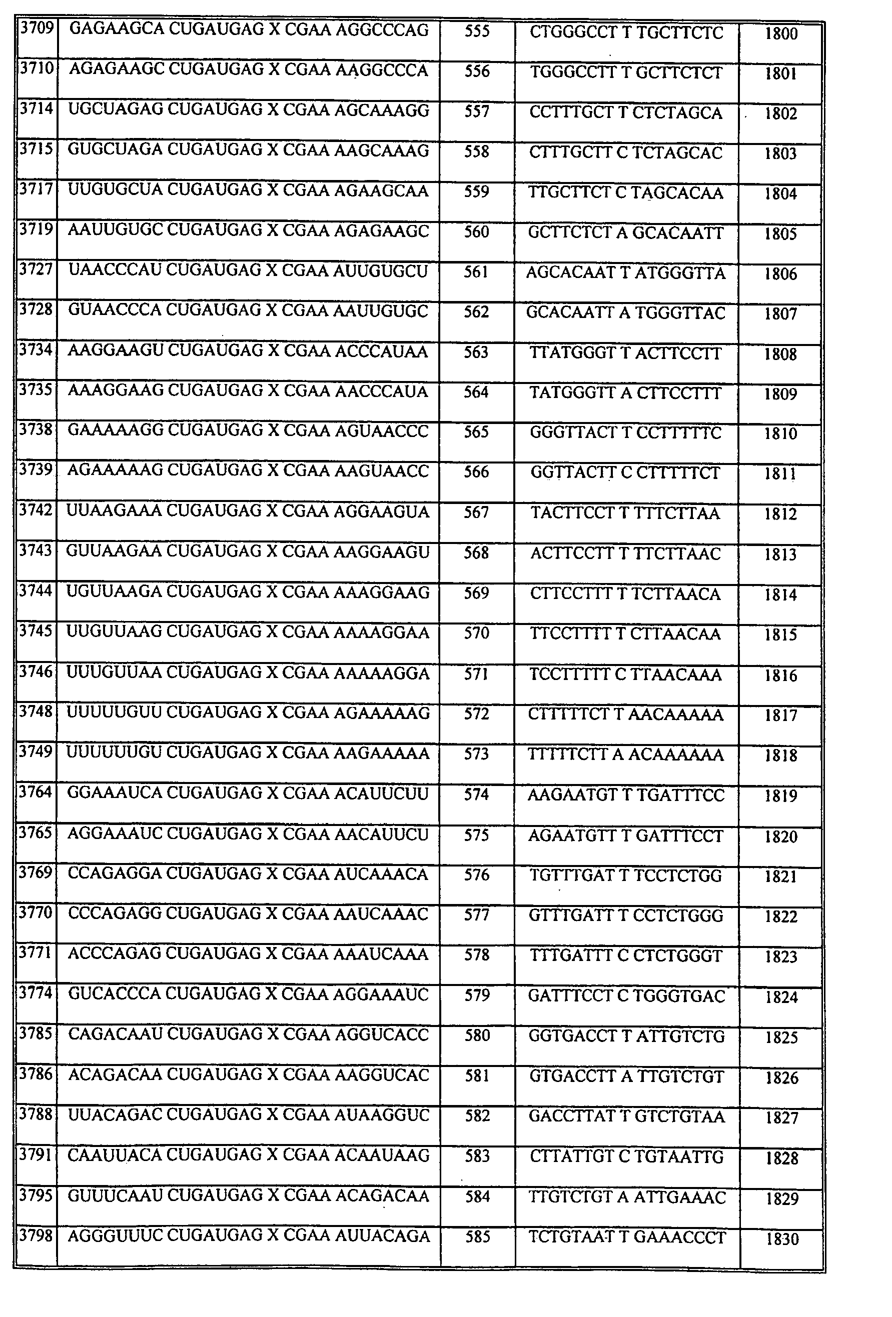

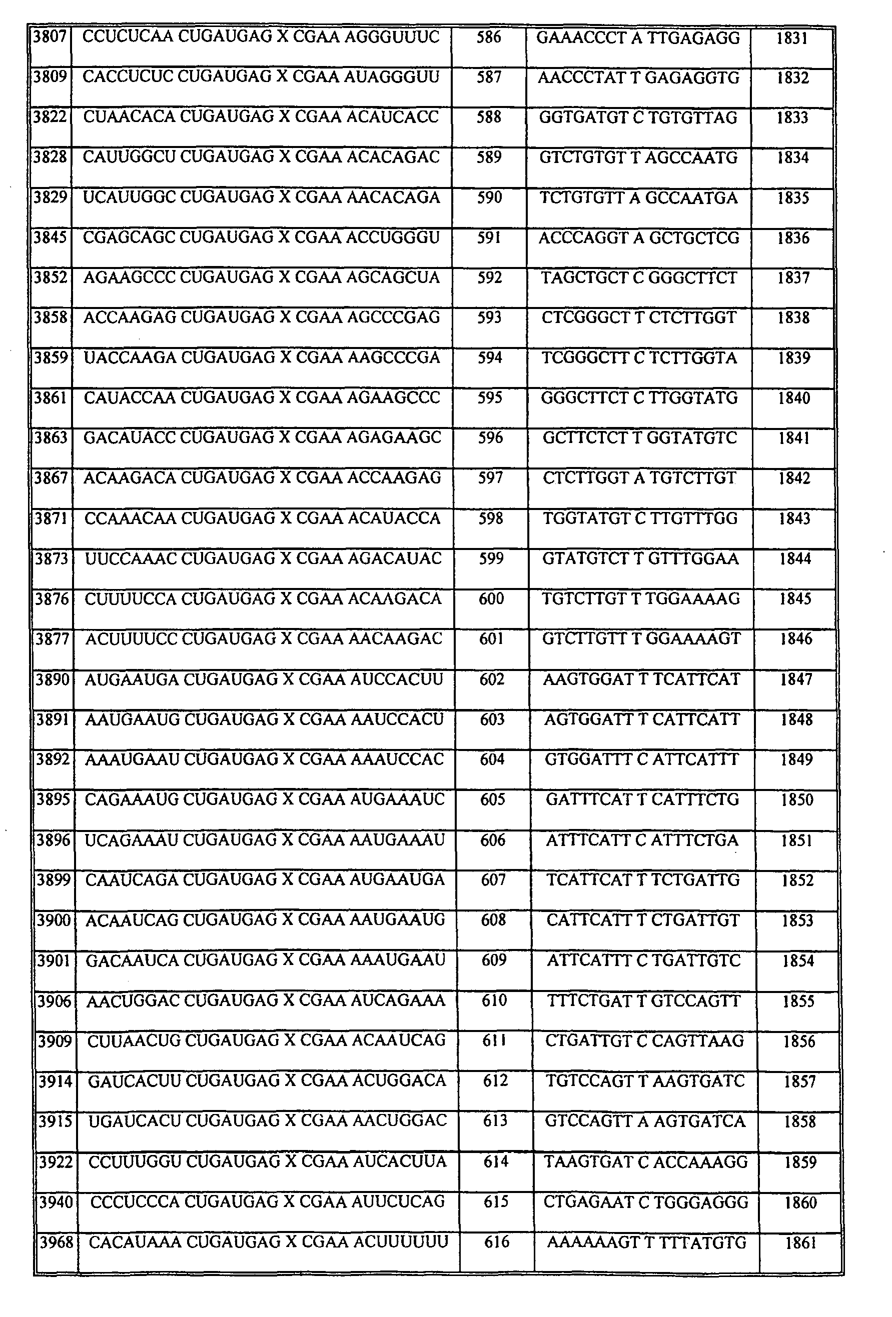

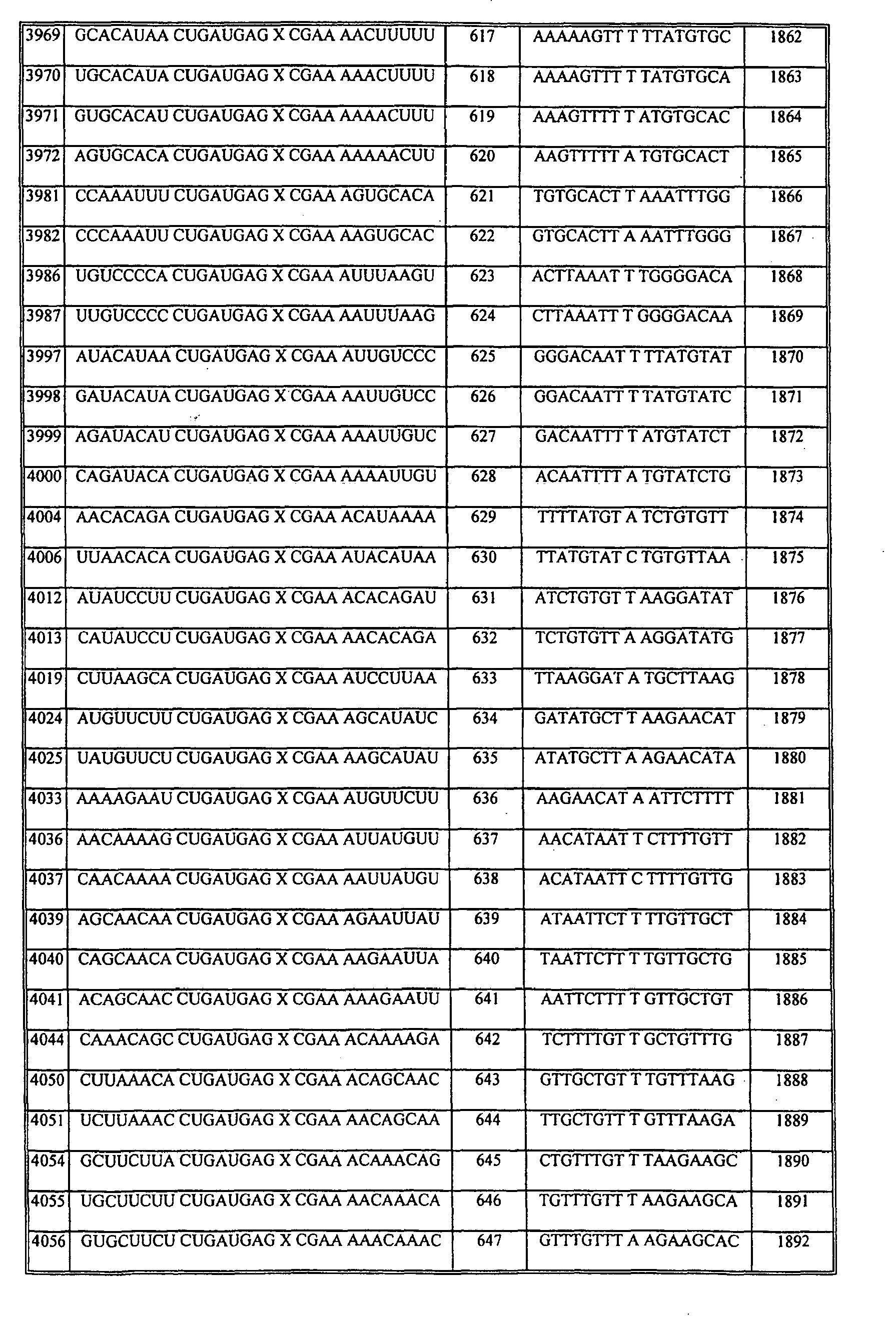

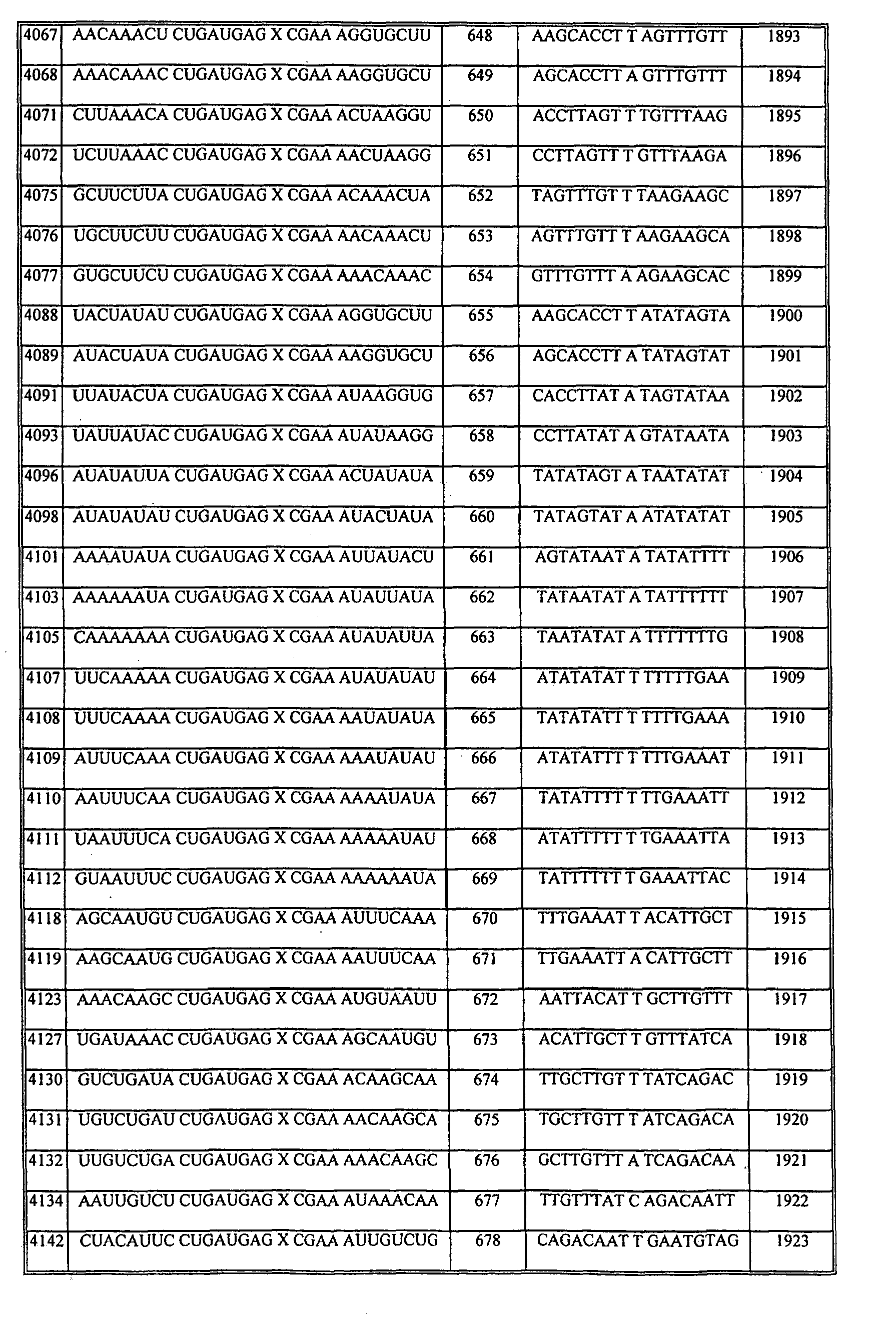

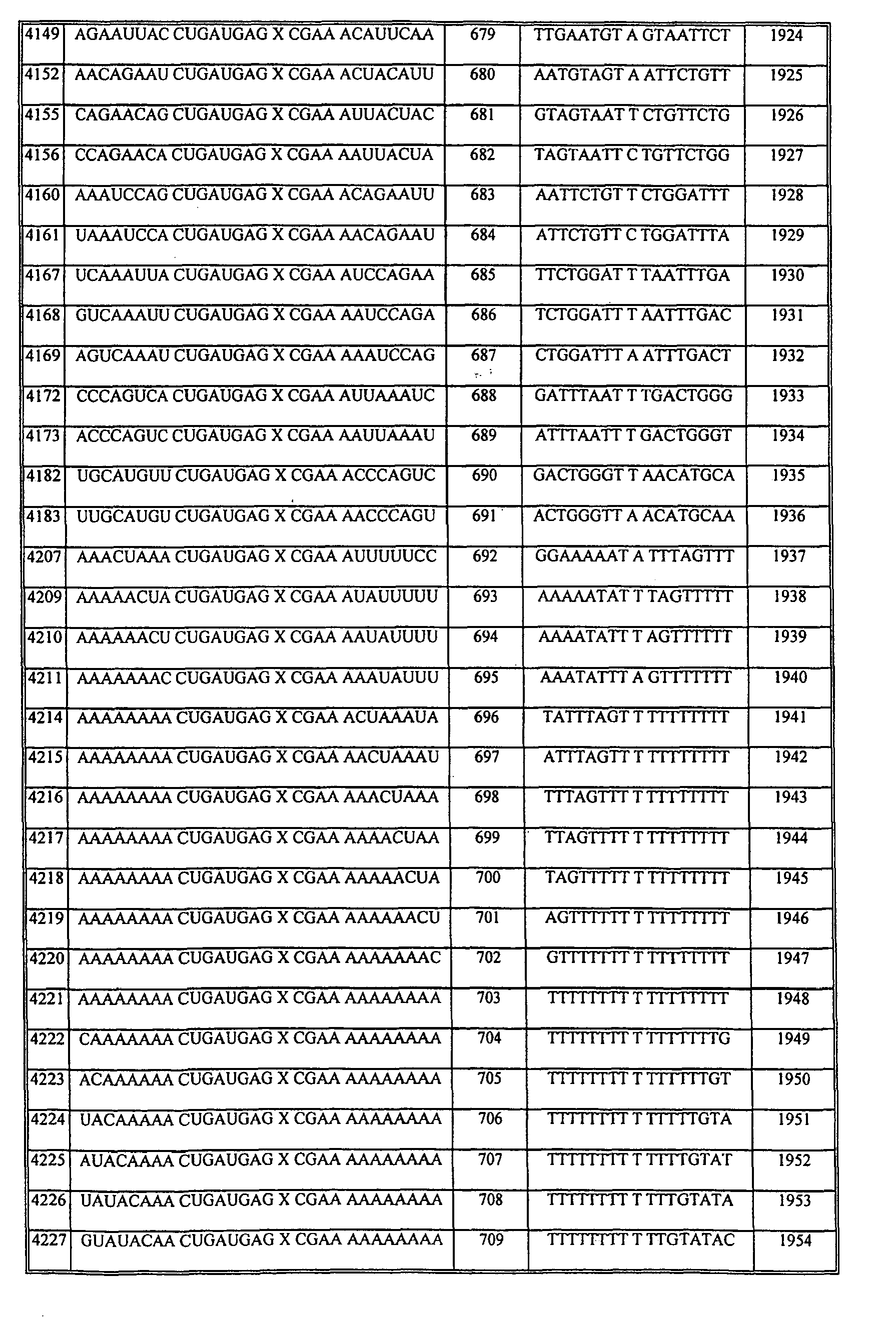

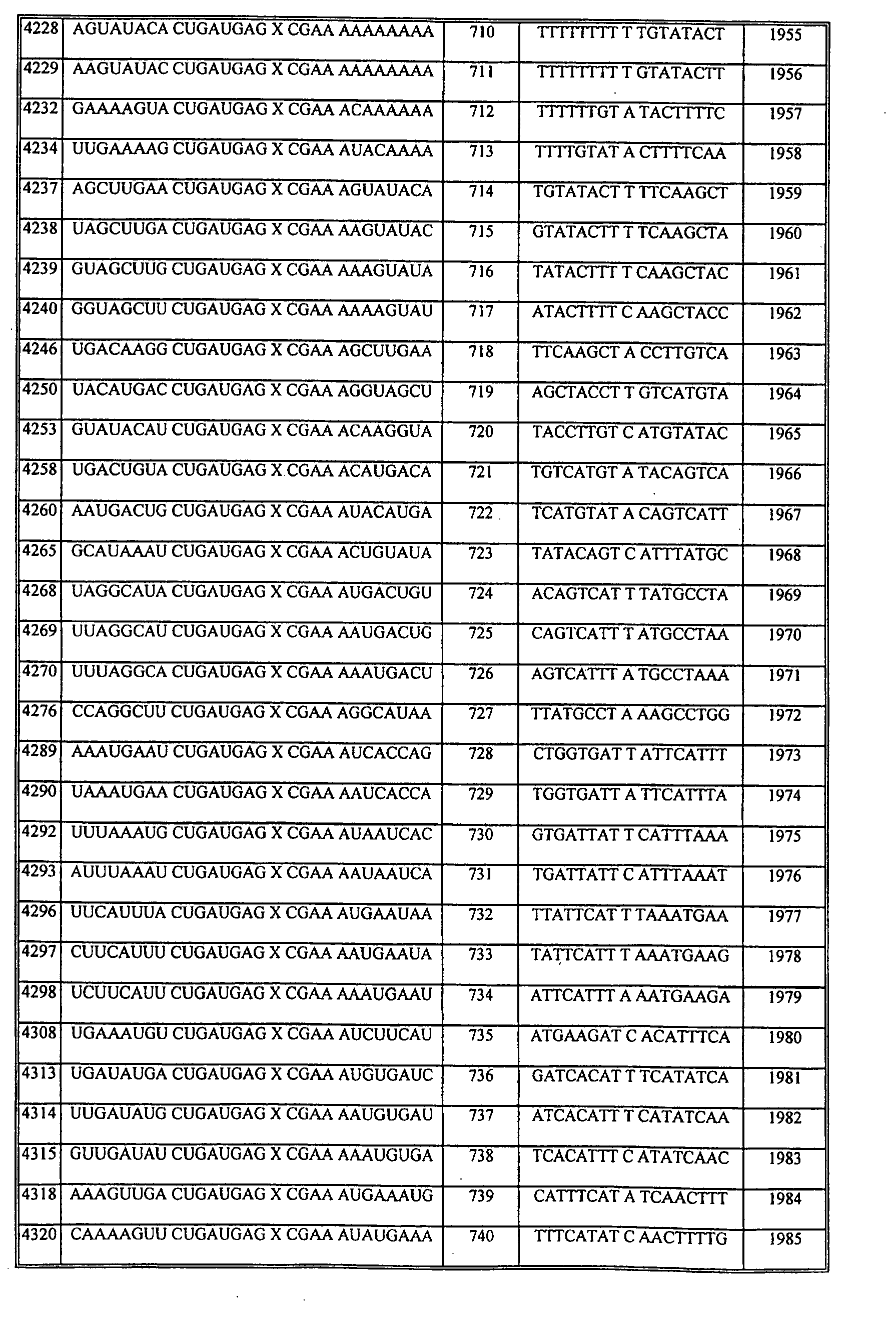

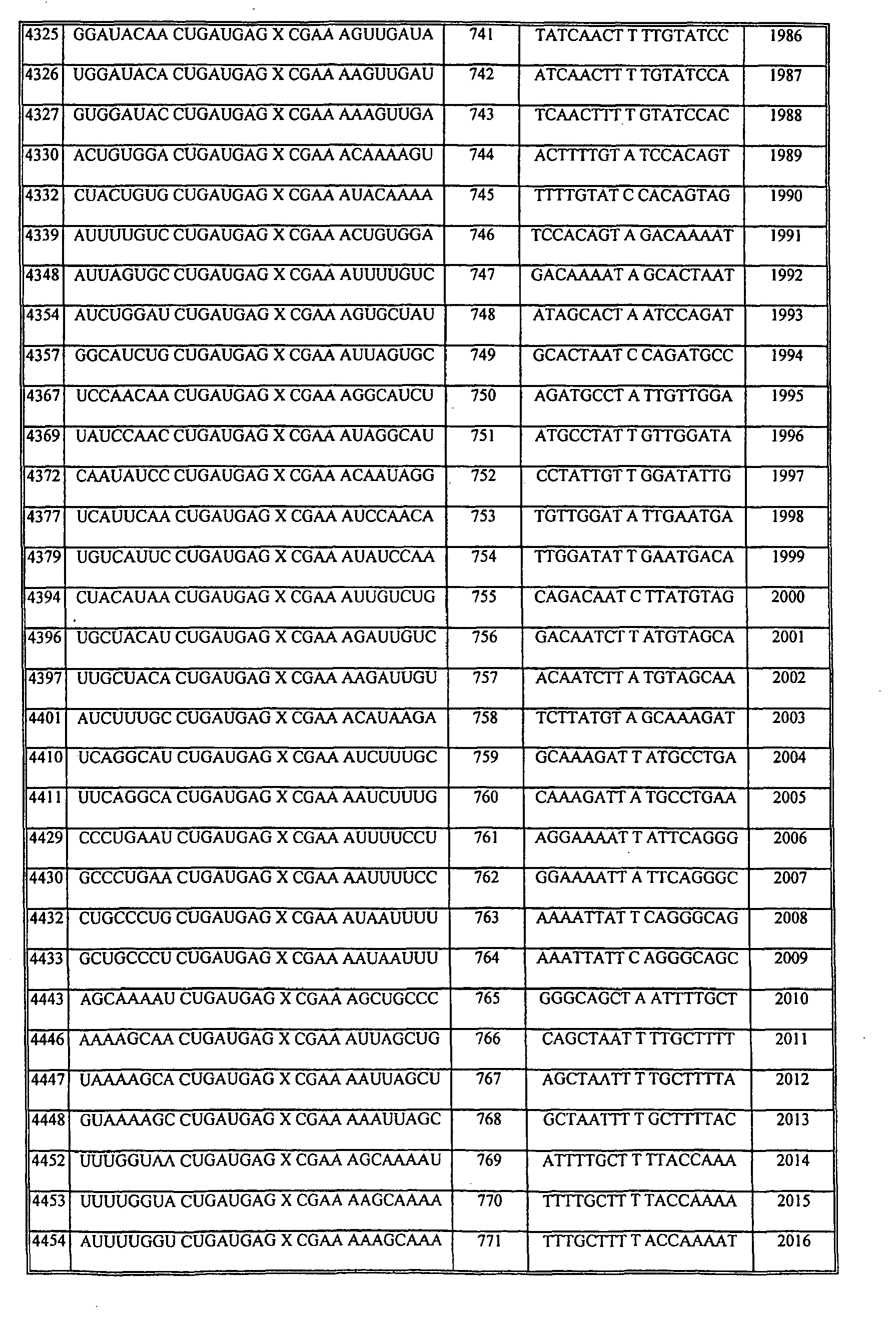

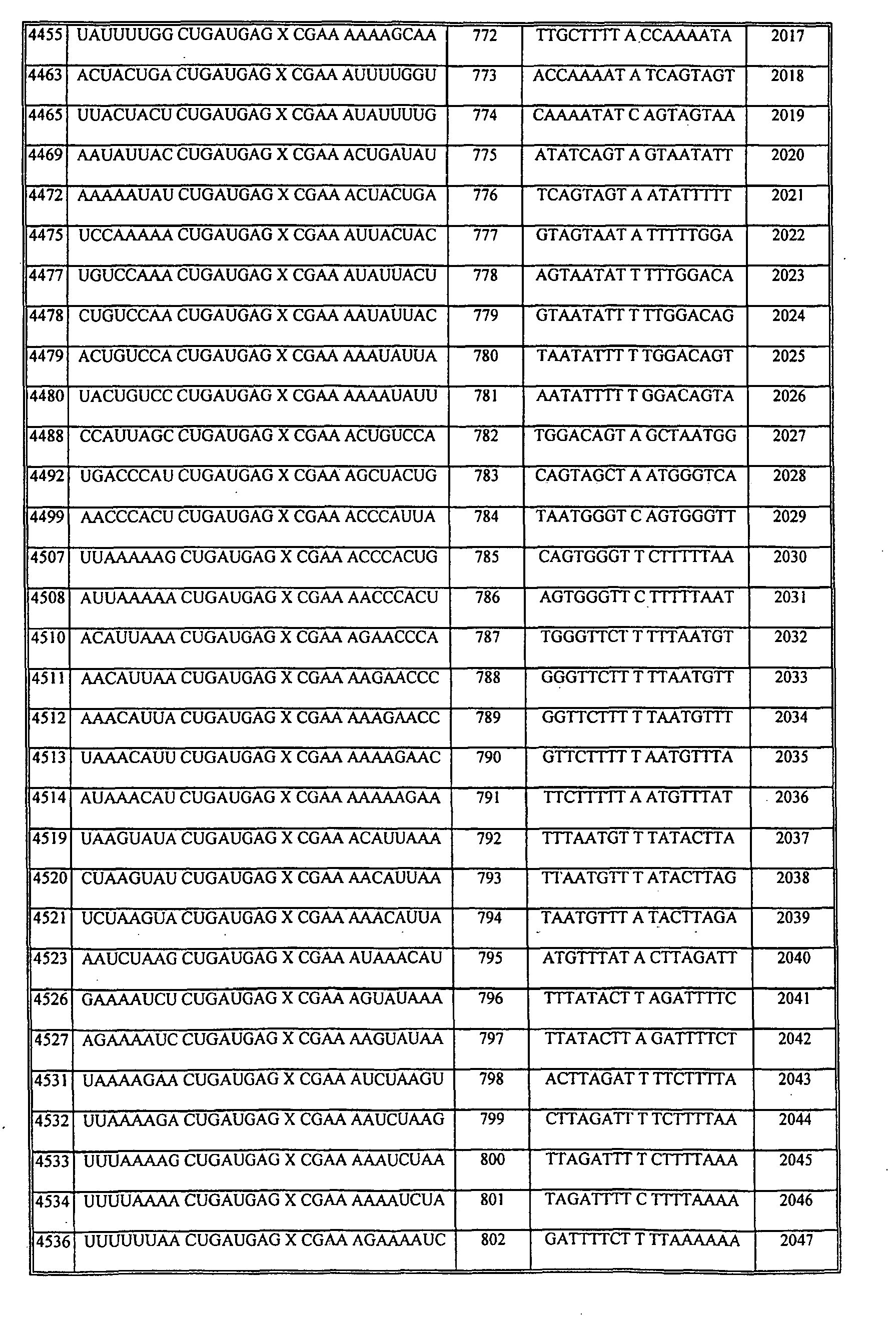

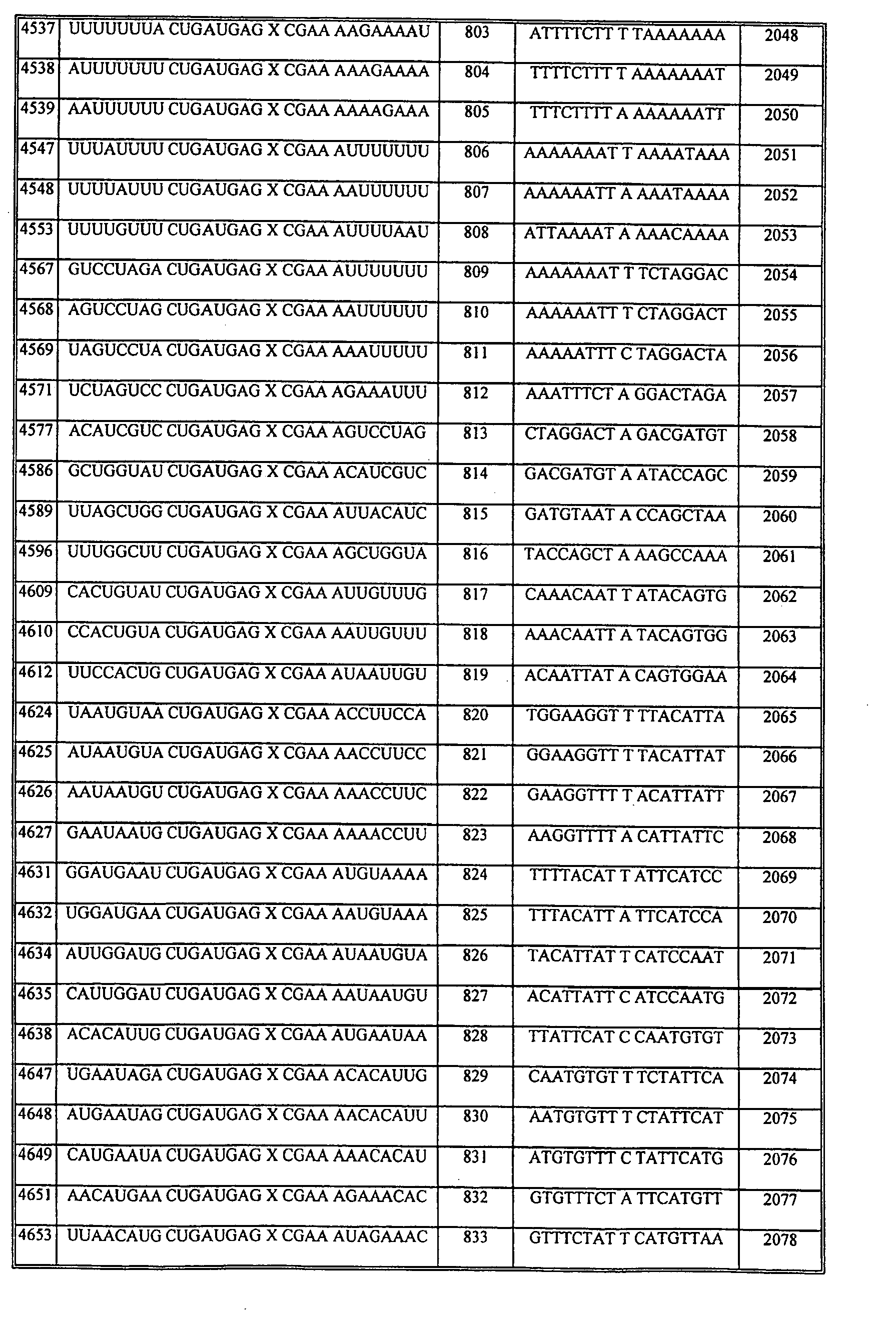

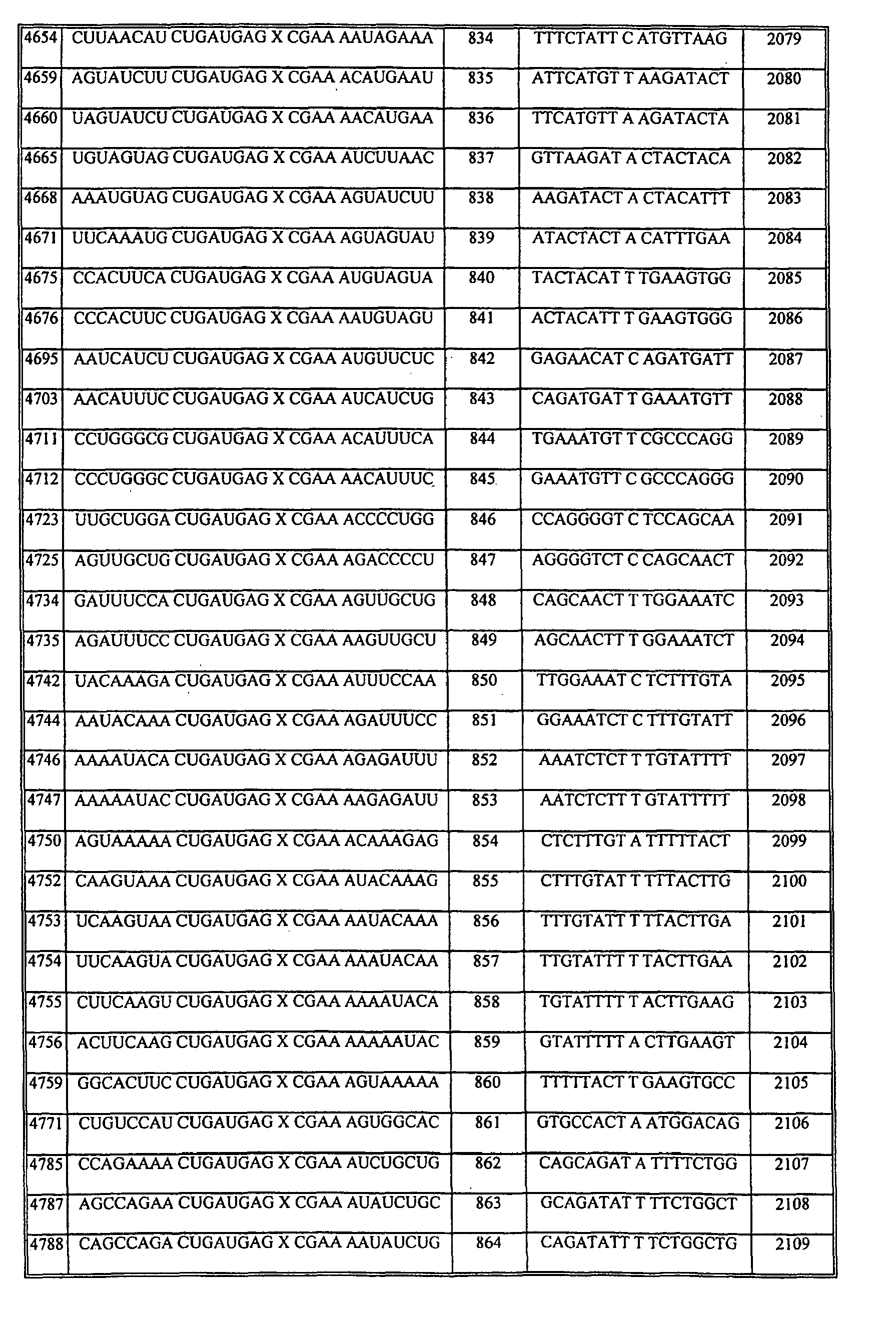

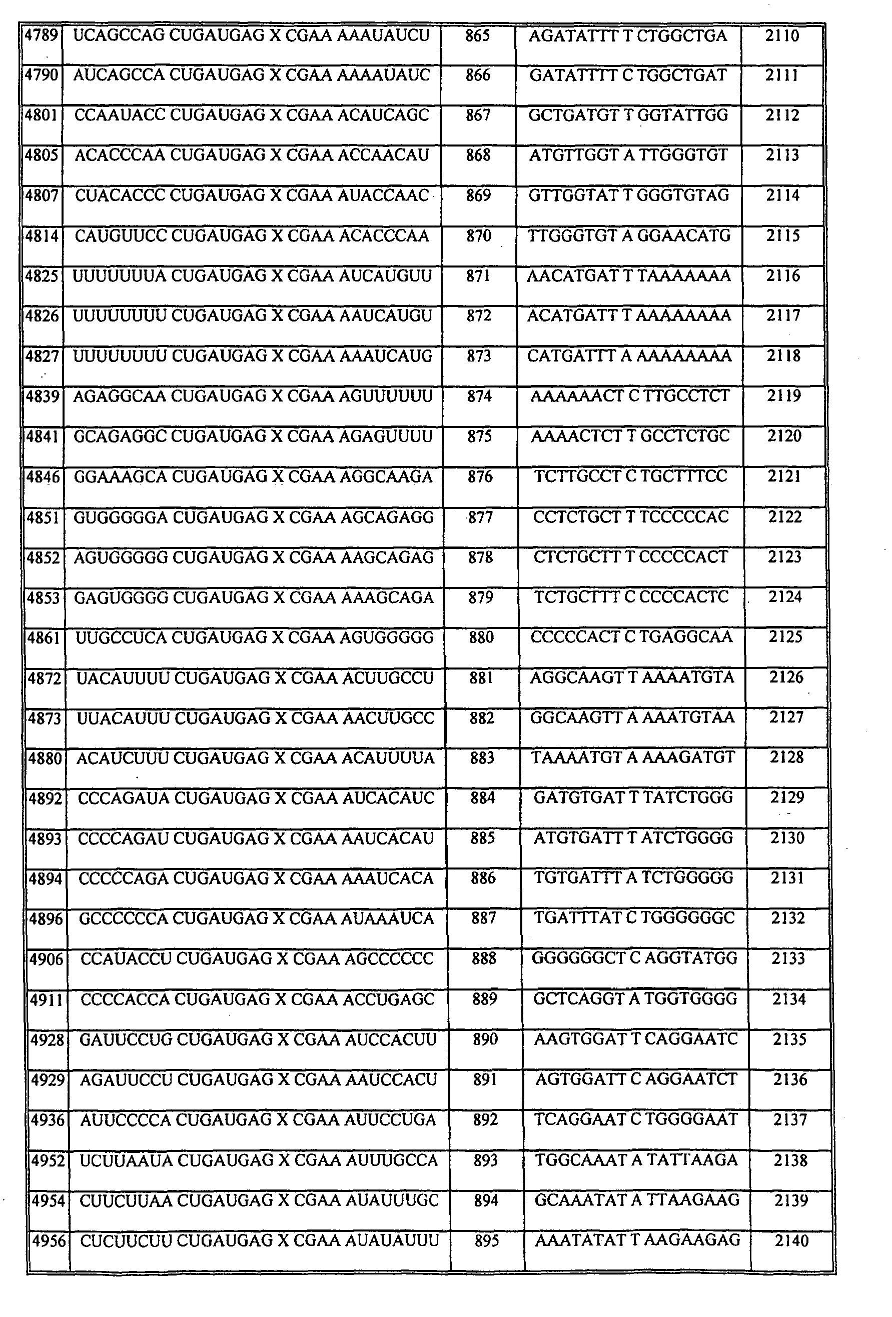

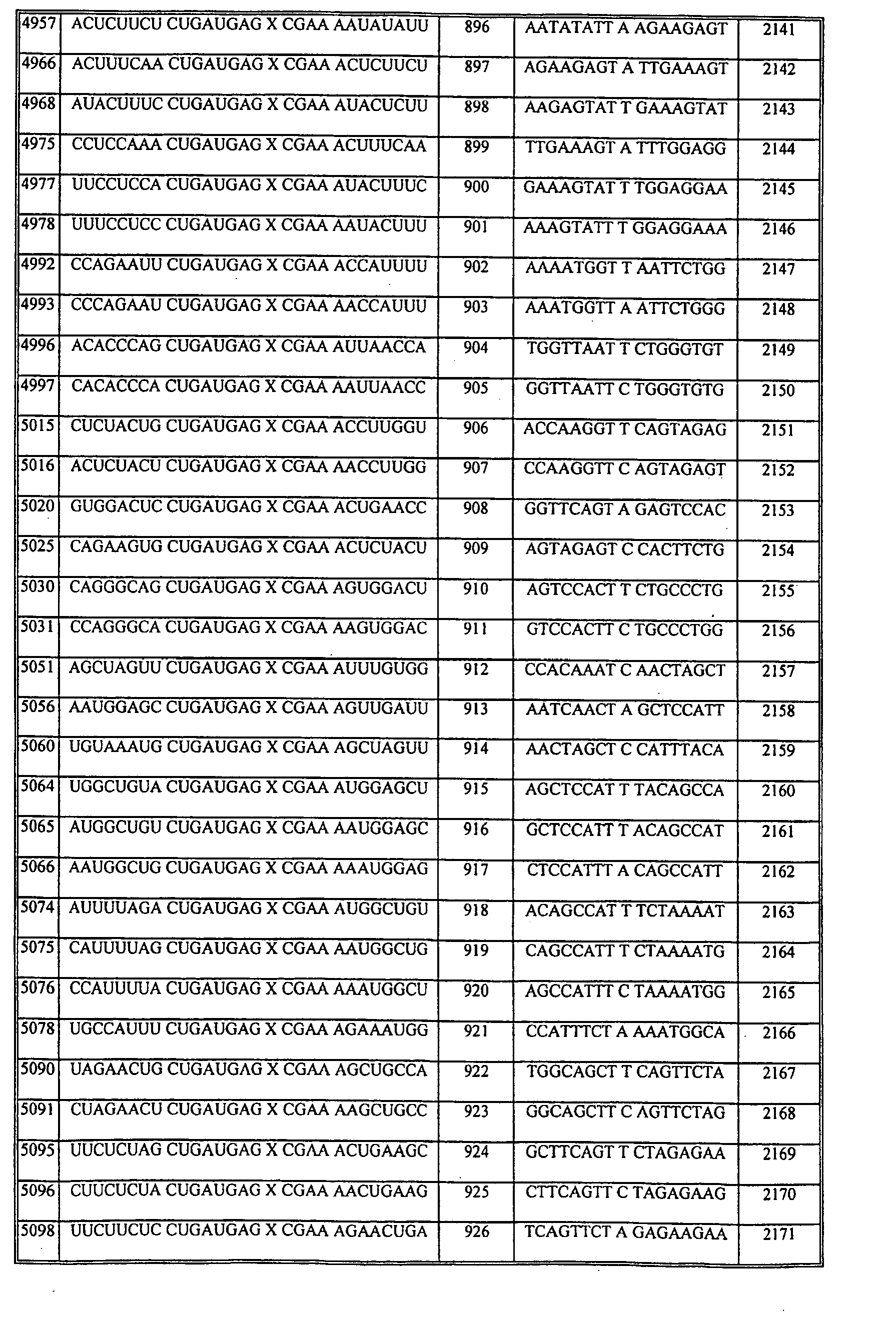

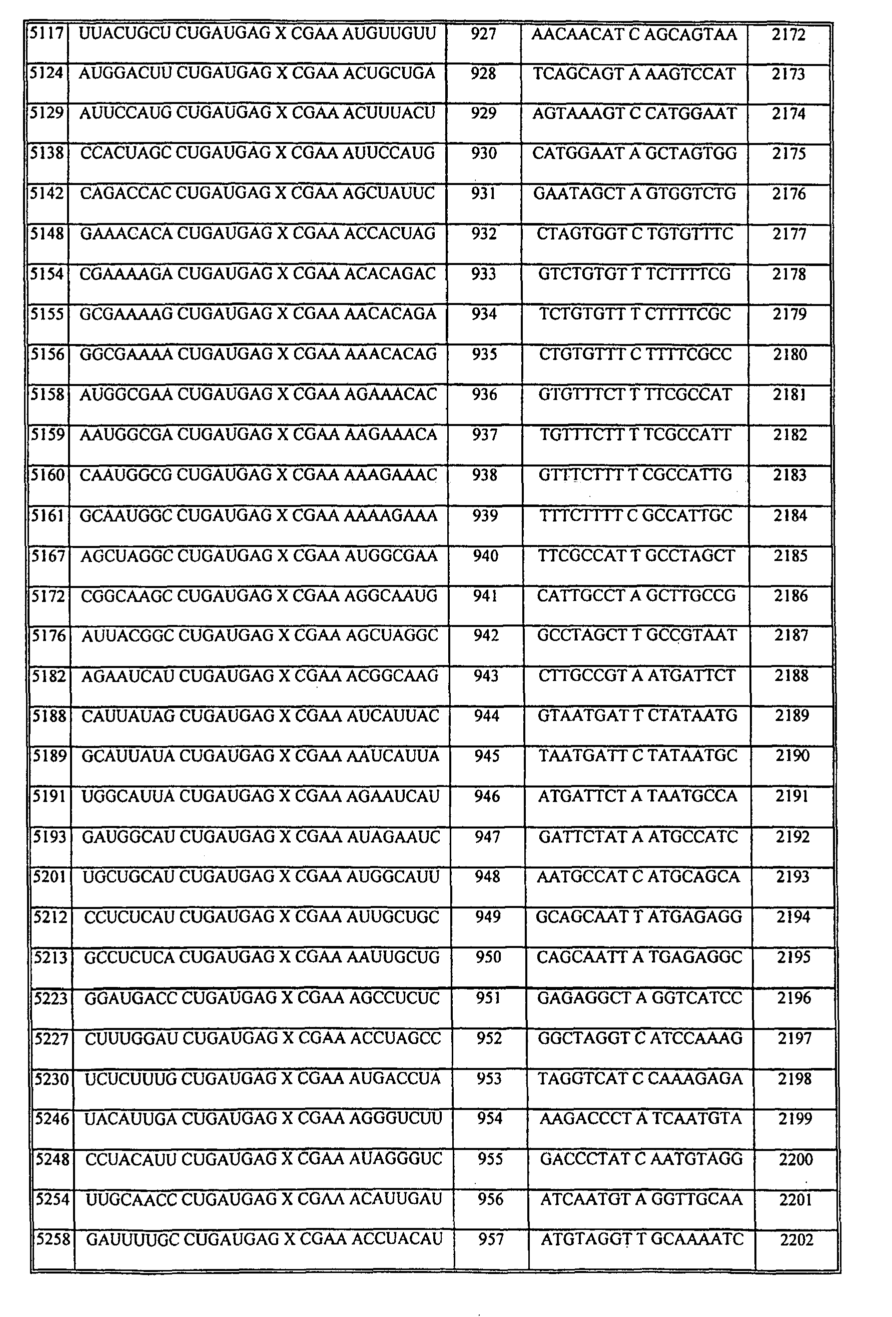

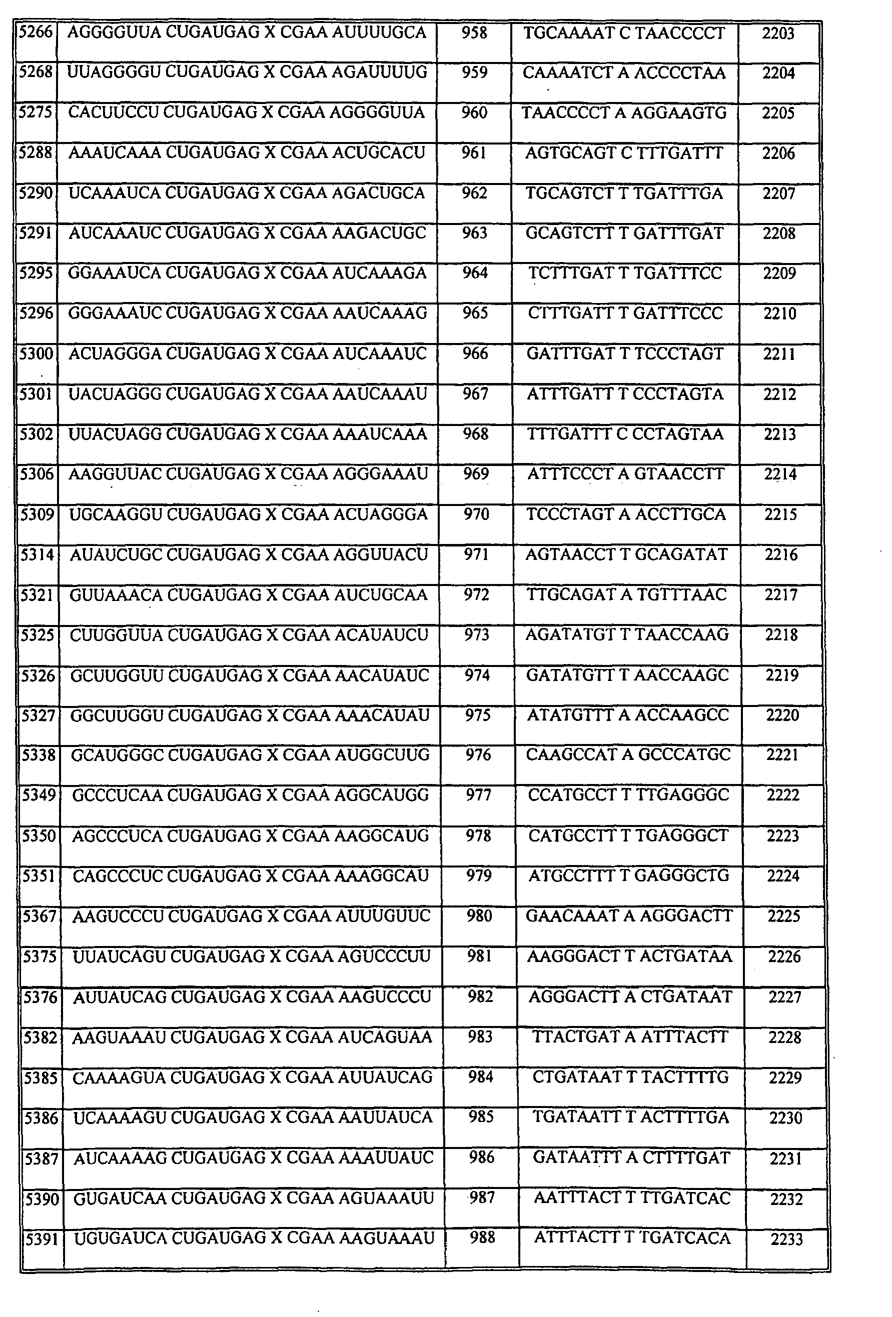

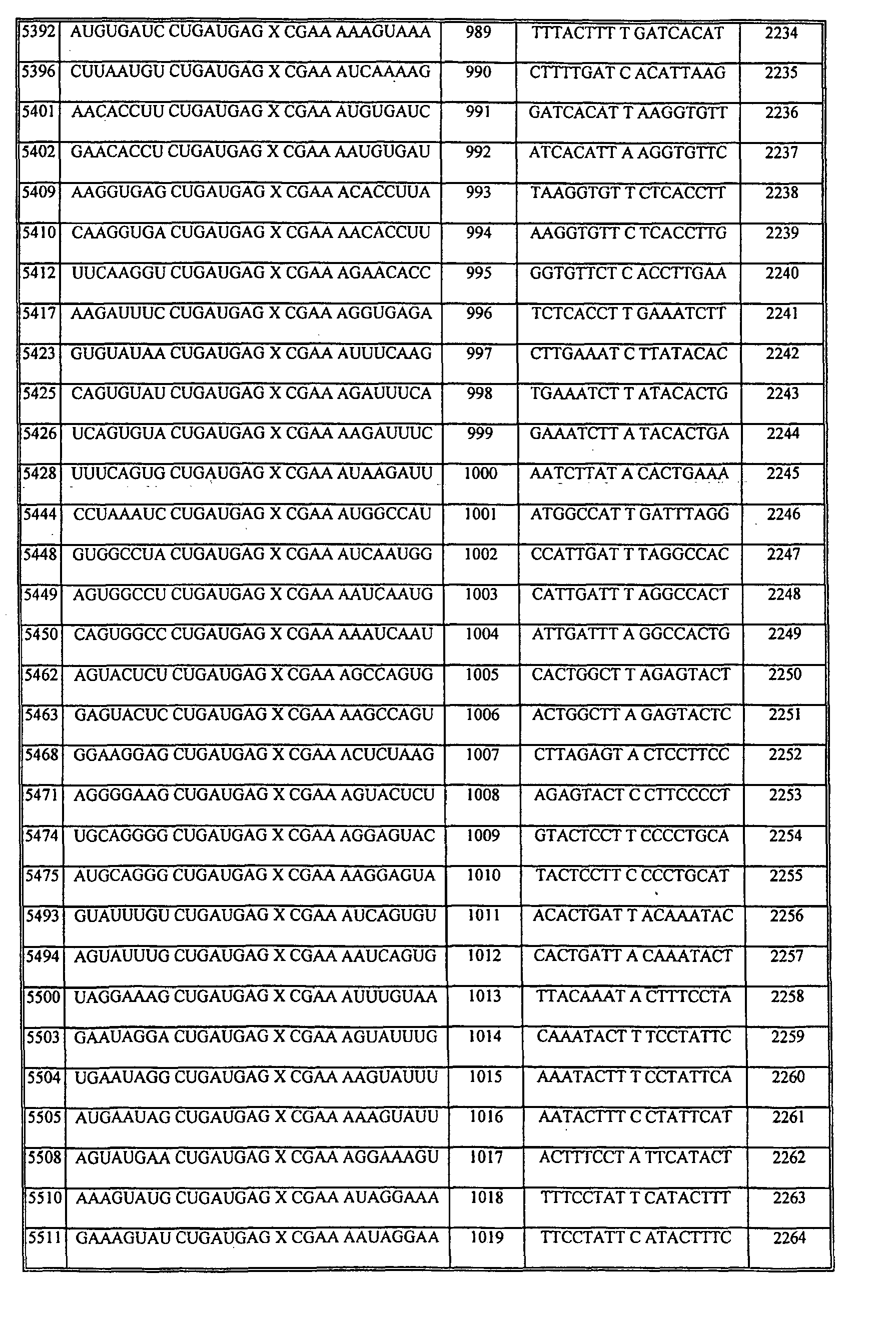

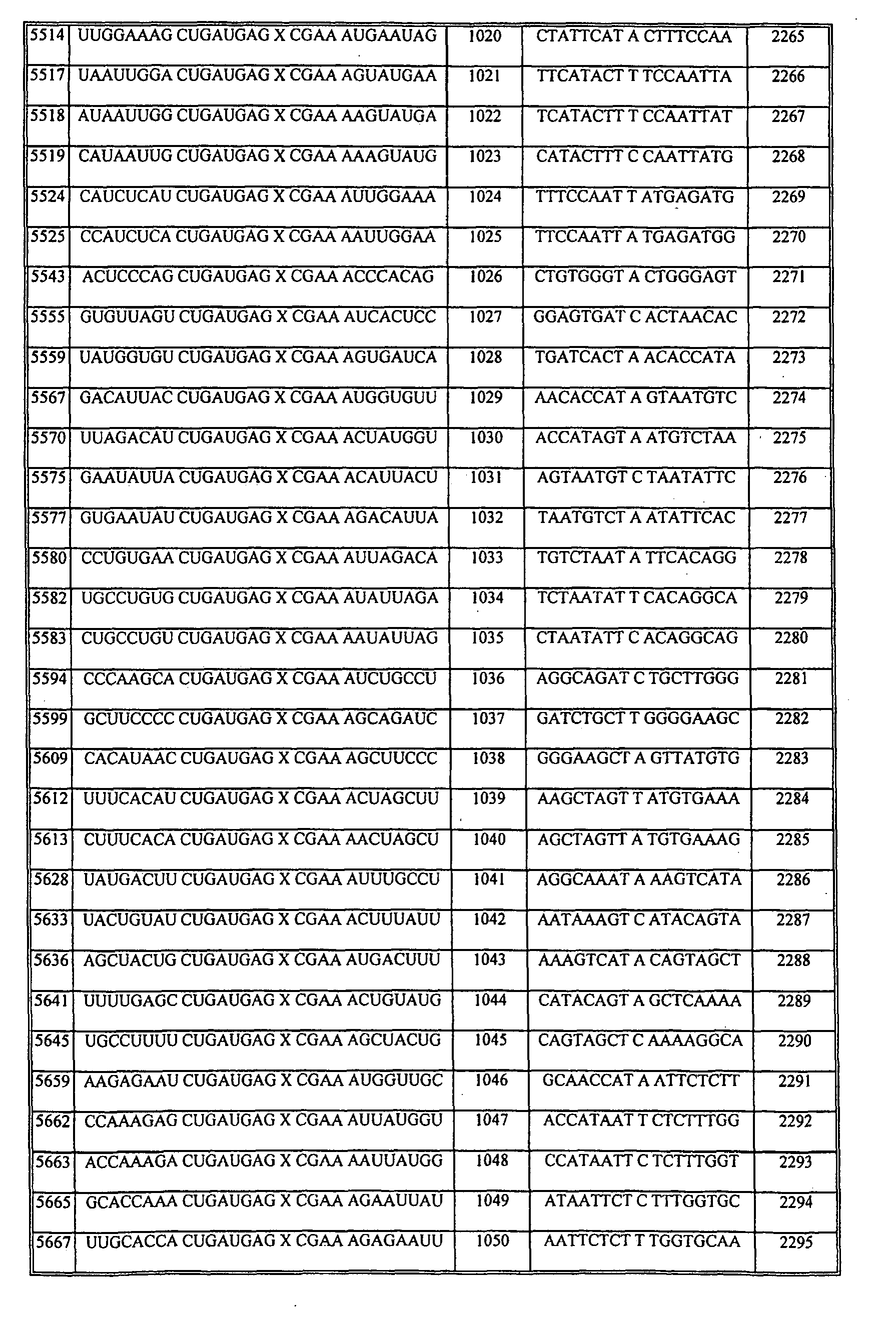

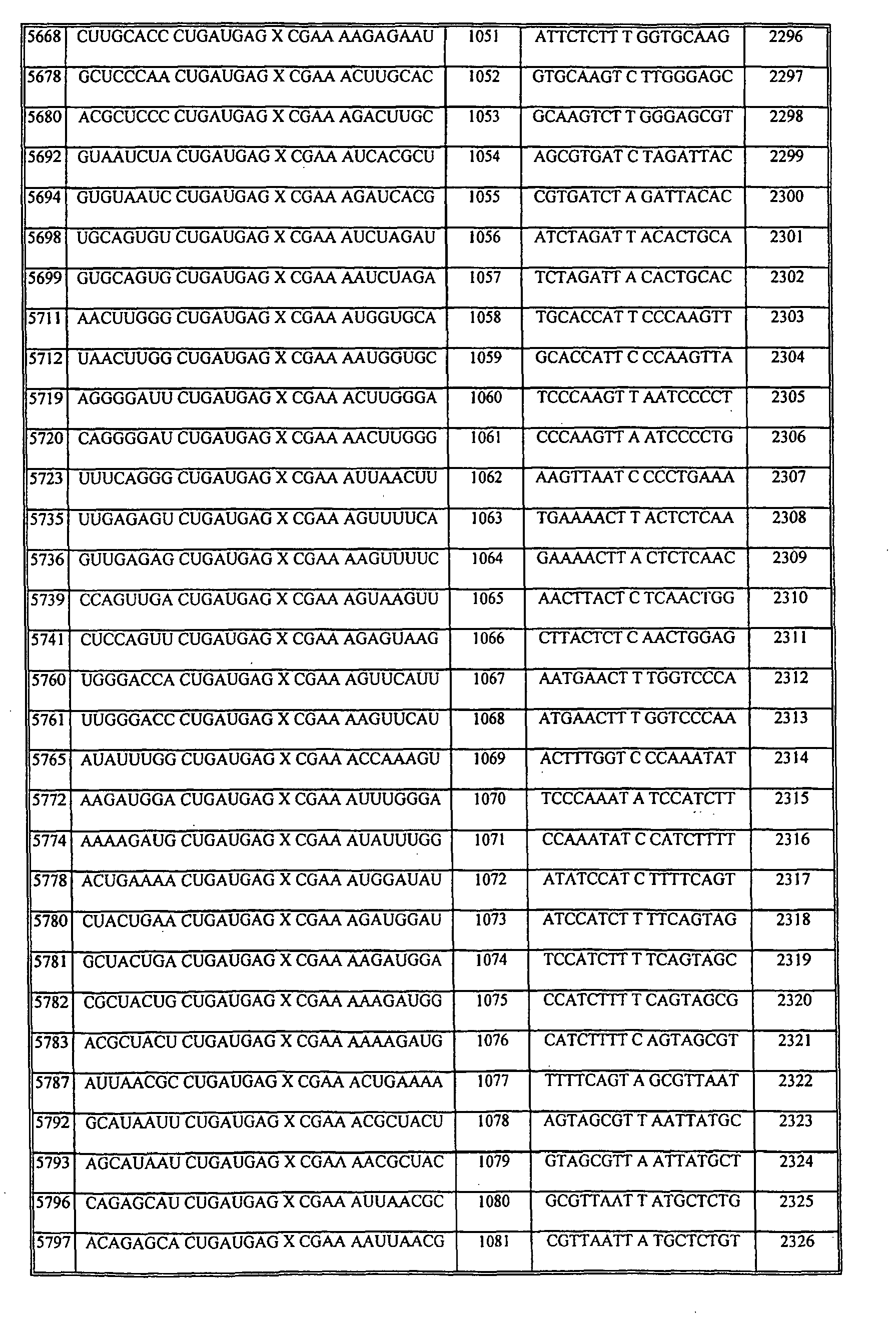

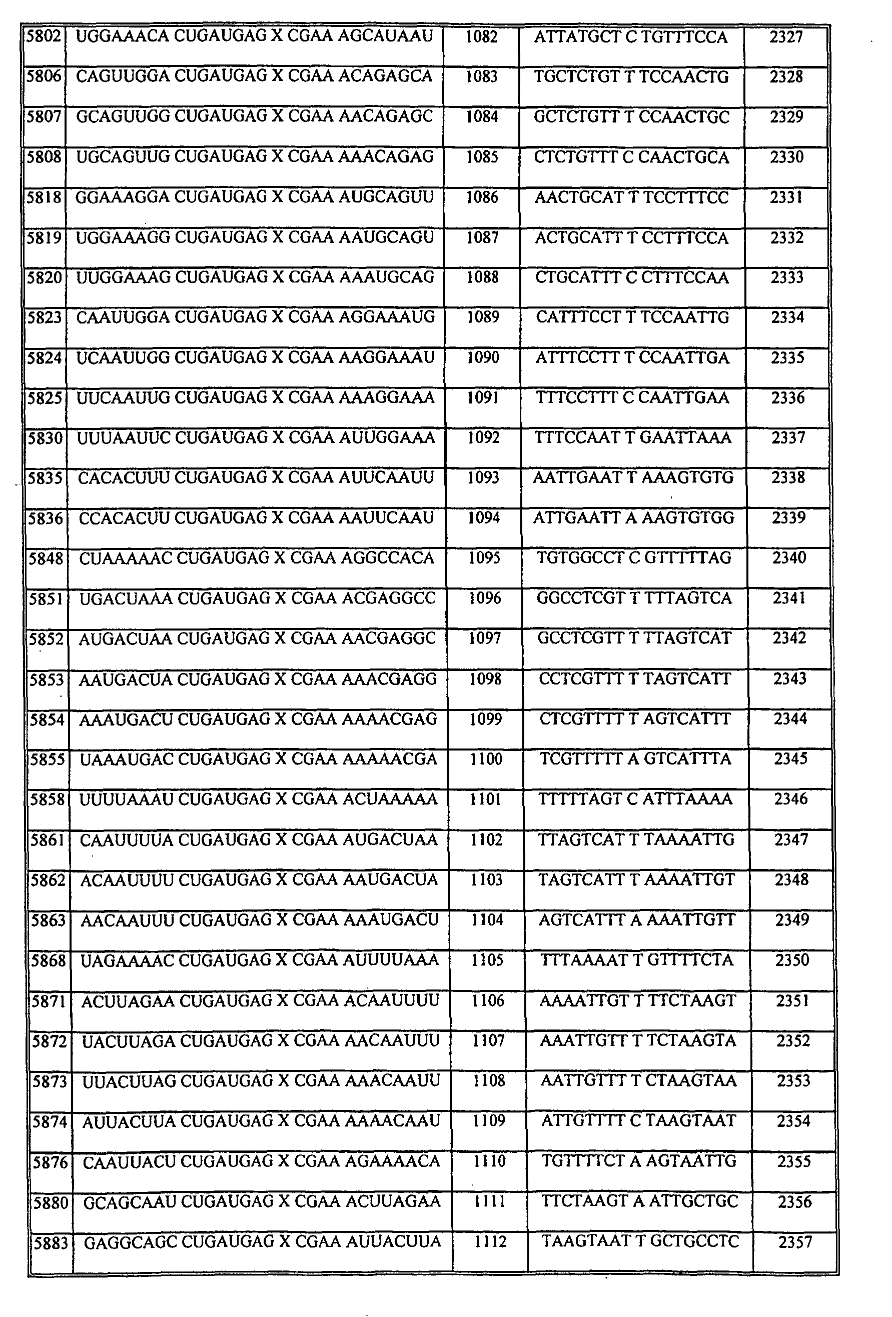

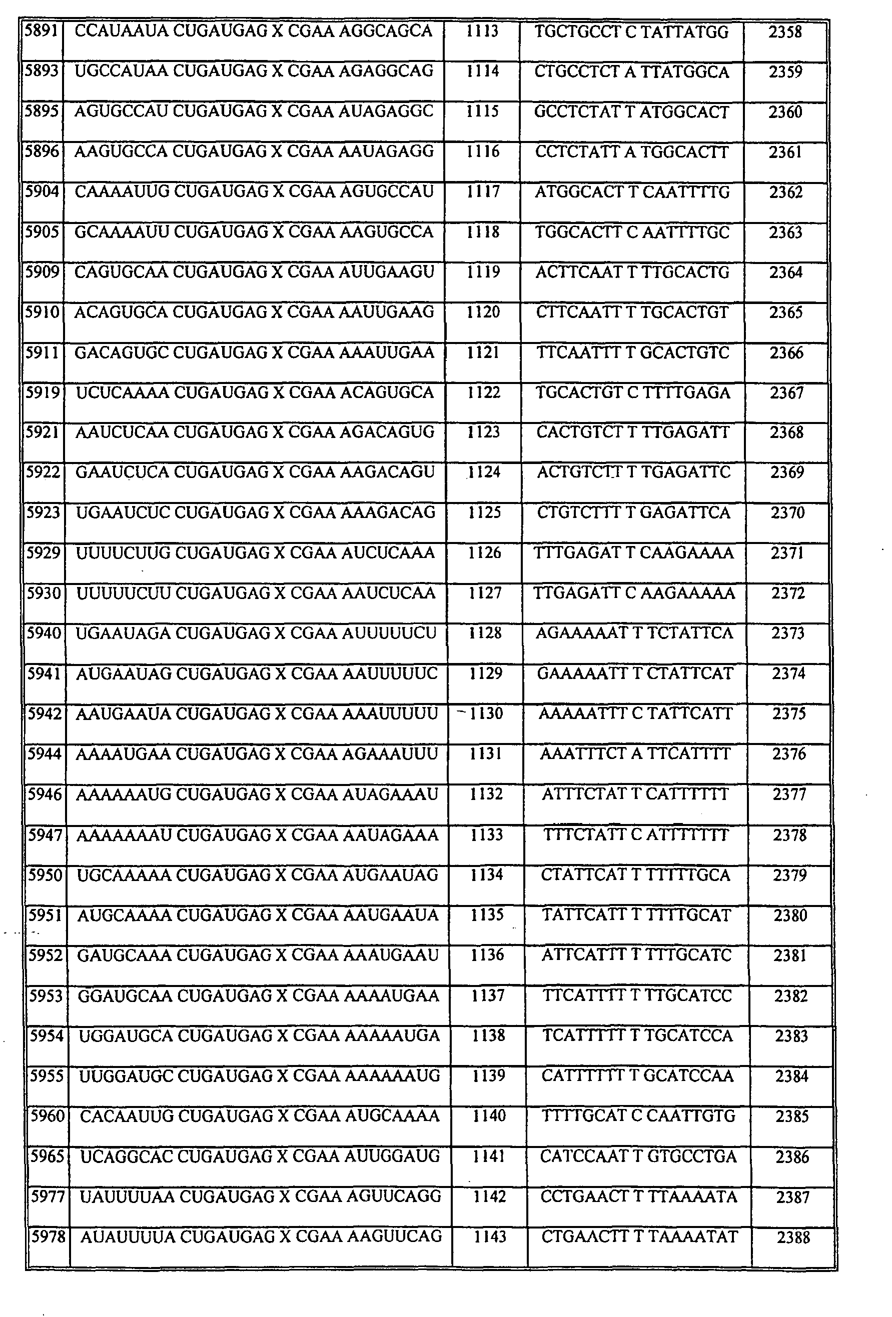

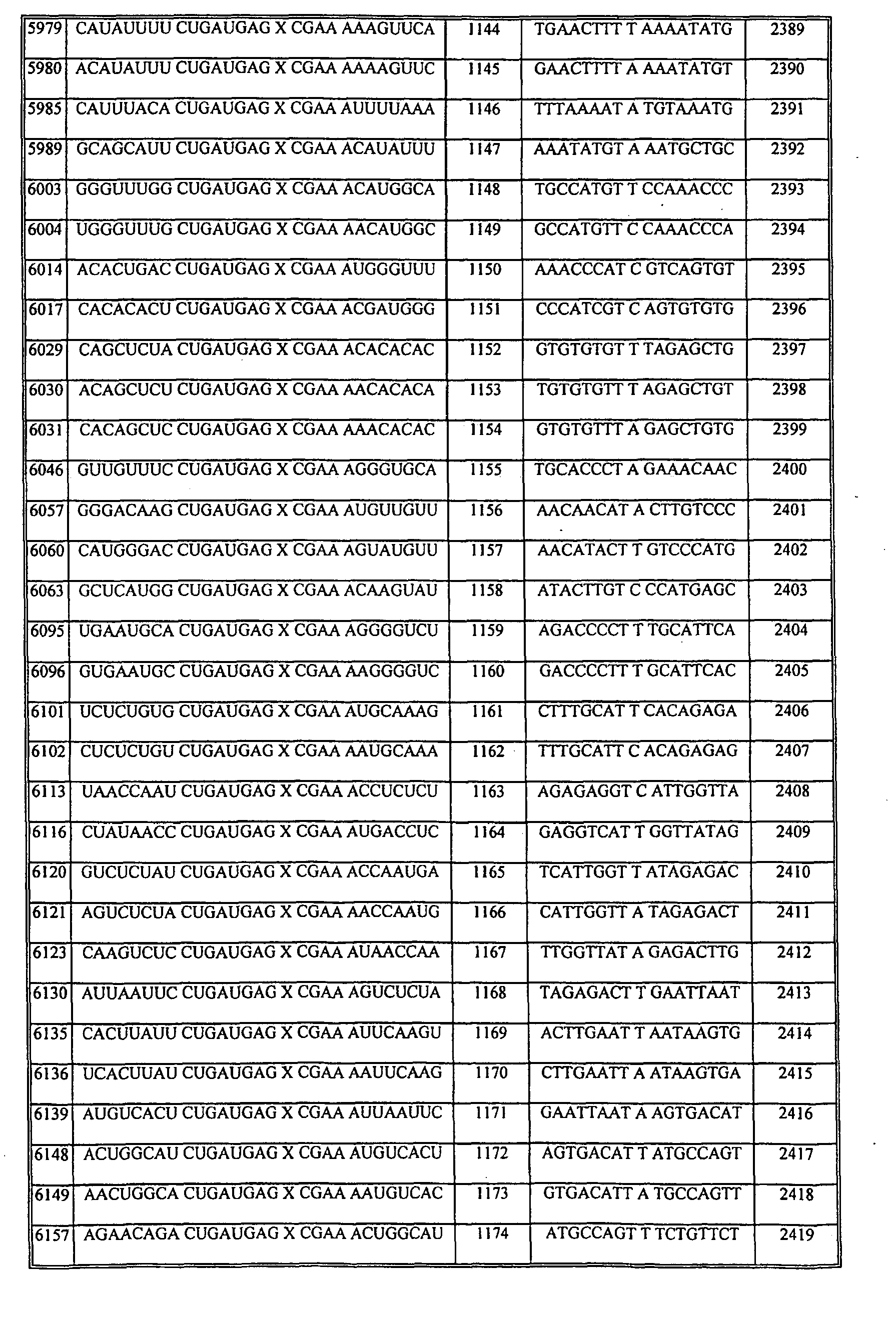

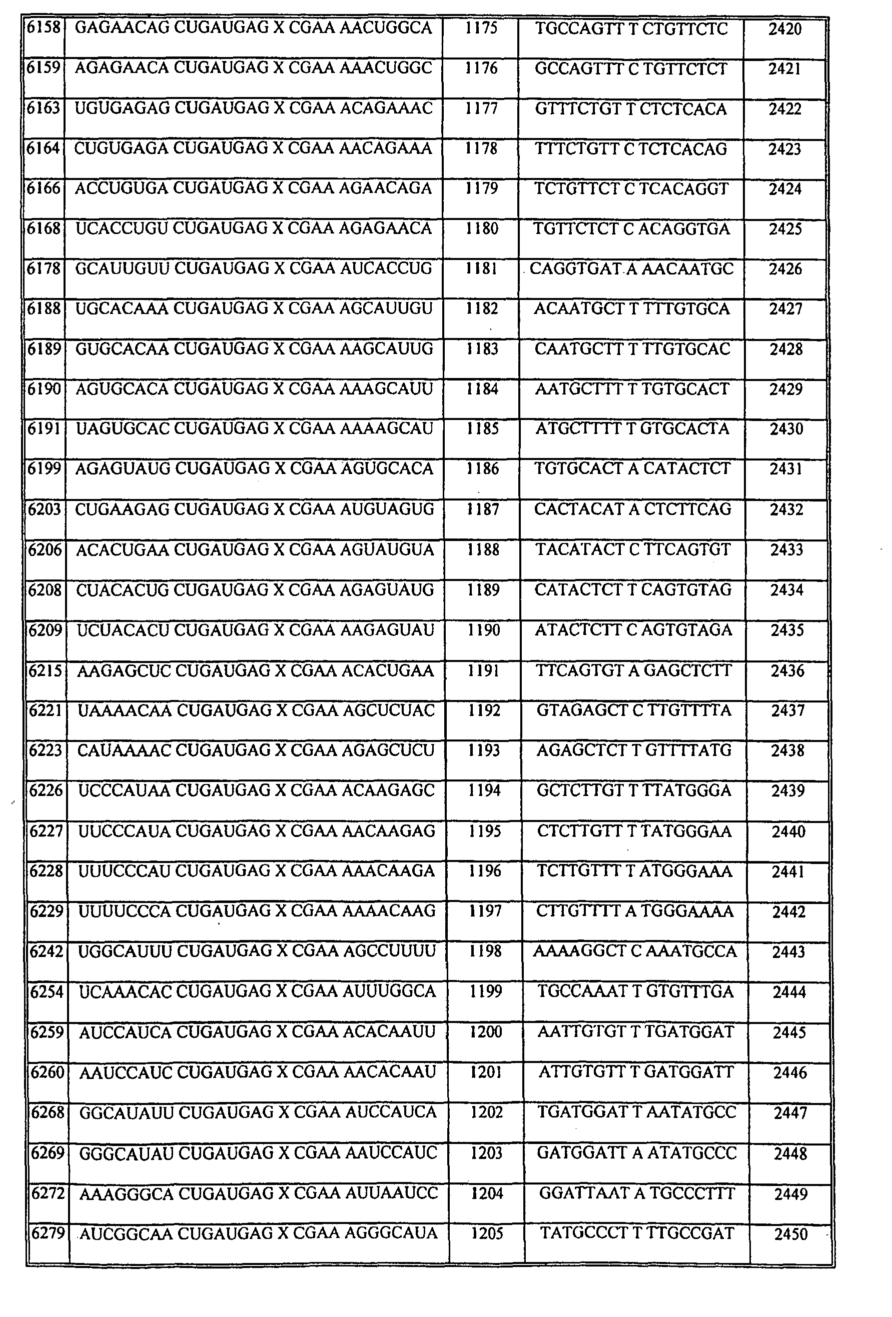

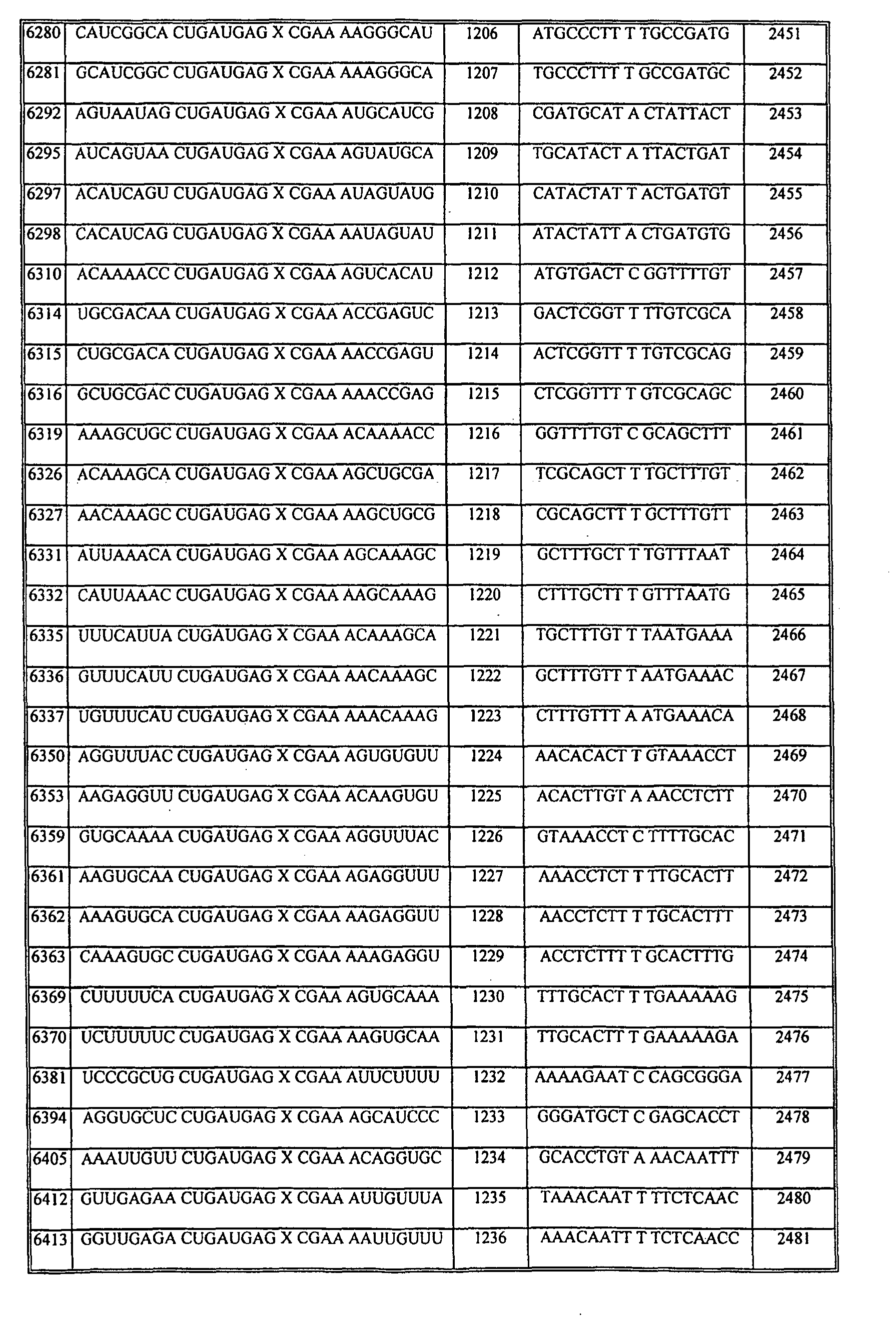

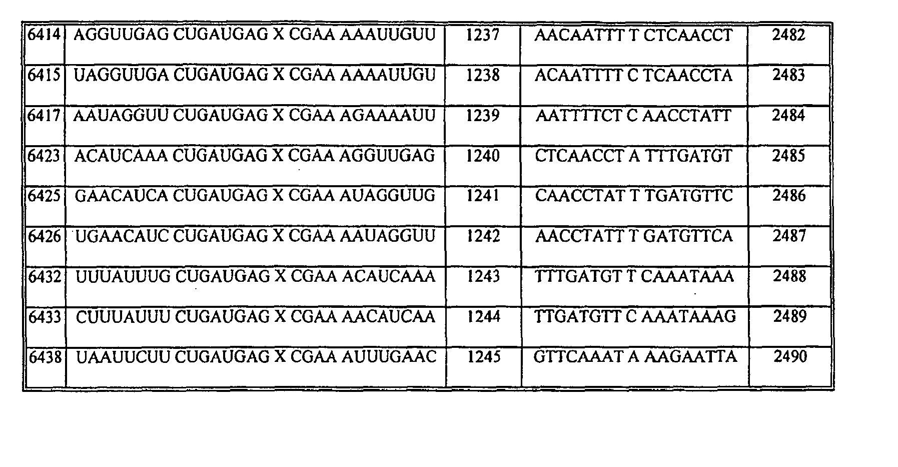

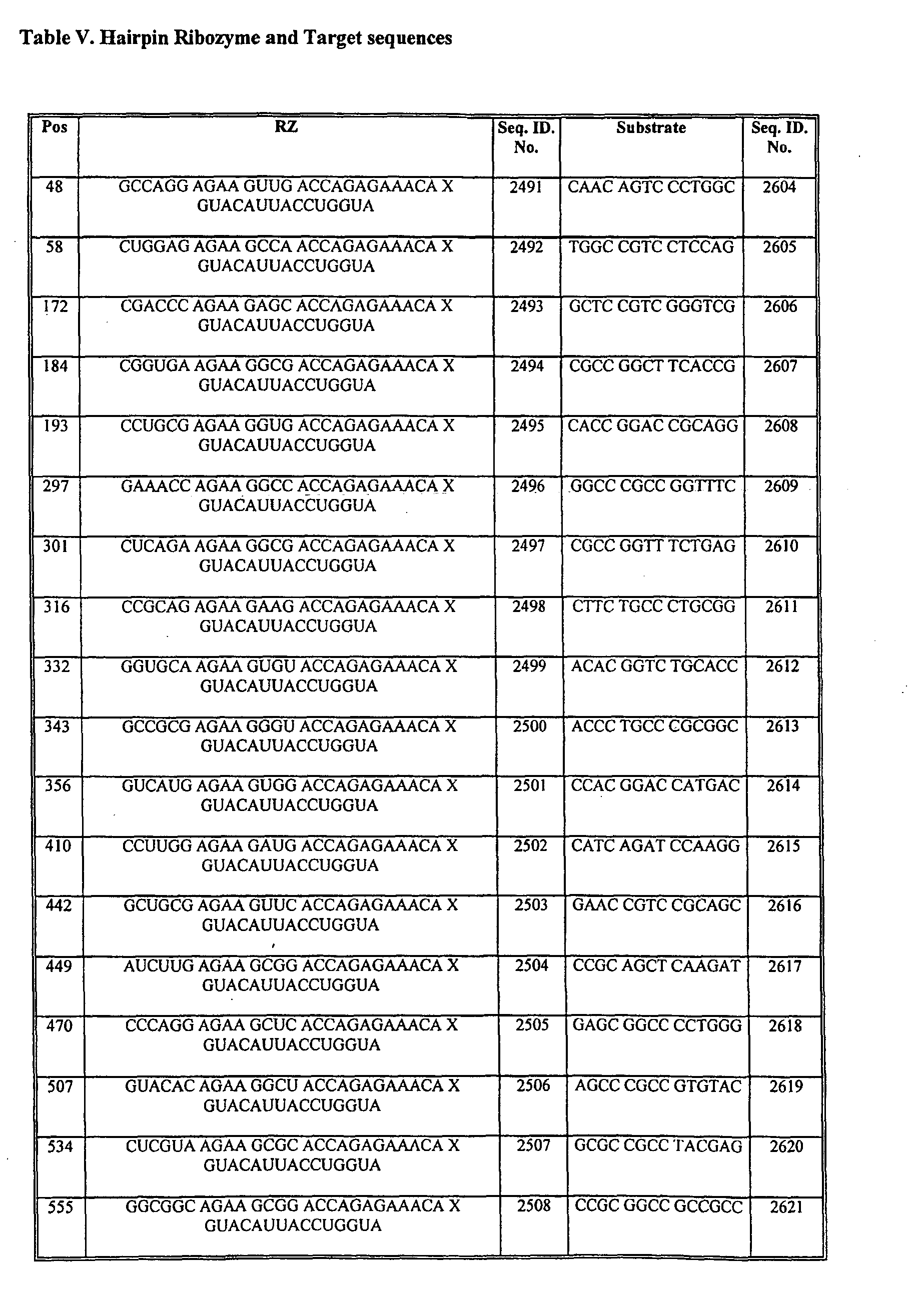

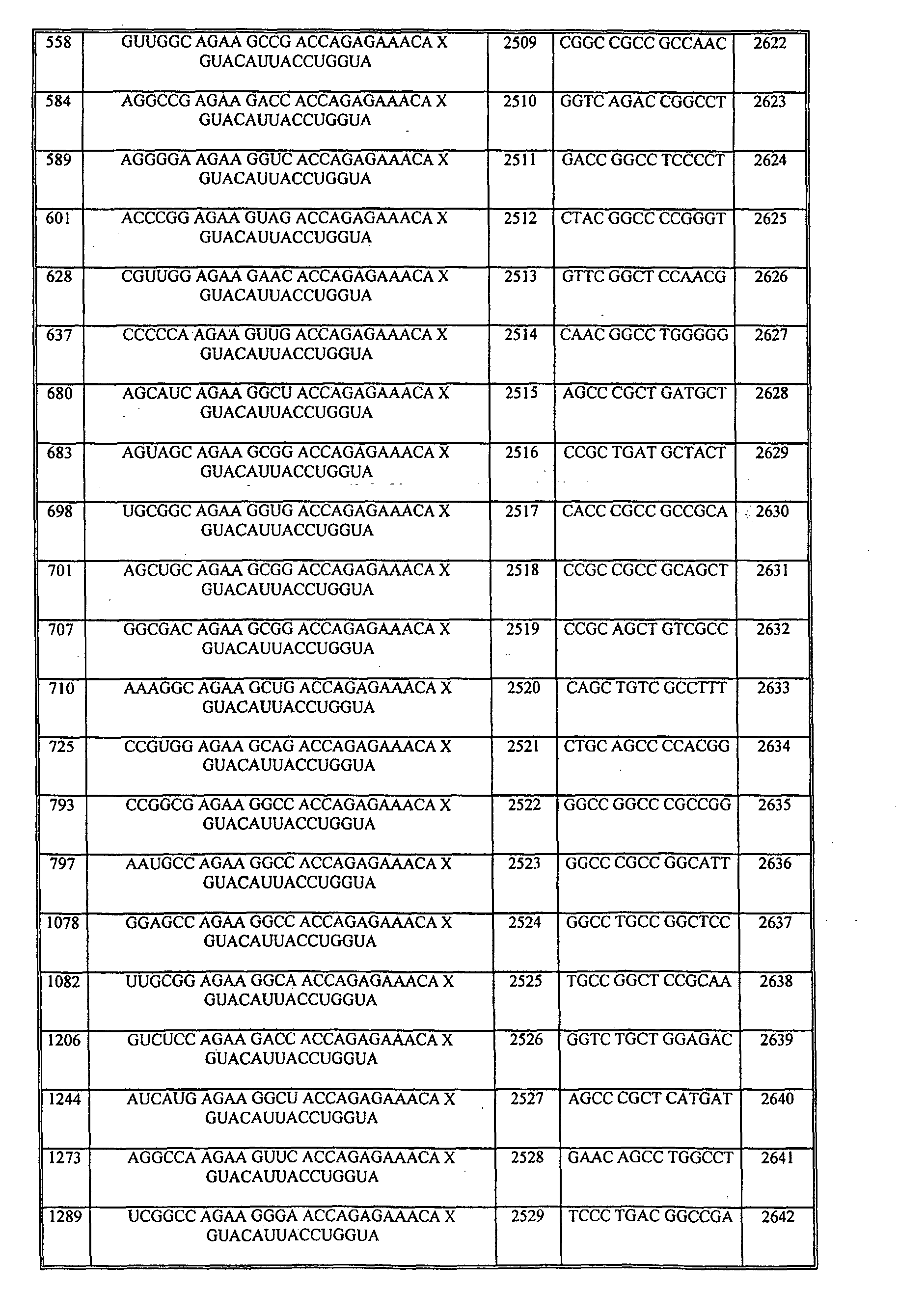

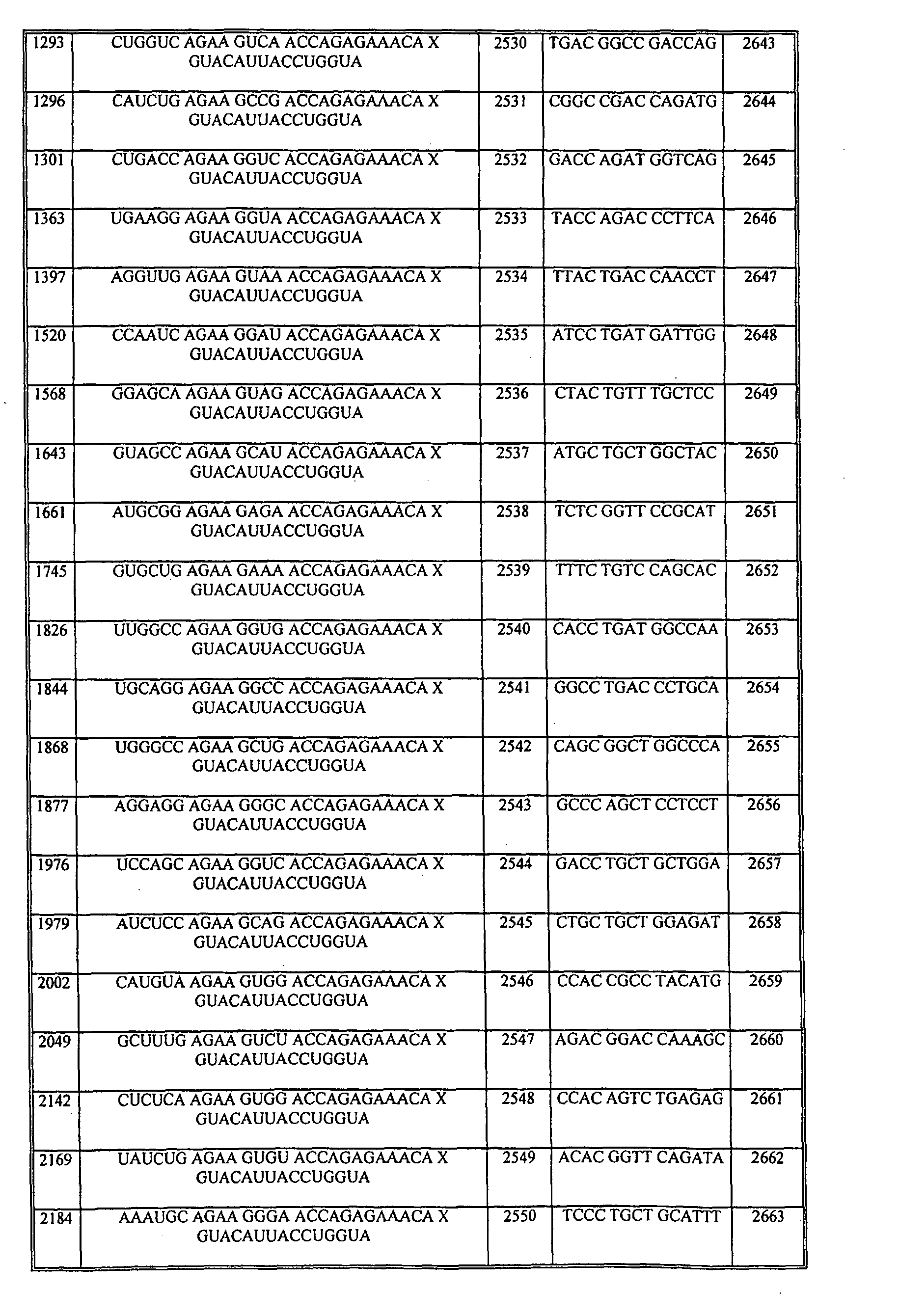

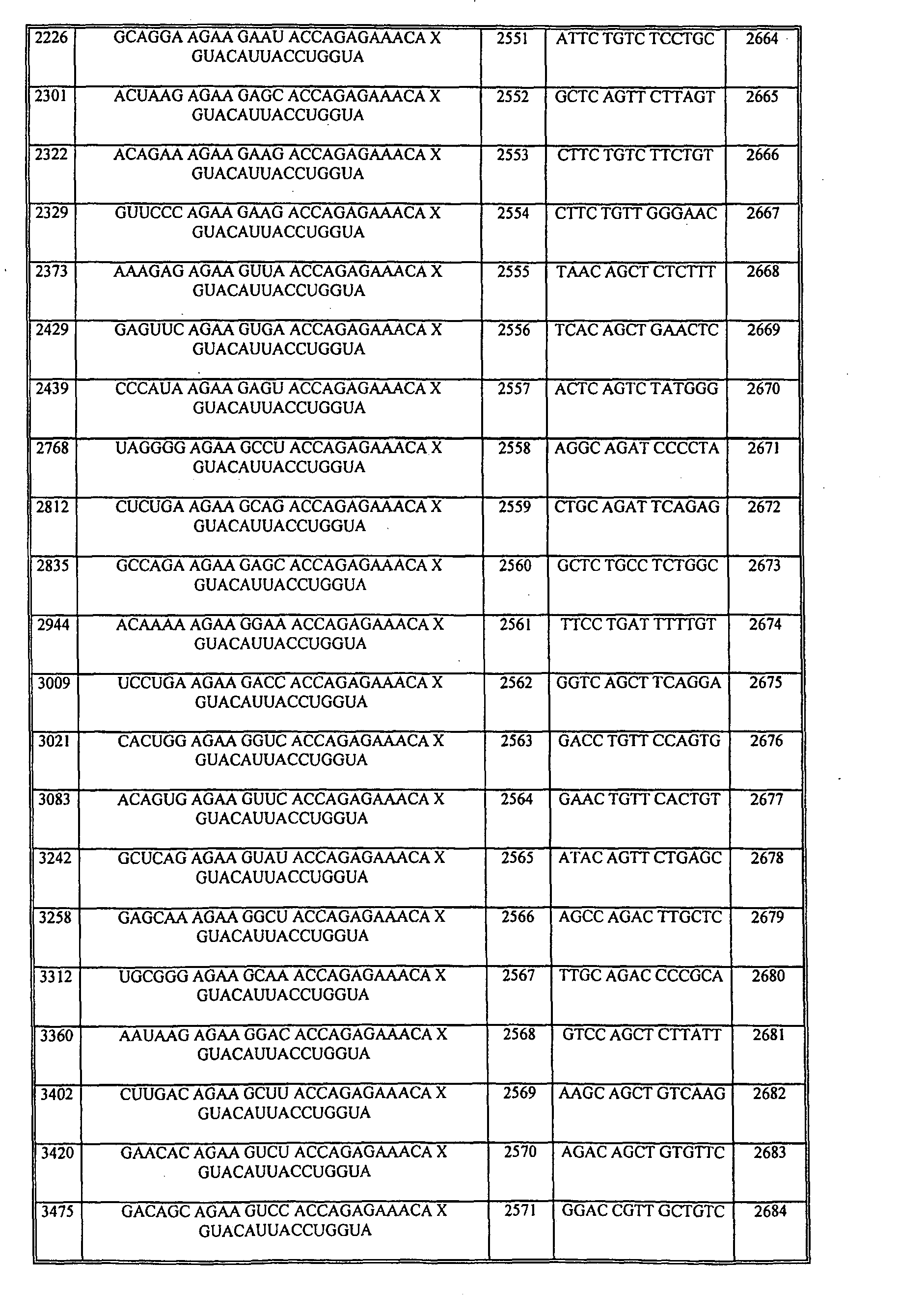

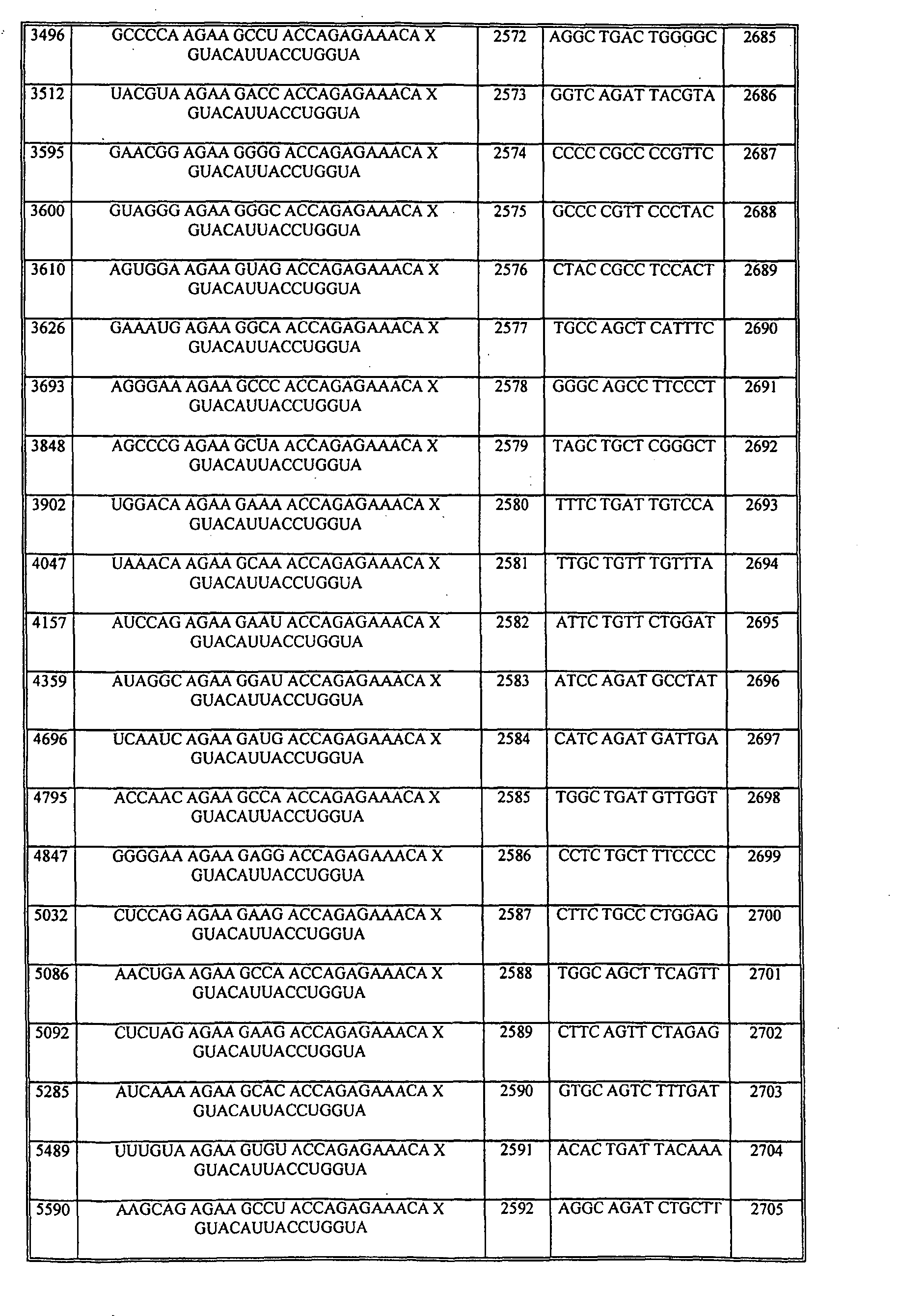

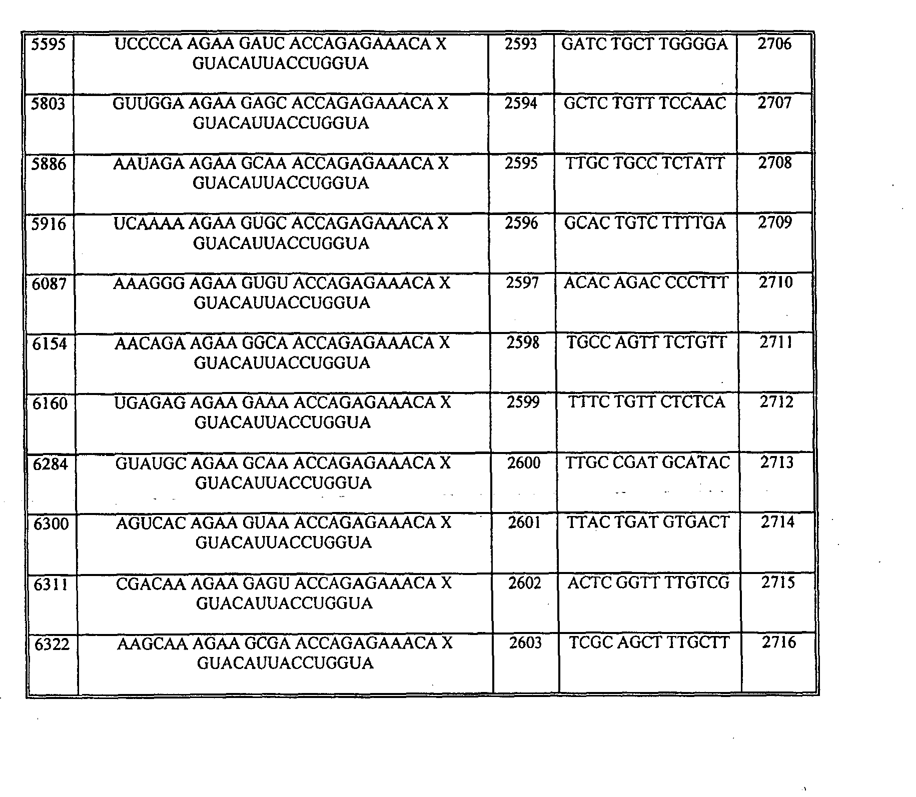

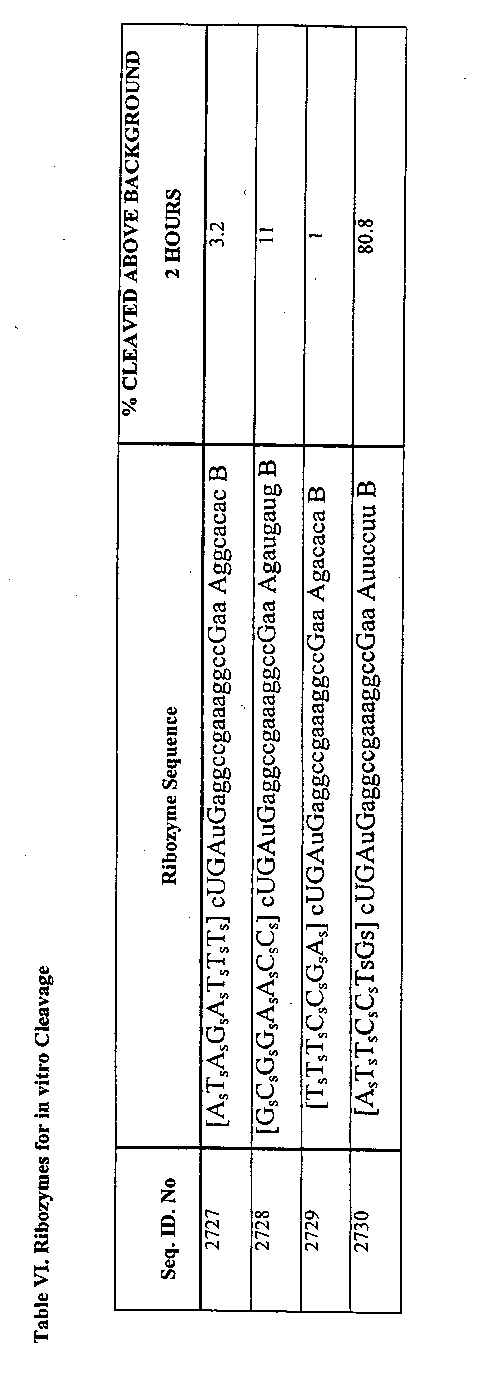

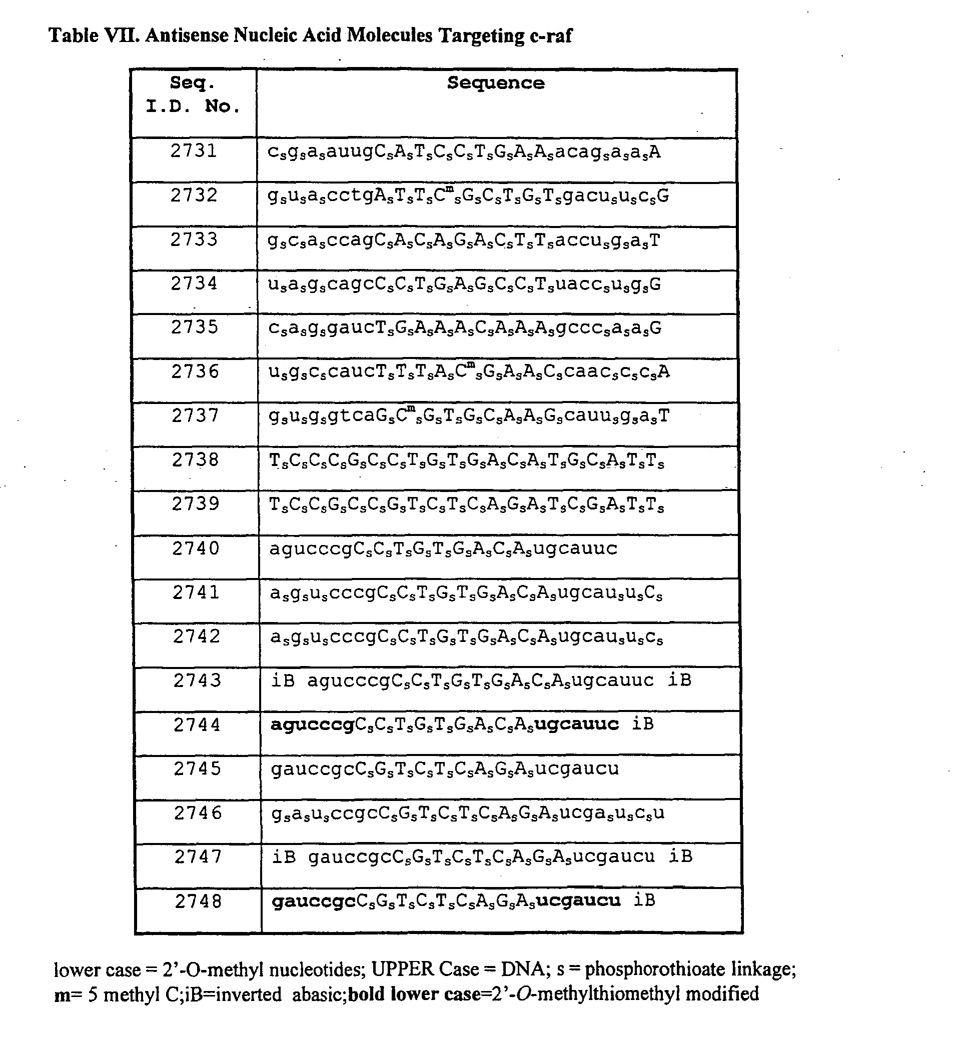

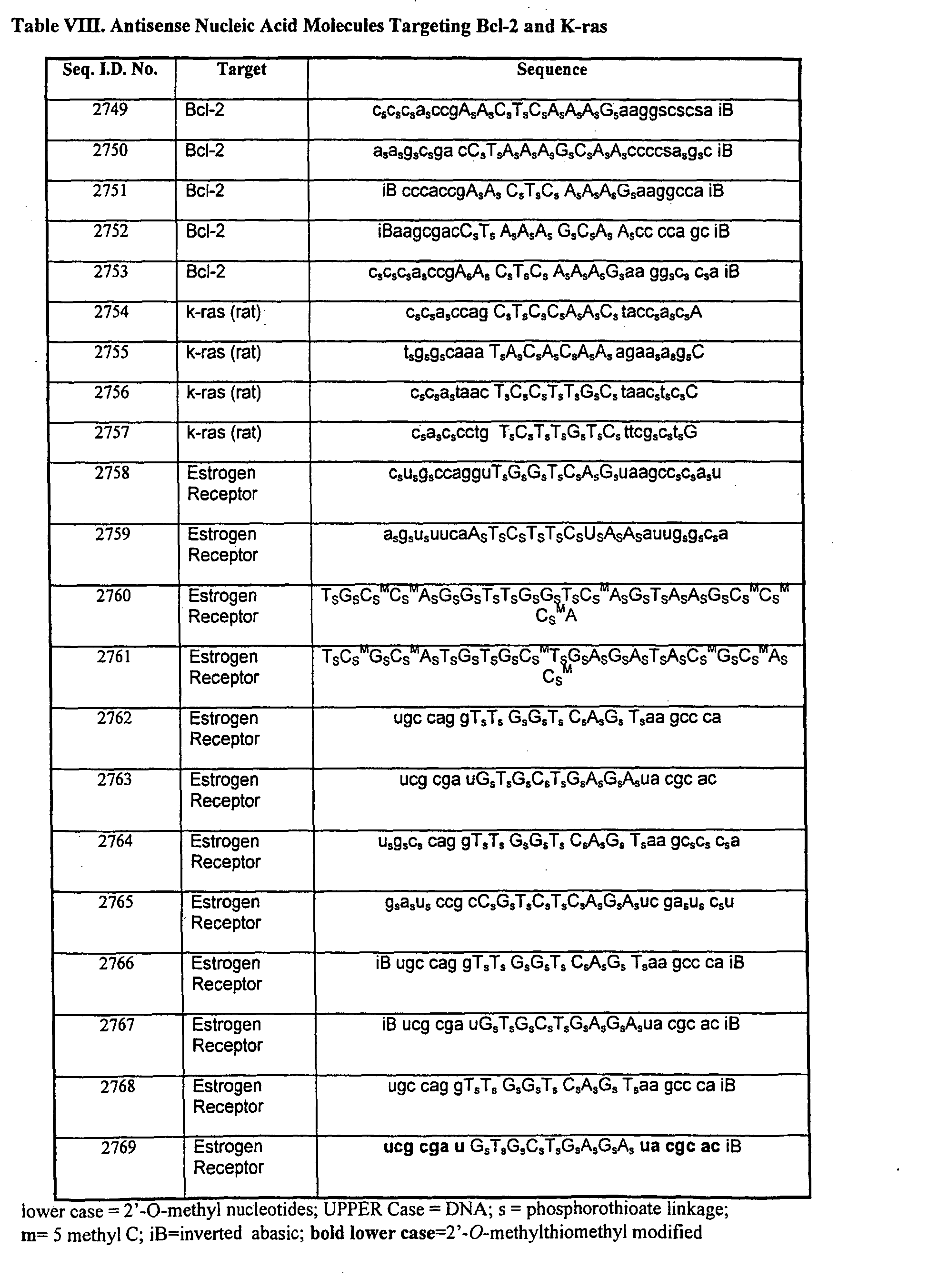

## Claims

1. A chemically synthesized double stranded nucleic acid molecule comprising:
(a) nucleotide sequence having between 14 and 24 nucleotides complementary to nucleotide sequence of a target RNA or a portion thereof;
(b) a 5'-cap, a 3'-cap, or both a 5' and 3'-cap on one of the strands of the double stranded nucleic acid molecule; and
(c) a mixture of modified nucleotides and unmodified nucleotides, wherein the modified nucleotides are 2'-flouro, 2'-O-methyl, 2'H or a combination thereof and the unmodified nucleotides are ribonucleotides

2. The double stranded nucleic acid molecule of claim 1, wherein said cap comprises an inverted nucleotide.

3. The double stranded nucleic acid molecule of claim 2, wherein said inverted nucleotide comprises an inverted deoxynucleotide.

4. The double stranded nucleic acid molecule of claim 1, wherein said cap comprises an inverted abasic moiety.

5. The double stranded nucleic acid molecule of claim 4, wherein said inverted abasic moiety comprises an inverted deoxyabasic moiety.

6. The double stranded nucleic acid molecule of claim 1, wherein said nucleic acid comprises one or more phosphorothioate internucleotide linkages.

7. The double stranded nucleic acid molecule of claim 1, wherein said target RNA is encoded by a mammalian gene.

8. A composition comprising the double stranded nucleic acid molecule of claim 1 in a pharmaceutically acceptable carrier or diluent.


### Annex to the application documents - subsequently filed sequences listing

SEQUENCE LISTING

<110> RIBOZYME PHARMACEUTICALS, INC.
      2950 Wilderness Place
      Boulder, Colorado 80301
      USA

<120> NUCLEIC ACID MOLECULES WITH NOVEL CHEMICAL COMPOSITIONS
      CAPABLE OF MODULATING GENE EXPRESSION

<130> 233/242-PCT

<140> PCT/US99/08547
<141> 1999-04-19

<150> 09/103,636
<151> 1998-06-23

<150> 60/082,404
<151> 1998-04-20

<160> 2786

<170> FastSEQ for Windows Version 3.0

<210> 1
<211> 29
<212> RNA
<213> Artificial Sequence

<220>
<223> Synthesized Hammerhead Ribozyme

<220>
<223> "n" represents stem II region of a HH ribozyme.

<400> 1
uuggcuuacu gaugagncga aacaucacu                                    29

<210> 2
<211> 29
<212> RNA
<213> Artificial Sequence

<220>
<223> Synthesized Hammerhead Ribozyme

<220>
<223> "n" represents stem II region of a HH ribozyme.

<400> 2

auuggcuucu gaugagncga aaacaucac                                          29

<210> 3
<211> 29
<212> RNA
<213> Artificial Sequence

<220>
<223> Synthesized Hammerhead Ribozyme

<220>
<223> "n" represents stem II region of a HH ribozyme.

<400> 3

cauuggcucu gaugagncga aaaacauca                                          29

<210> 4
<211> 29
<212> RNA
<213> Artificial Sequence

<220>
<223> Synthesized Hammerhead Ribozyme

<220>
<223> "n" represents stem II region of a HH ribozyme.

<400> 4

cuugcccucu gaugagncga aacauuggc                                          29

<210> 5
<211> 29
<212> RNA
<213> Artificial Sequence

<220>
<223> Synthesized Hammerhead Ribozyme

<220>
<223> "n" represents stem II region of a HH ribozyme.

<400> 5

cggccaggcu gaugagncga aacuguugc                                          29

<210> 6
<211> 29
<212> RNA
<213> Artificial Sequence

<220>
<223> Synthesized Hammerhead Ribozyme

<220>
<223> "n" represents stem II region of a HH ribozyme.

<400> 6

ugcuggagcu gaugagncga aacggccag 29

<210> 7
<211> 29
<212> RNA
<213> Artificial Sequence

<220>
<223> Synthesized Hammerhead Ribozyme

<220>
<223> "n" represents stem II region of a HH ribozyme.

<400> 7

aggugcugcu gaugagncga aaggacggc 29

<210> 8
<211> 29
<212> RNA
<213> Artificial Sequence

<220>
<223> Synthesized Hammerhead Ribozyme

<220>
<223> "n" represents stem II region of a HH ribozyme.

<400> 8

gcauuacacu gaugagncga aaggugcug 29

<210> 9
<211> 29
<212> RNA
<213> Artificial Sequence

<220>
<223> Synthesized Hammerhead Ribozyme

<220>
<223> "n" represents stem II region of a HH ribozyme.

<400> 9

ugcauuaccu gaugagncga aaaggugcu 29

<210> 10
<211> 29
<212> RNA
<213> Artificial Sequence

<220>
<223> Synthesized Hammerhead Ribozyme

<220>
<223> "n" represents stem II region of a HH ribozyme.

<400> 10

auaugcaucu gaugagncga aacaaaggu                                29

<210> 11
<211> 29
<212> RNA
<213> Artificial Sequence

<220>
<223> Synthesized Hammerhead Ribozyme

<220>
<223> "n" represents stem II region of a HH ribozyme.

<400> 11

cgagcucacu gaugagncga aaugcauua                                29

<210> 12
<211> 29
<212> RNA
<213> Artificial Sequence

<220>
<223> Synthesized Hammerhead Ribozyme

<220>
<223> "n" represents stem II region of a HH ribozyme.

<400> 12

ggucuccccu gaugagncga aagcucaua                                29

<210> 13
<211> 29
<212> RNA
<213> Artificial Sequence

<220>
<223> Synthesized Hammerhead Ribozyme

<220>
<223> "n" represents stem II region of a HH ribozyme.

<400> 13

acuuuaagcu gaugagncga aacuggucu                                        29

<210> 14
<211> 29
<212> RNA
<213> Artificial Sequence

<220>
<223> Synthesized Hammerhead Ribozyme

<220>
<223> "n" represents stem II region of a HH ribozyme.

<400> 14

ccaacuuucu gaugagncga aaguacugg                                        29

<210> 15
<211> 29
<212> RNA
<213> Artificial Sequence

<220>
<223> Synthesized Hammerhead Ribozyme

<220>
<223> "n" represents stem II region of a HH ribozyme.

<400> 15

uccaacuucu gaugagncga aaaguacug                                        29

<210> 16
<211> 29
<212> RNA
<213> Artificial Sequence

<220>
<223> Synthesized Hammerhead Ribozyme

<220>
<223> "n" represents stem II region of a HH ribozyme.

<400> 16

gggccucccu gaugagncga aacuuuaag                                    29

<210> 17
<211> 29
<212> RNA
<213> Artificial Sequence

<220>
<223> Synthesized Hammerhead Ribozyme

<220>
<223> "n" represents stem II region of a HH ribozyme.

<400> 17

ccaggacgcu gaugagncga aacgcccuc                                    29

<210> 18
<211> 29
<212> RNA
<213> Artificial Sequence

<220>
<223> Synthesized Hammerhead Ribozyme

<220>
<223> "n" represents stem II region of a HH ribozyme.

<400> 18

cccaggaccu gaugagncga aaacgcccu                                    29

<210> 19
<211> 29
<212> RNA
<213> Artificial Sequence

<220>
<223> Synthesized Hammerhead Ribozyme

<220>
<223> "n" represents stem II region of a HH ribozyme.

<400> 19

gcucccagcu gaugagncga aacgaacgc                                    29

<210> 20
<211> 29
<212> RNA
<213> Artificial Sequence

<220>

<223> Synthesized Hammerhead Ribozyme

<220>
<223> "n" represents stem II region of a HH ribozyme.

<400> 20

gacggagccu gaugagncga aagugcagc                                    29



<210> 21
<211> 29
<212> RNA
<213> Artificial Sequence

<220>
<223> Synthesized Hammerhead Ribozyme

<220>
<223> "n" represents stem II region of a HH ribozyme.

<400> 21

acccgacgcu gaugagncga aagcaagug                                    29



<210> 22
<211> 29
<212> RNA
<213> Artificial Sequence

<220>
<223> Synthesized Hammerhead Ribozyme

<220>
<223> "n" represents stem II region of a HH ribozyme.

<400> 22

ggcgaccccu gaugagncga aacggagca                                    29



<210> 23
<211> 29
<212> RNA
<213> Artificial Sequence

<220>
<223> Synthesized Hammerhead Ribozyme

<220>
<223> "n" represents stem II region of a HH ribozyme.

<400> 23

aagccggccu gaugagncga aacccgacg                                    29

<210> 24
<211> 29
<212> RNA
<213> Artificial Sequence

<220>
<223> Synthesized Hammerhead Ribozyme

<220>
<223> "n" represents stem II region of a HH ribozyme.

<400> 24

guccggugcu gaugagncga aagccggcg                                          29



<210> 25
<211> 29
<212> RNA
<213> Artificial Sequence

<220>
<223> Synthesized Hammerhead Ribozyme

<220>
<223> "n" represents stem II region of a HH ribozyme.

<400> 25

gguccggucu gaugagncga aaagccggc                                          29



<210> 26
<211> 29
<212> RNA
<213> Artificial Sequence

<220>
<223> Synthesized Hammerhead Ribozyme

<220>
<223> "n" represents stem II region of a HH ribozyme.

<400> 26

ugccccggcu gaugagncga aagccugcg                                          29



<210> 27
<211> 29
<212> RNA
<213> Artificial Sequence

<220>
<223> Synthesized Hammerhead Ribozyme

<220>

<223> "n" represents stem II region of a HH ribozyme.

<400> 27

cgacacgccu gaugagncga aagcucugg                                          29

<210> 28
<211> 29
<212> RNA
<213> Artificial Sequence

<220>
<223> Synthesized Hammerhead Ribozyme

<220>
<223> "n" represents stem II region of a HH ribozyme.

<400> 28

gucccgcccu gaugagncga aacacgcga                                          29

<210> 29
<211> 29
<212> RNA
<213> Artificial Sequence

<220>
<223> Synthesized Hammerhead Ribozyme

<220>
<223> "n" represents stem II region of a HH ribozyme.

<400> 29

uuagaggccu gaugagncga aacgcagcg                                          29

<210> 30
<211> 29
<212> RNA
<213> Artificial Sequence

<220>
<223> Synthesized Hammerhead Ribozyme

<220>
<223> "n" represents stem II region of a HH ribozyme.

<400> 30

cgagguuacu gaugagncga aaggcgacg                                          29

<210> 31
<211> 29

<212> RNA
<213> Artificial Sequence

<220>
<223> Synthesized Hammerhead Ribozyme

<220>
<223> "n" represents stem II region of a HH ribozyme.

<400> 31

cccgaggucu gaugagncga aagaggcga                                          29



<210> 32
<211> 29
<212> RNA
<213> Artificial Sequence

<220>
<223> Synthesized Hammerhead Ribozyme

<220>
<223> "n" represents stem II region of a HH ribozyme.

<400> 32

cacagccccu gaugagncga aagguuaga                                          29



<210> 33
<211> 29
<212> RNA
<213> Artificial Sequence

<220>
<223> Synthesized Hammerhead Ribozyme

<220>
<223> "n" represents stem II region of a HH ribozyme.

<400> 33

uggaaaaacu gaugagncga aagcacagc                                          29



<210> 34
<211> 29
<212> RNA
<213> Artificial Sequence

<220>
<223> Synthesized Hammerhead Ribozyme

<220>
<223> "n" represents stem II region of a HH ribozyme.

<400> 34

ccuggaaacu gaugagncga aagagcaca                                29

<210> 35
<211> 29
<212> RNA
<213> Artificial Sequence

<220>
<223> Synthesized Hammerhead Ribozyme

<220>
<223> "n" represents stem II region of a HH ribozyme.

<400> 35

accuggaacu gaugagncga aaagagcac                                29

<210> 36
<211> 29
<212> RNA
<213> Artificial Sequence

<220>
<223> Synthesized Hammerhead Ribozyme

<220>
<223> "n" represents stem II region of a HH ribozyme.

<400> 36

caccuggacu gaugagncga aaaagagca                                29

<210> 37
<211> 29
<212> RNA
<213> Artificial Sequence

<220>
<223> Synthesized Hammerhead Ribozyme

<220>
<223> "n" represents stem II region of a HH ribozyme.

<400> 37

ccaccuggcu gaugagncga aaaaagagc                                29

<210> 38
<211> 29
<212> RNA
<213> Artificial Sequence

<220>
<223> Synthesized Hammerhead Ribozyme

<220>
<223> "n" represents stem II region of a HH ribozyme.

<400> 38

gccaccugcu gaugagncga aaaaaagag 29

<210> 39
<211> 29
<212> RNA
<213> Artificial Sequence

<220>
<223> Synthesized Hammerhead Ribozyme

<220>
<223> "n" represents stem II region of a HH ribozyme.

<400> 39

ggcucagacu gaugagncga aaccggcgg 29

<210> 40
<211> 29
<212> RNA
<213> Artificial Sequence

<220>
<223> Synthesized Hammerhead Ribozyme

<220>
<223> "n" represents stem II region of a HH ribozyme.

<400> 40

aggcucagcu gaugagncga aaaccggcg 29

<210> 41
<211> 29
<212> RNA
<213> Artificial Sequence

<220>
<223> Synthesized Hammerhead Ribozyme

<220>
<223> "n" represents stem II region of a HH ribozyme.

<400> 41

aaggcucacu gaugagncga aaaaccggc 29

<210> 42
<211> 29
<212> RNA
<213> Artificial Sequence

<220>
<223> Synthesized Hammerhead Ribozyme

<220>
<223> "n" represents stem II region of a HH ribozyme.

<400> 42

cagggcagcu gaugagncga aaggcucag                                    29



<210> 43
<211> 29
<212> RNA
<213> Artificial Sequence

<220>
<223> Synthesized Hammerhead Ribozyme

<220>
<223> "n" represents stem II region of a HH ribozyme.

<400> 43

gcagggcacu gaugagncga aaaggcuca                                    29



<210> 44
<211> 29
<212> RNA
<213> Artificial Sequence

<220>
<223> Synthesized Hammerhead Ribozyme

<220>
<223> "n" represents stem II region of a HH ribozyme.

<400> 44

agggugcacu gaugagncga aaccguguc                                    29



<210> 45
<211> 29
<212> RNA
<213> Artificial Sequence

<220>
<223> Synthesized Hammerhead Ribozyme

<220>
<223> "n" represents stem II region of a HH ribozyme.

<400> 45

uuggugugcu gaugagncga aagggucau                                    29

<210> 46
<211> 29
<212> RNA
<213> Artificial Sequence

<220>
<223> Synthesized Hammerhead Ribozyme

<220>
<223> "n" represents stem II region of a HH ribozyme.

<400> 46

ccaucccacu gaugagncga aaugcuuug                                    29

<210> 47
<211> 29
<212> RNA
<213> Artificial Sequence

<220>
<223> Synthesized Hammerhead Ribozyme

<220>
<223> "n" represents stem II region of a HH ribozyme.

<400> 47

ugaugcagcu gaugagncga aagggccau                                    29

<210> 48
<211> 29
<212> RNA
<213> Artificial Sequence

<220>
<223> Synthesized Hammerhead Ribozyme

<220>
<223> "n" represents stem II region of a HH ribozyme.

<400> 48

uuggaucucu gaugagncga aaugcagua                                    29

<210> 49

<211> 29
<212> RNA
<213> Artificial Sequence

<220>
<223> Synthesized Hammerhead Ribozyme

<220>
<223> "n" represents stem II region of a HH ribozyme.

<400> 49

uucccuugcu gaugagncga aaucugaug                                          29



<210> 50
<211> 29
<212> RNA
<213> Artificial Sequence

<220>
<223> Synthesized Hammerhead Ribozyme

<220>
<223> "n" represents stem II region of a HH ribozyme.

<400> 50

gagcugcgcu gaugagncga aacgguuca                                          29



<210> 51
<211> 29
<212> RNA
<213> Artificial Sequence

<220>
<223> Synthesized Hammerhead Ribozyme

<220>
<223> "n" represents stem II region of a HH ribozyme.

<400> 51

gggaucuucu gaugagncga aagcugcgg                                          29



<210> 52
<211> 29
<212> RNA
<213> Artificial Sequence

<220>
<223> Synthesized Hammerhead Ribozyme

<220>
<223> "n" represents stem II region of a HH ribozyme.

<400> 52

uccaggggcu gaugagncga aaucuugag                                          29

<210> 53
<211> 29
<212> RNA
<213> Artificial Sequence

<220>
<223> Synthesized Hammerhead Ribozyme

<220>
<223> "n" represents stem II region of a HH ribozyme.

<400> 53

uguccaggcu gaugagncga aacaccucg                                          29

<210> 54
<211> 29
<212> RNA
<213> Artificial Sequence

<220>
<223> Synthesized Hammerhead Ribozyme

<220>
<223> "n" represents stem II region of a HH ribozyme.

<400> 54

gguaguugcu gaugagncga aacacggcg                                          29

<210> 55
<211> 29
<212> RNA
<213> Artificial Sequence

<220>
<223> Synthesized Hammerhead Ribozyme

<220>
<223> "n" represents stem II region of a HH ribozyme.

<400> 55

ccucggggcu gaugagncga aaguuguac                                          29

<210> 56
<211> 29
<212> RNA
<213> Artificial Sequence

<220>
<223> Synthesized Hammerhead Ribozyme

<220>
<223> "n" represents stem II region of a HH ribozyme.

<400> 56

ugaacucgcu gaugagncga aaggcggcg 29

<210> 57
<211> 29
<212> RNA
<213> Artificial Sequence

<220>
<223> Synthesized Hammerhead Ribozyme

<220>
<223> "n" represents stem II region of a HH ribozyme.

<400> 57

cggcguugcu gaugagncga aacucguag 29

<210> 58
<211> 29
<212> RNA
<213> Artificial Sequence

<220>
<223> Synthesized Hammerhead Ribozyme

<220>
<223> "n" represents stem II region of a HH ribozyme.

<400> 58

gcggcguucu gaugagncga aaacucgua 29

<210> 59
<211> 29
<212> RNA
<213> Artificial Sequence

<220>
<223> Synthesized Hammerhead Ribozyme

<220>
<223> "n" represents stem II region of a HH ribozyme.

<400> 59

ugaccguacu gaugagncga aaccugcgc 29

<210> 60
<211> 29
<212> RNA
<213> Artificial Sequence

<220>
<223> Synthesized Hammerhead Ribozyme

<220>
<223> "n" represents stem II region of a HH ribozyme.

<400> 60

ucugaccgcu gaugagncga aagaccugc                                    29

<210> 61
<211> 29
<212> RNA
<213> Artificial Sequence

<220>
<223> Synthesized Hammerhead Ribozyme

<220>
<223> "n" represents stem II region of a HH ribozyme.

<400> 61

gccggucucu gaugagncga aaccguaga                                    29

<210> 62
<211> 29
<212> RNA
<213> Artificial Sequence

<220>
<223> Synthesized Hammerhead Ribozyme

<220>
<223> "n" represents stem II region of a HH ribozyme.

<400> 62

ccguagggcu gaugagncga aaggccggu                                    29

<210> 63
<211> 29
<212> RNA
<213> Artificial Sequence

<220>
<223> Synthesized Hammerhead Ribozyme

<220>
<223> "n" represents stem II region of a HH ribozyme.

<400> 63

cggggccgcu gaugagncga aaggggagg 29

<210> 64
<211> 29
<212> RNA
<213> Artificial Sequence

<220>
<223> Synthesized Hammerhead Ribozyme

<220>
<223> "n" represents stem II region of a HH ribozyme.

<400> 64

cagccucacu gaugagncga aacccgggg 29

<210> 65
<211> 29
<212> RNA
<213> Artificial Sequence

<220>
<223> Synthesized Hammerhead Ribozyme

<220>
<223> "n" represents stem II region of a HH ribozyme.

<400> 65

uggagccgcu gaugagncga aacgccgca 29

<210> 66
<211> 29
<212> RNA
<213> Artificial Sequence

<220>
<223> Synthesized Hammerhead Ribozyme

<220>
<223> "n" represents stem II region of a HH ribozyme.

<400> 66

uuggagcccu gaugagncga aaacgccgc 29

<210> 67

<211> 29
<212> RNA
<213> Artificial Sequence

<220>
<223> Synthesized Hammerhead Ribozyme

<220>
<223> "n" represents stem II region of a HH ribozyme.

<400> 67

ggccguugcu gaugagncga aagccgaac                                          29



<210> 68
<211> 29
<212> RNA
<213> Artificial Sequence

<220>
<223> Synthesized Hammerhead Ribozyme

<220>
<223> "n" represents stem II region of a HH ribozyme.

<400> 68

ugggggacu gaugagncga aaccccca                                            29



<210> 69
<211> 29
<212> RNA
<213> Artificial Sequence

<220>
<223> Synthesized Hammerhead Ribozyme

<220>
<223> "n" represents stem II region of a HH ribozyme.

<400> 69
   guggggggcu gaugagncga aaacccccc                                           29



<210> 70
<211> 29
<212> RNA
<213> Artificial Sequence

<220>
<223> Synthesized Hammerhead Ribozyme

<220>
<223> "n" represents stem II region of a HH ribozyme.

<400> 70

agugggggcu gaugagncga aaaaccccc                                          29



<210> 71
<211> 29
<212> RNA
<213> Artificial Sequence

<220>
<223> Synthesized Hammerhead Ribozyme

<220>
<223> "n" represents stem II region of a HH ribozyme.

<400> 71

acgcuguucu gaugagncga aaguggggg                                          29



<210> 72
<211> 29
<212> RNA
<213> Artificial Sequence

<220>
<223> Synthesized Hammerhead Ribozyme

<220>
<223> "n" represents stem II region of a HH ribozyme.

<400> 72

ggcucggacu gaugagncga aacacgcug                                          29



<210> 73
<211> 29
<212> RNA
<213> Artificial Sequence

<220>
<223> Synthesized Hammerhead Ribozyme

<220>
<223> "n" represents stem II region of a HH ribozyme.

<400> 73

cgggcucgcu gaugagncga aagacacgc                                          29



<210> 74
<211> 29
<212> RNA
<213> Artificial Sequence

<220>
<223> Synthesized Hammerhead Ribozyme

<220>
<223> "n" represents stem II region of a HH ribozyme.

<400> 74

gggugcagcu gaugagncga aagcaucag                                    29

<210> 75
<211> 29
<212> RNA
<213> Artificial Sequence

<220>
<223> Synthesized Hammerhead Ribozyme

<220>
<223> "n" represents stem II region of a HH ribozyme.

<400> 75

ggaaaggccu gaugagncga aacagcugc                                    29

<210> 76
<211> 29
<212> RNA
<213> Artificial Sequence

<220>
<223> Synthesized Hammerhead Ribozyme

<220>
<223> "n" represents stem II region of a HH ribozyme.

<400> 76

cugcaggacu gaugagncga aaggcgaca                                    29

<210> 77
<211> 29
<212> RNA
<213> Artificial Sequence

<220>
<223> Synthesized Hammerhead Ribozyme

<220>
<223> "n" represents stem II region of a HH ribozyme.

<400> 77

gcugcaggcu gaugagncga aaaggcgac                                    29

<210> 78
<211> 29
<212> RNA
<213> Artificial Sequence

<220>
<223> Synthesized Hammerhead Ribozyme

<220>
<223> "n" represents stem II region of a HH ribozyme.

<400> 78

ggcugcagcu gaugagncga aaaaggcga 29

<210> 79
<211> 29
<212> RNA
<213> Artificial Sequence

<220>
<223> Synthesized Hammerhead Ribozyme

<220>
<223> "n" represents stem II region of a HH ribozyme.

<400> 79

ccagguagcu gaugagncga aagggcacc 29

<210> 80
<211> 29
<212> RNA
<213> Artificial Sequence

<220>
<223> Synthesized Hammerhead Ribozyme

<220>
<223> "n" represents stem II region of a HH ribozyme.

<400> 80

ucuccaggcu gaugagncga aaguagggc 29

<210> 81
<211> 29
<212> RNA
<213> Artificial Sequence

<220>
<223> Synthesized Hammerhead Ribozyme

<220>
<223> "n" represents stem II region of a HH ribozyme.

<400> 81

gcaccgugcu gaugagncga aagccgcug                                    29

<210> 82
<211> 29
<212> RNA
<213> Artificial Sequence

<220>
<223> Synthesized Hammerhead Ribozyme

<220>
<223> "n" represents stem II region of a HH ribozyme.

<400> 82

gccuguagcu gaugagncga aaugccggc                                    29

<210> 83
<211> 29
<212> RNA
<213> Artificial Sequence

<220>
<223> Synthesized Hammerhead Ribozyme

<220>
<223> "n" represents stem II region of a HH ribozyme.

<400> 83

ggccuguacu gaugagncga aaaugccgg                                    29

<210> 84
<211> 29
<212> RNA
<213> Artificial Sequence

<220>
<223> Synthesized Hammerhead Ribozyme

<220>
<223> "n" represents stem II region of a HH ribozyme.

<400> 84

uuggccugcu gaugagncga aagaaugcc                                    29

<210> 85
<211> 29
<212> RNA
<213> Artificial Sequence

<220>
<223> Synthesized Hammerhead Ribozyme

<220>
<223> "n" represents stem II region of a HH ribozyme.

<400> 85

auuaucugcu gaugagncga aauuuggcc                                          29

<210> 86
<211> 29
<212> RNA
<213> Artificial Sequence

<220>
<223> Synthesized Hammerhead Ribozyme

<220>
<223> "n" represents stem II region of a HH ribozyme.

<400> 86

gauuaucucu gaugagncga aaauuuggc                                          29

<210> 87
<211> 29
<212> RNA
<213> Artificial Sequence

<220>
<223> Synthesized Hammerhead Ribozyme

<220>
<223> "n" represents stem II region of a HH ribozyme.

<400> 87

gcgucgaucu gaugagncga aaucugaau                                          29

<210> 88
<211> 29
<212> RNA
<213> Artificial Sequence

<220>
<223> Synthesized Hammerhead Ribozyme

<220>
<223> "n" represents stem II region of a HH ribozyme.

<400> 88

cuggcguccu gaugagncga aauuaucug                                          29

<210> 89
<211> 29
<212> RNA
<213> Artificial Sequence

<220>
<223> Synthesized Hammerhead Ribozyme

<220>
<223> "n" represents stem II region of a HH ribozyme.

<400> 89

uacuggcccu gaugagncga aaucuuucu                                          29

<210> 90
<211> 29
<212> RNA
<213> Artificial Sequence

<220>
<223> Synthesized Hammerhead Ribozyme

<220>
<223> "n" represents stem II region of a HH ribozyme.

<400> 90

gucauuggcu gaugagncga aacuggcca                                          29

<210> 91
<211> 29
<212> RNA
<213> Artificial Sequence

<220>
<223> Synthesized Hammerhead Ribozyme

<220>
<223> "n" represents stem II region of a HH ribozyme.

<400> 91

cauagccacu gaugagncga aacuucccu                                          29

<210> 92
<211> 29
<212> RNA

<213> Artificial Sequence

<220>
<223> Synthesized Hammerhead Ribozyme

<220>
<223> "n" represents stem II region of a HH ribozyme.

<400> 92

agauuccacu gaugagncga aagccauac                                          29

<210> 93
<211> 29
<212> RNA
<213> Artificial Sequence

<220>
<223> Synthesized Hammerhead Ribozyme

<220>
<223> "n" represents stem II region of a HH ribozyme.

<400> 93

ccuuggcacu gaugagncga aauuccaua                                          29

<210> 94
<211> 29
<212> RNA
<213> Artificial Sequence

<220>
<223> Synthesized Hammerhead Ribozyme

<220>
<223> "n" represents stem II region of a HH ribozyme.

<400> 94

acaguagccu gaugagncga aagucuccu                                          29

<210> 95
<211> 29
<212> RNA
<213> Artificial Sequence

<220>
<223> Synthesized Hammerhead Ribozyme

<220>
<223> "n" represents stem II region of a HH ribozyme.

<400> 95

cugcacagcu gaugagncga aagcgaguc                                    29

<210> 96
<211> 29
<212> RNA
<213> Artificial Sequence

<220>
<223> Synthesized Hammerhead Ribozyme

<220>
<223> "n" represents stem II region of a HH ribozyme.

<400> 96

cugaagcacu gaugagncga aagucauug                                    29

<210> 97
<211> 29
<212> RNA
<213> Artificial Sequence

<220>
<223> Synthesized Hammerhead Ribozyme

<220>
<223> "n" represents stem II region of a HH ribozyme.

<400> 97

guagccugcu gaugagncga aagcauagu                                    29

<210> 98
<211> 29
<212> RNA
<213> Artificial Sequence

<220>
<223> Synthesized Hammerhead Ribozyme

<220>
<223> "n" represents stem II region of a HH ribozyme.

<400> 98

gguagccucu gaugagncga aaagcauag                                    29

<210> 99
<211> 29
<212> RNA
<213> Artificial Sequence

<220>

<223> Synthesized Hammerhead Ribozyme

<220>
<223> "n" represents stem II region of a HH ribozyme.

<400> 99

cauaauggcu gaugagncga aagccugaa                                    29



<210> 100
<211> 29
<212> RNA
<213> Artificial Sequence

<220>
<223> Synthesized Hammerhead Ribozyme

<220>
<223> "n" represents stem II region of a HH ribozyme.

<400> 100

gacuccaucu gaugagncga aaugguagc                                    29



<210> 101
<211> 29
<212> RNA
<213> Artificial Sequence

<220>
<223> Synthesized Hammerhead Ribozyme

<220>
<223> "n" represents stem II region of a HH ribozyme.

<400> 101

agacuccacu gaugagncga aaaugguag                                    29



<210> 102
<211> 29
<212> RNA
<213> Artificial Sequence

<220>
<223> Synthesized Hammerhead Ribozyme

<220>
<223> "n" represents stem II region of a HH ribozyme.

<400> 102

caggaccacu gaugagncga aacuccaua                                    29

<210> 103
<211> 29
<212> RNA
<213> Artificial Sequence

<220>
<223> Synthesized Hammerhead Ribozyme

<220>
<223> "n" represents stem II region of a HH ribozyme.

<400> 103

ccucacagcu gaugagncga aaccagacu 29

<210> 104
<211> 29
<212> RNA
<213> Artificial Sequence

<220>
<223> Synthesized Hammerhead Ribozyme

<220>
<223> "n" represents stem II region of a HH ribozyme.

<400> 104

ucuugaagcu gaugagncga aaggccuug 29

<210> 105
<211> 29
<212> RNA
<213> Artificial Sequence

<220>
<223> Synthesized Hammerhead Ribozyme

<220>
<223> "n" represents stem II region of a HH ribozyme.

<400> 105

cucuugaacu gaugagncga aaaggccuu 29

<210> 106
<211> 29
<212> RNA
<213> Artificial Sequence

<220>
<223> Synthesized Hammerhead Ribozyme

<220>
<223> "n" represents stem II region of a HH ribozyme.

<400> 106

uucucuugcu gaugagncga aagaaggcc                                29

<210> 107
<211> 29
<212> RNA
<213> Artificial Sequence

<220>
<223> Synthesized Hammerhead Ribozyme

<220>
<223> "n" represents stem II region of a HH ribozyme.

<400> 107

cuucucuucu gaugagncga aaagaaggc                                29

<210> 108
<211> 29
<212> RNA
<213> Artificial Sequence

<220>
<223> Synthesized Hammerhead Ribozyme

<220>
<223> "n" represents stem II region of a HH ribozyme.

<400> 108

uccuugaacu gaugagncga aacuucucu                                29

<210> 109
<211> 29
<212> RNA
<213> Artificial Sequence

<220>
<223> Synthesized Hammerhead Ribozyme

<220>
<223> "n" represents stem II region of a HH ribozyme.

<400> 109

uguccuugcu gaugagncga aauacuucu                                29

<210> 110

<211> 29
<212> RNA
<213> Artificial Sequence

<220>
<223> Synthesized Hammerhead Ribozyme

<220>
<223> "n" represents stem II region of a HH ribozyme.

<400> 110

auguccuucu gaugagncga aaauacuuc                                29

<210> 111
<211> 29
<212> RNA
<213> Artificial Sequence

<220>
<223> Synthesized Hammerhead Ribozyme

<220>
<223> "n" represents stem II region of a HH ribozyme.

<400> 111

auagucgucu gaugagncga aauguccuu                                29

<210> 112
<211> 29
<212> RNA
<213> Artificial Sequence

<220>
<223> Synthesized Hammerhead Ribozyme

<220>
<223> "n" represents stem II region of a HH ribozyme.

<400> 112

gacacauacu gaugagncga aagucguua                                29

<210> 113
<211> 29
<212> RNA
<213> Artificial Sequence

<220>
<223> Synthesized Hammerhead Ribozyme

<220>
<223> "n" represents stem II region of a HH ribozyme.

<400> 113

uggacacacu gaugagncga aauagucgu                                          29



<210> 114
<211> 29
<212> RNA
<213> Artificial Sequence

<220>
<223> Synthesized Hammerhead Ribozyme

<220>
<223> "n" represents stem II region of a HH ribozyme.

<400> 114

gguggcugcu gaugagncga aacacauau                                          29



<210> 115
<211> 29
<212> RNA
<213> Artificial Sequence

<220>
<223> Synthesized Hammerhead Ribozyme

<220>
<223> "n" represents stem II region of a HH ribozyme.

<400> 115

uuuuuauccu gaugagncga aauggugca                                          29



<210> 116
<211> 29
<212> RNA
<213> Artificial Sequence

<220>
<223> Synthesized Hammerhead Ribozyme

<220>
<223> "n" represents stem II region of a HH ribozyme.

<400> 116

ccuguuucu gaugagncga aaucaaugg                                          29



<210> 117
<211> 29
<212> RNA
<213> Artificial Sequence

<220>
<223> Synthesized Hammerhead Ribozyme

<220>
<223> "n" represents stem II region of a HH ribozyme.

<400> 117

cauuugcgcu gaugagncga aagccggca                                    29



<210> 118
<211> 29
<212> RNA
<213> Artificial Sequence

<220>
<223> Synthesized Hammerhead Ribozyme

<220>
<223> "n" represents stem II region of a HH ribozyme.

<400> 118

ccacuucgcu gaugagncga aagcauuug                                    29



<210> 119
<211> 29
<212> RNA
<213> Artificial Sequence

<220>
<223> Synthesized Hammerhead Ribozyme

<220>
<223> "n" represents stem II region of a HH ribozyme.

<400> 119

ucuuuucgcu gaugagncga aaucccacc                                    29



<210> 120
<211> 29
<212> RNA
<213> Artificial Sequence

<220>
<223> Synthesized Hammerhead Ribozyme

<220>
<223> "n" represents stem II region of a HH ribozyme.

<400> 120

uguguuuccu gaugagncga aacauucuc                                    29

<210> 121
<211> 29
<212> RNA
<213> Artificial Sequence

<220>
<223> Synthesized Hammerhead Ribozyme

<220>
<223> "n" represents stem II region of a HH ribozyme.

<400> 121
cuccagcacu gaugagncga aaccccacu                                          29

<210> 122
<211> 29
<212> RNA
<213> Artificial Sequence

<220>
<223> Synthesized Hammerhead Ribozyme

<220>
<223> "n" represents stem II region of a HH ribozyme.

<400> 122
cuuggccacu gaugagncga aagguuggc                                          29

<210> 123
<211> 29
<212> RNA
<213> Artificial Sequence

<220>
<223> Synthesized Hammerhead Ribozyme

<220>
<223> "n" represents stem II region of a HH ribozyme.

<400> 123
gcuuggcccu gaugagncga aaagguugg                                          29

<210> 124
<211> 29
<212> RNA
<213> Artificial Sequence

<220>
<223> Synthesized Hammerhead Ribozyme

<220>
<223> "n" represents stem II region of a HH ribozyme.

<400> 124

uugaucaucu gaugagncga aagcgggcu                                    29

<210> 125
<211> 29
<212> RNA
<213> Artificial Sequence

<220>
<223> Synthesized Hammerhead Ribozyme

<220>
<223> "n" represents stem II region of a HH ribozyme.

<400> 125

gagcguuucu gaugagncga aaucaugag                                    29

<210> 126
<211> 29
<212> RNA
<213> Artificial Sequence

<220>
<223> Synthesized Hammerhead Ribozyme

<220>
<223> "n" represents stem II region of a HH ribozyme.

<400> 126

ucuucuuacu gaugagncga aagcguuug                                    29

<210> 127
<211> 29
<212> RNA
<213> Artificial Sequence

<220>
<223> Synthesized Hammerhead Ribozyme

<220>
<223> "n" represents stem II region of a HH ribozyme.

<400> 127

guucuucucu gaugagncga aagagcguu                                    29

<210> 128
<211> 29
<212> RNA
<213> Artificial Sequence

<220>
<223> Synthesized Hammerhead Ribozyme

<220>
<223> "n" represents stem II region of a HH ribozyme.

<400> 128

ucagggaccu gaugagncga aaggccagg                                          29

<210> 129
<211> 29
<212> RNA
<213> Artificial Sequence

<220>
<223> Synthesized Hammerhead Ribozyme

<220>
<223> "n" represents stem II region of a HH ribozyme.

<400> 129

ccgucaggcu gaugagncga aacaaggcc                                          29

<210> 130
<211> 29
<212> RNA
<213> Artificial Sequence

<220>
<223> Synthesized Hammerhead Ribozyme

<220>
<223> "n" represents stem II region of a HH ribozyme.

<400> 130

aaggcacucu gaugagncga aaccaucug                                          29

<210> 131
<211> 29
<212> RNA
<213> Artificial Sequence

<220>
<223> Synthesized Hammerhead Ribozyme

<220>
<223> "n" represents stem II region of a HH ribozyme.

<400> 131

cauccaaccu gaugagncga aaggcacug                                          29

<210> 132
<211> 29
<212> RNA
<213> Artificial Sequence

<220>
<223> Synthesized Hammerhead Ribozyme

<220>
<223> "n" represents stem II region of a HH ribozyme.

<400> 132

cagcaucccu gaugagncga aacaaggca                                          29

<210> 133
<211> 29
<212> RNA
<213> Artificial Sequence

<220>
<223> Synthesized Hammerhead Ribozyme

<220>
<223> "n" represents stem II region of a HH ribozyme.

<400> 133

gaauagagcu gaugagncga aaugggggg                                          29

<210> 134
<211> 29
<212> RNA
<213> Artificial Sequence

<220>
<223> Synthesized Hammerhead Ribozyme

<220>
<223> "n" represents stem II region of a HH ribozyme.

<400> 134

ucggaauacu gaugagncga aaguauggg                                          29

<210> 135
<211> 29
<212> RNA

<213> Artificial Sequence

<220>
<223> Synthesized Hammerhead Ribozyme

<220>
<223> "n" represents stem II region of a HH ribozyme.

<400> 135

acucggaacu gaugagncga aagaguaug                                          29

<210> 136
<211> 29
<212> RNA
<213> Artificial Sequence

<220>
<223> Synthesized Hammerhead Ribozyme

<220>
<223> "n" represents stem II region of a HH ribozyme.

<400> 136

auacucggcu gaugagncga aauagagua                                          29

<210> 137
<211> 29
<212> RNA
<213> Artificial Sequence

<220>
<223> Synthesized Hammerhead Ribozyme

<220>
<223> "n" represents stem II region of a HH ribozyme.

<400> 137

cauacucgcu gaugagncga aaauagagu                                          29

<210> 138
<211> 29
<212> RNA
<213> Artificial Sequence

<220>
<223> Synthesized Hammerhead Ribozyme

<220>
<223> "n" represents stem II region of a HH ribozyme.

<400> 138

uaggaucacu gaugagncga aacucggaa 29

<210> 139
<211> 29
<212> RNA
<213> Artificial Sequence

<220>
<223> Synthesized Hammerhead Ribozyme

<220>
<223> "n" represents stem II region of a HH ribozyme.

<400> 139

ucugguagcu gaugagncga aaucauacu 29

<210> 140
<211> 29
<212> RNA
<213> Artificial Sequence

<220>
<223> Synthesized Hammerhead Ribozyme

<220>
<223> "n" represents stem II region of a HH ribozyme.

<400> 140

gggucuggcu gaugagncga aaggaucau 29

<210> 141
<211> 29
<212> RNA
<213> Artificial Sequence

<220>
<223> Synthesized Hammerhead Ribozyme

<220>
<223> "n" represents stem II region of a HH ribozyme.

<400> 141

cuucacugcu gaugagncga aagggucug 29

<210> 142
<211> 29
<212> RNA
<213> Artificial Sequence

<220>

<223> Synthesized Hammerhead Ribozyme

<220>
<223> "n" represents stem II region of a HH ribozyme.

<400> 142

gcuucacucu gaugagncga aaagggucu                                    29



<210> 143
<211> 29
<212> RNA
<213> Artificial Sequence

<220>
<223> Synthesized Hammerhead Ribozyme

<220>
<223> "n" represents stem II region of a HH ribozyme.

<400> 143

caucaucgcu gaugagncga aagcuucac                                    29



<210> 144
<211> 29
<212> RNA
<213> Artificial Sequence

<220>
<223> Synthesized Hammerhead Ribozyme

<220>
<223> "n" represents stem II region of a HH ribozyme.

<400> 144

ccaucauccu gaugagncga aaagcuuca                                    29



<210> 145
<211> 29
<212> RNA
<213> Artificial Sequence

<220>
<223> Synthesized Hammerhead Ribozyme

<220>
<223> "n" represents stem II region of a HH ribozyme.

<400> 145

uggucagucu gaugagncga aagcccauc                                    29

<210> 146
<211> 29
<212> RNA
<213> Artificial Sequence

<220>
<223> Synthesized Hammerhead Ribozyme

<220>
<223> "n" represents stem II region of a HH ribozyme.

<400> 146

uuggucagcu gaugagncga aaagcccau                                          29

<210> 147
<211> 29
<212> RNA
<213> Artificial Sequence

<220>
<223> Synthesized Hammerhead Ribozyme

<220>
<223> "n" represents stem II region of a HH ribozyme.

<400> 147

aucaugugcu gaugagncga aaccagcuc                                          29

<210> 148
<211> 29
<212> RNA
<213> Artificial Sequence

<220>
<223> Synthesized Hammerhead Ribozyme

<220>
<223> "n" represents stem II region of a HH ribozyme.

<400> 148

gaucaugucu gaugagncga aaaccagcu                                          29

<210> 149
<211> 29
<212> RNA
<213> Artificial Sequence

<220>
<223> Synthesized Hammerhead Ribozyme

<220>

<223> "n" represents stem II region of a HH ribozyme.

<400> 149

gcccaguucu gaugagncga aaucaugug                                          29

<210> 150
<211> 29
<212> RNA
<213> Artificial Sequence

<220>
<223> Synthesized Hammerhead Ribozyme

<220>
<223> "n" represents stem II region of a HH ribozyme.

<400> 150

aauccacacu gaugagncga aagccuggc                                          29

<210> 151
<211> 29
<212> RNA
<213> Artificial Sequence

<220>
<223> Synthesized Hammerhead Ribozyme

<220>
<223> "n" represents stem II region of a HH ribozyme.

<400> 151

aaauccaccu gaugagncga aaagccugg                                          29

<210> 152
<211> 29
<212> RNA
<213> Artificial Sequence

<220>
<223> Synthesized Hammerhead Ribozyme

<220>
<223> "n" represents stem II region of a HH ribozyme.

<400> 152

gagggucacu gaugagncga aauccacaa                                          29

<210> 153
<211> 29

<212> RNA
<213> Artificial Sequence

<220>
<223> Synthesized Hammerhead Ribozyme

<220>
<223> "n" represents stem II region of a HH ribozyme.

<400> 153

ggaggguccu gaugagncga aaauccaca                                          29



<210> 154
<211> 29
<212> RNA
<213> Artificial Sequence

<220>
<223> Synthesized Hammerhead Ribozyme

<220>
<223> "n" represents stem II region of a HH ribozyme.

<400> 154

ugaucaugcu gaugagncga aagggucaa                                          29



<210> 155
<211> 29
<212> RNA
<213> Artificial Sequence

<220>
<223> Synthesized Hammerhead Ribozyme

<220>
<223> "n" represents stem II region of a HH ribozyme.

<400> 155

guggaccucu gaugagncga aaucaugga                                          29



<210> 156
<211> 29
<212> RNA
<213> Artificial Sequence

<220>
<223> Synthesized Hammerhead Ribozyme

<220>
<223> "n" represents stem II region of a HH ribozyme.

<400> 156

agaaggugcu gaugagncga aaccugauc                                    29

<210> 157
<211> 29
<212> RNA
<213> Artificial Sequence

<220>
<223> Synthesized Hammerhead Ribozyme

<220>
<223> "n" represents stem II region of a HH ribozyme.

<400> 157

cauucuagcu gaugagncga aagguggac                                    29

<210> 158
<211> 29
<212> RNA
<213> Artificial Sequence

<220>
<223> Synthesized Hammerhead Ribozyme

<220>
<223> "n" represents stem II region of a HH ribozyme.

<400> 158

acauucuacu gaugagncga aaaggugga                                    29

<210> 159
<211> 29
<212> RNA
<213> Artificial Sequence

<220>
<223> Synthesized Hammerhead Ribozyme

<220>
<223> "n" represents stem II region of a HH ribozyme.

<400> 159

gcacauuccu gaugagncga aagaaggug                                    29

<210> 160
<211> 29
<212> RNA
<213> Artificial Sequence

<220>
<223> Synthesized Hammerhead Ribozyme

<220>
<223> "n" represents stem II region of a HH ribozyme.

<400> 160

aggaucuccu gaugagncga aagccaggc                                          29



<210> 161
<211> 29
<212> RNA
<213> Artificial Sequence

<220>
<223> Synthesized Hammerhead Ribozyme

<220>
<223> "n" represents stem II region of a HH ribozyme.

<400> 161

aucaucagcu gaugagncga aaucucuag                                          29



<210> 162
<211> 29
<212> RNA
<213> Artificial Sequence

<220>
<223> Synthesized Hammerhead Ribozyme

<220>
<223> "n" represents stem II region of a HH ribozyme.

<400> 162

acgagacccu gaugagncga aaucaucag                                          29



<210> 163
<211> 29
<212> RNA
<213> Artificial Sequence

<220>
<223> Synthesized Hammerhead Ribozyme

<220>
<223> "n" represents stem II region of a HH ribozyme.

<400> 163

ccagacgacu gaugagncga aaccaauca                                          29

<210> 164
<211> 29
<212> RNA
<213> Artificial Sequence

<220>
<223> Synthesized Hammerhead Ribozyme

<220>
<223> "n" represents stem II region of a HH ribozyme.

<400> 164

cgccagaccu gaugagncga aagaccaau                                          29

<210> 165
<211> 29
<212> RNA
<213> Artificial Sequence

<220>
<223> Synthesized Hammerhead Ribozyme

<220>
<223> "n" represents stem II region of a HH ribozyme.

<400> 165

gagcgccacu gaugagncga aacgagacc                                          29

<210> 166
<211> 29
<212> RNA
<213> Artificial Sequence

<220>
<223> Synthesized Hammerhead Ribozyme

<220>
<223> "n" represents stem II region of a HH ribozyme.

<400> 166

gcuccaugcu gaugagncga aagcgccag                                          29

<210> 167
<211> 29
<212> RNA
<213> Artificial Sequence

<220>
<223> Synthesized Hammerhead Ribozyme

<220>
<223> "n" represents stem II region of a HH ribozyme.

<400> 167

gcaaacagcu gaugagncga aagcuucac                                    29

<210> 168
<211> 29
<212> RNA
<213> Artificial Sequence

<220>
<223> Synthesized Hammerhead Ribozyme

<220>
<223> "n" represents stem II region of a HH ribozyme.

<400> 168

uaggagcacu gaugagncga aacaguagc                                    29

<210> 169
<211> 29
<212> RNA
<213> Artificial Sequence

<220>
<223> Synthesized Hammerhead Ribozyme

<220>
<223> "n" represents stem II region of a HH ribozyme.

<400> 169

uuaggagccu gaugagncga aaacaguag                                    29

<210> 170
<211> 29
<212> RNA
<213> Artificial Sequence

<220>
<223> Synthesized Hammerhead Ribozyme

<220>
<223> "n" represents stem II region of a HH ribozyme.

<400> 170

caaguuagcu gaugagncga aagcaaaca                                    29

<210> 171

<211> 29
<212> RNA
<213> Artificial Sequence

<220>
<223> Synthesized Hammerhead Ribozyme

<220>
<223> "n" represents stem II region of a HH ribozyme.

<400> 171

gagcaagucu gaugagncga aaggagcaa                                          29



<210> 172
<211> 29
<212> RNA
<213> Artificial Sequence

<220>
<223> Synthesized Hammerhead Ribozyme

<220>
<223> "n" represents stem II region of a HH ribozyme.

<400> 172

ccaagagccu gaugagncga aaguuagga                                          29



<210> 173
<211> 29
<212> RNA
<213> Artificial Sequence

<220>
<223> Synthesized Hammerhead Ribozyme

<220>
<223> "n" represents stem II region of a HH ribozyme.

<400> 173

cuguccaacu gaugagncga aagcaaguu                                          29



<210> 174
<211> 29
<212> RNA
<213> Artificial Sequence

<220>
<223> Synthesized Hammerhead Ribozyme

<220>
<223> "n" represents stem II region of a HH ribozyme.

<400> 174
   uccugucccu gaugagncga aagagcaag                                    29

<210> 175
<211> 29
<212> RNA
<213> Artificial Sequence

<220>
<223> Synthesized Hammerhead Ribozyme

<220>
<223> "n" represents stem II region of a HH ribozyme.

<400> 175

augcccuccu gaugagncga aacacauuu                                    29

<210> 176
<211> 29
<212> RNA
<213> Artificial Sequence

<220>
<223> Synthesized Hammerhead Ribozyme

<220>
<223> "n" represents stem II region of a HH ribozyme.

<400> 176

augucgaacu gaugagncga aaucuccac                                    29

<210> 177
<211> 29
<212> RNA
<213> Artificial Sequence

<220>
<223> Synthesized Hammerhead Ribozyme

<220>
<223> "n" represents stem II region of a HH ribozyme.

<400> 177

gcaugucgcu gaugagncga aagaucucc                                    29

<210> 178
<211> 29
<212> RNA
<213> Artificial Sequence

<220>
<223> Synthesized Hammerhead Ribozyme

<220>
<223> "n" represents stem II region of a HH ribozyme.

<400> 178

agcauguccu gaugagncga aaagaucuc                                    29

<210> 179
<211> 29
<212> RNA
<213> Artificial Sequence

<220>
<223> Synthesized Hammerhead Ribozyme

<220>
<223> "n" represents stem II region of a HH ribozyme.

<400> 179

agaugaugcu gaugagncga aagccagca                                    29

<210> 180
<211> 29
<212> RNA
<213> Artificial Sequence

<220>
<223> Synthesized Hammerhead Ribozyme

<220>
<223> "n" represents stem II region of a HH ribozyme.

<400> 180

accgagaucu gaugagncga aauguagcc                                    29

<210> 181
<211> 29
<212> RNA
<213> Artificial Sequence

<220>
<223> Synthesized Hammerhead Ribozyme

<220>
<223> "n" represents stem II region of a HH ribozyme.

<400> 181

ggaaccgacu gaugagncga aaugaugua                                    29

<210> 182
<211> 29
<212> RNA
<213> Artificial Sequence

<220>
<223> Synthesized Hammerhead Ribozyme

<220>
<223> "n" represents stem II region of a HH ribozyme.

<400> 182

gcggaacccu gaugagncga aagaugaug                                29

<210> 183
<211> 29
<212> RNA
<213> Artificial Sequence

<220>
<223> Synthesized Hammerhead Ribozyme

<220>
<223> "n" represents stem II region of a HH ribozyme.

<400> 183

ucaugcggcu gaugagncga aaccgagau                                29

<210> 184
<211> 29
<212> RNA
<213> Artificial Sequence

<220>
<223> Synthesized Hammerhead Ribozyme

<220>
<223> "n" represents stem II region of a HH ribozyme.

<400> 184

aucaugcgcu gaugagncga aaaccgaga                                29

<210> 185
<211> 29
<212> RNA
<213> Artificial Sequence

<220>
<223> Synthesized Hammerhead Ribozyme

<220>
<223> "n" represents stem II region of a HH ribozyme.

<400> 185

ucccugcacu gaugagncga aauucauca 29

<210> 186
<211> 29
<212> RNA
<213> Artificial Sequence

<220>
<223> Synthesized Hammerhead Ribozyme

<220>
<223> "n" represents stem II region of a HH ribozyme.

<400> 186

ggcacacacu gaugagncga aacuccucu 29

<210> 187
<211> 29
<212> RNA
<213> Artificial Sequence

<220>
<223> Synthesized Hammerhead Ribozyme

<220>
<223> "n" represents stem II region of a HH ribozyme.

<400> 187

aggcacaccu gaugagncga aaacuccuc 29

<210> 188
<211> 29
<212> RNA
<213> Artificial Sequence

<220>
<223> Synthesized Hammerhead Ribozyme

<220>
<223> "n" represents stem II region of a HH ribozyme.

<400> 188

auagauuucu gaugagncga aaggcacac 29

<210> 189
<211> 29
<212> RNA
<213> Artificial Sequence

<220>
<223> Synthesized Hammerhead Ribozyme

<220>
<223> "n" represents stem II region of a HH ribozyme.

<400> 189

aaauaauacu gaugagncga aauuugagg                                29

<210> 190
<211> 29
<212> RNA
<213> Artificial Sequence

<220>
<223> Synthesized Hammerhead Ribozyme

<220>
<223> "n" represents stem II region of a HH ribozyme.

<400> 190

caaaauaacu gaugagncga aagauuuga                                29

<210> 191
<211> 29
<212> RNA
<213> Artificial Sequence

<220>
<223> Synthesized Hammerhead Ribozyme

<220>
<223> "n" represents stem II region of a HH ribozyme.

<400> 191

agcaaaaucu gaugagncga aauagauuu                                29

<210> 192
<211> 29
<212> RNA
<213> Artificial Sequence

<220>
<223> Synthesized Hammerhead Ribozyme

<220>
<223> "n" represents stem II region of a HH ribozyme.

<400> 192

aagcaaaacu gaugagncga aaauagauu                                    29

<210> 193
<211> 29
<212> RNA
<213> Artificial Sequence

<220>
<223> Synthesized Hammerhead Ribozyme

<220>
<223> "n" represents stem II region of a HH ribozyme.

<400> 193

uuaagcaacu gaugagncga aauaauaga                                    29

<210> 194
<211> 29
<212> RNA
<213> Artificial Sequence

<220>
<223> Synthesized Hammerhead Ribozyme

<220>
<223> "n" represents stem II region of a HH ribozyme.

<400> 194

auuaagcacu gaugagncga aaauaauag                                    29

<210> 195
<211> 29
<212> RNA
<213> Artificial Sequence

<220>
<223> Synthesized Hammerhead Ribozyme

<220>
<223> "n" represents stem II region of a HH ribozyme.

<400> 195

aauuaagccu gaugagncga aaaauaaua                                    29

<210> 196
<211> 29
<212> RNA

<213> Artificial Sequence

<220>
<223> Synthesized Hammerhead Ribozyme

<220>
<223> "n" represents stem II region of a HH ribozyme.

<400> 196

ccagaauucu gaugagncga aagcaaaau                                    29

<210> 197
<211> 29
<212> RNA
<213> Artificial Sequence

<220>
<223> Synthesized Hammerhead Ribozyme

<220>
<223> "n" represents stem II region of a HH ribozyme.

<400> 197

uccagaaucu gaugagncga aaagcaaaa                                    29

<210> 198
<211> 29
<212> RNA
<213> Artificial Sequence

<220>
<223> Synthesized Hammerhead Ribozyme

<220>
<223> "n" represents stem II region of a HH ribozyme.

<400> 198

cacuccagcu gaugagncga aauuaagca                                    29

<210> 199
<211> 29
<212> RNA
<213> Artificial Sequence

<220>
<223> Synthesized Hammerhead Ribozyme

<220>
<223> "n" represents stem II region of a HH ribozyme.

<400> 199

acacuccacu gaugagncga aaauuaagc 29

<210> 200
<211> 29
<212> RNA
<213> Artificial Sequence

<220>
<223> Synthesized Hammerhead Ribozyme

<220>
<223> "n" represents stem II region of a HH ribozyme.

<400> 200

gaaaugugcu gaugagncga aacacucca 29

<210> 201
<211> 29
<212> RNA
<213> Artificial Sequence

<220>
<223> Synthesized Hammerhead Ribozyme

<220>
<223> "n" represents stem II region of a HH ribozyme.

<400> 201

uggacagacu gaugagncga aauguguac 29

<210> 202
<211> 29
<212> RNA
<213> Artificial Sequence

<220>
<223> Synthesized Hammerhead Ribozyme

<220>
<223> "n" represents stem II region of a HH ribozyme.

<400> 202

cuggacagcu gaugagncga aaaugugua 29

<210> 203
<211> 29
<212> RNA
<213> Artificial Sequence

<220>

<223> Synthesized Hammerhead Ribozyme

<220>
<223> "n" represents stem II region of a HH ribozyme.

<400> 203

gcuggacacu gaugagncga aaaaugugu 29

<210> 204
<211> 29
<212> RNA
<213> Artificial Sequence

<220>
<223> Synthesized Hammerhead Ribozyme

<220>
<223> "n" represents stem II region of a HH ribozyme.

<400> 204

gggugcugcu gaugagncga aacagaaau 29

<210> 205
<211> 29
<212> RNA
<213> Artificial Sequence

<220>
<223> Synthesized Hammerhead Ribozyme

<220>
<223> "n" represents stem II region of a HH ribozyme.

<400> 205

cuuccagacu gaugagncga aacuucagg 29

<210> 206
<211> 29
<212> RNA
<213> Artificial Sequence

<220>
<223> Synthesized Hammerhead Ribozyme

<220>
<223> "n" represents stem II region of a HH ribozyme.

<400> 206

cucuuccacu gaugagncga aagacuuca 29

<210> 207
<211> 29
<212> RNA
<213> Artificial Sequence

<220>
<223> Synthesized Hammerhead Ribozyme

<220>
<223> "n" represents stem II region of a HH ribozyme.

<400> 207

ucgguggacu gaugagncga aaugguccu                                        29

<210> 208
<211> 29
<212> RNA
<213> Artificial Sequence

<220>
<223> Synthesized Hammerhead Ribozyme

<220>
<223> "n" represents stem II region of a HH ribozyme.

<400> 208

acucggugcu gaugagncga aauaugguc                                        29

<210> 209
<211> 29
<212> RNA
<213> Artificial Sequence

<220>
<223> Synthesized Hammerhead Ribozyme

<220>
<223> "n" represents stem II region of a HH ribozyme.

<400> 209

uuguccagcu gaugagncga aacucggug                                        29

<210> 210
<211> 29
<212> RNA
<213> Artificial Sequence

<220>
<223> Synthesized Hammerhead Ribozyme

<220>

<223> "n" represents stem II region of a HH ribozyme.

<400> 210

gugucugucu gaugagncga aaucuuguc                                    29

<210> 211
<211> 29
<212> RNA
<213> Artificial Sequence

<220>
<223> Synthesized Hammerhead Ribozyme

<220>
<223> "n" represents stem II region of a HH ribozyme.

<400> 211

guggaucacu gaugagncga aagugucug                                    29

<210> 212
<211> 29
<212> RNA
<213> Artificial Sequence

<220>
<223> Synthesized Hammerhead Ribozyme

<220>
<223> "n" represents stem II region of a HH ribozyme.

<400> 212

gguggauccu gaugagncga aaagugucu                                    29

<210> 213
<211> 29
<212> RNA
<213> Artificial Sequence

<220>
<223> Synthesized Hammerhead Ribozyme

<220>
<223> "n" represents stem II region of a HH ribozyme.

<400> 213

aucaggugcu gaugagncga aaucaaagu                                    29

<210> 214
<211> 29

<212> RNA
<213> Artificial Sequence

<220>
<223> Synthesized Hammerhead Ribozyme

<220>
<223> "n" represents stem II region of a HH ribozyme.

<400> 214

augaggagcu gaugagncga aagcugggc                                          29



<210> 215
<211> 29
<212> RNA
<213> Artificial Sequence

<220>
<223> Synthesized Hammerhead Ribozyme

<220>
<223> "n" represents stem II region of a HH ribozyme.

<400> 215

aggaugagcu gaugagncga aaggagcug                                          29



<210> 216
<211> 29
<212> RNA
<213> Artificial Sequence

<220>
<223> Synthesized Hammerhead Ribozyme

<220>
<223> "n" represents stem II region of a HH ribozyme.

<400> 216

gagaggaucu gaugagncga aaggaggag                                          29



<210> 217
<211> 29
<212> RNA
<213> Artificial Sequence

<220>
<223> Synthesized Hammerhead Ribozyme

<220>
<223> "n" represents stem II region of a HH ribozyme.

<400> 217

ugggagagcu gaugagncga aaugaggag 29

<210> 218
<211> 29
<212> RNA
<213> Artificial Sequence

<220>
<223> Synthesized Hammerhead Ribozyme

<220>
<223> "n" represents stem II region of a HH ribozyme.

<400> 218

augugggacu gaugagncga aaggaugag 29

<210> 219
<211> 29
<212> RNA
<213> Artificial Sequence

<220>
<223> Synthesized Hammerhead Ribozyme

<220>
<223> "n" represents stem II region of a HH ribozyme.

<400> 219

ugauguggcu gaugagncga aagaggaug 29

<210> 220
<211> 29
<212> RNA
<213> Artificial Sequence

<220>
<223> Synthesized Hammerhead Ribozyme

<220>
<223> "n" represents stem II region of a HH ribozyme.

<400> 220

augugccucu gaugagncga aauguggga 29

<210> 221
<211> 29
<212> RNA
<213> Artificial Sequence

<220>
<223> Synthesized Hammerhead Ribozyme

<220>
<223> "n" represents stem II region of a HH ribozyme.

<400> 221

gccuuugucu gaugagncga aacucaugu 29

<210> 222
<211> 29
<212> RNA
<213> Artificial Sequence

<220>
<223> Synthesized Hammerhead Ribozyme

<220>
<223> "n" represents stem II region of a HH ribozyme.

<400> 222

gcuguacacu gaugagncga aaugcucca 29

<210> 223
<211> 29
<212> RNA
<213> Artificial Sequence

<220>
<223> Synthesized Hammerhead Ribozyme

<220>
<223> "n" represents stem II region of a HH ribozyme.

<400> 223

ucaugcugcu gaugagncga aacagaugc 29

<210> 224
<211> 29
<212> RNA
<213> Artificial Sequence

<220>
<223> Synthesized Hammerhead Ribozyme

<220>
<223> "n" represents stem II region of a HH ribozyme.

<400> 224

aggucauacu gaugagncga aaggggcac 29

<210> 225
<211> 29
<212> RNA
<213> Artificial Sequence

<220>
<223> Synthesized Hammerhead Ribozyme

<220>
<223> "n" represents stem II region of a HH ribozyme.

<400> 225

gcaggucacu gaugagncga aagaggggc                                29

<210> 226
<211> 29
<212> RNA
<213> Artificial Sequence

<220>
<223> Synthesized Hammerhead Ribozyme

<220>
<223> "n" represents stem II region of a HH ribozyme.

<400> 226

ggcgcaugcu gaugagncga aaggcggug                                29

<210> 227
<211> 29
<212> RNA
<213> Artificial Sequence

<220>
<223> Synthesized Hammerhead Ribozyme

<220>
<223> "n" represents stem II region of a HH ribozyme.

<400> 227

uccacggccu gaugagncga aagugggcg                                29

<210> 228
<211> 29
<212> RNA
<213> Artificial Sequence

<220>
<223> Synthesized Hammerhead Ribozyme

<220>
<223> "n" represents stem II region of a HH ribozyme.

<400> 228

ccuccacgcu gaugagncga aaugccccu                                    29

<210> 229
<211> 29
<212> RNA
<213> Artificial Sequence

<220>
<223> Synthesized Hammerhead Ribozyme

<220>
<223> "n" represents stem II region of a HH ribozyme.

<400> 229

caguggcccu gaugagncga aaguggcuu                                    29

<210> 230
<211> 29
<212> RNA
<213> Artificial Sequence

<220>
<223> Synthesized Hammerhead Ribozyme

<220>
<223> "n" represents stem II region of a HH ribozyme.

<400> 230

augaaguacu gaugagncga aagcccgca                                    29

<210> 231
<211> 29
<212> RNA
<213> Artificial Sequence

<220>
<223> Synthesized Hammerhead Ribozyme

<220>
<223> "n" represents stem II region of a HH ribozyme.

<400> 231

cgaugaagcu gaugagncga aagagcccg                                    29

<210> 232

<211> 29
<212> RNA
<213> Artificial Sequence

<220>
<223> Synthesized Hammerhead Ribozyme

<220>
<223> "n" represents stem II region of a HH ribozyme.

<400> 232

augcgaugcu gaugagncga aaguagagc                                          29

<210> 233
<211> 29
<212> RNA
<213> Artificial Sequence

<220>
<223> Synthesized Hammerhead Ribozyme

<220>
<223> "n" represents stem II region of a HH ribozyme.

<400> 233

aaugcgaucu gaugagncga aaaguagag                                          29

<210> 234
<211> 29
<212> RNA
<213> Artificial Sequence

<220>
<223> Synthesized Hammerhead Ribozyme

<220>
<223> "n" represents stem II region of a HH ribozyme.

<400> 234

aggaaugccu gaugagncga aaugaagua                                          29

<210> 235
<211> 29
<212> RNA
<213> Artificial Sequence

<220>
<223> Synthesized Hammerhead Ribozyme

<220>
<223> "n" represents stem II region of a HH ribozyme.

<400> 235

uugcaaggcu gaugagncga aaugcgaug                                          29

<210> 236
<211> 29
<212> RNA
<213> Artificial Sequence

<220>
<223> Synthesized Hammerhead Ribozyme

<220>
<223> "n" represents stem II region of a HH ribozyme.

<400> 236

uuugcaagcu gaugagncga aaaugcgau                                          29

<210> 237
<211> 29
<212> RNA
<213> Artificial Sequence

<220>
<223> Synthesized Hammerhead Ribozyme

<220>
<223> "n" represents stem II region of a HH ribozyme.

<400> 237

acuuuugccu gaugagncga aaggaaugc                                          29

<210> 238
<211> 29
<212> RNA
<213> Artificial Sequence

<220>
<223> Synthesized Hammerhead Ribozyme

<220>
<223> "n" represents stem II region of a HH ribozyme.

<400> 238

ugauguaacu gaugagncga aacuuuugc                                          29

<210> 239
<211> 29
<212> RNA
<213> Artificial Sequence

<220>
<223> Synthesized Hammerhead Ribozyme

<220>
<223> "n" represents stem II region of a HH ribozyme.

<400> 239

cgugaugucu gaugagncga aauacuuuu                                          29



<210> 240
<211> 29
<212> RNA
<213> Artificial Sequence

<220>
<223> Synthesized Hammerhead Ribozyme

<220>
<223> "n" represents stem II region of a HH ribozyme.

<400> 240

ccgugaugcu gaugagncga aaauacuuu                                          29



<210> 241
<211> 29
<212> RNA
<213> Artificial Sequence

<220>
<223> Synthesized Hammerhead Ribozyme

<220>
<223> "n" represents stem II region of a HH ribozyme.

<400> 241

ucccccgucu gaugagncga aauguaaua                                          29



<210> 242
<211> 29
<212> RNA
<213> Artificial Sequence

<220>
<223> Synthesized Hammerhead Ribozyme

<220>
<223> "n" represents stem II region of a HH ribozyme.

<400> 242

ggcagggacu gaugagncga aacccucug                                          29

<210> 243
<211> 29
<212> RNA
<213> Artificial Sequence

<220>
<223> Synthesized Hammerhead Ribozyme

<220>
<223> "n" represents stem II region of a HH ribozyme.

<400> 243

uggcagggcu gaugagncga aaacccucu                                    29



<210> 244
<211> 29
<212> RNA
<213> Artificial Sequence

<220>
<223> Synthesized Hammerhead Ribozyme

<220>
<223> "n" represents stem II region of a HH ribozyme.

<400> 244

guggcaggcu gaugagncga aaaacccuc                                    29



<210> 245
<211> 29
<212> RNA
<213> Artificial Sequence

<220>
<223> Synthesized Hammerhead Ribozyme

<220>
<223> "n" represents stem II region of a HH ribozyme.

<400> 245

agcucucacu gaugagncga aacuguggc                                    29



<210> 246
<211> 29
<212> RNA
<213> Artificial Sequence

<220>
<223> Synthesized Hammerhead Ribozyme

<220>
<223> "n" represents stem II region of a HH ribozyme.

<400> 246

gagccaggcu gaugagncga aagcucuca 29

<210> 247
<211> 29
<212> RNA
<213> Artificial Sequence

<220>
<223> Synthesized Hammerhead Ribozyme

<220>
<223> "n" represents stem II region of a HH ribozyme.

<400> 247

ccguguggcu gaugagncga aagccaggg 29

<210> 248
<211> 29
<212> RNA
<213> Artificial Sequence

<220>
<223> Synthesized Hammerhead Ribozyme

<220>
<223> "n" represents stem II region of a HH ribozyme.

<400> 248

auuaucugcu gaugagncga aaccgugug 29

<210> 249
<211> 29
<212> RNA
<213> Artificial Sequence

<220>
<223> Synthesized Hammerhead Ribozyme

<220>
<223> "n" represents stem II region of a HH ribozyme.

<400> 249

gauuaucucu gaugagncga aaaccgugu 29

<210> 250
<211> 29
<212> RNA
<213> Artificial Sequence

<220>
<223> Synthesized Hammerhead Ribozyme

<220>
<223> "n" represents stem II region of a HH ribozyme.

<400> 250

gcagggaucu gaugagncga aaucugaac                                    29

<210> 251
<211> 29
<212> RNA
<213> Artificial Sequence

<220>
<223> Synthesized Hammerhead Ribozyme

<220>
<223> "n" represents stem II region of a HH ribozyme.

<400> 251

gcagcaggcu gaugagncga aauuaucug                                    29

<210> 252
<211> 29
<212> RNA
<213> Artificial Sequence

<220>
<223> Synthesized Hammerhead Ribozyme

<220>
<223> "n" represents stem II region of a HH ribozyme.

<400> 252

gaggguaacu gaugagncga aaugcagca                                    29

<210> 253
<211> 29
<212> RNA
<213> Artificial Sequence

<220>
<223> Synthesized Hammerhead Ribozyme

<220>
<223> "n" represents stem II region of a HH ribozyme.

<400> 253

ugaggguacu gaugagncga aaaugcagc                                    29

<210> 254
<211> 29
<212> RNA
<213> Artificial Sequence

<220>
<223> Synthesized Hammerhead Ribozyme

<220>
<223> "n" represents stem II region of a HH ribozyme.

<400> 254

augagggucu gaugagncga aaaaugcag                                    29

<210> 255
<211> 29
<212> RNA
<213> Artificial Sequence

<220>
<223> Synthesized Hammerhead Ribozyme

<220>
<223> "n" represents stem II region of a HH ribozyme.

<400> 255

gaugagggcu gaugagncga aaaaaugca                                    29

<210> 256
<211> 29
<212> RNA
<213> Artificial Sequence

<220>
<223> Synthesized Hammerhead Ribozyme

<220>
<223> "n" represents stem II region of a HH ribozyme.

<400> 256

ugcaugaucu gaugagncga aaggguaaa                                    29

<210> 257
<211> 29
<212> RNA

<213> Artificial Sequence

<220>
<223> Synthesized Hammerhead Ribozyme

<220>
<223> "n" represents stem II region of a HH ribozyme.

<400> 257

uggugcaucu gaugagncga aaugagggu                                          29



<210> 258
<211> 29
<212> RNA
<213> Artificial Sequence

<220>
<223> Synthesized Hammerhead Ribozyme

<220>
<223> "n" represents stem II region of a HH ribozyme.

<400> 258

uuuggcuacu gaugagncga aaguggugc                                          29



<210> 259
<211> 29
<212> RNA
<213> Artificial Sequence

<220>
<223> Synthesized Hammerhead Ribozyme

<220>
<223> "n" represents stem II region of a HH ribozyme.

<400> 259

auuuggcucu gaugagncga aaaguggug                                          29



<210> 260
<211> 29
<212> RNA
<213> Artificial Sequence

<220>
<223> Synthesized Hammerhead Ribozyme

<220>
<223> "n" represents stem II region of a HH ribozyme.

<400> 260

aauuuggccu gaugagncga aaaaguggu 29

<210> 261
<211> 29
<212> RNA
<213> Artificial Sequence

<220>
<223> Synthesized Hammerhead Ribozyme

<220>
<223> "n" represents stem II region of a HH ribozyme.

<400> 261

ggagacagcu gaugagncga aauuuggcu 29

<210> 262
<211> 29
<212> RNA
<213> Artificial Sequence

<220>
<223> Synthesized Hammerhead Ribozyme

<220>
<223> "n" represents stem II region of a HH ribozyme.

<400> 262

aggagacacu gaugagncga aaauuuggc 29

<210> 263
<211> 29
<212> RNA
<213> Artificial Sequence

<220>
<223> Synthesized Hammerhead Ribozyme

<220>
<223> "n" represents stem II region of a HH ribozyme.

<400> 263

augcaggacu gaugagncga aacagaauu 29

<210> 264
<211> 29
<212> RNA
<213> Artificial Sequence

<220>

<223> Synthesized Hammerhead Ribozyme

<220>
<223> "n" represents stem II region of a HH ribozyme.

<400> 264

guaugcagcu gaugagncga aagacagaa                                          29

<210> 265
<211> 29
<212> RNA
<213> Artificial Sequence

<220>
<223> Synthesized Hammerhead Ribozyme

<220>
<223> "n" represents stem II region of a HH ribozyme.

<400> 265

ccggagugcu gaugagncga aaugcagga                                          29

<210> 266
<211> 29
<212> RNA
<213> Artificial Sequence

<220>
<223> Synthesized Hammerhead Ribozyme

<220>
<223> "n" represents stem II region of a HH ribozyme.

<400> 266

gcaugccgcu gaugagncga aaguguaug                                          29

<210> 267
<211> 29
<212> RNA
<213> Artificial Sequence

<220>
<223> Synthesized Hammerhead Ribozyme

<220>
<223> "n" represents stem II region of a HH ribozyme.

<400> 267

ugguguugcu gaugagncga aaugcaugc                                          29

<210> 268
<211> 29
<212> RNA
<213> Artificial Sequence

<220>
<223> Synthesized Hammerhead Ribozyme

<220>
<223> "n" represents stem II region of a HH ribozyme.

<400> 268

caucuagacu gaugagncga aagccauug                                          29

<210> 269
<211> 29
<212> RNA
<213> Artificial Sequence

<220>
<223> Synthesized Hammerhead Ribozyme

<220>
<223> "n" represents stem II region of a HH ribozyme.

<400> 269

ucaucuagcu gaugagncga aaagccauu                                          29

<210> 270
<211> 29
<212> RNA
<213> Artificial Sequence

<220>
<223> Synthesized Hammerhead Ribozyme

<220>
<223> "n" represents stem II region of a HH ribozyme.

<400> 270

cucaucuacu gaugagncga aaaagccau                                          29

<210> 271
<211> 29
<212> RNA
<213> Artificial Sequence

<220>
<223> Synthesized Hammerhead Ribozyme

<220>

<223> "n" represents stem II region of a HH ribozyme.

<400> 271

cacucauccu gaugagncga aagaaagcc                                    29

<210> 272
<211> 29
<212> RNA
<213> Artificial Sequence

<220>
<223> Synthesized Hammerhead Ribozyme

<220>
<223> "n" represents stem II region of a HH ribozyme.

<400> 272

agcaaaugcu gaugagncga aauggccac                                    29

<210> 273
<211> 29
<212> RNA
<213> Artificial Sequence

<220>
<223> Synthesized Hammerhead Ribozyme

<220>
<223> "n" represents stem II region of a HH ribozyme.

<400> 273

aagcaaaucu gaugagncga aaauggcca                                    29

<210> 274
<211> 29
<212> RNA
<213> Artificial Sequence

<220>
<223> Synthesized Hammerhead Ribozyme

<220>
<223> "n" represents stem II region of a HH ribozyme.

<400> 274

agcaagcacu gaugagncga aaugaaugg                                    29

<210> 275
<211> 29

<212> RNA
<213> Artificial Sequence

<220>
<223> Synthesized Hammerhead Ribozyme

<220>
<223> "n" represents stem II region of a HH ribozyme.

<400> 275

gagcaagccu gaugagncga aaaugaaug                                    29


<210> 276
<211> 29
<212> RNA
<213> Artificial Sequence

<220>
<223> Synthesized Hammerhead Ribozyme

<220>
<223> "n" represents stem II region of a HH ribozyme.

<400> 276

aacugagccu gaugagncga aagcaaaug                                    29


<210> 277
<211> 29
<212> RNA
<213> Artificial Sequence

<220>
<223> Synthesized Hammerhead Ribozyme

<220>
<223> "n" represents stem II region of a HH ribozyme.

<400> 277

uaagaacucu gaugagncga aagcaagca                                    29


<210> 278
<211> 29
<212> RNA
<213> Artificial Sequence

<220>
<223> Synthesized Hammerhead Ribozyme

<220>
<223> "n" represents stem II region of a HH ribozyme.

<400> 278

ccacuaagcu gaugagncga aacugagca                                    29

<210> 279
<211> 29
<212> RNA
<213> Artificial Sequence

<220>
<223> Synthesized Hammerhead Ribozyme

<220>
<223> "n" represents stem II region of a HH ribozyme.

<400> 279

gccacuaacu gaugagncga aaacugagc                                    29

<210> 280
<211> 29
<212> RNA
<213> Artificial Sequence

<220>
<223> Synthesized Hammerhead Ribozyme

<220>
<223> "n" represents stem II region of a HH ribozyme.

<400> 280

gugccacucu gaugagncga aagaacuga                                    29

<210> 281
<211> 29
<212> RNA
<213> Artificial Sequence

<220>
<223> Synthesized Hammerhead Ribozyme

<220>
<223> "n" represents stem II region of a HH ribozyme.

<400> 281

ugugccaccu gaugagncga aaagaacug                                    29

<210> 282
<211> 29
<212> RNA
<213> Artificial Sequence

<220>

<223> Synthesized Hammerhead Ribozyme

<220>
<223> "n" represents stem II region of a HH ribozyme.

<400> 282

agacagaacu gaugagncga aaugugcca                                          29



<210> 283
<211> 29
<212> RNA
<213> Artificial Sequence

<220>
<223> Synthesized Hammerhead Ribozyme

<220>
<223> "n" represents stem II region of a HH ribozyme.

<400> 283

gaagacagcu gaugagncga aagaugugc                                          29



<210> 284
<211> 29
<212> RNA
<213> Artificial Sequence

<220>
<223> Synthesized Hammerhead Ribozyme

<220>
<223> "n" represents stem II region of a HH ribozyme.

<400> 284

agaagacacu gaugagncga aaagaugug                                          29



<210> 285
<211> 29
<212> RNA
<213> Artificial Sequence

<220>
<223> Synthesized Hammerhead Ribozyme

<220>
<223> "n" represents stem II region of a HH ribozyme.

<400> 285

caacagaacu gaugagncga aacagaaga                                          29

<210> 286
<211> 29
<212> RNA
<213> Artificial Sequence

<220>
<223> Synthesized Hammerhead Ribozyme

<220>
<223> "n" represents stem II region of a HH ribozyme.

<400> 286

cccaacagcu gaugagncga aagacagaa                                    29



<210> 287
<211> 29
<212> RNA
<213> Artificial Sequence

<220>
<223> Synthesized Hammerhead Ribozyme

<220>
<223> "n" represents stem II region of a HH ribozyme.

<400> 287

ucccaacacu gaugagncga aaagacaga                                    29



<210> 288
<211> 29
<212> RNA
<213> Artificial Sequence

<220>
<223> Synthesized Hammerhead Ribozyme

<220>
<223> "n" represents stem II region of a HH ribozyme.

<400> 288

cuguuccccu gaugagncga aacagaaga                                    29



<210> 289
<211> 29
<212> RNA
<213> Artificial Sequence

<220>
<223> Synthesized Hammerhead Ribozyme

<220>

<223> "n" represents stem II region of a HH ribozyme.

<400> 289

agccuuggcu gaugagncga aaucccuuu                                    29

<210> 290
<211> 29
<212> RNA
<213> Artificial Sequence

<220>
<223> Synthesized Hammerhead Ribozyme

<220>
<223> "n" represents stem II region of a HH ribozyme.

<400> 290

uagccuugcu gaugagncga aaaucccuu                                    29

<210> 291
<211> 29
<212> RNA
<213> Artificial Sequence

<220>
<223> Synthesized Hammerhead Ribozyme

<220>
<223> "n" represents stem II region of a HH ribozyme.

<400> 291

caaagauucu gaugagncga aagccuugg                                    29

<210> 292
<211> 29
<212> RNA
<213> Artificial Sequence

<220>
<223> Synthesized Hammerhead Ribozyme

<220>
<223> "n" represents stem II region of a HH ribozyme.

<400> 292

guuacaaacu gaugagncga aauuuagcc                                    29

<210> 293
<211> 29

<212> RNA
<213> Artificial Sequence

<220>
<223> Synthesized Hammerhead Ribozyme

<220>
<223> "n" represents stem II region of a HH ribozyme.

<400> 293

cuguuacacu gaugagncga aagauuuag                                          29

<210> 294
<211> 29
<212> RNA
<213> Artificial Sequence

<220>
<223> Synthesized Hammerhead Ribozyme

<220>
<223> "n" represents stem II region of a HH ribozyme.

<400> 294

gcuguuaccu gaugagncga aaagauuua                                          29

<210> 295
<211> 29
<212> RNA
<213> Artificial Sequence

<220>
<223> Synthesized Hammerhead Ribozyme

<220>
<223> "n" represents stem II region of a HH ribozyme.

<400> 295

agagcugucu gaugagncga aacaaagau                                          29

<210> 296
<211> 29
<212> RNA
<213> Artificial Sequence

<220>
<223> Synthesized Hammerhead Ribozyme

<220>
<223> "n" represents stem II region of a HH ribozyme.

<400> 296

gggaaagacu gaugagncga aagcuguua                                    29

<210> 297
<211> 29
<212> RNA
<213> Artificial Sequence

<220>
<223> Synthesized Hammerhead Ribozyme

<220>
<223> "n" represents stem II region of a HH ribozyme.

<400> 297

gggggaaacu gaugagncga aagagcugu                                    29

<210> 298
<211> 29
<212> RNA
<213> Artificial Sequence

<220>
<223> Synthesized Hammerhead Ribozyme

<220>
<223> "n" represents stem II region of a HH ribozyme.

<400> 298

aagggggacu gaugagncga aagagagcu                                    29

<210> 299
<211> 29
<212> RNA
<213> Artificial Sequence

<220>
<223> Synthesized Hammerhead Ribozyme

<220>
<223> "n" represents stem II region of a HH ribozyme.

<400> 299

caagggggcu gaugagncga aaagagagc                                    29

<210> 300
<211> 29
<212> RNA
<213> Artificial Sequence

<220>
<223> Synthesized Hammerhead Ribozyme

<220>
<223> "n" represents stem II region of a HH ribozyme.

<400> 300

gcaaggggcu gaugagncga aaaagagag 29

<210> 301
<211> 29
<212> RNA
<213> Artificial Sequence

<220>
<223> Synthesized Hammerhead Ribozyme

<220>
<223> "n" represents stem II region of a HH ribozyme.

<400> 301
   aacauagccu gaugagncga aagggggaa 29

<210> 302
<211> 29
<212> RNA
<213> Artificial Sequence

<220>
<223> Synthesized Hammerhead Ribozyme

<220>
<223> "n" represents stem II region of a HH ribozyme.

<400> 302

uaguaacacu gaugagncga aagcaaggg 29

<210> 303
<211> 29
<212> RNA
<213> Artificial Sequence

<220>
<223> Synthesized Hammerhead Ribozyme

<220>
<223> "n" represents stem II region of a HH ribozyme.

<400> 303

cgcuuagucu gaugagncga aacauagca 29

<210> 304
<211> 29
<212> RNA
<213> Artificial Sequence

<220>
<223> Synthesized Hammerhead Ribozyme

<220>
<223> "n" represents stem II region of a HH ribozyme.

<400> 304

acgcuuagcu gaugagncga aaacauagc                                          29

<210> 305
<211> 29
<212> RNA
<213> Artificial Sequence

<220>
<223> Synthesized Hammerhead Ribozyme

<220>
<223> "n" represents stem II region of a HH ribozyme.

<400> 305

cucacgcucu gaugagncga aaguaacau                                          29

<210> 306
<211> 29
<212> RNA
<213> Artificial Sequence

<220>
<223> Synthesized Hammerhead Ribozyme

<220>
<223> "n" represents stem II region of a HH ribozyme.

<400> 306

gcuacgggcu gaugagncga aauccucac                                          29

<210> 307
<211> 29
<212> RNA
<213> Artificial Sequence

<220>
<223> Synthesized Hammerhead Ribozyme

<220>
<223> "n" represents stem II region of a HH ribozyme.

<400> 307

agcuacggcu gaugagncga aaauccuca                                    29

<210> 308
<211> 29
<212> RNA
<213> Artificial Sequence

<220>
<223> Synthesized Hammerhead Ribozyme

<220>
<223> "n" represents stem II region of a HH ribozyme.

<400> 308

ugaagagccu gaugagncga aacgggaau                                    29

<210> 309
<211> 29
<212> RNA
<213> Artificial Sequence

<220>
<223> Synthesized Hammerhead Ribozyme

<220>
<223> "n" represents stem II region of a HH ribozyme.

<400> 309

gcugugaacu gaugagncga aagcuacgg                                    29

<210> 310
<211> 29
<212> RNA
<213> Artificial Sequence

<220>
<223> Synthesized Hammerhead Ribozyme

<220>
<223> "n" represents stem II region of a HH ribozyme.

<400> 310

cagcugugcu gaugagncga aagagcuac                                    29

<210> 311

<211> 29
<212> RNA
<213> Artificial Sequence

<220>
<223> Synthesized Hammerhead Ribozyme

<220>
<223> "n" represents stem II region of a HH ribozyme.

<400> 311

ucagcugucu gaugagncga aaagagcua 29

<210> 312
<211> 29
<212> RNA
<213> Artificial Sequence

<220>
<223> Synthesized Hammerhead Ribozyme

<220>
<223> "n" represents stem II region of a HH ribozyme.

<400> 312

cauagacucu gaugagncga aaguucagc 29

<210> 313
<211> 29
<212> RNA
<213> Artificial Sequence

<220>
<223> Synthesized Hammerhead Ribozyme

<220>
<223> "n" represents stem II region of a HH ribozyme.

<400> 313

aacccauacu gaugagncga aacugaguu 29

<210> 314
<211> 29
<212> RNA
<213> Artificial Sequence

<220>
<223> Synthesized Hammerhead Ribozyme

<220>
<223> "n" represents stem II region of a HH ribozyme.

<400> 314

ccaacccacu gaugagncga aagacugag                                          29

<210> 315
<211> 29
<212> RNA
<213> Artificial Sequence

<220>
<223> Synthesized Hammerhead Ribozyme

<220>
<223> "n" represents stem II region of a HH ribozyme.

<400> 315

ugagcccccu gaugagncga aacccauag                                          29

<210> 316
<211> 29
<212> RNA
<213> Artificial Sequence

<220>
<223> Synthesized Hammerhead Ribozyme

<220>
<223> "n" represents stem II region of a HH ribozyme.

<400> 316

aguuaucucu gaugagncga aagccccaa                                          29

<210> 317
<211> 29
<212> RNA
<213> Artificial Sequence

<220>
<223> Synthesized Hammerhead Ribozyme

<220>
<223> "n" represents stem II region of a HH ribozyme.

<400> 317

cacagagucu gaugagncga aaucugagc                                          29

<210> 318
<211> 29
<212> RNA
<213> Artificial Sequence

<220>
<223> Synthesized Hammerhead Ribozyme

<220>
<223> "n" represents stem II region of a HH ribozyme.

<400> 318

aaugcacacu gaugagncga aaguuaucu                                    29

<210> 319
<211> 29
<212> RNA
<213> Artificial Sequence

<220>
<223> Synthesized Hammerhead Ribozyme

<220>
<223> "n" represents stem II region of a HH ribozyme.

<400> 319

guagcuuacu gaugagncga aaugcacag                                    29

<210> 320
<211> 29
<212> RNA
<213> Artificial Sequence

<220>
<223> Synthesized Hammerhead Ribozyme

<220>
<223> "n" represents stem II region of a HH ribozyme.

<400> 320

aguagcuucu gaugagncga aaaugcaca                                    29

<210> 321
<211> 29
<212> RNA
<213> Artificial Sequence

<220>
<223> Synthesized Hammerhead Ribozyme

<220>
<223> "n" represents stem II region of a HH ribozyme.

<400> 321

aaguagcucu gaugagncga aaaaugcac                                    29

<210> 322
<211> 29
<212> RNA
<213> Artificial Sequence

<220>
<223> Synthesized Hammerhead Ribozyme

<220>
<223> "n" represents stem II region of a HH ribozyme.

<400> 322

ucuacaagcu gaugagncga aagcuuaaa 29

<210> 323
<211> 29
<212> RNA
<213> Artificial Sequence

<220>
<223> Synthesized Hammerhead Ribozyme

<220>
<223> "n" represents stem II region of a HH ribozyme.

<400> 323

gucucuaccu gaugagncga aaguagcuu 29

<210> 324
<211> 29
<212> RNA
<213> Artificial Sequence

<220>
<223> Synthesized Hammerhead Ribozyme

<220>
<223> "n" represents stem II region of a HH ribozyme.

<400> 324

ugggucuccu gaugagncga aacaaguag 29

<210> 325
<211> 29
<212> RNA
<213> Artificial Sequence

<220>
<223> Synthesized Hammerhead Ribozyme

<220>
<223> "n" represents stem II region of a HH ribozyme.

<400> 325

aaaauguccu gaugagncga aacucucca                                    29

<210> 326
<211> 29
<212> RNA
<213> Artificial Sequence

<220>
<223> Synthesized Hammerhead Ribozyme

<220>
<223> "n" represents stem II region of a HH ribozyme.

<400> 326

agaggcaacu gaugagncga aaugucuac                                    29

<210> 327
<211> 29
<212> RNA
<213> Artificial Sequence

<220>
<223> Synthesized Hammerhead Ribozyme

<220>
<223> "n" represents stem II region of a HH ribozyme.

<400> 327

cagaggcacu gaugagncga aaaugucua                                    29

<210> 328
<211> 29
<212> RNA
<213> Artificial Sequence

<220>
<223> Synthesized Hammerhead Ribozyme

<220>
<223> "n" represents stem II region of a HH ribozyme.

<400> 328

ucagaggccu gaugagncga aaaaugucu                                    29

<210> 329
<211> 29
<212> RNA
<213> Artificial Sequence

<220>
<223> Synthesized Hammerhead Ribozyme

<220>
<223> "n" represents stem II region of a HH ribozyme.

<400> 329

gcuuaucacu gaugagncga aaggcaaaa                                          29



<210> 330
<211> 29
<212> RNA
<213> Artificial Sequence

<220>
<223> Synthesized Hammerhead Ribozyme

<220>
<223> "n" represents stem II region of a HH ribozyme.

<400> 330

aaagugcucu gaugagncga aaucagagg                                          29



<210> 331
<211> 29
<212> RNA
<213> Artificial Sequence

<220>
<223> Synthesized Hammerhead Ribozyme

<220>
<223> "n" represents stem II region of a HH ribozyme.

<400> 331

cauuuaaacu gaugagncga aagugcuua                                          29



<210> 332
<211> 29
<212> RNA
<213> Artificial Sequence

<220>
<223> Synthesized Hammerhead Ribozyme

<220>
<223> "n" represents stem II region of a HH ribozyme.

<400> 332

ccauuuaacu gaugagncga aaagugcuu                                          29

<210> 333
<211> 29
<212> RNA
<213> Artificial Sequence

<220>
<223> Synthesized Hammerhead Ribozyme

<220>
<223> "n" represents stem II region of a HH ribozyme.

<400> 333

gccauuuacu gaugagncga aaaagugcu                                          29

<210> 334
<211> 29
<212> RNA
<213> Artificial Sequence

<220>
<223> Synthesized Hammerhead Ribozyme

<220>
<223> "n" represents stem II region of a HH ribozyme.

<400> 334

agccauuucu gaugagncga aaaaagugc                                          29

<210> 335
<211> 29
<212> RNA
<213> Artificial Sequence

<220>
<223> Synthesized Hammerhead Ribozyme

<220>
<223> "n" represents stem II region of a HH ribozyme.

<400> 335

gagccauucu gaugagncga aaaaaagug                                          29

<210> 336
<211> 29
<212> RNA

<213> Artificial Sequence

<220>
<223> Synthesized Hammerhead Ribozyme

<220>
<223> "n" represents stem II region of a HH ribozyme.

<400> 336

uauucuuacu gaugagncga aagccauuu                                          29

<210> 337
<211> 29
<212> RNA
<213> Artificial Sequence

<220>
<223> Synthesized Hammerhead Ribozyme

<220>
<223> "n" represents stem II region of a HH ribozyme.

<400> 337

cuuauucucu gaugagncga aagagccau                                          29

<210> 338
<211> 29
<212> RNA
<213> Artificial Sequence

<220>
<223> Synthesized Hammerhead Ribozyme

<220>
<223> "n" represents stem II region of a HH ribozyme.

<400> 338

cuguggcucu gaugagncga aauucuuag                                          29

<210> 339
<211> 29
<212> RNA
<213> Artificial Sequence

<220>
<223> Synthesized Hammerhead Ribozyme

<220>
<223> "n" represents stem II region of a HH ribozyme.

<400> 339

ccacuuuacu gaugagncga aauucuuug                                    29

<210> 340
<211> 29
<212> RNA
<213> Artificial Sequence

<220>
<223> Synthesized Hammerhead Ribozyme

<220>
<223> "n" represents stem II region of a HH ribozyme.

<400> 340

gccacuuucu gaugagncga aaauucuuu                                    29

<210> 341
<211> 29
<212> RNA
<213> Artificial Sequence

<220>
<223> Synthesized Hammerhead Ribozyme

<220>
<223> "n" represents stem II region of a HH ribozyme.

<400> 341

agccacuucu gaugagncga aaaauucuu                                    29

<210> 342
<211> 29
<212> RNA
<213> Artificial Sequence

<220>
<223> Synthesized Hammerhead Ribozyme

<220>
<223> "n" represents stem II region of a HH ribozyme.

<400> 342

aauuaaagcu gaugagncga aagccacuu                                    29

<210> 343
<211> 29
<212> RNA
<213> Artificial Sequence

<220>

<223> Synthesized Hammerhead Ribozyme

<220>
<223> "n" represents stem II region of a HH ribozyme.

<400> 343

accaauuacu gaugagncga aaggagcca                                          29

<210> 344
<211> 29
<212> RNA
<213> Artificial Sequence

<220>
<223> Synthesized Hammerhead Ribozyme

<220>
<223> "n" represents stem II region of a HH ribozyme.

<400> 344

caccaauucu gaugagncga aaaggagcc                                          29

<210> 345
<211> 29
<212> RNA
<213> Artificial Sequence

<220>
<223> Synthesized Hammerhead Ribozyme

<220>
<223> "n" represents stem II region of a HH ribozyme.

<400> 345

ucaccaaucu gaugagncga aaaaggagc                                          29

<210> 346
<211> 29
<212> RNA
<213> Artificial Sequence

<220>
<223> Synthesized Hammerhead Ribozyme

<220>
<223> "n" represents stem II region of a HH ribozyme.

<400> 346

aagucacccu gaugagncga aauuaaagg                                          29

<210> 347
<211> 29
<212> RNA
<213> Artificial Sequence

<220>
<223> Synthesized Hammerhead Ribozyme

<220>
<223> "n" represents stem II region of a HH ribozyme.

<400> 347

cuuucucccu gaugagncga aagucacca                                    29

<210> 348
<211> 29
<212> RNA
<213> Artificial Sequence

<220>
<223> Synthesized Hammerhead Ribozyme

<220>
<223> "n" represents stem II region of a HH ribozyme.

<400> 348

ccuugacccu gaugagncga aagcuuucu                                    29

<210> 349
<211> 29
<212> RNA
<213> Artificial Sequence

<220>
<223> Synthesized Hammerhead Ribozyme

<220>
<223> "n" represents stem II region of a HH ribozyme.

<400> 349

aaacccuucu gaugagncga aaccuagcu                                    29

<210> 350
<211> 29
<212> RNA
<213> Artificial Sequence

<220>
<223> Synthesized Hammerhead Ribozyme

<220>

<223> "n" represents stem II region of a HH ribozyme.

<400> 350

cuauaauacu gaugagncga aacccuuga                                    29

<210> 351
<211> 29
<212> RNA
<213> Artificial Sequence

<220>
<223> Synthesized Hammerhead Ribozyme

<220>
<223> "n" represents stem II region of a HH ribozyme.

<400> 351

gcuauaaucu gaugagncga aaacccuug                                    29

<210> 352
<211> 29
<212> RNA
<213> Artificial Sequence

<220>
<223> Synthesized Hammerhead Ribozyme

<220>
<223> "n" represents stem II region of a HH ribozyme.

<400> 352

ugcuauaacu gaugagncga aaaacccuu                                    29

<210> 353
<211> 29
<212> RNA
<213> Artificial Sequence

<220>
<223> Synthesized Hammerhead Ribozyme

<220>
<223> "n" represents stem II region of a HH ribozyme.

<400> 353

ggugcuaucu gaugagncga aauaaaccc                                    29

<210> 354
<211> 29

<212> RNA
<213> Artificial Sequence

<220>
<223> Synthesized Hammerhead Ribozyme

<220>
<223> "n" represents stem II region of a HH ribozyme.

<400> 354

gggugcuacu gaugagncga aaauaaacc 29

<210> 355
<211> 29
<212> RNA
<213> Artificial Sequence

<220>
<223> Synthesized Hammerhead Ribozyme

<220>
<223> "n" represents stem II region of a HH ribozyme.

<400> 355

gagggugccu gaugagncga aauaauaaa 29

<210> 356
<211> 29
<212> RNA
<213> Artificial Sequence

<220>
<223> Synthesized Hammerhead Ribozyme

<220>
<223> "n" represents stem II region of a HH ribozyme.

<400> 356

gaauacaacu gaugagncga aagggugcu 29

<210> 357
<211> 29
<212> RNA
<213> Artificial Sequence

<220>
<223> Synthesized Hammerhead Ribozyme

<220>
<223> "n" represents stem II region of a HH ribozyme.

<400> 357

aggaauaccu gaugagncga aagagggug 29

<210> 358
<211> 29
<212> RNA
<213> Artificial Sequence

<220>
<223> Synthesized Hammerhead Ribozyme

<220>
<223> "n" represents stem II region of a HH ribozyme.

<400> 358

cauaggaacu gaugagncga aacaagagg 29

<210> 359
<211> 29
<212> RNA
<213> Artificial Sequence

<220>
<223> Synthesized Hammerhead Ribozyme

<220>
<223> "n" represents stem II region of a HH ribozyme.

<400> 359

gccauaggcu gaugagncga aauacaaga 29

<210> 360
<211> 29
<212> RNA
<213> Artificial Sequence

<220>
<223> Synthesized Hammerhead Ribozyme

<220>
<223> "n" represents stem II region of a HH ribozyme.

<400> 360

ugccauagcu gaugagncga aaauacaag 29

<210> 361
<211> 29
<212> RNA
<213> Artificial Sequence

<220>
<223> Synthesized Hammerhead Ribozyme

<220>
<223> "n" represents stem II region of a HH ribozyme.

<400> 361

cauugccacu gaugagncga aaggaauac 29

<210> 362
<211> 29
<212> RNA
<213> Artificial Sequence

<220>
<223> Synthesized Hammerhead Ribozyme

<220>
<223> "n" represents stem II region of a HH ribozyme.

<400> 362

cauaaaagcu gaugagncga aaugcauug 29

<210> 363
<211> 29
<212> RNA
<213> Artificial Sequence

<220>
<223> Synthesized Hammerhead Ribozyme

<220>
<223> "n" represents stem II region of a HH ribozyme.

<400> 363

uuucauaacu gaugagncga aaggaugca 29

<210> 364
<211> 29
<212> RNA
<213> Artificial Sequence

<220>
<223> Synthesized Hammerhead Ribozyme

<220>
<223> "n" represents stem II region of a HH ribozyme.

<400> 364

cuuucauacu gaugagncga aaaggaugc 29

<210> 365
<211> 29
<212> RNA
<213> Artificial Sequence

<220>
<223> Synthesized Hammerhead Ribozyme

<220>
<223> "n" represents stem II region of a HH ribozyme.

<400> 365

acuuucaucu gaugagncga aaaaggaug                                          29

<210> 366
<211> 29
<212> RNA
<213> Artificial Sequence

<220>
<223> Synthesized Hammerhead Ribozyme

<220>
<223> "n" represents stem II region of a HH ribozyme.

<400> 366

cacuuucacu gaugagncga aaaaaggau                                          29

<210> 367
<211> 29
<212> RNA
<213> Artificial Sequence

<220>
<223> Synthesized Hammerhead Ribozyme

<220>
<223> "n" represents stem II region of a HH ribozyme.

<400> 367

uuaaggugcu gaugagncga aaccacuuu                                          29

<210> 368
<211> 29
<212> RNA
<213> Artificial Sequence

<220>
<223> Synthesized Hammerhead Ribozyme

<220>
<223> "n" represents stem II region of a HH ribozyme.

<400> 368

aaagcuuucu gaugagncga aagguguac                                    29

<210> 369
<211> 29
<212> RNA
<213> Artificial Sequence

<220>
<223> Synthesized Hammerhead Ribozyme

<220>
<223> "n" represents stem II region of a HH ribozyme.

<400> 369

aaaagcuucu gaugagncga aaaggugua                                    29

<210> 370
<211> 29
<212> RNA
<213> Artificial Sequence

<220>
<223> Synthesized Hammerhead Ribozyme

<220>
<223> "n" represents stem II region of a HH ribozyme.

<400> 370

ucauauaacu gaugagncga aagcuuuaa                                    29

<210> 371
<211> 29
<212> RNA
<213> Artificial Sequence

<220>
<223> Synthesized Hammerhead Ribozyme

<220>
<223> "n" represents stem II region of a HH ribozyme.

<400> 371

gucauauacu gaugagncga aaagcuuua                                    29

<210> 372

<211> 29
<212> RNA
<213> Artificial Sequence

<220>
<223> Synthesized Hammerhead Ribozyme

<220>
<223> "n" represents stem II region of a HH ribozyme.

<400> 372

agucauaucu gaugagncga aaaagcuuu                                29

<210> 373
<211> 29
<212> RNA
<213> Artificial Sequence

<220>
<223> Synthesized Hammerhead Ribozyme

<220>
<223> "n" represents stem II region of a HH ribozyme.

<400> 373

cagucauacu gaugagncga aaaaagcuu                                29

<210> 374
<211> 29
<212> RNA
<213> Artificial Sequence

<220>
<223> Synthesized Hammerhead Ribozyme

<220>
<223> "n" represents stem II region of a HH ribozyme.

<400> 374

uacagucacu gaugagncga aauaaaagc                                29

<210> 375
<211> 29
<212> RNA
<213> Artificial Sequence

<220>
<223> Synthesized Hammerhead Ribozyme

<220>
<223> "n" represents stem II region of a HH ribozyme.

<400> 375

uacucugccu gaugagncga aacagucau                                29

<210> 376
<211> 29
<212> RNA
<213> Artificial Sequence

<220>
<223> Synthesized Hammerhead Ribozyme

<220>
<223> "n" represents stem II region of a HH ribozyme.

<400> 376

ucaccagacu gaugagncga aacucugcu                                29

<210> 377
<211> 29
<212> RNA
<213> Artificial Sequence

<220>
<223> Synthesized Hammerhead Ribozyme

<220>
<223> "n" represents stem II region of a HH ribozyme.

<400> 377

aaucaccacu gaugagncga aauacucug                                29

<210> 378
<211> 29
<212> RNA
<213> Artificial Sequence

<220>
<223> Synthesized Hammerhead Ribozyme

<220>
<223> "n" represents stem II region of a HH ribozyme.

<400> 378

gaauugaccu gaugagncga aaucaccag                                29

<210> 379
<211> 29
<212> RNA
<213> Artificial Sequence

<220>
<223> Synthesized Hammerhead Ribozyme

<220>
<223> "n" represents stem II region of a HH ribozyme.

<400> 379

agugaauucu gaugagncga aacaaucac                                          29



<210> 380
<211> 29
<212> RNA
<213> Artificial Sequence

<220>
<223> Synthesized Hammerhead Ribozyme

<220>
<223> "n" represents stem II region of a HH ribozyme.

<400> 380

gggaagugcu gaugagncga aauugacaa                                          29



<210> 381
<211> 29
<212> RNA
<213> Artificial Sequence

<220>
<223> Synthesized Hammerhead Ribozyme

<220>
<223> "n" represents stem II region of a HH ribozyme.

<400> 381

ggggaagucu gaugagncga aaauugaca                                          29



<210> 382
<211> 29
<212> RNA
<213> Artificial Sequence

<220>
<223> Synthesized Hammerhead Ribozyme

<220>
<223> "n" represents stem II region of a HH ribozyme.

<400> 382
   auagggggcu gaugagncga aagugaauu                                          29

<210> 383
<211> 29
<212> RNA
<213> Artificial Sequence

<220>
<223> Synthesized Hammerhead Ribozyme

<220>
<223> "n" represents stem II region of a HH ribozyme.

<400> 383

uauaggggcu gaugagncga aaagugaau                                          29

<210> 384
<211> 29
<212> RNA
<213> Artificial Sequence

<220>
<223> Synthesized Hammerhead Ribozyme

<220>
<223> "n" represents stem II region of a HH ribozyme.

<400> 384

uauaggggcu gaugagncga aaagugaau                                          29

<210> 385
<211> 29
<212> RNA
<213> Artificial Sequence

<220>
<223> Synthesized Hammerhead Ribozyme

<220>
<223> "n" represents stem II region of a HH ribozyme.

<400> 385
   uguauucccu gaugagncga aauaggggg                                           29

<210> 386
<211> 29
<212> RNA
<213> Artificial Sequence

<220>
<223> Synthesized Hammerhead Ribozyme

<220>
<223> "n" represents stem II region of a HH ribozyme.

<400> 386

gccccuugcu gaugagncga aauuccuau                                    29

<210> 387
<211> 29
<212> RNA
<213> Artificial Sequence

<220>
<223> Synthesized Hammerhead Ribozyme

<220>
<223> "n" represents stem II region of a HH ribozyme.

<400> 387

aacuagggcu gaugagncga aaucugccu                                    29

<210> 388
<211> 29
<212> RNA
<213> Artificial Sequence

<220>
<223> Synthesized Hammerhead Ribozyme

<220>
<223> "n" represents stem II region of a HH ribozyme.

<400> 388

uggccaaccu gaugagncga aaggggauc                                    29

<210> 389
<211> 29
<212> RNA
<213> Artificial Sequence

<220>
<223> Synthesized Hammerhead Ribozyme

<220>
<223> "n" represents stem II region of a HH ribozyme.

<400> 389

ucuuggcccu gaugagncga aacuagggg                                    29

<210> 390
<211> 29
<212> RNA
<213> Artificial Sequence

<220>
<223> Synthesized Hammerhead Ribozyme

<220>
<223> "n" represents stem II region of a HH ribozyme.

<400> 390

guuaaaaucu gaugagncga aagucuugg                                29

<210> 391
<211> 29
<212> RNA
<213> Artificial Sequence

<220>
<223> Synthesized Hammerhead Ribozyme

<220>
<223> "n" represents stem II region of a HH ribozyme.

<400> 391

aguuaaaacu gaugagncga aaagucuug                                29

<210> 392
<211> 29
<212> RNA
<213> Artificial Sequence

<220>
<223> Synthesized Hammerhead Ribozyme

<220>
<223> "n" represents stem II region of a HH ribozyme.

<400> 392

caaguuaacu gaugagncga aauaagucu                                29

<210> 393
<211> 29
<212> RNA
<213> Artificial Sequence

<220>
<223> Synthesized Hammerhead Ribozyme

<220>
<223> "n" represents stem II region of a HH ribozyme.

<400> 393

ucaaguuacu gaugagncga aaauaaguc                                    29

<210> 394
<211> 29
<212> RNA
<213> Artificial Sequence

<220>
<223> Synthesized Hammerhead Ribozyme

<220>
<223> "n" represents stem II region of a HH ribozyme.

<400> 394

aucaaguucu gaugagncga aaaauaagu                                    29

<210> 395
<211> 29
<212> RNA
<213> Artificial Sequence

<220>
<223> Synthesized Hammerhead Ribozyme

<220>
<223> "n" represents stem II region of a HH ribozyme.

<400> 395

uaucaagucu gaugagncga aaaaauaag                                    29

<210> 396
<211> 29
<212> RNA
<213> Artificial Sequence

<220>
<223> Synthesized Hammerhead Ribozyme

<220>
<223> "n" represents stem II region of a HH ribozyme.

<400> 396

aguguauccu gaugagncga aaguuaaaa                                    29

<210> 397
<211> 29
<212> RNA

<213> Artificial Sequence

<220>
<223> Synthesized Hammerhead Ribozyme

<220>
<223> "n" represents stem II region of a HH ribozyme.

<400> 397

cugcagugcu gaugagncga aaucaaguu                                    29

<210> 398
<211> 29
<212> RNA
<213> Artificial Sequence

<220>
<223> Synthesized Hammerhead Ribozyme

<220>
<223> "n" represents stem II region of a HH ribozyme.

<400> 398

acacucugcu gaugagncga aaucugcag                                    29

<210> 399
<211> 29
<212> RNA
<213> Artificial Sequence

<220>
<223> Synthesized Hammerhead Ribozyme

<220>
<223> "n" represents stem II region of a HH ribozyme.

<400> 399

gacacucucu gaugagncga aaaucugca                                    29

<210> 400
<211> 29
<212> RNA
<213> Artificial Sequence

<220>
<223> Synthesized Hammerhead Ribozyme

<220>
<223> "n" represents stem II region of a HH ribozyme.

<400> 400

agcuucagcu gaugagncga aacacucug                                    29

<210> 401
<211> 29
<212> RNA
<213> Artificial Sequence

<220>
<223> Synthesized Hammerhead Ribozyme

<220>
<223> "n" represents stem II region of a HH ribozyme.

<400> 401

cagaggcacu gaugagncga aagcuucag                                    29

<210> 402
<211> 29
<212> RNA
<213> Artificial Sequence

<220>
<223> Synthesized Hammerhead Ribozyme

<220>
<223> "n" represents stem II region of a HH ribozyme.

<400> 402

gaaagccacu gaugagncga aaggcagag                                    29

<210> 403
<211> 29
<212> RNA
<213> Artificial Sequence

<220>
<223> Synthesized Hammerhead Ribozyme

<220>
<223> "n" represents stem II region of a HH ribozyme.

<400> 403

ugaccggacu gaugagncga aagccagag                                    29

<210> 404
<211> 29
<212> RNA
<213> Artificial Sequence

<220>

<223> Synthesized Hammerhead Ribozyme

<220>
<223> "n" represents stem II region of a HH ribozyme.

<400> 404

augaccggcu gaugagncga aaagccaga                                    29

<210> 405
<211> 29
<212> RNA
<213> Artificial Sequence

<220>
<223> Synthesized Hammerhead Ribozyme

<220>
<223> "n" represents stem II region of a HH ribozyme.

<400> 405

caugaccgcu gaugagncga aaaagccag                                    29

<210> 406
<211> 29
<212> RNA
<213> Artificial Sequence

<220>
<223> Synthesized Hammerhead Ribozyme

<220>
<223> "n" represents stem II region of a HH ribozyme.

<400> 406

gaacccaucu gaugagncga aaccggaaa                                    29

<210> 407
<211> 29
<212> RNA
<213> Artificial Sequence

<220>
<223> Synthesized Hammerhead Ribozyme

<220>
<223> "n" represents stem II region of a HH ribozyme.

<400> 407

uuaacuggcu gaugagncga aacccauga                                    29

<210> 408
<211> 29
<212> RNA
<213> Artificial Sequence

<220>
<223> Synthesized Hammerhead Ribozyme

<220>
<223> "n" represents stem II region of a HH ribozyme.

<400> 408

auuaacugcu gaugagncga aaacccaug                                          29

<210> 409
<211> 29
<212> RNA
<213> Artificial Sequence

<220>
<223> Synthesized Hammerhead Ribozyme

<220>
<223> "n" represents stem II region of a HH ribozyme.

<400> 409

caugaauucu gaugagncga aacuggaac                                          29

<210> 410
<211> 29
<212> RNA
<213> Artificial Sequence

<220>
<223> Synthesized Hammerhead Ribozyme

<220>
<223> "n" represents stem II region of a HH ribozyme.

<400> 410

gcaugaaucu gaugagncga aaacuggaa                                          29

<210> 411
<211> 29
<212> RNA
<213> Artificial Sequence

<220>
<223> Synthesized Hammerhead Ribozyme

<220>

<223> "n" represents stem II region of a HH ribozyme.

<400> 411

gaggcaugcu gaugagncga aauuaacug                                    29

<210> 412
<211> 29
<212> RNA
<213> Artificial Sequence

<220>
<223> Synthesized Hammerhead Ribozyme

<220>
<223> "n" represents stem II region of a HH ribozyme.

<400> 412

ggaggcaucu gaugagncga aaauuaacu                                    29

<210> 413
<211> 29
<212> RNA
<213> Artificial Sequence

<220>
<223> Synthesized Hammerhead Ribozyme

<220>
<223> "n" represents stem II region of a HH ribozyme.

<400> 413

guccauggcu gaugagncga aaggcauga                                    29

<210> 414
<211> 29
<212> RNA
<213> Artificial Sequence

<220>
<223> Synthesized Hammerhead Ribozyme

<220>
<223> "n" represents stem II region of a HH ribozyme.

<400> 414

gcucuccacu gaugagncga aagguccau                                    29

<210> 415
<211> 29

<212> RNA
<213> Artificial Sequence

<220>
<223> Synthesized Hammerhead Ribozyme

<220>
<223> "n" represents stem II region of a HH ribozyme.

<400> 415

cuaagauccu gaugagncga aacuuguug                                          29



<210> 416
<211> 29
<212> RNA
<213> Artificial Sequence

<220>
<223> Synthesized Hammerhead Ribozyme

<220>
<223> "n" represents stem II region of a HH ribozyme.

<400> 416

uuaacuaacu gaugagncga aaucaacuu                                          29



<210> 417
<211> 29
<212> RNA
<213> Artificial Sequence

<220>
<223> Synthesized Hammerhead Ribozyme

<220>
<223> "n" represents stem II region of a HH ribozyme.

<400> 417

acuuaacucu gaugagncga aagaucaac                                          29



<210> 418
<211> 29
<212> RNA
<213> Artificial Sequence

<220>
<223> Synthesized Hammerhead Ribozyme

<220>
<223> "n" represents stem II region of a HH ribozyme.

<400> 418

gacuuaaccu gaugagncga aaagaucaa                                    29

<210> 419
<211> 29
<212> RNA
<213> Artificial Sequence

<220>
<223> Synthesized Hammerhead Ribozyme

<220>
<223> "n" represents stem II region of a HH ribozyme.

<400> 419

ggagacuucu gaugagncga aacuaagau                                    29

<210> 420
<211> 29
<212> RNA
<213> Artificial Sequence

<220>
<223> Synthesized Hammerhead Ribozyme

<220>
<223> "n" represents stem II region of a HH ribozyme.

<400> 420

gggagacucu gaugagncga aaacuaaga                                    29

<210> 421
<211> 29
<212> RNA
<213> Artificial Sequence

<220>
<223> Synthesized Hammerhead Ribozyme

<220>
<223> "n" represents stem II region of a HH ribozyme.

<400> 421

uauagggacu gaugagncga aacuuaacu                                    29

<210> 422
<211> 29
<212> RNA
<213> Artificial Sequence

<220>
<223> Synthesized Hammerhead Ribozyme

<220>
<223> "n" represents stem II region of a HH ribozyme.

<400> 422

cauauaggcu gaugagncga aagacuuaa                                          29



<210> 423
<211> 29
<212> RNA
<213> Artificial Sequence

<220>
<223> Synthesized Hammerhead Ribozyme

<220>
<223> "n" represents stem II region of a HH ribozyme.

<400> 423

cccucauacu gaugagncga aagggagac                                          29



<210> 424
<211> 29
<212> RNA
<213> Artificial Sequence

<220>
<223> Synthesized Hammerhead Ribozyme

<220>
<223> "n" represents stem II region of a HH ribozyme.

<400> 424

aucccucacu gaugagncga aauagggag                                          29



<210> 425
<211> 29
<212> RNA
<213> Artificial Sequence

<220>
<223> Synthesized Hammerhead Ribozyme

<220>
<223> "n" represents stem II region of a HH ribozyme.

<400> 425

caggaacucu gaugagncga aaucccuca                                          29

<210> 426
<211> 29
<212> RNA
<213> Artificial Sequence

<220>
<223> Synthesized Hammerhead Ribozyme

<220>
<223> "n" represents stem II region of a HH ribozyme.

<400> 426

aaaucaggcu gaugagncga aacuuaucc                                        29

<210> 427
<211> 29
<212> RNA
<213> Artificial Sequence

<220>
<223> Synthesized Hammerhead Ribozyme

<220>
<223> "n" represents stem II region of a HH ribozyme.

<400> 427

aaaaucagcu gaugagncga aaacuuauc                                        29

<210> 428
<211> 29
<212> RNA
<213> Artificial Sequence

<220>
<223> Synthesized Hammerhead Ribozyme

<220>
<223> "n" represents stem II region of a HH ribozyme.

<400> 428

aaaacaaacu gaugagncga aaucaggaa                                        29

<210> 429
<211> 29
<212> RNA
<213> Artificial Sequence

<220>
<223> Synthesized Hammerhead Ribozyme

<220>
<223> "n" represents stem II region of a HH ribozyme.

<400> 429

aaaaacaacu gaugagncga aaaucagga                                29

<210> 430
<211> 29
<212> RNA
<213> Artificial Sequence

<220>
<223> Synthesized Hammerhead Ribozyme

<220>
<223> "n" represents stem II region of a HH ribozyme.

<400> 430

uaaaaacacu gaugagncga aaaaucagg                                29

<210> 431
<211> 29
<212> RNA
<213> Artificial Sequence

<220>
<223> Synthesized Hammerhead Ribozyme

<220>
<223> "n" represents stem II region of a HH ribozyme.

<400> 431

auaaaaaccu gaugagncga aaaaaucag                                29

<210> 432
<211> 29
<212> RNA
<213> Artificial Sequence

<220>
<223> Synthesized Hammerhead Ribozyme

<220>
<223> "n" represents stem II region of a HH ribozyme.

<400> 432

aaaauaaacu gaugagncga aacaaaaau                                29

<210> 433

<211> 29
<212> RNA
<213> Artificial Sequence

<220>
<223> Synthesized Hammerhead Ribozyme

<220>
<223> "n" represents stem II region of a HH ribozyme.

<400> 433

aaaaauaacu gaugagncga aaacaaaaa                                    29



<210> 434
<211> 29
<212> RNA
<213> Artificial Sequence

<220>
<223> Synthesized Hammerhead Ribozyme

<220>
<223> "n" represents stem II region of a HH ribozyme.

<400> 434

caaaaauacu gaugagncga aaaacaaaa                                    29



<210> 435
<211> 29
<212> RNA
<213> Artificial Sequence

<220>
<223> Synthesized Hammerhead Ribozyme

<220>
<223> "n" represents stem II region of a HH ribozyme.

<400> 435

acaaaaaucu gaugagncga aaaaacaaa                                    29



<210> 436
<211> 29
<212> RNA
<213> Artificial Sequence

<220>
<223> Synthesized Hammerhead Ribozyme

<220>
<223> "n" represents stem II region of a HH ribozyme.

<400> 436

cacaaaaacu gaugagncga aaaaaacaa                                    29

<210> 437
<211> 29
<212> RNA
<213> Artificial Sequence

<220>
<223> Synthesized Hammerhead Ribozyme

<220>
<223> "n" represents stem II region of a HH ribozyme.

<400> 437

aacacaaacu gaugagncga aauaaaaac                                    29

<210> 438
<211> 29
<212> RNA
<213> Artificial Sequence

<220>
<223> Synthesized Hammerhead Ribozyme

<220>
<223> "n" represents stem II region of a HH ribozyme.

<400> 438

uaacacaacu gaugagncga aaauaaaaa                                    29

<210> 439
<211> 29
<212> RNA
<213> Artificial Sequence

<220>
<223> Synthesized Hammerhead Ribozyme

<220>
<223> "n" represents stem II region of a HH ribozyme.

<400> 439

guaacacacu gaugagncga aaaauaaaa                                    29

<210> 440
<211> 29
<212> RNA
<213> Artificial Sequence

<220>
<223> Synthesized Hammerhead Ribozyme

<220>
<223> "n" represents stem II region of a HH ribozyme.

<400> 440

uguaacaccu gaugagncga aaaaauaaa                                          29

<210> 441
<211> 29
<212> RNA
<213> Artificial Sequence

<220>
<223> Synthesized Hammerhead Ribozyme

<220>
<223> "n" represents stem II region of a HH ribozyme.

<400> 441

ucuuuugucu gaugagncga aacacaaaa                                          29

<210> 442
<211> 29
<212> RNA
<213> Artificial Sequence

<220>
<223> Synthesized Hammerhead Ribozyme

<220>
<223> "n" represents stem II region of a HH ribozyme.

<400> 442

uucuuuugcu gaugagncga aaacacaaa                                          29

<210> 443
<211> 29
<212> RNA
<213> Artificial Sequence

<220>
<223> Synthesized Hammerhead Ribozyme

<220>
<223> "n" represents stem II region of a HH ribozyme.

<400> 443

cagggaggcu gaugagncga aagggcuuu                                          29

<210> 444
<211> 29
<212> RNA
<213> Artificial Sequence

<220>
<223> Synthesized Hammerhead Ribozyme

<220>
<223> "n" represents stem II region of a HH ribozyme.

<400> 444

aguucaggcu gaugagncga aagggaggg                                    29

<210> 445
<211> 29
<212> RNA
<213> Artificial Sequence

<220>
<223> Synthesized Hammerhead Ribozyme

<220>
<223> "n" represents stem II region of a HH ribozyme.

<400> 445

cuuacugccu gaugagncga aaguucagg                                    29

<210> 446
<211> 29
<212> RNA
<213> Artificial Sequence

<220>
<223> Synthesized Hammerhead Ribozyme

<220>
<223> "n" represents stem II region of a HH ribozyme.

<400> 446

gcugaccucu gaugagncga aacugcaag                                    29

<210> 447
<211> 29
<212> RNA
<213> Artificial Sequence

<220>
<223> Synthesized Hammerhead Ribozyme

<220>
<223> "n" represents stem II region of a HH ribozyme.

<400> 447

cugaagcucu gaugagncga aaccuuacu                                    29

<210> 448
<211> 29
<212> RNA
<213> Artificial Sequence

<220>
<223> Synthesized Hammerhead Ribozyme

<220>
<223> "n" represents stem II region of a HH ribozyme.

<400> 448

agguccugcu gaugagncga aagcugacc                                    29

<210> 449
<211> 29
<212> RNA
<213> Artificial Sequence

<220>
<223> Synthesized Hammerhead Ribozyme

<220>
<223> "n" represents stem II region of a HH ribozyme.

<400> 449

cagguccucu gaugagncga aaagcugac                                    29

<210> 450
<211> 29
<212> RNA
<213> Artificial Sequence

<220>
<223> Synthesized Hammerhead Ribozyme

<220>
<223> "n" represents stem II region of a HH ribozyme.

<400> 450

cccacuggcu gaugagncga aacaggucc                                    29

<210> 451
<211> 29
<212> RNA
<213> Artificial Sequence

<220>
<223> Synthesized Hammerhead Ribozyme

<220>
<223> "n" represents stem II region of a HH ribozyme.

<400> 451

gcccacugcu gaugagncga aaacagguc                                    29

<210> 452
<211> 29
<212> RNA
<213> Artificial Sequence

<220>
<223> Synthesized Hammerhead Ribozyme

<220>
<223> "n" represents stem II region of a HH ribozyme.

<400> 452

gauccaagcu gaugagncga aacagugcc                                    29

<210> 453
<211> 29
<212> RNA
<213> Artificial Sequence

<220>
<223> Synthesized Hammerhead Ribozyme

<220>
<223> "n" represents stem II region of a HH ribozyme.

<400> 453

gaagaucccu gaugagncga aaguacagu                                    29

<210> 454
<211> 29
<212> RNA
<213> Artificial Sequence

<220>
<223> Synthesized Hammerhead Ribozyme

<220>
<223> "n" represents stem II region of a HH ribozyme.

<400> 454

gccgggaacu gaugagncga aauccaagu                                29

<210> 455
<211> 29
<212> RNA
<213> Artificial Sequence

<220>
<223> Synthesized Hammerhead Ribozyme

<220>
<223> "n" represents stem II region of a HH ribozyme.

<400> 455

acgccgggcu gaugagncga aagauccaa                                29

<210> 456
<211> 29
<212> RNA
<213> Artificial Sequence

<220>
<223> Synthesized Hammerhead Ribozyme

<220>
<223> "n" represents stem II region of a HH ribozyme.

<400> 456

cacgccggcu gaugagncga aaagaucca                                29

<210> 457
<211> 29
<212> RNA
<213> Artificial Sequence

<220>
<223> Synthesized Hammerhead Ribozyme

<220>
<223> "n" represents stem II region of a HH ribozyme.

<400> 457

cccugugucu gaugagncga aaggcacac                                29

<210> 458
<211> 29
<212> RNA

<213> Artificial Sequence

<220>
<223> Synthesized Hammerhead Ribozyme

<220>
<223> "n" represents stem II region of a HH ribozyme.

<400> 458

ccccugugcu gaugagncga aaaggcaca                                          29



<210> 459
<211> 29
<212> RNA
<213> Artificial Sequence

<220>
<223> Synthesized Hammerhead Ribozyme

<220>
<223> "n" represents stem II region of a HH ribozyme.

<400> 459

ccacagugcu gaugagncga aacaguuca                                          29



<210> 460
<211> 29
<212> RNA
<213> Artificial Sequence

<220>
<223> Synthesized Hammerhead Ribozyme

<220>
<223> "n" represents stem II region of a HH ribozyme.

<400> 460

accacagucu gaugagncga aaacaguuc                                          29



<210> 461
<211> 29
<212> RNA
<213> Artificial Sequence

<220>
<223> Synthesized Hammerhead Ribozyme

<220>
<223> "n" represents stem II region of a HH ribozyme.

<400> 461

cuaccauucu gaugagncga aacccucau                                    29

<210> 462
<211> 29
<212> RNA
<213> Artificial Sequence

<220>
<223> Synthesized Hammerhead Ribozyme

<220>
<223> "n" represents stem II region of a HH ribozyme.

<400> 462

cuuucaaccu gaugagncga aaccauuua                                    29

<210> 463
<211> 29
<212> RNA
<213> Artificial Sequence

<220>
<223> Synthesized Hammerhead Ribozyme

<220>
<223> "n" represents stem II region of a HH ribozyme.

<400> 463

cuccuuuccu gaugagncga aacuaccau                                    29

<210> 464
<211> 29
<212> RNA
<213> Artificial Sequence

<220>
<223> Synthesized Hammerhead Ribozyme

<220>
<223> "n" represents stem II region of a HH ribozyme.

<400> 464

cuaaaugccu gaugagncga aacaccagg                                    29

<210> 465
<211> 29
<212> RNA
<213> Artificial Sequence

<220>

<223> Synthesized Hammerhead Ribozyme

<220>
<223> "n" represents stem II region of a HH ribozyme.

<400> 465

cagggcuacu gaugagncga aaugcaaca                                29

<210> 466
<211> 29
<212> RNA
<213> Artificial Sequence

<220>
<223> Synthesized Hammerhead Ribozyme

<220>
<223> "n" represents stem II region of a HH ribozyme.

<400> 466

ccagggcucu gaugagncga aaaugcaac                                29

<210> 467
<211> 29
<212> RNA
<213> Artificial Sequence

<220>
<223> Synthesized Hammerhead Ribozyme

<220>
<223> "n" represents stem II region of a HH ribozyme.

<400> 467

cccagggccu gaugagncga aaaaugcaa                                29

<210> 468
<211> 29
<212> RNA
<213> Artificial Sequence

<220>
<223> Synthesized Hammerhead Ribozyme

<220>
<223> "n" represents stem II region of a HH ribozyme.

<400> 468

ugcacaagcu gaugagncga aacuguuca                                29

<210> 469
<211> 29
<212> RNA
<213> Artificial Sequence

<220>
<223> Synthesized Hammerhead Ribozyme

<220>
<223> "n" represents stem II region of a HH ribozyme.

<400> 469

uccugcaccu gaugagncga aaguacugu                                          29

<210> 470
<211> 29
<212> RNA
<213> Artificial Sequence

<220>
<223> Synthesized Hammerhead Ribozyme

<220>
<223> "n" represents stem II region of a HH ribozyme.

<400> 470

gccacaaccu gaugagncga aauccugca                                          29

<210> 471
<211> 29
<212> RNA
<213> Artificial Sequence

<220>
<223> Synthesized Hammerhead Ribozyme

<220>
<223> "n" represents stem II region of a HH ribozyme.

<400> 471

guagccaccu gaugagncga aacaauccu                                          29

<210> 472
<211> 29
<212> RNA
<213> Artificial Sequence

<220>
<223> Synthesized Hammerhead Ribozyme

<220>

<223> "n" represents stem II region of a HH ribozyme.

<400> 472

uucucuagcu gaugagncga aagccacaa                                          29

<210> 473
<211> 29
<212> RNA
<213> Artificial Sequence

<220>
<223> Synthesized Hammerhead Ribozyme

<220>
<223> "n" represents stem II region of a HH ribozyme.

<400> 473

uuguucuccu gaugagncga aaguagcca                                          29

<210> 474
<211> 29
<212> RNA
<213> Artificial Sequence

<220>
<223> Synthesized Hammerhead Ribozyme

<220>
<223> "n" represents stem II region of a HH ribozyme.

<400> 474

uucugccccu gaugagncga aacuuuccc                                          29

<210> 475
<211> 29
<212> RNA
<213> Artificial Sequence

<220>
<223> Synthesized Hammerhead Ribozyme

<220>
<223> "n" represents stem II region of a HH ribozyme.

<400> 475

cagaacugcu gaugagncga aauccaguu                                          29

<210> 476
<211> 29

<212> RNA
<213> Artificial Sequence

<220>
<223> Synthesized Hammerhead Ribozyme

<220>
<223> "n" represents stem II region of a HH ribozyme.

<400> 476

gugcucagcu gaugagncga aacuguauc 29

<210> 477
<211> 29
<212> RNA
<213> Artificial Sequence

<220>
<223> Synthesized Hammerhead Ribozyme

<220>
<223> "n" represents stem II region of a HH ribozyme.

<400> 477

ugugcucacu gaugagncga aaacuguau 29

<210> 478
<211> 29
<212> RNA
<213> Artificial Sequence

<220>
<223> Synthesized Hammerhead Ribozyme

<220>
<223> "n" represents stem II region of a HH ribozyme.

<400> 478

accugagccu gaugagncga aagucuggc 29

<210> 479
<211> 29
<212> RNA
<213> Artificial Sequence

<220>
<223> Synthesized Hammerhead Ribozyme

<220>
<223> "n" represents stem II region of a HH ribozyme.

<400> 479

ggccaccucu gaugagncga aagcaaguc                                    29

<210> 480
<211> 29
<212> RNA
<213> Artificial Sequence

<220>
<223> Synthesized Hammerhead Ribozyme

<220>
<223> "n" represents stem II region of a HH ribozyme.

<400> 480

uuccuaggcu gaugagncga aagcugcag                                    29

<210> 481
<211> 29
<212> RNA
<213> Artificial Sequence

<220>
<223> Synthesized Hammerhead Ribozyme

<220>
<223> "n" represents stem II region of a HH ribozyme.

<400> 481

aauguucccu gaugagncga aagguagcu                                    29

<210> 482
<211> 29
<212> RNA
<213> Artificial Sequence

<220>
<223> Synthesized Hammerhead Ribozyme

<220>
<223> "n" represents stem II region of a HH ribozyme.

<400> 482

cugcaaggcu gaugagncga aauguuccu                                    29

<210> 483
<211> 29
<212> RNA
<213> Artificial Sequence

<220>
<223> Synthesized Hammerhead Ribozyme

<220>
<223> "n" represents stem II region of a HH ribozyme.

<400> 483

ucugcaagcu gaugagncga aaauguucc 29

<210> 484
<211> 29
<212> RNA
<213> Artificial Sequence

<220>
<223> Synthesized Hammerhead Ribozyme

<220>
<223> "n" represents stem II region of a HH ribozyme.

<400> 484

gggucugccu gaugagncga aaggaaugu 29

<210> 485
<211> 29
<212> RNA
<213> Artificial Sequence

<220>
<223> Synthesized Hammerhead Ribozyme

<220>
<223> "n" represents stem II region of a HH ribozyme.

<400> 485

ccaaaggccu gaugagncga aaugcgggg 29

<210> 486
<211> 29
<212> RNA
<213> Artificial Sequence

<220>
<223> Synthesized Hammerhead Ribozyme

<220>
<223> "n" represents stem II region of a HH ribozyme.

<400> 486

cacccccacu gaugagncga aaggcaaug 29

<210> 487
<211> 29
<212> RNA
<213> Artificial Sequence

<220>
<223> Synthesized Hammerhead Ribozyme

<220>
<223> "n" represents stem II region of a HH ribozyme.

<400> 487

gcaccccccu gaugagncga aaaggcaau                                          29



<210> 488
<211> 29
<212> RNA
<213> Artificial Sequence

<220>
<223> Synthesized Hammerhead Ribozyme

<220>
<223> "n" represents stem II region of a HH ribozyme.

<400> 488

accccaggcu gaugagncga aaucccagg                                          29



<210> 489
<211> 29
<212> RNA
<213> Artificial Sequence

<220>
<223> Synthesized Hammerhead Ribozyme

<220>
<223> "n" represents stem II region of a HH ribozyme.

<400> 489

agcuggaccu gaugagncga aaccccagg                                          29



<210> 490
<211> 29
<212> RNA
<213> Artificial Sequence

<220>
<223> Synthesized Hammerhead Ribozyme

<220>
<223> "n" represents stem II region of a HH ribozyme.

<400> 490

aagagcugcu gaugagncga aacuacccc                                    29

<210> 491
<211> 29
<212> RNA
<213> Artificial Sequence

<220>
<223> Synthesized Hammerhead Ribozyme

<220>
<223> "n" represents stem II region of a HH ribozyme.

<400> 491

augaauaacu gaugagncga aagcuggac                                    29

<210> 492
<211> 29
<212> RNA
<213> Artificial Sequence

<220>
<223> Synthesized Hammerhead Ribozyme

<220>
<223> "n" represents stem II region of a HH ribozyme.

<400> 492

aaaugaaucu gaugagncga aagagcugg                                    29

<210> 493
<211> 29
<212> RNA
<213> Artificial Sequence

<220>
<223> Synthesized Hammerhead Ribozyme

<220>
<223> "n" represents stem II region of a HH ribozyme.

<400> 493

gaaaugaacu gaugagncga aaagagcug                                    29

<210> 494

<211> 29
<212> RNA
<213> Artificial Sequence

<220>
<223> Synthesized Hammerhead Ribozyme

<220>
<223> "n" represents stem II region of a HH ribozyme.

<400> 494

gggaaaugcu gaugagncga aauaagagc                                          29

<210> 495
<211> 29
<212> RNA
<213> Artificial Sequence

<220>
<223> Synthesized Hammerhead Ribozyme

<220>
<223> "n" represents stem II region of a HH ribozyme.

<400> 495

ugggaaaucu gaugagncga aaauaagag                                          29

<210> 496
<211> 29
<212> RNA
<213> Artificial Sequence

<220>
<223> Synthesized Hammerhead Ribozyme

<220>
<223> "n" represents stem II region of a HH ribozyme.

<400> 496

cgcugggacu gaugagncga aaugaauaa                                          29

<210> 497
<211> 29
<212> RNA
<213> Artificial Sequence

<220>
<223> Synthesized Hammerhead Ribozyme

<220>
<223> "n" represents stem II region of a HH ribozyme.

<400> 497

acgcugggcu gaugagncga aaaugaaua                                          29

<210> 498
<211> 29
<212> RNA
<213> Artificial Sequence

<220>
<223> Synthesized Hammerhead Ribozyme

<220>
<223> "n" represents stem II region of a HH ribozyme.

<400> 498

cacgcuggcu gaugagncga aaaaugaau                                          29

<210> 499
<211> 29
<212> RNA
<213> Artificial Sequence

<220>
<223> Synthesized Hammerhead Ribozyme

<220>
<223> "n" represents stem II region of a HH ribozyme.

<400> 499

cuucuucccu gaugagncga aaccagggc                                          29

<210> 500
<211> 29
<212> RNA
<213> Artificial Sequence

<220>
<223> Synthesized Hammerhead Ribozyme

<220>
<223> "n" represents stem II region of a HH ribozyme.

<400> 500

uacaacuucu gaugagncga aacagcugc                                          29

<210> 501
<211> 29
<212> RNA
<213> Artificial Sequence

<220>
<223> Synthesized Hammerhead Ribozyme

<220>
<223> "n" represents stem II region of a HH ribozyme.

<400> 501

cugucuaccu gaugagncga aacuugaca 29

<210> 502
<211> 29
<212> RNA
<213> Artificial Sequence

<220>
<223> Synthesized Hammerhead Ribozyme

<220>
<223> "n" represents stem II region of a HH ribozyme.

<400> 502

cagcuguccu gaugagncga aacaacuug 29

<210> 503
<211> 29
<212> RNA
<213> Artificial Sequence

<220>
<223> Synthesized Hammerhead Ribozyme

<220>
<223> "n" represents stem II region of a HH ribozyme.

<400> 503

auuguaggcu gaugagncga aacacagcu 29

<210> 504
<211> 29
<212> RNA
<213> Artificial Sequence

<220>
<223> Synthesized Hammerhead Ribozyme

<220>
<223> "n" represents stem II region of a HH ribozyme.

<400> 504

aauuguagcu gaugagncga aaacacagc 29

<210> 505
<211> 29
<212> RNA
<213> Artificial Sequence

<220>
<223> Synthesized Hammerhead Ribozyme

<220>
<223> "n" represents stem II region of a HH ribozyme.

<400> 505

gccaauugcu gaugagncga aaggaacac                                          29

<210> 506
<211> 29
<212> RNA
<213> Artificial Sequence

<220>
<223> Synthesized Hammerhead Ribozyme

<220>
<223> "n" represents stem II region of a HH ribozyme.

<400> 506

gcugggcccu gaugagncga aauuguagg                                          29

<210> 507
<211> 29
<212> RNA
<213> Artificial Sequence

<220>
<223> Synthesized Hammerhead Ribozyme

<220>
<223> "n" represents stem II region of a HH ribozyme.

<400> 507

gugacagccu gaugagncga aacgguccc                                          29

<210> 508
<211> 29
<212> RNA
<213> Artificial Sequence

<220>
<223> Synthesized Hammerhead Ribozyme

<220>
<223> "n" represents stem II region of a HH ribozyme.

<400> 508

ugaguagucu gaugagncga aacagcaac 29

<210> 509
<211> 29
<212> RNA
<213> Artificial Sequence

<220>
<223> Synthesized Hammerhead Ribozyme

<220>
<223> "n" represents stem II region of a HH ribozyme.

<400> 509

agccugagcu gaugagncga aagugacag 29

<210> 510
<211> 29
<212> RNA
<213> Artificial Sequence

<220>
<223> Synthesized Hammerhead Ribozyme

<220>
<223> "n" represents stem II region of a HH ribozyme.

<400> 510

gucagccucu gaugagncga aaguaguga 29

<210> 511
<211> 29
<212> RNA
<213> Artificial Sequence

<220>
<223> Synthesized Hammerhead Ribozyme

<220>
<223> "n" represents stem II region of a HH ribozyme.

<400> 511

cguaaucucu gaugagncga aaccaggcc 29

<210> 512
<211> 29
<212> RNA
<213> Artificial Sequence

<220>
<223> Synthesized Hammerhead Ribozyme

<220>
<223> "n" represents stem II region of a HH ribozyme.

<400> 512

gcauacgucu gaugagncga aaucugacc                                29

<210> 513
<211> 29
<212> RNA
<213> Artificial Sequence

<220>
<223> Synthesized Hammerhead Ribozyme

<220>
<223> "n" represents stem II region of a HH ribozyme.

<400> 513

ggcauacgcu gaugagncga aaaucugac                                29

<210> 514
<211> 29
<212> RNA
<213> Artificial Sequence

<220>
<223> Synthesized Hammerhead Ribozyme

<220>
<223> "n" represents stem II region of a HH ribozyme.

<400> 514

caagggcacu gaugagncga aacguaauc                                29

<210> 515
<211> 29
<212> RNA
<213> Artificial Sequence

<220>
<223> Synthesized Hammerhead Ribozyme

<220>
<223> "n" represents stem II region of a HH ribozyme.

<400> 515

aaaccacccu gaugagncga aagggcaua                                          29

<210> 516
<211> 29
<212> RNA
<213> Artificial Sequence

<220>
<223> Synthesized Hammerhead Ribozyme

<220>
<223> "n" represents stem II region of a HH ribozyme.

<400> 516

uaucucuacu gaugagncga aaccaccaa                                          29

<210> 517
<211> 29
<212> RNA
<213> Artificial Sequence

<220>
<223> Synthesized Hammerhead Ribozyme

<220>
<223> "n" represents stem II region of a HH ribozyme.

<400> 517

uuaucucucu gaugagncga aaaccacca                                          29

<210> 518
<211> 29
<212> RNA
<213> Artificial Sequence

<220>
<223> Synthesized Hammerhead Ribozyme

<220>
<223> "n" represents stem II region of a HH ribozyme.

<400> 518

auuaucuccu gaugagncga aaaaccacc                                          29

<210> 519
<211> 29
<212> RNA

<213> Artificial Sequence

<220>
<223> Synthesized Hammerhead Ribozyme

<220>
<223> "n" represents stem II region of a HH ribozyme.

<400> 519

uuuuggaucu gaugagncga aaucucuaa                                          29



<210> 520
<211> 29
<212> RNA
<213> Artificial Sequence

<220>
<223> Synthesized Hammerhead Ribozyme

<220>
<223> "n" represents stem II region of a HH ribozyme.

<400> 520

ugauuuugcu gaugagncga aauuaucuc                                          29



<210> 521
<211> 29
<212> RNA
<213> Artificial Sequence

<220>
<223> Synthesized Hammerhead Ribozyme

<220>
<223> "n" represents stem II region of a HH ribozyme.

<400> 521

caaacccucu gaugagncga aauuuugga                                          29



<210> 522
<211> 29
<212> RNA
<213> Artificial Sequence

<220>
<223> Synthesized Hammerhead Ribozyme

<220>
<223> "n" represents stem II region of a HH ribozyme.

<400> 522

ccaaaccacu gaugagncga aacccugau                                    29

<210> 523
<211> 29
<212> RNA
<213> Artificial Sequence

<220>
<223> Synthesized Hammerhead Ribozyme

<220>
<223> "n" represents stem II region of a HH ribozyme.

<400> 523

cccaaacccu gaugagncga aaacccuga                                    29

<210> 524
<211> 29
<212> RNA
<213> Artificial Sequence

<220>
<223> Synthesized Hammerhead Ribozyme

<220>
<223> "n" represents stem II region of a HH ribozyme.

<400> 524

cuuccccacu gaugagncga aaccaaacc                                    29

<210> 525
<211> 29
<212> RNA
<213> Artificial Sequence

<220>
<223> Synthesized Hammerhead Ribozyme

<220>
<223> "n" represents stem II region of a HH ribozyme.

<400> 525

ucuucccccu gaugagncga aaaccaaac                                    29

<210> 526
<211> 29
<212> RNA
<213> Artificial Sequence

<220>

<223> Synthesized Hammerhead Ribozyme

<220>
<223> "n" represents stem II region of a HH ribozyme.

<400> 526
   agggggagcu gaugagncga aauuuucuu                                    29

<210> 527
<211> 29
<212> RNA
<213> Artificial Sequence

<220>
<223> Synthesized Hammerhead Ribozyme

<220>
<223> "n" represents stem II region of a HH ribozyme.

<400> 527

ggaaggggcu gaugagncga aaggauuuu                                    29

<210> 528
<211> 29
<212> RNA
<213> Artificial Sequence

<220>
<223> Synthesized Hammerhead Ribozyme

<220>
<223> "n" represents stem II region of a HH ribozyme.

<400> 528

gggggaggcu gaugagncga aagggggag                                    29

<210> 529
<211> 29
<212> RNA
<213> Artificial Sequence

<220>
<223> Synthesized Hammerhead Ribozyme

<220>
<223> "n" represents stem II region of a HH ribozyme.

<400> 529
   cgggggagcu gaugagncga aaaggggga                                    29

<210> 530
<211> 29
<212> RNA
<213> Artificial Sequence

<220>
<223> Synthesized Hammerhead Ribozyme

<220>
<223> "n" represents stem II region of a HH ribozyme.

<400> 530

gggcggggcu gaugagncga aaggaaggg                                          29



<210> 531
<211> 29
<212> RNA
<213> Artificial Sequence

<220>
<223> Synthesized Hammerhead Ribozyme

<220>
<223> "n" represents stem II region of a HH ribozyme.

<400> 531

cgguagggcu gaugagncga aacggggcg                                          29



<210> 532
<211> 29
<212> RNA
<213> Artificial Sequence

<220>
<223> Synthesized Hammerhead Ribozyme

<220>
<223> "n" represents stem II region of a HH ribozyme.

<400> 532

gcgguaggcu gaugagncga aaacggggc                                          29



<210> 533
<211> 29
<212> RNA
<213> Artificial Sequence

<220>
<223> Synthesized Hammerhead Ribozyme

<220>

<223> "n" represents stem II region of a HH ribozyme.

<400> 533

ggaggcggcu gaugagncga aagggaacg 29

<210> 534
<211> 29
<212> RNA
<213> Artificial Sequence

<220>
<223> Synthesized Hammerhead Ribozyme

<220>
<223> "n" represents stem II region of a HH ribozyme.

<400> 534

caggagugcu gaugagncga aaggcggua 29

<210> 535
<211> 29
<212> RNA
<213> Artificial Sequence

<220>
<223> Synthesized Hammerhead Ribozyme

<220>
<223> "n" represents stem II region of a HH ribozyme.

<400> 535

gcuggcagcu gaugagncga aaguggagg 29

<210> 536
<211> 29
<212> RNA
<213> Artificial Sequence

<220>
<223> Synthesized Hammerhead Ribozyme

<220>
<223> "n" represents stem II region of a HH ribozyme.

<400> 536

aaggaaaucu gaugagncga aagcuggca 29

<210> 537
<211> 29

<212> RNA
<213> Artificial Sequence

<220>
<223> Synthesized Hammerhead Ribozyme

<220>
<223> "n" represents stem II region of a HH ribozyme.

<400> 537

uugaaggacu gaugagncga aaugagcug                                29

<210> 538
<211> 29
<212> RNA
<213> Artificial Sequence

<220>
<223> Synthesized Hammerhead Ribozyme

<220>
<223> "n" represents stem II region of a HH ribozyme.

<400> 538

auugaaggcu gaugagncga aaaugagcu                                29

<210> 539
<211> 29
<212> RNA
<213> Artificial Sequence

<220>
<223> Synthesized Hammerhead Ribozyme

<220>
<223> "n" represents stem II region of a HH ribozyme.

<400> 539

aauugaagcu gaugagncga aaaaugagc                                29

<210> 540
<211> 29
<212> RNA
<213> Artificial Sequence

<220>
<223> Synthesized Hammerhead Ribozyme

<220>
<223> "n" represents stem II region of a HH ribozyme.

<400> 540

ggaaauugcu gaugagncga aaggaaaug 29

<210> 541
<211> 29
<212> RNA
<213> Artificial Sequence

<220>
<223> Synthesized Hammerhead Ribozyme

<220>
<223> "n" represents stem II region of a HH ribozyme.

<400> 541

aggaaauucu gaugagncga aaaggaaau 29

<210> 542
<211> 29
<212> RNA
<213> Artificial Sequence

<220>
<223> Synthesized Hammerhead Ribozyme

<220>
<223> "n" represents stem II region of a HH ribozyme.

<400> 542

ucaaaggacu gaugagncga aauugaagg 29

<210> 543
<211> 29
<212> RNA
<213> Artificial Sequence

<220>
<223> Synthesized Hammerhead Ribozyme

<220>
<223> "n" represents stem II region of a HH ribozyme.

<400> 543

gucaaaggcu gaugagncga aaauugaag 29

<210> 544
<211> 29
<212> RNA
<213> Artificial Sequence

<220>
<223> Synthesized Hammerhead Ribozyme

<220>
<223> "n" represents stem II region of a HH ribozyme.

<400> 544

ggucaaagcu gaugagncga aaaauugaa 29

<210> 545
<211> 29
<212> RNA
<213> Artificial Sequence

<220>
<223> Synthesized Hammerhead Ribozyme

<220>
<223> "n" represents stem II region of a HH ribozyme.

<400> 545

auaggucacu gaugagncga aaggaaauu 29

<210> 546
<211> 29
<212> RNA
<213> Artificial Sequence

<220>
<223> Synthesized Hammerhead Ribozyme

<220>
<223> "n" represents stem II region of a HH ribozyme.

<400> 546

uauagguccu gaugagncga aaaggaaau 29

<210> 547
<211> 29
<212> RNA
<213> Artificial Sequence

<220>
<223> Synthesized Hammerhead Ribozyme

<220>
<223> "n" represents stem II region of a HH ribozyme.

<400> 547

uuagccuacu gaugagncga aaggucaaa 29

<210> 548
<211> 29
<212> RNA
<213> Artificial Sequence

<220>
<223> Synthesized Hammerhead Ribozyme

<220>
<223> "n" represents stem II region of a HH ribozyme.

<400> 548

uuuuagcccu gaugagncga aauagguca                                          29



<210> 549
<211> 29
<212> RNA
<213> Artificial Sequence

<220>
<223> Synthesized Hammerhead Ribozyme

<220>
<223> "n" represents stem II region of a HH ribozyme.

<400> 549

uucuuuuucu gaugagncga aagccuaua                                          29



<210> 550
<211> 29
<212> RNA
<213> Artificial Sequence

<220>
<223> Synthesized Hammerhead Ribozyme

<220>
<223> "n" represents stem II region of a HH ribozyme.

<400> 550

gcuggaaucu gaugagncga aagccuuuc                                          29



<210> 551
<211> 29
<212> RNA
<213> Artificial Sequence

<220>
<223> Synthesized Hammerhead Ribozyme

<220>
<223> "n" represents stem II region of a HH ribozyme.

<400> 551

guggcuggcu gaugagncga aaugagccu                                    29

<210> 552
<211> 29
<212> RNA
<213> Artificial Sequence

<220>
<223> Synthesized Hammerhead Ribozyme

<220>
<223> "n" represents stem II region of a HH ribozyme.

<400> 552

uguggcugcu gaugagncga aaaugagcc                                    29

<210> 553
<211> 29
<212> RNA
<213> Artificial Sequence

<220>
<223> Synthesized Hammerhead Ribozyme

<220>
<223> "n" represents stem II region of a HH ribozyme.

<400> 553

gcccagggcu gaugagncga aaggcugcc                                    29

<210> 554
<211> 29
<212> RNA
<213> Artificial Sequence

<220>
<223> Synthesized Hammerhead Ribozyme

<220>
<223> "n" represents stem II region of a HH ribozyme.

<400> 554

ggcccaggcu gaugagncga aaaggcugc                                    29

<210> 555

<211> 29
<212> RNA
<213> Artificial Sequence

<220>
<223> Synthesized Hammerhead Ribozyme

<220>
<223> "n" represents stem II region of a HH ribozyme.

<400> 555

gagaagcacu gaugagncga aaggcccag                                    29



<210> 556
<211> 29
<212> RNA
<213> Artificial Sequence

<220>
<223> Synthesized Hammerhead Ribozyme

<220>
<223> "n" represents stem II region of a HH ribozyme.

<400> 556

agagaagccu gaugagncga aaaggccca                                    29



<210> 557
<211> 29
<212> RNA
<213> Artificial Sequence

<220>
<223> Synthesized Hammerhead Ribozyme

<220>
<223> "n" represents stem II region of a HH ribozyme.

<400> 557

ugcuagagcu gaugagncga aagcaaagg                                    29



<210> 558
<211> 29
<212> RNA
<213> Artificial Sequence

<220>
<223> Synthesized Hammerhead Ribozyme

<220>
<223> "n" represents stem II region of a HH ribozyme.

<400> 558

gugcuagacu gaugagncga aaagcaaag                                29

<210> 559
<211> 29
<212> RNA
<213> Artificial Sequence

<220>
<223> Synthesized Hammerhead Ribozyme

<220>
<223> "n" represents stem II region of a HH ribozyme.

<400> 559

uugugcuacu gaugagncga aagaagcaa                                29

<210> 560
<211> 29
<212> RNA
<213> Artificial Sequence

<220>
<223> Synthesized Hammerhead Ribozyme

<220>
<223> "n" represents stem II region of a HH ribozyme.

<400> 560

aauugugccu gaugagncga aagagaagc                                29

<210> 561
<211> 29
<212> RNA
<213> Artificial Sequence

<220>
<223> Synthesized Hammerhead Ribozyme

<220>
<223> "n" represents stem II region of a HH ribozyme.

<400> 561

uaacccaucu gaugagncga aauugugcu                                29

<210> 562
<211> 29
<212> RNA
<213> Artificial Sequence

<220>
<223> Synthesized Hammerhead Ribozyme

<220>
<223> "n" represents stem II region of a HH ribozyme.

<400> 562

guaacccacu gaugagncga aaauugugc                                    29



<210> 563
<211> 29
<212> RNA
<213> Artificial Sequence

<220>
<223> Synthesized Hammerhead Ribozyme

<220>
<223> "n" represents stem II region of a HH ribozyme.

<400> 563

aaggaagucu gaugagncga aacccauaa                                    29



<210> 564
<211> 29
<212> RNA
<213> Artificial Sequence

<220>
<223> Synthesized Hammerhead Ribozyme

<220>
<223> "n" represents stem II region of a HH ribozyme.

<400> 564

aaaggaagcu gaugagncga aaacccaua                                    29



<210> 565
<211> 29
<212> RNA
<213> Artificial Sequence

<220>
<223> Synthesized Hammerhead Ribozyme

<220>
<223> "n" represents stem II region of a HH ribozyme.

<400> 565

gaaaaaggcu gaugagncga aaguaaccc                                    29

<210> 566
<211> 29
<212> RNA
<213> Artificial Sequence

<220>
<223> Synthesized Hammerhead Ribozyme

<220>
<223> "n" represents stem II region of a HH ribozyme.

<400> 566

agaaaaagcu gaugagncga aaaguaacc 29

<210> 567
<211> 29
<212> RNA
<213> Artificial Sequence

<220>
<223> Synthesized Hammerhead Ribozyme

<220>
<223> "n" represents stem II region of a HH ribozyme.

<400> 567

uuaagaaacu gaugagncga aaggaagua 29

<210> 568
<211> 29
<212> RNA
<213> Artificial Sequence

<220>
<223> Synthesized Hammerhead Ribozyme

<220>
<223> "n" represents stem II region of a HH ribozyme.

<400> 568

guuaagaacu gaugagncga aaaggaagu 29

<210> 569
<211> 29
<212> RNA
<213> Artificial Sequence

<220>
<223> Synthesized Hammerhead Ribozyme

<220>
<223> "n" represents stem II region of a HH ribozyme.

<400> 569

uguuaagacu gaugagncga aaaaggaag                                    29

<210> 570
<211> 29
<212> RNA
<213> Artificial Sequence

<220>
<223> Synthesized Hammerhead Ribozyme

<220>
<223> "n" represents stem II region of a HH ribozyme.

<400> 570

uuguuaagcu gaugagncga aaaaaggaa                                    29

<210> 571
<211> 29
<212> RNA
<213> Artificial Sequence

<220>
<223> Synthesized Hammerhead Ribozyme

<220>
<223> "n" represents stem II region of a HH ribozyme.

<400> 571

uuuguuaacu gaugagncga aaaaaagga                                    29

<210> 572
<211> 29
<212> RNA
<213> Artificial Sequence

<220>
<223> Synthesized Hammerhead Ribozyme

<220>
<223> "n" represents stem II region of a HH ribozyme.

<400> 572

uuuuuguucu gaugagncga aagaaaaag                                    29

<210> 573
<211> 29
<212> RNA
<213> Artificial Sequence

<220>
<223> Synthesized Hammerhead Ribozyme

<220>
<223> "n" represents stem II region of a HH ribozyme.

<400> 573

uuuuuugucu gaugagncga aaagaaaaa                                          29

<210> 574
<211> 29
<212> RNA
<213> Artificial Sequence

<220>
<223> Synthesized Hammerhead Ribozyme

<220>
<223> "n" represents stem II region of a HH ribozyme.

<400> 574

ggaaaucacu gaugagncga aacauucuu                                          29

<210> 575
<211> 29
<212> RNA
<213> Artificial Sequence

<220>
<223> Synthesized Hammerhead Ribozyme

<220>
<223> "n" represents stem II region of a HH ribozyme.

<400> 575

aggaaauccu gaugagncga aaacauucu                                          29

<210> 576
<211> 29
<212> RNA
<213> Artificial Sequence

<220>
<223> Synthesized Hammerhead Ribozyme

<220>
<223> "n" represents stem II region of a HH ribozyme.

<400> 576

ccagaggacu gaugagncga aaucaaaca                                29

<210> 577
<211> 29
<212> RNA
<213> Artificial Sequence

<220>
<223> Synthesized Hammerhead Ribozyme

<220>
<223> "n" represents stem II region of a HH ribozyme.

<400> 577

cccagaggcu gaugagncga aaaucaaac                                29

<210> 578
<211> 29
<212> RNA
<213> Artificial Sequence

<220>
<223> Synthesized Hammerhead Ribozyme

<220>
<223> "n" represents stem II region of a HH ribozyme.

<400> 578

acccagagcu gaugagncga aaaaucaaa                                29

<210> 579
<211> 29
<212> RNA
<213> Artificial Sequence

<220>
<223> Synthesized Hammerhead Ribozyme

<220>
<223> "n" represents stem II region of a HH ribozyme.

<400> 579

gucacccacu gaugagncga aaggaaauc                                29

<210> 580
<211> 29
<212> RNA

<213> Artificial Sequence

<220>
<223> Synthesized Hammerhead Ribozyme

<220>
<223> "n" represents stem II region of a HH ribozyme.

<400> 580

cagacaaucu gaugagncga aaggucacc                                          29

<210> 581
<211> 29
<212> RNA
<213> Artificial Sequence

<220>
<223> Synthesized Hammerhead Ribozyme

<220>
<223> "n" represents stem II region of a HH ribozyme.

<400> 581

acagacaacu gaugagncga aaaggucac                                          29

<210> 582
<211> 29
<212> RNA
<213> Artificial Sequence

<220>
<223> Synthesized Hammerhead Ribozyme

<220>
<223> "n" represents stem II region of a HH ribozyme.

<400> 582

uuacagaccu gaugagncga aauaagguc                                          29

<210> 583
<211> 29
<212> RNA
<213> Artificial Sequence

<220>
<223> Synthesized Hammerhead Ribozyme

<220>
<223> "n" represents stem II region of a HH ribozyme.

<400> 583

caauuacacu gaugagncga aacaauaag                                    29

<210> 584
<211> 29
<212> RNA
<213> Artificial Sequence

<220>
<223> Synthesized Hammerhead Ribozyme

<220>
<223> "n" represents stem II region of a HH ribozyme.

<400> 584

guuucaaucu gaugagncga aacagacaa                                    29

<210> 585
<211> 29
<212> RNA
<213> Artificial Sequence

<220>
<223> Synthesized Hammerhead Ribozyme

<220>
<223> "n" represents stem II region of a HH ribozyme.

<400> 585

aggguuuccu gaugagncga aauuacaga                                    29

<210> 586
<211> 29
<212> RNA
<213> Artificial Sequence

<220>
<223> Synthesized Hammerhead Ribozyme

<220>
<223> "n" represents stem II region of a HH ribozyme.

<400> 586

ccucucaacu gaugagncga aaggguuuc                                    29

<210> 587
<211> 29
<212> RNA
<213> Artificial Sequence

<220>

<223> Synthesized Hammerhead Ribozyme

<220>
<223> "n" represents stem II region of a HH ribozyme.

<400> 587

caccucuccu gaugagncga aauaggguu                                          29



<210> 588
<211> 29
<212> RNA
<213> Artificial Sequence

<220>
<223> Synthesized Hammerhead Ribozyme

<220>
<223> "n" represents stem II region of a HH ribozyme.

<400> 588

cuaacacacu gaugagncga aacaucacc                                          29



<210> 589
<211> 29
<212> RNA
<213> Artificial Sequence

<220>
<223> Synthesized Hammerhead Ribozyme

<220>
<223> "n" represents stem II region of a HH ribozyme.

<400> 589

cauuggcucu gaugagncga aacacagac                                          29



<210> 590
<211> 29
<212> RNA
<213> Artificial Sequence

<220>
<223> Synthesized Hammerhead Ribozyme

<220>
<223> "n" represents stem II region of a HH ribozyme.

<400> 590

ucauuggccu gaugagncga aaacacaga                                          29

<210> 591
<211> 29
<212> RNA
<213> Artificial Sequence

<220>
<223> Synthesized Hammerhead Ribozyme

<220>
<223> "n" represents stem II region of a HH ribozyme.

<400> 591

cgagcagccu gaugagncga aaccugggu                                          29



<210> 592
<211> 29
<212> RNA
<213> Artificial Sequence

<220>
<223> Synthesized Hammerhead Ribozyme

<220>
<223> "n" represents stem II region of a HH ribozyme.

<400> 592

agaagccccu gaugagncga aagcagcua                                          29



<210> 593
<211> 29
<212> RNA
<213> Artificial Sequence

<220>
<223> Synthesized Hammerhead Ribozyme

<220>
<223> "n" represents stem II region of a HH ribozyme.

<400> 593

accaagagcu gaugagncga aagcccgag                                          29



<210> 594
<211> 29
<212> RNA
<213> Artificial Sequence

<220>
<223> Synthesized Hammerhead Ribozyme

<220>

<223> "n" represents stem II region of a HH ribozyme.

<400> 594

uaccaagacu gaugagncga aaagcccga                                        29

<210> 595
<211> 29
<212> RNA
<213> Artificial Sequence

<220>
<223> Synthesized Hammerhead Ribozyme

<220>
<223> "n" represents stem II region of a HH ribozyme.

<400> 595

cauaccaacu gaugagncga aagaagccc                                        29

<210> 596
<211> 29
<212> RNA
<213> Artificial Sequence

<220>
<223> Synthesized Hammerhead Ribozyme

<220>
<223> "n" represents stem II region of a HH ribozyme.

<400> 596

gacauacccu gaugagncga aagagaagc                                        29

<210> 597
<211> 29
<212> RNA
<213> Artificial Sequence

<220>
<223> Synthesized Hammerhead Ribozyme

<220>
<223> "n" represents stem II region of a HH ribozyme.

<400> 597

acaagacacu gaugagncga aaccaagag                                        29

<210> 598
<211> 29

<212> RNA
<213> Artificial Sequence

<220>
<223> Synthesized Hammerhead Ribozyme

<220>
<223> "n" represents stem II region of a HH ribozyme.

<400> 598

ccaaacaacu gaugagncga aacauacca 29

<210> 599
<211> 29
<212> RNA
<213> Artificial Sequence

<220>
<223> Synthesized Hammerhead Ribozyme

<220>
<223> "n" represents stem II region of a HH ribozyme.

<400> 599

uuccaaaccu gaugagncga aagacauac 29

<210> 600
<211> 29
<212> RNA
<213> Artificial Sequence

<220>
<223> Synthesized Hammerhead Ribozyme

<220>
<223> "n" represents stem II region of a HH ribozyme.

<400> 600

cuuuuccacu gaugagncga aacaagaca 29

<210> 601
<211> 29
<212> RNA
<213> Artificial Sequence

<220>
<223> Synthesized Hammerhead Ribozyme

<220>
<223> "n" represents stem II region of a HH ribozyme.

<400> 601

acuuuucccu gaugagncga aaacaagac 29

<210> 602
<211> 29
<212> RNA
<213> Artificial Sequence

<220>
<223> Synthesized Hammerhead Ribozyme

<220>
<223> "n" represents stem II region of a HH ribozyme.

<400> 602

augaaugacu gaugagncga aauccacuu 29

<210> 603
<211> 29
<212> RNA
<213> Artificial Sequence

<220>
<223> Synthesized Hammerhead Ribozyme

<220>
<223> "n" represents stem II region of a HH ribozyme.

<400> 603

aaugaaugcu gaugagncga aaauccacu 29

<210> 604
<211> 29
<212> RNA
<213> Artificial Sequence

<220>
<223> Synthesized Hammerhead Ribozyme

<220>
<223> "n" represents stem II region of a HH ribozyme.

<400> 604

aaaugaaucu gaugagncga aaaauccac 29

<210> 605
<211> 29
<212> RNA
<213> Artificial Sequence

<220>
<223> Synthesized Hammerhead Ribozyme

<220>
<223> "n" represents stem II region of a HH ribozyme.

<400> 605

cagaaaugcu gaugagncga aaugaaauc                                    29

<210> 606
<211> 29
<212> RNA
<213> Artificial Sequence

<220>
<223> Synthesized Hammerhead Ribozyme

<220>
<223> "n" represents stem II region of a HH ribozyme.

<400> 606

ucagaaaucu gaugagncga aaaugaaau                                    29

<210> 607
<211> 29
<212> RNA
<213> Artificial Sequence

<220>
<223> Synthesized Hammerhead Ribozyme

<220>
<223> "n" represents stem II region of a HH ribozyme.

<400> 607

caaucagacu gaugagncga aaugaauga                                    29

<210> 608
<211> 29
<212> RNA
<213> Artificial Sequence

<220>
<223> Synthesized Hammerhead Ribozyme

<220>
<223> "n" represents stem II region of a HH ribozyme.

<400> 608

acaaucagcu gaugagncga aaaugaaug                                    29

<210> 609
<211> 29
<212> RNA
<213> Artificial Sequence

<220>
<223> Synthesized Hammerhead Ribozyme

<220>
<223> "n" represents stem II region of a HH ribozyme.

<400> 609

gacaaucacu gaugagncga aaaaugaau                                          29



<210> 610
<211> 29
<212> RNA
<213> Artificial Sequence

<220>
<223> Synthesized Hammerhead Ribozyme

<220>
<223> "n" represents stem II region of a HH ribozyme.

<400> 610

aacuggaccu gaugagncga aaucagaaa                                          29



<210> 611
<211> 29
<212> RNA
<213> Artificial Sequence

<220>
<223> Synthesized Hammerhead Ribozyme

<220>
<223> "n" represents stem II region of a HH ribozyme.

<400> 611

cuuaacugcu gaugagncga aacaaucag                                          29



<210> 612
<211> 29
<212> RNA
<213> Artificial Sequence

<220>
<223> Synthesized Hammerhead Ribozyme

<220>
<223> "n" represents stem II region of a HH ribozyme.

<400> 612

gaucacuucu gaugagncga aacuggaca                                    29

<210> 613
<211> 29
<212> RNA
<213> Artificial Sequence

<220>
<223> Synthesized Hammerhead Ribozyme

<220>
<223> "n" represents stem II region of a HH ribozyme.

<400> 613

ugaucacucu gaugagncga aaacuggac                                    29

<210> 614
<211> 29
<212> RNA
<213> Artificial Sequence

<220>
<223> Synthesized Hammerhead Ribozyme

<220>
<223> "n" represents stem II region of a HH ribozyme.

<400> 614

ccuuuggucu gaugagncga aaucacuua                                    29

<210> 615
<211> 29
<212> RNA
<213> Artificial Sequence

<220>
<223> Synthesized Hammerhead Ribozyme

<220>
<223> "n" represents stem II region of a HH ribozyme.

<400> 615

cccucccacu gaugagncga aauucucag                                    29

<210> 616

<211> 29
<212> RNA
<213> Artificial Sequence

<220>
<223> Synthesized Hammerhead Ribozyme

<220>
<223> "n" represents stem II region of a HH ribozyme.

<400> 616
   cacauaaacu gaugagncga aacuuuuuu                                          29

<210> 617
<211> 29
<212> RNA
<213> Artificial Sequence

<220>
<223> Synthesized Hammerhead Ribozyme

<220>
<223> "n" represents stem II region of a HH ribozyme.

<400> 617
   gcacauaacu gaugagncga aaacuuuuu                                          29

<210> 618
<211> 29
<212> RNA
<213> Artificial Sequence

<220>
<223> Synthesized Hammerhead Ribozyme

<220>
<223> "n" represents stem II region of a HH ribozyme.

<400> 618
   ugcacauacu gaugagncga aaaacuuuu                                          29

<210> 619
<211> 29
<212> RNA
<213> Artificial Sequence

<220>
<223> Synthesized Hammerhead Ribozyme

<220>
<223> "n" represents stem II region of a HH ribozyme.

<400> 619

gugcacaucu gaugagncga aaaaacuuu                                    29



<210> 620
<211> 29
<212> RNA
<213> Artificial Sequence

<220>
<223> Synthesized Hammerhead Ribozyme

<220>
<223> "n" represents stem II region of a HH ribozyme.

<400> 620

agugcacacu gaugagncga aaaaaacuu                                    29



<210> 621
<211> 29
<212> RNA
<213> Artificial Sequence

<220>
<223> Synthesized Hammerhead Ribozyme

<220>
<223> "n" represents stem II region of a HH ribozyme.

<400> 621

ccaaauuucu gaugagncga aagugcaca                                    29



<210> 622
<211> 29
<212> RNA
<213> Artificial Sequence

<220>
<223> Synthesized Hammerhead Ribozyme

<220>
<223> "n" represents stem II region of a HH ribozyme.

<400> 622

cccaaauucu gaugagncga aaagugcac                                    29



<210> 623
<211> 29
<212> RNA
<213> Artificial Sequence

<220>
<223> Synthesized Hammerhead Ribozyme

<220>
<223> "n" represents stem II region of a HH ribozyme.

<400> 623

uguccccacu gaugagncga aauuuaagu                                    29



<210> 624
<211> 29
<212> RNA
<213> Artificial Sequence

<220>
<223> Synthesized Hammerhead Ribozyme

<220>
<223> "n" represents stem II region of a HH ribozyme.

<400> 624
   uugucccccu gaugagncga aaauuuaag                                    29



<210> 625
<211> 29
<212> RNA
<213> Artificial Sequence

<220>
<223> Synthesized Hammerhead Ribozyme

<220>
<223> "n" represents stem II region of a HH ribozyme.

<400> 625

auacauaacu gaugagncga aauuguccc                                    29



<210> 626
<211> 29
<212> RNA
<213> Artificial Sequence

<220>
<223> Synthesized Hammerhead Ribozyme

<220>
<223> "n" represents stem II region of a HH ribozyme.

<400> 626

gauacauacu gaugagncga aaauugucc                                    29

<210> 627
<211> 29
<212> RNA
<213> Artificial Sequence

<220>
<223> Synthesized Hammerhead Ribozyme

<220>
<223> "n" represents stem II region of a HH ribozyme.

<400> 627

agauacaucu gaugagncga aaaauuguc                                29

<210> 628
<211> 29
<212> RNA
<213> Artificial Sequence

<220>
<223> Synthesized Hammerhead Ribozyme

<220>
<223> "n" represents stem II region of a HH ribozyme.

<400> 628

cagauacacu gaugagncga aaaaauugu                                29

<210> 629
<211> 29
<212> RNA
<213> Artificial Sequence

<220>
<223> Synthesized Hammerhead Ribozyme

<220>
<223> "n" represents stem II region of a HH ribozyme.

<400> 629

aacacagacu gaugagncga aacauaaaa                                29

<210> 630
<211> 29
<212> RNA
<213> Artificial Sequence

<220>
<223> Synthesized Hammerhead Ribozyme

<220>
<223> "n" represents stem II region of a HH ribozyme.

<400> 630

uuaacacacu gaugagncga aauacauaa                                    29



<210> 631
<211> 29
<212> RNA
<213> Artificial Sequence

<220>
<223> Synthesized Hammerhead Ribozyme

<220>
<223> "n" represents stem II region of a HH ribozyme.

<400> 631

auauccuucu gaugagncga aacacagau                                    29



<210> 632
<211> 29
<212> RNA
<213> Artificial Sequence

<220>
<223> Synthesized Hammerhead Ribozyme

<220>
<223> "n" represents stem II region of a HH ribozyme.

<400> 632

cauauccucu gaugagncga aaacacaga                                    29



<210> 633
<211> 29
<212> RNA
<213> Artificial Sequence

<220>
<223> Synthesized Hammerhead Ribozyme

<220>
<223> "n" represents stem II region of a HH ribozyme.

<400> 633

cuuaagcacu gaugagncga aauccuuaa                                    29

<210> 634
<211> 29
<212> RNA
<213> Artificial Sequence

<220>
<223> Synthesized Hammerhead Ribozyme

<220>
<223> "n" represents stem II region of a HH ribozyme.

<400> 634

auguucuucu gaugagncga aagcauauc                                29

<210> 635
<211> 29
<212> RNA
<213> Artificial Sequence

<220>
<223> Synthesized Hammerhead Ribozyme

<220>
<223> "n" represents stem II region of a HH ribozyme.

<400> 635

uauguucucu gaugagncga aaagcauau                                29

<210> 636
<211> 29
<212> RNA
<213> Artificial Sequence

<220>
<223> Synthesized Hammerhead Ribozyme

<220>
<223> "n" represents stem II region of a HH ribozyme.

<400> 636

aaaagaaucu gaugagncga aauguucuu                                29

<210> 637
<211> 29
<212> RNA
<213> Artificial Sequence

<220>
<223> Synthesized Hammerhead Ribozyme

<220>
<223> "n" represents stem II region of a HH ribozyme.

<400> 637

aacaaaagcu gaugagncga aauuauguu                                29

<210> 638
<211> 29
<212> RNA
<213> Artificial Sequence

<220>
<223> Synthesized Hammerhead Ribozyme

<220>
<223> "n" represents stem II region of a HH ribozyme.

<400> 638

caacaaaacu gaugagncga aaauuaugu                                29

<210> 639
<211> 29
<212> RNA
<213> Artificial Sequence

<220>
<223> Synthesized Hammerhead Ribozyme

<220>
<223> "n" represents stem II region of a HH ribozyme.

<400> 639

agcaacaacu gaugagncga aagaauuau                                29

<210> 640
<211> 29
<212> RNA
<213> Artificial Sequence

<220>
<223> Synthesized Hammerhead Ribozyme

<220>
<223> "n" represents stem II region of a HH ribozyme.

<400> 640

cagcaacacu gaugagncga aaagaauua                                29

<210> 641
<211> 29
<212> RNA

<213> Artificial Sequence

<220>
<223> Synthesized Hammerhead Ribozyme

<220>
<223> "n" represents stem II region of a HH ribozyme.

<400> 641

acagcaaccu gaugagncga aaaagaauu                                    29

<210> 642
<211> 29
<212> RNA
<213> Artificial Sequence

<220>
<223> Synthesized Hammerhead Ribozyme

<220>
<223> "n" represents stem II region of a HH ribozyme.

<400> 642

caaacagccu gaugagncga aacaaaaga                                    29

<210> 643
<211> 29
<212> RNA
<213> Artificial Sequence

<220>
<223> Synthesized Hammerhead Ribozyme

<220>
<223> "n" represents stem II region of a HH ribozyme.

<400> 643

cuuaaacacu gaugagncga aacagcaac                                    29

<210> 644
<211> 29
<212> RNA
<213> Artificial Sequence

<220>
<223> Synthesized Hammerhead Ribozyme

<220>
<223> "n" represents stem II region of a HH ribozyme.

<400> 644

ucuuaaaccu gaugagncga aaacagcaa 29

<210> 645
<211> 29
<212> RNA
<213> Artificial Sequence

<220>
<223> Synthesized Hammerhead Ribozyme

<220>
<223> "n" represents stem II region of a HH ribozyme.

<400> 645

gcuucuuacu gaugagncga aacaaacag 29

<210> 646
<211> 29
<212> RNA
<213> Artificial Sequence

<220>
<223> Synthesized Hammerhead Ribozyme

<220>
<223> "n" represents stem II region of a HH ribozyme.

<400> 646

ugcuucuucu gaugagncga aaacaaaca 29

<210> 647
<211> 29
<212> RNA
<213> Artificial Sequence

<220>
<223> Synthesized Hammerhead Ribozyme

<220>
<223> "n" represents stem II region of a HH ribozyme.

<400> 647

gugcuucucu gaugagncga aaaacaaac 29

<210> 648
<211> 29
<212> RNA
<213> Artificial Sequence

<220>

<223> Synthesized Hammerhead Ribozyme

<220>
<223> "n" represents stem II region of a HH ribozyme.

<400> 648

aacaaacucu gaugagncga aaggugcuu                                          29



<210> 649
<211> 29
<212> RNA
<213> Artificial Sequence

<220>
<223> Synthesized Hammerhead Ribozyme

<220>
<223> "n" represents stem II region of a HH ribozyme.

<400> 649

aaacaaaccu gaugagncga aaaggugcu                                          29



<210> 650
<211> 29
<212> RNA
<213> Artificial Sequence

<220>
<223> Synthesized Hammerhead Ribozyme

<220>
<223> "n" represents stem II region of a HH ribozyme.

<400> 650

cuuaaacacu gaugagncga aacuaaggu                                          29



<210> 651
<211> 29
<212> RNA
<213> Artificial Sequence

<220>
<223> Synthesized Hammerhead Ribozyme

<220>
<223> "n" represents stem II region of a HH ribozyme.

<400> 651

ucuuaaaccu gaugagncga aaacuaagg                                          29

<210> 652
<211> 29
<212> RNA
<213> Artificial Sequence

<220>
<223> Synthesized Hammerhead Ribozyme

<220>
<223> "n" represents stem II region of a HH ribozyme.

<400> 652

gcuucuuacu gaugagncga aacaaacua                                    29

<210> 653
<211> 29
<212> RNA
<213> Artificial Sequence

<220>
<223> Synthesized Hammerhead Ribozyme

<220>
<223> "n" represents stem II region of a HH ribozyme.

<400> 653

ugcuucuucu gaugagncga aaacaaacu                                    29

<210> 654
<211> 29
<212> RNA
<213> Artificial Sequence

<220>
<223> Synthesized Hammerhead Ribozyme

<220>
<223> "n" represents stem II region of a HH ribozyme.

<400> 654

gugcuucucu gaugagncga aaaacaaac                                    29

<210> 655
<211> 29
<212> RNA
<213> Artificial Sequence

<220>
<223> Synthesized Hammerhead Ribozyme

<220>

<223> "n" represents stem II region of a HH ribozyme.

<400> 655

uacuauaucu gaugagncga aaggugcuu                                    29

<210> 656
<211> 29
<212> RNA
<213> Artificial Sequence

<220>
<223> Synthesized Hammerhead Ribozyme

<220>
<223> "n" represents stem II region of a HH ribozyme.

<400> 656

auacuauacu gaugagncga aaaggugcu                                    29

<210> 657
<211> 29
<212> RNA
<213> Artificial Sequence

<220>
<223> Synthesized Hammerhead Ribozyme

<220>
<223> "n" represents stem II region of a HH ribozyme.

<400> 657

uuauacuacu gaugagncga aauaaggug                                    29

<210> 658
<211> 29
<212> RNA
<213> Artificial Sequence

<220>
<223> Synthesized Hammerhead Ribozyme

<220>
<223> "n" represents stem II region of a HH ribozyme.

<400> 658

uauuauaccu gaugagncga aauauaagg                                    29

<210> 659
<211> 29

<212> RNA
<213> Artificial Sequence

<220>
<223> Synthesized Hammerhead Ribozyme

<220>
<223> "n" represents stem II region of a HH ribozyme.

<400> 659

auauauuacu gaugagncga aacuauaua 29

<210> 660
<211> 29
<212> RNA
<213> Artificial Sequence

<220>
<223> Synthesized Hammerhead Ribozyme

<220>
<223> "n" represents stem II region of a HH ribozyme.

<400> 660

auauauaucu gaugagncga aauacuaua 29

<210> 661
<211> 29
<212> RNA
<213> Artificial Sequence

<220>
<223> Synthesized Hammerhead Ribozyme

<220>
<223> "n" represents stem II region of a HH ribozyme.

<400> 661

aaaauauacu gaugagncga aauuauacu 29

<210> 662
<211> 29
<212> RNA
<213> Artificial Sequence

<220>
<223> Synthesized Hammerhead Ribozyme

<220>
<223> "n" represents stem II region of a HH ribozyme.

<400> 662

aaaaaauacu gaugagncga aauauuaua 29

<210> 663
<211> 29
<212> RNA
<213> Artificial Sequence

<220>
<223> Synthesized Hammerhead Ribozyme

<220>
<223> "n" represents stem II region of a HH ribozyme.

<400> 663

caaaaaaacu gaugagncga aauauauua 29

<210> 664
<211> 29
<212> RNA
<213> Artificial Sequence

<220>
<223> Synthesized Hammerhead Ribozyme

<220>
<223> "n" represents stem II region of a HH ribozyme.

<400> 664

uucaaaaacu gaugagncga aauauauau 29

<210> 665
<211> 29
<212> RNA
<213> Artificial Sequence

<220>
<223> Synthesized Hammerhead Ribozyme

<220>
<223> "n" represents stem II region of a HH ribozyme.

<400> 665

uuucaaaacu gaugagncga aaauauaua 29

<210> 666
<211> 29
<212> RNA
<213> Artificial Sequence

<220>
<223> Synthesized Hammerhead Ribozyme

<220>
<223> "n" represents stem II region of a HH ribozyme.

<400> 666

auuucaaacu gaugagncga aaaauauau 29

<210> 667
<211> 29
<212> RNA
<213> Artificial Sequence

<220>
<223> Synthesized Hammerhead Ribozyme

<220>
<223> "n" represents stem II region of a HH ribozyme.

<400> 667

aauuucaacu gaugagncga aaaaauaua 29

<210> 668
<211> 29
<212> RNA
<213> Artificial Sequence

<220>
<223> Synthesized Hammerhead Ribozyme

<220>
<223> "n" represents stem II region of a HH ribozyme.

<400> 668

uaauuucacu gaugagncga aaaaaauau 29

<210> 669
<211> 29
<212> RNA
<213> Artificial Sequence

<220>
<223> Synthesized Hammerhead Ribozyme

<220>
<223> "n" represents stem II region of a HH ribozyme.

<400> 669

guaauuuccu gaugagncga aaaaaaaua 29

<210> 670
<211> 29
<212> RNA
<213> Artificial Sequence

<220>
<223> Synthesized Hammerhead Ribozyme

<220>
<223> "n" represents stem II region of a HH ribozyme.

<400> 670

agcaaugucu gaugagncga aauuucaaa                                29

<210> 671
<211> 29
<212> RNA
<213> Artificial Sequence

<220>
<223> Synthesized Hammerhead Ribozyme

<220>
<223> "n" represents stem II region of a HH ribozyme.

<400> 671

aagcaaugcu gaugagncga aaauuucaa                                29

<210> 672
<211> 29
<212> RNA
<213> Artificial Sequence

<220>
<223> Synthesized Hammerhead Ribozyme

<220>
<223> "n" represents stem II region of a HH ribozyme.

<400> 672

aaacaagccu gaugagncga aauguaauu                                29

<210> 673
<211> 29
<212> RNA
<213> Artificial Sequence

<220>
<223> Synthesized Hammerhead Ribozyme

<220>
<223> "n" represents stem II region of a HH ribozyme.

<400> 673

ugauaaaccu gaugagncga aagcaaugu                                    29

<210> 674
<211> 29
<212> RNA
<213> Artificial Sequence

<220>
<223> Synthesized Hammerhead Ribozyme

<220>
<223> "n" represents stem II region of a HH ribozyme.

<400> 674

gucugauacu gaugagncga aacaagcaa                                    29

<210> 675
<211> 29
<212> RNA
<213> Artificial Sequence

<220>
<223> Synthesized Hammerhead Ribozyme

<220>
<223> "n" represents stem II region of a HH ribozyme.

<400> 675

ugucugaucu gaugagncga aaacaagca                                    29

<210> 676
<211> 29
<212> RNA
<213> Artificial Sequence

<220>
<223> Synthesized Hammerhead Ribozyme

<220>
<223> "n" represents stem II region of a HH ribozyme.

<400> 676

uugucugacu gaugagncga aaaacaagc                                    29

<210> 677

<211> 29
<212> RNA
<213> Artificial Sequence

<220>
<223> Synthesized Hammerhead Ribozyme

<220>
<223> "n" represents stem II region of a HH ribozyme.

<400> 677

aauugucucu gaugagncga aauaaacaa                                          29



<210> 678
<211> 29
<212> RNA
<213> Artificial Sequence

<220>
<223> Synthesized Hammerhead Ribozyme

<220>
<223> "n" represents stem II region of a HH ribozyme.

<400> 678

cuacauuccu gaugagncga aauugucug                                          29



<210> 679
<211> 29
<212> RNA
<213> Artificial Sequence

<220>
<223> Synthesized Hammerhead Ribozyme

<220>
<223> "n" represents stem II region of a HH ribozyme.

<400> 679

agaauuaccu gaugagncga aacauucaa                                          29



<210> 680
<211> 29
<212> RNA
<213> Artificial Sequence

<220>
<223> Synthesized Hammerhead Ribozyme

<220>
<223> "n" represents stem II region of a HH ribozyme.

<400> 680

aacagaaucu gaugagncga aacuacauu                                        29

<210> 681
<211> 29
<212> RNA
<213> Artificial Sequence

<220>
<223> Synthesized Hammerhead Ribozyme

<220>
<223> "n" represents stem II region of a HH ribozyme.

<400> 681

cagaacagcu gaugagncga aauuacuac                                        29

<210> 682
<211> 29
<212> RNA
<213> Artificial Sequence

<220>
<223> Synthesized Hammerhead Ribozyme

<220>
<223> "n" represents stem II region of a HH ribozyme.

<400> 682

ccagaacacu gaugagncga aaauuacua                                        29

<210> 683
<211> 29
<212> RNA
<213> Artificial Sequence

<220>
<223> Synthesized Hammerhead Ribozyme

<220>
<223> "n" represents stem II region of a HH ribozyme.

<400> 683

aaauccagcu gaugagncga aacagaauu                                        29

<210> 684
<211> 29
<212> RNA
<213> Artificial Sequence

<220>
<223> Synthesized Hammerhead Ribozyme

<220>
<223> "n" represents stem II region of a HH ribozyme.

<400> 684

uaaauccacu gaugagncga aaacagaau                                29

<210> 685
<211> 29
<212> RNA
<213> Artificial Sequence

<220>
<223> Synthesized Hammerhead Ribozyme

<220>
<223> "n" represents stem II region of a HH ribozyme.

<400> 685

ucaaauuacu gaugagncga aauccagaa                                29

<210> 686
<211> 29
<212> RNA
<213> Artificial Sequence

<220>
<223> Synthesized Hammerhead Ribozyme

<220>
<223> "n" represents stem II region of a HH ribozyme.

<400> 686

gucaaauucu gaugagncga aaauccaga                                29

<210> 687
<211> 29
<212> RNA
<213> Artificial Sequence

<220>
<223> Synthesized Hammerhead Ribozyme

<220>
<223> "n" represents stem II region of a HH ribozyme.

<400> 687

agucaaaucu gaugagncga aaaauccag                                29

<210> 688
<211> 29
<212> RNA
<213> Artificial Sequence

<220>
<223> Synthesized Hammerhead Ribozyme

<220>
<223> "n" represents stem II region of a HH ribozyme.

<400> 688

cccagucacu gaugagncga aauuaaauc                                        29

<210> 689
<211> 29
<212> RNA
<213> Artificial Sequence

<220>
<223> Synthesized Hammerhead Ribozyme

<220>
<223> "n" represents stem II region of a HH ribozyme.

<400> 689

acccaguccu gaugagncga aaauuaaau                                        29

<210> 690
<211> 29
<212> RNA
<213> Artificial Sequence

<220>
<223> Synthesized Hammerhead Ribozyme

<220>
<223> "n" represents stem II region of a HH ribozyme.

<400> 690

ugcauguucu gaugagncga aacccaguc                                        29

<210> 691
<211> 29
<212> RNA
<213> Artificial Sequence

<220>
<223> Synthesized Hammerhead Ribozyme

<220>
<223> "n" represents stem II region of a HH ribozyme.

<400> 691

uugcaugucu gaugagncga aaacccagu                                    29

<210> 692
<211> 29
<212> RNA
<213> Artificial Sequence

<220>
<223> Synthesized Hammerhead Ribozyme

<220>
<223> "n" represents stem II region of a HH ribozyme.

<400> 692

aaacuaaacu gaugagncga aauuuuucc                                    29

<210> 693
<211> 29
<212> RNA
<213> Artificial Sequence

<220>
<223> Synthesized Hammerhead Ribozyme

<220>
<223> "n" represents stem II region of a HH ribozyme.

<400> 693

aaaaacuacu gaugagncga aauauuuuu                                    29

<210> 694
<211> 29
<212> RNA
<213> Artificial Sequence

<220>
<223> Synthesized Hammerhead Ribozyme

<220>
<223> "n" represents stem II region of a HH ribozyme.

<400> 694

aaaaaacucu gaugagncga aaauauuuu                                    29

<210> 695
<211> 29
<212> RNA
<213> Artificial Sequence

<220>
<223> Synthesized Hammerhead Ribozyme

<220>
<223> "n" represents stem II region of a HH ribozyme.

<400> 695

aaaaaaaccu gaugagncga aaaauauuu                                          29

<210> 696
<211> 29
<212> RNA
<213> Artificial Sequence

<220>
<223> Synthesized Hammerhead Ribozyme

<220>
<223> "n" represents stem II region of a HH ribozyme.

<400> 696

aaaaaaaacu gaugagncga aacuaaaua                                          29

<210> 697
<211> 29
<212> RNA
<213> Artificial Sequence

<220>
<223> Synthesized Hammerhead Ribozyme

<220>
<223> "n" represents stem II region of a HH ribozyme.

<400> 697

aaaaaaaacu gaugagncga aaacuaaau                                          29

<210> 698
<211> 29
<212> RNA
<213> Artificial Sequence

<220>
<223> Synthesized Hammerhead Ribozyme

<220>
<223> "n" represents stem II region of a HH ribozyme.

<400> 698

aaaaaaaacu gaugagncga aaaacuaaa                                29

<210> 699
<211> 29
<212> RNA
<213> Artificial Sequence

<220>
<223> Synthesized Hammerhead Ribozyme

<220>
<223> "n" represents stem II region of a HH ribozyme.

<400> 699

aaaaaaaacu gaugagncga aaaaacuaa                                29

<210> 700
<211> 29
<212> RNA
<213> Artificial Sequence

<220>
<223> Synthesized Hammerhead Ribozyme

<220>
<223> "n" represents stem II region of a HH ribozyme.

<400> 700

aaaaaaaacu gaugagncga aaaaaacua                                29

<210> 701
<211> 29
<212> RNA
<213> Artificial Sequence

<220>
<223> Synthesized Hammerhead Ribozyme

<220>
<223> "n" represents stem II region of a HH ribozyme.

<400> 701

aaaaaaaacu gaugagncga aaaaaaacu                                29

<210> 702
<211> 29
<212> RNA

<213> Artificial Sequence

<220>
<223> Synthesized Hammerhead Ribozyme

<220>
<223> "n" represents stem II region of a HH ribozyme.

<400> 702

aaaaaaaacu gaugagncga aaaaaaaac                                          29



<210> 703
<211> 29
<212> RNA
<213> Artificial Sequence

<220>
<223> Synthesized Hammerhead Ribozyme

<220>
<223> "n" represents stem II region of a HH ribozyme.

<400> 703

aaaaaaaacu gaugagncga aaaaaaaaa                                          29



<210> 704
<211> 29
<212> RNA
<213> Artificial Sequence

<220>
<223> Synthesized Hammerhead Ribozyme

<220>
<223> "n" represents stem II region of a HH ribozyme.

<400> 704

caaaaaaacu gaugagncga aaaaaaaaa                                          29



<210> 705
<211> 29
<212> RNA
<213> Artificial Sequence

<220>
<223> Synthesized Hammerhead Ribozyme

<220>
<223> "n" represents stem II region of a HH ribozyme.

<400> 705

acaaaaaacu gaugagncga aaaaaaaaa                                    29

<210> 706
<211> 29
<212> RNA
<213> Artificial Sequence

<220>
<223> Synthesized Hammerhead Ribozyme

<220>
<223> "n" represents stem II region of a HH ribozyme.

<400> 706

uacaaaaacu gaugagncga aaaaaaaaa                                    29

<210> 707
<211> 29
<212> RNA
<213> Artificial Sequence

<220>
<223> Synthesized Hammerhead Ribozyme

<220>
<223> "n" represents stem II region of a HH ribozyme.

<400> 707

auacaaaacu gaugagncga aaaaaaaaa                                    29

<210> 708
<211> 29
<212> RNA
<213> Artificial Sequence

<220>
<223> Synthesized Hammerhead Ribozyme

<220>
<223> "n" represents stem II region of a HH ribozyme.

<400> 708

uauacaaacu gaugagncga aaaaaaaaa                                    29

<210> 709
<211> 29
<212> RNA
<213> Artificial Sequence

<220>

<223> Synthesized Hammerhead Ribozyme

<220>
<223> "n" represents stem II region of a HH ribozyme.

<400> 709

guauacaacu gaugagncga aaaaaaaa                                          29



<210> 710
<211> 29
<212> RNA
<213> Artificial Sequence

<220>
<223> Synthesized Hammerhead Ribozyme

<220>
<223> "n" represents stem II region of a HH ribozyme.

<400> 710

aguauacacu gaugagncga aaaaaaaa                                          29



<210> 711
<211> 29
<212> RNA
<213> Artificial Sequence

<220>
<223> Synthesized Hammerhead Ribozyme

<220>
<223> "n" represents stem II region of a HH ribozyme.

<400> 711

aaguauaccu gaugagncga aaaaaaaa                                          29



<210> 712
<211> 29
<212> RNA
<213> Artificial Sequence

<220>
<223> Synthesized Hammerhead Ribozyme

<220>
<223> "n" represents stem II region of a HH ribozyme.

<400> 712

gaaaaguacu gaugagncga aacaaaaa                                          29

<210> 713
<211> 29
<212> RNA
<213> Artificial Sequence

<220>
<223> Synthesized Hammerhead Ribozyme

<220>
<223> "n" represents stem II region of a HH ribozyme.

<400> 713

uugaaaagcu gaugagncga aauacaaaa                                    29



<210> 714
<211> 29
<212> RNA
<213> Artificial Sequence

<220>
<223> Synthesized Hammerhead Ribozyme

<220>
<223> "n" represents stem II region of a HH ribozyme.

<400> 714

agcuugaacu gaugagncga aaguauaca                                    29



<210> 715
<211> 29
<212> RNA
<213> Artificial Sequence

<220>
<223> Synthesized Hammerhead Ribozyme

<220>
<223> "n" represents stem II region of a HH ribozyme.

<400> 715

uagcuugacu gaugagncga aaaguauac                                    29



<210> 716
<211> 29
<212> RNA
<213> Artificial Sequence

<220>
<223> Synthesized Hammerhead Ribozyme

<220>

<223> "n" represents stem II region of a HH ribozyme.

<400> 716

guagcuugcu gaugagncga aaaaguaua                                    29

<210> 717
<211> 29
<212> RNA
<213> Artificial Sequence

<220>
<223> Synthesized Hammerhead Ribozyme

<220>
<223> "n" represents stem II region of a HH ribozyme.

<400> 717

gguagcuucu gaugagncga aaaaaguau                                    29

<210> 718
<211> 29
<212> RNA
<213> Artificial Sequence

<220>
<223> Synthesized Hammerhead Ribozyme

<220>
<223> "n" represents stem II region of a HH ribozyme.

<400> 718

ugacaaggcu gaugagncga aagcuugaa                                    29

<210> 719
<211> 29
<212> RNA
<213> Artificial Sequence

<220>
<223> Synthesized Hammerhead Ribozyme

<220>
<223> "n" represents stem II region of a HH ribozyme.

<400> 719

uacaugaccu gaugagncga aagguagcu                                    29

<210> 720
<211> 29

<212> RNA
<213> Artificial Sequence

<220>
<223> Synthesized Hammerhead Ribozyme

<220>
<223> "n" represents stem II region of a HH ribozyme.

<400> 720

guauacaucu gaugagncga aacaaggua                                          29

<210> 721
<211> 29
<212> RNA
<213> Artificial Sequence

<220>
<223> Synthesized Hammerhead Ribozyme

<220>
<223> "n" represents stem II region of a HH ribozyme.

<400> 721

ugacuguacu gaugagncga aacaugaca                                          29

<210> 722
<211> 29
<212> RNA
<213> Artificial Sequence

<220>
<223> Synthesized Hammerhead Ribozyme

<220>
<223> "n" represents stem II region of a HH ribozyme.

<400> 722

aaugacugcu gaugagncga aauacauga                                          29

<210> 723
<211> 29
<212> RNA
<213> Artificial Sequence

<220>
<223> Synthesized Hammerhead Ribozyme

<220>
<223> "n" represents stem II region of a HH ribozyme.

<400> 723

gcauaaaucu gaugagncga aacuguaua                                    29

<210> 724
<211> 29
<212> RNA
<213> Artificial Sequence

<220>
<223> Synthesized Hammerhead Ribozyme

<220>
<223> "n" represents stem II region of a HH ribozyme.

<400> 724

uaggcauacu gaugagncga aaugacugu                                    29

<210> 725
<211> 29
<212> RNA
<213> Artificial Sequence

<220>
<223> Synthesized Hammerhead Ribozyme

<220>
<223> "n" represents stem II region of a HH ribozyme.

<400> 725

uuaggcaucu gaugagncga aaaugacug                                    29

<210> 726
<211> 29
<212> RNA
<213> Artificial Sequence

<220>
<223> Synthesized Hammerhead Ribozyme

<220>
<223> "n" represents stem II region of a HH ribozyme.

<400> 726

uuuaggcacu gaugagncga aaaaugacu                                    29

<210> 727
<211> 29
<212> RNA
<213> Artificial Sequence

<220>
<223> Synthesized Hammerhead Ribozyme

<220>
<223> "n" represents stem II region of a HH ribozyme.

<400> 727

ccaggcuucu gaugagncga aaggcauaa 29

<210> 728
<211> 29
<212> RNA
<213> Artificial Sequence

<220>
<223> Synthesized Hammerhead Ribozyme

<220>
<223> "n" represents stem II region of a HH ribozyme.

<400> 728

aaaugaaucu gaugagncga aaucaccag 29

<210> 729
<211> 29
<212> RNA
<213> Artificial Sequence

<220>
<223> Synthesized Hammerhead Ribozyme

<220>
<223> "n" represents stem II region of a HH ribozyme.

<400> 729

uaaaugaacu gaugagncga aaaucacca 29

<210> 730
<211> 29
<212> RNA
<213> Artificial Sequence

<220>
<223> Synthesized Hammerhead Ribozyme

<220>
<223> "n" represents stem II region of a HH ribozyme.

<400> 730

uuuaaaugcu gaugagncga aauaaucac 29

<210> 731
<211> 29
<212> RNA
<213> Artificial Sequence

<220>
<223> Synthesized Hammerhead Ribozyme

<220>
<223> "n" represents stem II region of a HH ribozyme.

<400> 731

auuuaaaucu gaugagncga aaauaauca 29

<210> 732
<211> 29
<212> RNA
<213> Artificial Sequence

<220>
<223> Synthesized Hammerhead Ribozyme

<220>
<223> "n" represents stem II region of a HH ribozyme.

<400> 732

uucauuuacu gaugagncga aaugaauaa 29

<210> 733
<211> 29
<212> RNA
<213> Artificial Sequence

<220>
<223> Synthesized Hammerhead Ribozyme

<220>
<223> "n" represents stem II region of a HH ribozyme.

<400> 733

cuucauuucu gaugagncga aaaugaaua 29

<210> 734
<211> 29
<212> RNA
<213> Artificial Sequence

<220>
<223> Synthesized Hammerhead Ribozyme

<220>
<223> "n" represents stem II region of a HH ribozyme.

<400> 734

ucuucauucu gaugagncga aaaaugaau                                    29

<210> 735
<211> 29
<212> RNA
<213> Artificial Sequence

<220>
<223> Synthesized Hammerhead Ribozyme

<220>
<223> "n" represents stem II region of a HH ribozyme.

<400> 735

ugaaaugucu gaugagncga aaucuucau                                    29

<210> 736
<211> 29
<212> RNA
<213> Artificial Sequence

<220>
<223> Synthesized Hammerhead Ribozyme

<220>
<223> "n" represents stem II region of a HH ribozyme.

<400> 736

ugauaugacu gaugagncga aaugugauc                                    29

<210> 737
<211> 29
<212> RNA
<213> Artificial Sequence

<220>
<223> Synthesized Hammerhead Ribozyme

<220>
<223> "n" represents stem II region of a HH ribozyme.

<400> 737

uugauaugcu gaugagncga aaaugugau                                    29

<210> 738

<211> 29
<212> RNA
<213> Artificial Sequence

<220>
<223> Synthesized Hammerhead Ribozyme

<220>
<223> "n" represents stem II region of a HH ribozyme.

<400> 738

guugauaucu gaugagncga aaaauguga                                          29



<210> 739
<211> 29
<212> RNA
<213> Artificial Sequence

<220>
<223> Synthesized Hammerhead Ribozyme

<220>
<223> "n" represents stem II region of a HH ribozyme.

<400> 739

aaaguugacu gaugagncga aaugaaaug                                          29



<210> 740
<211> 29
<212> RNA
<213> Artificial Sequence

<220>
<223> Synthesized Hammerhead Ribozyme

<220>
<223> "n" represents stem II region of a HH ribozyme.

<400> 740

caaaaguucu gaugagncga aauaugaaa                                          29



<210> 741
<211> 29
<212> RNA
<213> Artificial Sequence

<220>
<223> Synthesized Hammerhead Ribozyme

<220>
<223> "n" represents stem II region of a HH ribozyme.

<400> 741

ggauacaacu gaugagncga aaguugaua                                29

<210> 742
<211> 29
<212> RNA
<213> Artificial Sequence

<220>
<223> Synthesized Hammerhead Ribozyme

<220>
<223> "n" represents stem II region of a HH ribozyme.

<400> 742

uggauacacu gaugagncga aaaguugau                                29

<210> 743
<211> 29
<212> RNA
<213> Artificial Sequence

<220>
<223> Synthesized Hammerhead Ribozyme

<220>
<223> "n" represents stem II region of a HH ribozyme.

<400> 743

guggauaccu gaugagncga aaaaguuga                                29

<210> 744
<211> 29
<212> RNA
<213> Artificial Sequence

<220>
<223> Synthesized Hammerhead Ribozyme

<220>
<223> "n" represents stem II region of a HH ribozyme.

<400> 744

acuguggacu gaugagncga aacaaaagu                                29

<210> 745
<211> 29
<212> RNA
<213> Artificial Sequence

<220>
<223> Synthesized Hammerhead Ribozyme

<220>
<223> "n" represents stem II region of a HH ribozyme.

<400> 745

cuacugugcu gaugagncga aauacaaaa                                    29

<210> 746
<211> 29
<212> RNA
<213> Artificial Sequence

<220>
<223> Synthesized Hammerhead Ribozyme

<220>
<223> "n" represents stem II region of a HH ribozyme.

<400> 746

auuuuguccu gaugagncga aacugugga                                    29

<210> 747
<211> 29
<212> RNA
<213> Artificial Sequence

<220>
<223> Synthesized Hammerhead Ribozyme

<220>
<223> "n" represents stem II region of a HH ribozyme.

<400> 747

auuagugccu gaugagncga aauuuuguc                                    29

<210> 748
<211> 29
<212> RNA
<213> Artificial Sequence

<220>
<223> Synthesized Hammerhead Ribozyme

<220>
<223> "n" represents stem II region of a HH ribozyme.

<400> 748

aucuggaucu gaugagncga aagugcuau                                    29

<210> 749
<211> 29
<212> RNA
<213> Artificial Sequence

<220>
<223> Synthesized Hammerhead Ribozyme

<220>
<223> "n" represents stem II region of a HH ribozyme.

<400> 749

ggcaucugcu gaugagncga aauuagugc 29

<210> 750
<211> 29
<212> RNA
<213> Artificial Sequence

<220>
<223> Synthesized Hammerhead Ribozyme

<220>
<223> "n" represents stem II region of a HH ribozyme.

<400> 750

uccaacaacu gaugagncga aaggcaucu 29

<210> 751
<211> 29
<212> RNA
<213> Artificial Sequence

<220>
<223> Synthesized Hammerhead Ribozyme

<220>
<223> "n" represents stem II region of a HH ribozyme.

<400> 751

uauccaaccu gaugagncga aauaggcau 29

<210> 752
<211> 29
<212> RNA
<213> Artificial Sequence

<220>
<223> Synthesized Hammerhead Ribozyme

<220>
<223> "n" represents stem II region of a HH ribozyme.

<400> 752

caauaucccu gaugagncga aacaauagg                                    29



<210> 753
<211> 29
<212> RNA
<213> Artificial Sequence

<220>
<223> Synthesized Hammerhead Ribozyme

<220>
<223> "n" represents stem II region of a HH ribozyme.

<400> 753

ucauucaacu gaugagncga aauccaaca                                    29



<210> 754
<211> 29
<212> RNA
<213> Artificial Sequence

<220>
<223> Synthesized Hammerhead Ribozyme

<220>
<223> "n" represents stem II region of a HH ribozyme.

<400> 754

ugucauuccu gaugagncga aauauccaa                                    29



<210> 755
<211> 29
<212> RNA
<213> Artificial Sequence

<220>
<223> Synthesized Hammerhead Ribozyme

<220>
<223> "n" represents stem II region of a HH ribozyme.

<400> 755

cuacauaacu gaugagncga aauugucug                                    29

<210> 756
<211> 29
<212> RNA
<213> Artificial Sequence

<220>
<223> Synthesized Hammerhead Ribozyme

<220>
<223> "n" represents stem II region of a HH ribozyme.

<400> 756

ugcuacaucu gaugagncga aagauuguc 29

<210> 757
<211> 29
<212> RNA
<213> Artificial Sequence

<220>
<223> Synthesized Hammerhead Ribozyme

<220>
<223> "n" represents stem II region of a HH ribozyme.

<400> 757

uugcuacacu gaugagncga aaagauugu 29

<210> 758
<211> 29
<212> RNA
<213> Artificial Sequence

<220>
<223> Synthesized Hammerhead Ribozyme

<220>
<223> "n" represents stem II region of a HH ribozyme.

<400> 758

aucuuugccu gaugagncga aacauaaga 29

<210> 759
<211> 29
<212> RNA
<213> Artificial Sequence

<220>
<223> Synthesized Hammerhead Ribozyme

<220>
<223> "n" represents stem II region of a HH ribozyme.

<400> 759

ucaggcaucu gaugagncga aaucuuugc                                    29

<210> 760
<211> 29
<212> RNA
<213> Artificial Sequence

<220>
<223> Synthesized Hammerhead Ribozyme

<220>
<223> "n" represents stem II region of a HH ribozyme.

<400> 760

uucaggcacu gaugagncga aaaucuuug                                    29

<210> 761
<211> 29
<212> RNA
<213> Artificial Sequence

<220>
<223> Synthesized Hammerhead Ribozyme

<220>
<223> "n" represents stem II region of a HH ribozyme.

<400> 761

cccugaaucu gaugagncga aauuuuccu                                    29

<210> 762
<211> 29
<212> RNA
<213> Artificial Sequence

<220>
<223> Synthesized Hammerhead Ribozyme

<220>
<223> "n" represents stem II region of a HH ribozyme.

<400> 762

gcccugaacu gaugagncga aaauuuucc                                    29

<210> 763
<211> 29
<212> RNA

<213> Artificial Sequence

<220>
<223> Synthesized Hammerhead Ribozyme

<220>
<223> "n" represents stem II region of a HH ribozyme.

<400> 763

cugcccugcu gaugagncga aauaauuuu                                          29



<210> 764
<211> 29
<212> RNA
<213> Artificial Sequence

<220>
<223> Synthesized Hammerhead Ribozyme

<220>
<223> "n" represents stem II region of a HH ribozyme.

<400> 764

gcugcccucu gaugagncga aaauaauuu                                          29



<210> 765
<211> 29
<212> RNA
<213> Artificial Sequence

<220>
<223> Synthesized Hammerhead Ribozyme

<220>
<223> "n" represents stem II region of a HH ribozyme.

<400> 765

agcaaaaucu gaugagncga aagcugccc                                          29



<210> 766
<211> 29
<212> RNA
<213> Artificial Sequence

<220>
<223> Synthesized Hammerhead Ribozyme

<220>
<223> "n" represents stem II region of a HH ribozyme.

<400> 766

aaaagcaacu gaugagncga aauuagcug 29

<210> 767
<211> 29
<212> RNA
<213> Artificial Sequence

<220>
<223> Synthesized Hammerhead Ribozyme

<220>
<223> "n" represents stem II region of a HH ribozyme.

<400> 767

uaaaagcacu gaugagncga aaauuagcu 29

<210> 768
<211> 29
<212> RNA
<213> Artificial Sequence

<220>
<223> Synthesized Hammerhead Ribozyme

<220>
<223> "n" represents stem II region of a HH ribozyme.

<400> 768

guaaaagccu gaugagncga aaaauuagc 29

<210> 769
<211> 29
<212> RNA
<213> Artificial Sequence

<220>
<223> Synthesized Hammerhead Ribozyme

<220>
<223> "n" represents stem II region of a HH ribozyme.

<400> 769

uuugguaacu gaugagncga aagcaaaau 29

<210> 770
<211> 29
<212> RNA
<213> Artificial Sequence

<220>

<223> Synthesized Hammerhead Ribozyme

<220>
<223> "n" represents stem II region of a HH ribozyme.

<400> 770

uuuugguacu gaugagncga aaagcaaaa                                          29

<210> 771
<211> 29
<212> RNA
<213> Artificial Sequence

<220>
<223> Synthesized Hammerhead Ribozyme

<220>
<223> "n" represents stem II region of a HH ribozyme.

<400> 771

auuuuggucu gaugagncga aaaagcaaa                                          29

<210> 772
<211> 29
<212> RNA
<213> Artificial Sequence

<220>
<223> Synthesized Hammerhead Ribozyme

<220>
<223> "n" represents stem II region of a HH ribozyme.

<400> 772

uauuuuggcu gaugagncga aaaaagcaa                                          29

<210> 773
<211> 29
<212> RNA
<213> Artificial Sequence

<220>
<223> Synthesized Hammerhead Ribozyme

<220>
<223> "n" represents stem II region of a HH ribozyme.

<400> 773

acuacugacu gaugagncga aauuuuggu                                          29

<210> 774
<211> 29
<212> RNA
<213> Artificial Sequence

<220>
<223> Synthesized Hammerhead Ribozyme

<220>
<223> "n" represents stem II region of a HH ribozyme.

<400> 774

uuacuacucu gaugagncga aauauuuug                                          29



<210> 775
<211> 29
<212> RNA
<213> Artificial Sequence

<220>
<223> Synthesized Hammerhead Ribozyme

<220>
<223> "n" represents stem II region of a HH ribozyme.

<400> 775

aauauuaccu gaugagncga aacugauau                                          29



<210> 776
<211> 29
<212> RNA
<213> Artificial Sequence

<220>
<223> Synthesized Hammerhead Ribozyme

<220>
<223> "n" represents stem II region of a HH ribozyme.

<400> 776

aaaaauaucu gaugagncga aacuacuga                                          29



<210> 777
<211> 29
<212> RNA
<213> Artificial Sequence

<220>
<223> Synthesized Hammerhead Ribozyme

<220>

<223> "n" represents stem II region of a HH ribozyme.

<400> 777

uccaaaaacu gaugagncga aauuacuac                                    29

<210> 778
<211> 29
<212> RNA
<213> Artificial Sequence

<220>
<223> Synthesized Hammerhead Ribozyme

<220>
<223> "n" represents stem II region of a HH ribozyme.

<400> 778

uguccaaacu gaugagncga aauauuacu                                    29

<210> 779
<211> 29
<212> RNA
<213> Artificial Sequence

<220>
<223> Synthesized Hammerhead Ribozyme

<220>
<223> "n" represents stem II region of a HH ribozyme.

<400> 779

cuguccaacu gaugagncga aaauauuac                                    29

<210> 780
<211> 29
<212> RNA
<213> Artificial Sequence

<220>
<223> Synthesized Hammerhead Ribozyme

<220>
<223> "n" represents stem II region of a HH ribozyme.

<400> 780

acuguccacu gaugagncga aaaauauua                                    29

<210> 781
<211> 29

<212> RNA
<213> Artificial Sequence

<220>
<223> Synthesized Hammerhead Ribozyme

<220>
<223> "n" represents stem II region of a HH ribozyme.

<400> 781

uacugucccu gaugagncga aaaaauauu                                          29

<210> 782
<211> 29
<212> RNA
<213> Artificial Sequence

<220>
<223> Synthesized Hammerhead Ribozyme

<220>
<223> "n" represents stem II region of a HH ribozyme.

<400> 782

ccauuagccu gaugagncga aacugucca                                          29

<210> 783
<211> 29
<212> RNA
<213> Artificial Sequence

<220>
<223> Synthesized Hammerhead Ribozyme

<220>
<223> "n" represents stem II region of a HH ribozyme.

<400> 783

ugacccaucu gaugagncga aagcuacug                                          29

<210> 784
<211> 29
<212> RNA
<213> Artificial Sequence

<220>
<223> Synthesized Hammerhead Ribozyme

<220>
<223> "n" represents stem II region of a HH ribozyme.

<400> 784

aacccacucu gaugagncga aacccauua 29

<210> 785
<211> 29
<212> RNA
<213> Artificial Sequence

<220>
<223> Synthesized Hammerhead Ribozyme

<220>
<223> "n" represents stem II region of a HH ribozyme.

<400> 785

uuaaaaagcu gaugagncga aacccacug 29

<210> 786
<211> 29
<212> RNA
<213> Artificial Sequence

<220>
<223> Synthesized Hammerhead Ribozyme

<220>
<223> "n" represents stem II region of a HH ribozyme.

<400> 786

auuaaaaacu gaugagncga aaacccacu 29

<210> 787
<211> 29
<212> RNA
<213> Artificial Sequence

<220>
<223> Synthesized Hammerhead Ribozyme

<220>
<223> "n" represents stem II region of a HH ribozyme.

<400> 787

acauuaaacu gaugagncga aagaaccca 29

<210> 788
<211> 29
<212> RNA
<213> Artificial Sequence

<220>
<223> Synthesized Hammerhead Ribozyme

<220>
<223> "n" represents stem II region of a HH ribozyme.

<400> 788

aacauuaacu gaugagncga aaagaaccc                                29

<210> 789
<211> 29
<212> RNA
<213> Artificial Sequence

<220>
<223> Synthesized Hammerhead Ribozyme

<220>
<223> "n" represents stem II region of a HH ribozyme.

<400> 789

aaacauuacu gaugagncga aaaagaacc                                29

<210> 790
<211> 29
<212> RNA
<213> Artificial Sequence

<220>
<223> Synthesized Hammerhead Ribozyme

<220>
<223> "n" represents stem II region of a HH ribozyme.

<400> 790

uaaacauucu gaugagncga aaaaagaac                                29

<210> 791
<211> 29
<212> RNA
<213> Artificial Sequence

<220>
<223> Synthesized Hammerhead Ribozyme

<220>
<223> "n" represents stem II region of a HH ribozyme.

<400> 791

auaaacaucu gaugagncga aaaaaagaa                                29

<210> 792
<211> 29
<212> RNA
<213> Artificial Sequence

<220>
<223> Synthesized Hammerhead Ribozyme

<220>
<223> "n" represents stem II region of a HH ribozyme.

<400> 792

uaaguauacu gaugagncga aacauuaaa                                    29

<210> 793
<211> 29
<212> RNA
<213> Artificial Sequence

<220>
<223> Synthesized Hammerhead Ribozyme

<220>
<223> "n" represents stem II region of a HH ribozyme.

<400> 793

cuaaguaucu gaugagncga aaacauuaa                                    29

<210> 794
<211> 29
<212> RNA
<213> Artificial Sequence

<220>
<223> Synthesized Hammerhead Ribozyme

<220>
<223> "n" represents stem II region of a HH ribozyme.

<400> 794

ucuaaguacu gaugagncga aaaacauua                                    29

<210> 795
<211> 29
<212> RNA
<213> Artificial Sequence

<220>
<223> Synthesized Hammerhead Ribozyme

<220>
<223> "n" represents stem II region of a HH ribozyme.

<400> 795

aaucuaagcu gaugagncga aauaaacau                                    29

<210> 796
<211> 29
<212> RNA
<213> Artificial Sequence

<220>
<223> Synthesized Hammerhead Ribozyme

<220>
<223> "n" represents stem II region of a HH ribozyme.

<400> 796

gaaaaucucu gaugagncga aaguauaaa                                    29

<210> 797
<211> 29
<212> RNA
<213> Artificial Sequence

<220>
<223> Synthesized Hammerhead Ribozyme

<220>
<223> "n" represents stem II region of a HH ribozyme.

<400> 797

agaaaauccu gaugagncga aaaguauaa                                    29

<210> 798
<211> 29
<212> RNA
<213> Artificial Sequence

<220>
<223> Synthesized Hammerhead Ribozyme

<220>
<223> "n" represents stem II region of a HH ribozyme.

<400> 798

uaaaagaacu gaugagncga aaucuaagu                                    29

<210> 799

<211> 29
<212> RNA
<213> Artificial Sequence

<220>
<223> Synthesized Hammerhead Ribozyme

<220>
<223> "n" represents stem II region of a HH ribozyme.

<400> 799

uuaaaagacu gaugagncga aaaucuaag                                          29

<210> 800
<211> 29
<212> RNA
<213> Artificial Sequence

<220>
<223> Synthesized Hammerhead Ribozyme

<220>
<223> "n" represents stem II region of a HH ribozyme.

<400> 800

uuuaaaagcu gaugagncga aaaaucuaa                                          29

<210> 801
<211> 29
<212> RNA
<213> Artificial Sequence

<220>
<223> Synthesized Hammerhead Ribozyme

<220>
<223> "n" represents stem II region of a HH ribozyme.

<400> 801

uuuuaaaacu gaugagncga aaaaaucua                                          29

<210> 802
<211> 29
<212> RNA
<213> Artificial Sequence

<220>
<223> Synthesized Hammerhead Ribozyme

<220>
<223> "n" represents stem II region of a HH ribozyme.

<400> 802

uuuuuuaacu gaugagncga aagaaaauc                                          29

<210> 803
<211> 29
<212> RNA
<213> Artificial Sequence

<220>
<223> Synthesized Hammerhead Ribozyme

<220>
<223> "n" represents stem II region of a HH ribozyme.

<400> 803

uuuuuuuacu gaugagncga aaagaaaau                                          29

<210> 804
<211> 29
<212> RNA
<213> Artificial Sequence

<220>
<223> Synthesized Hammerhead Ribozyme

<220>
<223> "n" represents stem II region of a HH ribozyme.

<400> 804

auuuuuuucu gaugagncga aaaagaaaa                                          29

<210> 805
<211> 29
<212> RNA
<213> Artificial Sequence

<220>
<223> Synthesized Hammerhead Ribozyme

<220>
<223> "n" represents stem II region of a HH ribozyme.

<400> 805

aauuuuuucu gaugagncga aaaaagaaa                                          29

<210> 806
<211> 29
<212> RNA
<213> Artificial Sequence

<220>
<223> Synthesized Hammerhead Ribozyme

<220>
<223> "n" represents stem II region of a HH ribozyme.

<400> 806
   uuuauuuucu gaugagncga aauuuuuuu                                    29

<210> 807
<211> 29
<212> RNA
<213> Artificial Sequence

<220>
<223> Synthesized Hammerhead Ribozyme

<220>
<223> "n" represents stem II region of a HH ribozyme.

<400> 807
   uuuuauuucu gaugagncga aaauuuuuu                                    29

<210> 808
<211> 29
<212> RNA
<213> Artificial Sequence

<220>
<223> Synthesized Hammerhead Ribozyme

<220>
<223> "n" represents stem II region of a HH ribozyme.

<400> 808

uuuuguuucu gaugagncga aauuuuaau                                   29

<210> 809
<211> 29
<212> RNA
<213> Artificial Sequence

<220>
<223> Synthesized Hammerhead Ribozyme

<220>
<223> "n" represents stem II region of a HH ribozyme.

<400> 809
   guccuagacu gaugagncga aauuuuuuu                                    29

<210> 810
<211> 29
<212> RNA
<213> Artificial Sequence

<220>
<223> Synthesized Hammerhead Ribozyme

<220>
<223> "n" represents stem II region of a HH ribozyme.

<400> 810

aguccuagcu gaugagncga aaauuuuuu                                    29

<210> 811
<211> 29
<212> RNA
<213> Artificial Sequence

<220>
<223> Synthesized Hammerhead Ribozyme

<220>
<223> "n" represents stem II region of a HH ribozyme.

<400> 811

uaguccuacu gaugagncga aaaauuuuu                                    29

<210> 812
<211> 29
<212> RNA
<213> Artificial Sequence

<220>
<223> Synthesized Hammerhead Ribozyme

<220>
<223> "n" represents stem II region of a HH ribozyme.

<400> 812

ucuagucccu gaugagncga aagaaauuu                                    29

<210> 813
<211> 29
<212> RNA
<213> Artificial Sequence

<220>
<223> Synthesized Hammerhead Ribozyme

<220>
<223> "n" represents stem II region of a HH ribozyme.

<400> 813

acaucguccu gaugagncga aaguccuag                                    29

<210> 814
<211> 29
<212> RNA
<213> Artificial Sequence

<220>
<223> Synthesized Hammerhead Ribozyme

<220>
<223> "n" represents stem II region of a HH ribozyme.

<400> 814

gcugguaucu gaugagncga aacaucguc                                    29

<210> 815
<211> 29
<212> RNA
<213> Artificial Sequence

<220>
<223> Synthesized Hammerhead Ribozyme

<220>
<223> "n" represents stem II region of a HH ribozyme.

<400> 815

uuagcuggcu gaugagncga aauuacauc                                    29

<210> 816
<211> 29
<212> RNA
<213> Artificial Sequence

<220>
<223> Synthesized Hammerhead Ribozyme

<220>
<223> "n" represents stem II region of a HH ribozyme.

<400> 816

uuuggcuucu gaugagncga aagcuggua                                    29

<210> 817
<211> 29
<212> RNA
<213> Artificial Sequence

<220>
<223> Synthesized Hammerhead Ribozyme

<220>
<223> "n" represents stem II region of a HH ribozyme.

<400> 817

cacuguaucu gaugagncga aauuguuug                                          29

<210> 818
<211> 29
<212> RNA
<213> Artificial Sequence

<220>
<223> Synthesized Hammerhead Ribozyme

<220>
<223> "n" represents stem II region of a HH ribozyme.

<400> 818

ccacuguacu gaugagncga aaauuguuu                                          29

<210> 819
<211> 29
<212> RNA
<213> Artificial Sequence

<220>
<223> Synthesized Hammerhead Ribozyme

<220>
<223> "n" represents stem II region of a HH ribozyme.

<400> 819

uuccacugcu gaugagncga aauaauugu                                          29

<210> 820
<211> 29
<212> RNA
<213> Artificial Sequence

<220>
<223> Synthesized Hammerhead Ribozyme

<220>
<223> "n" represents stem II region of a HH ribozyme.

<400> 820

uaauguaacu gaugagncga aaccuucca                                29

<210> 821
<211> 29
<212> RNA
<213> Artificial Sequence

<220>
<223> Synthesized Hammerhead Ribozyme

<220>
<223> "n" represents stem II region of a HH ribozyme.

<400> 821

auaauguacu gaugagncga aaaccuucc                                29

<210> 822
<211> 29
<212> RNA
<213> Artificial Sequence

<220>
<223> Synthesized Hammerhead Ribozyme

<220>
<223> "n" represents stem II region of a HH ribozyme.

<400> 822

aauaaugucu gaugagncga aaaaccuuc                                29

<210> 823
<211> 29
<212> RNA
<213> Artificial Sequence

<220>
<223> Synthesized Hammerhead Ribozyme

<220>
<223> "n" represents stem II region of a HH ribozyme.

<400> 823

gaauaaugcu gaugagncga aaaaaccuu                                29

<210> 824
<211> 29
<212> RNA

<213> Artificial Sequence

<220>
<223> Synthesized Hammerhead Ribozyme

<220>
<223> "n" represents stem II region of a HH ribozyme.

<400> 824

ggaugaaucu gaugagncga aauguaaaa                                          29



<210> 825
<211> 29
<212> RNA
<213> Artificial Sequence

<220>
<223> Synthesized Hammerhead Ribozyme

<220>
<223> "n" represents stem II region of a HH ribozyme.

<400> 825

uggaugaacu gaugagncga aaauguaaa                                          29



<210> 826
<211> 29
<212> RNA
<213> Artificial Sequence

<220>
<223> Synthesized Hammerhead Ribozyme

<220>
<223> "n" represents stem II region of a HH ribozyme.

<400> 826

auuggaugcu gaugagncga aauaaugua                                          29



<210> 827
<211> 29
<212> RNA
<213> Artificial Sequence

<220>
<223> Synthesized Hammerhead Ribozyme

<220>
<223> "n" represents stem II region of a HH ribozyme.

<400> 827

cauuggaucu gaugagncga aaauaaugu 29

<210> 828
<211> 29
<212> RNA
<213> Artificial Sequence

<220>
<223> Synthesized Hammerhead Ribozyme

<220>
<223> "n" represents stem II region of a HH ribozyme.

<400> 828

acacauugcu gaugagncga aaugaauaa 29

<210> 829
<211> 29
<212> RNA
<213> Artificial Sequence

<220>
<223> Synthesized Hammerhead Ribozyme

<220>
<223> "n" represents stem II region of a HH ribozyme.

<400> 829

ugaauagacu gaugagncga aacacauug 29

<210> 830
<211> 29
<212> RNA
<213> Artificial Sequence

<220>
<223> Synthesized Hammerhead Ribozyme

<220>
<223> "n" represents stem II region of a HH ribozyme.

<400> 830

augaauagcu gaugagncga aaacacauu 29

<210> 831
<211> 29
<212> RNA
<213> Artificial Sequence

<220>

<223> Synthesized Hammerhead Ribozyme

<220>
<223> "n" represents stem II region of a HH ribozyme.

<400> 831

caugaauacu gaugagncga aaaacacau                                    29



<210> 832
<211> 29
<212> RNA
<213> Artificial Sequence

<220>
<223> Synthesized Hammerhead Ribozyme

<220>
<223> "n" represents stem II region of a HH ribozyme.

<400> 832

aacaugaacu gaugagncga aagaaacac                                    29



<210> 833
<211> 29
<212> RNA
<213> Artificial Sequence

<220>
<223> Synthesized Hammerhead Ribozyme

<220>
<223> "n" represents stem II region of a HH ribozyme.

<400> 833

uuaacaugcu gaugagncga aauagaaac                                    29



<210> 834
<211> 29
<212> RNA
<213> Artificial Sequence

<220>
<223> Synthesized Hammerhead Ribozyme

<220>
<223> "n" represents stem II region of a HH ribozyme.

<400> 834

cuuaacaucu gaugagncga aaauagaaa                                    29

<210> 835
<211> 29
<212> RNA
<213> Artificial Sequence

<220>
<223> Synthesized Hammerhead Ribozyme

<220>
<223> "n" represents stem II region of a HH ribozyme.

<400> 835

aguaucuucu gaugagncga aacaugaau                                          29

<210> 836
<211> 29
<212> RNA
<213> Artificial Sequence

<220>
<223> Synthesized Hammerhead Ribozyme

<220>
<223> "n" represents stem II region of a HH ribozyme.

<400> 836

uaguaucucu gaugagncga aaacaugaa                                          29

<210> 837
<211> 29
<212> RNA
<213> Artificial Sequence

<220>
<223> Synthesized Hammerhead Ribozyme

<220>
<223> "n" represents stem II region of a HH ribozyme.

<400> 837

uguaguagcu gaugagncga aaucuuaac                                          29

<210> 838
<211> 29
<212> RNA
<213> Artificial Sequence

<220>
<223> Synthesized Hammerhead Ribozyme

<220>

<223> "n" represents stem II region of a HH ribozyme.

<400> 838

aaauguagcu gaugagncga aaguaucuu                                    29

<210> 839
<211> 29
<212> RNA
<213> Artificial Sequence

<220>
<223> Synthesized Hammerhead Ribozyme

<220>
<223> "n" represents stem II region of a HH ribozyme.

<400> 839

uucaaaugcu gaugagncga aaguaguau                                    29

<210> 840
<211> 29
<212> RNA
<213> Artificial Sequence

<220>
<223> Synthesized Hammerhead Ribozyme

<220>
<223> "n" represents stem II region of a HH ribozyme.

<400> 840

ccacuucacu gaugagncga aauguagua                                    29

<210> 841
<211> 29
<212> RNA
<213> Artificial Sequence

<220>
<223> Synthesized Hammerhead Ribozyme

<220>
<223> "n" represents stem II region of a HH ribozyme.

<400> 841

cccacuuccu gaugagncga aaauguagu                                    29

<210> 842
<211> 29

<212> RNA
<213> Artificial Sequence

<220>
<223> Synthesized Hammerhead Ribozyme

<220>
<223> "n" represents stem II region of a HH ribozyme.

<400> 842

aaucaucucu gaugagncga aauguucuc                                          29



<210> 843
<211> 29
<212> RNA
<213> Artificial Sequence

<220>
<223> Synthesized Hammerhead Ribozyme

<220>
<223> "n" represents stem II region of a HH ribozyme.

<400> 843

aacauuuccu gaugagncga aaucaucug                                          29



<210> 844
<211> 29
<212> RNA
<213> Artificial Sequence

<220>
<223> Synthesized Hammerhead Ribozyme

<220>
<223> "n" represents stem II region of a HH ribozyme.

<400> 844

ccugggcgcu gaugagncga aacauuuca                                          29



<210> 845
<211> 29
<212> RNA
<213> Artificial Sequence

<220>
<223> Synthesized Hammerhead Ribozyme

<220>
<223> "n" represents stem II region of a HH ribozyme.

<400> 845

cccugggccu gaugagncga aaacauuuc                                    29

<210> 846
<211> 29
<212> RNA
<213> Artificial Sequence

<220>
<223> Synthesized Hammerhead Ribozyme

<220>
<223> "n" represents stem II region of a HH ribozyme.

<400> 846

uugcuggacu gaugagncga aaccccugg                                    29

<210> 847
<211> 29
<212> RNA
<213> Artificial Sequence

<220>
<223> Synthesized Hammerhead Ribozyme

<220>
<223> "n" represents stem II region of a HH ribozyme.

<400> 847

aguugcugcu gaugagncga aagaccccu                                    29

<210> 848
<211> 29
<212> RNA
<213> Artificial Sequence

<220>
<223> Synthesized Hammerhead Ribozyme

<220>
<223> "n" represents stem II region of a HH ribozyme.

<400> 848

gauuuccacu gaugagncga aaguugcug                                    29

<210> 849
<211> 29
<212> RNA
<213> Artificial Sequence

<220>
<223> Synthesized Hammerhead Ribozyme

<220>
<223> "n" represents stem II region of a HH ribozyme.

<400> 849

agauuucccu gaugagncga aaaguugcu 29

<210> 850
<211> 29
<212> RNA
<213> Artificial Sequence

<220>
<223> Synthesized Hammerhead Ribozyme

<220>
<223> "n" represents stem II region of a HH ribozyme.

<400> 850

uacaaagacu gaugagncga aauuuccaa 29

<210> 851
<211> 29
<212> RNA
<213> Artificial Sequence

<220>
<223> Synthesized Hammerhead Ribozyme

<220>
<223> "n" represents stem II region of a HH ribozyme.

<400> 851

aauacaaacu gaugagncga aagauuucc 29

<210> 852
<211> 29
<212> RNA
<213> Artificial Sequence

<220>
<223> Synthesized Hammerhead Ribozyme

<220>
<223> "n" represents stem II region of a HH ribozyme.

<400> 852

aaaauacacu gaugagncga aagagauuu 29

<210> 853
<211> 29
<212> RNA
<213> Artificial Sequence

<220>
<223> Synthesized Hammerhead Ribozyme

<220>
<223> "n" represents stem II region of a HH ribozyme.

<400> 853

aaaaauaccu gaugagncga aaagagauu                                    29



<210> 854
<211> 29
<212> RNA
<213> Artificial Sequence

<220>
<223> Synthesized Hammerhead Ribozyme

<220>
<223> "n" represents stem II region of a HH ribozyme.

<400> 854

aguaaaaacu gaugagncga aacaaagag                                    29



<210> 855
<211> 29
<212> RNA
<213> Artificial Sequence

<220>
<223> Synthesized Hammerhead Ribozyme

<220>
<223> "n" represents stem II region of a HH ribozyme.

<400> 855

caaguaaacu gaugagncga aauacaaag                                    29



<210> 856
<211> 29
<212> RNA
<213> Artificial Sequence

<220>
<223> Synthesized Hammerhead Ribozyme

<220>
<223> "n" represents stem II region of a HH ribozyme.

<400> 856

ucaaguaacu gaugagncga aaauacaaa                                          29

<210> 857
<211> 29
<212> RNA
<213> Artificial Sequence

<220>
<223> Synthesized Hammerhead Ribozyme

<220>
<223> "n" represents stem II region of a HH ribozyme.

<400> 857

uucaaguacu gaugagncga aaaauacaa                                          29

<210> 858
<211> 29
<212> RNA
<213> Artificial Sequence

<220>
<223> Synthesized Hammerhead Ribozyme

<220>
<223> "n" represents stem II region of a HH ribozyme.

<400> 858

cuucaagucu gaugagncga aaaaauaca                                          29

<210> 859
<211> 29
<212> RNA
<213> Artificial Sequence

<220>
<223> Synthesized Hammerhead Ribozyme

<220>
<223> "n" represents stem II region of a HH ribozyme.

<400> 859

acuucaagcu gaugagncga aaaaaauac                                          29

<210> 860

<211> 29
<212> RNA
<213> Artificial Sequence

<220>
<223> Synthesized Hammerhead Ribozyme

<220>
<223> "n" represents stem II region of a HH ribozyme.

<400> 860

ggcacuuccu gaugagncga aaguaaaaa                                29

<210> 861
<211> 29
<212> RNA
<213> Artificial Sequence

<220>
<223> Synthesized Hammerhead Ribozyme

<220>
<223> "n" represents stem II region of a HH ribozyme.

<400> 861

cuguccaucu gaugagncga aaguggcac                                29

<210> 862
<211> 29
<212> RNA
<213> Artificial Sequence

<220>
<223> Synthesized Hammerhead Ribozyme

<220>
<223> "n" represents stem II region of a HH ribozyme.

<400> 862

ccagaaaacu gaugagncga aaucugcug                                29

<210> 863
<211> 29
<212> RNA
<213> Artificial Sequence

<220>
<223> Synthesized Hammerhead Ribozyme

<220>
<223> "n" represents stem II region of a HH ribozyme.

<400> 863

agccagaacu gaugagncga aauaucugc                                          29

<210> 864
<211> 29
<212> RNA
<213> Artificial Sequence

<220>
<223> Synthesized Hammerhead Ribozyme

<220>
<223> "n" represents stem II region of a HH ribozyme.

<400> 864

cagccagacu gaugagncga aaauaucug                                          29

<210> 865
<211> 29
<212> RNA
<213> Artificial Sequence

<220>
<223> Synthesized Hammerhead Ribozyme

<220>
<223> "n" represents stem II region of a HH ribozyme.

<400> 865

ucagccagcu gaugagncga aaaauaucu                                          29

<210> 866
<211> 29
<212> RNA
<213> Artificial Sequence

<220>
<223> Synthesized Hammerhead Ribozyme

<220>
<223> "n" represents stem II region of a HH ribozyme.

<400> 866

aucagccacu gaugagncga aaaaauauc                                          29

<210> 867
<211> 29
<212> RNA
<213> Artificial Sequence

<220>
<223> Synthesized Hammerhead Ribozyme

<220>
<223> "n" represents stem II region of a HH ribozyme.

<400> 867

ccaauacccu gaugagncga aacaucagc                                    29

<210> 868
<211> 29
<212> RNA
<213> Artificial Sequence

<220>
<223> Synthesized Hammerhead Ribozyme

<220>
<223> "n" represents stem II region of a HH ribozyme.

<400> 868

acacccaacu gaugagncga aaccaacau                                    29

<210> 869
<211> 29
<212> RNA
<213> Artificial Sequence

<220>
<223> Synthesized Hammerhead Ribozyme

<220>
<223> "n" represents stem II region of a HH ribozyme.

<400> 869

cuacaccccu gaugagncga aauaccaac                                    29

<210> 870
<211> 29
<212> RNA
<213> Artificial Sequence

<220>
<223> Synthesized Hammerhead Ribozyme

<220>
<223> "n" represents stem II region of a HH ribozyme.

<400> 870

cauguucccu gaugagncga aacacccaa                                    29

<210> 871
<211> 29
<212> RNA
<213> Artificial Sequence

<220>
<223> Synthesized Hammerhead Ribozyme

<220>
<223> "n" represents stem II region of a HH ribozyme.

<400> 871

uuuuuuuacu gaugagncga aaucauguu                                29

<210> 872
<211> 29
<212> RNA
<213> Artificial Sequence

<220>
<223> Synthesized Hammerhead Ribozyme

<220>
<223> "n" represents stem II region of a HH ribozyme.

<400> 872

uuuuuuuucu gaugagncga aaaucaugu                                29

<210> 873
<211> 29
<212> RNA
<213> Artificial Sequence

<220>
<223> Synthesized Hammerhead Ribozyme

<220>
<223> "n" represents stem II region of a HH ribozyme.

<400> 873

uuuuuuuucu gaugagncga aaaaucaug                                29

<210> 874
<211> 29
<212> RNA
<213> Artificial Sequence

<220>
<223> Synthesized Hammerhead Ribozyme

<220>
<223> "n" represents stem II region of a HH ribozyme.

<400> 874

agaggcaacu gaugagncga aaguuuuuu                                29

<210> 875
<211> 29
<212> RNA
<213> Artificial Sequence

<220>
<223> Synthesized Hammerhead Ribozyme

<220>
<223> "n" represents stem II region of a HH ribozyme.

<400> 875

gcagaggccu gaugagncga aagaguuuu                                29

<210> 876
<211> 29
<212> RNA
<213> Artificial Sequence

<220>
<223> Synthesized Hammerhead Ribozyme

<220>
<223> "n" represents stem II region of a HH ribozyme.

<400> 876

ggaaagcacu gaugagncga aaggcaaga                                29

<210> 877
<211> 29
<212> RNA
<213> Artificial Sequence

<220>
<223> Synthesized Hammerhead Ribozyme

<220>
<223> "n" represents stem II region of a HH ribozyme.

<400> 877
   gugggggacu gaugagncga aagcagagg                                29

<210> 878
<211> 29
<212> RNA
<213> Artificial Sequence

<220>
<223> Synthesized Hammerhead Ribozyme

<220>
<223> "n" represents stem II region of a HH ribozyme.

<400> 878
   agugggggcu gaugagncga aaagcagag                                          29

<210> 879
<211> 29
<212> RNA
<213> Artificial Sequence

<220>
<223> Synthesized Hammerhead Ribozyme

<220>
<223> "n" represents stem II region of a HH ribozyme.

<400> 879

gaguggggcu gaugagncga aaaagcaga                                          29

<210> 880
<211> 29
<212> RNA
<213> Artificial Sequence

<220>
<223> Synthesized Hammerhead Ribozyme

<220>
<223> "n" represents stem II region of a HH ribozyme.

<400> 880

uugccucacu gaugagncga aaguggggg                                          29

<210> 881
<211> 29
<212> RNA
<213> Artificial Sequence

<220>
<223> Synthesized Hammerhead Ribozyme

<220>
<223> "n" represents stem II region of a HH ribozyme.

<400> 881

uacauuuucu gaugagncga aacuugccu                                    29

<210> 882
<211> 29
<212> RNA
<213> Artificial Sequence

<220>
<223> Synthesized Hammerhead Ribozyme

<220>
<223> "n" represents stem II region of a HH ribozyme.

<400> 882

uuacauuucu gaugagncga aaacuugcc                                    29

<210> 883
<211> 29
<212> RNA
<213> Artificial Sequence

<220>
<223> Synthesized Hammerhead Ribozyme

<220>
<223> "n" represents stem II region of a HH ribozyme.

<400> 883

acaucuuucu gaugagncga aacauuuua                                    29

<210> 884
<211> 29
<212> RNA
<213> Artificial Sequence

<220>
<223> Synthesized Hammerhead Ribozyme

<220>
<223> "n" represents stem II region of a HH ribozyme.

<400> 884

cccagauacu gaugagncga aaucacauc                                    29

<210> 885
<211> 29
<212> RNA

<213> Artificial Sequence

<220>
<223> Synthesized Hammerhead Ribozyme

<220>
<223> "n" represents stem II region of a HH ribozyme.

<400> 885

ccccagaucu gaugagncga aaaucacau                                          29



<210> 886
<211> 29
<212> RNA
<213> Artificial Sequence

<220>
<223> Synthesized Hammerhead Ribozyme

<220>
<223> "n" represents stem II region of a HH ribozyme.

<400> 886

cccccagacu gaugagncga aaaaucaca                                          29



<210> 887
<211> 29
<212> RNA
<213> Artificial Sequence

<220>
<223> Synthesized Hammerhead Ribozyme

<220>
<223> "n" represents stem II region of a HH ribozyme.

<400> 887
   gccccccacu gaugagncga aauaaauca                                          29



<210> 888
<211> 29
<212> RNA
<213> Artificial Sequence

<220>
<223> Synthesized Hammerhead Ribozyme

<220>
<223> "n" represents stem II region of a HH ribozyme.

<400> 888

ccauaccucu gaugagncga aagcccccc                                29

<210> 889
<211> 29
<212> RNA
<213> Artificial Sequence

<220>
<223> Synthesized Hammerhead Ribozyme

<220>
<223> "n" represents stem II region of a HH ribozyme.

<400> 889

ccccaccacu gaugagncga aaccugagc                                29

<210> 890
<211> 29
<212> RNA
<213> Artificial Sequence

<220>
<223> Synthesized Hammerhead Ribozyme

<220>
<223> "n" represents stem II region of a HH ribozyme.

<400> 890

gauuccugcu gaugagncga aauccacuu                                29

<210> 891
<211> 29
<212> RNA
<213> Artificial Sequence

<220>
<223> Synthesized Hammerhead Ribozyme

<220>
<223> "n" represents stem II region of a HH ribozyme.

<400> 891

agauuccucu gaugagncga aaauccacu                                29

<210> 892
<211> 29
<212> RNA
<213> Artificial Sequence

<220>

<223> Synthesized Hammerhead Ribozyme

<220>
<223> "n" represents stem II region of a HH ribozyme.

<400> 892

auuccccacu gaugagncga aauuccuga                                    29

<210> 893
<211> 29
<212> RNA
<213> Artificial Sequence

<220>
<223> Synthesized Hammerhead Ribozyme

<220>
<223> "n" represents stem II region of a HH ribozyme.

<400> 893

ucuuaauacu gaugagncga aauuugcca                                    29

<210> 894
<211> 29
<212> RNA
<213> Artificial Sequence

<220>
<223> Synthesized Hammerhead Ribozyme

<220>
<223> "n" represents stem II region of a HH ribozyme.

<400> 894

cuucuuaacu gaugagncga aauauuugc                                    29

<210> 895
<211> 29
<212> RNA
<213> Artificial Sequence

<220>
<223> Synthesized Hammerhead Ribozyme

<220>
<223> "n" represents stem II region of a HH ribozyme.

<400> 895

cucuucuucu gaugagncga aauauauuu                                    29

<210> 896
<211> 29
<212> RNA
<213> Artificial Sequence

<220>
<223> Synthesized Hammerhead Ribozyme

<220>
<223> "n" represents stem II region of a HH ribozyme.

<400> 896

acucuucucu gaugagncga aaauauauu 29

<210> 897
<211> 29
<212> RNA
<213> Artificial Sequence

<220>
<223> Synthesized Hammerhead Ribozyme

<220>
<223> "n" represents stem II region of a HH ribozyme.

<400> 897

acuuucaacu gaugagncga aacucuucu 29

<210> 898
<211> 29
<212> RNA
<213> Artificial Sequence

<220>
<223> Synthesized Hammerhead Ribozyme

<220>
<223> "n" represents stem II region of a HH ribozyme.

<400> 898

auacuuuccu gaugagncga aauacucuu 29

<210> 899
<211> 29
<212> RNA
<213> Artificial Sequence

<220>
<223> Synthesized Hammerhead Ribozyme

<220>

<223> "n" represents stem II region of a HH ribozyme.

<400> 899

ccuccaaacu gaugagncga aacuuucaa                                    29

<210> 900
<211> 29
<212> RNA
<213> Artificial Sequence

<220>
<223> Synthesized Hammerhead Ribozyme

<220>
<223> "n" represents stem II region of a HH ribozyme.

<400> 900

uuccuccacu gaugagncga aauacuuuc                                    29

<210> 901
<211> 29
<212> RNA
<213> Artificial Sequence

<220>
<223> Synthesized Hammerhead Ribozyme

<220>
<223> "n" represents stem II region of a HH ribozyme.

<400> 901

uuuccucccu gaugagncga aaauacuuu                                    29

<210> 902
<211> 29
<212> RNA
<213> Artificial Sequence

<220>
<223> Synthesized Hammerhead Ribozyme

<220>
<223> "n" represents stem II region of a HH ribozyme.

<400> 902

ccagaauucu gaugagncga aaccauuuu                                    29

<210> 903
<211> 29

<212> RNA
<213> Artificial Sequence

<220>
<223> Synthesized Hammerhead Ribozyme

<220>
<223> "n" represents stem II region of a HH ribozyme.

<400> 903

cccagaaucu gaugagncga aaaccauuu 29

<210> 904
<211> 29
<212> RNA
<213> Artificial Sequence

<220>
<223> Synthesized Hammerhead Ribozyme

<220>
<223> "n" represents stem II region of a HH ribozyme.

<400> 904

acacccagcu gaugagncga aauuaacca 29

<210> 905
<211> 29
<212> RNA
<213> Artificial Sequence

<220>
<223> Synthesized Hammerhead Ribozyme

<220>
<223> "n" represents stem II region of a HH ribozyme.

<400> 905

cacacccacu gaugagncga aaauuaacc 29

<210> 906
<211> 29
<212> RNA
<213> Artificial Sequence

<220>
<223> Synthesized Hammerhead Ribozyme

<220>
<223> "n" represents stem II region of a HH ribozyme.

<400> 906

cucuacugcu gaugagncga aaccuuggu 29

<210> 907
<211> 29
<212> RNA
<213> Artificial Sequence

<220>
<223> Synthesized Hammerhead Ribozyme

<220>
<223> "n" represents stem II region of a HH ribozyme.

<400> 907

acucuacucu gaugagncga aaaccuugg 29

<210> 908
<211> 29
<212> RNA
<213> Artificial Sequence

<220>
<223> Synthesized Hammerhead Ribozyme

<220>
<223> "n" represents stem II region of a HH ribozyme.

<400> 908

guggacuccu gaugagncga aacugaacc 29

<210> 909
<211> 29
<212> RNA
<213> Artificial Sequence

<220>
<223> Synthesized Hammerhead Ribozyme

<220>
<223> "n" represents stem II region of a HH ribozyme.

<400> 909

cagaagugcu gaugagncga aacucuacu 29

<210> 910
<211> 29
<212> RNA
<213> Artificial Sequence

<220>
<223> Synthesized Hammerhead Ribozyme

<220>
<223> "n" represents stem II region of a HH ribozyme.

<400> 910

cagggcagcu gaugagncga aaguggacu                                          29



<210> 911
<211> 29
<212> RNA
<213> Artificial Sequence

<220>
<223> Synthesized Hammerhead Ribozyme

<220>
<223> "n" represents stem II region of a HH ribozyme.

<400> 911

ccagggcacu gaugagncga aaaguggac                                          29



<210> 912
<211> 29
<212> RNA
<213> Artificial Sequence

<220>
<223> Synthesized Hammerhead Ribozyme

<220>
<223> "n" represents stem II region of a HH ribozyme.

<400> 912

agcuaguucu gaugagncga aauuugugg                                          29



<210> 913
<211> 29
<212> RNA
<213> Artificial Sequence

<220>
<223> Synthesized Hammerhead Ribozyme

<220>
<223> "n" represents stem II region of a HH ribozyme.

<400> 913

aauggagccu gaugagncga aaguugauu                                          29

<210> 914
<211> 29
<212> RNA
<213> Artificial Sequence

<220>
<223> Synthesized Hammerhead Ribozyme

<220>
<223> "n" represents stem II region of a HH ribozyme.

<400> 914

uguaaaugcu gaugagncga aagcuaguu                                          29



<210> 915
<211> 29
<212> RNA
<213> Artificial Sequence

<220>
<223> Synthesized Hammerhead Ribozyme

<220>
<223> "n" represents stem II region of a HH ribozyme.

<400> 915

uggcuguacu gaugagncga aauggagcu                                          29



<210> 916
<211> 29
<212> RNA
<213> Artificial Sequence

<220>
<223> Synthesized Hammerhead Ribozyme

<220>
<223> "n" represents stem II region of a HH ribozyme.

<400> 916

auggcugucu gaugagncga aaauggagc                                          29



<210> 917
<211> 29
<212> RNA
<213> Artificial Sequence

<220>
<223> Synthesized Hammerhead Ribozyme

<220>
<223> "n" represents stem II region of a HH ribozyme.

<400> 917

aauggcugcu gaugagncga aaaauggag                                    29

<210> 918
<211> 29
<212> RNA
<213> Artificial Sequence

<220>
<223> Synthesized Hammerhead Ribozyme

<220>
<223> "n" represents stem II region of a HH ribozyme.

<400> 918

auuuuagacu gaugagncga aauggcugu                                    29

<210> 919
<211> 29
<212> RNA
<213> Artificial Sequence

<220>
<223> Synthesized Hammerhead Ribozyme

<220>
<223> "n" represents stem II region of a HH ribozyme.

<400> 919

cauuuuagcu gaugagncga aaauggcug                                    29

<210> 920
<211> 29
<212> RNA
<213> Artificial Sequence

<220>
<223> Synthesized Hammerhead Ribozyme

<220>
<223> "n" represents stem II region of a HH ribozyme.

<400> 920

ccauuuuacu gaugagncga aaaauggcu                                    29

<210> 921
<211> 29
<212> RNA
<213> Artificial Sequence

<220>
<223> Synthesized Hammerhead Ribozyme

<220>
<223> "n" represents stem II region of a HH ribozyme.

<400> 921

ugccauuucu gaugagncga aagaaaugg                                    29

<210> 922
<211> 29
<212> RNA
<213> Artificial Sequence

<220>
<223> Synthesized Hammerhead Ribozyme

<220>
<223> "n" represents stem II region of a HH ribozyme.

<400> 922

uagaacugcu gaugagncga aagcugcca                                    29

<210> 923
<211> 29
<212> RNA
<213> Artificial Sequence

<220>
<223> Synthesized Hammerhead Ribozyme

<220>
<223> "n" represents stem II region of a HH ribozyme.

<400> 923

cuagaacucu gaugagncga aaagcugcc                                    29

<210> 924
<211> 29
<212> RNA
<213> Artificial Sequence

<220>
<223> Synthesized Hammerhead Ribozyme

<220>
<223> "n" represents stem II region of a HH ribozyme.

<400> 924

uucucuagcu gaugagncga aacugaagc                                    29



<210> 925
<211> 29
<212> RNA
<213> Artificial Sequence

<220>
<223> Synthesized Hammerhead Ribozyme

<220>
<223> "n" represents stem II region of a HH ribozyme.

<400> 925

cuucucuacu gaugagncga aaacugaag                                    29



<210> 926
<211> 29
<212> RNA
<213> Artificial Sequence

<220>
<223> Synthesized Hammerhead Ribozyme

<220>
<223> "n" represents stem II region of a HH ribozyme.

<400> 926

uucuucuccu gaugagncga aagaacuga                                    29



<210> 927
<211> 29
<212> RNA
<213> Artificial Sequence

<220>
<223> Synthesized Hammerhead Ribozyme

<220>
<223> "n" represents stem II region of a HH ribozyme.

<400> 927

uuacugcucu gaugagncga aauguuguu                                    29



<210> 928
<211> 29
<212> RNA

<213> Artificial Sequence

<220>
<223> Synthesized Hammerhead Ribozyme

<220>
<223> "n" represents stem II region of a HH ribozyme.

<400> 928

auggacuucu gaugagncga aacugcuga                                        29

<210> 929
<211> 29
<212> RNA
<213> Artificial Sequence

<220>
<223> Synthesized Hammerhead Ribozyme

<220>
<223> "n" represents stem II region of a HH ribozyme.

<400> 929

auuccaugcu gaugagncga aacuuuacu                                        29

<210> 930
<211> 29
<212> RNA
<213> Artificial Sequence

<220>
<223> Synthesized Hammerhead Ribozyme

<220>
<223> "n" represents stem II region of a HH ribozyme.

<400> 930

ccacuagccu gaugagncga aauuccaug                                        29

<210> 931
<211> 29
<212> RNA
<213> Artificial Sequence

<220>
<223> Synthesized Hammerhead Ribozyme

<220>
<223> "n" represents stem II region of a HH ribozyme.

<400> 931

cagaccaccu gaugagncga aagcuauuc                                29

<210> 932
<211> 29
<212> RNA
<213> Artificial Sequence

<220>
<223> Synthesized Hammerhead Ribozyme

<220>
<223> "n" represents stem II region of a HH ribozyme.

<400> 932

gaaacacacu gaugagncga aaccacuag                                29

<210> 933
<211> 29
<212> RNA
<213> Artificial Sequence

<220>
<223> Synthesized Hammerhead Ribozyme

<220>
<223> "n" represents stem II region of a HH ribozyme.

<400> 933

cgaaaagacu gaugagncga aacacagac                                29

<210> 934
<211> 29
<212> RNA
<213> Artificial Sequence

<220>
<223> Synthesized Hammerhead Ribozyme

<220>
<223> "n" represents stem II region of a HH ribozyme.

<400> 934

gcgaaaagcu gaugagncga aaacacaga                                29

<210> 935
<211> 29
<212> RNA
<213> Artificial Sequence

<220>

<223> Synthesized Hammerhead Ribozyme

<220>
<223> "n" represents stem II region of a HH ribozyme.

<400> 935

ggcgaaaacu gaugagncga aaaacacag                                    29



<210> 936
<211> 29
<212> RNA
<213> Artificial Sequence

<220>
<223> Synthesized Hammerhead Ribozyme

<220>
<223> "n" represents stem II region of a HH ribozyme.

<400> 936

auggcgaacu gaugagncga aagaaacac                                    29



<210> 937
<211> 29
<212> RNA
<213> Artificial Sequence

<220>
<223> Synthesized Hammerhead Ribozyme

<220>
<223> "n" represents stem II region of a HH ribozyme.

<400> 937

aauggcgacu gaugagncga aaagaaaca                                    29



<210> 938
<211> 29
<212> RNA
<213> Artificial Sequence

<220>
<223> Synthesized Hammerhead Ribozyme

<220>
<223> "n" represents stem II region of a HH ribozyme.

<400> 938

caauggcgcu gaugagncga aaaagaaac                                    29

<210> 939
<211> 29
<212> RNA
<213> Artificial Sequence

<220>
<223> Synthesized Hammerhead Ribozyme

<220>
<223> "n" represents stem II region of a HH ribozyme.

<400> 939

gcaauggccu gaugagncga aaaaagaaa                                        29

<210> 940
<211> 29
<212> RNA
<213> Artificial Sequence

<220>
<223> Synthesized Hammerhead Ribozyme

<220>
<223> "n" represents stem II region of a HH ribozyme.

<400> 940

agcuaggccu gaugagncga aauggcgaa                                        29

<210> 941
<211> 29
<212> RNA
<213> Artificial Sequence

<220>
<223> Synthesized Hammerhead Ribozyme

<220>
<223> "n" represents stem II region of a HH ribozyme.

<400> 941

cggcaagccu gaugagncga aaggcaaug                                        29

<210> 942
<211> 29
<212> RNA
<213> Artificial Sequence

<220>
<223> Synthesized Hammerhead Ribozyme

<220>

<223> "n" represents stem II region of a HH ribozyme.

<400> 942

auuacggccu gaugagncga aagcuaggc                                    29

<210> 943
<211> 29
<212> RNA
<213> Artificial Sequence

<220>
<223> Synthesized Hammerhead Ribozyme

<220>
<223> "n" represents stem II region of a HH ribozyme.

<400> 943

agaaucaucu gaugagncga aacggcaag                                    29

<210> 944
<211> 29
<212> RNA
<213> Artificial Sequence

<220>
<223> Synthesized Hammerhead Ribozyme

<220>
<223> "n" represents stem II region of a HH ribozyme.

<400> 944

cauuauagcu gaugagncga aaucauuac                                    29

<210> 945
<211> 29
<212> RNA
<213> Artificial Sequence

<220>
<223> Synthesized Hammerhead Ribozyme

<220>
<223> "n" represents stem II region of a HH ribozyme.

<400> 945

gcauuauacu gaugagncga aaaucauua                                    29

<210> 946
<211> 29

<212> RNA
<213> Artificial Sequence

<220>
<223> Synthesized Hammerhead Ribozyme

<220>
<223> "n" represents stem II region of a HH ribozyme.

<400> 946

uggcauuacu gaugagncga aagaaucau 29

<210> 947
<211> 29
<212> RNA
<213> Artificial Sequence

<220>
<223> Synthesized Hammerhead Ribozyme

<220>
<223> "n" represents stem II region of a HH ribozyme.

<400> 947

gauggcaucu gaugagncga aauagaauc 29

<210> 948
<211> 29
<212> RNA
<213> Artificial Sequence

<220>
<223> Synthesized Hammerhead Ribozyme

<220>
<223> "n" represents stem II region of a HH ribozyme.

<400> 948

ugcugcaucu gaugagncga aauggcauu 29

<210> 949
<211> 29
<212> RNA
<213> Artificial Sequence

<220>
<223> Synthesized Hammerhead Ribozyme

<220>
<223> "n" represents stem II region of a HH ribozyme.

<400> 949

ccucucaucu gaugagncga aauugcugc                                    29

<210> 950
<211> 29
<212> RNA
<213> Artificial Sequence

<220>
<223> Synthesized Hammerhead Ribozyme

<220>
<223> "n" represents stem II region of a HH ribozyme.

<400> 950

gccucucacu gaugagncga aaauugcug                                    29

<210> 951
<211> 29
<212> RNA
<213> Artificial Sequence

<220>
<223> Synthesized Hammerhead Ribozyme

<220>
<223> "n" represents stem II region of a HH ribozyme.

<400> 951

ggaugacccu gaugagncga aagccucuc                                    29

<210> 952
<211> 29
<212> RNA
<213> Artificial Sequence

<220>
<223> Synthesized Hammerhead Ribozyme

<220>
<223> "n" represents stem II region of a HH ribozyme.

<400> 952

cuuuggaucu gaugagncga aaccuagcc                                    29

<210> 953
<211> 29
<212> RNA
<213> Artificial Sequence

<220>
<223> Synthesized Hammerhead Ribozyme

<220>
<223> "n" represents stem II region of a HH ribozyme.

<400> 953

ucucuuugcu gaugagncga aaugaccua 29

<210> 954
<211> 29
<212> RNA
<213> Artificial Sequence

<220>
<223> Synthesized Hammerhead Ribozyme

<220>
<223> "n" represents stem II region of a HH ribozyme.

<400> 954

uacauugacu gaugagncga aagggucuu 29

<210> 955
<211> 29
<212> RNA
<213> Artificial Sequence

<220>
<223> Synthesized Hammerhead Ribozyme

<220>
<223> "n" represents stem II region of a HH ribozyme.

<400> 955

ccuacauucu gaugagncga aauaggguc 29

<210> 956
<211> 29
<212> RNA
<213> Artificial Sequence

<220>
<223> Synthesized Hammerhead Ribozyme

<220>
<223> "n" represents stem II region of a HH ribozyme.

<400> 956

uugcaacccu gaugagncga aacauugau 29

<210> 957
<211> 29
<212> RNA
<213> Artificial Sequence

<220>
<223> Synthesized Hammerhead Ribozyme

<220>
<223> "n" represents stem II region of a HH ribozyme.

<400> 957

gauuuugccu gaugagncga aaccuacau                                29

<210> 958
<211> 29
<212> RNA
<213> Artificial Sequence

<220>
<223> Synthesized Hammerhead Ribozyme

<220>
<223> "n" represents stem II region of a HH ribozyme.

<400> 958

agggguuacu gaugagncga aauuuugca                                29

<210> 959
<211> 29
<212> RNA
<213> Artificial Sequence

<220>
<223> Synthesized Hammerhead Ribozyme

<220>
<223> "n" represents stem II region of a HH ribozyme.

<400> 959

uuaggggucu gaugagncga aagauuuug                                29

<210> 960
<211> 29
<212> RNA
<213> Artificial Sequence

<220>
<223> Synthesized Hammerhead Ribozyme

<220>
<223> "n" represents stem II region of a HH ribozyme.

<400> 960

cacuuccucu gaugagncga aaggggguua                                29

<210> 961
<211> 29
<212> RNA
<213> Artificial Sequence

<220>
<223> Synthesized Hammerhead Ribozyme

<220>
<223> "n" represents stem II region of a HH ribozyme.

<400> 961

aaaucaaacu gaugagncga aacugcacu                                29

<210> 962
<211> 29
<212> RNA
<213> Artificial Sequence

<220>
<223> Synthesized Hammerhead Ribozyme

<220>
<223> "n" represents stem II region of a HH ribozyme.

<400> 962

ucaaaucacu gaugagncga aagacugca                                29

<210> 963
<211> 29
<212> RNA
<213> Artificial Sequence

<220>
<223> Synthesized Hammerhead Ribozyme

<220>
<223> "n" represents stem II region of a HH ribozyme.

<400> 963

aucaaauccu gaugagncga aaagacugc                                29

<210> 964

<211> 29
<212> RNA
<213> Artificial Sequence

<220>
<223> Synthesized Hammerhead Ribozyme

<220>
<223> "n" represents stem II region of a HH ribozyme.

<400> 964

ggaaaucacu gaugagncga aaucaaaga 29

<210> 965
<211> 29
<212> RNA
<213> Artificial Sequence

<220>
<223> Synthesized Hammerhead Ribozyme

<220>
<223> "n" represents stem II region of a HH ribozyme.

<400> 965

gggaaauccu gaugagncga aaaucaaag 29

<210> 966
<211> 29
<212> RNA
<213> Artificial Sequence

<220>
<223> Synthesized Hammerhead Ribozyme

<220>
<223> "n" represents stem II region of a HH ribozyme.

<400> 966

acuagggacu gaugagncga aaucaaauc 29

<210> 967
<211> 29
<212> RNA
<213> Artificial Sequence

<220>
<223> Synthesized Hammerhead Ribozyme

<220>
<223> "n" represents stem II region of a HH ribozyme.

<400> 967

uacuagggcu gaugagncga aaaucaaau                                          29

<210> 968
<211> 29
<212> RNA
<213> Artificial Sequence

<220>
<223> Synthesized Hammerhead Ribozyme

<220>
<223> "n" represents stem II region of a HH ribozyme.

<400> 968

uuacuaggcu gaugagncga aaaaucaaa                                          29

<210> 969
<211> 29
<212> RNA
<213> Artificial Sequence

<220>
<223> Synthesized Hammerhead Ribozyme

<220>
<223> "n" represents stem II region of a HH ribozyme.

<400> 969

aagguuaccu gaugagncga aagggaaau                                          29

<210> 970
<211> 29
<212> RNA
<213> Artificial Sequence

<220>
<223> Synthesized Hammerhead Ribozyme

<220>
<223> "n" represents stem II region of a HH ribozyme.

<400> 970

ugcaaggucu gaugagncga aacuaggga                                          29

<210> 971
<211> 29
<212> RNA
<213> Artificial Sequence

<220>
<223> Synthesized Hammerhead Ribozyme

<220>
<223> "n" represents stem II region of a HH ribozyme.

<400> 971

auaucugccu gaugagncga aagguuacu 29

<210> 972
<211> 29
<212> RNA
<213> Artificial Sequence

<220>
<223> Synthesized Hammerhead Ribozyme

<220>
<223> "n" represents stem II region of a HH ribozyme.

<400> 972

guuaaacacu gaugagncga aaucugcaa 29

<210> 973
<211> 29
<212> RNA
<213> Artificial Sequence

<220>
<223> Synthesized Hammerhead Ribozyme

<220>
<223> "n" represents stem II region of a HH ribozyme.

<400> 973

cuugguuacu gaugagncga aacauaucu 29

<210> 974
<211> 29
<212> RNA
<213> Artificial Sequence

<220>
<223> Synthesized Hammerhead Ribozyme

<220>
<223> "n" represents stem II region of a HH ribozyme.

<400> 974

gcuugguucu gaugagncga aaacauauc 29

<210> 975
<211> 29
<212> RNA
<213> Artificial Sequence

<220>
<223> Synthesized Hammerhead Ribozyme

<220>
<223> "n" represents stem II region of a HH ribozyme.

<400> 975

ggcuuggucu gaugagncga aaaacauau                                29

<210> 976
<211> 29
<212> RNA
<213> Artificial Sequence

<220>
<223> Synthesized Hammerhead Ribozyme

<220>
<223> "n" represents stem II region of a HH ribozyme.

<400> 976

gcaugggccu gaugagncga aauggcuug                                29

<210> 977
<211> 29
<212> RNA
<213> Artificial Sequence

<220>
<223> Synthesized Hammerhead Ribozyme

<220>
<223> "n" represents stem II region of a HH ribozyme.

<400> 977

gcccucaacu gaugagncga aaggcaugg                                29

<210> 978
<211> 29
<212> RNA
<213> Artificial Sequence

<220>
<223> Synthesized Hammerhead Ribozyme

<220>
<223> "n" represents stem II region of a HH ribozyme.

<400> 978

agcccucacu gaugagncga aaaggcaug 29

<210> 979
<211> 29
<212> RNA
<213> Artificial Sequence

<220>
<223> Synthesized Hammerhead Ribozyme

<220>
<223> "n" represents stem II region of a HH ribozyme.

<400> 979

cagcccuccu gaugagncga aaaaggcau 29

<210> 980
<211> 29
<212> RNA
<213> Artificial Sequence

<220>
<223> Synthesized Hammerhead Ribozyme

<220>
<223> "n" represents stem II region of a HH ribozyme.

<400> 980

aagucccucu gaugagncga aauuuguuc 29

<210> 981
<211> 29
<212> RNA
<213> Artificial Sequence

<220>
<223> Synthesized Hammerhead Ribozyme

<220>
<223> "n" represents stem II region of a HH ribozyme.

<400> 981

uuaucagucu gaugagncga aagucccuu 29

<210> 982
<211> 29
<212> RNA
<213> Artificial Sequence

<220>
<223> Synthesized Hammerhead Ribozyme

<220>
<223> "n" represents stem II region of a HH ribozyme.

<400> 982

auuaucagcu gaugagncga aaagucccu                                    29

<210> 983
<211> 29
<212> RNA
<213> Artificial Sequence

<220>
<223> Synthesized Hammerhead Ribozyme

<220>
<223> "n" represents stem II region of a HH ribozyme.

<400> 983

aaguaaaucu gaugagncga aaucaguaa                                    29

<210> 984
<211> 29
<212> RNA
<213> Artificial Sequence

<220>
<223> Synthesized Hammerhead Ribozyme

<220>
<223> "n" represents stem II region of a HH ribozyme.

<400> 984

caaaaguacu gaugagncga aauuaucag                                    29

<210> 985
<211> 29
<212> RNA
<213> Artificial Sequence

<220>
<223> Synthesized Hammerhead Ribozyme

<220>
<223> "n" represents stem II region of a HH ribozyme.

<400> 985

ucaaaagucu gaugagncga aaauuauca                                          29

<210> 986
<211> 29
<212> RNA
<213> Artificial Sequence

<220>
<223> Synthesized Hammerhead Ribozyme

<220>
<223> "n" represents stem II region of a HH ribozyme.

<400> 986

aucaaaagcu gaugagncga aaaauuauc                                          29

<210> 987
<211> 29
<212> RNA
<213> Artificial Sequence

<220>
<223> Synthesized Hammerhead Ribozyme

<220>
<223> "n" represents stem II region of a HH ribozyme.

<400> 987

gugaucaacu gaugagncga aaguaaauu                                          29

<210> 988
<211> 29
<212> RNA
<213> Artificial Sequence

<220>
<223> Synthesized Hammerhead Ribozyme

<220>
<223> "n" represents stem II region of a HH ribozyme.

<400> 988

ugugaucacu gaugagncga aaaguaaau                                          29

<210> 989
<211> 29
<212> RNA

<213> Artificial Sequence

<220>
<223> Synthesized Hammerhead Ribozyme

<220>
<223> "n" represents stem II region of a HH ribozyme.

<400> 989

augugauccu gaugagncga aaaaguaaa                                          29



<210> 990
<211> 29
<212> RNA
<213> Artificial Sequence

<220>
<223> Synthesized Hammerhead Ribozyme

<220>
<223> "n" represents stem II region of a HH ribozyme.

<400> 990

cuuaaugucu gaugagncga aaucaaaag                                          29



<210> 991
<211> 29
<212> RNA
<213> Artificial Sequence

<220>
<223> Synthesized Hammerhead Ribozyme

<220>
<223> "n" represents stem II region of a HH ribozyme.

<400> 991

aacaccuucu gaugagncga aaugugauc                                          29



<210> 992
<211> 29
<212> RNA
<213> Artificial Sequence

<220>
<223> Synthesized Hammerhead Ribozyme

<220>
<223> "n" represents stem II region of a HH ribozyme.

<400> 992

gaacaccucu gaugagncga aaaugugau 29

<210> 993
<211> 29
<212> RNA
<213> Artificial Sequence

<220>
<223> Synthesized Hammerhead Ribozyme

<220>
<223> "n" represents stem II region of a HH ribozyme.

<400> 993

aaggugagcu gaugagncga aacaccuua 29

<210> 994
<211> 29
<212> RNA
<213> Artificial Sequence

<220>
<223> Synthesized Hammerhead Ribozyme

<220>
<223> "n" represents stem II region of a HH ribozyme.

<400> 994

caaggugacu gaugagncga aaacaccuu 29

<210> 995
<211> 29
<212> RNA
<213> Artificial Sequence

<220>
<223> Synthesized Hammerhead Ribozyme

<220>
<223> "n" represents stem II region of a HH ribozyme.

<400> 995

uucaaggucu gaugagncga aagaacacc 29

<210> 996
<211> 29
<212> RNA
<213> Artificial Sequence

<220>

<223> Synthesized Hammerhead Ribozyme

<220>
<223> "n" represents stem II region of a HH ribozyme.

<400> 996

aagauuuccu gaugagncga aaggugaga                                    29



<210> 997
<211> 29
<212> RNA
<213> Artificial Sequence

<220>
<223> Synthesized Hammerhead Ribozyme

<220>
<223> "n" represents stem II region of a HH ribozyme.

<400> 997

guguauaacu gaugagncga aauuucaag                                    29



<210> 998
<211> 29
<212> RNA
<213> Artificial Sequence

<220>
<223> Synthesized Hammerhead Ribozyme

<220>
<223> "n" represents stem II region of a HH ribozyme.

<400> 998

caguguaucu gaugagncga aagauuuca                                    29



<210> 999
<211> 29
<212> RNA
<213> Artificial Sequence

<220>
<223> Synthesized Hammerhead Ribozyme

<220>
<223> "n" represents stem II region of a HH ribozyme.

<400> 999

ucaguguacu gaugagncga aaagauuuc                                    29

<210> 1000
<211> 29
<212> RNA
<213> Artificial Sequence

<220>
<223> Synthesized Hammerhead Ribozyme

<220>
<223> "n" represents stem II region of a HH ribozyme.

<400> 1000

uuucagugcu gaugagncga aauaagauu                                    29

<210> 1001
<211> 29
<212> RNA
<213> Artificial Sequence

<220>
<223> Synthesized Hammerhead Ribozyme

<220>
<223> "n" represents stem II region of a HH ribozyme.

<400> 1001

ccuaaauccu gaugagncga aauggccau                                    29

<210> 1002
<211> 29
<212> RNA
<213> Artificial Sequence

<220>
<223> Synthesized Hammerhead Ribozyme

<220>
<223> "n" represents stem II region of a HH ribozyme.

<400> 1002

guggccuacu gaugagncga aaucaaugg                                    29

<210> 1003
<211> 29
<212> RNA
<213> Artificial Sequence

<220>
<223> Synthesized Hammerhead Ribozyme

<220>

<223> "n" represents stem II region of a HH ribozyme.

<400> 1003

aguggccucu gaugagncga aaaucaaug                                    29

<210> 1004
<211> 29
<212> RNA
<213> Artificial Sequence

<220>
<223> Synthesized Hammerhead Ribozyme

<220>
<223> "n" represents stem II region of a HH ribozyme.

<400> 1004

caguggcccu gaugagncga aaaaucaau                                    29

<210> 1005
<211> 29
<212> RNA
<213> Artificial Sequence

<220>
<223> Synthesized Hammerhead Ribozyme

<220>
<223> "n" represents stem II region of a HH ribozyme.

<400> 1005

aguacucucu gaugagncga aagccagug                                    29

<210> 1006
<211> 29
<212> RNA
<213> Artificial Sequence

<220>
<223> Synthesized Hammerhead Ribozyme

<220>
<223> "n" represents stem II region of a HH ribozyme.

<400> 1006

gaguacuccu gaugagncga aaagccagu                                    29

<210> 1007
<211> 29

<212> RNA
<213> Artificial Sequence

<220>
<223> Synthesized Hammerhead Ribozyme

<220>
<223> "n" represents stem II region of a HH ribozyme.

<400> 1007

ggaaggagcu gaugagncga aacucuaag 29

<210> 1008
<211> 29
<212> RNA
<213> Artificial Sequence

<220>
<223> Synthesized Hammerhead Ribozyme

<220>
<223> "n" represents stem II region of a HH ribozyme.

<400> 1008

aggggaagcu gaugagncga aaguacucu 29

<210> 1009
<211> 29
<212> RNA
<213> Artificial Sequence

<220>
<223> Synthesized Hammerhead Ribozyme

<220>
<223> "n" represents stem II region of a HH ribozyme.

<400> 1009

ugcaggggcu gaugagncga aaggaguac 29

<210> 1010
<211> 29
<212> RNA
<213> Artificial Sequence

<220>
<223> Synthesized Hammerhead Ribozyme

<220>
<223> "n" represents stem II region of a HH ribozyme.

<400> 1010

augcagggcu gaugagncga aaaggagua 29

<210> 1011
<211> 29
<212> RNA
<213> Artificial Sequence

<220>
<223> Synthesized Hammerhead Ribozyme

<220>
<223> "n" represents stem II region of a HH ribozyme.

<400> 1011

guauuugucu gaugagncga aaucagugu 29

<210> 1012
<211> 29
<212> RNA
<213> Artificial Sequence

<220>
<223> Synthesized Hammerhead Ribozyme

<220>
<223> "n" represents stem II region of a HH ribozyme.

<400> 1012

aguauuugcu gaugagncga aaaucagug 29

<210> 1013
<211> 29
<212> RNA
<213> Artificial Sequence

<220>
<223> Synthesized Hammerhead Ribozyme

<220>
<223> "n" represents stem II region of a HH ribozyme.

<400> 1013

uaggaaagcu gaugagncga aauuuguaa 29

<210> 1014
<211> 29
<212> RNA
<213> Artificial Sequence

<220>
<223> Synthesized Hammerhead Ribozyme

<220>
<223> "n" represents stem II region of a HH ribozyme.

<400> 1014

gaauaggacu gaugagncga aaguauuug                                    29

<210> 1015
<211> 29
<212> RNA
<213> Artificial Sequence

<220>
<223> Synthesized Hammerhead Ribozyme

<220>
<223> "n" represents stem II region of a HH ribozyme.

<400> 1015

ugaauaggcu gaugagncga aaaguauuu                                    29

<210> 1016
<211> 29
<212> RNA
<213> Artificial Sequence

<220>
<223> Synthesized Hammerhead Ribozyme

<220>
<223> "n" represents stem II region of a HH ribozyme.

<400> 1016

augaauagcu gaugagncga aaaaguauu                                    29

<210> 1017
<211> 29
<212> RNA
<213> Artificial Sequence

<220>
<223> Synthesized Hammerhead Ribozyme

<220>
<223> "n" represents stem II region of a HH ribozyme.

<400> 1017

aguaugaacu gaugagncga aaggaaagu                                    29

<210> 1018
<211> 29
<212> RNA
<213> Artificial Sequence

<220>
<223> Synthesized Hammerhead Ribozyme

<220>
<223> "n" represents stem II region of a HH ribozyme.

<400> 1018

aaaguaugcu gaugagncga aauaggaaa 29

<210> 1019
<211> 29
<212> RNA
<213> Artificial Sequence

<220>
<223> Synthesized Hammerhead Ribozyme

<220>
<223> "n" represents stem II region of a HH ribozyme.

<400> 1019

gaaaguaucu gaugagncga aaauaggaa 29

<210> 1020
<211> 29
<212> RNA
<213> Artificial Sequence

<220>
<223> Synthesized Hammerhead Ribozyme

<220>
<223> "n" represents stem II region of a HH ribozyme.

<400> 1020

uuggaaagcu gaugagncga aaugaauag 29

<210> 1021
<211> 29
<212> RNA
<213> Artificial Sequence

<220>
<223> Synthesized Hammerhead Ribozyme

<220>
<223> "n" represents stem II region of a HH ribozyme.

<400> 1021

uaauuggacu gaugagncga aaguaugaa                                    29

<210> 1022
<211> 29
<212> RNA
<213> Artificial Sequence

<220>
<223> Synthesized Hammerhead Ribozyme

<220>
<223> "n" represents stem II region of a HH ribozyme.

<400> 1022

auaauuggcu gaugagncga aaaguauga                                    29

<210> 1023
<211> 29
<212> RNA
<213> Artificial Sequence

<220>
<223> Synthesized Hammerhead Ribozyme

<220>
<223> "n" represents stem II region of a HH ribozyme.

<400> 1023

cauaauugcu gaugagncga aaaaguaug                                    29

<210> 1024
<211> 29
<212> RNA
<213> Artificial Sequence

<220>
<223> Synthesized Hammerhead Ribozyme

<220>
<223> "n" represents stem II region of a HH ribozyme.

<400> 1024

caucucaucu gaugagncga aauuggaaa                                    29

<210> 1025

<211> 29
<212> RNA
<213> Artificial Sequence

<220>
<223> Synthesized Hammerhead Ribozyme

<220>
<223> "n" represents stem II region of a HH ribozyme.

<400> 1025

ccaucucacu gaugagncga aaauuggaa 29

<210> 1026
<211> 29
<212> RNA
<213> Artificial Sequence

<220>
<223> Synthesized Hammerhead Ribozyme

<220>
<223> "n" represents stem II region of a HH ribozyme.

<400> 1026

acucccagcu gaugagncga aacccacag 29

<210> 1027
<211> 29
<212> RNA
<213> Artificial Sequence

<220>
<223> Synthesized Hammerhead Ribozyme

<220>
<223> "n" represents stem II region of a HH ribozyme.

<400> 1027

guguuagucu gaugagncga aaucacucc 29

<210> 1028
<211> 29
<212> RNA
<213> Artificial Sequence

<220>
<223> Synthesized Hammerhead Ribozyme

<220>
<223> "n" represents stem II region of a HH ribozyme.

<400> 1028
   uauggugucu gaugagncga aagugauca                                     29

<210> 1029
<211> 29
<212> RNA
<213> Artificial Sequence

<220>
<223> Synthesized Hammerhead Ribozyme

<220>
<223> "n" represents stem II region of a HH ribozyme.

<400> 1029

gacauuaccu gaugagncga aaugguguu                                          29

<210> 1030
<211> 29
<212> RNA
<213> Artificial Sequence

<220>
<223> Synthesized Hammerhead Ribozyme

<220>
<223> "n" represents stem II region of a HH ribozyme.

<400> 1030

uuagacaucu gaugagncga aacuauggu                                          29

<210> 1031
<211> 29
<212> RNA
<213> Artificial Sequence

<220>
<223> Synthesized Hammerhead Ribozyme

<220>
<223> "n" represents stem II region of a HH ribozyme.

<400> 1031

gaauauuacu gaugagncga aacauuacu                                          29

<210> 1032
<211> 29
<212> RNA
<213> Artificial Sequence

<220>
<223> Synthesized Hammerhead Ribozyme

<220>
<223> "n" represents stem II region of a HH ribozyme.

<400> 1032

gugaauaucu gaugagncga aagacauua                                    29



<210> 1033
<211> 29
<212> RNA
<213> Artificial Sequence

<220>
<223> Synthesized Hammerhead Ribozyme

<220>
<223> "n" represents stem II region of a HH ribozyme.

<400> 1033

ccugugaacu gaugagncga aauuagaca                                    29



<210> 1034
<211> 29
<212> RNA
<213> Artificial Sequence

<220>
<223> Synthesized Hammerhead Ribozyme

<220>
<223> "n" represents stem II region of a HH ribozyme.

<400> 1034

ugccugugcu gaugagncga aauauuaga                                    29



<210> 1035
<211> 29
<212> RNA
<213> Artificial Sequence

<220>
<223> Synthesized Hammerhead Ribozyme

<220>
<223> "n" represents stem II region of a HH ribozyme.

<400> 1035

cugccugucu gaugagncga aaauauuag                                    29

<210> 1036
<211> 29
<212> RNA
<213> Artificial Sequence

<220>
<223> Synthesized Hammerhead Ribozyme

<220>
<223> "n" represents stem II region of a HH ribozyme.

<400> 1036

cccaagcacu gaugagncga aaucugccu                                          29



<210> 1037
<211> 29
<212> RNA
<213> Artificial Sequence

<220>
<223> Synthesized Hammerhead Ribozyme

<220>
<223> "n" represents stem II region of a HH ribozyme.

<400> 1037

gcuucccccu gaugagncga aagcagauc                                          29



<210> 1038
<211> 29
<212> RNA
<213> Artificial Sequence

<220>
<223> Synthesized Hammerhead Ribozyme

<220>
<223> "n" represents stem II region of a HH ribozyme.

<400> 1038

cacauaaccu gaugagncga aagcuuccc                                          29



<210> 1039
<211> 29
<212> RNA
<213> Artificial Sequence

<220>
<223> Synthesized Hammerhead Ribozyme

<220>
<223> "n" represents stem II region of a HH ribozyme.

<400> 1039

uuucacaucu gaugagncga aacuagcuu                                    29

<210> 1040
<211> 29
<212> RNA
<213> Artificial Sequence

<220>
<223> Synthesized Hammerhead Ribozyme

<220>
<223> "n" represents stem II region of a HH ribozyme.

<400> 1040

cuuucacacu gaugagncga aaacuagcu                                    29

<210> 1041
<211> 29
<212> RNA
<213> Artificial Sequence

<220>
<223> Synthesized Hammerhead Ribozyme

<220>
<223> "n" represents stem II region of a HH ribozyme.

<400> 1041

uaugacuucu gaugagncga aauuugccu                                    29

<210> 1042
<211> 29
<212> RNA
<213> Artificial Sequence

<220>
<223> Synthesized Hammerhead Ribozyme

<220>
<223> "n" represents stem II region of a HH ribozyme.

<400> 1042

uacuguaucu gaugagncga aacuuuauu                                    29

<210> 1043
<211> 29
<212> RNA
<213> Artificial Sequence

<220>
<223> Synthesized Hammerhead Ribozyme

<220>
<223> "n" represents stem II region of a HH ribozyme.

<400> 1043

agcuacugcu gaugagncga aaugacuuu                                    29

<210> 1044
<211> 29
<212> RNA
<213> Artificial Sequence

<220>
<223> Synthesized Hammerhead Ribozyme

<220>
<223> "n" represents stem II region of a HH ribozyme.

<400> 1044

uuuugagccu gaugagncga aacuguaug                                    29

<210> 1045
<211> 29
<212> RNA
<213> Artificial Sequence

<220>
<223> Synthesized Hammerhead Ribozyme

<220>
<223> "n" represents stem II region of a HH ribozyme.

<400> 1045

ugccuuuucu gaugagncga aagcuacug                                    29

<210> 1046
<211> 29
<212> RNA
<213> Artificial Sequence

<220>
<223> Synthesized Hammerhead Ribozyme

<220>
<223> "n" represents stem II region of a HH ribozyme.

<400> 1046

aagagaaucu gaugagncga aaugguugc                                29

<210> 1047
<211> 29
<212> RNA
<213> Artificial Sequence

<220>
<223> Synthesized Hammerhead Ribozyme

<220>
<223> "n" represents stem II region of a HH ribozyme.

<400> 1047

ccaaagagcu gaugagncga aauuauggu                                29

<210> 1048
<211> 29
<212> RNA
<213> Artificial Sequence

<220>
<223> Synthesized Hammerhead Ribozyme

<220>
<223> "n" represents stem II region of a HH ribozyme.

<400> 1048

accaaagacu gaugagncga aaauuaugg                                29

<210> 1049
<211> 29
<212> RNA
<213> Artificial Sequence

<220>
<223> Synthesized Hammerhead Ribozyme

<220>
<223> "n" represents stem II region of a HH ribozyme.

<400> 1049

gcaccaaacu gaugagncga aagaauuau                                29

<210> 1050
<211> 29
<212> RNA

<213> Artificial Sequence

<220>
<223> Synthesized Hammerhead Ribozyme

<220>
<223> "n" represents stem II region of a HH ribozyme.

<400> 1050

uugcaccacu gaugagncga aagagaauu                                        29



<210> 1051
<211> 29
<212> RNA
<213> Artificial Sequence

<220>
<223> Synthesized Hammerhead Ribozyme

<220>
<223> "n" represents stem II region of a HH ribozyme.

<400> 1051

cuugcacccu gaugagncga aaagagaau                                        29



<210> 1052
<211> 29
<212> RNA
<213> Artificial Sequence

<220>
<223> Synthesized Hammerhead Ribozyme

<220>
<223> "n" represents stem II region of a HH ribozyme.

<400> 1052

gcucccaacu gaugagncga aacuugcac                                        29



<210> 1053
<211> 29
<212> RNA
<213> Artificial Sequence

<220>
<223> Synthesized Hammerhead Ribozyme

<220>
<223> "n" represents stem II region of a HH ribozyme.

<400> 1053

acgcuccccu gaugagncga aagacuugc 29

<210> 1054
<211> 29
<212> RNA
<213> Artificial Sequence

<220>
<223> Synthesized Hammerhead Ribozyme

<220>
<223> "n" represents stem II region of a HH ribozyme.

<400> 1054

guaaucuacu gaugagncga aaucacgcu 29

<210> 1055
<211> 29
<212> RNA
<213> Artificial Sequence

<220>
<223> Synthesized Hammerhead Ribozyme

<220>
<223> "n" represents stem II region of a HH ribozyme.

<400> 1055

guguaauccu gaugagncga aagaucacg 29

<210> 1056
<211> 29
<212> RNA
<213> Artificial Sequence

<220>
<223> Synthesized Hammerhead Ribozyme

<220>
<223> "n" represents stem II region of a HH ribozyme.

<400> 1056

ugcagugucu gaugagncga aaucuagau 29

<210> 1057
<211> 29
<212> RNA
<213> Artificial Sequence

<220>

<223> Synthesized Hammerhead Ribozyme

<220>
<223> "n" represents stem II region of a HH ribozyme.

<400> 1057

gugcagugcu gaugagncga aaaucuaga                                          29



<210> 1058
<211> 29
<212> RNA
<213> Artificial Sequence

<220>
<223> Synthesized Hammerhead Ribozyme

<220>
<223> "n" represents stem II region of a HH ribozyme.

<400> 1058

aacuugggcu gaugagncga aauggugca                                          29



<210> 1059
<211> 29
<212> RNA
<213> Artificial Sequence

<220>
<223> Synthesized Hammerhead Ribozyme

<220>
<223> "n" represents stem II region of a HH ribozyme.

<400> 1059

uaacuuggcu gaugagncga aaauggugc                                          29



<210> 1060
<211> 29
<212> RNA
<213> Artificial Sequence

<220>
<223> Synthesized Hammerhead Ribozyme

<220>
<223> "n" represents stem II region of a HH ribozyme.

<400> 1060

aggggauucu gaugagncga aacuuggga                                          29

<210> 1061
<211> 29
<212> RNA
<213> Artificial Sequence

<220>
<223> Synthesized Hammerhead Ribozyme

<220>
<223> "n" represents stem II region of a HH ribozyme.

<400> 1061

caggggaucu gaugagncga aaacuuggg                                          29

<210> 1062
<211> 29
<212> RNA
<213> Artificial Sequence

<220>
<223> Synthesized Hammerhead Ribozyme

<220>
<223> "n" represents stem II region of a HH ribozyme.

<400> 1062

uuucagggcu gaugagncga aauuaacuu                                          29

<210> 1063
<211> 29
<212> RNA
<213> Artificial Sequence

<220>
<223> Synthesized Hammerhead Ribozyme

<220>
<223> "n" represents stem II region of a HH ribozyme.

<400> 1063

uugagagucu gaugagncga aaguuuuca                                          29

<210> 1064
<211> 29
<212> RNA
<213> Artificial Sequence

<220>
<223> Synthesized Hammerhead Ribozyme

<220>

<223> "n" represents stem II region of a HH ribozyme.

<400> 1064

guugagagcu gaugagncga aaaguuuuc                                    29

<210> 1065
<211> 29
<212> RNA
<213> Artificial Sequence

<220>
<223> Synthesized Hammerhead Ribozyme

<220>
<223> "n" represents stem II region of a HH ribozyme.

<400> 1065

ccaguugacu gaugagncga aaguaaguu                                    29

<210> 1066
<211> 29
<212> RNA
<213> Artificial Sequence

<220>
<223> Synthesized Hammerhead Ribozyme

<220>
<223> "n" represents stem II region of a HH ribozyme.

<400> 1066

cuccaguucu gaugagncga aagaguaag                                    29

<210> 1067
<211> 29
<212> RNA
<213> Artificial Sequence

<220>
<223> Synthesized Hammerhead Ribozyme

<220>
<223> "n" represents stem II region of a HH ribozyme.

<400> 1067

ugggaccacu gaugagncga aaguucauu                                    29

<210> 1068
<211> 29

<212> RNA
<213> Artificial Sequence

<220>
<223> Synthesized Hammerhead Ribozyme

<220>
<223> "n" represents stem II region of a HH ribozyme.

<400> 1068

uugggacccu gaugagncga aaaguucau                                          29



<210> 1069
<211> 29
<212> RNA
<213> Artificial Sequence

<220>
<223> Synthesized Hammerhead Ribozyme

<220>
<223> "n" represents stem II region of a HH ribozyme.

<400> 1069

auauuuggcu gaugagncga aaccaaagu                                          29



<210> 1070
<211> 29
<212> RNA
<213> Artificial Sequence

<220>
<223> Synthesized Hammerhead Ribozyme

<220>
<223> "n" represents stem II region of a HH ribozyme.

<400> 1070

aagauggacu gaugagncga aauuuggga                                          29



<210> 1071
<211> 29
<212> RNA
<213> Artificial Sequence

<220>
<223> Synthesized Hammerhead Ribozyme

<220>
<223> "n" represents stem II region of a HH ribozyme.

<400> 1071

aaaagaugcu gaugagncga aauauuugg                                    29

<210> 1072
<211> 29
<212> RNA
<213> Artificial Sequence

<220>
<223> Synthesized Hammerhead Ribozyme

<220>
<223> "n" represents stem II region of a HH ribozyme.

<400> 1072

acugaaaacu gaugagncga aauggauau                                    29

<210> 1073
<211> 29
<212> RNA
<213> Artificial Sequence

<220>
<223> Synthesized Hammerhead Ribozyme

<220>
<223> "n" represents stem II region of a HH ribozyme.

<400> 1073

cuacugaacu gaugagncga aagauggau                                    29

<210> 1074
<211> 29
<212> RNA
<213> Artificial Sequence

<220>
<223> Synthesized Hammerhead Ribozyme

<220>
<223> "n" represents stem II region of a HH ribozyme.

<400> 1074

gcuacugacu gaugagncga aaagaugga                                    29

<210> 1075
<211> 29
<212> RNA
<213> Artificial Sequence

<220>
<223> Synthesized Hammerhead Ribozyme

<220>
<223> "n" represents stem II region of a HH ribozyme.

<400> 1075

cgcuacugcu gaugagncga aaaagaugg                                        29



<210> 1076
<211> 29
<212> RNA
<213> Artificial Sequence

<220>
<223> Synthesized Hammerhead Ribozyme

<220>
<223> "n" represents stem II region of a HH ribozyme.

<400> 1076

acgcuacucu gaugagncga aaaaagaug                                        29



<210> 1077
<211> 29
<212> RNA
<213> Artificial Sequence

<220>
<223> Synthesized Hammerhead Ribozyme

<220>
<223> "n" represents stem II region of a HH ribozyme.

<400> 1077

auuaacgccu gaugagncga aacugaaaa                                        29



<210> 1078
<211> 29
<212> RNA
<213> Artificial Sequence

<220>
<223> Synthesized Hammerhead Ribozyme

<220>
<223> "n" represents stem II region of a HH ribozyme.

<400> 1078

gcauaauucu gaugagncga aacgcuacu                                        29

<210> 1079
<211> 29
<212> RNA
<213> Artificial Sequence

<220>
<223> Synthesized Hammerhead Ribozyme

<220>
<223> "n" represents stem II region of a HH ribozyme.

<400> 1079

agcauaaucu gaugagncga aaacgcuac                                          29

<210> 1080
<211> 29
<212> RNA
<213> Artificial Sequence

<220>
<223> Synthesized Hammerhead Ribozyme

<220>
<223> "n" represents stem II region of a HH ribozyme.

<400> 1080

cagagcaucu gaugagncga aauuaacgc                                          29

<210> 1081
<211> 29
<212> RNA
<213> Artificial Sequence

<220>
<223> Synthesized Hammerhead Ribozyme

<220>
<223> "n" represents stem II region of a HH ribozyme.

<400> 1081

acagagcacu gaugagncga aaauuaacg                                          29

<210> 1082
<211> 29
<212> RNA
<213> Artificial Sequence

<220>
<223> Synthesized Hammerhead Ribozyme

<220>
<223> "n" represents stem II region of a HH ribozyme.

<400> 1082

uggaaacacu gaugagncga aagcauaau                                          29

<210> 1083
<211> 29
<212> RNA
<213> Artificial Sequence

<220>
<223> Synthesized Hammerhead Ribozyme

<220>
<223> "n" represents stem II region of a HH ribozyme.

<400> 1083

caguuggacu gaugagncga aacagagca                                          29

<210> 1084
<211> 29
<212> RNA
<213> Artificial Sequence

<220>
<223> Synthesized Hammerhead Ribozyme

<220>
<223> "n" represents stem II region of a HH ribozyme.

<400> 1084

gcaguuggcu gaugagncga aaacagagc                                          29

<210> 1085
<211> 29
<212> RNA
<213> Artificial Sequence

<220>
<223> Synthesized Hammerhead Ribozyme

<220>
<223> "n" represents stem II region of a HH ribozyme.

<400> 1085

ugcaguugcu gaugagncga aaaacagag                                          29

<210> 1086

<211> 29
<212> RNA
<213> Artificial Sequence

<220>
<223> Synthesized Hammerhead Ribozyme

<220>
<223> "n" represents stem II region of a HH ribozyme.

<400> 1086

ggaaaggacu gaugagncga aaugcaguu                                    29

<210> 1087
<211> 29
<212> RNA
<213> Artificial Sequence

<220>
<223> Synthesized Hammerhead Ribozyme

<220>
<223> "n" represents stem II region of a HH ribozyme.

<400> 1087

uggaaaggcu gaugagncga aaaugcagu                                    29

<210> 1088
<211> 29
<212> RNA
<213> Artificial Sequence

<220>
<223> Synthesized Hammerhead Ribozyme

<220>
<223> "n" represents stem II region of a HH ribozyme.

<400> 1088

uuggaaagcu gaugagncga aaaaugcag                                    29

<210> 1089
<211> 29
<212> RNA
<213> Artificial Sequence

<220>
<223> Synthesized Hammerhead Ribozyme

<220>
<223> "n" represents stem II region of a HH ribozyme.

<400> 1089

caauuggacu gaugagncga aaggaaaug                                29

<210> 1090
<211> 29
<212> RNA
<213> Artificial Sequence

<220>
<223> Synthesized Hammerhead Ribozyme

<220>
<223> "n" represents stem II region of a HH ribozyme.

<400> 1090

ucaauuggcu gaugagncga aaaggaaau                                29

<210> 1091
<211> 29
<212> RNA
<213> Artificial Sequence

<220>
<223> Synthesized Hammerhead Ribozyme

<220>
<223> "n" represents stem II region of a HH ribozyme.

<400> 1091

uucaauugcu gaugagncga aaaaggaaa                                29

<210> 1092
<211> 29
<212> RNA
<213> Artificial Sequence

<220>
<223> Synthesized Hammerhead Ribozyme

<220>
<223> "n" represents stem II region of a HH ribozyme.

<400> 1092

uuuaauuccu gaugagncga aauuggaaa                                29

<210> 1093
<211> 29
<212> RNA
<213> Artificial Sequence

<220>
<223> Synthesized Hammerhead Ribozyme

<220>
<223> "n" represents stem II region of a HH ribozyme.

<400> 1093

cacacuuucu gaugagncga aauucaauu                                    29

<210> 1094
<211> 29
<212> RNA
<213> Artificial Sequence

<220>
<223> Synthesized Hammerhead Ribozyme

<220>
<223> "n" represents stem II region of a HH ribozyme.

<400> 1094

ccacacuucu gaugagncga aaauucaau                                    29

<210> 1095
<211> 29
<212> RNA
<213> Artificial Sequence

<220>
<223> Synthesized Hammerhead Ribozyme

<220>
<223> "n" represents stem II region of a HH ribozyme.

<400> 1095

cuaaaaaccu gaugagncga aaggccaca                                    29

<210> 1096
<211> 29
<212> RNA
<213> Artificial Sequence

<220>
<223> Synthesized Hammerhead Ribozyme

<220>
<223> "n" represents stem II region of a HH ribozyme.

<400> 1096

ugacuaaacu gaugagncga aacgaggcc                                    29

<210> 1097
<211> 29
<212> RNA
<213> Artificial Sequence

<220>
<223> Synthesized Hammerhead Ribozyme

<220>
<223> "n" represents stem II region of a HH ribozyme.

<400> 1097

augacuaacu gaugagncga aaacgaggc                                        29

<210> 1098
<211> 29
<212> RNA
<213> Artificial Sequence

<220>
<223> Synthesized Hammerhead Ribozyme

<220>
<223> "n" represents stem II region of a HH ribozyme.

<400> 1098

aaugacuacu gaugagncga aaaacgagg                                        29

<210> 1099
<211> 29
<212> RNA
<213> Artificial Sequence

<220>
<223> Synthesized Hammerhead Ribozyme

<220>
<223> "n" represents stem II region of a HH ribozyme.

<400> 1099

aaaugacucu gaugagncga aaaaacgag                                        29

<210> 1100
<211> 29
<212> RNA
<213> Artificial Sequence

<220>
<223> Synthesized Hammerhead Ribozyme

<220>
<223> "n" represents stem II region of a HH ribozyme.

<400> 1100

uaaaugaccu gaugagncga aaaaaacga                                    29



<210> 1101
<211> 29
<212> RNA
<213> Artificial Sequence

<220>
<223> Synthesized Hammerhead Ribozyme

<220>
<223> "n" represents stem II region of a HH ribozyme.

<400> 1101

uuuuaaaucu gaugagncga aacuaaaaa                                    29



<210> 1102
<211> 29
<212> RNA
<213> Artificial Sequence

<220>
<223> Synthesized Hammerhead Ribozyme

<220>
<223> "n" represents stem II region of a HH ribozyme.

<400> 1102

caauuuuacu gaugagncga aaugacuaa                                    29



<210> 1103
<211> 29
<212> RNA
<213> Artificial Sequence

<220>
<223> Synthesized Hammerhead Ribozyme

<220>
<223> "n" represents stem II region of a HH ribozyme.

<400> 1103

acaauuuucu gaugagncga aaaugacua                                    29

<210> 1104
<211> 29
<212> RNA
<213> Artificial Sequence

<220>
<223> Synthesized Hammerhead Ribozyme

<220>
<223> "n" represents stem II region of a HH ribozyme.

<400> 1104

aacaauuucu gaugagncga aaaaugacu                                          29

<210> 1105
<211> 29
<212> RNA
<213> Artificial Sequence

<220>
<223> Synthesized Hammerhead Ribozyme

<220>
<223> "n" represents stem II region of a HH ribozyme.

<400> 1105

uagaaaaccu gaugagncga aauuuuaaa                                          29

<210> 1106
<211> 29
<212> RNA
<213> Artificial Sequence

<220>
<223> Synthesized Hammerhead Ribozyme

<220>
<223> "n" represents stem II region of a HH ribozyme.

<400> 1106

acuuagaacu gaugagncga aacaauuuu                                          29

<210> 1107
<211> 29
<212> RNA
<213> Artificial Sequence

<220>
<223> Synthesized Hammerhead Ribozyme

<220>
<223> "n" represents stem II region of a HH ribozyme.

<400> 1107

uacuuagacu gaugagncga aaacaauuu                                          29

<210> 1108
<211> 29
<212> RNA
<213> Artificial Sequence

<220>
<223> Synthesized Hammerhead Ribozyme

<220>
<223> "n" represents stem II region of a HH ribozyme.

<400> 1108

uuacuuagcu gaugagncga aaaacaauu                                          29

<210> 1109
<211> 29
<212> RNA
<213> Artificial Sequence

<220>
<223> Synthesized Hammerhead Ribozyme

<220>
<223> "n" represents stem II region of a HH ribozyme.

<400> 1109

auuacuuacu gaugagncga aaaaacaau                                          29

<210> 1110
<211> 29
<212> RNA
<213> Artificial Sequence

<220>
<223> Synthesized Hammerhead Ribozyme

<220>
<223> "n" represents stem II region of a HH ribozyme.

<400> 1110

caauuacucu gaugagncga aagaaaaca                                          29

<210> 1111
<211> 29
<212> RNA

<213> Artificial Sequence

<220>
<223> Synthesized Hammerhead Ribozyme

<220>
<223> "n" represents stem II region of a HH ribozyme.

<400> 1111

gcagcaaucu gaugagncga aacuuagaa                                          29



<210> 1112
<211> 29
<212> RNA
<213> Artificial Sequence

<220>
<223> Synthesized Hammerhead Ribozyme

<220>
<223> "n" represents stem II region of a HH ribozyme.

<400> 1112

gaggcagccu gaugagncga aauuacuua                                          29



<210> 1113
<211> 29
<212> RNA
<213> Artificial Sequence

<220>
<223> Synthesized Hammerhead Ribozyme

<220>
<223> "n" represents stem II region of a HH ribozyme.

<400> 1113

ccauaauacu gaugagncga aaggcagca                                          29



<210> 1114
<211> 29
<212> RNA
<213> Artificial Sequence

<220>
<223> Synthesized Hammerhead Ribozyme

<220>
<223> "n" represents stem II region of a HH ribozyme.

<400> 1114

ugccauaacu gaugagncga aagaggcag 29

<210> 1115
<211> 29
<212> RNA
<213> Artificial Sequence

<220>
<223> Synthesized Hammerhead Ribozyme

<220>
<223> "n" represents stem II region of a HH ribozyme.

<400> 1115

agugccaucu gaugagncga aauagaggc 29

<210> 1116
<211> 29
<212> RNA
<213> Artificial Sequence

<220>
<223> Synthesized Hammerhead Ribozyme

<220>
<223> "n" represents stem II region of a HH ribozyme.

<400> 1116

aagugccacu gaugagncga aaauagagg 29

<210> 1117
<211> 29
<212> RNA
<213> Artificial Sequence

<220>
<223> Synthesized Hammerhead Ribozyme

<220>
<223> "n" represents stem II region of a HH ribozyme.

<400> 1117

caaaauugcu gaugagncga aagugccau 29

<210> 1118
<211> 29
<212> RNA
<213> Artificial Sequence

<220>

<223> Synthesized Hammerhead Ribozyme

<220>
<223> "n" represents stem II region of a HH ribozyme.

<400> 1118

gcaaaauucu gaugagncga aaagugcca                                    29

<210> 1119
<211> 29
<212> RNA
<213> Artificial Sequence

<220>
<223> Synthesized Hammerhead Ribozyme

<220>
<223> "n" represents stem II region of a HH ribozyme.

<400> 1119

cagugcaacu gaugagncga aauugaagu                                    29

<210> 1120
<211> 29
<212> RNA
<213> Artificial Sequence

<220>
<223> Synthesized Hammerhead Ribozyme

<220>
<223> "n" represents stem II region of a HH ribozyme.

<400> 1120

acagugcacu gaugagncga aaauugaag                                    29

<210> 1121
<211> 29
<212> RNA
<213> Artificial Sequence

<220>
<223> Synthesized Hammerhead Ribozyme

<220>
<223> "n" represents stem II region of a HH ribozyme.

<400> 1121

gacagugccu gaugagncga aaaauugaa                                    29

<210> 1122
<211> 29
<212> RNA
<213> Artificial Sequence

<220>
<223> Synthesized Hammerhead Ribozyme

<220>
<223> "n" represents stem II region of a HH ribozyme.

<400> 1122

ucucaaaacu gaugagncga aacagugca                                        29

<210> 1123
<211> 29
<212> RNA
<213> Artificial Sequence

<220>
<223> Synthesized Hammerhead Ribozyme

<220>
<223> "n" represents stem II region of a HH ribozyme.

<400> 1123

aaucucaacu gaugagncga aagacagug                                        29

<210> 1124
<211> 29
<212> RNA
<213> Artificial Sequence

<220>
<223> Synthesized Hammerhead Ribozyme

<220>
<223> "n" represents stem II region of a HH ribozyme.

<400> 1124

gaaucucacu gaugagncga aaagacagu                                        29

<210> 1125
<211> 29
<212> RNA
<213> Artificial Sequence

<220>
<223> Synthesized Hammerhead Ribozyme

<220>

<223> "n" represents stem II region of a HH ribozyme.

<400> 1125

ugaaucuccu gaugagncga aaaagacag 29

<210> 1126
<211> 29
<212> RNA
<213> Artificial Sequence

<220>
<223> Synthesized Hammerhead Ribozyme

<220>
<223> "n" represents stem II region of a HH ribozyme.

<400> 1126

uuuucuugcu gaugagncga aaucucaaa 29

<210> 1127
<211> 29
<212> RNA
<213> Artificial Sequence

<220>
<223> Synthesized Hammerhead Ribozyme

<220>
<223> "n" represents stem II region of a HH ribozyme.

<400> 1127

uuuuucuucu gaugagncga aaaucucaa 29

<210> 1128
<211> 29
<212> RNA
<213> Artificial Sequence

<220>
<223> Synthesized Hammerhead Ribozyme

<220>
<223> "n" represents stem II region of a HH ribozyme.

<400> 1128

ugaauagacu gaugagncga aauuuuucu 29

<210> 1129
<211> 29

<212> RNA
<213> Artificial Sequence

<220>
<223> Synthesized Hammerhead Ribozyme

<220>
<223> "n" represents stem II region of a HH ribozyme.

<400> 1129

augaauagcu gaugagncga aaauuuuuc                                    29



<210> 1130
<211> 29
<212> RNA
<213> Artificial Sequence

<220>
<223> Synthesized Hammerhead Ribozyme

<220>
<223> "n" represents stem II region of a HH ribozyme.

<400> 1130

aaugaauacu gaugagncga aaaauuuuu                                    29



<210> 1131
<211> 29
<212> RNA
<213> Artificial Sequence

<220>
<223> Synthesized Hammerhead Ribozyme

<220>
<223> "n" represents stem II region of a HH ribozyme.

<400> 1131

aaaaugaacu gaugagncga aagaaauuu                                    29



<210> 1132
<211> 29
<212> RNA
<213> Artificial Sequence

<220>
<223> Synthesized Hammerhead Ribozyme

<220>
<223> "n" represents stem II region of a HH ribozyme.

<400> 1132

aaaaaaugcu gaugagncga aauagaaau                                29

<210> 1133
<211> 29
<212> RNA
<213> Artificial Sequence

<220>
<223> Synthesized Hammerhead Ribozyme

<220>
<223> "n" represents stem II region of a HH ribozyme.

<400> 1133

aaaaaaaucu gaugagncga aaauagaaa                                29

<210> 1134
<211> 29
<212> RNA
<213> Artificial Sequence

<220>
<223> Synthesized Hammerhead Ribozyme

<220>
<223> "n" represents stem II region of a HH ribozyme.

<400> 1134

ugcaaaaacu gaugagncga aaugaauag                                29

<210> 1135
<211> 29
<212> RNA
<213> Artificial Sequence

<220>
<223> Synthesized Hammerhead Ribozyme

<220>
<223> "n" represents stem II region of a HH ribozyme.

<400> 1135

augcaaaacu gaugagncga aaaugaaua                                29

<210> 1136
<211> 29
<212> RNA
<213> Artificial Sequence

<220>
<223> Synthesized Hammerhead Ribozyme

<220>
<223> "n" represents stem II region of a HH ribozyme.

<400> 1136

gaugcaaacu gaugagncga aaaaugaau                                    29



<210> 1137
<211> 29
<212> RNA
<213> Artificial Sequence

<220>
<223> Synthesized Hammerhead Ribozyme

<220>
<223> "n" represents stem II region of a HH ribozyme.

<400> 1137

ggaugcaacu gaugagncga aaaaaugaa                                    29



<210> 1138
<211> 29
<212> RNA
<213> Artificial Sequence

<220>
<223> Synthesized Hammerhead Ribozyme

<220>
<223> "n" represents stem II region of a HH ribozyme.

<400> 1138

uggaugcacu gaugagncga aaaaaauga                                    29



<210> 1139
<211> 29
<212> RNA
<213> Artificial Sequence

<220>
<223> Synthesized Hammerhead Ribozyme

<220>
<223> "n" represents stem II region of a HH ribozyme.

<400> 1139

uuggaugccu gaugagncga aaaaaaaug                                    29

<210> 1140
<211> 29
<212> RNA
<213> Artificial Sequence

<220>
<223> Synthesized Hammerhead Ribozyme

<220>
<223> "n" represents stem II region of a HH ribozyme.

<400> 1140

cacaauugcu gaugagncga aaugcaaaa 29

<210> 1141
<211> 29
<212> RNA
<213> Artificial Sequence

<220>
<223> Synthesized Hammerhead Ribozyme

<220>
<223> "n" represents stem II region of a HH ribozyme.

<400> 1141

ucaggcaccu gaugagncga aauuggaug 29

<210> 1142
<211> 29
<212> RNA
<213> Artificial Sequence

<220>
<223> Synthesized Hammerhead Ribozyme

<220>
<223> "n" represents stem II region of a HH ribozyme.

<400> 1142

uauuuuaacu gaugagncga aaguucagg 29

<210> 1143
<211> 29
<212> RNA
<213> Artificial Sequence

<220>
<223> Synthesized Hammerhead Ribozyme

<220>
<223> "n" represents stem II region of a HH ribozyme.

<400> 1143

auauuuuacu gaugagncga aaaguucag                                29

<210> 1144
<211> 29
<212> RNA
<213> Artificial Sequence

<220>
<223> Synthesized Hammerhead Ribozyme

<220>
<223> "n" represents stem II region of a HH ribozyme.

<400> 1144

cauauuuucu gaugagncga aaaaguuca                                29

<210> 1145
<211> 29
<212> RNA
<213> Artificial Sequence

<220>
<223> Synthesized Hammerhead Ribozyme

<220>
<223> "n" represents stem II region of a HH ribozyme.

<400> 1145

acauauuucu gaugagncga aaaaaguuc                                29

<210> 1146
<211> 29
<212> RNA
<213> Artificial Sequence

<220>
<223> Synthesized Hammerhead Ribozyme

<220>
<223> "n" represents stem II region of a HH ribozyme.

<400> 1146

cauuuacacu gaugagncga aauuuuaaa                                29

<210> 1147

<211> 29
<212> RNA
<213> Artificial Sequence

<220>
<223> Synthesized Hammerhead Ribozyme

<220>
<223> "n" represents stem II region of a HH ribozyme.

<400> 1147

gcagcauucu gaugagncga aacauauuu 29

<210> 1148
<211> 29
<212> RNA
<213> Artificial Sequence

<220>
<223> Synthesized Hammerhead Ribozyme

<220>
<223> "n" represents stem II region of a HH ribozyme.

<400> 1148

ggguuuggcu gaugagncga aacauggca 29

<210> 1149
<211> 29
<212> RNA
<213> Artificial Sequence

<220>
<223> Synthesized Hammerhead Ribozyme

<220>
<223> "n" represents stem II region of a HH ribozyme.

<400> 1149

uggguuugcu gaugagncga aaacauggc 29

<210> 1150
<211> 29
<212> RNA
<213> Artificial Sequence

<220>
<223> Synthesized Hammerhead Ribozyme

<220>
<223> "n" represents stem II region of a HH ribozyme.

<400> 1150
   acacugaccu gaugagncga aauggguuu                                29

<210> 1151
<211> 29
<212> RNA
<213> Artificial Sequence

<220>
<223> Synthesized Hammerhead Ribozyme

<220>
<223> "n" represents stem II region of a HH ribozyme.

<400> 1151

cacacacucu gaugagncga aacgauggg                                29

<210> 1152
<211> 29
<212> RNA
<213> Artificial Sequence

<220>
<223> Synthesized Hammerhead Ribozyme

<220>
<223> "n" represents stem II region of a HH ribozyme.

<400> 1152

cagcucuacu gaugagncga aacacacac                                29

<210> 1153
<211> 29
<212> RNA
<213> Artificial Sequence

<220>
<223> Synthesized Hammerhead Ribozyme

<220>
<223> "n" represents stem II region of a HH ribozyme.

<400> 1153

acagcucucu gaugagncga aaacacaca                                29

<210> 1154
<211> 29
<212> RNA
<213> Artificial Sequence

<220>
<223> Synthesized Hammerhead Ribozyme

<220>
<223> "n" represents stem II region of a HH ribozyme.

<400> 1154

cacagcuccu gaugagncga aaaacacac                                    29



<210> 1155
<211> 29
<212> RNA
<213> Artificial Sequence

<220>
<223> Synthesized Hammerhead Ribozyme

<220>
<223> "n" represents stem II region of a HH ribozyme.

<400> 1155

guuguuuccu gaugagncga aagggugca                                    29



<210> 1156
<211> 29
<212> RNA
<213> Artificial Sequence

<220>
<223> Synthesized Hammerhead Ribozyme

<220>
<223> "n" represents stem II region of a HH ribozyme.

<400> 1156

gggacaagcu gaugagncga aauguuguu                                    29



<210> 1157
<211> 29
<212> RNA
<213> Artificial Sequence

<220>
<223> Synthesized Hammerhead Ribozyme

<220>
<223> "n" represents stem II region of a HH ribozyme.

<400> 1157

caugggaccu gaugagncga aaguauguu                                    29

<210> 1158
<211> 29
<212> RNA
<213> Artificial Sequence

<220>
<223> Synthesized Hammerhead Ribozyme

<220>
<223> "n" represents stem II region of a HH ribozyme.

<400> 1158

gcucauggcu gaugagncga aacaaguau                                        29

<210> 1159
<211> 29
<212> RNA
<213> Artificial Sequence

<220>
<223> Synthesized Hammerhead Ribozyme

<220>
<223> "n" represents stem II region of a HH ribozyme.

<400> 1159

ugaaugcacu gaugagncga aaggggucu                                        29

<210> 1160
<211> 29
<212> RNA
<213> Artificial Sequence

<220>
<223> Synthesized Hammerhead Ribozyme

<220>
<223> "n" represents stem II region of a HH ribozyme.

<400> 1160

gugaaugccu gaugagncga aaagggguc                                        29

<210> 1161
<211> 29
<212> RNA
<213> Artificial Sequence

<220>
<223> Synthesized Hammerhead Ribozyme

<220>
<223> "n" represents stem II region of a HH ribozyme.

<400> 1161

ucucugugcu gaugagncga aaugcaaag                                    29



<210> 1162
<211> 29
<212> RNA
<213> Artificial Sequence

<220>
<223> Synthesized Hammerhead Ribozyme

<220>
<223> "n" represents stem II region of a HH ribozyme.

<400> 1162

cucucugucu gaugagncga aaaugcaaa                                    29



<210> 1163
<211> 29
<212> RNA
<213> Artificial Sequence

<220>
<223> Synthesized Hammerhead Ribozyme

<220>
<223> "n" represents stem II region of a HH ribozyme.

<400> 1163

uaaccaaucu gaugagncga aaccucucu                                    29



<210> 1164
<211> 29
<212> RNA
<213> Artificial Sequence

<220>
<223> Synthesized Hammerhead Ribozyme

<220>
<223> "n" represents stem II region of a HH ribozyme.

<400> 1164

cuauaacccu gaugagncga aaugaccuc                                    29

<210> 1165
<211> 29
<212> RNA
<213> Artificial Sequence

<220>
<223> Synthesized Hammerhead Ribozyme

<220>
<223> "n" represents stem II region of a HH ribozyme.

<400> 1165

gucucuaucu gaugagncga aaccaauga                                    29

<210> 1166
<211> 29
<212> RNA
<213> Artificial Sequence

<220>
<223> Synthesized Hammerhead Ribozyme

<220>
<223> "n" represents stem II region of a HH ribozyme.

<400> 1166

agucucuacu gaugagncga aaaccaaug                                    29

<210> 1167
<211> 29
<212> RNA
<213> Artificial Sequence

<220>
<223> Synthesized Hammerhead Ribozyme

<220>
<223> "n" represents stem II region of a HH ribozyme.

<400> 1167

caagucuccu gaugagncga aauaaccaa                                    29

<210> 1168
<211> 29
<212> RNA
<213> Artificial Sequence

<220>
<223> Synthesized Hammerhead Ribozyme

<220>
<223> "n" represents stem II region of a HH ribozyme.

<400> 1168

auuaauuccu gaugagncga aagucucua                                          29



<210> 1169
<211> 29
<212> RNA
<213> Artificial Sequence

<220>
<223> Synthesized Hammerhead Ribozyme

<220>
<223> "n" represents stem II region of a HH ribozyme.

<400> 1169

cacuuauucu gaugagncga aauucaagu                                          29



<210> 1170
<211> 29
<212> RNA
<213> Artificial Sequence

<220>
<223> Synthesized Hammerhead Ribozyme

<220>
<223> "n" represents stem II region of a HH ribozyme.

<400> 1170

ucacuuaucu gaugagncga aaauucaag                                          29



<210> 1171
<211> 29
<212> RNA
<213> Artificial Sequence

<220>
<223> Synthesized Hammerhead Ribozyme

<220>
<223> "n" represents stem II region of a HH ribozyme.

<400> 1171

augucacucu gaugagncga aauuaauuc                                          29



<210> 1172
<211> 29
<212> RNA

<213> Artificial Sequence

<220>
<223> Synthesized Hammerhead Ribozyme

<220>
<223> "n" represents stem II region of a HH ribozyme.

<400> 1172

acuggcaucu gaugagncga aaugucacu                                          29



<210> 1173
<211> 29
<212> RNA
<213> Artificial Sequence

<220>
<223> Synthesized Hammerhead Ribozyme

<220>
<223> "n" represents stem II region of a HH ribozyme.

<400> 1173

aacuggcacu gaugagncga aaaugucac                                          29



<210> 1174
<211> 29
<212> RNA
<213> Artificial Sequence

<220>
<223> Synthesized Hammerhead Ribozyme

<220>
<223> "n" represents stem II region of a HH ribozyme.

<400> 1174

agaacagacu gaugagncga aacuggcau                                          29



<210> 1175
<211> 29
<212> RNA
<213> Artificial Sequence

<220>
<223> Synthesized Hammerhead Ribozyme

<220>
<223> "n" represents stem II region of a HH ribozyme.

<400> 1175

gagaacagcu gaugagncga aaacuggca                                    29

<210> 1176
<211> 29
<212> RNA
<213> Artificial Sequence

<220>
<223> Synthesized Hammerhead Ribozyme

<220>
<223> "n" represents stem II region of a HH ribozyme.

<400> 1176

agagaacacu gaugagncga aaaacuggc                                    29

<210> 1177
<211> 29
<212> RNA
<213> Artificial Sequence

<220>
<223> Synthesized Hammerhead Ribozyme

<220>
<223> "n" represents stem II region of a HH ribozyme.

<400> 1177

ugugagagcu gaugagncga aacagaaac                                    29

<210> 1178
<211> 29
<212> RNA
<213> Artificial Sequence

<220>
<223> Synthesized Hammerhead Ribozyme

<220>
<223> "n" represents stem II region of a HH ribozyme.

<400> 1178

cugugagacu gaugagncga aaacagaaa                                    29

<210> 1179
<211> 29
<212> RNA
<213> Artificial Sequence

<220>

<223> Synthesized Hammerhead Ribozyme

<220>
<223> "n" represents stem II region of a HH ribozyme.

<400> 1179

accugugacu gaugagncga aagaacaga                                          29



<210> 1180
<211> 29
<212> RNA
<213> Artificial Sequence

<220>
<223> Synthesized Hammerhead Ribozyme

<220>
<223> "n" represents stem II region of a HH ribozyme.

<400> 1180

ucaccugucu gaugagncga aagagaaca                                          29



<210> 1181
<211> 29
<212> RNA
<213> Artificial Sequence

<220>
<223> Synthesized Hammerhead Ribozyme

<220>
<223> "n" represents stem II region of a HH ribozyme.

<400> 1181

gcauuguucu gaugagncga aaucaccug                                          29



<210> 1182
<211> 29
<212> RNA
<213> Artificial Sequence

<220>
<223> Synthesized Hammerhead Ribozyme

<220>
<223> "n" represents stem II region of a HH ribozyme.

<400> 1182

ugcacaaacu gaugagncga aagcauugu                                          29

<210> 1183
<211> 29
<212> RNA
<213> Artificial Sequence

<220>
<223> Synthesized Hammerhead Ribozyme

<220>
<223> "n" represents stem II region of a HH ribozyme.

<400> 1183

gugcacaacu gaugagncga aaagcauug                                    29



<210> 1184
<211> 29
<212> RNA
<213> Artificial Sequence

<220>
<223> Synthesized Hammerhead Ribozyme

<220>
<223> "n" represents stem II region of a HH ribozyme.

<400> 1184

agugcacacu gaugagncga aaaagcauu                                    29



<210> 1185
<211> 29
<212> RNA
<213> Artificial Sequence

<220>
<223> Synthesized Hammerhead Ribozyme

<220>
<223> "n" represents stem II region of a HH ribozyme.

<400> 1185

uagugcaccu gaugagncga aaaaagcau                                    29



<210> 1186
<211> 29
<212> RNA
<213> Artificial Sequence

<220>
<223> Synthesized Hammerhead Ribozyme

<220>

<223> "n" represents stem II region of a HH ribozyme.

<400> 1186

agaguaugcu gaugagncga aagugcaca                                    29

<210> 1187
<211> 29
<212> RNA
<213> Artificial Sequence

<220>
<223> Synthesized Hammerhead Ribozyme

<220>
<223> "n" represents stem II region of a HH ribozyme.

<400> 1187

cugaagagcu gaugagncga aauguagug                                    29

<210> 1188
<211> 29
<212> RNA
<213> Artificial Sequence

<220>
<223> Synthesized Hammerhead Ribozyme

<220>
<223> "n" represents stem II region of a HH ribozyme.

<400> 1188

acacugaacu gaugagncga aaguaugua                                    29

<210> 1189
<211> 29
<212> RNA
<213> Artificial Sequence

<220>
<223> Synthesized Hammerhead Ribozyme

<220>
<223> "n" represents stem II region of a HH ribozyme.

<400> 1189

cuacacugcu gaugagncga aagaguaug                                    29

<210> 1190
<211> 29

<212> RNA
<213> Artificial Sequence

<220>
<223> Synthesized Hammerhead Ribozyme

<220>
<223> "n" represents stem II region of a HH ribozyme.

<400> 1190

ucuacacucu gaugagncga aaagaguau 29

<210> 1191
<211> 29
<212> RNA
<213> Artificial Sequence

<220>
<223> Synthesized Hammerhead Ribozyme

<220>
<223> "n" represents stem II region of a HH ribozyme.

<400> 1191

aagagcuccu gaugagncga aacacugaa 29

<210> 1192
<211> 29
<212> RNA
<213> Artificial Sequence

<220>
<223> Synthesized Hammerhead Ribozyme

<220>
<223> "n" represents stem II region of a HH ribozyme.

<400> 1192

uaaaacaacu gaugagncga aagcucuac 29

<210> 1193
<211> 29
<212> RNA
<213> Artificial Sequence

<220>
<223> Synthesized Hammerhead Ribozyme

<220>
<223> "n" represents stem II region of a HH ribozyme.

<400> 1193

cauaaaaccu gaugagncga aagagcucu 29

<210> 1194
<211> 29
<212> RNA
<213> Artificial Sequence

<220>
<223> Synthesized Hammerhead Ribozyme

<220>
<223> "n" represents stem II region of a HH ribozyme.

<400> 1194

ucccauaacu gaugagncga aacaagagc 29

<210> 1195
<211> 29
<212> RNA
<213> Artificial Sequence

<220>
<223> Synthesized Hammerhead Ribozyme

<220>
<223> "n" represents stem II region of a HH ribozyme.

<400> 1195

uucccauacu gaugagncga aaacaagag 29

<210> 1196
<211> 29
<212> RNA
<213> Artificial Sequence

<220>
<223> Synthesized Hammerhead Ribozyme

<220>
<223> "n" represents stem II region of a HH ribozyme.

<400> 1196

uuucccaucu gaugagncga aaaacaaga 29

<210> 1197
<211> 29
<212> RNA
<213> Artificial Sequence

<220>
<223> Synthesized Hammerhead Ribozyme

<220>
<223> "n" represents stem II region of a HH ribozyme.

<400> 1197

uuuucccacu gaugagncga aaaaacaag                                    29



<210> 1198
<211> 29
<212> RNA
<213> Artificial Sequence

<220>
<223> Synthesized Hammerhead Ribozyme

<220>
<223> "n" represents stem II region of a HH ribozyme.

<400> 1198

uggcauuucu gaugagncga aagccuuuu                                    29



<210> 1199
<211> 29
<212> RNA
<213> Artificial Sequence

<220>
<223> Synthesized Hammerhead Ribozyme

<220>
<223> "n" represents stem II region of a HH ribozyme.

<400> 1199

ucaaacaccu gaugagncga aauuuggca                                    29



<210> 1200
<211> 29
<212> RNA
<213> Artificial Sequence

<220>
<223> Synthesized Hammerhead Ribozyme

<220>
<223> "n" represents stem II region of a HH ribozyme.

<400> 1200

auccaucacu gaugagncga aacacaauu                                    29

<210> 1201
<211> 29
<212> RNA
<213> Artificial Sequence

<220>
<223> Synthesized Hammerhead Ribozyme

<220>
<223> "n" represents stem II region of a HH ribozyme.

<400> 1201

aauccauccu gaugagncga aaacacaau                                    29

<210> 1202
<211> 29
<212> RNA
<213> Artificial Sequence

<220>
<223> Synthesized Hammerhead Ribozyme

<220>
<223> "n" represents stem II region of a HH ribozyme.

<400> 1202

ggcauauucu gaugagncga aauccauca                                    29

<210> 1203
<211> 29
<212> RNA
<213> Artificial Sequence

<220>
<223> Synthesized Hammerhead Ribozyme

<220>
<223> "n" represents stem II region of a HH ribozyme.

<400> 1203

gggcauaucu gaugagncga aaauccauc                                    29

<210> 1204
<211> 29
<212> RNA
<213> Artificial Sequence

<220>
<223> Synthesized Hammerhead Ribozyme

<220>
<223> "n" represents stem II region of a HH ribozyme.

<400> 1204

aaagggcacu gaugagncga aauuaaucc                                    29

<210> 1205
<211> 29
<212> RNA
<213> Artificial Sequence

<220>
<223> Synthesized Hammerhead Ribozyme

<220>
<223> "n" represents stem II region of a HH ribozyme.

<400> 1205

aucggcaacu gaugagncga aagggcaua                                    29

<210> 1206
<211> 29
<212> RNA
<213> Artificial Sequence

<220>
<223> Synthesized Hammerhead Ribozyme

<220>
<223> "n" represents stem II region of a HH ribozyme.

<400> 1206

caucggcacu gaugagncga aaagggcau                                    29

<210> 1207
<211> 29
<212> RNA
<213> Artificial Sequence

<220>
<223> Synthesized Hammerhead Ribozyme

<220>
<223> "n" represents stem II region of a HH ribozyme.

<400> 1207

gcaucggccu gaugagncga aaaagggca                                    29

<210> 1208

<211> 29
<212> RNA
<213> Artificial Sequence

<220>
<223> Synthesized Hammerhead Ribozyme

<220>
<223> "n" represents stem II region of a HH ribozyme.

<400> 1208

aguaauagcu gaugagncga aaugcaucg                                    29

<210> 1209
<211> 29
<212> RNA
<213> Artificial Sequence

<220>
<223> Synthesized Hammerhead Ribozyme

<220>
<223> "n" represents stem II region of a HH ribozyme.

<400> 1209

aucaguaacu gaugagncga aaguaugca                                    29

<210> 1210
<211> 29
<212> RNA
<213> Artificial Sequence

<220>
<223> Synthesized Hammerhead Ribozyme

<220>
<223> "n" represents stem II region of a HH ribozyme.

<400> 1210

acaucagucu gaugagncga aauaguaug                                    29

<210> 1211
<211> 29
<212> RNA
<213> Artificial Sequence

<220>
<223> Synthesized Hammerhead Ribozyme

<220>
<223> "n" represents stem II region of a HH ribozyme.

<400> 1211

cacaucagcu gaugagncga aaauaguau                                          29

<210> 1212
<211> 29
<212> RNA
<213> Artificial Sequence

<220>
<223> Synthesized Hammerhead Ribozyme

<220>
<223> "n" represents stem II region of a HH ribozyme.

<400> 1212

acaaaacccu gaugagncga aagucacau                                          29

<210> 1213
<211> 29
<212> RNA
<213> Artificial Sequence

<220>
<223> Synthesized Hammerhead Ribozyme

<220>
<223> "n" represents stem II region of a HH ribozyme.

<400> 1213

ugcgacaacu gaugagncga aaccgaguc                                          29

<210> 1214
<211> 29
<212> RNA
<213> Artificial Sequence

<220>
<223> Synthesized Hammerhead Ribozyme

<220>
<223> "n" represents stem II region of a HH ribozyme.

<400> 1214

cugcgacacu gaugagncga aaaccgagu                                          29

<210> 1215
<211> 29
<212> RNA
<213> Artificial Sequence

<220>
<223> Synthesized Hammerhead Ribozyme

<220>
<223> "n" represents stem II region of a HH ribozyme.

<400> 1215

gcugcgaccu gaugagncga aaaaccgag                                    29



<210> 1216
<211> 29
<212> RNA
<213> Artificial Sequence

<220>
<223> Synthesized Hammerhead Ribozyme

<220>
<223> "n" represents stem II region of a HH ribozyme.

<400> 1216

aaagcugccu gaugagncga aacaaaacc                                    29



<210> 1217
<211> 29
<212> RNA
<213> Artificial Sequence

<220>
<223> Synthesized Hammerhead Ribozyme

<220>
<223> "n" represents stem II region of a HH ribozyme.

<400> 1217

acaaagcacu gaugagncga aagcugcga                                    29



<210> 1218
<211> 29
<212> RNA
<213> Artificial Sequence

<220>
<223> Synthesized Hammerhead Ribozyme

<220>
<223> "n" represents stem II region of a HH ribozyme.

<400> 1218

aacaaagccu gaugagncga aaagcugcg                                    29

<210> 1219
<211> 29
<212> RNA
<213> Artificial Sequence

<220>
<223> Synthesized Hammerhead Ribozyme

<220>
<223> "n" represents stem II region of a HH ribozyme.

<400> 1219

auuaaacacu gaugagncga aagcaaagc                                29

<210> 1220
<211> 29
<212> RNA
<213> Artificial Sequence

<220>
<223> Synthesized Hammerhead Ribozyme

<220>
<223> "n" represents stem II region of a HH ribozyme.

<400> 1220

cauuaaaccu gaugagncga aaagcaaag                                29

<210> 1221
<211> 29
<212> RNA
<213> Artificial Sequence

<220>
<223> Synthesized Hammerhead Ribozyme

<220>
<223> "n" represents stem II region of a HH ribozyme.

<400> 1221

uuucauuacu gaugagncga aacaaagca                                29

<210> 1222
<211> 29
<212> RNA
<213> Artificial Sequence

<220>
<223> Synthesized Hammerhead Ribozyme

<220>
<223> "n" represents stem II region of a HH ribozyme.

<400> 1222

guuucauucu gaugagncga aaacaaagc                                29

<210> 1223
<211> 29
<212> RNA
<213> Artificial Sequence

<220>
<223> Synthesized Hammerhead Ribozyme

<220>
<223> "n" represents stem II region of a HH ribozyme.

<400> 1223

uguuucaucu gaugagncga aaaacaaag                                29

<210> 1224
<211> 29
<212> RNA
<213> Artificial Sequence

<220>
<223> Synthesized Hammerhead Ribozyme

<220>
<223> "n" represents stem II region of a HH ribozyme.

<400> 1224

agguuuaccu gaugagncga aaguguguu                                29

<210> 1225
<211> 29
<212> RNA
<213> Artificial Sequence

<220>
<223> Synthesized Hammerhead Ribozyme

<220>
<223> "n" represents stem II region of a HH ribozyme.

<400> 1225

aagagguucu gaugagncga aacaagugu                                29

<210> 1226
<211> 29
<212> RNA
<213> Artificial Sequence

<220>
<223> Synthesized Hammerhead Ribozyme

<220>
<223> "n" represents stem II region of a HH ribozyme.

<400> 1226

gugcaaaacu gaugagncga aagguuuac                                          29

<210> 1227
<211> 29
<212> RNA
<213> Artificial Sequence

<220>
<223> Synthesized Hammerhead Ribozyme

<220>
<223> "n" represents stem II region of a HH ribozyme.

<400> 1227

aagugcaacu gaugagncga aagagguuu                                          29

<210> 1228
<211> 29
<212> RNA
<213> Artificial Sequence

<220>
<223> Synthesized Hammerhead Ribozyme

<220>
<223> "n" represents stem II region of a HH ribozyme.

<400> 1228

aaagugcacu gaugagncga aaagagguu                                          29

<210> 1229
<211> 29
<212> RNA
<213> Artificial Sequence

<220>
<223> Synthesized Hammerhead Ribozyme

<220>
<223> "n" represents stem II region of a HH ribozyme.

<400> 1229

caaagugccu gaugagncga aaaagaggu                                    29



<210> 1230
<211> 29
<212> RNA
<213> Artificial Sequence

<220>
<223> Synthesized Hammerhead Ribozyme

<220>
<223> "n" represents stem II region of a HH ribozyme.

<400> 1230

cuuuuucacu gaugagncga aagugcaaa                                    29



<210> 1231
<211> 29
<212> RNA
<213> Artificial Sequence

<220>
<223> Synthesized Hammerhead Ribozyme

<220>
<223> "n" represents stem II region of a HH ribozyme.

<400> 1231

ucuuuuuccu gaugagncga aaagugcaa                                    29



<210> 1232
<211> 29
<212> RNA
<213> Artificial Sequence

<220>
<223> Synthesized Hammerhead Ribozyme

<220>
<223> "n" represents stem II region of a HH ribozyme.

<400> 1232

ucccgcugcu gaugagncga aauucuuuu                                    29



<210> 1233
<211> 29
<212> RNA

<213> Artificial Sequence

<220>
<223> Synthesized Hammerhead Ribozyme

<220>
<223> "n" represents stem II region of a HH ribozyme.

<400> 1233

aggugcuccu gaugagncga aagcauccc                                          29

<210> 1234
<211> 29
<212> RNA
<213> Artificial Sequence

<220>
<223> Synthesized Hammerhead Ribozyme

<220>
<223> "n" represents stem II region of a HH ribozyme.

<400> 1234

aaauuguucu gaugagncga aacaggugc                                          29

<210> 1235
<211> 29
<212> RNA
<213> Artificial Sequence

<220>
<223> Synthesized Hammerhead Ribozyme

<220>
<223> "n" represents stem II region of a HH ribozyme.

<400> 1235

guugagaacu gaugagncga aauuguuua                                          29

<210> 1236
<211> 29
<212> RNA
<213> Artificial Sequence

<220>
<223> Synthesized Hammerhead Ribozyme

<220>
<223> "n" represents stem II region of a HH ribozyme.

<400> 1236

gguugagacu gaugagncga aaauuguuu 29

<210> 1237
<211> 29
<212> RNA
<213> Artificial Sequence

<220>
<223> Synthesized Hammerhead Ribozyme

<220>
<223> "n" represents stem II region of a HH ribozyme.

<400> 1237

agguugagcu gaugagncga aaaauuguu 29

<210> 1238
<211> 29
<212> RNA
<213> Artificial Sequence

<220>
<223> Synthesized Hammerhead Ribozyme

<220>
<223> "n" represents stem II region of a HH ribozyme.

<400> 1238

uagguugacu gaugagncga aaaaauugu 29

<210> 1239
<211> 29
<212> RNA
<213> Artificial Sequence

<220>
<223> Synthesized Hammerhead Ribozyme

<220>
<223> "n" represents stem II region of a HH ribozyme.

<400> 1239

aauagguucu gaugagncga aagaaaauu 29

<210> 1240
<211> 29
<212> RNA
<213> Artificial Sequence

<220>

<223> Synthesized Hammerhead Ribozyme

<220>
<223> "n" represents stem II region of a HH ribozyme.

<400> 1240

acaucaaacu gaugagncga aagguugag                                          29



<210> 1241
<211> 29
<212> RNA
<213> Artificial Sequence

<220>
<223> Synthesized Hammerhead Ribozyme

<220>
<223> "n" represents stem II region of a HH ribozyme.

<400> 1241

gaacaucacu gaugagncga aauagguug                                          29



<210> 1242
<211> 29
<212> RNA
<213> Artificial Sequence

<220>
<223> Synthesized Hammerhead Ribozyme

<220>
<223> "n" represents stem II region of a HH ribozyme.

<400> 1242

ugaacauccu gaugagncga aaauagguu                                          29



<210> 1243
<211> 29
<212> RNA
<213> Artificial Sequence

<220>
<223> Synthesized Hammerhead Ribozyme

<220>
<223> "n" represents stem II region of a HH ribozyme.

<400> 1243

uuuauuugcu gaugagncga aacaucaaa                                          29

<210> 1244
<211> 29
<212> RNA
<213> Artificial Sequence

<220>
<223> Synthesized Hammerhead Ribozyme

<220>
<223> "n" represents stem II region of a HH ribozyme.

<400> 1244

cuuuauuucu gaugagncga aaacaucaa 29

<210> 1245
<211> 29
<212> RNA
<213> Artificial Sequence

<220>
<223> Synthesized Hammerhead Ribozyme

<220>
<223> "n" represents stem II region of a HH ribozyme.

<400> 1245

uaauucuucu gaugagncga aauuugaac 29

<210> 1246
<211> 17
<212> RNA
<213> Homo sapiens

<400> 1246

agtgatgttt aagccaa 17

<210> 1247
<211> 17
<212> RNA
<213> Homo sapiens

<400> 1247

GTGATGTTTA AGCCAAT 17

<210> 1248
<211> 17

<212> RNA
<213> Homo sapiens

<400> 1248

tgatgtttaa gccaatg                                                    17

<210> 1249
<211> 17
<212> RNA
<213> Homo sapiens

<400> 1249

gccaatgtca gggcaag                                                    17

<210> 1250
<211> 17
<212> RNA
<213> Homo sapiens

<400> 1250

gcaacagtcc ctggccg                                                    17

<210> 1251
<211> 17
<212> RNA
<213> Homo sapiens

<400> 1251

ctggccgtcc tccagca                                                    17

<210> 1252
<211> 17
<212> RNA
<213> Homo sapiens

<400> 1252

gccgtcctcc agcacct                                                    17

<210> 1253
<211> 17
<212> RNA
<213> Homo sapiens

<400> 1253

cagcaccttt gtaatgc                                                    17

<210> 1254
<211> 17
<212> RNA
<213> Homo sapiens

<400> 1254

agcacctttg taatgca                                                    17

<210> 1255
<211> 17
<212> RNA
<213> Homo sapiens

<400> 1255

acctttgtaa tgcatat                                                    17

<210> 1256
<211> 17
<212> RNA
<213> Homo sapiens

<400> 1256

taatgcatat gagctcg                                                    17

<210> 1257
<211> 17
<212> RNA
<213> Homo sapiens

<400> 1257

tatgagctcg ggagacc                                                    17

<210> 1258
<211> 17
<212> RNA
<213> Homo sapiens

<400> 1258

agaccagtac ttaaagt                                                    17

<210> 1259
<211> 17

<212> RNA
<213> Homo sapiens

<400> 1259

ccagtactta aagttgg 17

<210> 1260
<211> 17
<212> RNA
<213> Homo sapiens

<400> 1260

cagtacttaa agttgga 17

<210> 1261
<211> 17
<212> RNA
<213> Homo sapiens

<400> 1261

cttaaagttg gaggccc 17

<210> 1262
<211> 17
<212> RNA
<213> Homo sapiens

<400> 1262

gagggcgttc gtcctgg 17

<210> 1263
<211> 17
<212> RNA
<213> Homo sapiens

<400> 1263

agggcgttcg tcctggg 17

<210> 1264
<211> 17
<212> RNA
<213> Homo sapiens

<400> 1264

gcgttcgtcc tgggagc 17

<210> 1265
<211> 17
<212> RNA
<213> Homo sapiens

<400> 1265

gctgcacttg ctccgtc 17

<210> 1266
<211> 17
<212> RNA
<213> Homo sapiens

<400> 1266

cacttgctcc gtcgggt 17

<210> 1267
<211> 17
<212> RNA
<213> Homo sapiens

<400> 1267

tgctccgtcg ggtcgcc 17

<210> 1268
<211> 17
<212> RNA
<213> Homo sapiens

<400> 1268

cgtcgggtcg ccggctt 17

<210> 1269
<211> 17
<212> RNA
<213> Homo sapiens

<400> 1269

cgccggcttc accggac 17

<210> 1270
<211> 17
<212> RNA

<213> Homo sapiens

<400> 1270

gccggcttca ccggacc                                                    17

<210> 1271
<211> 17
<212> RNA
<213> Homo sapiens

<400> 1271

cgcaggctcc cggggca                                                    17

<210> 1272
<211> 17
<212> RNA
<213> Homo sapiens

<400> 1272

ccagagctcg cgtgtcg                                                    17

<210> 1273
<211> 17
<212> RNA
<213> Homo sapiens

<400> 1273

tcgcgtgtcg gcgggac                                                    17

<210> 1274
<211> 17
<212> RNA
<213> Homo sapiens

<400> 1274

cgctgcgtcg cctctaa                                                    17

<210> 1275
<211> 17
<212> RNA
<213> Homo sapiens

<400> 1275

cgtcgcctct aacctcg                                                    17

<210> 1276
<211> 17
<212> RNA
<213> Homo sapiens

<400> 1276

tcgcctctaa cctcggg                                                    17

<210> 1277
<211> 17
<212> RNA
<213> Homo sapiens

<400> 1277

tctaacctcg ggctgtg                                                    17

<210> 1278
<211> 17
<212> RNA
<213> Homo sapiens

<400> 1278

gctgtgctct tttcca                                                     17

<210> 1279
<211> 17
<212> RNA
<213> Homo sapiens

<400> 1279

tgtgctcttt ttccagg                                                    17

<210> 1280
<211> 17
<212> RNA
<213> Homo sapiens

<400> 1280

gtgctctttt tccaggt                                                    17

<210> 1281
<211> 17
<212> RNA
<213> Homo sapiens

<400> 1281

tgctcttttt ccaggtg                                                    17

<210> 1282
<211> 17
<212> RNA
<213> Homo sapiens

<400> 1282

gctctttttc caggtgg                                                    17

<210> 1283
<211> 17
<212> RNA
<213> Homo sapiens

<400> 1283

ctctttttcc aggtggc                                                    17

<210> 1284
<211> 17
<212> RNA
<213> Homo sapiens

<400> 1284

ccgccggttt ctgagcc                                                    17

<210> 1285
<211> 17
<212> RNA
<213> Homo sapiens

<400> 1285

cgccggtttc tgagcct                                                    17

<210> 1286
<211> 17
<212> RNA
<213> Homo sapiens

<400> 1286

gccggtttct gagcctt                                                    17

<210> 1287
<211> 17
<212> RNA
<213> Homo sapiens

<400> 1287

ctgagccttc tgccctg                                                    17

<210> 1288
<211> 17
<212> RNA
<213> Homo sapiens

<400> 1288

tgagccttct gccctgc                                                    17

<210> 1289
<211> 17
<212> RNA
<213> Homo sapiens

<400> 1289

gacacggtct gcaccct                                                    17

<210> 1290
<211> 17
<212> RNA
<213> Homo sapiens

<400> 1290

atgaccctcc acaccaa                                                    17

<210> 1291
<211> 17
<212> RNA
<213> Homo sapiens

<400> 1291

caaagcatct gggatgg                                                    17

<210> 1292
<211> 17
<212> RNA
<213> Homo sapiens

<400> 1292

atggccctac tgcatca                                                    17

<210> 1293
<211> 17
<212> RNA
<213> Homo sapiens

<400> 1293

tactgcatca gatccaa                                                    17

<210> 1294
<211> 17
<212> RNA
<213> Homo sapiens

<400> 1294

catcagatcc aagggaa                                                    17

<210> 1295
<211> 17
<212> RNA
<213> Homo sapiens

<400> 1295

tgaaccgtcc gcagctc                                                    17

<210> 1296
<211> 17
<212> RNA
<213> Homo sapiens

<400> 1296

ccgcagctca agatccc                                                    17

<210> 1297
<211> 17
<212> RNA
<213> Homo sapiens

<400> 1297

ctcaagatcc ccctgga                                                    17

<210> 1298
<211> 17
<212> RNA
<213> Homo sapiens

<400> 1298

cgaggtgtac ctggaca 17

<210> 1299
<211> 17
<212> RNA
<213> Homo sapiens

<400> 1299

cgccgtgtac aactacc 17

<210> 1300
<211> 17
<212> RNA
<213> Homo sapiens

<400> 1300

gtacaactac cccgagg 17

<210> 1301
<211> 17
<212> RNA
<213> Homo sapiens

<400> 1301

cgccgcctac gagttca 17

<210> 1302
<211> 17
<212> RNA
<213> Homo sapiens

<400> 1302

ctacgagttc aacgccg 17

<210> 1303
<211> 17
<212> RNA
<213> Homo sapiens

<400> 1303

tacgagttca acgccgc                                                    17

<210> 1304
<211> 17
<212> RNA
<213> Homo sapiens

<400> 1304

gcgcaggtct acggtca                                                    17

<210> 1305
<211> 17
<212> RNA
<213> Homo sapiens

<400> 1305

gcaggtctac ggtcaga                                                    17

<210> 1306
<211> 17
<212> RNA
<213> Homo sapiens

<400> 1306

tctacggtca gaccggc                                                    17

<210> 1307
<211> 17
<212> RNA
<213> Homo sapiens

<400> 1307

accggcctcc cctacgg                                                    17

<210> 1308
<211> 17
<212> RNA
<213> Homo sapiens

<400> 1308

cctcccctac ggccccg                                                    17

<210> 1309

<211> 17
<212> RNA
<213> Homo sapiens

<400> 1309

ccccgggtct gaggctg                                                    17

<210> 1310
<211> 17
<212> RNA
<213> Homo sapiens

<400> 1310

tgcggcgttc ggctcca                                                    17

<210> 1311
<211> 17
<212> RNA
<213> Homo sapiens

<400> 1311

gcggcgttcg gctccaa                                                    17

<210> 1312
<211> 17
<212> RNA
<213> Homo sapiens

<400> 1312

gttcggctcc aacggcc                                                    17

<210> 1313
<211> 17
<212> RNA
<213> Homo sapiens

<400> 1313

tgggggtttt cccccca                                                    17

<210> 1314
<211> 17
<212> RNA
<213> Homo sapiens

<400> 1314

gggggggtttc cccccac 17

<210> 1315
<211> 17
<212> RNA
<213> Homo sapiens

<400> 1315

gggggtttcc ccccact 17

<210> 1316
<211> 17
<212> RNA
<213> Homo sapiens

<400> 1316

cccccactca acagcgt 17

<210> 1317
<211> 17
<212> RNA
<213> Homo sapiens

<400> 1317

cagcgtgtct ccgagcc 17

<210> 1318
<211> 17
<212> RNA
<213> Homo sapiens

<400> 1318

gcgtgtctcc gagcccg 17

<210> 1319
<211> 17
<212> RNA
<213> Homo sapiens

<400> 1319

ctgatgctac tgcaccc 17

<210> 1320
<211> 17
<212> RNA
<213> Homo sapiens

<400> 1320

gcagctgtcg cctttcc                                                    17

<210> 1321
<211> 17
<212> RNA
<213> Homo sapiens

<400> 1321

tgtcgccttt cctgcag                                                    17

<210> 1322
<211> 17
<212> RNA
<213> Homo sapiens

<400> 1322

gtcgcctttc ctgcagc                                                    17

<210> 1323
<211> 17
<212> RNA
<213> Homo sapiens

<400> 1323

tcgcctttcc tgcagcc                                                    17

<210> 1324
<211> 17
<212> RNA
<213> Homo sapiens

<400> 1324

ggtgccctac tacctgg                                                    17

<210> 1325
<211> 17
<212> RNA
<213> Homo sapiens

<400> 1325

gccctactac ctggaga                                                    17

<210> 1326
<211> 17
<212> RNA
<213> Homo sapiens

<400> 1326

cagcggctac acggtgc                                                    17

<210> 1327
<211> 17
<212> RNA
<213> Homo sapiens

<400> 1327

gccggcattc tacaggc                                                    17

<210> 1328
<211> 17
<212> RNA
<213> Homo sapiens

<400> 1328

ccggcattct acaggcc                                                    17

<210> 1329
<211> 17
<212> RNA
<213> Homo sapiens

<400> 1329

ggcattctac aggccaa                                                    17

<210> 1330
<211> 17
<212> RNA
<213> Homo sapiens

<400> 1330

ggccaaattc agataat                                                    17

<210> 1331

<211> 17
<212> RNA
<213> Homo sapiens

<400> 1331

gccaaattca gataatc                                                    17

<210> 1332
<211> 17
<212> RNA
<213> Homo sapiens

<400> 1332

attcagataa tcgacgc                                                    17

<210> 1333
<211> 17
<212> RNA
<213> Homo sapiens

<400> 1333

cagataatcg acgccag                                                    17

<210> 1334
<211> 17
<212> RNA
<213> Homo sapiens

<400> 1334

agaaagattg gccagta                                                    17

<210> 1335
<211> 17
<212> RNA
<213> Homo sapiens

<400> 1335

tggccagtac caatgac                                                    17

<210> 1336
<211> 17
<212> RNA
<213> Homo sapiens

<400> 1336

agggaagtat ggctatg                                                    17

<210> 1337
<211> 17
<212> RNA
<213> Homo sapiens

<400> 1337

gtatggctat ggaatct                                                    17

<210> 1338
<211> 17
<212> RNA
<213> Homo sapiens

<400> 1338

tatggaatct gccaagg                                                    17

<210> 1339
<211> 17
<212> RNA
<213> Homo sapiens

<400> 1339

aggagactcg ctactgt                                                    17

<210> 1340
<211> 17
<212> RNA
<213> Homo sapiens

<400> 1340

gactcgctac tgtgcag                                                    17

<210> 1341
<211> 17
<212> RNA
<213> Homo sapiens

<400> 1341

caatgactat gcttcag                                                    17

<210> 1342
<211> 17

<212> RNA
<213> Homo sapiens

<400> 1342

actatgcttc aggctac                                                    17

<210> 1343
<211> 17
<212> RNA
<213> Homo sapiens

<400> 1343

ctatgcttca ggctacc                                                    17

<210> 1344
<211> 17
<212> RNA
<213> Homo sapiens

<400> 1344

ttcaggctac cattatg                                                    17

<210> 1345
<211> 17
<212> RNA
<213> Homo sapiens

<400> 1345

gctaccatta tggagtc                                                    17

<210> 1346
<211> 17
<212> RNA
<213> Homo sapiens

<400> 1346

ctaccattat ggagtct                                                    17

<210> 1347
<211> 17
<212> RNA
<213> Homo sapiens

<400> 1347

tatggagtct ggtcctg                                                    17

<210> 1348
<211> 17
<212> RNA
<213> Homo sapiens

<400> 1348

agtctggtcc tgtgagg 17

<210> 1349
<211> 17
<212> RNA
<213> Homo sapiens

<400> 1349

caaggccttc ttcaaga 17

<210> 1350
<211> 17
<212> RNA
<213> Homo sapiens

<400> 1350

aaggccttct tcaagag 17

<210> 1351
<211> 17
<212> RNA
<213> Homo sapiens

<400> 1351

ggccttcttc aagagaa 17

<210> 1352
<211> 17
<212> RNA
<213> Homo sapiens

<400> 1352

gccttcttca agagaag 17

<210> 1353
<211> 17
<212> RNA

<213> Homo sapiens

<400> 1353

agagaagtat tcaagga 17

<210> 1354
<211> 17
<212> RNA
<213> Homo sapiens

<400> 1354

agaagtattc aaggaca 17

<210> 1355
<211> 17
<212> RNA
<213> Homo sapiens

<400> 1355

gaagtattca aggacat 17

<210> 1356
<211> 17
<212> RNA
<213> Homo sapiens

<400> 1356

aaggacataa cgactat 17

<210> 1357
<211> 17
<212> RNA
<213> Homo sapiens

<400> 1357

taacgactat atgtgtc 17

<210> 1358
<211> 17
<212> RNA
<213> Homo sapiens

<400> 1358

acgactatat gtgtcca 17

<210> 1359
<211> 17
<212> RNA
<213> Homo sapiens

<400> 1359

atatgtgtcc agccacc                                                    17

<210> 1360
<211> 17
<212> RNA
<213> Homo sapiens

<400> 1360

tgcaccattg ataaaaa                                                    17

<210> 1361
<211> 17
<212> RNA
<213> Homo sapiens

<400> 1361

ccattgataa aaacagg                                                    17

<210> 1362
<211> 17
<212> RNA
<213> Homo sapiens

<400> 1362

tgccggctcc gcaaatg                                                    17

<210> 1363
<211> 17
<212> RNA
<213> Homo sapiens

<400> 1363

caaatgctac gaagtgg                                                    17

<210> 1364
<211> 17
<212> RNA
<213> Homo sapiens

<400> 1364

ggtgggatac gaaaaga                                                    17

<210> 1365
<211> 17
<212> RNA
<213> Homo sapiens

<400> 1365

gagaatgttg aaacaca                                                    17

<210> 1366
<211> 17
<212> RNA
<213> Homo sapiens

<400> 1366

agtggggtct gctggag                                                    17

<210> 1367
<211> 17
<212> RNA
<213> Homo sapiens

<400> 1367

gccaaccttt ggccaag                                                    17

<210> 1368
<211> 17
<212> RNA
<213> Homo sapiens

<400> 1368

ccaacctttg gccaagc                                                    17

<210> 1369
<211> 17
<212> RNA
<213> Homo sapiens

<400> 1369

agcccgctca tgatcaa                                                    17

<210> 1370
<211> 17
<212> RNA
<213> Homo sapiens

<400> 1370

ctcatgatca aacgctc                                                    17

<210> 1371
<211> 17
<212> RNA
<213> Homo sapiens

<400> 1371

caaacgctct aagaaga                                                    17

<210> 1372
<211> 17
<212> RNA
<213> Homo sapiens

<400> 1372

aacgctctaa gaagaac                                                    17

<210> 1373
<211> 17
<212> RNA
<213> Homo sapiens

<400> 1373

cctggccttg tccctga                                                    17

<210> 1374
<211> 17
<212> RNA
<213> Homo sapiens

<400> 1374

ggccttgtcc ctgacgg                                                    17

<210> 1375
<211> 17
<212> RNA
<213> Homo sapiens

<400> 1375

cagatggtca gtgcctt                                                    17

<210> 1376
<211> 17
<212> RNA
<213> Homo sapiens

<400> 1376

cagtgccttg ttggatg                                                    17

<210> 1377
<211> 17
<212> RNA
<213> Homo sapiens

<400> 1377

tgccttgttg gatgctg                                                    17

<210> 1378
<211> 17
<212> RNA
<213> Homo sapiens

<400> 1378

ccccccatac tctattc                                                    17

<210> 1379
<211> 17
<212> RNA
<213> Homo sapiens

<400> 1379

cccatactct attccga                                                    17

<210> 1380
<211> 17
<212> RNA
<213> Homo sapiens

<400> 1380

catactctat tccgagt                                                    17

<210> 1381
<211> 17
<212> RNA
<213> Homo sapiens

<400> 1381

tactctattc cgagtat                                                    17

<210> 1382
<211> 17
<212> RNA
<213> Homo sapiens

<400> 1382

actctattcc gagtatg                                                    17

<210> 1383
<211> 17
<212> RNA
<213> Homo sapiens

<400> 1383

ttccgagtat gatccta                                                    17

<210> 1384
<211> 17
<212> RNA
<213> Homo sapiens

<400> 1384

agtatgatcc taccaga                                                    17

<210> 1385
<211> 17
<212> RNA
<213> Homo sapiens

<400> 1385

atgatcctac cagaccc                                                    17

<210> 1386
<211> 17
<212> RNA
<213> Homo sapiens

<400> 1386

cagacccttc agtgaag                                                    17

<210> 1387
<211> 17
<212> RNA
<213> Homo sapiens

<400> 1387

agacccttca gtgaagc                                                    17

<210> 1388
<211> 17
<212> RNA
<213> Homo sapiens

<400> 1388

gtgaagcttc gatgatg                                                    17

<210> 1389
<211> 17
<212> RNA
<213> Homo sapiens

<400> 1389

tgaagcttcg atgatgg                                                    17

<210> 1390
<211> 17
<212> RNA
<213> Homo sapiens

<400> 1390

gatgggctta ctgacca                                                    17

<210> 1391
<211> 17
<212> RNA
<213> Homo sapiens

<400> 1391

atgggcttac tgaccaa                                                    17

<210> 1392

<211> 17
<212> RNA
<213> Homo sapiens

<400> 1392

gagctggttc acatgat                                                    17

<210> 1393
<211> 17
<212> RNA
<213> Homo sapiens

<400> 1393

agctggttca catgatc                                                    17

<210> 1394
<211> 17
<212> RNA
<213> Homo sapiens

<400> 1394

cacatgatca actgggc                                                    17

<210> 1395
<211> 17
<212> RNA
<213> Homo sapiens

<400> 1395

gccaggcttt gtggatt                                                    17

<210> 1396
<211> 17
<212> RNA
<213> Homo sapiens

<400> 1396

ccaggctttg tggattt                                                    17

<210> 1397
<211> 17
<212> RNA
<213> Homo sapiens

<400> 1397

ttgtggattt gaccctc                                                    17

<210> 1398
<211> 17
<212> RNA
<213> Homo sapiens

<400> 1398

tgtggatttg accctcc                                                    17

<210> 1399
<211> 17
<212> RNA
<213> Homo sapiens

<400> 1399

ttgaccctcc atgatca                                                    17

<210> 1400
<211> 17
<212> RNA
<213> Homo sapiens

<400> 1400

tccatgatca ggtccac                                                    17

<210> 1401
<211> 17
<212> RNA
<213> Homo sapiens

<400> 1401

gatcaggtcc accttct                                                    17

<210> 1402
<211> 17
<212> RNA
<213> Homo sapiens

<400> 1402

gtccaccttc tagaatg                                                    17

<210> 1403
<211> 17

<212> RNA
<213> Homo sapiens

<400> 1403

tccaccttct agaatgt 17

<210> 1404
<211> 17
<212> RNA
<213> Homo sapiens

<400> 1404

caccttctag aatgtgc 17

<210> 1405
<211> 17
<212> RNA
<213> Homo sapiens

<400> 1405

gcctggctag agatcct 17

<210> 1406
<211> 17
<212> RNA
<213> Homo sapiens

<400> 1406

ctagagatcc tgatgat 17

<210> 1407
<211> 17
<212> RNA
<213> Homo sapiens

<400> 1407

ctgatgattg gtctcgt 17

<210> 1408
<211> 17
<212> RNA
<213> Homo sapiens

<400> 1408

tgattggtct cgtctgg 17

<210> 1409
<211> 17
<212> RNA
<213> Homo sapiens

<400> 1409

attggtctcg tctggcg                                                    17

<210> 1410
<211> 17
<212> RNA
<213> Homo sapiens

<400> 1410

ggtctcgtct ggcgctc                                                    17

<210> 1411
<211> 17
<212> RNA
<213> Homo sapiens

<400> 1411

ctggcgctcc atggagc                                                    17

<210> 1412
<211> 17
<212> RNA
<213> Homo sapiens

<400> 1412

gtgaagctac tgtttgc                                                    17

<210> 1413
<211> 17
<212> RNA
<213> Homo sapiens

<400> 1413

gctactgttt gctccta                                                    17

<210> 1414
<211> 17
<212> RNA

<213> Homo sapiens

<400> 1414

ctactgtttg ctcctaa                                                    17

<210> 1415
<211> 17
<212> RNA
<213> Homo sapiens

<400> 1415

tgtttgctcc taacttg                                                    17

<210> 1416
<211> 17
<212> RNA
<213> Homo sapiens

<400> 1416

ttgctcctaa cttgctc                                                    17

<210> 1417
<211> 17
<212> RNA
<213> Homo sapiens

<400> 1417

tcctaacttg ctcttgg                                                    17

<210> 1418
<211> 17
<212> RNA
<213> Homo sapiens

<400> 1418

aacttgctct tggacag                                                    17

<210> 1419
<211> 17
<212> RNA
<213> Homo sapiens

<400> 1419

cttgctcttg gacagga                                                    17

<210> 1420
<211> 17
<212> RNA
<213> Homo sapiens

<400> 1420

aaatgtgtag agggcat                                                    17

<210> 1421
<211> 17
<212> RNA
<213> Homo sapiens

<400> 1421

gtggagatct tcgacat                                                    17

<210> 1422
<211> 17
<212> RNA
<213> Homo sapiens

<400> 1422

ggagatcttc gacatgc                                                    17

<210> 1423
<211> 17
<212> RNA
<213> Homo sapiens

<400> 1423

gagatcttcg acatgct                                                    17

<210> 1424
<211> 17
<212> RNA
<213> Homo sapiens

<400> 1424

tgctggctac atcatct                                                    17

<210> 1425
<211> 17
<212> RNA
<213> Homo sapiens

<400> 1425

ggctacatca tctcggt                                                    17

<210> 1426
<211> 17
<212> RNA
<213> Homo sapiens

<400> 1426

tacatcatct cggttcc                                                    17

<210> 1427
<211> 17
<212> RNA
<213> Homo sapiens

<400> 1427

catcatctcg gttccgc                                                    17

<210> 1428
<211> 17
<212> RNA
<213> Homo sapiens

<400> 1428

atctcggttc cgcatga                                                    17

<210> 1429
<211> 17
<212> RNA
<213> Homo sapiens

<400> 1429

tctcggttcc gcatgat                                                    17

<210> 1430
<211> 17
<212> RNA
<213> Homo sapiens

<400> 1430

tgatgaatct gcaggga                                                    17

<210> 1431
<211> 17
<212> RNA
<213> Homo sapiens

<400> 1431

agaggagttt gtgtgcc                                                    17

<210> 1432
<211> 17
<212> RNA
<213> Homo sapiens

<400> 1432

gaggagtttg tgtgcct                                                    17

<210> 1433
<211> 17
<212> RNA
<213> Homo sapiens

<400> 1433

gtgtgcctca aatctat                                                    17

<210> 1434
<211> 17
<212> RNA
<213> Homo sapiens

<400> 1434

cctcaaatct attattt                                                    17

<210> 1435
<211> 17
<212> RNA
<213> Homo sapiens

<400> 1435

tcaaatctat tattttg                                                    17

<210> 1436
<211> 17
<212> RNA
<213> Homo sapiens

<400> 1436

aaatctatta ttttgct 17

<210> 1437
<211> 17
<212> RNA
<213> Homo sapiens

<400> 1437

aatctattat tttgctt 17

<210> 1438
<211> 17
<212> RNA
<213> Homo sapiens

<400> 1438

tctattattt tgcttaa 17

<210> 1439
<211> 17
<212> RNA
<213> Homo sapiens

<400> 1439

ctattatttt gcttaat 17

<210> 1440
<211> 17
<212> RNA
<213> Homo sapiens

<400> 1440

tattattttg cttaatt 17

<210> 1441
<211> 17
<212> RNA
<213> Homo sapiens

<400> 1441

attttgctta attctgg 17

<210> 1442

<211> 17
<212> RNA
<213> Homo sapiens

<400> 1442

ttttgcttaa ttctgga                                                    17

<210> 1443
<211> 17
<212> RNA
<213> Homo sapiens

<400> 1443

tgcttaattc tggagtg                                                    17

<210> 1444
<211> 17
<212> RNA
<213> Homo sapiens

<400> 1444

gcttaattct ggagtgt                                                    17

<210> 1445
<211> 17
<212> RNA
<213> Homo sapiens

<400> 1445

tggagtgtac acatttc                                                    17

<210> 1446
<211> 17
<212> RNA
<213> Homo sapiens

<400> 1446

gtacacattt ctgtcca                                                    17

<210> 1447
<211> 17
<212> RNA
<213> Homo sapiens

<400> 1447

tacacatttc tgtccag                                                    17

<210> 1448
<211> 17
<212> RNA
<213> Homo sapiens

<400> 1448

acacatttct gtccagc                                                    17

<210> 1449
<211> 17
<212> RNA
<213> Homo sapiens

<400> 1449

atttctgtcc agcaccc                                                    17

<210> 1450
<211> 17
<212> RNA
<213> Homo sapiens

<400> 1450

cctgaagtct ctggaag                                                    17

<210> 1451
<211> 17
<212> RNA
<213> Homo sapiens

<400> 1451

tgaagtctct ggaagag                                                    17

<210> 1452
<211> 17
<212> RNA
<213> Homo sapiens

<400> 1452

aggaccatat ccaccga                                                    17

<210> 1453
<211> 17

<212> RNA
<213> Homo sapiens

<400> 1453

gaccatatcc accgagt                                                      17



<210> 1454
<211> 17
<212> RNA
<213> Homo sapiens

<400> 1454

caccgagtcc tggacaa                                                      17



<210> 1455
<211> 17
<212> RNA
<213> Homo sapiens

<400> 1455

gacaagatca cagacac                                                      17



<210> 1456
<211> 17
<212> RNA
<213> Homo sapiens

<400> 1456

cagacacttt gatccac                                                      17



<210> 1457
<211> 17
<212> RNA
<213> Homo sapiens

<400> 1457

agacactttg atccacc                                                      17



<210> 1458
<211> 17
<212> RNA
<213> Homo sapiens

<400> 1458

actttgatcc acctgat                                                      17

<210> 1459
<211> 17
<212> RNA
<213> Homo sapiens

<400> 1459

gcccagctcc tcctcat                                                    17

<210> 1460
<211> 17
<212> RNA
<213> Homo sapiens

<400> 1460

cagctcctcc tcatcct                                                    17

<210> 1461
<211> 17
<212> RNA
<213> Homo sapiens

<400> 1461

ctcctcctca tcctctc                                                    17

<210> 1462
<211> 17
<212> RNA
<213> Homo sapiens

<400> 1462

ctcctcatcc tctccca                                                    17

<210> 1463
<211> 17
<212> RNA
<213> Homo sapiens

<400> 1463

ctcatcctct cccacat                                                    17

<210> 1464
<211> 17
<212> RNA

<213> Homo sapiens

<400> 1464

catcctctcc cacatca                                                    17

<210> 1465
<211> 17
<212> RNA
<213> Homo sapiens

<400> 1465

tcccacatca ggcacat                                                    17

<210> 1466
<211> 17
<212> RNA
<213> Homo sapiens

<400> 1466

acatgagtaa caaaggc                                                    17

<210> 1467
<211> 17
<212> RNA
<213> Homo sapiens

<400> 1467

tggagcatct gtacagc                                                    17

<210> 1468
<211> 17
<212> RNA
<213> Homo sapiens

<400> 1468

gcatctgtac agcatga                                                    17

<210> 1469
<211> 17
<212> RNA
<213> Homo sapiens

<400> 1469

gtgcccctct atgacct                                                    17

<210> 1470
<211> 17
<212> RNA
<213> Homo sapiens

<400> 1470

gcccctctat gacctgc                                                    17

<210> 1471
<211> 17
<212> RNA
<213> Homo sapiens

<400> 1471

caccgcctac atgcgcc                                                    17

<210> 1472
<211> 17
<212> RNA
<213> Homo sapiens

<400> 1472

cgcccactag ccgtgga                                                    17

<210> 1473
<211> 17
<212> RNA
<213> Homo sapiens

<400> 1473

aggggcatcc gtggagg                                                    17

<210> 1474
<211> 17
<212> RNA
<213> Homo sapiens

<400> 1474

aagccacttg gccactg                                                    17

<210> 1475
<211> 17
<212> RNA
<213> Homo sapiens

<400> 1475

tgcgggctct acttcat                                                    17

<210> 1476
<211> 17
<212> RNA
<213> Homo sapiens

<400> 1476

cgggctctac ttcatcg                                                    17

<210> 1477
<211> 17
<212> RNA
<213> Homo sapiens

<400> 1477

gctctacttc atcgcat                                                    17

<210> 1478
<211> 17
<212> RNA
<213> Homo sapiens

<400> 1478

ctctacttca tcgcatt                                                    17

<210> 1479
<211> 17
<212> RNA
<213> Homo sapiens

<400> 1479

tacttcatcg cattcct                                                    17

<210> 1480
<211> 17
<212> RNA
<213> Homo sapiens

<400> 1480

catcgcattc cttgcaa                                                    17

<210> 1481
<211> 17
<212> RNA
<213> Homo sapiens

<400> 1481

atcgcattcc ttgcaaa 17

<210> 1482
<211> 17
<212> RNA
<213> Homo sapiens

<400> 1482

gcattccttg caaaagt 17

<210> 1483
<211> 17
<212> RNA
<213> Homo sapiens

<400> 1483

gcaaaagtat tacatca 17

<210> 1484
<211> 17
<212> RNA
<213> Homo sapiens

<400> 1484

aaaagtatta catcacg 17

<210> 1485
<211> 17
<212> RNA
<213> Homo sapiens

<400> 1485

aaagtattac atcacgg 17

<210> 1486
<211> 17
<212> RNA
<213> Homo sapiens

<400> 1486

tattacatca cggggga                                                    17

<210> 1487
<211> 17
<212> RNA
<213> Homo sapiens

<400> 1487

cagagggttt ccctgcc                                                    17

<210> 1488
<211> 17
<212> RNA
<213> Homo sapiens

<400> 1488

agagggtttc cctgcca                                                    17

<210> 1489
<211> 17
<212> RNA
<213> Homo sapiens

<400> 1489

gagggtttcc ctgccac                                                    17

<210> 1490
<211> 17
<212> RNA
<213> Homo sapiens

<400> 1490

gccacagtct gagagct                                                    17

<210> 1491
<211> 17
<212> RNA
<213> Homo sapiens

<400> 1491

tgagagctcc ctggctc                                                    17

<210> 1492
<211> 17
<212> RNA
<213> Homo sapiens

<400> 1492

ccctggctcc cacacgg 17

<210> 1493
<211> 17
<212> RNA
<213> Homo sapiens

<400> 1493

cacacggttc agataat 17

<210> 1494
<211> 17
<212> RNA
<213> Homo sapiens

<400> 1494

acacggttca gataatc 17

<210> 1495
<211> 17
<212> RNA
<213> Homo sapiens

<400> 1495

gttcagataa tccctgc 17

<210> 1496
<211> 17
<212> RNA
<213> Homo sapiens

<400> 1496

cagataatcc ctgctgc 17

<210> 1497
<211> 17
<212> RNA
<213> Homo sapiens

<400> 1497

tgctgcattt taccctc 17

<210> 1498
<211> 17
<212> RNA
<213> Homo sapiens

<400> 1498

gctgcatttt accctca 17

<210> 1499
<211> 17
<212> RNA
<213> Homo sapiens

<400> 1499

ctgcatttta ccctcat 17

<210> 1500
<211> 17
<212> RNA
<213> Homo sapiens

<400> 1500

tgcattttac cctcatc 17

<210> 1501
<211> 17
<212> RNA
<213> Homo sapiens

<400> 1501

tttaccctca tcatgca 17

<210> 1502
<211> 17
<212> RNA
<213> Homo sapiens

<400> 1502

accctcatca tgcacca 17

<210> 1503

<211> 17
<212> RNA
<213> Homo sapiens

<400> 1503

gcaccacttt agccaaa 17

<210> 1504
<211> 17
<212> RNA
<213> Homo sapiens

<400> 1504

caccacttta gccaaat 17

<210> 1505
<211> 17
<212> RNA
<213> Homo sapiens

<400> 1505

accactttag ccaaatt 17

<210> 1506
<211> 17
<212> RNA
<213> Homo sapiens

<400> 1506

agccaaattc tgtctcc 17

<210> 1507
<211> 17
<212> RNA
<213> Homo sapiens

<400> 1507

gccaaattct gtctcct 17

<210> 1508
<211> 17
<212> RNA
<213> Homo sapiens

<400> 1508

aattctgtct cctgcat                                                    17

<210> 1509
<211> 17
<212> RNA
<213> Homo sapiens

<400> 1509

ttctgtctcc tgcatac                                                    17

<210> 1510
<211> 17
<212> RNA
<213> Homo sapiens

<400> 1510

tcctgcatac actccgg                                                    17

<210> 1511
<211> 17
<212> RNA
<213> Homo sapiens

<400> 1511

catacactcc ggcatgc                                                    17

<210> 1512
<211> 17
<212> RNA
<213> Homo sapiens

<400> 1512

gcatgcatcc aacacca                                                    17

<210> 1513
<211> 17
<212> RNA
<213> Homo sapiens

<400> 1513

caatggcttt ctagatg                                                    17

<210> 1514
<211> 17

<212> RNA
<213> Homo sapiens

<400> 1514

aatggctttc tagatga                                                    17

<210> 1515
<211> 17
<212> RNA
<213> Homo sapiens

<400> 1515

atggctttct agatgag                                                    17

<210> 1516
<211> 17
<212> RNA
<213> Homo sapiens

<400> 1516

ggctttctag atgagtg                                                    17

<210> 1517
<211> 17
<212> RNA
<213> Homo sapiens

<400> 1517

gtggccattc atttgct                                                    17

<210> 1518
<211> 17
<212> RNA
<213> Homo sapiens

<400> 1518

tggccattca tttgctt                                                    17

<210> 1519
<211> 17
<212> RNA
<213> Homo sapiens

<400> 1519

ccattcattt gcttgct                                                    17

<210> 1520
<211> 17
<212> RNA
<213> Homo sapiens

<400> 1520

cattcatttg cttgctc                                                    17

<210> 1521
<211> 17
<212> RNA
<213> Homo sapiens

<400> 1521

catttgcttg ctcagtt                                                    17

<210> 1522
<211> 17
<212> RNA
<213> Homo sapiens

<400> 1522

tgcttgctca gttctta                                                    17

<210> 1523
<211> 17
<212> RNA
<213> Homo sapiens

<400> 1523

tgctcagttc ttagtgg                                                    17

<210> 1524
<211> 17
<212> RNA
<213> Homo sapiens

<400> 1524

gctcagttct tagtggc                                                    17

<210> 1525
<211> 17
<212> RNA

<213> Homo sapiens

<400> 1525

tcagttctta gtggcac                                                    17

<210> 1526
<211> 17
<212> RNA
<213> Homo sapiens

<400> 1526

cagttcttag tggcaca                                                    17

<210> 1527
<211> 17
<212> RNA
<213> Homo sapiens

<400> 1527

tggcacatct tctgtct                                                    17

<210> 1528
<211> 17
<212> RNA
<213> Homo sapiens

<400> 1528

gcacatcttc tgtcttc                                                    17

<210> 1529
<211> 17
<212> RNA
<213> Homo sapiens

<400> 1529

cacatcttct gtcttct                                                    17

<210> 1530
<211> 17
<212> RNA
<213> Homo sapiens

<400> 1530

tcttctgtct tctgttg                                                    17

<210> 1531
<211> 17
<212> RNA
<213> Homo sapiens

<400> 1531

ttctgtcttc tgttggg 17

<210> 1532
<211> 17
<212> RNA
<213> Homo sapiens

<400> 1532

tctgtcttct gttggga 17

<210> 1533
<211> 17
<212> RNA
<213> Homo sapiens

<400> 1533

tcttctgttg ggaacag 17

<210> 1534
<211> 17
<212> RNA
<213> Homo sapiens

<400> 1534

aaagggattc caaggct 17

<210> 1535
<211> 17
<212> RNA
<213> Homo sapiens

<400> 1535

aagggattcc aaggcta 17

<210> 1536
<211> 17
<212> RNA
<213> Homo sapiens

<400> 1536

ccaaggctaa atctttg                                                    17

<210> 1537
<211> 17
<212> RNA
<213> Homo sapiens

<400> 1537

ggctaaatct ttgtaac                                                    17

<210> 1538
<211> 17
<212> RNA
<213> Homo sapiens

<400> 1538

ctaaatcttt gtaacag                                                    17

<210> 1539
<211> 17
<212> RNA
<213> Homo sapiens

<400> 1539

taaatctttg taacagc                                                    17

<210> 1540
<211> 17
<212> RNA
<213> Homo sapiens

<400> 1540

atctttgtaa cagctct                                                    17

<210> 1541
<211> 17
<212> RNA
<213> Homo sapiens

<400> 1541

taacagctct ctttccc                                                    17

<210> 1542
<211> 17
<212> RNA
<213> Homo sapiens

<400> 1542

acagctctct ttccccc                                                    17

<210> 1543
<211> 17
<212> RNA
<213> Homo sapiens

<400> 1543
   agctctcttt ccccctt                                                   17

<210> 1544
<211> 17
<212> RNA
<213> Homo sapiens

<400> 1544
   gctctctttc ccccttg                                                   17

<210> 1545
<211> 17
<212> RNA
<213> Homo sapiens

<400> 1545

ctctctttcc cccttgc                                                    17

<210> 1546
<211> 17
<212> RNA
<213> Homo sapiens

<400> 1546
   ttcccccttg ctatgtt                                                   17

<210> 1547
<211> 17
<212> RNA
<213> Homo sapiens

<400> 1547

cccttgctat gttacta                                                    17

<210> 1548
<211> 17
<212> RNA
<213> Homo sapiens

<400> 1548

tgctatgtta ctaagcg                                                    17

<210> 1549
<211> 17
<212> RNA
<213> Homo sapiens

<400> 1549

gctatgttac taagcgt                                                    17

<210> 1550
<211> 17
<212> RNA
<213> Homo sapiens

<400> 1550

atgttactaa gcgtgag                                                    17

<210> 1551
<211> 17
<212> RNA
<213> Homo sapiens

<400> 1551

gtgaggattc ccgtagc                                                    17

<210> 1552
<211> 17
<212> RNA
<213> Homo sapiens

<400> 1552

tgaggattcc cgtagct                                                    17

<210> 1553
<211> 17
<212> RNA
<213> Homo sapiens

<400> 1553

attcccgtag ctcttca                                                    17

<210> 1554
<211> 17
<212> RNA
<213> Homo sapiens

<400> 1554

ccgtagctct tcacagc                                                    17

<210> 1555
<211> 17
<212> RNA
<213> Homo sapiens

<400> 1555

gtagctcttc acagctg                                                    17

<210> 1556
<211> 17
<212> RNA
<213> Homo sapiens

<400> 1556

tagctcttca cagctga                                                    17

<210> 1557
<211> 17
<212> RNA
<213> Homo sapiens

<400> 1557

gctgaactca gtctatg                                                    17

<210> 1558
<211> 17
<212> RNA
<213> Homo sapiens

<400> 1558

aactcagtct atgggtt 17

<210> 1559
<211> 17
<212> RNA
<213> Homo sapiens

<400> 1559

ctcagtctat gggttgg 17

<210> 1560
<211> 17
<212> RNA
<213> Homo sapiens

<400> 1560

ctatgggttg gggctca 17

<210> 1561
<211> 17
<212> RNA
<213> Homo sapiens

<400> 1561

ttggggctca gataact 17

<210> 1562
<211> 17
<212> RNA
<213> Homo sapiens

<400> 1562

gctcagataa ctctgtg 17

<210> 1563
<211> 17
<212> RNA
<213> Homo sapiens

<400> 1563

agataactct gtgcatt 17

<210> 1564

<211> 17
<212> RNA
<213> Homo sapiens

<400> 1564

ctgtgcattt aagctac                                                    17



<210> 1565
<211> 17
<212> RNA
<213> Homo sapiens

<400> 1565

tgtgcattta agctact                                                    17



<210> 1566
<211> 17
<212> RNA
<213> Homo sapiens

<400> 1566

gtgcatttaa gctactt                                                    17



<210> 1567
<211> 17
<212> RNA
<213> Homo sapiens

<400> 1567

tttaagctac ttgtaga                                                    17



<210> 1568
<211> 17
<212> RNA
<213> Homo sapiens

<400> 1568

aagctacttg tagagac                                                    17



<210> 1569
<211> 17
<212> RNA
<213> Homo sapiens

<400> 1569

ctacttgtag agaccca 17

<210> 1570
<211> 17
<212> RNA
<213> Homo sapiens

<400> 1570

tggagagtag acatttt 17

<210> 1571
<211> 17
<212> RNA
<213> Homo sapiens

<400> 1571

gtagacattt tgcctct 17

<210> 1572
<211> 17
<212> RNA
<213> Homo sapiens

<400> 1572

tagacatttt gcctctg 17

<210> 1573
<211> 17
<212> RNA
<213> Homo sapiens

<400> 1573

agacattttg cctctga 17

<210> 1574
<211> 17
<212> RNA
<213> Homo sapiens

<400> 1574

ttttgcctct gataagc 17

<210> 1575
<211> 17

<212> RNA
<213> Homo sapiens

<400> 1575

cctctgataa gcacttt                                                    17

<210> 1576
<211> 17
<212> RNA
<213> Homo sapiens

<400> 1576

taagcacttt ttaaatg                                                    17

<210> 1577
<211> 17
<212> RNA
<213> Homo sapiens

<400> 1577

aagcactttt taaatgg                                                    17

<210> 1578
<211> 17
<212> RNA
<213> Homo sapiens

<400> 1578

agcacttttt aaatggc                                                    17

<210> 1579
<211> 17
<212> RNA
<213> Homo sapiens

<400> 1579

gcacttttta aatggct                                                    17

<210> 1580
<211> 17
<212> RNA
<213> Homo sapiens

<400> 1580

cactttttaa atggctc                                                    17

<210> 1581
<211> 17
<212> RNA
<213> Homo sapiens

<400> 1581

aaatggctct aagaata 17

<210> 1582
<211> 17
<212> RNA
<213> Homo sapiens

<400> 1582

atggctctaa gaataag 17

<210> 1583
<211> 17
<212> RNA
<213> Homo sapiens

<400> 1583

ctaagaataa gccacag 17

<210> 1584
<211> 17
<212> RNA
<213> Homo sapiens

<400> 1584

caaagaattt aaagtgg 17

<210> 1585
<211> 17
<212> RNA
<213> Homo sapiens

<400> 1585

aaagaattta aagtggc 17

<210> 1586
<211> 17
<212> RNA

<213> Homo sapiens

<400> 1586

aagaatttaa agtggct 17

<210> 1587
<211> 17
<212> RNA
<213> Homo sapiens

<400> 1587

aagtggctcc tttaatt 17

<210> 1588
<211> 17
<212> RNA
<213> Homo sapiens

<400> 1588

tggctccttt aattggt 17

<210> 1589
<211> 17
<212> RNA
<213> Homo sapiens

<400> 1589

ggctccttta attggtg 17

<210> 1590
<211> 17
<212> RNA
<213> Homo sapiens

<400> 1590

gctcctttaa ttggtga 17

<210> 1591
<211> 17
<212> RNA
<213> Homo sapiens

<400> 1591

cctttaattg gtgactt 17

<210> 1592
<211> 17
<212> RNA
<213> Homo sapiens

<400> 1592

tggtgacttg gagaaag 17

<210> 1593
<211> 17
<212> RNA
<213> Homo sapiens

<400> 1593

agaaagctag gtcaagg 17

<210> 1594
<211> 17
<212> RNA
<213> Homo sapiens

<400> 1594

agctaggtca agggttt 17

<210> 1595
<211> 17
<212> RNA
<213> Homo sapiens

<400> 1595

tcaagggttt attatag 17

<210> 1596
<211> 17
<212> RNA
<213> Homo sapiens

<400> 1596

caagggttta ttatagc 17

<210> 1597
<211> 17
<212> RNA
<213> Homo sapiens

<400> 1597

aagggtttat tatagca                                                    17

<210> 1598
<211> 17
<212> RNA
<213> Homo sapiens

<400> 1598

gggtttatta tagcacc                                                    17

<210> 1599
<211> 17
<212> RNA
<213> Homo sapiens

<400> 1599

ggtttattat agcaccc                                                    17

<210> 1600
<211> 17
<212> RNA
<213> Homo sapiens

<400> 1600

tttattatag caccctc                                                    17

<210> 1601
<211> 17
<212> RNA
<213> Homo sapiens

<400> 1601

agcaccctct tgtattc                                                    17

<210> 1602
<211> 17
<212> RNA
<213> Homo sapiens

<400> 1602

caccctcttg tattcct                                                    17

<210> 1603
<211> 17
<212> RNA
<213> Homo sapiens

<400> 1603

cctcttgtat tcctatg 17

<210> 1604
<211> 17
<212> RNA
<213> Homo sapiens

<400> 1604

tcttgtattc ctatggc 17

<210> 1605
<211> 17
<212> RNA
<213> Homo sapiens

<400> 1605

cttgtattcc tatggca 17

<210> 1606
<211> 17
<212> RNA
<213> Homo sapiens

<400> 1606

gtattcctat ggcaatg 17

<210> 1607
<211> 17
<212> RNA
<213> Homo sapiens

<400> 1607

caatgcatcc ttttatg 17

<210> 1608
<211> 17
<212> RNA
<213> Homo sapiens

<400> 1608

tgcatccttt tatgaaa                                                    17

<210> 1609
<211> 17
<212> RNA
<213> Homo sapiens

<400> 1609

gcatcctttt atgaaag                                                    17

<210> 1610
<211> 17
<212> RNA
<213> Homo sapiens

<400> 1610

catcctttta tgaaagt                                                    17

<210> 1611
<211> 17
<212> RNA
<213> Homo sapiens

<400> 1611

atccttttat gaaagtg                                                    17

<210> 1612
<211> 17
<212> RNA
<213> Homo sapiens

<400> 1612

aaagtggtac accttaa                                                    17

<210> 1613
<211> 17
<212> RNA
<213> Homo sapiens

<400> 1613

gtacacctta aagcttt                                                    17

<210> 1614
<211> 17
<212> RNA
<213> Homo sapiens

<400> 1614

tacaccttaa agctttt                                                    17

<210> 1615
<211> 17
<212> RNA
<213> Homo sapiens

<400> 1615

ttaaagcttt tatatga                                                    17

<210> 1616
<211> 17
<212> RNA
<213> Homo sapiens

<400> 1616

taaagctttt atatgac                                                    17

<210> 1617
<211> 17
<212> RNA
<213> Homo sapiens

<400> 1617

aaagctttta tatgact                                                    17

<210> 1618
<211> 17
<212> RNA
<213> Homo sapiens

<400> 1618

aagcttttat atgactg                                                    17

<210> 1619
<211> 17
<212> RNA
<213> Homo sapiens

<400> 1619

gcttttatat gactgta                                                    17

<210> 1620
<211> 17
<212> RNA
<213> Homo sapiens

<400> 1620

atgactgtag cagagta                                                    17

<210> 1621
<211> 17
<212> RNA
<213> Homo sapiens

<400> 1621

agcagagtat ctggtga                                                    17

<210> 1622
<211> 17
<212> RNA
<213> Homo sapiens

<400> 1622

cagagtatct ggtgatt                                                    17

<210> 1623
<211> 17
<212> RNA
<213> Homo sapiens

<400> 1623

ctggtgattg tcaattc                                                    17

<210> 1624
<211> 17
<212> RNA
<213> Homo sapiens

<400> 1624

gtgattgtca attcact                                                    17

<210> 1625

<211> 17
<212> RNA
<213> Homo sapiens

<400> 1625

ttgtcaattc acttccc 17

<210> 1626
<211> 17
<212> RNA
<213> Homo sapiens

<400> 1626

tgtcaattca cttcccc 17

<210> 1627
<211> 17
<212> RNA
<213> Homo sapiens

<400> 1627
   aattcacttc cccctat 17

<210> 1628
<211> 17
<212> RNA
<213> Homo sapiens

<400> 1628

attcacttcc ccctata 17

<210> 1629
<211> 17
<212> RNA
<213> Homo sapiens

<400> 1629

ttccccctat aggaata 17

<210> 1630
<211> 17
<212> RNA
<213> Homo sapiens

<400> 1630

ccccctatag gaataca                                                    17

<210> 1631
<211> 17
<212> RNA
<213> Homo sapiens

<400> 1631

ataggaatac aaggggc                                                    17

<210> 1632
<211> 17
<212> RNA
<213> Homo sapiens

<400> 1632

aggcagatcc cctagtt                                                    17

<210> 1633
<211> 17
<212> RNA
<213> Homo sapiens

<400> 1633

gatcccctag ttggcca                                                    17

<210> 1634
<211> 17
<212> RNA
<213> Homo sapiens

<400> 1634

cccctagttg gccaaga                                                    17

<210> 1635
<211> 17
<212> RNA
<213> Homo sapiens

<400> 1635

ccaagactta ttttaac                                                    17

<210> 1636
<211> 17

<212> RNA
<213> Homo sapiens

<400> 1636

caagacttat tttaact 17

<210> 1637
<211> 17
<212> RNA
<213> Homo sapiens

<400> 1637

agacttattt taacttg 17

<210> 1638
<211> 17
<212> RNA
<213> Homo sapiens

<400> 1638

gacttatttt aacttga 17

<210> 1639
<211> 17
<212> RNA
<213> Homo sapiens

<400> 1639

acttatttta acttgat 17

<210> 1640
<211> 17
<212> RNA
<213> Homo sapiens

<400> 1640

cttattttaa cttgata 17

<210> 1641
<211> 17
<212> RNA
<213> Homo sapiens

<400> 1641

ttttaacttg atacact 17

<210> 1642
<211> 17
<212> RNA
<213> Homo sapiens

<400> 1642

aacttgatac actgcag                                                    17

<210> 1643
<211> 17
<212> RNA
<213> Homo sapiens

<400> 1643

ctgcagattc agagtgt                                                    17

<210> 1644
<211> 17
<212> RNA
<213> Homo sapiens

<400> 1644

tgcagattca gagtgtc                                                    17

<210> 1645
<211> 17
<212> RNA
<213> Homo sapiens

<400> 1645

cagagtgtcc tgaagct                                                    17

<210> 1646
<211> 17
<212> RNA
<213> Homo sapiens

<400> 1646

ctgaagctct gcctctg                                                    17

<210> 1647
<211> 17
<212> RNA

<213> Homo sapiens

<400> 1647

ctctgcctct ggctttc                                                    17

<210> 1648
<211> 17
<212> RNA
<213> Homo sapiens

<400> 1648

ctctggcttt ccggtca                                                    17

<210> 1649
<211> 17
<212> RNA
<213> Homo sapiens

<400> 1649

tctggctttc cggtcat                                                    17

<210> 1650
<211> 17
<212> RNA
<213> Homo sapiens

<400> 1650

ctggctttcc ggtcatg                                                    17

<210> 1651
<211> 17
<212> RNA
<213> Homo sapiens

<400> 1651

tttccggtca tgggttc                                                    17

<210> 1652
<211> 17
<212> RNA
<213> Homo sapiens

<400> 1652

tcatgggttc cagttaa                                                    17

<210> 1653
<211> 17
<212> RNA
<213> Homo sapiens

<400> 1653

catgggttcc agttaat                                                    17

<210> 1654
<211> 17
<212> RNA
<213> Homo sapiens

<400> 1654

gttccagtta attcatg                                                    17

<210> 1655
<211> 17
<212> RNA
<213> Homo sapiens

<400> 1655

ttccagttaa ttcatgc                                                    17

<210> 1656
<211> 17
<212> RNA
<213> Homo sapiens

<400> 1656

cagttaattc atgcctc                                                    17

<210> 1657
<211> 17
<212> RNA
<213> Homo sapiens

<400> 1657

agttaattca tgcctcc                                                    17

<210> 1658
<211> 17
<212> RNA
<213> Homo sapiens

<400> 1658

tcatgcctcc catggac                                                    17

<210> 1659
<211> 17
<212> RNA
<213> Homo sapiens

<400> 1659

atggacctat ggagagc                                                    17

<210> 1660
<211> 17
<212> RNA
<213> Homo sapiens

<400> 1660

caacaagttg atcttag                                                    17

<210> 1661
<211> 17
<212> RNA
<213> Homo sapiens

<400> 1661

aagttgatct tagttaa                                                    17

<210> 1662
<211> 17
<212> RNA
<213> Homo sapiens

<400> 1662

gttgatctta gttaagt                                                    17

<210> 1663
<211> 17
<212> RNA
<213> Homo sapiens

<400> 1663

ttgatcttag ttaagtc                                                    17

<210> 1664
<211> 17
<212> RNA
<213> Homo sapiens

<400> 1664

atcttagtta agtctcc                                                    17

<210> 1665
<211> 17
<212> RNA
<213> Homo sapiens

<400> 1665

tcttagttaa gtctccc                                                    17

<210> 1666
<211> 17
<212> RNA
<213> Homo sapiens

<400> 1666

agttaagtct ccctata                                                    17

<210> 1667
<211> 17
<212> RNA
<213> Homo sapiens

<400> 1667

ttaagtctcc ctatatg                                                    17

<210> 1668
<211> 17
<212> RNA
<213> Homo sapiens

<400> 1668

gtctccctat atgaggg                                                    17

<210> 1669
<211> 17
<212> RNA
<213> Homo sapiens

<400> 1669

ctccctatat gagggat                                                    17

<210> 1670
<211> 17
<212> RNA
<213> Homo sapiens

<400> 1670

tgagggataa gttcctg                                                    17

<210> 1671
<211> 17
<212> RNA
<213> Homo sapiens

<400> 1671

ggataagttc ctgattt                                                    17

<210> 1672
<211> 17
<212> RNA
<213> Homo sapiens

<400> 1672

gataagttcc tgatttt                                                    17

<210> 1673
<211> 17
<212> RNA
<213> Homo sapiens

<400> 1673

ttcctgattt ttgtttt                                                    17

<210> 1674
<211> 17
<212> RNA
<213> Homo sapiens

<400> 1674

tcctgatttt tgttttt                                                    17

<210> 1675
<211> 17
<212> RNA
<213> Homo sapiens

<400> 1675

cctgattttt gttttta                                                    17

<210> 1676
<211> 17
<212> RNA
<213> Homo sapiens

<400> 1676

ctgattttg tttttat                                                     17

<210> 1677
<211> 17
<212> RNA
<213> Homo sapiens

<400> 1677

atttttgttt ttatttt                                                    17

<210> 1678
<211> 17
<212> RNA
<213> Homo sapiens

<400> 1678

tttttgtttt tattttt                                                    17

<210> 1679
<211> 17
<212> RNA
<213> Homo sapiens

<400> 1679

ttttgttttt atttttg                                                    17

<210> 1680
<211> 17
<212> RNA
<213> Homo sapiens

<400> 1680

tttgttttta tttttgt                                                    17

<210> 1681
<211> 17
<212> RNA
<213> Homo sapiens

<400> 1681

ttgtttttat ttttgtg                                                    17

<210> 1682
<211> 17
<212> RNA
<213> Homo sapiens

<400> 1682

gtttttattt ttgtgtt                                                    17

<210> 1683
<211> 17
<212> RNA
<213> Homo sapiens

<400> 1683

tttttatttt tgtgtta                                                    17

<210> 1684
<211> 17
<212> RNA
<213> Homo sapiens

<400> 1684

ttttattttt gtgttac                                                    17

<210> 1685
<211> 17
<212> RNA
<213> Homo sapiens

<400> 1685

tttatttttg tgttaca                                                    17

<210> 1686

<211> 17
<212> RNA
<213> Homo sapiens

<400> 1686

ttttgtgtta caaaaga                                                    17

<210> 1687
<211> 17
<212> RNA
<213> Homo sapiens

<400> 1687

tttgtgttac aaaagaa                                                    17

<210> 1688
<211> 17
<212> RNA
<213> Homo sapiens

<400> 1688

aaagccctcc ctccctg                                                    17

<210> 1689
<211> 17
<212> RNA
<213> Homo sapiens

<400> 1689

ccctccctcc ctgaact                                                    17

<210> 1690
<211> 17
<212> RNA
<213> Homo sapiens

<400> 1690

cctgaacttg cagtaag                                                    17

<210> 1691
<211> 17
<212> RNA
<213> Homo sapiens

<400> 1691

cttgcagtaa ggtcagc 17

<210> 1692
<211> 17
<212> RNA
<213> Homo sapiens

<400> 1692

agtaaggtca gcttcag 17

<210> 1693
<211> 17
<212> RNA
<213> Homo sapiens

<400> 1693

ggtcagcttc aggacct 17

<210> 1694
<211> 17
<212> RNA
<213> Homo sapiens

<400> 1694

gtcagcttca ggacctg 17

<210> 1695
<211> 17
<212> RNA
<213> Homo sapiens

<400> 1695

ggacctgttc cagtggg 17

<210> 1696
<211> 17
<212> RNA
<213> Homo sapiens

<400> 1696

gacctgttcc agtgggc 17

<210> 1697
<211> 17

<212> RNA
<213> Homo sapiens

<400> 1697

ggcactgtac ttggatc 17

<210> 1698
<211> 17
<212> RNA
<213> Homo sapiens

<400> 1698

actgtacttg gatcttc 17

<210> 1699
<211> 17
<212> RNA
<213> Homo sapiens

<400> 1699

acttggatct tcccggc 17

<210> 1700
<211> 17
<212> RNA
<213> Homo sapiens

<400> 1700

ttggatcttc ccggcgt 17

<210> 1701
<211> 17
<212> RNA
<213> Homo sapiens

<400> 1701

tggatcttcc cggcgtg 17

<210> 1702
<211> 17
<212> RNA
<213> Homo sapiens

<400> 1702

gtgtgcctta cacaggg 17

<210> 1703
<211> 17
<212> RNA
<213> Homo sapiens

<400> 1703

tgtgccttac acagggg                                                    17

<210> 1704
<211> 17
<212> RNA
<213> Homo sapiens

<400> 1704

tgaactgttc actgtgg                                                    17

<210> 1705
<211> 17
<212> RNA
<213> Homo sapiens

<400> 1705

gaactgttca ctgtggt                                                    17

<210> 1706
<211> 17
<212> RNA
<213> Homo sapiens

<400> 1706

atgagggtaa atggtag                                                    17

<210> 1707
<211> 17
<212> RNA
<213> Homo sapiens

<400> 1707

taaatggtag ttgaaag                                                    17

<210> 1708
<211> 17
<212> RNA

<213> Homo sapiens

<400> 1708

atggtagttg aaaggag                                                    17

<210> 1709
<211> 17
<212> RNA
<213> Homo sapiens

<400> 1709

cctggtgttg catttag                                                    17

<210> 1710
<211> 17
<212> RNA
<213> Homo sapiens

<400> 1710

tgttgcattt agccctg                                                    17

<210> 1711
<211> 17
<212> RNA
<213> Homo sapiens

<400> 1711

gttgcattta gccctgg                                                    17

<210> 1712
<211> 17
<212> RNA
<213> Homo sapiens

<400> 1712

ttgcatttag ccctggg                                                    17

<210> 1713
<211> 17
<212> RNA
<213> Homo sapiens

<400> 1713

tgaacagtac ttgtgca                                                    17

<210> 1714
<211> 17
<212> RNA
<213> Homo sapiens

<400> 1714

acagtacttg tgcagga                                                    17

<210> 1715
<211> 17
<212> RNA
<213> Homo sapiens

<400> 1715

tgcaggattg ttgtggc                                                    17

<210> 1716
<211> 17
<212> RNA
<213> Homo sapiens

<400> 1716

aggattgttg tggctac                                                    17

<210> 1717
<211> 17
<212> RNA
<213> Homo sapiens

<400> 1717

ttgtggctac tagagaa                                                    17

<210> 1718
<211> 17
<212> RNA
<213> Homo sapiens

<400> 1718

tggctactag agaacaa                                                    17

<210> 1719
<211> 17
<212> RNA
<213> Homo sapiens

<400> 1719

gggaaagtag ggcagaa                                                    17

<210> 1720
<211> 17
<212> RNA
<213> Homo sapiens

<400> 1720

aactggatac agttctg                                                    17

<210> 1721
<211> 17
<212> RNA
<213> Homo sapiens

<400> 1721

gatacagttc tgagcac                                                    17

<210> 1722
<211> 17
<212> RNA
<213> Homo sapiens

<400> 1722

atacagttct gagcaca                                                    17

<210> 1723
<211> 17
<212> RNA
<213> Homo sapiens

<400> 1723

gccagacttg ctcaggt                                                    17

<210> 1724
<211> 17
<212> RNA
<213> Homo sapiens

<400> 1724

gacttgctca ggtggcc                                                    17

<210> 1725
<211> 17
<212> RNA
<213> Homo sapiens

<400> 1725

ctgcagctac ctaggaa 17

<210> 1726
<211> 17
<212> RNA
<213> Homo sapiens

<400> 1726

agctacctag gaacatt 17

<210> 1727
<211> 17
<212> RNA
<213> Homo sapiens

<400> 1727

aggaacattc cttgcag 17

<210> 1728
<211> 17
<212> RNA
<213> Homo sapiens

<400> 1728

ggaacattcc ttgcaga 17

<210> 1729
<211> 17
<212> RNA
<213> Homo sapiens

<400> 1729

acattccttg cagaccc 17

<210> 1730
<211> 17
<212> RNA
<213> Homo sapiens

<400> 1730

ccccgcattg cctttgg                                                      17

<210> 1731
<211> 17
<212> RNA
<213> Homo sapiens

<400> 1731

cattgccttt gggggtg                                                      17

<210> 1732
<211> 17
<212> RNA
<213> Homo sapiens

<400> 1732

attgcctttg ggggtgc                                                      17

<210> 1733
<211> 17
<212> RNA
<213> Homo sapiens

<400> 1733

cctgggatcc ctggggt                                                      17

<210> 1734
<211> 17
<212> RNA
<213> Homo sapiens

<400> 1734

cctggggtag tccagct                                                      17

<210> 1735
<211> 17
<212> RNA
<213> Homo sapiens

<400> 1735

ggggtagtcc agctctt                                                      17

<210> 1736
<211> 17
<212> RNA
<213> Homo sapiens

<400> 1736

gtccagctct tattcat                                                    17

<210> 1737
<211> 17
<212> RNA
<213> Homo sapiens

<400> 1737

ccagctctta ttcattt                                                    17

<210> 1738
<211> 17
<212> RNA
<213> Homo sapiens

<400> 1738

cagctcttat tcatttc                                                    17

<210> 1739
<211> 17
<212> RNA
<213> Homo sapiens

<400> 1739

gctcttattc atttccc                                                    17

<210> 1740
<211> 17
<212> RNA
<213> Homo sapiens

<400> 1740

ctcttattca tttccca                                                    17

<210> 1741
<211> 17
<212> RNA
<213> Homo sapiens

<400> 1741

ttattcattt cccagcg 17

<210> 1742
<211> 17
<212> RNA
<213> Homo sapiens

<400> 1742

tattcatttc ccagcgt 17

<210> 1743
<211> 17
<212> RNA
<213> Homo sapiens

<400> 1743

attcatttcc cagcgtg 17

<210> 1744
<211> 17
<212> RNA
<213> Homo sapiens

<400> 1744

gccctggttg gaagaag 17

<210> 1745
<211> 17
<212> RNA
<213> Homo sapiens

<400> 1745

gcagctgtca agttgta 17

<210> 1746
<211> 17
<212> RNA
<213> Homo sapiens

<400> 1746

tgtcaagttg tagacag 17

<210> 1747

<211> 17
<212> RNA
<213> Homo sapiens

<400> 1747

caagttgtag acagctg 17

<210> 1748
<211> 17
<212> RNA
<213> Homo sapiens

<400> 1748

agctgtgttc ctacaat 17

<210> 1749
<211> 17
<212> RNA
<213> Homo sapiens

<400> 1749

gctgtgttcc tacaatt 17

<210> 1750
<211> 17
<212> RNA
<213> Homo sapiens

<400> 1750

gtgttcctac aattggc 17

<210> 1751
<211> 17
<212> RNA
<213> Homo sapiens

<400> 1751

cctacaattg gcccagc 17

<210> 1752
<211> 17
<212> RNA
<213> Homo sapiens

<400> 1752

gggaccgttg ctgtcac                                                    17

<210> 1753
<211> 17
<212> RNA
<213> Homo sapiens

<400> 1753

gttgctgtca ctactca                                                    17

<210> 1754
<211> 17
<212> RNA
<213> Homo sapiens

<400> 1754

ctgtcactac tcaggct                                                    17

<210> 1755
<211> 17
<212> RNA
<213> Homo sapiens

<400> 1755

tcactactca ggctgac                                                    17

<210> 1756
<211> 17
<212> RNA
<213> Homo sapiens

<400> 1756

ggcctggtca gattacg                                                    17

<210> 1757
<211> 17
<212> RNA
<213> Homo sapiens

<400> 1757

ggtcagatta cgtatgc                                                    17

<210> 1758
<211> 17

<212> RNA
<213> Homo sapiens

<400> 1758

gtcagattac gtatgcc                                                    17

<210> 1759
<211> 17
<212> RNA
<213> Homo sapiens

<400> 1759

gattacgtat gcccttg                                                    17

<210> 1760
<211> 17
<212> RNA
<213> Homo sapiens

<400> 1760

tatgcccttg gtggttt                                                    17

<210> 1761
<211> 17
<212> RNA
<213> Homo sapiens

<400> 1761

ttggtggttt agagata                                                    17

<210> 1762
<211> 17
<212> RNA
<213> Homo sapiens

<400> 1762

tggtggttta gagataa                                                    17

<210> 1763
<211> 17
<212> RNA
<213> Homo sapiens

<400> 1763

ggtggtttag agataat                                                    17

<210> 1764
<211> 17
<212> RNA
<213> Homo sapiens

<400> 1764

ttagagataa tccaaaa 17

<210> 1765
<211> 17
<212> RNA
<213> Homo sapiens

<400> 1765

gagataatcc aaaatca 17

<210> 1766
<211> 17
<212> RNA
<213> Homo sapiens

<400> 1766

tccaaaatca gggtttg 17

<210> 1767
<211> 17
<212> RNA
<213> Homo sapiens

<400> 1767

atcagggttt ggtttgg 17

<210> 1768
<211> 17
<212> RNA
<213> Homo sapiens

<400> 1768

tcagggtttg gtttggg 17

<210> 1769
<211> 17
<212> RNA

<213> Homo sapiens

<400> 1769

ggtttggttt ggggaag                                                      17

<210> 1770
<211> 17
<212> RNA
<213> Homo sapiens

<400> 1770

gtttggtttg gggaaga                                                      17

<210> 1771
<211> 17
<212> RNA
<213> Homo sapiens

<400> 1771
   aagaaaatcc tccccct                                                      17

<210> 1772
<211> 17
<212> RNA
<213> Homo sapiens

<400> 1772

aaaatcctcc cccttcc                                                      17

<210> 1773
<211> 17
<212> RNA
<213> Homo sapiens

<400> 1773
   ctcccccttc ctccccc                                                      17

<210> 1774
<211> 17
<212> RNA
<213> Homo sapiens

<400> 1774

tcccccttcc tcccccg                                                      17

<210> 1775
<211> 17
<212> RNA
<213> Homo sapiens

<400> 1775

cccttcctcc cccgccc                                                    17

<210> 1776
<211> 17
<212> RNA
<213> Homo sapiens

<400> 1776

cgccccgttc cctaccg                                                    17

<210> 1777
<211> 17
<212> RNA
<213> Homo sapiens

<400> 1777

gccccgttcc ctaccgc                                                    17

<210> 1778
<211> 17
<212> RNA
<213> Homo sapiens

<400> 1778

cgttccctac cgcctcc                                                    17

<210> 1779
<211> 17
<212> RNA
<213> Homo sapiens

<400> 1779

taccgcctcc actcctg                                                    17

<210> 1780
<211> 17
<212> RNA
<213> Homo sapiens

<400> 1780

cctccactcc tgccagc                                                    17

<210> 1781
<211> 17
<212> RNA
<213> Homo sapiens

<400> 1781

tgccagctca tttcctt                                                    17

<210> 1782
<211> 17
<212> RNA
<213> Homo sapiens

<400> 1782

cagctcattt ccttcaa                                                    17

<210> 1783
<211> 17
<212> RNA
<213> Homo sapiens

<400> 1783

agctcatttc cttcaat                                                    17

<210> 1784
<211> 17
<212> RNA
<213> Homo sapiens

<400> 1784

gctcatttcc ttcaatt                                                    17

<210> 1785
<211> 17
<212> RNA
<213> Homo sapiens

<400> 1785

catttccttc aatttcc                                                    17

<210> 1786
<211> 17
<212> RNA
<213> Homo sapiens

<400> 1786

atttccttca atttcct                                                    17

<210> 1787
<211> 17
<212> RNA
<213> Homo sapiens

<400> 1787

ccttcaattt cctttga                                                    17

<210> 1788
<211> 17
<212> RNA
<213> Homo sapiens

<400> 1788

cttcaatttc ctttgac                                                    17

<210> 1789
<211> 17
<212> RNA
<213> Homo sapiens

<400> 1789

ttcaatttcc tttgacc                                                    17

<210> 1790
<211> 17
<212> RNA
<213> Homo sapiens

<400> 1790

aatttccttt gacctat                                                    17

<210> 1791
<211> 17
<212> RNA
<213> Homo sapiens

<400> 1791

atttcctttg acctata                                                    17

<210> 1792
<211> 17
<212> RNA
<213> Homo sapiens

<400> 1792

tttgacctat aggctaa                                                    17

<210> 1793
<211> 17
<212> RNA
<213> Homo sapiens

<400> 1793

tgacctatag gctaaaa                                                    17

<210> 1794
<211> 17
<212> RNA
<213> Homo sapiens

<400> 1794

tataggctaa aaaagaa                                                    17

<210> 1795
<211> 17
<212> RNA
<213> Homo sapiens

<400> 1795

gaaaggctca ttccagc                                                    17

<210> 1796
<211> 17
<212> RNA
<213> Homo sapiens

<400> 1796

aggctcattc cagccac                                                    17

<210> 1797
<211> 17
<212> RNA
<213> Homo sapiens

<400> 1797

ggctcattcc agccaca 17

<210> 1798
<211> 17
<212> RNA
<213> Homo sapiens

<400> 1798

ggcagccttc cctgggc 17

<210> 1799
<211> 17
<212> RNA
<213> Homo sapiens

<400> 1799

gcagccttcc ctgggcc 17

<210> 1800
<211> 17
<212> RNA
<213> Homo sapiens

<400> 1800

ctgggccttt gcttctc 17

1801:

<210> 1801
<211> 17
<212> RNA
<213> Homo sapiens

<400> 1801

tgggcctttg cttctct 17

<210> 1802
<211> 17
<212> RNA
<213> Homo sapiens

<400> 1802

cctttgcttc tctagca                                                    17

<210> 1803
<211> 17
<212> RNA
<213> Homo sapiens

<400> 1803

ctttgcttct ctagcac                                                    17

<210> 1804
<211> 17
<212> RNA
<213> Homo sapiens

<400> 1804

ttgcttctct agcacaa                                                    17

<210> 1805
<211> 17
<212> RNA
<213> Homo sapiens

<400> 1805

gcttctctag cacaatt                                                    17

<210> 1806
<211> 17
<212> RNA
<213> Homo sapiens

<400> 1806

agcacaatta tgggtta                                                    17

<210> 1807
<211> 17
<212> RNA
<213> Homo sapiens

<400> 1807

gcacaattat gggttac                                                    17

<210> 1808
<211> 17
<212> RNA
<213> Homo sapiens

<400> 1808

ttatgggtta cttcctt 17

<210> 1809
<211> 17
<212> RNA
<213> Homo sapiens

<400> 1809

tatgggttac ttccttt 17

<210> 1810
<211> 17
<212> RNA
<213> Homo sapiens

<400> 1810

gggttacttc ctttttc 17

<210> 1811
<211> 17
<212> RNA
<213> Homo sapiens

<400> 1811

ggttacttcc tttttct 17

<210> 1812
<211> 17
<212> RNA
<213> Homo sapiens

<400> 1812

tacttccttt ttcttaa 17

<210> 1813
<211> 17
<212> RNA
<213> Homo sapiens

<400> 1813

acttcctttt tcttaac                                                    17

<210> 1814
<211> 17
<212> RNA
<213> Homo sapiens

<400> 1814

cttccttttt cttaaca                                                    17

<210> 1815
<211> 17
<212> RNA
<213> Homo sapiens

<400> 1815

ttcctttttc ttaacaa                                                    17

<210> 1816
<211> 17
<212> RNA
<213> Homo sapiens

<400> 1816

tcctttttct taacaaa                                                    17

<210> 1817
<211> 17
<212> RNA
<213> Homo sapiens

<400> 1817

ctttttctta acaaaaa                                                    17

<210> 1818
<211> 17
<212> RNA
<213> Homo sapiens

<400> 1818

tttttcttaa caaaaaa                                                    17

<210> 1819
<211> 17
<212> RNA
<213> Homo sapiens

<400> 1819

aagaatgttt gatttcc                                                    17

<210> 1820
<211> 17
<212> RNA
<213> Homo sapiens

<400> 1820

agaatgtttg atttcct                                                    17

<210> 1821
<211> 17
<212> RNA
<213> Homo sapiens

<400> 1821

tgtttgattt cctctgg                                                    17

<210> 1822
<211> 17
<212> RNA
<213> Homo sapiens

<400> 1822

gtttgatttc ctctggg                                                    17

<210> 1823
<211> 17
<212> RNA
<213> Homo sapiens

<400> 1823

tttgatttcc tctgggt                                                    17

<210> 1824
<211> 17
<212> RNA
<213> Homo sapiens

<400> 1824

gatttcctct gggtgac                                                    17

<210> 1825
<211> 17
<212> RNA
<213> Homo sapiens

<400> 1825

ggtgacctta ttgtctg                                                    17

<210> 1826
<211> 17
<212> RNA
<213> Homo sapiens

<400> 1826

gtgaccttat tgtctgt                                                    17

<210> 1827
<211> 17
<212> RNA
<213> Homo sapiens

<400> 1827

gaccttattg tctgtaa                                                    17

<210> 1828
<211> 17
<212> RNA
<213> Homo sapiens

<400> 1828

cttattgtct gtaattg                                                    17

<210> 1829
<211> 17
<212> RNA
<213> Homo sapiens

<400> 1829

ttgtctgtaa ttgaaac                                                    17

<210> 1830

<211> 17
<212> RNA
<213> Homo sapiens

<400> 1830

tctgtaattg aaaccct                                                    17

<210> 1831
<211> 17
<212> RNA
<213> Homo sapiens

<400> 1831

gaaaccctat tgagagg                                                    17

<210> 1832
<211> 17
<212> RNA
<213> Homo sapiens

<400> 1832

aaccctattg agaggtg                                                    17

<210> 1833
<211> 17
<212> RNA
<213> Homo sapiens

<400> 1833

ggtgatgtct gtgttag                                                    17

<210> 1834
<211> 17
<212> RNA
<213> Homo sapiens

<400> 1834

gtctgtgtta gccaatg                                                    17

<210> 1835
<211> 17
<212> RNA
<213> Homo sapiens

<400> 1835

tctgtgttag ccaatga                                                    17

<210> 1836
<211> 17
<212> RNA
<213> Homo sapiens

<400> 1836

acccaggtag ctgctcg                                                    17

<210> 1837
<211> 17
<212> RNA
<213> Homo sapiens

<400> 1837

tagctgctcg ggcttct                                                    17

<210> 1838
<211> 17
<212> RNA
<213> Homo sapiens

<400> 1838

ctcgggcttc tcttggt                                                    17

<210> 1839
<211> 17
<212> RNA
<213> Homo sapiens

<400> 1839

tcgggcttct cttggta                                                    17

<210> 1840
<211> 17
<212> RNA
<213> Homo sapiens

<400> 1840

gggcttctct tggtatg                                                    17

<210> 1841
<211> 17

<212> RNA
<213> Homo sapiens

<400> 1841

gcttctcttg gtatgtc                                                    17

<210> 1842
<211> 17
<212> RNA
<213> Homo sapiens

<400> 1842

ctcttggtat gtcttgt                                                    17

<210> 1843
<211> 17
<212> RNA
<213> Homo sapiens

<400> 1843

tggtatgtct tgtttgg                                                    17

<210> 1844
<211> 17
<212> RNA
<213> Homo sapiens

<400> 1844

gtatgtcttg tttggaa                                                    17

<210> 1845
<211> 17
<212> RNA
<213> Homo sapiens

<400> 1845

tgtcttgttt ggaaaag                                                    17

<210> 1846
<211> 17
<212> RNA
<213> Homo sapiens

<400> 1846

gtcttgtttg gaaaagt                                                    17

<210> 1847
<211> 17
<212> RNA
<213> Homo sapiens

<400> 1847

aagtggattt cattcat 17

<210> 1848
<211> 17
<212> RNA
<213> Homo sapiens

<400> 1848

agtggatttc attcatt 17

<210> 1849
<211> 17
<212> RNA
<213> Homo sapiens

<400> 1849

gtggatttca ttcattt 17

<210> 1850
<211> 17
<212> RNA
<213> Homo sapiens

<400> 1850

gatttcattc atttctg 17

<210> 1851
<211> 17
<212> RNA
<213> Homo sapiens

<400> 1851

atttcattca tttctga 17

<210> 1852
<211> 17
<212> RNA

<213> Homo sapiens

<400> 1852

tcattcattt ctgattg 17

<210> 1853
<211> 17
<212> RNA
<213> Homo sapiens

<400> 1853

cattcatttc tgattgt 17

<210> 1854
<211> 17
<212> RNA
<213> Homo sapiens

<400> 1854

attcatttct gattgtc 17

<210> 1855
<211> 17
<212> RNA
<213> Homo sapiens

<400> 1855

tttctgattg tccagtt 17

<210> 1856
<211> 17
<212> RNA
<213> Homo sapiens

<400> 1856

ctgattgtcc agttaag 17

<210> 1857
<211> 17
<212> RNA
<213> Homo sapiens

<400> 1857

tgtccagtta agtgatc 17

<210> 1858
<211> 17
<212> RNA
<213> Homo sapiens

<400> 1858

gtccagttaa gtgatca                                                    17

<210> 1859
<211> 17
<212> RNA
<213> Homo sapiens

<400> 1859

taagtgatca ccaaagg                                                    17

<210> 1860
<211> 17
<212> RNA
<213> Homo sapiens

<400> 1860

ctgagaatct gggaggg                                                    17

<210> 1861
<211> 17
<212> RNA
<213> Homo sapiens

<400> 1861

aaaaaagttt ttatgtg                                                    17

<210> 1862
<211> 17
<212> RNA
<213> Homo sapiens

<400> 1862

aaaaagtttt tatgtgc                                                    17

<210> 1863
<211> 17
<212> RNA
<213> Homo sapiens

<400> 1863

aaaagttttt atgtgca                                                    17

<210> 1864
<211> 17
<212> RNA
<213> Homo sapiens

<400> 1864

aaagttttta tgtgcac                                                    17

<210> 1865
<211> 17
<212> RNA
<213> Homo sapiens

<400> 1865

aagtttttat gtgcact                                                    17

<210> 1866
<211> 17
<212> RNA
<213> Homo sapiens

<400> 1866

tgtgcactta aatttgg                                                    17

<210> 1867
<211> 17
<212> RNA
<213> Homo sapiens

<400> 1867

gtgcacttaa atttggg                                                    17

<210> 1868
<211> 17
<212> RNA
<213> Homo sapiens

<400> 1868

acttaaattt ggggaca                                                    17

<210> 1869
<211> 17
<212> RNA
<213> Homo sapiens

<400> 1869

cttaaatttg gggacaa                                                    17

<210> 1870
<211> 17
<212> RNA
<213> Homo sapiens

<400> 1870

gggacaattt tatgtat                                                    17

<210> 1871
<211> 17
<212> RNA
<213> Homo sapiens

<400> 1871

ggacaatttt atgtatc                                                    17

<210> 1872
<211> 17
<212> RNA
<213> Homo sapiens

<400> 1872

gacaatttta tgtatct                                                    17

<210> 1873
<211> 17
<212> RNA
<213> Homo sapiens

<400> 1873

acaattttat gtatctg                                                    17

<210> 1874
<211> 17
<212> RNA
<213> Homo sapiens

<400> 1874

ttttatgtat ctgtgtt                                                    17

<210> 1875
<211> 17
<212> RNA
<213> Homo sapiens

<400> 1875

ttatgtatct gtgttaa                                                    17

<210> 1876
<211> 17
<212> RNA
<213> Homo sapiens

<400> 1876

atctgtgtta aggatat                                                    17

<210> 1877
<211> 17
<212> RNA
<213> Homo sapiens

<400> 1877

tctgtgttaa ggatatg                                                    17

<210> 1878
<211> 17
<212> RNA
<213> Homo sapiens

<400> 1878

ttaaggatat gcttaag                                                    17

<210> 1879
<211> 17
<212> RNA
<213> Homo sapiens

<400> 1879

gatatgctta agaacat                                                    17

<210> 1880
<211> 17
<212> RNA
<213> Homo sapiens

<400> 1880

atatgcttaa gaacata 17

<210> 1881
<211> 17
<212> RNA
<213> Homo sapiens

<400> 1881

aagaacataa ttctttt 17

<210> 1882
<211> 17
<212> RNA
<213> Homo sapiens

<400> 1882

aacataattc ttttgtt 17

<210> 1883
<211> 17
<212> RNA
<213> Homo sapiens

<400> 1883

acataattct tttgttg 17

<210> 1884
<211> 17
<212> RNA
<213> Homo sapiens

<400> 1884

ataattcttt tgttgct 17

<210> 1885
<211> 17
<212> RNA
<213> Homo sapiens

<400> 1885

taattctttt gttgctg                                                    17

<210> 1886
<211> 17
<212> RNA
<213> Homo sapiens

<400> 1886

aattcttttg ttgctgt                                                    17

<210> 1887
<211> 17
<212> RNA
<213> Homo sapiens

<400> 1887

tcttttgttg ctgtttg                                                    17

<210> 1888
<211> 17
<212> RNA
<213> Homo sapiens

<400> 1888

gttgctgttt gtttaag                                                    17

<210> 1889
<211> 17
<212> RNA
<213> Homo sapiens

<400> 1889

ttgctgtttg tttaaga                                                    17

<210> 1890
<211> 17
<212> RNA
<213> Homo sapiens

<400> 1890

ctgtttgttt aagaagc                                                    17

<210> 1891

<211> 17
<212> RNA
<213> Homo sapiens

<400> 1891

tgtttgttta agaagca                                                    17

<210> 1892
<211> 17
<212> RNA
<213> Homo sapiens

<400> 1892

gtttgtttaa gaagcac                                                    17

<210> 1893
<211> 17
<212> RNA
<213> Homo sapiens

<400> 1893

aagcacctta gtttgtt                                                    17

<210> 1894
<211> 17
<212> RNA
<213> Homo sapiens

<400> 1894

agcaccttag tttgttt                                                    17

<210> 1895
<211> 17
<212> RNA
<213> Homo sapiens

<400> 1895

accttagttt gtttaag                                                    17

<210> 1896
<211> 17
<212> RNA
<213> Homo sapiens

<400> 1896

ccttagtttg tttaaga                                                    17

<210> 1897
<211> 17
<212> RNA
<213> Homo sapiens

<400> 1897

tagtttgttt aagaagc                                                    17

<210> 1898
<211> 17
<212> RNA
<213> Homo sapiens

<400> 1898

agtttgttta agaagca                                                    17

<210> 1899
<211> 17
<212> RNA
<213> Homo sapiens

<400> 1899

gtttgtttaa gaagcac                                                    17

<210> 1900
<211> 17
<212> RNA
<213> Homo sapiens

<400> 1900

aagcacctta tatagta                                                    17

<210> 1901
<211> 17
<212> RNA
<213> Homo sapiens

<400> 1901

agcaccttat atagtat                                                    17

<210> 1902
<211> 17

<212> RNA
<213> Homo sapiens

<400> 1902

caccttatat agtataa                                                    17

<210> 1903
<211> 17
<212> RNA
<213> Homo sapiens

<400> 1903

ccttatatag tataata                                                    17

<210> 1904
<211> 17
<212> RNA
<213> Homo sapiens

<400> 1904

tatatagtat aatatat                                                    17

<210> 1905
<211> 17
<212> RNA
<213> Homo sapiens

<400> 1905

tatagtataa tatatat                                                    17

<210> 1906
<211> 17
<212> RNA
<213> Homo sapiens

<400> 1906

agtataatat atatttt                                                    17

<210> 1907
<211> 17
<212> RNA
<213> Homo sapiens

<400> 1907

tataatatat atttttt                                                    17

<210> 1908
<211> 17
<212> RNA
<213> Homo sapiens

<400> 1908

taatatatat tttttg                                                    17

<210> 1909
<211> 17
<212> RNA
<213> Homo sapiens

<400> 1909

atatatattt ttttgaa                                                   17

<210> 1910
<211> 17
<212> RNA
<213> Homo sapiens

<400> 1910

tatatatttt tttgaaa                                                   17

<210> 1911
<211> 17
<212> RNA
<213> Homo sapiens

<400> 1911

atatattttt ttgaaat                                                   17

<210> 1912
<211> 17
<212> RNA
<213> Homo sapiens

<400> 1912

tatatttttt tgaaatt                                                   17

<210> 1913
<211> 17
<212> RNA

<213> Homo sapiens

<400> 1913

atattttttt gaaatta                                                    17

<210> 1914
<211> 17
<212> RNA
<213> Homo sapiens

<400> 1914

tatttttttg aaattac                                                    17

<210> 1915
<211> 17
<212> RNA
<213> Homo sapiens

<400> 1915

tttgaaatta cattgct                                                    17

<210> 1916
<211> 17
<212> RNA
<213> Homo sapiens

<400> 1916

ttgaaattac attgctt                                                    17

<210> 1917
<211> 17
<212> RNA
<213> Homo sapiens

<400> 1917

aattacattg cttgttt                                                    17

<210> 1918
<211> 17
<212> RNA
<213> Homo sapiens

<400> 1918

acattgcttg tttatca                                                    17

<210> 1919
<211> 17
<212> RNA
<213> Homo sapiens

<400> 1919

ttgcttgttt atcagac 17

<210> 1920
<211> 17
<212> RNA
<213> Homo sapiens

<400> 1920

tgcttgttta tcagaca 17

<210> 1921
<211> 17
<212> RNA
<213> Homo sapiens

<400> 1921

gcttgtttat cagacaa 17

<210> 1922
<211> 17
<212> RNA
<213> Homo sapiens

<400> 1922

ttgtttatca gacaatt 17

<210> 1923
<211> 17
<212> RNA
<213> Homo sapiens

<400> 1923

cagacaattg aatgtag 17

<210> 1924
<211> 17
<212> RNA
<213> Homo sapiens

<400> 1924

ttgaatgtag taattct                                                    17

<210> 1925
<211> 17
<212> RNA
<213> Homo sapiens

<400> 1925

aatgtagtaa ttctgtt                                                    17

<210> 1926
<211> 17
<212> RNA
<213> Homo sapiens

<400> 1926

gtagtaattc tgttctg                                                    17

<210> 1927
<211> 17
<212> RNA
<213> Homo sapiens

<400> 1927

tagtaattct gttctgg                                                    17

<210> 1928
<211> 17
<212> RNA
<213> Homo sapiens

<400> 1928

aattctgttc tggattt                                                    17

<210> 1929
<211> 17
<212> RNA
<213> Homo sapiens

<400> 1929

attctgttct ggattta                                                    17

<210> 1930
<211> 17
<212> RNA
<213> Homo sapiens

<400> 1930

ttctggattt aatttga 17

<210> 1931
<211> 17
<212> RNA
<213> Homo sapiens

<400> 1931

tctggattta atttgac 17

<210> 1932
<211> 17
<212> RNA
<213> Homo sapiens

<400> 1932

ctggatttaa tttgact 17

<210> 1933
<211> 17
<212> RNA
<213> Homo sapiens

<400> 1933

gatttaattt gactggg 17

<210> 1934
<211> 17
<212> RNA
<213> Homo sapiens

<400> 1934

atttaatttg actgggt 17

<210> 1935
<211> 17
<212> RNA
<213> Homo sapiens

<400> 1935

gactgggtta acatgca                                                    17

<210> 1936
<211> 17
<212> RNA
<213> Homo sapiens

<400> 1936

actgggttaa catgcaa                                                    17

<210> 1937
<211> 17
<212> RNA
<213> Homo sapiens

<400> 1937

ggaaaaatat ttagttt                                                    17

<210> 1938
<211> 17
<212> RNA
<213> Homo sapiens

<400> 1938

aaaaatattt agttttt                                                    17

<210> 1939
<211> 17
<212> RNA
<213> Homo sapiens

<400> 1939

aaaatattta gtttttt                                                    17

<210> 1940
<211> 17
<212> RNA
<213> Homo sapiens

<400> 1940

aaatatttag ttttttt                                                    17

<210> 1941
<211> 17
<212> RNA
<213> Homo sapiens

<400> 1941

tatttagttt ttttttt                                                    17

<210> 1942
<211> 17
<212> RNA
<213> Homo sapiens

<400> 1942

atttagtttt ttttttt                                                    17

<210> 1943
<211> 17
<212> RNA
<213> Homo sapiens

<400> 1943

tttagttttt ttttttt                                                    17

<210> 1944
<211> 17
<212> RNA
<213> Homo sapiens

<400> 1944

ttagtttttt ttttttt                                                    17

<210> 1945
<211> 17
<212> RNA
<213> Homo sapiens

<400> 1945

tagttttttt ttttttt                                                    17

<210> 1946
<211> 17
<212> RNA
<213> Homo sapiens

<400> 1946

agtttttttt ttttttt 17

<210> 1947
<211> 17
<212> RNA
<213> Homo sapiens

<400> 1947

gttttttttt ttttttt 17

<210> 1948
<211> 17
<212> RNA
<213> Homo sapiens

<400> 1948

tttttttttt ttttttt 17

<210> 1949
<211> 17
<212> RNA
<213> Homo sapiens

<400> 1949

tttttttttt ttttttg 17

<210> 1950
<211> 17
<212> RNA
<213> Homo sapiens

<400> 1950

tttttttttt tttttgt 17

<210> 1951
<211> 17
<212> RNA
<213> Homo sapiens

<400> 1951

tttttttttt ttttgta 17

<210> 1952

<211> 17
<212> RNA
<213> Homo sapiens

<400> 1952

tttttttttt tttgtat                                                    17

<210> 1953
<211> 17
<212> RNA
<213> Homo sapiens

<400> 1953

tttttttttt ttgtata                                                    17

<210> 1954
<211> 17
<212> RNA
<213> Homo sapiens

<400> 1954

tttttttttt tgtatac                                                    17

<210> 1955
<211> 17
<212> RNA
<213> Homo sapiens

<400> 1955

tttttttttt gtatact                                                    17

<210> 1956
<211> 17
<212> RNA
<213> Homo sapiens

<400> 1956

tttttttttg tatactt                                                    17

<210> 1957
<211> 17
<212> RNA
<213> Homo sapiens

<400> 1957

ttttttgtat acttttc                                                    17

<210> 1958
<211> 17
<212> RNA
<213> Homo sapiens

<400> 1958

ttttgtatac ttttcaa                                                    17

<210> 1959
<211> 17
<212> RNA
<213> Homo sapiens

<400> 1959

tgtatacttt tcaagct                                                    17

<210> 1960
<211> 17
<212> RNA
<213> Homo sapiens

<400> 1960

gtatactttt caagcta                                                    17

<210> 1961
<211> 17
<212> RNA
<213> Homo sapiens

<400> 1961

tatacttttc aagctac                                                    17

<210> 1962
<211> 17
<212> RNA
<213> Homo sapiens

<400> 1962

atacttttca agctacc                                                    17

<210> 1963
<211> 17

<212> RNA
<213> Homo sapiens

<400> 1963

ttcaagctac cttgtca 17

<210> 1964
<211> 17
<212> RNA
<213> Homo sapiens

<400> 1964

agctaccttg tcatgta 17

<210> 1965
<211> 17
<212> RNA
<213> Homo sapiens

<400> 1965

taccttgtca tgtatac 17

<210> 1966
<211> 17
<212> RNA
<213> Homo sapiens

<400> 1966

tgtcatgtat acagtca 17

<210> 1967
<211> 17
<212> RNA
<213> Homo sapiens

<400> 1967

tcatgtatac agtcatt 17

<210> 1968
<211> 17
<212> RNA
<213> Homo sapiens

<400> 1968

tatacagtca tttatgc 17

<210> 1969
<211> 17
<212> RNA
<213> Homo sapiens

<400> 1969

acagtcattt atgccta                                                    17

<210> 1970
<211> 17
<212> RNA
<213> Homo sapiens

<400> 1970

cagtcattta tgcctaa                                                    17

<210> 1971
<211> 17
<212> RNA
<213> Homo sapiens

<400> 1971

agtcatttat gcctaaa                                                    17

<210> 1972
<211> 17
<212> RNA
<213> Homo sapiens

<400> 1972

ttatgcctaa agcctgg                                                    17

<210> 1973
<211> 17
<212> RNA
<213> Homo sapiens

<400> 1973

ctggtgatta ttcattt                                                    17

<210> 1974
<211> 17
<212> RNA

<213> Homo sapiens

<400> 1974

tggtgattat tcattta                                                    17

<210> 1975
<211> 17
<212> RNA
<213> Homo sapiens

<400> 1975

gtgattattc atttaaa                                                    17

<210> 1976
<211> 17
<212> RNA
<213> Homo sapiens

<400> 1976

tgattattca tttaaat                                                    17

<210> 1977
<211> 17
<212> RNA
<213> Homo sapiens

<400> 1977

ttattcattt aaatgaa                                                    17

<210> 1978
<211> 17
<212> RNA
<213> Homo sapiens

<400> 1978

tattcattta aatgaag                                                    17

<210> 1979
<211> 17
<212> RNA
<213> Homo sapiens

<400> 1979

attcatttaa atgaaga                                                    17

<210> 1980
<211> 17
<212> RNA
<213> Homo sapiens

<400> 1980

atgaagatca catttca 17

<210> 1981
<211> 17
<212> RNA
<213> Homo sapiens

<400> 1981

gatcacattt catatca 17

<210> 1982
<211> 17
<212> RNA
<213> Homo sapiens

<400> 1982

atcacatttc atatcaa 17

<210> 1983
<211> 17
<212> RNA
<213> Homo sapiens

<400> 1983

tcacatttca tatcaac 17

<210> 1984
<211> 17
<212> RNA
<213> Homo sapiens

<400> 1984

catttcatat caacttt 17

<210> 1985
<211> 17
<212> RNA
<213> Homo sapiens

<400> 1985

tttcatatca acttttg                                                    17

<210> 1986
<211> 17
<212> RNA
<213> Homo sapiens

<400> 1986

tatcaacttt tgtatcc                                                    17

<210> 1987
<211> 17
<212> RNA
<213> Homo sapiens

<400> 1987

atcaactttt gtatcca                                                    17

<210> 1988
<211> 17
<212> RNA
<213> Homo sapiens

<400> 1988

tcaacttttg tatccac                                                    17

<210> 1989
<211> 17
<212> RNA
<213> Homo sapiens

<400> 1989

acttttgtat ccacagt                                                    17

<210> 1990
<211> 17
<212> RNA
<213> Homo sapiens

<400> 1990

ttttgtatcc acagtag                                                    17

<210> 1991
<211> 17
<212> RNA
<213> Homo sapiens

<400> 1991

tccacagtag acaaaat                                                    17

<210> 1992
<211> 17
<212> RNA
<213> Homo sapiens

<400> 1992

gacaaaatag cactaat                                                    17

<210> 1993
<211> 17
<212> RNA
<213> Homo sapiens

<400> 1993

atagcactaa tccagat                                                    17

<210> 1994
<211> 17
<212> RNA
<213> Homo sapiens

<400> 1994

gcactaatcc agatgcc                                                    17

<210> 1995
<211> 17
<212> RNA
<213> Homo sapiens

<400> 1995

agatgcctat tgttgga                                                    17

<210> 1996
<211> 17
<212> RNA
<213> Homo sapiens

<400> 1996

atgcctattg ttggata                                                    17

<210> 1997
<211> 17
<212> RNA
<213> Homo sapiens

<400> 1997

cctattgttg gatattg                                                    17

<210> 1998
<211> 17
<212> RNA
<213> Homo sapiens

<400> 1998

tgttggatat tgaatga                                                    17

<210> 1999
<211> 17
<212> RNA
<213> Homo sapiens

<400> 1999

ttggatattg aatgaca                                                    17

<210> 2000
<211> 17
<212> RNA
<213> Homo sapiens

<400> 2000

cagacaatct tatgtag                                                    17

<210> 2001
<211> 17
<212> RNA
<213> Homo sapiens

<400> 2001

gacaatctta tgtagca                                                    17

<210> 2002
<211> 17
<212> RNA
<213> Homo sapiens

<400> 2002

acaatcttat gtagcaa 17

<210> 2003
<211> 17
<212> RNA
<213> Homo sapiens

<400> 2003

tcttatgtag caaagat 17

<210> 2004
<211> 17
<212> RNA
<213> Homo sapiens

<400> 2004

gcaaagatta tgcctga 17

<210> 2005
<211> 17
<212> RNA
<213> Homo sapiens

<400> 2005

caaagattat gcctgaa 17

<210> 2006
<211> 17
<212> RNA
<213> Homo sapiens

<400> 2006

aggaaaatta ttcaggg 17

<210> 2007
<211> 17
<212> RNA
<213> Homo sapiens

<400> 2007

ggaaaattat tcagggc                                                    17

<210> 2008
<211> 17
<212> RNA
<213> Homo sapiens

<400> 2008

aaaattattc agggcag                                                    17

<210> 2009
<211> 17
<212> RNA
<213> Homo sapiens

<400> 2009

aaattattca gggcagc                                                    17

<210> 2010
<211> 17
<212> RNA
<213> Homo sapiens

<400> 2010

gggcagctaa ttttgct                                                    17

<210> 2011
<211> 17
<212> RNA
<213> Homo sapiens

<400> 2011

cagctaattt tgctttt                                                    17

<210> 2012
<211> 17
<212> RNA
<213> Homo sapiens

<400> 2012

agctaatttt gctttta                                                    17

<210> 2013

<211> 17
<212> RNA
<213> Homo sapiens

<400> 2013

gctaattttg cttttac                                                    17

<210> 2014
<211> 17
<212> RNA
<213> Homo sapiens

<400> 2014

attttgcttt taccaaa                                                    17

<210> 2015
<211> 17
<212> RNA
<213> Homo sapiens

<400> 2015

ttttgctttt accaaaa                                                    17

<210> 2016
<211> 17
<212> RNA
<213> Homo sapiens

<400> 2016

tttgctttta ccaaaat                                                    17

<210> 2017
<211> 17
<212> RNA
<213> Homo sapiens

<400> 2017

ttgcttttac caaaata                                                    17

<210> 2018
<211> 17
<212> RNA
<213> Homo sapiens

<400> 2018

accaaaatat cagtagt                                                    17

<210> 2019
<211> 17
<212> RNA
<213> Homo sapiens

<400> 2019

caaaatatca gtagtaa                                                    17

<210> 2020
<211> 17
<212> RNA
<213> Homo sapiens

<400> 2020

atatcagtag taatatt                                                    17

<210> 2021
<211> 17
<212> RNA
<213> Homo sapiens

<400> 2021

tcagtagtaa tattttt                                                    17

<210> 2022
<211> 17
<212> RNA
<213> Homo sapiens

<400> 2022

gtagtaatat ttttgga                                                    17

<210> 2023
<211> 17
<212> RNA
<213> Homo sapiens

<400> 2023

agtaatattt ttggaca                                                    17

<210> 2024
<211> 17
<212> RNA

<213> Homo sapiens

<400> 2024

gtaatatttt tggacag                                                    17

<210> 2025
<211> 17
<212> RNA
<213> Homo sapiens

<400> 2025

taatattttt ggacagt                                                    17

<210> 2026
<211> 17
<212> RNA
<213> Homo sapiens

<400> 2026

aatatttttg gacagta                                                    17

<210> 2027
<211> 17
<212> RNA
<213> Homo sapiens

<400> 2027

tggacagtag ctaatgg                                                    17

<210> 2028
<211> 17
<212> RNA
<213> Homo sapiens

<400> 2028

cagtagctaa tgggtca                                                    17

<210> 2029
<211> 17
<212> RNA
<213> Homo sapiens

<400> 2029

taatgggtca gtgggtt                                                    17

<210> 2030
<211> 17
<212> RNA
<213> Homo sapiens

<400> 2030

cagtgggttc tttttaa 17

<210> 2031
<211> 17
<212> RNA
<213> Homo sapiens

<400> 2031

agtgggttct ttttaat 17

<210> 2032
<211> 17
<212> RNA
<213> Homo sapiens

<400> 2032

tgggttcttt ttaatgt 17

<210> 2033
<211> 17
<212> RNA
<213> Homo sapiens

<400> 2033

gggttctttt taatgtt 17

<210> 2034
<211> 17
<212> RNA
<213> Homo sapiens

<400> 2034

ggttcttttt aatgttt 17

<210> 2035
<211> 17
<212> RNA
<213> Homo sapiens

<400> 2035

gttcttttta atgttta                                                    17

<210> 2036
<211> 17
<212> RNA
<213> Homo sapiens

<400> 2036

ttctttttaa tgtttat                                                    17

<210> 2037
<211> 17
<212> RNA
<213> Homo sapiens

<400> 2037

tttaatgttt atactta                                                    17

<210> 2038
<211> 17
<212> RNA
<213> Homo sapiens

<400> 2038

ttaatgttta tacttag                                                    17

<210> 2039
<211> 17
<212> RNA
<213> Homo sapiens

<400> 2039

taatgtttat acttaga                                                    17

<210> 2040
<211> 17
<212> RNA
<213> Homo sapiens

<400> 2040

atgtttatac ttagatt                                                    17

<210> 2041
<211> 17
<212> RNA
<213> Homo sapiens

<400> 2041

tttatactta gattttc                                                    17

<210> 2042
<211> 17
<212> RNA
<213> Homo sapiens

<400> 2042

ttatacttag attttct                                                    17

<210> 2043
<211> 17
<212> RNA
<213> Homo sapiens

<400> 2043

acttagattt tctttta                                                    17

<210> 2044
<211> 17
<212> RNA
<213> Homo sapiens

<400> 2044

cttagatttt cttttaa                                                    17

<210> 2045
<211> 17
<212> RNA
<213> Homo sapiens

<400> 2045

ttagattttc ttttaaa                                                    17

<210> 2046
<211> 17
<212> RNA
<213> Homo sapiens

<400> 2046

tagattttct tttaaaa                                                    17

<210> 2047
<211> 17
<212> RNA
<213> Homo sapiens

<400> 2047

gattttcttt taaaaaa                                                    17

<210> 2048
<211> 17
<212> RNA
<213> Homo sapiens

<400> 2048

attttctttt aaaaaaa                                                    17

<210> 2049
<211> 17
<212> RNA
<213> Homo sapiens

<400> 2049

ttttctttta aaaaaat                                                    17

<210> 2050
<211> 17
<212> RNA
<213> Homo sapiens

<400> 2050

tttcttttaa aaaaatt                                                    17

<210> 2051
<211> 17
<212> RNA
<213> Homo sapiens

<400> 2051

aaaaaaatta aaataaa                                                    17

<210> 2052
<211> 17
<212> RNA
<213> Homo sapiens

<400> 2052

aaaaaattaa aataaaa                                                    17

<210> 2053
<211> 17
<212> RNA
<213> Homo sapiens

<400> 2053

attaaaataa aacaaaa                                                    17

<210> 2054
<211> 17
<212> RNA
<213> Homo sapiens

<400> 2054

aaaaaaattt ctaggac                                                    17

<210> 2055
<211> 17
<212> RNA
<213> Homo sapiens

<400> 2055

aaaaaatttc taggact                                                    17

<210> 2056
<211> 17
<212> RNA
<213> Homo sapiens

<400> 2056

aaaaatttct aggacta                                                    17

<210> 2057
<211> 17
<212> RNA
<213> Homo sapiens

<400> 2057

aaatttctag gactaga 17

<210> 2058
<211> 17
<212> RNA
<213> Homo sapiens

<400> 2058

ctaggactag acgatgt 17

<210> 2059
<211> 17
<212> RNA
<213> Homo sapiens

<400> 2059

gacgatgtaa taccagc 17

<210> 2060
<211> 17
<212> RNA
<213> Homo sapiens

<400> 2060

gatgtaatac cagctaa 17

<210> 2061
<211> 17
<212> RNA
<213> Homo sapiens

<400> 2061

taccagctaa agccaaa 17

<210> 2062
<211> 17
<212> RNA
<213> Homo sapiens

<400> 2062

caaacaatta tacagtg 17

<210> 2063

<211> 17
<212> RNA
<213> Homo sapiens

<400> 2063

aaacaattat acagtgg                                                    17

<210> 2064
<211> 17
<212> RNA
<213> Homo sapiens

<400> 2064

acaattatac agtggaa                                                    17

<210> 2065
<211> 17
<212> RNA
<213> Homo sapiens

<400> 2065

tggaaggttt tacatta                                                    17

<210> 2066
<211> 17
<212> RNA
<213> Homo sapiens

<400> 2066

ggaaggtttt acattat                                                    17

<210> 2067
<211> 17
<212> RNA
<213> Homo sapiens

<400> 2067

gaaggtttta cattatt                                                    17

<210> 2068
<211> 17
<212> RNA
<213> Homo sapiens

<400> 2068

aaggttttac attattc                                                    17

<210> 2069
<211> 17
<212> RNA
<213> Homo sapiens

<400> 2069

ttttacatta ttcatcc                                                    17

<210> 2070
<211> 17
<212> RNA
<213> Homo sapiens

<400> 2070

tttacattat tcatcca                                                    17

<210> 2071
<211> 17
<212> RNA
<213> Homo sapiens

<400> 2071

tacattattc atccaat                                                    17

<210> 2072
<211> 17
<212> RNA
<213> Homo sapiens

<400> 2072

acattattca tccaatg                                                    17

<210> 2073
<211> 17
<212> RNA
<213> Homo sapiens

<400> 2073

ttattcatcc aatgtgt                                                    17

<210> 2074
<211> 17

<212> RNA
<213> Homo sapiens

<400> 2074

caatgtgttt ctattca                                                    17

<210> 2075
<211> 17
<212> RNA
<213> Homo sapiens

<400> 2075

aatgtgtttc tattcat                                                    17

<210> 2076
<211> 17
<212> RNA
<213> Homo sapiens

<400> 2076

atgtgtttct attcatg                                                    17

<210> 2077
<211> 17
<212> RNA
<213> Homo sapiens

<400> 2077

gtgtttctat tcatgtt                                                    17

<210> 2078
<211> 17
<212> RNA
<213> Homo sapiens

<400> 2078

gtttctattc atgttaa                                                    17

<210> 2079
<211> 17
<212> RNA
<213> Homo sapiens

<400> 2079

tttctattca tgttaag                                                    17

<210> 2080
<211> 17
<212> RNA
<213> Homo sapiens

<400> 2080

attcatgtta agatact                                                    17

<210> 2081
<211> 17
<212> RNA
<213> Homo sapiens

<400> 2081

ttcatgttaa gatacta                                                    17

<210> 2082
<211> 17
<212> RNA
<213> Homo sapiens

<400> 2082

gttaagatac tactaca                                                    17

<210> 2083
<211> 17
<212> RNA
<213> Homo sapiens

<400> 2083

aagatactac tacattt                                                    17

<210> 2084
<211> 17
<212> RNA
<213> Homo sapiens

<400> 2084

atactactac atttgaa                                                    17

<210> 2085
<211> 17
<212> RNA

<213> Homo sapiens

<400> 2085

tactacattt gaagtgg 17

<210> 2086
<211> 17
<212> RNA
<213> Homo sapiens

<400> 2086

actacatttg aagtggg 17

<210> 2087
<211> 17
<212> RNA
<213> Homo sapiens

<400> 2087

gagaacatca gatgatt 17

<210> 2088
<211> 17
<212> RNA
<213> Homo sapiens

<400> 2088

cagatgattg aaatgtt 17

<210> 2089
<211> 17
<212> RNA
<213> Homo sapiens

<400> 2089

tgaaatgttc gcccagg 17

<210> 2090
<211> 17
<212> RNA
<213> Homo sapiens

<400> 2090

gaaatgttcg cccaggg 17

<210> 2091
<211> 17
<212> RNA
<213> Homo sapiens

<400> 2091

ccaggggtct ccagcaa 17

<210> 2092
<211> 17
<212> RNA
<213> Homo sapiens

<400> 2092

aggggtctcc agcaact 17

<210> 2093
<211> 17
<212> RNA
<213> Homo sapiens

<400> 2093

cagcaacttt ggaaatc 17

<210> 2094
<211> 17
<212> RNA
<213> Homo sapiens

<400> 2094

agcaactttg gaaatct 17

<210> 2095
<211> 17
<212> RNA
<213> Homo sapiens

<400> 2095

ttggaaatct ctttgta 17

<210> 2096
<211> 17
<212> RNA
<213> Homo sapiens

<400> 2096

ggaaatctct ttgtatt 17

<210> 2097
<211> 17
<212> RNA
<213> Homo sapiens

<400> 2097

aaatctcttt gtatttt 17

<210> 2098
<211> 17
<212> RNA
<213> Homo sapiens

<400> 2098

aatctctttg tattttt 17

<210> 2099
<211> 17
<212> RNA
<213> Homo sapiens

<400> 2099

ctctttgtat ttttact 17

<210> 2100
<211> 17
<212> RNA
<213> Homo sapiens

<400> 2100

ctttgtattt ttacttg 17

<210> 2101
<211> 17
<212> RNA
<213> Homo sapiens

<400> 2101

tttgtatttt tacttga 17

<210> 2102
<211> 17
<212> RNA
<213> Homo sapiens

<400> 2102

ttgtattttt acttgaa                                                    17

<210> 2103
<211> 17
<212> RNA
<213> Homo sapiens

<400> 2103

tgtattttta cttgaag                                                    17

<210> 2104
<211> 17
<212> RNA
<213> Homo sapiens

<400> 2104

gtatttttac ttgaagt                                                    17

<210> 2105
<211> 17
<212> RNA
<213> Homo sapiens

<400> 2105

tttttacttg aagtgcc                                                    17

<210> 2106
<211> 17
<212> RNA
<213> Homo sapiens

<400> 2106

gtgccactaa tggacag                                                    17

<210> 2107
<211> 17
<212> RNA
<213> Homo sapiens

<400> 2107

cagcagatat tttctgg                                                    17

<210> 2108
<211> 17
<212> RNA
<213> Homo sapiens

<400> 2108

gcagatattt tctggct                                                    17

<210> 2109
<211> 17
<212> RNA
<213> Homo sapiens

<400> 2109

cagatatttt ctggctg                                                    17

<210> 2110
<211> 17
<212> RNA
<213> Homo sapiens

<400> 2110

agatattttc tggctga                                                    17

<210> 2111
<211> 17
<212> RNA
<213> Homo sapiens

<400> 2111

gatattttct ggctgat                                                    17

<210> 2112
<211> 17
<212> RNA
<213> Homo sapiens

<400> 2112

gctgatgttg gtattgg                                                    17

<210> 2113
<211> 17
<212> RNA
<213> Homo sapiens

<400> 2113

atgttggtat tgggtgt                                                    17

<210> 2114
<211> 17
<212> RNA
<213> Homo sapiens

<400> 2114

gttggtattg ggtgtag                                                    17

<210> 2115
<211> 17
<212> RNA
<213> Homo sapiens

<400> 2115

ttgggtgtag gaacatg                                                    17

<210> 2116
<211> 17
<212> RNA
<213> Homo sapiens

<400> 2116

aacatgattt aaaaaaa                                                    17

<210> 2117
<211> 17
<212> RNA
<213> Homo sapiens

<400> 2117

acatgattta aaaaaaa                                                    17

<210> 2118
<211> 17
<212> RNA
<213> Homo sapiens

<400> 2118

catgatttaa aaaaaaa                                                    17

<210> 2119
<211> 17
<212> RNA
<213> Homo sapiens

<400> 2119

aaaaaactct tgcctct                                                    17

<210> 2120
<211> 17
<212> RNA
<213> Homo sapiens

<400> 2120

aaaactcttg cctctgc                                                    17

<210> 2121
<211> 17
<212> RNA
<213> Homo sapiens

<400> 2121

tcttgcctct gctttcc                                                    17

<210> 2122
<211> 17
<212> RNA
<213> Homo sapiens

<400> 2122

cctctgcttt cccccac                                                    17

<210> 2123
<211> 17
<212> RNA
<213> Homo sapiens

<400> 2123

ctctgctttc ccccact                                                    17

<210> 2124

<211> 17
<212> RNA
<213> Homo sapiens

<400> 2124

tctgctttcc cccactc                                                    17

<210> 2125
<211> 17
<212> RNA
<213> Homo sapiens

<400> 2125

cccccactct gaggcaa                                                    17

<210> 2126
<211> 17
<212> RNA
<213> Homo sapiens

<400> 2126

aggcaagtta aaatgta                                                    17

<210> 2127
<211> 17
<212> RNA
<213> Homo sapiens

<400> 2127

ggcaagttaa aatgtaa                                                    17

<210> 2128
<211> 17
<212> RNA
<213> Homo sapiens

<400> 2128

taaaatgtaa aagatgt                                                    17

<210> 2129
<211> 17
<212> RNA
<213> Homo sapiens

<400> 2129

gatgtgattt atctggg                                                    17

<210> 2130
<211> 17
<212> RNA
<213> Homo sapiens

<400> 2130

atgtgattta tctgggg                                                    17

<210> 2131
<211> 17
<212> RNA
<213> Homo sapiens

<400> 2131

tgtgatttat ctggggg                                                    17

<210> 2132
<211> 17
<212> RNA
<213> Homo sapiens

<400> 2132
   tgatttatct ggggggc                                                     17

<210> 2133
<211> 17
<212> RNA
<213> Homo sapiens

<400> 2133

ggggggctca ggtatgg                                                    17

<210> 2134
<211> 17
<212> RNA
<213> Homo sapiens

<400> 2134

gctcaggtat ggtgggg                                                    17

<210> 2135
<211> 17

<212> RNA
<213> Homo sapiens

<400> 2135

aagtggattc aggaatc 17

<210> 2136
<211> 17
<212> RNA
<213> Homo sapiens

<400> 2136

agtggattca ggaatct 17

<210> 2137
<211> 17
<212> RNA
<213> Homo sapiens

<400> 2137

tcaggaatct ggggaat 17

<210> 2138
<211> 17
<212> RNA
<213> Homo sapiens

<400> 2138

tggcaaatat attaaga 17

<210> 2139
<211> 17
<212> RNA
<213> Homo sapiens

<400> 2139

gcaaatatat taagaag 17

<210> 2140
<211> 17
<212> RNA
<213> Homo sapiens

<400> 2140

aaatatatta agaagag 17

<210> 2141
<211> 17
<212> RNA
<213> Homo sapiens

<400> 2141

aatatattaa gaagagt                                                    17

<210> 2142
<211> 17
<212> RNA
<213> Homo sapiens

<400> 2142

agaagagtat tgaaagt                                                    17

<210> 2143
<211> 17
<212> RNA
<213> Homo sapiens

<400> 2143

aagagtattg aaagtat                                                    17

<210> 2144
<211> 17
<212> RNA
<213> Homo sapiens

<400> 2144

ttgaaagtat ttggagg                                                    17

<210> 2145
<211> 17
<212> RNA
<213> Homo sapiens

<400> 2145

gaaagtattt ggaggaa                                                    17

<210> 2146
<211> 17
<212> RNA

<213> Homo sapiens

<400> 2146

aaagtatttg gaggaaa 17

<210> 2147
<211> 17
<212> RNA
<213> Homo sapiens

<400> 2147

aaaatggtta attctgg 17

<210> 2148
<211> 17
<212> RNA
<213> Homo sapiens

<400> 2148

aaatggttaa ttctggg 17

<210> 2149
<211> 17
<212> RNA
<213> Homo sapiens

<400> 2149

tggttaattc tgggtgt 17

<210> 2150
<211> 17
<212> RNA
<213> Homo sapiens

<400> 2150

ggttaattct gggtgtg 17

<210> 2151
<211> 17
<212> RNA
<213> Homo sapiens

<400> 2151

accaaggttc agtagag 17

<210> 2152
<211> 17
<212> RNA
<213> Homo sapiens

<400> 2152

ccaaggttca gtagagt 17

<210> 2153
<211> 17
<212> RNA
<213> Homo sapiens

<400> 2153

ggttcagtag agtccac 17

<210> 2154
<211> 17
<212> RNA
<213> Homo sapiens

<400> 2154

agtagagtcc acttctg 17

<210> 2155
<211> 17
<212> RNA
<213> Homo sapiens

<400> 2155

agtccacttc tgccctg 17

<210> 2156
<211> 17
<212> RNA
<213> Homo sapiens

<400> 2156

gtccacttct gccctgg 17

<210> 2157
<211> 17
<212> RNA
<213> Homo sapiens

<400> 2157

ccacaaatca actagct                                                    17

<210> 2158
<211> 17
<212> RNA
<213> Homo sapiens

<400> 2158

aatcaactag ctccatt                                                    17

<210> 2159
<211> 17
<212> RNA
<213> Homo sapiens

<400> 2159

aactagctcc atttaca                                                    17

<210> 2160
<211> 17
<212> RNA
<213> Homo sapiens

<400> 2160

agctccattt acagcca                                                    17

<210> 2161
<211> 17
<212> RNA
<213> Homo sapiens

<400> 2161

gctccattta cagccat                                                    17

<210> 2162
<211> 17
<212> RNA
<213> Homo sapiens

<400> 2162

ctccatttac agccatt                                                    17

<210> 2163
<211> 17
<212> RNA
<213> Homo sapiens

<400> 2163

acagccattt ctaaaat 17

<210> 2164
<211> 17
<212> RNA
<213> Homo sapiens

<400> 2164

cagccatttc taaaatg 17

<210> 2165
<211> 17
<212> RNA
<213> Homo sapiens

<400> 2165

agccatttct aaaatgg 17

<210> 2166
<211> 17
<212> RNA
<213> Homo sapiens

<400> 2166

ccatttctaa aatggca 17

<210> 2167
<211> 17
<212> RNA
<213> Homo sapiens

<400> 2167

tggcagcttc agttcta 17

<210> 2168
<211> 17
<212> RNA
<213> Homo sapiens

<400> 2168

ggcagcttca gttctag                                                    17

<210> 2169
<211> 17
<212> RNA
<213> Homo sapiens

<400> 2169

gcttcagttc tagagaa                                                    17

<210> 2170
<211> 17
<212> RNA
<213> Homo sapiens

<400> 2170

cttcagttct agagaag                                                    17

<210> 2171
<211> 17
<212> RNA
<213> Homo sapiens

<400> 2171

tcagttctag agaagaa                                                    17

<210> 2172
<211> 17
<212> RNA
<213> Homo sapiens

<400> 2172

aacaacatca gcagtaa                                                    17

<210> 2173
<211> 17
<212> RNA
<213> Homo sapiens

<400> 2173

tcagcagtaa agtccat                                                    17

<210> 2174
<211> 17
<212> RNA
<213> Homo sapiens

<400> 2174

agtaaagtcc atggaat                                                    17

<210> 2175
<211> 17
<212> RNA
<213> Homo sapiens

<400> 2175

catggaatag ctagtgg                                                    17

<210> 2176
<211> 17
<212> RNA
<213> Homo sapiens

<400> 2176

gaatagctag tggtctg                                                    17

<210> 2177
<211> 17
<212> RNA
<213> Homo sapiens

<400> 2177

ctagtggtct gtgtttc                                                    17

<210> 2178
<211> 17
<212> RNA
<213> Homo sapiens

<400> 2178

gtctgtgttt cttttcg                                                    17

<210> 2179
<211> 17
<212> RNA
<213> Homo sapiens

<400> 2179

tctgtgtttc ttttcgc 17

<210> 2180
<211> 17
<212> RNA
<213> Homo sapiens

<400> 2180

ctgtgtttct tttcgcc 17

<210> 2181
<211> 17
<212> RNA
<213> Homo sapiens

<400> 2181

gtgtttcttt tcgccat 17

<210> 2182
<211> 17
<212> RNA
<213> Homo sapiens

<400> 2182

tgtttctttt cgccatt 17

<210> 2183
<211> 17
<212> RNA
<213> Homo sapiens

<400> 2183

gtttcttttc gccattg 17

<210> 2184
<211> 17
<212> RNA
<213> Homo sapiens

<400> 2184

tttcttttcg ccattgc 17

<210> 2185

<211> 17
<212> RNA
<213> Homo sapiens

<400> 2185

ttcgccattg cctagct                                                    17



<210> 2186
<211> 17
<212> RNA
<213> Homo sapiens

<400> 2186

cattgcctag cttgccg                                                    17



<210> 2187
<211> 17
<212> RNA
<213> Homo sapiens

<400> 2187

gcctagcttg ccgtaat                                                    17



<210> 2188
<211> 17
<212> RNA
<213> Homo sapiens

<400> 2188

cttgccgtaa tgattct                                                    17



<210> 2189
<211> 17
<212> RNA
<213> Homo sapiens

<400> 2189

gtaatgattc tataatg                                                    17



<210> 2190
<211> 17
<212> RNA
<213> Homo sapiens

<400> 2190

taatgattct ataatgc                                                17

<210> 2191
<211> 17
<212> RNA
<213> Homo sapiens

<400> 2191

atgattctat aatgcca                                                17

<210> 2192
<211> 17
<212> RNA
<213> Homo sapiens

<400> 2192

gattctataa tgccatc                                                17

<210> 2193
<211> 17
<212> RNA
<213> Homo sapiens

<400> 2193

aatgccatca tgcagca                                                17

<210> 2194
<211> 17
<212> RNA
<213> Homo sapiens

<400> 2194

gcagcaatta tgagagg                                                17

<210> 2195
<211> 17
<212> RNA
<213> Homo sapiens

<400> 2195

cagcaattat gagaggc                                                17

<210> 2196
<211> 17

<212> RNA
<213> Homo sapiens

<400> 2196

gagaggctag gtcatcc                                                    17



<210> 2197
<211> 17
<212> RNA
<213> Homo sapiens

<400> 2197

ggctaggtca tccaaag                                                    17



<210> 2198
<211> 17
<212> RNA
<213> Homo sapiens

<400> 2198

taggtcatcc aaagaga                                                    17



<210> 2199
<211> 17
<212> RNA
<213> Homo sapiens

<400> 2199

aagaccctat caatgta                                                    17



<210> 2200
<211> 17
<212> RNA
<213> Homo sapiens

<400> 2200

gaccctatca atgtagg                                                    17



<210> 2201
<211> 17
<212> RNA
<213> Homo sapiens

<400> 2201

atcaatgtag gttgcaa                                                    17

<210> 2202
<211> 17
<212> RNA
<213> Homo sapiens

<400> 2202

atgtaggttg caaaatc 17

<210> 2203
<211> 17
<212> RNA
<213> Homo sapiens

<400> 2203

tgcaaaatct aacccct 17

<210> 2204
<211> 17
<212> RNA
<213> Homo sapiens

<400> 2204

caaaatctaa cccctaa 17

<210> 2205
<211> 17
<212> RNA
<213> Homo sapiens

<400> 2205

taacccctaa ggaagtg 17

<210> 2206
<211> 17
<212> RNA
<213> Homo sapiens

<400> 2206

agtgcagtct ttgattt 17

<210> 2207
<211> 17
<212> RNA

<213> Homo sapiens

<400> 2207

tgcagtcttt gatttga                                                    17

<210> 2208
<211> 17
<212> RNA
<213> Homo sapiens

<400> 2208

gcagtctttg atttgat                                                    17

<210> 2209
<211> 17
<212> RNA
<213> Homo sapiens

<400> 2209

tctttgattt gatttcc                                                    17

<210> 2210
<211> 17
<212> RNA
<213> Homo sapiens

<400> 2210

ctttgatttg atttccc                                                    17

<210> 2211
<211> 17
<212> RNA
<213> Homo sapiens

<400> 2211

gatttgattt ccctagt                                                    17

<210> 2212
<211> 17
<212> RNA
<213> Homo sapiens

<400> 2212

atttgatttc cctagta                                                    17

<210> 2213
<211> 17
<212> RNA
<213> Homo sapiens

<400> 2213

tttgatttcc ctagtaa 17

<210> 2214
<211> 17
<212> RNA
<213> Homo sapiens

<400> 2214

atttccctag taacctt 17

<210> 2215
<211> 17
<212> RNA
<213> Homo sapiens

<400> 2215

tccctagtaa ccttgca 17

<210> 2216
<211> 17
<212> RNA
<213> Homo sapiens

<400> 2216

agtaaccttg cagatat 17

<210> 2217
<211> 17
<212> RNA
<213> Homo sapiens

<400> 2217

ttgcagatat gtttaac 17

<210> 2218
<211> 17
<212> RNA
<213> Homo sapiens

<400> 2218

agatatgttt aaccaag                                                    17

<210> 2219
<211> 17
<212> RNA
<213> Homo sapiens

<400> 2219

gatatgttta accaagc                                                    17

<210> 2220
<211> 17
<212> RNA
<213> Homo sapiens

<400> 2220

atatgtttaa ccaagcc                                                    17

<210> 2221
<211> 17
<212> RNA
<213> Homo sapiens

<400> 2221

caagccatag cccatgc                                                    17

<210> 2222
<211> 17
<212> RNA
<213> Homo sapiens

<400> 2222

ccatgccttt tgagggc                                                    17

<210> 2223
<211> 17
<212> RNA
<213> Homo sapiens

<400> 2223

catgcctttt gagggct                                                    17

<210> 2224
<211> 17
<212> RNA
<213> Homo sapiens

<400> 2224

atgccttttg agggctg                                                    17

<210> 2225
<211> 17
<212> RNA
<213> Homo sapiens

<400> 2225

gaacaaataa gggactt                                                    17

<210> 2226
<211> 17
<212> RNA
<213> Homo sapiens

<400> 2226

aagggactta ctgataa                                                    17

<210> 2227
<211> 17
<212> RNA
<213> Homo sapiens

<400> 2227

agggacttac tgataat                                                    17

<210> 2228
<211> 17
<212> RNA
<213> Homo sapiens

<400> 2228

ttactgataa tttactt                                                    17

<210> 2229
<211> 17
<212> RNA
<213> Homo sapiens

<400> 2229

ctgataattt acttttg                                                    17

<210> 2230
<211> 17
<212> RNA
<213> Homo sapiens

<400> 2230

tgataattta cttttga                                                    17

<210> 2231
<211> 17
<212> RNA
<213> Homo sapiens

<400> 2231

gataatttac ttttgat                                                    17

<210> 2232
<211> 17
<212> RNA
<213> Homo sapiens

<400> 2232

aatttacttt tgatcac                                                    17

<210> 2233
<211> 17
<212> RNA
<213> Homo sapiens

<400> 2233

atttactttt gatcaca                                                    17

<210> 2234
<211> 17
<212> RNA
<213> Homo sapiens

<400> 2234

tttacttttg atcacat                                                    17

<210> 2235
<211> 17
<212> RNA
<213> Homo sapiens

<400> 2235

cttttgatca cattaag                                                    17

<210> 2236
<211> 17
<212> RNA
<213> Homo sapiens

<400> 2236

gatcacatta aggtgtt                                                    17

<210> 2237
<211> 17
<212> RNA
<213> Homo sapiens

<400> 2237

atcacattaa ggtgttc                                                    17

<210> 2238
<211> 17
<212> RNA
<213> Homo sapiens

<400> 2238

taaggtgttc tcacctt                                                    17

<210> 2239
<211> 17
<212> RNA
<213> Homo sapiens

<400> 2239

aaggtgttct caccttg                                                    17

<210> 2240
<211> 17
<212> RNA
<213> Homo sapiens

<400> 2240

ggtgttctca ccttgaa 17

<210> 2241
<211> 17
<212> RNA
<213> Homo sapiens

<400> 2241

tctcaccttg aaatctt 17

<210> 2242
<211> 17
<212> RNA
<213> Homo sapiens

<400> 2242

cttgaaatct tatacac 17

<210> 2243
<211> 17
<212> RNA
<213> Homo sapiens

<400> 2243

tgaaatctta tacactg 17

<210> 2244
<211> 17
<212> RNA
<213> Homo sapiens

<400> 2244

gaaatcttat acactga 17

<210> 2245
<211> 17
<212> RNA
<213> Homo sapiens

<400> 2245

aatcttatac actgaaa 17

<210> 2246

<211> 17
<212> RNA
<213> Homo sapiens

<400> 2246

atggccattg atttagg                                                    17

<210> 2247
<211> 17
<212> RNA
<213> Homo sapiens

<400> 2247

ccattgattt aggccac                                                    17

<210> 2248
<211> 17
<212> RNA
<213> Homo sapiens

<400> 2248

cattgattta ggccact                                                    17

<210> 2249
<211> 17
<212> RNA
<213> Homo sapiens

<400> 2249

attgatttag gccactg                                                    17

<210> 2250
<211> 17
<212> RNA
<213> Homo sapiens

<400> 2250

cactggctta gagtact                                                    17

<210> 2251
<211> 17
<212> RNA
<213> Homo sapiens

<400> 2251

actggcttag agtactc                                                    17

<210> 2252
<211> 17
<212> RNA
<213> Homo sapiens

<400> 2252

cttagagtac tccttcc                                                    17

<210> 2253
<211> 17
<212> RNA
<213> Homo sapiens

<400> 2253

agagtactcc ttcccct                                                    17

<210> 2254
<211> 17
<212> RNA
<213> Homo sapiens

<400> 2254

gtactccttc ccctgca                                                    17

<210> 2255
<211> 17
<212> RNA
<213> Homo sapiens

<400> 2255

tactccttcc cctgcat                                                    17

<210> 2256
<211> 17
<212> RNA
<213> Homo sapiens

<400> 2256

acactgatta caaatac                                                    17

<210> 2257
<211> 17

<212> RNA
<213> Homo sapiens

<400> 2257

cactgattac aaatact 17

<210> 2258
<211> 17
<212> RNA
<213> Homo sapiens

<400> 2258

ttacaaatac tttccta 17

<210> 2259
<211> 17
<212> RNA
<213> Homo sapiens

<400> 2259

caaatacttt cctattc 17

<210> 2260
<211> 17
<212> RNA
<213> Homo sapiens

<400> 2260

aaatactttc ctattca 17

<210> 2261
<211> 17
<212> RNA
<213> Homo sapiens

<400> 2261

aatactttcc tattcat 17

<210> 2262
<211> 17
<212> RNA
<213> Homo sapiens

<400> 2262

actttcctat tcatact 17

<210> 2263
<211> 17
<212> RNA
<213> Homo sapiens

<400> 2263

tttcctattc atacttt                                                    17

<210> 2264
<211> 17
<212> RNA
<213> Homo sapiens

<400> 2264

ttcctattca tactttc                                                    17

<210> 2265
<211> 17
<212> RNA
<213> Homo sapiens

<400> 2265

ctattcatac tttccaa                                                    17

<210> 2266
<211> 17
<212> RNA
<213> Homo sapiens

<400> 2266

ttcatacttt ccaatta                                                    17

<210> 2267
<211> 17
<212> RNA
<213> Homo sapiens

<400> 2267

tcatactttc caattat                                                    17

<210> 2268
<211> 17
<212> RNA

<213> Homo sapiens

<400> 2268

catactttcc aattatg                                                    17

<210> 2269
<211> 17
<212> RNA
<213> Homo sapiens

<400> 2269

tttccaatta tgagatg                                                    17

<210> 2270
<211> 17
<212> RNA
<213> Homo sapiens

<400> 2270

ttccaattat gagatgg                                                    17

<210> 2271
<211> 17
<212> RNA
<213> Homo sapiens

<400> 2271

ctgtgggtac tgggagt                                                    17

<210> 2272
<211> 17
<212> RNA
<213> Homo sapiens

<400> 2272

ggagtgatca ctaacac                                                    17

<210> 2273
<211> 17
<212> RNA
<213> Homo sapiens

<400> 2273

tgatcactaa caccata                                                    17

<210> 2274
<211> 17
<212> RNA
<213> Homo sapiens

<400> 2274

aacaccatag taatgtc 17

<210> 2275
<211> 17
<212> RNA
<213> Homo sapiens

<400> 2275

accatagtaa tgtctaa 17

<210> 2276
<211> 17
<212> RNA
<213> Homo sapiens

<400> 2276

agtaatgtct aatattc 17

<210> 2277
<211> 17
<212> RNA
<213> Homo sapiens

<400> 2277

taatgtctaa tattcac 17

<210> 2278
<211> 17
<212> RNA
<213> Homo sapiens

<400> 2278

tgtctaatat tcacagg 17

<210> 2279
<211> 17
<212> RNA
<213> Homo sapiens

<400> 2279

tctaatattc acaggca                                                    17

<210> 2280
<211> 17
<212> RNA
<213> Homo sapiens

<400> 2280

ctaatattca caggcag                                                    17

<210> 2281
<211> 17
<212> RNA
<213> Homo sapiens

<400> 2281

aggcagatct gcttggg                                                    17

<210> 2282
<211> 17
<212> RNA
<213> Homo sapiens

<400> 2282

gatctgcttg ggaagc                                                     17

<210> 2283
<211> 17
<212> RNA
<213> Homo sapiens

<400> 2283

gggaagctag ttatgtg                                                    17

<210> 2284
<211> 17
<212> RNA
<213> Homo sapiens

<400> 2284

aagctagtta tgtgaaa                                                    17

<210> 2285
<211> 17
<212> RNA
<213> Homo sapiens

<400> 2285

agctagttat gtgaaag                                                    17

<210> 2286
<211> 17
<212> RNA
<213> Homo sapiens

<400> 2286

aggcaaataa agtcata                                                    17

<210> 2287
<211> 17
<212> RNA
<213> Homo sapiens

<400> 2287

aataaagtca tacagta                                                    17

<210> 2288
<211> 17
<212> RNA
<213> Homo sapiens

<400> 2288

aaagtcatac agtagct                                                    17

<210> 2289
<211> 17
<212> RNA
<213> Homo sapiens

<400> 2289

catacagtag ctcaaaa                                                    17

<210> 2290
<211> 17
<212> RNA
<213> Homo sapiens

<400> 2290

cagtagctca aaaggca                                                    17

<210> 2291
<211> 17
<212> RNA
<213> Homo sapiens

<400> 2291

gcaaccataa ttctctt                                                    17

<210> 2292
<211> 17
<212> RNA
<213> Homo sapiens

<400> 2292

accataattc tctttgg                                                    17

<210> 2293
<211> 17
<212> RNA
<213> Homo sapiens

<400> 2293

ccataattct ctttggt                                                    17

<210> 2294
<211> 17
<212> RNA
<213> Homo sapiens

<400> 2294

ataattctct ttggtgc                                                    17

<210> 2295
<211> 17
<212> RNA
<213> Homo sapiens

<400> 2295

aattctcttt ggtgcaa                                                    17

<210> 2296
<211> 17
<212> RNA
<213> Homo sapiens

<400> 2296

attctctttg gtgcaag 17

<210> 2297
<211> 17
<212> RNA
<213> Homo sapiens

<400> 2297

gtgcaagtct tgggagc 17

<210> 2298
<211> 17
<212> RNA
<213> Homo sapiens

<400> 2298

gcaagtcttg ggagcgt 17

<210> 2299
<211> 17
<212> RNA
<213> Homo sapiens

<400> 2299

agcgtgatct agattac 17

<210> 2300
<211> 17
<212> RNA
<213> Homo sapiens

<400> 2300

cgtgatctag attacac 17

<210> 2301
<211> 17
<212> RNA
<213> Homo sapiens

<400> 2301

atctagatta cactgca                                                    17

<210> 2302
<211> 17
<212> RNA
<213> Homo sapiens

<400> 2302

tctagattac actgcac                                                    17

<210> 2303
<211> 17
<212> RNA
<213> Homo sapiens

<400> 2303

tgcaccattc ccaagtt                                                    17

<210> 2304
<211> 17
<212> RNA
<213> Homo sapiens

<400> 2304

gcaccattcc caagtta                                                    17

<210> 2305
<211> 17
<212> RNA
<213> Homo sapiens

<400> 2305

tcccaagtta atcccct                                                    17

<210> 2306
<211> 17
<212> RNA
<213> Homo sapiens

<400> 2306

cccaagttaa tcccctg                                                    17

<210> 2307

<211> 17
<212> RNA
<213> Homo sapiens

<400> 2307

aagttaatcc cctgaaa                                                    17

<210> 2308
<211> 17
<212> RNA
<213> Homo sapiens

<400> 2308

tgaaaactta ctctcaa                                                    17

<210> 2309
<211> 17
<212> RNA
<213> Homo sapiens

<400> 2309

gaaaacttac tctcaac                                                    17

<210> 2310
<211> 17
<212> RNA
<213> Homo sapiens

<400> 2310

aacttactct caactgg                                                    17

<210> 2311
<211> 17
<212> RNA
<213> Homo sapiens

<400> 2311

cttactctca actggag                                                    17

<210> 2312
<211> 17
<212> RNA
<213> Homo sapiens

<400> 2312

aatgaacttt ggtccca 17

<210> 2313
<211> 17
<212> RNA
<213> Homo sapiens

<400> 2313

atgaactttg gtcccaa 17

<210> 2314
<211> 17
<212> RNA
<213> Homo sapiens

<400> 2314

actttggtcc caaatat 17

<210> 2315
<211> 17
<212> RNA
<213> Homo sapiens

<400> 2315

tcccaaatat ccatctt 17

<210> 2316
<211> 17
<212> RNA
<213> Homo sapiens

<400> 2316

ccaaatatcc atctttt 17

<210> 2317
<211> 17
<212> RNA
<213> Homo sapiens

<400> 2317

atatccatct tttcagt 17

<210> 2318
<211> 17

<212> RNA
<213> Homo sapiens

<400> 2318

atccatcttt tcagtag                                                    17



<210> 2319
<211> 17
<212> RNA
<213> Homo sapiens

<400> 2319

tccatctttt cagtagc                                                    17



<210> 2320
<211> 17
<212> RNA
<213> Homo sapiens

<400> 2320

ccatcttttc agtagcg                                                    17



<210> 2321
<211> 17
<212> RNA
<213> Homo sapiens

<400> 2321

catcttttca gtagcgt                                                    17



<210> 2322
<211> 17
<212> RNA
<213> Homo sapiens

<400> 2322

ttttcagtag cgttaat                                                    17



<210> 2323
<211> 17
<212> RNA
<213> Homo sapiens

<400> 2323

agtagcgtta attatgc                                                    17

<210> 2324
<211> 17
<212> RNA
<213> Homo sapiens

<400> 2324

gtagcgttaa ttatgct                                                    17

<210> 2325
<211> 17
<212> RNA
<213> Homo sapiens

<400> 2325

gcgttaatta tgctctg                                                    17

<210> 2326
<211> 17
<212> RNA
<213> Homo sapiens

<400> 2326

cgttaattat gctctgt                                                    17

<210> 2327
<211> 17
<212> RNA
<213> Homo sapiens

<400> 2327

attatgctct gtttcca                                                    17

<210> 2328
<211> 17
<212> RNA
<213> Homo sapiens

<400> 2328

tgctctgttt ccaactg                                                    17

<210> 2329
<211> 17
<212> RNA

<213> Homo sapiens

<400> 2329

gctctgtttc caactgc 17

<210> 2330
<211> 17
<212> RNA
<213> Homo sapiens

<400> 2330

ctctgtttcc aactgca 17

<210> 2331
<211> 17
<212> RNA
<213> Homo sapiens

<400> 2331

aactgcattt cctttcc 17

<210> 2332
<211> 17
<212> RNA
<213> Homo sapiens

<400> 2332

actgcatttc ctttcca 17

<210> 2333
<211> 17
<212> RNA
<213> Homo sapiens

<400> 2333

ctgcatttcc tttccaa 17

<210> 2334
<211> 17
<212> RNA
<213> Homo sapiens

<400> 2334

catttccttt ccaattg 17

<210> 2335
<211> 17
<212> RNA
<213> Homo sapiens

<400> 2335

atttcctttc caattga                                                    17

<210> 2336
<211> 17
<212> RNA
<213> Homo sapiens

<400> 2336

tttcctttcc aattgaa                                                    17

<210> 2337
<211> 17
<212> RNA
<213> Homo sapiens

<400> 2337

tttccaattg aattaaa                                                    17

<210> 2338
<211> 17
<212> RNA
<213> Homo sapiens

<400> 2338

aattgaatta aagtgtg                                                    17

<210> 2339
<211> 17
<212> RNA
<213> Homo sapiens

<400> 2339

attgaattaa agtgtgg                                                    17

<210> 2340
<211> 17
<212> RNA
<213> Homo sapiens

<400> 2340

tgtggcctcg tttttag 17

<210> 2341
<211> 17
<212> RNA
<213> Homo sapiens

<400> 2341

ggcctcgttt ttagtca 17

<210> 2342
<211> 17
<212> RNA
<213> Homo sapiens

<400> 2342

gcctcgtttt tagtcat 17

<210> 2343
<211> 17
<212> RNA
<213> Homo sapiens

<400> 2343

cctcgttttt agtcatt 17

<210> 2344
<211> 17
<212> RNA
<213> Homo sapiens

<400> 2344

ctcgttttta gtcattt 17

<210> 2345
<211> 17
<212> RNA
<213> Homo sapiens

<400> 2345

tcgtttttag tcattta 17

<210> 2346
<211> 17
<212> RNA
<213> Homo sapiens

<400> 2346

tttttagtca tttaaaa 17

<210> 2347
<211> 17
<212> RNA
<213> Homo sapiens

<400> 2347

ttagtcattt aaaattg 17

<210> 2348
<211> 17
<212> RNA
<213> Homo sapiens

<400> 2348

tagtcattta aaattgt 17

<210> 2349
<211> 17
<212> RNA
<213> Homo sapiens

<400> 2349

agtcatttaa aattgtt 17

<210> 2350
<211> 17
<212> RNA
<213> Homo sapiens

<400> 2350

tttaaaattg ttttcta 17

<210> 2351
<211> 17
<212> RNA
<213> Homo sapiens

<400> 2351

aaaattgttt tctaagt                                                    17

<210> 2352
<211> 17
<212> RNA
<213> Homo sapiens

<400> 2352

aaattgtttt ctaagta                                                    17

<210> 2353
<211> 17
<212> RNA
<213> Homo sapiens

<400> 2353

aattgttttc taagtaa                                                    17

<210> 2354
<211> 17
<212> RNA
<213> Homo sapiens

<400> 2354

attgttttct aagtaat                                                    17

<210> 2355
<211> 17
<212> RNA
<213> Homo sapiens

<400> 2355

tgttttctaa gtaattg                                                    17

<210> 2356
<211> 17
<212> RNA
<213> Homo sapiens

<400> 2356

ttctaagtaa ttgctgc                                                    17

<210> 2357
<211> 17
<212> RNA
<213> Homo sapiens

<400> 2357

taagtaattg ctgcctc                                                    17

<210> 2358
<211> 17
<212> RNA
<213> Homo sapiens

<400> 2358

tgctgcctct attatgg                                                    17

<210> 2359
<211> 17
<212> RNA
<213> Homo sapiens

<400> 2359

ctgcctctat tatggca                                                    17

<210> 2360
<211> 17
<212> RNA
<213> Homo sapiens

<400> 2360

gcctctatta tggcact                                                    17

<210> 2361
<211> 17
<212> RNA
<213> Homo sapiens

<400> 2361

cctctattat ggcactt                                                    17

<210> 2362
<211> 17
<212> RNA
<213> Homo sapiens

<400> 2362

atggcacttc aattttg                                                    17

<210> 2363
<211> 17
<212> RNA
<213> Homo sapiens

<400> 2363

tggcacttca attttgc                                                    17

<210> 2364
<211> 17
<212> RNA
<213> Homo sapiens

<400> 2364

acttcaattt tgcactg                                                    17

<210> 2365
<211> 17
<212> RNA
<213> Homo sapiens

<400> 2365

cttcaatttt gcactgt                                                    17

<210> 2366
<211> 17
<212> RNA
<213> Homo sapiens

<400> 2366

ttcaattttg cactgtc                                                    17

<210> 2367
<211> 17
<212> RNA
<213> Homo sapiens

<400> 2367

tgcactgtct tttgaga                                                    17

<210> 2368

<211> 17
<212> RNA
<213> Homo sapiens

<400> 2368

cactgtcttt tgagatt                                                    17

<210> 2369
<211> 17
<212> RNA
<213> Homo sapiens

<400> 2369

actgtctttt gagattc                                                    17

<210> 2370
<211> 17
<212> RNA
<213> Homo sapiens

<400> 2370

ctgtcttttg agattca                                                    17

<210> 2371
<211> 17
<212> RNA
<213> Homo sapiens

<400> 2371

tttgagattc aagaaaa                                                    17

<210> 2372
<211> 17
<212> RNA
<213> Homo sapiens

<400> 2372

ttgagattca agaaaaa                                                    17

<210> 2373
<211> 17
<212> RNA
<213> Homo sapiens

<400> 2373

agaaaaattt ctattca 17

<210> 2374
<211> 17
<212> RNA
<213> Homo sapiens

<400> 2374

gaaaaatttc tattcat 17

<210> 2375
<211> 17
<212> RNA
<213> Homo sapiens

<400> 2375

aaaaatttct attcatt 17

<210> 2376
<211> 17
<212> RNA
<213> Homo sapiens

<400> 2376

aaatttctat tcatttt 17

<210> 2377
<211> 17
<212> RNA
<213> Homo sapiens

<400> 2377

atttctattc attttt 17

<210> 2378
<211> 17
<212> RNA
<213> Homo sapiens

<400> 2378

tttctattca ttttttt 17

<210> 2379
<211> 17

<212> RNA
<213> Homo sapiens

<400> 2379

ctattcattt ttttgca                                                    17

<210> 2380
<211> 17
<212> RNA
<213> Homo sapiens

<400> 2380

tattcatttt tttgcat                                                    17

<210> 2381
<211> 17
<212> RNA
<213> Homo sapiens

<400> 2381

attcattttt ttgcatc                                                    17

<210> 2382
<211> 17
<212> RNA
<213> Homo sapiens

<400> 2382

ttcatttttt tgcatcc                                                    17

<210> 2383
<211> 17
<212> RNA
<213> Homo sapiens

<400> 2383

tcattttttt gcatcca                                                    17

<210> 2384
<211> 17
<212> RNA
<213> Homo sapiens

<400> 2384
   catttttttg catccaa                                                     17

<210> 2385
<211> 17
<212> RNA
<213> Homo sapiens

<400> 2385

ttttgcatcc aattgtg                                                    17

<210> 2386
<211> 17
<212> RNA
<213> Homo sapiens

<400> 2386

catccaattg tgcctga                                                    17

<210> 2387
<211> 17
<212> RNA
<213> Homo sapiens

<400> 2387

cctgaacttt taaaata                                                    17

<210> 2388
<211> 17
<212> RNA
<213> Homo sapiens

<400> 2388

ctgaactttt aaaatat                                                    17

<210> 2389
<211> 17
<212> RNA
<213> Homo sapiens

<400> 2389

tgaactttta aaatatg                                                    17

<210> 2390
<211> 17
<212> RNA

<213> Homo sapiens

<400> 2390

gaacttttaa aatatgt 17

<210> 2391
<211> 17
<212> RNA
<213> Homo sapiens

<400> 2391

tttaaaatat gtaaatg 17

<210> 2392
<211> 17
<212> RNA
<213> Homo sapiens

<400> 2392

aaatatgtaa atgctgc 17

<210> 2393
<211> 17
<212> RNA
<213> Homo sapiens

<400> 2393

tgccatgttc caaaccc 17

<210> 2394
<211> 17
<212> RNA
<213> Homo sapiens

<400> 2394

gccatgttcc aaaccca 17

<210> 2395
<211> 17
<212> RNA
<213> Homo sapiens

<400> 2395

aaacccatcg tcagtgt 17

<210> 2396
<211> 17
<212> RNA
<213> Homo sapiens

<400> 2396

cccatcgtca gtgtgtg 17

<210> 2397
<211> 17
<212> RNA
<213> Homo sapiens

<400> 2397

gtgtgtgttt agagctg 17

<210> 2398
<211> 17
<212> RNA
<213> Homo sapiens

<400> 2398

tgtgtgttta gagctgt 17

<210> 2399
<211> 17
<212> RNA
<213> Homo sapiens

<400> 2399

gtgtgtttag agctgtg 17

<210> 2400
<211> 17
<212> RNA
<213> Homo sapiens

<400> 2400

tgcaccctag aaacaac 17

<210> 2401
<211> 17
<212> RNA
<213> Homo sapiens

<400> 2401

aacaacatac ttgtccc                                                    17

<210> 2402
<211> 17
<212> RNA
<213> Homo sapiens

<400> 2402

aacatacttg tcccatg                                                    17

<210> 2403
<211> 17
<212> RNA
<213> Homo sapiens

<400> 2403

atacttgtcc catgagc                                                    17

<210> 2404
<211> 17
<212> RNA
<213> Homo sapiens

<400> 2404

agaccccttt gcattca                                                    17

<210> 2405
<211> 17
<212> RNA
<213> Homo sapiens

<400> 2405

gacccctttg cattcac                                                    17

<210> 2406
<211> 17
<212> RNA
<213> Homo sapiens

<400> 2406

ctttgcattc acagaga                                                    17

<210> 2407
<211> 17
<212> RNA
<213> Homo sapiens

<400> 2407

tttgcattca cagagag                                                    17

<210> 2408
<211> 17
<212> RNA
<213> Homo sapiens

<400> 2408

agagaggtca ttggtta                                                    17

<210> 2409
<211> 17
<212> RNA
<213> Homo sapiens

<400> 2409

gaggtcattg gttatag                                                    17

<210> 2410
<211> 17
<212> RNA
<213> Homo sapiens

<400> 2410

tcattggtta tagagac                                                    17

<210> 2411
<211> 17
<212> RNA
<213> Homo sapiens

<400> 2411

cattggttat agagact                                                    17

<210> 2412
<211> 17
<212> RNA
<213> Homo sapiens

<400> 2412

ttggttatag agacttg                                                    17

<210> 2413
<211> 17
<212> RNA
<213> Homo sapiens

<400> 2413

tagagacttg aattaat                                                    17

<210> 2414
<211> 17
<212> RNA
<213> Homo sapiens

<400> 2414

acttgaatta ataagtg                                                    17

<210> 2415
<211> 17
<212> RNA
<213> Homo sapiens

<400> 2415

cttgaattaa taagtga                                                    17

<210> 2416
<211> 17
<212> RNA
<213> Homo sapiens

<400> 2416

gaattaataa gtgacat                                                    17

<210> 2417
<211> 17
<212> RNA
<213> Homo sapiens

<400> 2417

agtgacatta tgccagt                                                    17

<210> 2418
<211> 17
<212> RNA
<213> Homo sapiens

<400> 2418

gtgacattat gccagtt                                                    17

<210> 2419
<211> 17
<212> RNA
<213> Homo sapiens

<400> 2419

atgccagttt ctgttct                                                    17

<210> 2420
<211> 17
<212> RNA
<213> Homo sapiens

<400> 2420

tgccagtttc tgttctc                                                    17

<210> 2421
<211> 17
<212> RNA
<213> Homo sapiens

<400> 2421

gccagtttct gttctct                                                    17

<210> 2422
<211> 17
<212> RNA
<213> Homo sapiens

<400> 2422

gtttctgttc tctcaca                                                    17

<210> 2423
<211> 17
<212> RNA
<213> Homo sapiens

<400> 2423

tttctgttct ctcacag 17

<210> 2424
<211> 17
<212> RNA
<213> Homo sapiens

<400> 2424

tctgttctct cacaggt 17

<210> 2425
<211> 17
<212> RNA
<213> Homo sapiens

<400> 2425

tgttctctca caggtga 17

<210> 2426
<211> 17
<212> RNA
<213> Homo sapiens

<400> 2426

caggtgataa acaatgc 17

<210> 2427
<211> 17
<212> RNA
<213> Homo sapiens

<400> 2427

acaatgcttt ttgtgca 17

<210> 2428
<211> 17
<212> RNA
<213> Homo sapiens

<400> 2428

caatgctttt tgtgcac 17

<210> 2429

<211> 17
<212> RNA
<213> Homo sapiens

<400> 2429

aatgcttttt gtgcact                                                    17

<210> 2430
<211> 17
<212> RNA
<213> Homo sapiens

<400> 2430

atgctttttg tgcacta                                                    17

<210> 2431
<211> 17
<212> RNA
<213> Homo sapiens

<400> 2431

tgtgcactac atactct                                                    17

<210> 2432
<211> 17
<212> RNA
<213> Homo sapiens

<400> 2432

cactacatac tcttcag                                                    17

<210> 2433
<211> 17
<212> RNA
<213> Homo sapiens

<400> 2433

tacatactct tcagtgt                                                    17

<210> 2434
<211> 17
<212> RNA
<213> Homo sapiens

<400> 2434

catactcttc agtgtag                                                    17

<210> 2435
<211> 17
<212> RNA
<213> Homo sapiens

<400> 2435

atactcttca gtgtaga                                                    17

<210> 2436
<211> 17
<212> RNA
<213> Homo sapiens

<400> 2436

ttcagtgtag agctctt                                                    17

<210> 2437
<211> 17
<212> RNA
<213> Homo sapiens

<400> 2437

gtagagctct tgtttta                                                    17

<210> 2438
<211> 17
<212> RNA
<213> Homo sapiens

<400> 2438

agagctcttg ttttatg                                                    17

<210> 2439
<211> 17
<212> RNA
<213> Homo sapiens

<400> 2439

gctcttgttt tatggga                                                    17

<210> 2440
<211> 17

<212> RNA
<213> Homo sapiens

<400> 2440

ctcttgtttt atgggaa                                                    17



<210> 2441
<211> 17
<212> RNA
<213> Homo sapiens

<400> 2441

tcttgtttta tgggaaa                                                    17



<210> 2442
<211> 17
<212> RNA
<213> Homo sapiens

<400> 2442

cttgttttat gggaaaa                                                    17



<210> 2443
<211> 17
<212> RNA
<213> Homo sapiens

<400> 2443

aaaaggctca aatgcca                                                    17



<210> 2444
<211> 17
<212> RNA
<213> Homo sapiens

<400> 2444

tgccaaattg tgtttga                                                    17



<210> 2445
<211> 17
<212> RNA
<213> Homo sapiens

<400> 2445

aattgtgttt gatggat                                                    17

<210> 2446
<211> 17
<212> RNA
<213> Homo sapiens

<400> 2446

attgtgtttg atggatt                                                    17

<210> 2447
<211> 17
<212> RNA
<213> Homo sapiens

<400> 2447

tgatggatta atatgcc                                                    17

<210> 2448
<211> 17
<212> RNA
<213> Homo sapiens

<400> 2448

gatggattaa tatgccc                                                    17

<210> 2449
<211> 17
<212> RNA
<213> Homo sapiens

<400> 2449

ggattaatat gcccttt                                                    17

<210> 2450
<211> 17
<212> RNA
<213> Homo sapiens

<400> 2450

tatgcccttt tgccgat                                                    17

<210> 2451
<211> 17
<212> RNA

<213> Homo sapiens

<400> 2451

atgccctttt gccgatg                                                    17

<210> 2452
<211> 17
<212> RNA
<213> Homo sapiens

<400> 2452

tgcccttttg ccgatgc                                                    17

<210> 2453
<211> 17
<212> RNA
<213> Homo sapiens

<400> 2453

cgatgcatac tattact                                                    17

<210> 2454
<211> 17
<212> RNA
<213> Homo sapiens

<400> 2454

tgcatactat tactgat                                                    17

<210> 2455
<211> 17
<212> RNA
<213> Homo sapiens

<400> 2455

catactatta ctgatgt                                                    17

<210> 2456
<211> 17
<212> RNA
<213> Homo sapiens

<400> 2456

atactattac tgatgtg                                                    17

<210> 2457
<211> 17
<212> RNA
<213> Homo sapiens

<400> 2457

atgtgactcg gttttgt 17

<210> 2458
<211> 17
<212> RNA
<213> Homo sapiens

<400> 2458

gactcggttt tgtcgca 17

<210> 2459
<211> 17
<212> RNA
<213> Homo sapiens

<400> 2459

actcggtttt gtcgcag 17

<210> 2460
<211> 17
<212> RNA
<213> Homo sapiens

<400> 2460

ctcggttttg tcgcagc 17

<210> 2461
<211> 17
<212> RNA
<213> Homo sapiens

<400> 2461

ggttttgtcg cagcttt 17

<210> 2462
<211> 17
<212> RNA
<213> Homo sapiens

<400> 2462

tcgcagcttt gctttgt                                                    17

<210> 2463
<211> 17
<212> RNA
<213> Homo sapiens

<400> 2463

cgcagctttg ctttgtt                                                    17

<210> 2464
<211> 17
<212> RNA
<213> Homo sapiens

<400> 2464

gctttgcttt gtttaat                                                    17

<210> 2465
<211> 17
<212> RNA
<213> Homo sapiens

<400> 2465

ctttgctttg tttaatg                                                    17

<210> 2466
<211> 17
<212> RNA
<213> Homo sapiens

<400> 2466

tgctttgttt aatgaaa                                                    17

<210> 2467
<211> 17
<212> RNA
<213> Homo sapiens

<400> 2467

gctttgttta atgaaac                                                    17

<210> 2468
<211> 17
<212> RNA
<213> Homo sapiens

<400> 2468

ctttgtttaa tgaaaca 17

<210> 2469
<211> 17
<212> RNA
<213> Homo sapiens

<400> 2469

aacacacttg taaacct 17

<210> 2470
<211> 17
<212> RNA
<213> Homo sapiens

<400> 2470

acacttgtaa acctctt 17

<210> 2471
<211> 17
<212> RNA
<213> Homo sapiens

<400> 2471

gtaaacctct tttgcac 17

<210> 2472
<211> 17
<212> RNA
<213> Homo sapiens

<400> 2472

aaacctcttt tgcactt 17

<210> 2473
<211> 17
<212> RNA
<213> Homo sapiens

<400> 2473

aacctctttt gcacttt                                                    17

<210> 2474
<211> 17
<212> RNA
<213> Homo sapiens

<400> 2474

acctcttttg cactttg                                                    17

<210> 2475
<211> 17
<212> RNA
<213> Homo sapiens

<400> 2475

tttgcacttt gaaaaag                                                    17

<210> 2476
<211> 17
<212> RNA
<213> Homo sapiens

<400> 2476

ttgcactttg aaaaaga                                                    17

<210> 2477
<211> 17
<212> RNA
<213> Homo sapiens

<400> 2477

aaaagaatcc agcggga                                                    17

<210> 2478
<211> 17
<212> RNA
<213> Homo sapiens

<400> 2478

gggatgctcg agcacct                                                    17

<210> 2479
<211> 17
<212> RNA
<213> Homo sapiens

<400> 2479

gcacctgtaa acaattt 17

<210> 2480
<211> 17
<212> RNA
<213> Homo sapiens

<400> 2480

taaacaattt tctcaac 17

<210> 2481
<211> 17
<212> RNA
<213> Homo sapiens

<400> 2481

aaacaatttt ctcaacc 17

<210> 2482
<211> 17
<212> RNA
<213> Homo sapiens

<400> 2482

aacaattttc tcaacct 17

<210> 2483
<211> 17
<212> RNA
<213> Homo sapiens

<400> 2483

acaattttct caaccta 17

<210> 2484
<211> 17
<212> RNA
<213> Homo sapiens

<400> 2484

aattttctca acctatt                                                    17

<210> 2485
<211> 17
<212> RNA
<213> Homo sapiens

<400> 2485

ctcaacctat ttgatgt                                                    17

<210> 2486
<211> 17
<212> RNA
<213> Homo sapiens

<400> 2486

caacctattt gatgttc                                                    17

<210> 2487
<211> 17
<212> RNA
<213> Homo sapiens

<400> 2487

aacctatttg atgttca                                                    17

<210> 2488
<211> 17
<212> RNA
<213> Homo sapiens

<400> 2488

tttgatgttc aaataaa                                                    17

<210> 2489
<211> 17
<212> RNA
<213> Homo sapiens

<400> 2489

ttgatgttca aataaag                                                    17

<210> 2490

<211> 17
<212> RNA
<213> Homo sapiens

<400> 2490

gttcaaataa agaatta                                                    17



<210> 2491
<211> 42
<212> RNA
<213> Artificial Sequence

<220>
<223> Synthesized Hairpin Ribozyme

<220>
<223> "n" represents stem IV region of a HP ribozyme.

<400> 2491

gccaggagaa guugaccaga gaaacangua cauuaccugg ua                        42



<210> 2492
<211> 42
<212> RNA
<213> Artificial Sequence

<220>
<223> Synthesized Hairpin Ribozyme

<220>
<223> "n" represents stem IV region of a HP ribozyme.

<400> 2492

cuggagagaa gccaaccaga gaaacangua cauuaccugg ua                        42



<210> 2493
<211> 42
<212> RNA
<213> Artificial Sequence

<220>
<223> Synthesized Hairpin Ribozyme

<220>
<223> "n" represents stem IV region of a HP ribozyme.

<400> 2493

cgacccagaa gagcaccaga gaaacangua cauuaccugg ua                        42

<210> 2494
<211> 42
<212> RNA
<213> Artificial Sequence

<220>
<223> Synthesized Hairpin Ribozyme

<220>
<223> "n" represents stem IV region of a HP ribozyme.

<400> 2494

cggugaagaa ggcgaccaga gaaacangua cauuaccugg ua                42

<210> 2495
<211> 42
<212> RNA
<213> Artificial Sequence

<220>
<223> Synthesized Hairpin Ribozyme

<220>
<223> "n" represents stem IV region of a HP ribozyme.

<400> 2495

ccugcgagaa ggugaccaga gaaacangua cauuaccugg ua                42

<210> 2496
<211> 42
<212> RNA
<213> Artificial Sequence

<220>
<223> Synthesized Hairpin Ribozyme

<220>
<223> "n" represents stem IV region of a HP ribozyme.

<400> 2496

gaaaccagaa ggccaccaga gaaacangua cauuaccugg ua                42

<210> 2497
<211> 42
<212> RNA
<213> Artificial Sequence

<220>
<223> Synthesized Hairpin Ribozyme

<220>
<223> "n" represents stem IV region of a HP ribozyme.

<400> 2497

cucagaagaa ggcgaccaga gaaacangua cauuaccugg ua                    42

<210> 2498
<211> 42
<212> RNA
<213> Artificial Sequence

<220>
<223> Synthesized Hairpin Ribozyme

<220>
<223> "n" represents stem IV region of a HP ribozyme.

<400> 2498

ccgcagagaa gaagaccaga gaaacangua cauuaccugg ua                    42

<210> 2499
<211> 42
<212> RNA
<213> Artificial Sequence

<220>
<223> Synthesized Hairpin Ribozyme

<220>
<223> "n" represents stem IV region of a HP ribozyme.

<400> 2499

ggugcaagaa guguaccaga gaaacangua cauuaccugg ua                    42

<210> 2500
<211> 42
<212> RNA
<213> Artificial Sequence

<220>
<223> Synthesized Hairpin Ribozyme

<220>
<223> "n" represents stem IV region of a HP ribozyme.

<400> 2500

gccgcgagaa ggguaccaga gaaacangua cauuaccugg ua                    42

<210> 2501
<211> 42
<212> RNA

<213> Artificial Sequence

<220>
<223> Synthesized Hairpin Ribozyme

<220>
<223> "n" represents stem IV region of a HP ribozyme.

<400> 2501

gucaugagaa guggaccaga gaaacangua cauuaccugg ua 42

<210> 2502
<211> 42
<212> RNA
<213> Artificial Sequence

<220>
<223> Synthesized Hairpin Ribozyme

<220>
<223> "n" represents stem IV region of a HP ribozyme.

<400> 2502

ccuuggagaa gaugaccaga gaaacangua cauuaccugg ua 42

<210> 2503
<211> 42
<212> RNA
<213> Artificial Sequence

<220>
<223> Synthesized Hairpin Ribozyme

<220>
<223> "n" represents stem IV region of a HP ribozyme.

<400> 2503

gcugcgagaa guucaccaga gaaacangua cauuaccugg ua 42

<210> 2504
<211> 42
<212> RNA
<213> Artificial Sequence

<220>
<223> Synthesized Hairpin Ribozyme

<220>
<223> "n" represents stem IV region of a HP ribozyme.

<400> 2504

aucuugagaa gcggaccaga gaaacangua cauuaccugg ua 42

<210> 2505
<211> 42
<212> RNA
<213> Artificial Sequence

<220>
<223> Synthesized Hairpin Ribozyme

<220>
<223> "n" represents stem IV region of a HP ribozyme.

<400> 2505

cccaggagaa gcucaccaga gaaacangua cauuaccugg ua 42

<210> 2506
<211> 42
<212> RNA
<213> Artificial Sequence

<220>
<223> Synthesized Hairpin Ribozyme

<220>
<223> "n" represents stem IV region of a HP ribozyme.

<400> 2506

guacacagaa ggcuaccaga gaaacangua cauuaccugg ua 42

<210> 2507
<211> 42
<212> RNA
<213> Artificial Sequence

<220>
<223> Synthesized Hairpin Ribozyme

<220>
<223> "n" represents stem IV region of a HP ribozyme.

<400> 2507

cucguaagaa gcgcaccaga gaaacangua cauuaccugg ua 42

<210> 2508
<211> 42
<212> RNA
<213> Artificial Sequence

<220>

<223> Synthesized Hairpin Ribozyme

<220>
<223> "n" represents stem IV region of a HP ribozyme.

<400> 2508

ggcggcagaa gcggaccaga gaaacangua cauuaccugg ua 42

<210> 2509
<211> 42
<212> RNA
<213> Artificial Sequence

<220>
<223> Synthesized Hairpin Ribozyme

<220>
<223> "n" represents stem IV region of a HP ribozyme.

<400> 2509

guuggcagaa gccgaccaga gaaacangua cauuaccugg ua 42

<210> 2510
<211> 42
<212> RNA
<213> Artificial Sequence

<220>
<223> Synthesized Hairpin Ribozyme

<220>
<223> "n" represents stem IV region of a HP ribozyme.

<400> 2510

aggccgagaa gaccaccaga gaaacangua cauuaccugg ua 42

<210> 2511
<211> 42
<212> RNA
<213> Artificial Sequence

<220>
<223> Synthesized Hairpin Ribozyme

<220>
<223> "n" represents stem IV region of a HP ribozyme.

<400> 2511

aggggaagaa ggucaccaga gaaacangua cauuaccugg ua 42

<210> 2512
<211> 42
<212> RNA
<213> Artificial Sequence

<220>
<223> Synthesized Hairpin Ribozyme

<220>
<223> "n" represents stem IV region of a HP ribozyme.

<400> 2512

acccggagaa guagaccaga gaaacangua cauuaccugg ua                42

<210> 2513
<211> 42
<212> RNA
<213> Artificial Sequence

<220>
<223> Synthesized Hairpin Ribozyme

<220>
<223> "n" represents stem IV region of a HP ribozyme.

<400> 2513

cguuggagaa gaacaccaga gaaacangua cauuaccugg ua                42

<210> 2514
<211> 42
<212> RNA
<213> Artificial Sequence

<220>
<223> Synthesized Hairpin Ribozyme

<220>
<223> "n" represents stem IV region of a HP ribozyme.

<400> 2514

cccccaagaa guugaccaga gaaacangua cauuaccugg ua                42

<210> 2515
<211> 42
<212> RNA
<213> Artificial Sequence

<220>
<223> Synthesized Hairpin Ribozyme

<220>

<223> "n" represents stem IV region of a HP ribozyme.

<400> 2515

agcaucagaa ggcuaccaga gaaacangua cauuaccugg ua                42

<210> 2516
<211> 42
<212> RNA
<213> Artificial Sequence

<220>
<223> Synthesized Hairpin Ribozyme

<220>
<223> "n" represents stem IV region of a HP ribozyme.

<400> 2516

aguagcagaa gcggaccaga gaaacangua cauuaccugg ua                42

<210> 2517
<211> 42
<212> RNA
<213> Artificial Sequence

<220>
<223> Synthesized Hairpin Ribozyme

<220>
<223> "n" represents stem IV region of a HP ribozyme.

<400> 2517

ugcggcagaa ggugaccaga gaaacangua cauuaccugg ua                42

<210> 2518
<211> 42
<212> RNA
<213> Artificial Sequence

<220>
<223> Synthesized Hairpin Ribozyme

<220>
<223> "n" represents stem IV region of a HP ribozyme.

<400> 2518

agcugcagaa gcggaccaga gaaacangua cauuaccugg ua                42

<210> 2519
<211> 42

<212> RNA
<213> Artificial Sequence

<220>
<223> Synthesized Hairpin Ribozyme

<220>
<223> "n" represents stem IV region of a HP ribozyme.

<400> 2519

ggcgacagaa gcggaccaga gaaacangua cauuaccugg ua 42

<210> 2520
<211> 42
<212> RNA
<213> Artificial Sequence

<220>
<223> Synthesized Hairpin Ribozyme

<220>
<223> "n" represents stem IV region of a HP ribozyme.

<400> 2520

aaaggcagaa gcugaccaga gaaacangua cauuaccugg ua 42

<210> 2521
<211> 42
<212> RNA
<213> Artificial Sequence

<220>
<223> Synthesized Hairpin Ribozyme

<220>
<223> "n" represents stem IV region of a HP ribozyme.

<400> 2521

ccguggagaa gcagaccaga gaaacangua cauuaccugg ua 42

<210> 2522
<211> 42
<212> RNA
<213> Artificial Sequence

<220>
<223> Synthesized Hairpin Ribozyme

<220>
<223> "n" represents stem IV region of a HP ribozyme.

<400> 2522

ccggcgagaa ggccaccaga gaaacangua cauuaccugg ua                    42

<210> 2523
<211> 42
<212> RNA
<213> Artificial Sequence

<220>
<223> Synthesized Hairpin Ribozyme

<220>
<223> "n" represents stem IV region of a HP ribozyme.

<400> 2523

aaugccagaa ggccaccaga gaaacangua cauuaccugg ua                    42

<210> 2524
<211> 42
<212> RNA
<213> Artificial Sequence

<220>
<223> Synthesized Hairpin Ribozyme

<220>
<223> "n" represents stem IV region of a HP ribozyme.

<400> 2524

ggagccagaa ggccaccaga gaaacangua cauuaccugg ua                    42

<210> 2525
<211> 42
<212> RNA
<213> Artificial Sequence

<220>
<223> Synthesized Hairpin Ribozyme

<220>
<223> "n" represents stem IV region of a HP ribozyme.

<400> 2525

uugcggagaa ggcaaccaga gaaacangua cauuaccugg ua                    42

<210> 2526
<211> 42
<212> RNA
<213> Artificial Sequence

<220>
<223> Synthesized Hairpin Ribozyme

<220>
<223> "n" represents stem IV region of a HP ribozyme.

<400> 2526

gucuccagaa gaccaccaga gaaacangua cauuaccugg ua                42

<210> 2527
<211> 42
<212> RNA
<213> Artificial Sequence

<220>
<223> Synthesized Hairpin Ribozyme

<220>
<223> "n" represents stem IV region of a HP ribozyme.

<400> 2527

aucaugagaa ggcuaccaga gaaacangua cauuaccugg ua                42

<210> 2528
<211> 42
<212> RNA
<213> Artificial Sequence

<220>
<223> Synthesized Hairpin Ribozyme

<220>
<223> "n" represents stem IV region of a HP ribozyme.

<400> 2528

aggccaagaa guucaccaga gaaacangua cauuaccugg ua                42

<210> 2529
<211> 42
<212> RNA
<213> Artificial Sequence

<220>
<223> Synthesized Hairpin Ribozyme

<220>
<223> "n" represents stem IV region of a HP ribozyme.

<400> 2529

ucggccagaa gggaaccaga gaaacangua cauuaccugg ua                42

<210> 2530
<211> 42
<212> RNA
<213> Artificial Sequence

<220>
<223> Synthesized Hairpin Ribozyme

<220>
<223> "n" represents stem IV region of a HP ribozyme.

<400> 2530

cuggucagaa gucaaccaga gaaacangua cauuaccugg ua                42

<210> 2531
<211> 42
<212> RNA
<213> Artificial Sequence

<220>
<223> Synthesized Hairpin Ribozyme

<220>
<223> "n" represents stem IV region of a HP ribozyme.

<400> 2531

caucugagaa gccgaccaga gaaacangua cauuaccugg ua                42

<210> 2532
<211> 42
<212> RNA
<213> Artificial Sequence

<220>
<223> Synthesized Hairpin Ribozyme

<220>
<223> "n" represents stem IV region of a HP ribozyme.

<400> 2532

cugaccagaa ggucaccaga gaaacangua cauuaccugg ua                42

<210> 2533
<211> 42
<212> RNA
<213> Artificial Sequence

<220>
<223> Synthesized Hairpin Ribozyme

<220>
<223> "n" represents stem IV region of a HP ribozyme.

<400> 2533

ugaaggagaa gguaaccaga gaaacangua cauuaccugg ua                42

<210> 2534
<211> 42
<212> RNA
<213> Artificial Sequence

<220>
<223> Synthesized Hairpin Ribozyme

<220>
<223> "n" represents stem IV region of a HP ribozyme.

<400> 2534

agguugagaa guaaaccaga gaaacangua cauuaccugg ua                42

<210> 2535
<211> 42
<212> RNA
<213> Artificial Sequence

<220>
<223> Synthesized Hairpin Ribozyme

<220>
<223> "n" represents stem IV region of a HP ribozyme.

<400> 2535

ccaaucagaa ggauaccaga gaaacangua cauuaccugg ua                42

<210> 2536
<211> 42
<212> RNA
<213> Artificial Sequence

<220>
<223> Synthesized Hairpin Ribozyme

<220>
<223> "n" represents stem IV region of a HP ribozyme.

<400> 2536

ggagcaagaa guagaccaga gaaacangua cauuaccugg ua                42

<210> 2537
<211> 42
<212> RNA
<213> Artificial Sequence

<220>
<223> Synthesized Hairpin Ribozyme

<220>
<223> "n" represents stem IV region of a HP ribozyme.

<400> 2537

guagccagaa gcauaccaga gaaacangua cauuaccugg ua                42

<210> 2538
<211> 42
<212> RNA
<213> Artificial Sequence

<220>
<223> Synthesized Hairpin Ribozyme

<220>
<223> "n" represents stem IV region of a HP ribozyme.

<400> 2538

augcggagaa gagaaccaga gaaacangua cauuaccugg ua                42

<210> 2539
<211> 42
<212> RNA
<213> Artificial Sequence

<220>
<223> Synthesized Hairpin Ribozyme

<220>
<223> "n" represents stem IV region of a HP ribozyme.

<400> 2539

gugcugagaa gaaaaccaga gaaacangua cauuaccugg ua                42

<210> 2540
<211> 42
<212> RNA
<213> Artificial Sequence

<220>
<223> Synthesized Hairpin Ribozyme

<220>
<223> "n" represents stem IV region of a HP ribozyme.

<400> 2540

uuggccagaa ggugaccaga gaaacangua cauuaccugg ua                    42

<210> 2541
<211> 42
<212> RNA
<213> Artificial Sequence

<220>
<223> Synthesized Hairpin Ribozyme

<220>
<223> "n" represents stem IV region of a HP ribozyme.

<400> 2541

ugcaggagaa ggccaccaga gaaacangua cauuaccugg ua                    42

<210> 2542
<211> 42
<212> RNA
<213> Artificial Sequence

<220>
<223> Synthesized Hairpin Ribozyme

<220>
<223> "n" represents stem IV region of a HP ribozyme.

<400> 2542

ugggccagaa gcugaccaga gaaacangua cauuaccugg ua                    42

<210> 2543
<211> 42
<212> RNA
<213> Artificial Sequence

<220>
<223> Synthesized Hairpin Ribozyme

<220>
<223> "n" represents stem IV region of a HP ribozyme.

<400> 2543

aggaggagaa gggcaccaga gaaacangua cauuaccugg ua                    42

<210> 2544
<211> 42
<212> RNA

<213> Artificial Sequence

<220>
<223> Synthesized Hairpin Ribozyme

<220>
<223> "n" represents stem IV region of a HP ribozyme.

<400> 2544

uccagcagaa ggucaccaga gaaacangua cauuaccugg ua                    42



<210> 2545
<211> 42
<212> RNA
<213> Artificial Sequence

<220>
<223> Synthesized Hairpin Ribozyme

<220>
<223> "n" represents stem IV region of a HP ribozyme.

<400> 2545

aucuccagaa gcagaccaga gaaacangua cauuaccugg ua                    42



<210> 2546
<211> 42
<212> RNA
<213> Artificial Sequence

<220>
<223> Synthesized Hairpin Ribozyme

<220>
<223> "n" represents stem IV region of a HP ribozyme.

<400> 2546

cauguaagaa guggaccaga gaaacangua cauuaccugg ua                    42



<210> 2547
<211> 42
<212> RNA
<213> Artificial Sequence

<220>
<223> Synthesized Hairpin Ribozyme

<220>
<223> "n" represents stem IV region of a HP ribozyme.

<400> 2547

gcuuugagaa gucuaccaga gaaacangua cauuaccugg ua                    42

<210> 2548
<211> 42
<212> RNA
<213> Artificial Sequence

<220>
<223> Synthesized Hairpin Ribozyme

<220>
<223> "n" represents stem IV region of a HP ribozyme.

<400> 2548

cucucaagaa guggaccaga gaaacangua cauuaccugg ua                    42

<210> 2549
<211> 42
<212> RNA
<213> Artificial Sequence

<220>
<223> Synthesized Hairpin Ribozyme

<220>
<223> "n" represents stem IV region of a HP ribozyme.

<400> 2549

uaucugagaa guguaccaga gaaacangua cauuaccugg ua                    42

<210> 2550
<211> 42
<212> RNA
<213> Artificial Sequence

<220>
<223> Synthesized Hairpin Ribozyme

<220>
<223> "n" represents stem IV region of a HP ribozyme.

<400> 2550

aaaugcagaa gggaaccaga gaaacangua cauuaccugg ua                    42

<210> 2551
<211> 42
<212> RNA
<213> Artificial Sequence

<220>

<223> Synthesized Hairpin Ribozyme

<220>
<223> "n" represents stem IV region of a HP ribozyme.

<400> 2551

gcaggaagaa gaauaccaga gaaacangua cauuaccugg ua                42

<210> 2552
<211> 42
<212> RNA
<213> Artificial Sequence

<220>
<223> Synthesized Hairpin Ribozyme

<220>
<223> "n" represents stem IV region of a HP ribozyme.

<400> 2552

acuaagagaa gagcaccaga gaaacangua cauuaccugg ua                42

<210> 2553
<211> 42
<212> RNA
<213> Artificial Sequence

<220>
<223> Synthesized Hairpin Ribozyme

<220>
<223> "n" represents stem IV region of a HP ribozyme.

<400> 2553

acagaaagaa gaagaccaga gaaacangua cauuaccugg ua                42

<210> 2554
<211> 42
<212> RNA
<213> Artificial Sequence

<220>
<223> Synthesized Hairpin Ribozyme

<220>
<223> "n" represents stem IV region of a HP ribozyme.

<400> 2554

guucccagaa gaagaccaga gaaacangua cauuaccugg ua                42

<210> 2555
<211> 42
<212> RNA
<213> Artificial Sequence

<220>
<223> Synthesized Hairpin Ribozyme

<220>
<223> "n" represents stem IV region of a HP ribozyme.

<400> 2555

aaagagagaa guuaaccaga gaaacangua cauuaccugg ua                42

<210> 2556
<211> 42
<212> RNA
<213> Artificial Sequence

<220>
<223> Synthesized Hairpin Ribozyme

<220>
<223> "n" represents stem IV region of a HP ribozyme.

<400> 2556

gaguucagaa gugaaccaga gaaacangua cauuaccugg ua                42

<210> 2557
<211> 42
<212> RNA
<213> Artificial Sequence

<220>
<223> Synthesized Hairpin Ribozyme

<220>
<223> "n" represents stem IV region of a HP ribozyme.

<400> 2557

cccauaagaa gaguaccaga gaaacangua cauuaccugg ua                42

<210> 2558
<211> 42
<212> RNA
<213> Artificial Sequence

<220>
<223> Synthesized Hairpin Ribozyme

<220>

<223> "n" represents stem IV region of a HP ribozyme.

<400> 2558

uaggggagaa gccuaccaga gaaacangua cauuaccugg ua 42

<210> 2559
<211> 42
<212> RNA
<213> Artificial Sequence

<220>
<223> Synthesized Hairpin Ribozyme

<220>
<223> "n" represents stem IV region of a HP ribozyme.

<400> 2559

cucugaagaa gcagaccaga gaaacangua cauuaccugg ua 42

<210> 2560
<211> 42
<212> RNA
<213> Artificial Sequence

<220>
<223> Synthesized Hairpin Ribozyme

<220>
<223> "n" represents stem IV region of a HP ribozyme.

<400> 2560

gccagaagaa gagcaccaga gaaacangua cauuaccugg ua 42

<210> 2561
<211> 42
<212> RNA
<213> Artificial Sequence

<220>
<223> Synthesized Hairpin Ribozyme

<220>
<223> "n" represents stem IV region of a HP ribozyme.

<400> 2561

acaaaaagaa ggaaaccaga gaaacangua cauuaccugg ua 42

<210> 2562
<211> 42

<212> RNA
<213> Artificial Sequence

<220>
<223> Synthesized Hairpin Ribozyme

<220>
<223> "n" represents stem IV region of a HP ribozyme.

<400> 2562

uccugaagaa gaccaccaga gaaacangua cauuaccugg ua 42

<210> 2563
<211> 42
<212> RNA
<213> Artificial Sequence

<220>
<223> Synthesized Hairpin Ribozyme

<220>
<223> "n" represents stem IV region of a HP ribozyme.

<400> 2563

cacuggagaa ggucaccaga gaaacangua cauuaccugg ua 42

<210> 2564
<211> 42
<212> RNA
<213> Artificial Sequence

<220>
<223> Synthesized Hairpin Ribozyme

<220>
<223> "n" represents stem IV region of a HP ribozyme.

<400> 2564

acagugagaa guucaccaga gaaacangua cauuaccugg ua 42

<210> 2565
<211> 42
<212> RNA
<213> Artificial Sequence

<220>
<223> Synthesized Hairpin Ribozyme

<220>
<223> "n" represents stem IV region of a HP ribozyme.

<400> 2565

gcucagagaa guauaccaga gaaacangua cauuaccugg ua                42

<210> 2566
<211> 42
<212> RNA
<213> Artificial Sequence

<220>
<223> Synthesized Hairpin Ribozyme

<220>
<223> "n" represents stem IV region of a HP ribozyme.

<400> 2566

gagcaaagaa ggcuaccaga gaaacangua cauuaccugg ua                42

<210> 2567
<211> 42
<212> RNA
<213> Artificial Sequence

<220>
<223> Synthesized Hairpin Ribozyme

<220>
<223> "n" represents stem IV region of a HP ribozyme.

<400> 2567

ugcgggagaa gcaaaccaga gaaacangua cauuaccugg ua                42

<210> 2568
<211> 42
<212> RNA
<213> Artificial Sequence

<220>
<223> Synthesized Hairpin Ribozyme

<220>
<223> "n" represents stem IV region of a HP ribozyme.

<400> 2568

aauaagagaa ggacaccaga gaaacangua cauuaccugg ua                42

<210> 2569
<211> 42
<212> RNA
<213> Artificial Sequence

<220>
<223> Synthesized Hairpin Ribozyme

<220>
<223> "n" represents stem IV region of a HP ribozyme.

<400> 2569

cuugacagaa gcuuaccaga gaaacangua cauuaccugg ua 42

<210> 2570
<211> 42
<212> RNA
<213> Artificial Sequence

<220>
<223> Synthesized Hairpin Ribozyme

<220>
<223> "n" represents stem IV region of a HP ribozyme.

<400> 2570

gaacacagaa gucuaccaga gaaacangua cauuaccugg ua 42

<210> 2571
<211> 42
<212> RNA
<213> Artificial Sequence

<220>
<223> Synthesized Hairpin Ribozyme

<220>
<223> "n" represents stem IV region of a HP ribozyme.

<400> 2571

gacagcagaa guccaccaga gaaacangua cauuaccugg ua 42

<210> 2572
<211> 42
<212> RNA
<213> Artificial Sequence

<220>
<223> Synthesized Hairpin Ribozyme

<220>
<223> "n" represents stem IV region of a HP ribozyme.

<400> 2572

gccccaagaa gccuaccaga gaaacangua cauuaccugg ua 42

<210> 2573
<211> 42
<212> RNA
<213> Artificial Sequence

<220>
<223> Synthesized Hairpin Ribozyme

<220>
<223> "n" represents stem IV region of a HP ribozyme.

<400> 2573

uacguaagaa gaccaccaga gaaacangua cauuaccugg ua                42

<210> 2574
<211> 42
<212> RNA
<213> Artificial Sequence

<220>
<223> Synthesized Hairpin Ribozyme

<220>
<223> "n" represents stem IV region of a HP ribozyme.

<400> 2574

gaacggagaa ggggaccaga gaaacangua cauuaccugg ua                42

<210> 2575
<211> 42
<212> RNA
<213> Artificial Sequence

<220>
<223> Synthesized Hairpin Ribozyme

<220>
<223> "n" represents stem IV region of a HP ribozyme.

<400> 2575

guagggagaa gggcaccaga gaaacangua cauuaccugg ua                42

<210> 2576
<211> 42
<212> RNA
<213> Artificial Sequence

<220>
<223> Synthesized Hairpin Ribozyme

<220>
<223> "n" represents stem IV region of a HP ribozyme.

<400> 2576

aguggaagaa guagaccaga gaaacangua cauuaccugg ua                42

<210> 2577
<211> 42
<212> RNA
<213> Artificial Sequence

<220>
<223> Synthesized Hairpin Ribozyme

<220>
<223> "n" represents stem IV region of a HP ribozyme.

<400> 2577

gaaaugagaa ggcaaccaga gaaacangua cauuaccugg ua                42

<210> 2578
<211> 42
<212> RNA
<213> Artificial Sequence

<220>
<223> Synthesized Hairpin Ribozyme

<220>
<223> "n" represents stem IV region of a HP ribozyme.

<400> 2578

agggaaagaa gcccaccaga gaaacangua cauuaccugg ua                42

<210> 2579
<211> 42
<212> RNA
<213> Artificial Sequence

<220>
<223> Synthesized Hairpin Ribozyme

<220>
<223> "n" represents stem IV region of a HP ribozyme.

<400> 2579

agcccgagaa gcuaaccaga gaaacangua cauuaccugg ua                42

<210> 2580

<211> 42
<212> RNA
<213> Artificial Sequence

<220>
<223> Synthesized Hairpin Ribozyme

<220>
<223> "n" represents stem IV region of a HP ribozyme.

<400> 2580

uggacaagaa gaaaaccaga gaaacangua cauuaccugg ua                42

<210> 2581
<211> 42
<212> RNA
<213> Artificial Sequence

<220>
<223> Synthesized Hairpin Ribozyme

<220>
<223> "n" represents stem IV region of a HP ribozyme.

<400> 2581

uaaacaagaa gcaaaccaga gaaacangua cauuaccugg ua                42

<210> 2582
<211> 42
<212> RNA
<213> Artificial Sequence

<220>
<223> Synthesized Hairpin Ribozyme

<220>
<223> "n" represents stem IV region of a HP ribozyme.

<400> 2582

auccagagaa gaauaccaga gaaacangua cauuaccugg ua                42

<210> 2583
<211> 42
<212> RNA
<213> Artificial Sequence

<220>
<223> Synthesized Hairpin Ribozyme

<220>
<223> "n" represents stem IV region of a HP ribozyme.

<400> 2583

auaggcagaa ggauaccaga gaaacangua cauuaccugg ua                    42

<210> 2584
<211> 42
<212> RNA
<213> Artificial Sequence

<220>
<223> Synthesized Hairpin Ribozyme

<220>
<223> "n" represents stem IV region of a HP ribozyme.

<400> 2584

ucaaucagaa gaugaccaga gaaacangua cauuaccugg ua                    42

<210> 2585
<211> 42
<212> RNA
<213> Artificial Sequence

<220>
<223> Synthesized Hairpin Ribozyme

<220>
<223> "n" represents stem IV region of a HP ribozyme.

<400> 2585

accaacagaa gccaaccaga gaaacangua cauuaccugg ua                    42

<210> 2586
<211> 42
<212> RNA
<213> Artificial Sequence

<220>
<223> Synthesized Hairpin Ribozyme

<220>
<223> "n" represents stem IV region of a HP ribozyme.

<400> 2586

ggggaaagaa gaggaccaga gaaacangua cauuaccugg ua                    42

<210> 2587
<211> 42
<212> RNA
<213> Artificial Sequence

<220>
<223> Synthesized Hairpin Ribozyme

<220>
<223> "n" represents stem IV region of a HP ribozyme.

<400> 2587

cuccagagaa gaagaccaga gaaacangua cauuaccugg ua 42

<210> 2588
<211> 42
<212> RNA
<213> Artificial Sequence

<220>
<223> Synthesized Hairpin Ribozyme

<220>
<223> "n" represents stem IV region of a HP ribozyme.

<400> 2588

aacugaagaa gccaaccaga gaaacangua cauuaccugg ua 42

<210> 2589
<211> 42
<212> RNA
<213> Artificial Sequence

<220>
<223> Synthesized Hairpin Ribozyme

<220>
<223> "n" represents stem IV region of a HP ribozyme.

<400> 2589

cucuagagaa gaagaccaga gaaacangua cauuaccugg ua 42

<210> 2590
<211> 42
<212> RNA
<213> Artificial Sequence

<220>
<223> Synthesized Hairpin Ribozyme

<220>
<223> "n" represents stem IV region of a HP ribozyme.

<400> 2590

aucaaaagaa gcacaccaga gaaacangua cauuaccugg ua 42

<210> 2591
<211> 42
<212> RNA
<213> Artificial Sequence

<220>
<223> Synthesized Hairpin Ribozyme

<220>
<223> "n" represents stem IV region of a HP ribozyme.

<400> 2591

uuuguaagaa guguaccaga gaaacangua cauuaccugg ua                42

<210> 2592
<211> 42
<212> RNA
<213> Artificial Sequence

<220>
<223> Synthesized Hairpin Ribozyme

<220>
<223> "n" represents stem IV region of a HP ribozyme.

<400> 2592

aagcagagaa gccuaccaga gaaacangua cauuaccugg ua                42

<210> 2593
<211> 42
<212> RNA
<213> Artificial Sequence

<220>
<223> Synthesized Hairpin Ribozyme

<220>
<223> "n" represents stem IV region of a HP ribozyme.

<400> 2593

uccccaagaa gaucaccaga gaaacangua cauuaccugg ua                42

<210> 2594
<211> 42
<212> RNA
<213> Artificial Sequence

<220>
<223> Synthesized Hairpin Ribozyme

<220>
<223> "n" represents stem IV region of a HP ribozyme.

<400> 2594

guuggaagaa gagcaccaga gaaacangua cauuaccugg ua                42

<210> 2595
<211> 42
<212> RNA
<213> Artificial Sequence

<220>
<223> Synthesized Hairpin Ribozyme

<220>
<223> "n" represents stem IV region of a HP ribozyme.

<400> 2595

aauagaagaa gcaaaccaga gaaacangua cauuaccugg ua                42

<210> 2596
<211> 42
<212> RNA
<213> Artificial Sequence

<220>
<223> Synthesized Hairpin Ribozyme

<220>
<223> "n" represents stem IV region of a HP ribozyme.

<400> 2596

ucaaaaagaa gugcaccaga gaaacangua cauuaccugg ua                42

<210> 2597
<211> 42
<212> RNA
<213> Artificial Sequence

<220>
<223> Synthesized Hairpin Ribozyme

<220>
<223> "n" represents stem IV region of a HP ribozyme.

<400> 2597

aaagggagaa guguaccaga gaaacangua cauuaccugg ua                42

<210> 2598
<211> 42
<212> RNA
<213> Artificial Sequence

<220>
<223> Synthesized Hairpin Ribozyme

<220>
<223> "n" represents stem IV region of a HP ribozyme.

<400> 2598

aacagaagaa ggcaaccaga gaaacangua cauuaccugg ua 42

<210> 2599
<211> 42
<212> RNA
<213> Artificial Sequence

<220>
<223> Synthesized Hairpin Ribozyme

<220>
<223> "n" represents stem IV region of a HP ribozyme.

<400> 2599

ugagagagaa gaaaaccaga gaaacangua cauuaccugg ua 42

<210> 2600
<211> 42
<212> RNA
<213> Artificial Sequence

<220>
<223> Synthesized Hairpin Ribozyme

<220>
<223> "n" represents stem IV region of a HP ribozyme.

<400> 2600

guaugcagaa gcaaaccaga gaaacangua cauuaccugg ua 42

<210> 2601
<211> 42
<212> RNA
<213> Artificial Sequence

<220>
<223> Synthesized Hairpin Ribozyme

<220>
<223> "n" represents stem IV region of a HP ribozyme.

<400> 2601

agucacagaa guaaaccaga gaaacangua cauuaccugg ua                    42



<210> 2602
<211> 42
<212> RNA
<213> Artificial Sequence

<220>
<223> Synthesized Hairpin Ribozyme

<220>
<223> "n" represents stem IV region of a HP ribozyme.

<400> 2602

cgacaaagaa gaguaccaga gaaacangua cauuaccugg ua                    42



<210> 2603
<211> 42
<212> RNA
<213> Artificial Sequence

<220>
<223> Synthesized Hairpin Ribozyme

<220>
<223> "n" represents stem IV region of a HP ribozyme.

<400> 2603

aagcaaagaa gcgaaccaga gaaacangua cauuaccugg ua                    42



<210> 2604
<211> 14
<212> RNA
<213> Homo Sapiens

<400> 2604

caacagtccc tggc                                                   14



<210> 2605
<211> 14
<212> RNA
<213> Homo sapiens

<400> 2605

tggccgtcct ccag                                                   14

<210> 2606
<211> 14
<212> RNA
<213> Homo sapiens

<400> 2606

gctccgtcgg gtcg                                                          14

<210> 2607
<211> 14
<212> RNA
<213> Homo sapiens

<400> 2607

cgccggcttc accg                                                          14

<210> 2608
<211> 14
<212> RNA
<213> Homo sapiens

<400> 2608

caccggaccg cagg                                                          14

<210> 2609
<211> 14
<212> RNA
<213> Homo sapiens

<400> 2609

ggcccgccgg tttc                                                          14

<210> 2610
<211> 14
<212> RNA
<213> Homo sapiens

<400> 2610

cgccggtttc tgag                                                          14

<210> 2611
<211> 14
<212> RNA
<213> Homo sapiens

<400> 2611

cttctgccct gcgg                                                          14

<210> 2612
<211> 14
<212> RNA
<213> Homo sapiens

<400> 2612

acacggtctg cacc                                                          14

<210> 2613
<211> 14
<212> RNA
<213> Homo sapiens

<400> 2613

accctgcccg cggc                                                          14

<210> 2614
<211> 14
<212> RNA
<213> Homo sapiens

<400> 2614

ccacggacca tgac                                                          14

<210> 2615
<211> 14
<212> RNA
<213> Homo sapiens

<400> 2615

catcagatcc aagg                                                          14

<210> 2616
<211> 14
<212> RNA
<213> Homo sapiens

<400> 2616

gaaccgtccg cagc                                                          14

<210> 2617
<211> 14
<212> RNA
<213> Artificial Sequence

<400> 2617

ccgcagctca agat                                                    14

<210> 2618
<211> 14
<212> RNA
<213> Homo sapiens

<400> 2618

gagcggcccc tggg                                                    14

<210> 2619
<211> 14
<212> RNA
<213> Homo sapiens

<400> 2619

agcccgccgt gtac                                                    14

<210> 2620
<211> 14
<212> RNA
<213> Homo sapiens

<400> 2620

gcgccgccta cgag                                                    14

<210> 2621
<211> 14
<212> RNA
<213> Homo sapiens

<400> 2621

ccgcggccgc cgcc                                                    14

<210> 2622
<211> 14
<212> RNA
<213> Homo sapiens

<400> 2622

cggccgccgc caac                                                          14

<210> 2623
<211> 14
<212> RNA
<213> Homo sapiens

<400> 2623

ggtcagaccg gcct                                                          14

<210> 2624
<211> 14
<212> RNA
<213> Homo sapiens

<400> 2624

gaccggcctc ccct                                                          14

<210> 2625
<211> 14
<212> RNA
<213> Homo sapiens

<400> 2625

ctacggcccc gggt                                                          14

<210> 2626
<211> 14
<212> RNA
<213> Homo sapiens

<400> 2626

gttcggctcc aacg                                                          14

<210> 2627
<211> 14
<212> RNA
<213> Homo sapiens

<400> 2627

caacggcctg gggg                                                          14

<210> 2628
<211> 14
<212> RNA
<213> Homo sapiens

<400> 2628

agcccgctga tgct                                                         14

<210> 2629
<211> 14
<212> RNA
<213> Homo sapiens

<400> 2629

ccgctgatgc tact                                                         14

<210> 2630
<211> 14
<212> RNA
<213> Homo sapiens

<400> 2630

cacccgccgc cgca                                                         14

<210> 2631
<211> 14
<212> RNA
<213> Homo sapiens

<400> 2631

ccgccgccgc agct                                                         14

<210> 2632
<211> 14
<212> RNA
<213> Homo sapiens

<400> 2632

ccgcagctgt cgcc                                                         14

<210> 2633
<211> 14
<212> RNA
<213> Homo sapiens

<400> 2633

cagctgtcgc cttt 14

<210> 2634
<211> 14
<212> RNA
<213> Homo sapiens

<400> 2634

ctgcagcccc acgg 14

<210> 2635
<211> 14
<212> RNA
<213> Homo sapiens

<400> 2635

ggccggcccg ccgg 14

<210> 2636
<211> 14
<212> RNA
<213> Homo sapiens

<400> 2636

ggcccgccgg catt 14

<210> 2637
<211> 14
<212> RNA
<213> Homo sapiens

<400> 2637

ggcctgccgg ctcc 14

<210> 2638
<211> 14
<212> RNA
<213> Homo sapiens

<400> 2638

tgccggctcc gcaa 14

<210> 2639

<211> 14
<212> RNA
<213> Homo sapiens

<400> 2639

ggtctgctgg agac                                                          14

<210> 2640
<211> 14
<212> RNA
<213> Homo sapiens

<400> 2640

agcccgctca tgat                                                          14

<210> 2641
<211> 14
<212> RNA
<213> Homo sapiens

<400> 2641

gaacagcctg gcct                                                          14

<210> 2642
<211> 14
<212> RNA
<213> Homo sapiens

<400> 2642

tccctgacgg ccga                                                          14

<210> 2643
<211> 14
<212> RNA
<213> Homo sapiens

<400> 2643

tgacggccga ccag                                                          14

<210> 2644
<211> 14
<212> RNA
<213> Homo sapiens

<400> 2644

cggccgacca gatg                                                          14

<210> 2645
<211> 14
<212> RNA
<213> Homo sapiens

<400> 2645

gaccagatgg tcag                                                          14

<210> 2646
<211> 14
<212> RNA
<213> Homo sapiens

<400> 2646

taccagaccc ttca                                                          14

<210> 2647
<211> 14
<212> RNA
<213> Homo sapiens

<400> 2647

ttactgacca acct                                                          14

<210> 2648
<211> 14
<212> RNA
<213> Homo sapiens

<400> 2648

atcctgatga ttgg                                                          14

<210> 2649
<211> 14
<212> RNA
<213> Homo sapiens

<400> 2649

ctactgtttg ctcc                                                          14

<210> 2650
<211> 14

<212> RNA
<213> Homo sapiens

<400> 2650

atgctgctgg ctac                                                          14

<210> 2651
<211> 14
<212> RNA
<213> Homo sapiens

<400> 2651

tctcggttcc gcat                                                          14

<210> 2652
<211> 14
<212> RNA
<213> Homo sapiens

<400> 2652

tttctgtcca gcac                                                          14

<210> 2653
<211> 14
<212> RNA
<213> Homo sapiens

<400> 2653

cacctgatgg ccaa                                                          14

<210> 2654
<211> 14
<212> RNA
<213> Homo sapiens

<400> 2654

ggcctgaccc tgca                                                          14

<210> 2655
<211> 14
<212> RNA
<213> Homo sapiens

<400> 2655

cagcggctgg ccca                                                          14

<210> 2656
<211> 14
<212> RNA
<213> Homo sapiens

<400> 2656

gcccagctcc tcct 14

<210> 2657
<211> 14
<212> RNA
<213> Homo sapiens

<400> 2657

gacctgctgc tgga 14

<210> 2658
<211> 14
<212> RNA
<213> Homo sapiens

<400> 2658

ctgctgctgg agat 14

<210> 2659
<211> 14
<212> RNA
<213> Homo sapiens

<400> 2659

ccaccgccta catg 14

<210> 2660
<211> 14
<212> RNA
<213> Homo sapiens

<400> 2660

agacggacca aagc 14

<210> 2661
<211> 14
<212> RNA

<213> Homo sapiens

<400> 2661

ccacagtctg agag                                                          14

<210> 2662
<211> 14
<212> RNA
<213> Homo sapiens

<400> 2662

acacggttca gata                                                          14

<210> 2663
<211> 14
<212> RNA
<213> Homo sapiens

<400> 2663

tccctgctgc attt                                                          14

<210> 2664
<211> 14
<212> RNA
<213> Homo sapiens

<400> 2664

attctgtctc ctgc                                                          14

<210> 2665
<211> 14
<212> RNA
<213> Homo sapiens

<400> 2665

gctcagttct tagt                                                          14

<210> 2666
<211> 14
<212> RNA
<213> Homo sapiens

<400> 2666

cttctgtctt ctgt                                                          14

<210> 2667
<211> 14
<212> RNA
<213> Homo sapiens

<400> 2667

cttctgttgg gaac                                                          14

<210> 2668
<211> 14
<212> RNA
<213> Homo sapiens

<400> 2668

taacagctct cttt                                                          14

<210> 2669
<211> 14
<212> RNA
<213> Homo sapiens

<400> 2669

tcacagctga actc                                                          14

<210> 2670
<211> 14
<212> RNA
<213> Homo sapiens

<400> 2670

actcagtcta tggg                                                          14

<210> 2671
<211> 14
<212> RNA
<213> Homo sapiens

<400> 2671

aggcagatcc ccta                                                          14

<210> 2672
<211> 14
<212> RNA
<213> Homo sapiens

<400> 2672

ctgcagattc agag                                                          14

<210> 2673
<211> 14
<212> RNA
<213> Homo sapiens

<400> 2673

gctctgcctc tggc                                                          14

<210> 2674
<211> 14
<212> RNA
<213> Homo sapiens

<400> 2674

ttcctgattt ttgt                                                          14

<210> 2675
<211> 14
<212> RNA
<213> Homo sapiens

<400> 2675

ggtcagcttc agga                                                          14

<210> 2676
<211> 14
<212> RNA
<213> Homo sapiens

<400> 2676

gacctgttcc agtg                                                          14

<210> 2677
<211> 14
<212> RNA
<213> Homo sapiens

<400> 2677

gaactgttca ctgt                                                          14

<210> 2678
<211> 14
<212> RNA
<213> Homo sapiens

<400> 2678

atacagttct gagc 14

<210> 2679
<211> 14
<212> RNA
<213> Homo sapiens

<400> 2679

agccagactt gctc 14

<210> 2680
<211> 14
<212> RNA
<213> Homo sapiens

<400> 2680

ttgcagaccc cgca 14

<210> 2681
<211> 14
<212> RNA
<213> Homo sapiens

<400> 2681

gtccagctct tatt 14

<210> 2682
<211> 14
<212> RNA
<213> Homo sapiens

<400> 2682

aagcagctgt caag 14

<210> 2683
<211> 14
<212> RNA
<213> Homo sapiens

<400> 2683

agacagctgt gttc                                                         14

<210> 2684
<211> 14
<212> RNA
<213> Homo sapiens

<400> 2684

ggaccgttgc tgtc                                                         14

<210> 2685
<211> 14
<212> RNA
<213> Homo sapiens

<400> 2685

aggctgactg gggc                                                         14

<210> 2686
<211> 14
<212> RNA
<213> Homo sapiens

<400> 2686

ggtcagatta cgta                                                         14

<210> 2687
<211> 14
<212> RNA
<213> Homo sapiens

<400> 2687

cccccgcccc gttc                                                         14

<210> 2688
<211> 14
<212> RNA
<213> Homo sapiens

<400> 2688

gccccgttcc ctac                                                         14

<210> 2689
<211> 14
<212> RNA
<213> Homo sapiens

<400> 2689

ctaccgcctc cact 14

<210> 2690
<211> 14
<212> RNA
<213> Homo sapiens

<400> 2690

tgccagctca tttc 14

<210> 2691
<211> 14
<212> RNA
<213> Homo sapiens

<400> 2691

gggcagcctt ccct 14

<210> 2692
<211> 14
<212> RNA
<213> Homo sapiens

<400> 2692

tagctgctcg ggct 14

<210> 2693
<211> 14
<212> RNA
<213> Homo sapiens

<400> 2693

tttctgattg tcca 14

<210> 2694
<211> 14
<212> RNA
<213> Homo sapiens

<400> 2694

ttgctgtttg ttta 14

<210> 2695
<211> 14
<212> RNA
<213> Homo sapiens

<400> 2695

attctgttct ggat 14

<210> 2696
<211> 14
<212> RNA
<213> Homo sapiens

<400> 2696

atccagatgc ctat 14

<210> 2697
<211> 14
<212> RNA
<213> Homo sapiens

<400> 2697

catcagatga ttga 14

<210> 2698
<211> 14
<212> RNA
<213> Homo sapiens

<400> 2698

tggctgatgt tggt 14

<210> 2699
<211> 14
<212> RNA
<213> Homo sapiens

<400> 2699

cctctgcttt cccc 14

<210> 2700

<211> 14
<212> RNA
<213> Homo sapiens

<400> 2700

cttctgccct ggag                                                          14

<210> 2701
<211> 14
<212> RNA
<213> Homo sapiens

<400> 2701

tggcagcttc agtt                                                          14

<210> 2702
<211> 14
<212> RNA
<213> Homo sapiens

<400> 2702

cttcagttct agag                                                          14

<210> 2703
<211> 14
<212> RNA
<213> Homo sapiens

<400> 2703

gtgcagtctt tgat                                                          14

<210> 2704
<211> 14
<212> RNA
<213> Homo sapiens

<400> 2704

acactgatta caaa                                                          14

<210> 2705
<211> 14
<212> RNA
<213> Homo sapiens

<400> 2705

aggcagatct gctt                                                    14

<210> 2706
<211> 14
<212> RNA
<213> Homo sapiens

<400> 2706

gatctgcttg ggga                                                    14

<210> 2707
<211> 14
<212> RNA
<213> Homo sapiens

<400> 2707

gctctgtttc caac                                                    14

<210> 2708
<211> 14
<212> RNA
<213> Homo sapiens

<400> 2708

ttgctgcctc tatt                                                    14

<210> 2709
<211> 14
<212> RNA
<213> Homo sapiens

<400> 2709

gcactgtctt ttga                                                    14

<210> 2710
<211> 14
<212> RNA
<213> Homo sapiens

<400> 2710

acacagaccc cttt                                                    14

<210> 2711
<211> 14

<212> RNA
<213> Homo sapiens

<400> 2711

tgccagtttc tgtt                                                          14

<210> 2712
<211> 14
<212> RNA
<213> Homo sapiens

<400> 2712

tttctgttct ctca                                                          14

<210> 2713
<211> 14
<212> RNA
<213> Homo sapiens

<400> 2713

ttgccgatgc atac                                                          14

<210> 2714
<211> 14
<212> RNA
<213> Homo sapiens

<400> 2714

ttactgatgt gact                                                          14

<210> 2715
<211> 14
<212> RNA
<213> Homo sapiens

<400> 2715

actcggtttt gtcg                                                          14

<210> 2716
<211> 14
<212> RNA
<213> Homo sapiens

<400> 2716

tcgcagcttt gctt                                                          14

<210> 2717
<211> 20
<212> RNA
<213> Artificial Sequence

<220>
<223> Synthesized nucleic acid molecule

<400> 2717

ucccgcctgt gacaugcaut                                                20

<210> 2718
<211> 25
<212> RNA
<213> Artificial Sequence

<220>
<223> Synthesized nucleic acid molecule

<400> 2718

agcauccaac aaggcacuga ccauc                                          25

<210> 2719
<211> 25
<212> RNA
<213> Artificial Sequence

<220>
<223> Synthesized nucleic acid molecule

<400> 2719

cagcauccaa caaggcacug accau                                          25

<210> 2720
<211> 25
<212> RNA
<213> Artificial Sequence

<220>
<223> Synthesized nucleic acid molecule

<400> 2720

cugccaggut ggtcaguaag cccau                                          25

<210> 2721
<211> 25

<212> RNA
<213> Artificial Sequence

<220>
<223> Synthesized nucleic acid molecule

<400> 2721

uuucccuggt tcctgtccaa gagca                                          25



<210> 2722
<211> 23
<212> RNA
<213> Artificial Sequence

<220>
<223> Synthesized nucleic acid molecule

<400> 2722

ccagcauctc cagcagcagg uca                                            23



<210> 2723
<211> 23
<212> RNA
<213> Artificial Sequence

<220>
<223> Synthesized nucleic acid molecule

<400> 2723

cguccagcat ctccagcagc agg                                            23



<210> 2724
<211> 38
<212> RNA
<213> Artificial Sequence

<220>
<223> Synthesized nucleic acid molecule

<400> 2724

atagatttcu gaugaggccg aaaggccgaa aggcacac                            38



<210> 2725
<211> 41
<212> RNA
<213> Artificial Sequence

<220>
<223> Synthesized nucleic acid molecule

<400> 2725

ggucagucug augaggccgu uaggccgaaa gcccatcatc g                                41

<210> 2726
<211> 41
<212> RNA
<213> Artificial Sequence

<220>
<223> Synthesized nucleic acid molecule

<400> 2726

gucggcccuu augaggccgu uaggccgaua cagutagcct a                                41

<210> 2727
<211> 38
<212> RNA
<213> Artificial Sequence

<220>
<223> Synthesized nucleic acid molecule

<400> 2727

atagatttcu gaugaggccg aaaggccgaa aggcacac                                    38

<210> 2728
<211> 38
<212> RNA
<213> Artificial Sequence

<220>
<223> Synthesized nucleic acid molecule

<400> 2728

gcggaacccu gaugaggccg aaaggccgaa agaugaug                                    38

<210> 2729
<211> 36
<212> RNA
<213> Artificial Sequence

<220>
<223> Synthesized nucleic acid molecule

<400> 2729

tttccgacug augaggccga aaggccgaaa gacaca                                      36

<210> 2730
<211> 36
<212> RNA
<213> Artificial Sequence

<220>
<223> Synthesized nucleic acid molecule

<400> 2730

attcctgcug augaggccga aaggccgaaa uuccuu                                36

<210> 2731
<211> 23
<212> RNA
<213> Artificial Sequence

<220>
<223> Synthesized nucleic acid molecule

<400> 2731

cgaauugcat cctgaaacag aaa                                              23

<210> 2732
<211> 23
<212> RNA
<213> Artificial Sequence

<220>
<223> Synthesized nucleic acid molecule

<400> 2732

guacctgatt cgctgtgacu ucg                                              23

<210> 2733
<211> 23
<212> RNA
<213> Artificial Sequence

<220>
<223> Synthesized nucleic acid molecule

<400> 2733

gcaccagcac agacttaccu gat                                              23

<210> 2734
<211> 23
<212> RNA

<213> Artificial Sequence

<220>
<223> Synthesized nucleic acid molecule

<400> 2734

uagcagccct gagccuuacc ugg                                              23



<210> 2735
<211> 23
<212> RNA
<213> Artificial Sequence

<220>
<223> Synthesized nucleic acid molecule

<400> 2735

caggauctga aacaaagccc aag                                              23



<210> 2736
<211> 23
<212> RNA
<213> Artificial Sequence

<220>
<223> Synthesized nucleic acid molecule

<400> 2736

ugccaucttt acgaaccaac cca                                              23



<210> 2737
<211> 23
<212> RNA
<213> Artificial Sequence

<220>
<223> Synthesized nucleic acid molecule

<400> 2737

guggtcagcg tgcaagcauu gat                                              23



<210> 2738
<211> 20
<212> RNA
<213> Artificial Sequence

<220>
<223> Synthesized nucleic acid molecule

<400> 2738

tcccgcctgt gacatgcatt                                                    20

<210> 2739
<211> 20
<212> RNA
<213> Artificial Sequence

<220>
<223> Synthesized nucleic acid molecule

<400> 2739

tccgccgtct cagatcgatt                                                    20

<210> 2740
<211> 23
<212> RNA
<213> Artificial Sequence

<220>
<223> Synthesized nucleic acid molecule

<400> 2740

agucccgcct gtgacaugca uuc                                                23

<210> 2741
<211> 23
<212> RNA
<213> Artificial Sequence

<220>
<223> Synthesized nucleic acid molecule

<400> 2741

agucccgcct gtgacaugca uuc                                                23

<210> 2742
<211> 23
<212> RNA
<213> Artificial Sequence

<220>
<223> Synthesized nucleic acid molecule

<400> 2742

agucccgcct gtgacaugca uuc                                                23

<210> 2743
<211> 23
<212> RNA
<213> Artificial Sequence

<220>
<223> Synthesized nucleic acid molecule

<400> 2743

agucccgcct gtgacaugca uuc                                              23

<210> 2744
<211> 23
<212> RNA
<213> Artificial Sequence

<220>
<223> Synthesized nucleic acid molecule

<400> 2744

agucccgcct gtgacaugca uuc                                              23

<210> 2745
<211> 23
<212> RNA
<213> Artificial Sequence

<220>
<223> Synthesized nucleic acid molecule

<400> 2745

gauccgccgt ctcagaucga ucu                                              23

<210> 2746
<211> 23
<212> RNA
<213> Artificial Sequence

<220>
<223> Synthesized nucleic acid molecule

<400> 2746

gauccgccgt ctcagaucga ucu                                              23

<210> 2747
<211> 23
<212> RNA
<213> Artificial Sequence

<220>
<223> Synthesized nucleic acid molecule

<400> 2747

gauccgccgt ctcagaucga ucu                                              23



<210> 2748
<211> 23
<212> RNA
<213> Artificial Sequence

<220>
<223> Synthesized nucleic acid molecule

<400> 2748

gauccgccgt ctcagaucga ucu                                              23



<210> 2749
<211> 26
<212> RNA
<213> Artificial Sequence

<220>
<223> Synthesized nucleic acid molecule

<220>
<223> The letter "s" stands for g or c.

<400> 2749

cccaccgaac tcaaagaagg scscsa                                           26



<210> 2750
<211> 24
<212> RNA
<213> Artificial Sequence

<220>
<223> Synthesized nucleic acid molecule

<220>
<223> The letter "s" stands for g or c.

<400> 2750

aagcgaccta aagcaacccc sagc                                             24



<210> 2751
<211> 23
<212> RNA

<213> Artificial Sequence

<220>
<223> Synthesized nucleic acid molecule

<400> 2751

cccaccgaac tcaaagaagg cca                                              23

<210> 2752
<211> 23
<212> RNA
<213> Artificial Sequence

<220>
<223> Synthesized nucleic acid molecule

<400> 2752

aagcgaccta aagcaacccc agc                                              23

<210> 2753
<211> 23
<212> RNA
<213> Artificial Sequence

<220>
<223> Synthesized nucleic acid molecule

<400> 2753

cccaccgaac tcaaagaagg cca                                              23

<210> 2754
<211> 21
<212> RNA
<213> Artificial Sequence

<220>
<223> Synthesized nucleic acid molecule

<400> 2754

ccaccagctc caactaccac a                                                21

<210> 2755
<211> 21
<212> RNA
<213> Artificial Sequence

<220>
<223> Synthesized nucleic acid molecule

<400> 2755

tggcaaatac acaaagaaag c                                                21

<210> 2756
<211> 21
<212> RNA
<213> Artificial Sequence

<220>
<223> Synthesized nucleic acid molecule

<400> 2756

ccataactcc ttgctaactc c                                                21

<210> 2757
<211> 21
<212> RNA
<213> Artificial Sequence

<220>
<223> Synthesized nucleic acid molecule

<400> 2757

caccctgtct tgtcttcgct g                                                21

<210> 2758
<211> 25
<212> RNA
<213> Artificial Sequence

<220>
<223> Synthesized nucleic acid molecule

<400> 2758

cugccaggut ggtcaguaag cccau                                            25

<210> 2759
<211> 23
<212> RNA
<213> Artificial Sequence

<220>
<223> Synthesized nucleic acid molecule

<400> 2759

aguuucaatc ttcuaaauug gca                                              23

<210> 2760
<211> 23
<212> RNA
<213> Artificial Sequence

<220>
<223> Synthesized nucleic acid molecule

<400> 2760

tgccaggttg gtcagtaagc cca                                              23

<210> 2761
<211> 22
<212> RNA
<213> Artificial Sequence

<220>
<223> Synthesized nucleic acid molecule

<400> 2761

tcgcatgtgc tgagatacgc ac                                               22

<210> 2762
<211> 23
<212> RNA
<213> Artificial Sequence

<220>
<223> Synthesized nucleic acid molecule

<400> 2762

ugccaggttg gtcagtaagc cca                                              23

<210> 2763
<211> 23
<212> RNA
<213> Artificial Sequence

<220>
<223> Synthesized nucleic acid molecule

<400> 2763

ucgcgaugtg ctgagauacg cac                                              23

<210> 2764
<211> 23
<212> RNA
<213> Artificial Sequence

<220>
<223> Synthesized nucleic acid molecule

<400> 2764

ugccaggttg gtcagtaagc cca                                              23

<210> 2765
<211> 23
<212> RNA
<213> Artificial Sequence

<220>
<223> Synthesized nucleic acid molecule

<400> 2765

gauccgccgt ctcagaucga ucu                                              23

<210> 2766
<211> 23
<212> RNA
<213> Artificial Sequence

<220>
<223> Synthesized nucleic acid molecule

<400> 2766

ugccaggttg gtcagtaagc cca                                              23

<210> 2767
<211> 23
<212> RNA
<213> Artificial Sequence

<220>
<223> Synthesized nucleic acid molecule

<400> 2767

ucgcgaugtg ctgagauacg cac                                              23

<210> 2768
<211> 23
<212> RNA
<213> Artificial Sequence

<220>
<223> Synthesized nucleic acid molecule

<400> 2768

ugccaggttg gtcagtaagc cca                                                23

<210> 2769
<211> 23
<212> RNA
<213> Artificial Sequence

<220>
<223> Synthesized nucleic acid molecule

<400> 2769

ucgcgaugtg ctgagauacg cac                                                23

<210> 2770
<211> 23
<212> RNA
<213> Artificial Sequence

<220>
<223> Synthesized nucleic acid molecule

<400> 2770

gaaaugcaaa guuccagaau cgg                                                23

<210> 2771
<211> 17
<212> RNA
<213> Artificial Sequence

<220>
<223> Synthesized nucleic acid molecule

<400> 2771

uccucgcugg ggcuaag                                                       17

<210> 2772
<211> 13
<212> RNA
<213> Artificial Sequence

<220>
<223> Synthesized nucleic acid molecule

<220>
<223> The letter "n" stands for any base.

<400> 2772

cugangagcg aaa                                                           13

<210> 2773
<211> 15
<212> RNA
<213> Artificial Sequence

<220>
<223> Synthesized nucleic acid molecule

<220>
<223> The letter "n" stands for any  base;
      The letter "y" stands for c or u;
      The letter "h" stands for a, c or u.

<400> 2773

nnnnnnnyng hynnn                                                        15



<210> 2774
<211> 47
<212> RNA
<213> Artificial Sequence

<220>
<223> Synthesized nucleic acid molecule

<220>
<223> The letter "n" stands for any base;
      The letter "h" stands for a, c or u.

<400> 2774
   nnnngaagnn nnnnnnnnna aahannnnnn nacauuacnn nnnnnnn                       47



<210> 2775
<211> 48
<212> DNA
<213> Artificial Sequence

<220>
<223> Synthesized nucleic acid molecule

<220>
<223> The letter "n" stands for any base.

<400> 2775

cnnnnnnnnn nnnnnnnnnn nncugangag nnnnnncgaa annnnnnc                    48



<210> 2776
<211> 48
<212> DNA
<213> Artificial Sequence

<220>
<223> Synthesized nucleic acid molecule

<220>
<223> The letter "n" stands for any base.

<400> 2776

cnnnnnnncu gangagnnnn nncgaaannn nnnnnnnnnn nnnnnnnc 48

<210> 2777
<211> 41
<212> DNA
<213> Artificial Sequence

<220>
<223> Synthesized nucleic acid molecule

<220>
<223> The letter "n" stands for any base.

<400> 2777

cnnnnnnncu gangagnnnn nncgaaannn nnnnnnnnnn c 41

<210> 2778
<211> 34
<212> DNA
<213> Artificial Sequence

<220>
<223> Synthesized nucleic acid molecule

<220>
<223> The letter "n" stands for any base.

<400> 2778

cnnnnnncug angagnnnnn ncgaaannnn nnnc 34

<210> 2779
<211> 32
<212> DNA
<213> Artificial Sequence

<220>
<223> Synthesized nucleic acid molecule

<220>
<223> The letter "n" stands for any base.

<400> 2779

cnnnncugan gagnnnnnnc gaaannnnnn nc 32

<210> 2780
<211> 33
<212> DNA
<213> Artificial Sequence

<220>
<223> Synthesized nucleic acid molecule

<220>
<223> The letter "n" stands for any base.

<400> 2780

nnnnnnncug angagnnnnn ncgaaannnn nnc                                    33

<210> 2781
<211> 41
<212> DNA
<213> Artificial Sequence

<220>
<223> Synthesized nucleic acid molecule

<220>
<223> The letter "n" stands for any base.

<400> 2781

cnnnnnnnnn nnnnncugan gagnnnnnnc gaaannnnnn c                           41

<210> 2782
<211> 30
<212> DNA
<213> Artificial Sequence

<220>
<223> Synthesized nucleic acid molecule

<220>
<223> The letter "n" stands for any base.

<400> 2782

nnnnnnncug angagnnnnn ncgaaannnc                                        30

<210> 2783
<211> 41
<212> DNA
<213> Artificial Sequence

<220>
<223> Synthesized nucleic acid molecule

<220>
<223> The letter "n" stands for any base.

<400> 2783

cnnnnnnnnn nnnnncugan gagnnnnnnc gaaannnnnn c                41

<210> 2784
<211> 41
<212> DNA
<213> Artificial Sequence

<220>
<223> Synthesized nucleic acid molecule

<220>
<223> The letter "n" stands for any base.

<400> 2784

cnnnnnnncu gangagnnnn nncgaaannn nnnnnnnnnn c                41

<210> 2785
<211> 45
<212> DNA
<213> Artificial Sequence

<220>
<223> Synthesized nucleic acid molecule

<220>
<223> The letter "n" stands for any base.

<400> 2785

cnnnncugan gagnnnnnnc gaaannnnnn nnnnnnnnnn nnnnc            45

<210> 2786
<211> 45
<212> DNA
<213> Artificial Sequence

<220>
<223> Synthesized nucleic acid molecule

<220>
<223> The letter "n" stands for any base.

<400> 2786

cnnnnnnnnn nnnnnnnnnn nncugangag nnnnnncgaa annnc            45